(19) Europäisches Patentamt
European Patent Office
Office européen des brevets

(11) EP 4 755 880 A1

(12) **EUROPEAN PATENT APPLICATION**
published in accordance with Art. 153(4) EPC

(43) Date of publication:
**10.06.2026 Bulletin 2026/24**

(21) Application number: **24848152.5**

(22) Date of filing: **26.07.2024**

(51) International Patent Classification (IPC):
***C07D 401/12*** *(2006.01)* ***C07D 401/14*** *(2006.01)*
***A61K 31/498*** *(2006.01)* ***A61P 35/00*** *(2006.01)*

(52) Cooperative Patent Classification (CPC):
**A61K 31/498; A61K 31/553; A61P 3/00; A61P 3/10; A61P 9/00; A61P 9/10; A61P 19/02; A61P 19/10; A61P 25/00; A61P 25/28; A61P 29/00; A61P 35/00; A61P 35/02; C07D 401/12; C07D 401/14;** (Cont.)

(86) International application number:
**PCT/CN2024/107683**

(87) International publication number:
**WO 2025/026195 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.07.2023 CN 202310938270**
**17.11.2023 CN 202311537467**

(71) Applicant: **Sunshine Lake Pharma Co., Ltd.**
**Dongguan, Guangdong 523000 (CN)**

(72) Inventors:
- **LIU, Bing**
  **Dongguan, Guangdong 523871 (CN)**
- **BAI, Shun**
  **Dongguan, Guangdong 523871 (CN)**
- **WANG, Feng**
  **Dongguan, Guangdong 523871 (CN)**
- **PAN, Jiawei**
  **Dongguan, Guangdong 523871 (CN)**
- **TANG, Jianyao**
  **Dongguan, Guangdong 523871 (CN)**
- **LIN, Mengling**
  **Dongguan, Guangdong 523871 (CN)**
- **RAO, Zhipeng**
  **Dongguan, Guangdong 523871 (CN)**
- **ZHANG, Yingjun**
  **Dongguan, Guangdong 523871 (CN)**

(74) Representative: **Hoffmann Eitle**
**Patent- und Rechtsanwälte PartmbB**
**Arabellastraße 30**
**81925 München (DE)**

# (54) SUBSTITUTED NITROGEN-CONTAINING BICYCLIC COMPOUND AND USE THEREOF

(57) The present invention relates to the field of medicines, and relates to a substituted nitrogen-containing bicyclic compound and a use thereof. Specifically, the present invention relates to a novel substituted nitrogen-containing bicyclic compound and a pharmaceutical composition comprising the compound, wherein the compound and the pharmaceutical composition can be used for inhibiting PARP1, and show higher selectivity for PARP1 than for other PARP subtypes. The present invention also relates to a method for preparing the compound and the pharmaceutical composition, and a use of the compound and the pharmaceutical composition in preparation of medications for treating PARP1-mediated diseases, in particular cancer.

EP 4 755 880 A1

(52) Cooperative Patent Classification (CPC): (Cont.)
**C07D 405/14; C07D 417/14; C07D 498/14**

## Description

## FIELD OF THE INVENTION

This invention belongs to the field of pharmaceutical technology, specifically relating to novel substituted nitrogen-containing bicyclic compounds and pharmaceutical compositions comprising these compounds, as well as methods of use and applications thereof. In particular, the novel substituted nitrogen-containing bicyclic compounds described in this invention can be used to inhibit PARP1 for the prevention, treatment or relief of PARP 1 mediated diseases, especially cancer.

## BACKGROUND ART

Cancer refers to malignant tumors originating from epithelial tissue and is the most common type of malignant tumor. Generally, the term "cancer" is used to refer to all malignant tumors. Cancer is a group of diseases characterized by abnormal cell differentiation and proliferation. In its later stages, it can spread to other areas of the body, including bones and vital organs. Cancer is characterized by abnormal cell differentiation and proliferation, uncontrolled growth, metastasis, and invasiveness. Its occurrence is a complex, multi-factorial, and multi-step process, divided into three stages: carcinogenesis, tumor promotion, and progression. The causes of malignant tumors are not fully understood, but they are closely related to infection, smoking, occupational exposure, environmental pollution, genetic factors, and an unhealthy diet.

Cancer is one of the major diseases that endanger human health and disrupt family and social harmony. It is a major global health problem and remains a leading cause of death. Since 2010, cancer has ranked second among the causes of death in humans, second only to cardiovascular and cerebrovascular diseases. The global cancer situation is becoming increasingly severe, with both incidence and mortality rates continuing to rise. According to relevant organizations, with population growth and aging, as well as social development and the development of unhealthy lifestyles, the number of new cancer cases worldwide may reach 22 million per year in the next 20 years, and the number of deaths caused by cancer may also soar to 13 million per year during the same period. Globally, approximately 60% of new cancer cases occur in developing countries, and these countries account for 70% of annual cancer deaths worldwide.

Despite significant progress in the feasibility of various cancer treatment options, currently available chemotherapy remains unsatisfactory, and the prognosis for most patients diagnosed with cancer remains poor. Therefore, the development of new anti-tumor drugs (or anti-cancer drugs) is of paramount importance.

Poly(ADP)-ribose polymerase (PARP) is a class of ribozymes that catalyze the ribosylation of ADP and are widely found in eukaryotic cells. There are at least 18 subtypes, all of which contain highly conserved PARP catalytic sequences. Based on the degree of modification that catalyzes ADP ribosylation, they can be divided into three categories: the first category catalyzes the formation of long-chain and branched poly(ADP-ribose) chains (PAR), including PARP1, PARP2, Tank1, and Tank2; the second category catalyzes the formation of mono(ADP-ribosyl) transferases (MAR), including PARP3, 4, 6-8, 10-12, and 14-16; and the third category does not have enzymatic catalytic ability, including PARP9 and PARP13. Although these subtypes share similar catalytic domains, only the PARP1 and PARP2 subtypes contain a DNA-binding domain, enabling them to bind to damaged DNA and repair it via the base excision repair (BER) pathway. PARP1 was the first to be discovered and has the highest intracellular content, accounting for 85%-90% of total intracellular PARP activity. It is primarily involved in DNA damage repair and is the most important PARP enzyme. It is also the most extensively studied and widely applied subtype, involved in the treatment of diseases such as cancer, stroke, inflammation, diabetes, myocardial ischemia, and neurodegenerative diseases.

PARP1 consists of 1014 amino acid residues with a relative molecular mass of 116 kDa. Its primary structure is highly conserved in eukaryotes (e.g., human and mouse amino acid sequences share 92% homology) and includes three domains: a C-terminal catalytic domain, an intermediate self-regulating domain (AD), and an N-terminal domain (DBD). Wherein, the C-terminal catalytic domain includes two active catalytic sites: a donor domain and an acceptor domain. The intermediate self-regulating domain includes two closely linked nuclear localization signal sequences and possesses Capase-3 cleavage function. The N-terminal domain includes three zinc finger motifs.

PARP1 is structurally very similar to PARP2 and is an important protein-modifying enzyme involved in DNA damage repair, exhibiting high expression in various tumors. Wherein, the PARP1 subtype plays a crucial role in DNA damage repair pathways, undertaking over 90% of the repair work. This pathway is abnormally active in tumor cells; therefore, inhibiting PARP1 activity can suppress tumor growth. In recent years, several PARP1 inhibitors have been approved for marketing, and several others have entered clinical trials. This demonstrates that PARP1 inhibitors have become a hot topic in anti-tumor drug development.

The mechanisms of action of PARP1 inhibitors in tumors are complex and diverse, primarily including the synthetic lethal hypothesis, the hypothesis of involvement in DNA damage repair in tumor cells, and the hypothesis of involvement in the regulation of nuclear factor-κB (NF-κB) and heat shock protein 70 (HSP70) in tumor cells. Although the

mechanisms of action of PARP1 inhibitors are not fully understood, their tumor-suppressive effects have brought new hope to cancer treatment.

Many chemotherapy drugs exert their anti-tumor effects by disrupting the structure of tumor cell DNA. Tumor cells can use PARP1 to repair damaged DNA, thus developing resistance to chemotherapy drugs. Therefore, PARP1 inhibitors can act as sensitizers, used in combination with other chemotherapy drugs to overcome resistance and improve efficacy. Furthermore, studies have found that using PARP1 inhibitors alone on tumor cells with BRCA1/2 (breast cancer 1/2) deletions or mutations can also produce good anti-tumor effects.

Compared to other clinical PARP1/2 inhibitors, PARP inhibitors with increased selectivity for PARP1 offer advantages in terms of improved efficacy and reduced toxicity. Furthermore, strong selective inhibition of PARP1 leads to PARP1 capture on DNA, causing DNA double-strand breaks (DSBs) by collapsing the replication fork in S phase. PARP1-DNA capture is an effective mechanism for selectively killing tumor cells with homologous recombination deficiency (HRD).

Therefore, further research is needed to find better, more effective, and safer PARP inhibitors, especially PARP inhibitors selective for PARP1.

Through continuous research efforts, the inventors have obtained an unexpected new class of highly selective PARP1 inhibitors. The substituted nitrogen-containing bicyclic compounds described in this invention possess strong PARP1 inhibitory activity and can therefore be used to treat PARP1 mediated diseases, particularly cancer. Furthermore, the substituted nitrogen-containing bicyclic compounds described in this invention exhibit significantly higher selectivity for PARP1 than other PARP subtypes (such as PARP2).

## SUMMARY

This invention provides a class of novel substituted nitrogen-containing bicyclic compounds as highly selective PARP1 inhibitors, which can be used to inhibit PARP1 and therefore can be used to treat PARP1 mediated diseases, particularly cancer. Furthermore, the substituted nitrogen-containing bicyclic compounds described in this invention exhibit significantly higher selectivity for PARP1 than other PARP subtypes (such as PARP2). Furthermore, experiments have shown that the substituted nitrogen-containing bicyclic compounds of this invention are stable, have good safety profiles, and possess favorable pharmacodynamic and pharmacokinetic properties, such as a good brain-plasma ratio, good bioavailability or good metabolic stability. Therefore, they have promising clinical application prospects.

This invention also provides methods for preparing such compounds, pharmaceutical compositions containing such compounds, and the use of such compounds and pharmaceutical compositions containing such compounds in the preparation of pharmaceuticals.

In one aspect, the present invention relates to a compound, which is a compound of formula (I), or a stereoisomer, tautomer, N-oxide, hydrate, solvate, metabolite, pharmaceutically acceptable salt or prodrug thereof of a compound of formula (I).

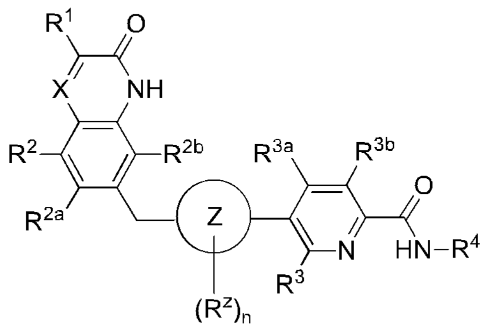

(I),

wherein:

X is $CR^x$ or N;

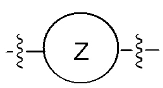

is 3-12 membered heterocyclyl;

$R^1$ is H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, -

C(=O)-($C_1$-$C_6$ alkyl), -C(=O)-($C_1$-$C_6$ alkoxy), $C_1$-$C_6$ alkyl, $C_2$-$C_6$ alkenyl, $C_2$-$C_6$ alkynyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino, $C_1$-$C_6$ hydroxyalkyl, $C_3$-$C_8$ cycloalkyl or 3-8 membered heterocyclyl;

each of $R^{2a}$ and $R^{2b}$ is independently H, D, F, Cl, Br, I, -CN, $-NO_2$, $-NH_2$, -OH, -SH, -COOH, $-C(=O)NH_2$, - C(=O) $NHCH_3$, $-C(=O)N(CH_3)_2$, -C(=O)-($C_1$-$C_6$ alkyl), -C(=O)-($C_1$-$C_6$ alkoxy), $C_1$-$C_6$ alkyl, $C_2$-$C_6$ alkenyl, $C_2$-$C_6$ alkynyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino, $C_1$-$C_6$ hydroxyalkyl, $C_1$-$C_6$ cyanoalkyl, $C_3$-$C_8$ cycloalkyl, 3-8 membered heterocyclyl, $C_6$-$C_{10}$ aryl or 5-10 membered heteroaryl;

$R^2$ is -CN, $-NO_2$, $-NH_2$, -OH, -SH, -COOH, $-C(=O)NH_2$, $-C(=O)NHCH_3$, $-C(=O)N(CH_3)_2$, -C(=O)-($C_1$-$C_6$ alkyl), -C(=O)-($C_1$-$C_6$ alkoxy), $C_1$-$C_6$ alkyl, $C_2$-$C_6$ alkenyl, $C_2$-$C_6$ alkynyl, $C_1$-$C_6$ alkoxy, $C_1$-$C_6$ haloalkoxy, $C_1$-$C_6$ alkylthio, $C_1$-$C_6$ alkylamino, $C_1$-$C_6$ hydroxyalkyl, $C_1$-$C_6$ cyanoalkyl, $C_3$-$C_8$ cycloalkyl-L-, 3-8 membered heterocyclyl-L-, $C_6$-$C_{10}$ aryl-L- or 5-10 membered heteroaryl-L-; wherein, $R^2$ is unsubstituted or substituted by 1, 2, 3, 4 or 5 $R^w$;

Each -L- is independently a bond, $-NR^n-$, -O-, -S-, -C(=O)-, -C(=O)O-, $-C(=O)N(R^{n1})-$ or $-(CR^aR^b)_m-$;

m is 1, 2, 3, 4, 5 or 6;

each of $R^n$ and $R^{n1}$ is independently H, D, $C_1$-$C_6$ alkyl, $C_1$-$C_6$ haloalkyl, $C_3$-$C_8$ cycloalkyl, 3-8 membered heterocyclyl, $C_6$-$C_{10}$ aryl or 5-10 membered heteroaryl;

each of $R^a$ and $R^b$ is independently H, D, F, Cl, Br, I, -CN, $-NO_2$, $-NH_2$, -OH, -SH, $C_1$-$C_6$ alkyl, $C_2$-$C_6$ alkenyl, $C_2$-$C_6$ alkynyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino or $C_1$-$C_6$ hydroxyalkyl;

each of $R^3$, $R^{3a}$ and $R^{3b}$ is independently H, D, F, Cl, Br, I, -CN, $-NO_2$, $-NH_2$, -OH, -SH, -COOH, $-C(=O)NH_2$, -C(=O) $NHCH_3$, $-C(=O)N(CH_3)_2$, -C(=O)-($C_1$-$C_6$ alkyl), -C(=O)-($C_1$-$C_6$ alkoxy), $C_1$-$C_6$ alkyl, $C_2$-$C_6$ alkenyl, $C_2$-$C_6$ alkynyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino or $C_1$-$C_6$ hydroxyalkyl;

$R^4$ is H, D, $C_1$-$C_6$ alkyl, $C_2$-$C_6$ alkenyl, $C_2$-$C_6$ alkynyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino, $C_3$-$C_8$ cycloalkyl, 3-8 membered heterocyclyl, $C_6$-$C_{10}$ aryl or 5-10 membered heteroaryl, wherein each of the $C_1$-$C_6$ alkyl, $C_3$-$C_8$ cycloalkyl, 3-8 membered heterocyclyl, $C_6$-$C_{10}$ aryl and 5-10 membered heteroaryl is independently and optionally substituted by 1, 2, 3, 4 or 5 groups selected from D, F, Cl, Br, 1, -OH, $-NH_2$, $-NO_2$, -CN, $C_1$-$C_6$ alkyl, $C_1$-$C_6$ haloalkyl, $C_1$-$C_6$ alkoxy and $C_1$-$C_6$ haloalkoxy;

each of $R^x$ and $R^z$ is independently H, D, F, Cl, Br, I, -CN, $-NO_2$, $-NH_2$, -OH, -COOH, $-C(=O)NH_2$, $C_1$-$C_6$ alkyl, $C_1$-$C_6$ haloalkyl, $C_1$-$C_6$ alkoxy, $C_1$-$C_6$ haloalkoxy or $C_1$-$C_6$ hydroxyalkyl;

each $R^w$ is independently H, D, F, Cl, Br, I, -CN, $-NO_2$, $-NH_2$, -OH, -SH, -COOH, $-C(=O)NH_2$, $-C(=O)NHCH_3$, -C(=O) $N(CH_3)_2$, -C(=O)-($C_1$-$C_6$ alkyl), -C(=O)-($C_1$-$C_6$ alkoxy), $C_1$-$C_6$ alkyl, $C_2$-$C_6$ alkenyl, $C_2$-$C_6$ alkynyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino, $C_1$-$C_6$ hydroxyalkyl, $C_3$-$C_8$ cycloalkyl, 3-8 membered heterocyclyl, $C_6$-$C_{10}$ aryl or 5-10 membered heteroaryl;

n is 1, 2, 3, 4, 5 or 6;

wherein, the compounds represented by formula (I) do not include the following compounds:

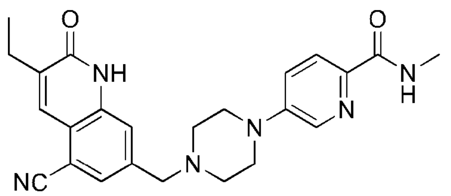 , 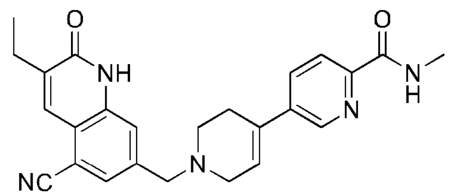 ,

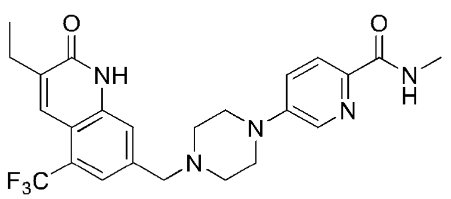 , 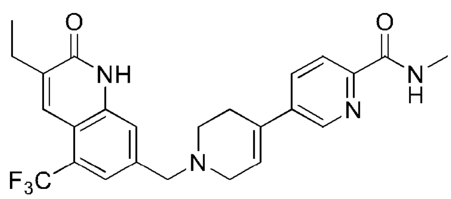 ,

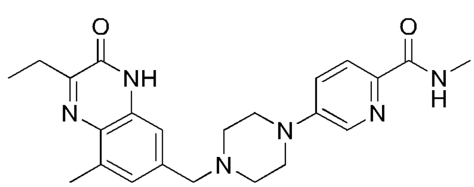 , 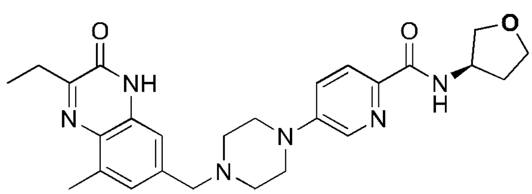 ,

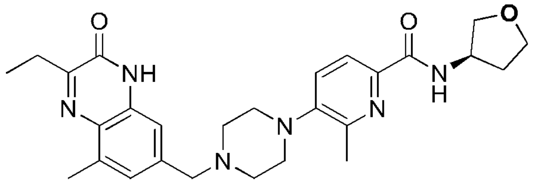,
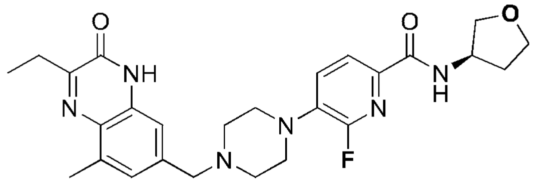,
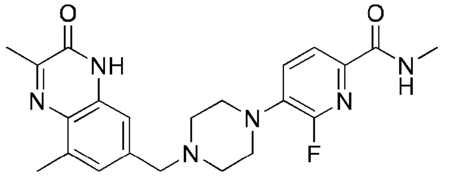,
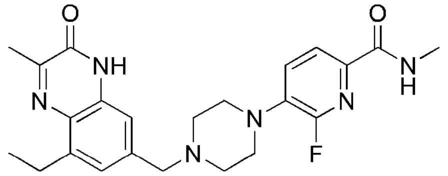,
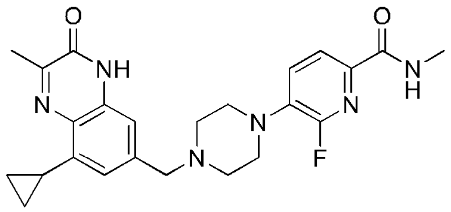,
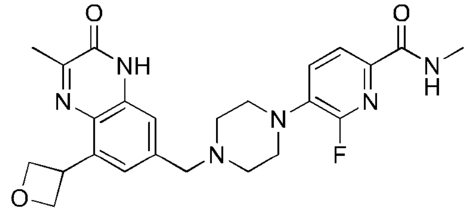,
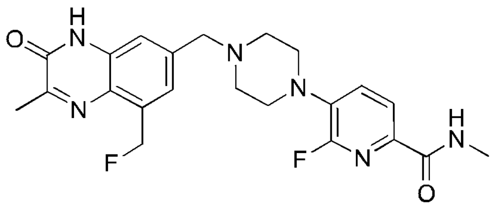,
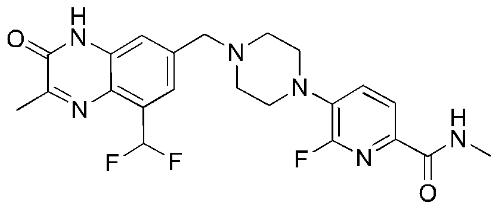,
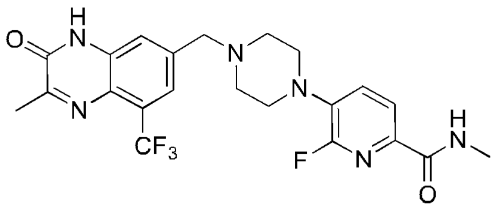,
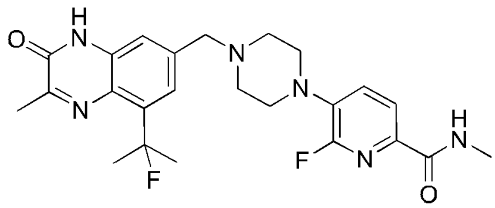,
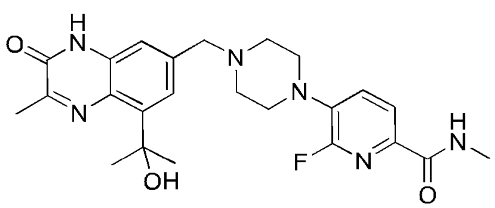,
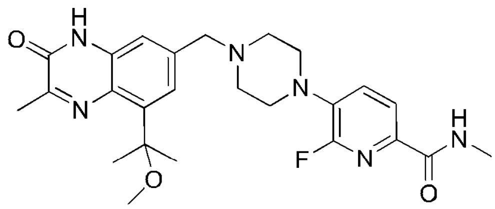,
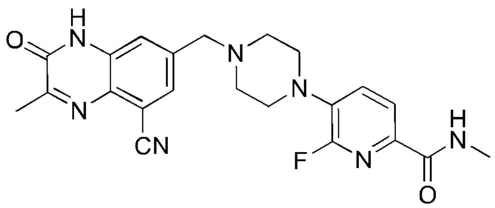,
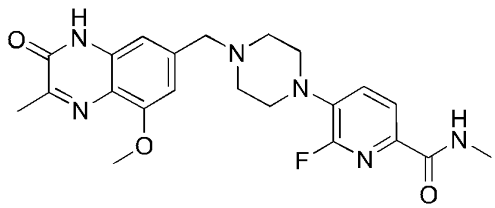,

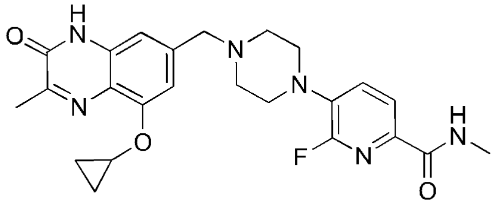
,
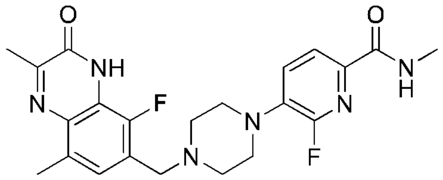
,
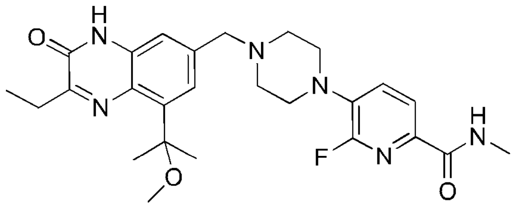
,
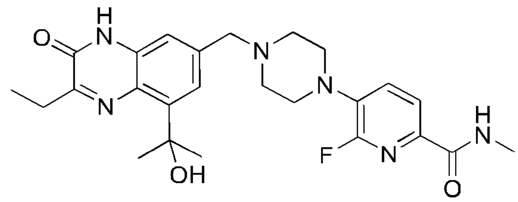
,
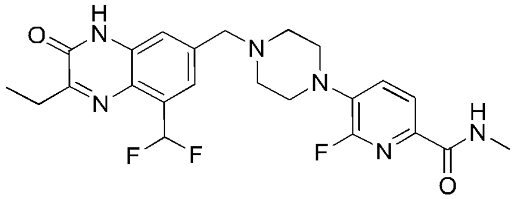
,
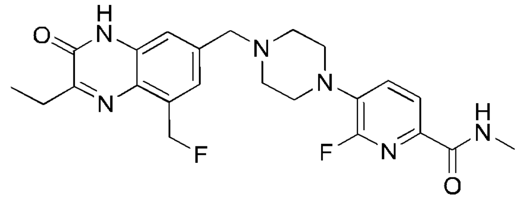
,
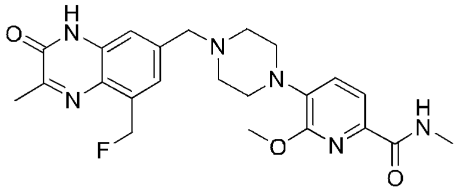
,
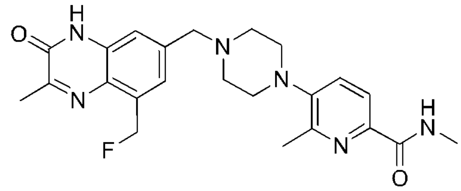
,
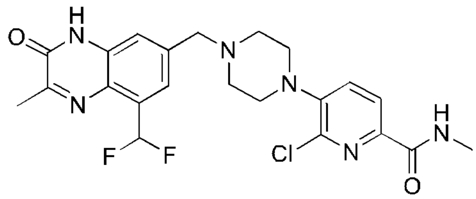
,
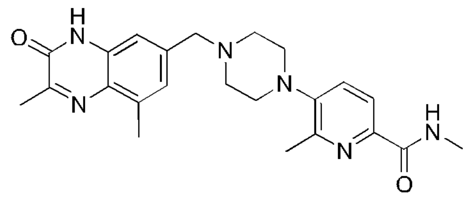

and

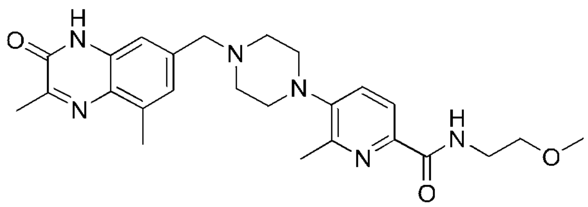
.

In another embodiment,

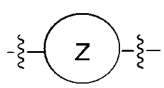

is

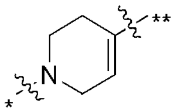, 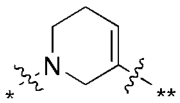, 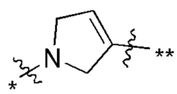,

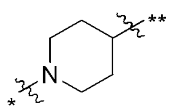, 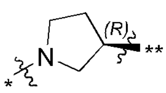, 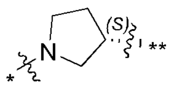, 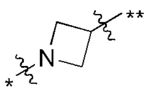, 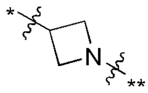, 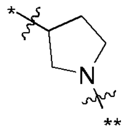, 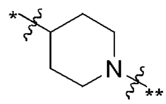,

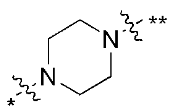, 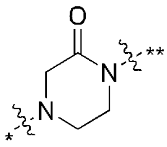, 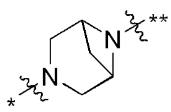, 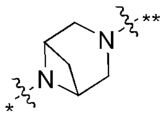, 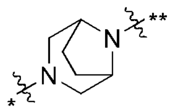, 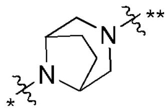,

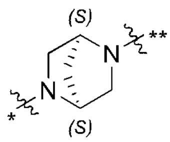, 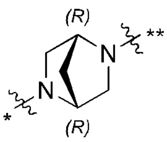, 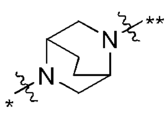, 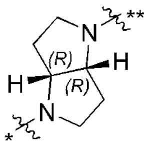, 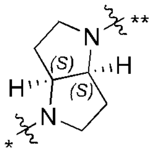, 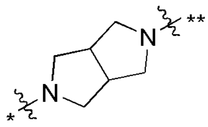,

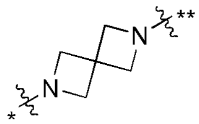, 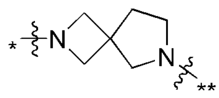, 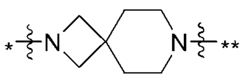, 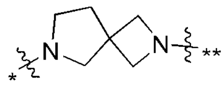, 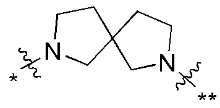,

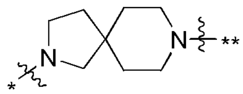, 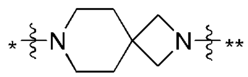, 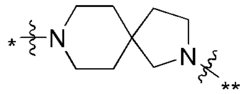, 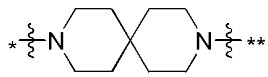,

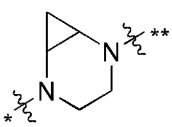, 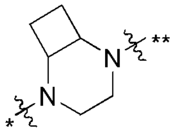, 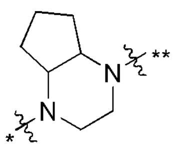 or 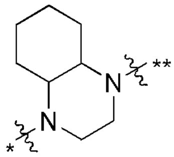;

wherein * indicates the connection of - $CH_2$- on the left and ** indicates the connection of pyridinyl on the right.

In one embodiment, each $R^1$ is independently H, D, F, Cl, Br, I, -CN, -$NO_2$, -$NH_2$, -OH, -SH, -COOH, - C(=O)$NH_2$, -C(=O)$NHCH_3$, -C(=O)N($CH_3$)$_2$, -C(=O)-($C_1$-$C_4$ alkyl), -C(=O)-($C_1$-$C_4$ alkoxy), $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino, $C_1$-$C_4$ hydroxyalkyl, $C_3$-$C_6$ cycloalkyl or 3-6 membered heterocyclyl;

each of $R^3$, $R^{3a}$ and $R^{3b}$ is independently H, D, F, Cl, Br, I, -CN, -$NO_2$, -$NH_2$, -OH, -SH, -COOH, -C(=O)$NH_2$, -C(=O)$NHCH_3$, -C(=O)N($CH_3$)$_2$, -C(=O)-($C_1$-$C_4$ alkyl), -C(=O)-($C_1$-$C_4$ alkoxy), $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino or $C_1$-$C_4$ hydroxyalkyl;

each of $R^x$ and $R^z$ is independently H, D, F, Cl, Br, I, -CN, -$NO_2$, -$NH_2$, -OH, -COOH, -C(=O)$NH_2$, $C_1$-$C_4$ alkyl, $C_1$-$C_4$ haloalkyl, $C_1$-$C_4$ alkoxy, $C_1$-$C_4$ haloalkoxy or $C_1$-$C_4$ hydroxyalkyl;

In another embodiment, $R^1$ is H, D, F, Cl, Br, I, -CN, -$NO_2$, -$NH_2$, -OH, -SH, -COOH, -C(=O)$NH_2$, - C(=O)$NHCH_3$,

$-C(=O)N(CH_3)_2$, $-C(=O)-CH_3$, $-C(=O)-OCH_3$, methyl, ethyl, *n*-propyl, isopropyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, n-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, hydroxymethyl, 2-hydroxyethyl, cyclopropyl, cyclobutyl, cyclopentyl, cyclohexyl, aziridine, pyrrolidinyl, tetrahydrofuranyl, piperidinyl, piperazinyl or morpholinyl;

each of $R^3$, $R^{3a}$ and $R^{3b}$ is independently H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, $-C(=O)-CH_3$, $-C(=O)-OCH_3$, methyl, ethyl, *n*-propyl, isopropyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, *n*-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, hydroxymethyl or 2-hydroxyethyl;

each of $R^x$ and $R^z$ is independently H, D, F, Cl, Br, I, -CN, $-NO_2$, $-NH_2$, -OH, -COOH, $-C(=O)NH_2$, methyl, ethyl, *n*-propyl, isopropyl, $-CHF_2$, $-CF_3$, $-CHFCH_2F$, $-CF_2CHF_2$, $-CH_2CF_3$, $-CH_2CF_2CHF_2$, methoxy, ethoxy, n-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, hydroxymethyl or 2-hydroxyethyl.

In one embodiment, $R^4$ is H, D, $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino, $C_3$-$C_6$ cycloalkyl, 3-6 membered heterocyclyl, $C_6$-$C_{10}$ aryl or 5-6 membered heteroaryl, wherein the $C_1$-$C_4$ alkyl, $C_3$-$C_6$ cycloalkyl, 3-6 membered heterocyclyl, $C_6$-$C_{10}$ aryl and 5-6 membered heteroaryl are optionally and independently substituted by 1, 2, 3, 4 or 5 groups selected from D, F, Cl, Br, 1, -OH, $-NH_2$, $-NO_2$, -CN, $C_1$-$C_4$ alkyl, $C_1$-$C_4$ haloalkyl, $C_1$-$C_4$ alkoxy, and $C_1$-$C_4$ haloalkoxy.

In another embodiment, $R^4$ is H, D, methyl, ethyl, *n*-propyl, isopropyl, allyl, propenyl, propargyl, propynyl, - $CHF_2$, $-CF_3$, $-CHFCH_2F$, $-CF_2CHF_2$, $-CH_2CF_3$, $-CH_2CF_2CHF_2$, methoxy, ethoxy, *n*-propyloxy, isopropyloxy, - $OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, cyclopropyl, cyclobutyl, cyclopentyl, cyclohexyl, aziridine, oxacyclobutyl, pyrrolyl, tetrahydrofuranyl, tetrahydropyranyl, piperidinyl, piperazine, morpholinyl, phenyl, indole, naphthyl, pyrrolyl, pyrazolyl, imidazolyl, triazolyl, tetrazolyl, furanyl, thiophenyl, thiazolyl, oxazolyl, pyridinyl, pyrimidinyl, pyrazinyl or pyridazinyl, wherein, each of the methyl, ethyl, n-propyl, isopropyl, cyclopropyl, cyclobutyl, cyclopentyl, cyclohexyl, azacyclobutyl, oxacyclobutyl, pyrrolyl, tetrahydrofuranyl, tetrahydropyranyl, piperidinyl, piperazine, morpholinyl, phenyl, indole, naphthyl, pyrrolyl, pyrazolyl, imidazoleyl, triazolyl, tetrazolyl, furanyl, thiophenyl, thiazolyl, oxazolyl, pyridinyl, pyrimidinyl, pyrazinyl and pyridazinyl is independently and optionally substituted by 1, 2, 3, 4 or 5 groups selected from D, F, Cl, Br, I, -OH, $-NH_2$, $-NO_2$, -CN, methyl, ethyl, *n*-propyl, isopropyl, $-CHF_2$, $-CF_3$, $-CHFCH_2F$, $-CF_2CHF_2$, $-CH_2CF_3$, $-CH_2CF_2CHF_2$, methoxy, ethoxy, *n*-propyloxy, isopropyloxy, $-OCHF_2$, - $OCF_3$, $-OCHFCH_2F$, $-OCF_2CHF_2$, $-OCH_2CF_3$ and $-OCH_2CF_2CHF_2$.

In one embodiment, each of $R^{2a}$ and $R^{2b}$ is independently H, D, F, Cl, Br, I, -CN, $-NO_2$, $-NH_2$, -OH, -SH, - COOH, $-C(=O)NH_2$, $-C(=O)NHCH_3$, $-C(=O)N(CH_3)_2$, $-C(=O)-(C_1$-$C_4$ alkyl), $-C(=O)-(C_1$-$C_4$ alkoxy), $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino, $C_1$-$C_4$ hydroxyalkyl, $C_1$-$C_4$ cyanoalkyl, $C_3$-$C_6$ cycloalkyl, 3-6 membered heterocyclyl, $C_6$-$C_{10}$ aryl or 5-6 membered heteroaryl.

In one embodiment, each of $R^n$ and $R^{n1}$ is independently H, D, $C_1$-$C_4$ alkyl, $C_1$-$C_4$ haloalkyl, $C_3$-$C_6$ cycloalkyl, 3-6 membered heterocyclyl, $C_6$-$C_{10}$ aryl or 5-6 membered heteroaryl;
each of $R^a$ and $R^b$ is independently H, D, F, Cl, Br, I, -CN, $-NO_2$, $-NH_2$, -OH, -SH, $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino or $C_1$-$C_4$ hydroxyalkyl.

In one embodiment, $R^2$ is -CN, $-NO_2$, $-NH_2$, -OH, -SH, -COOH, $-C(=O)NH_2$, $-C(=O)NHCH_3$, $-C(=O)N(CH_3)_2$, $-C(=O)-(C_1$-$C_4$ alkyl), $-C(=O)-(C_1$-$C_4$ alkoxy), $C_1$-$C_4$ alkyl, $C_2$-$C_4$ alkenyl, $C_2$-$C_4$ alkynyl, $C_1$-$C_4$ alkoxy, $C_1$-$C_4$ haloalkoxy, $C_1$-$C_4$ alkylthio, $C_1$-$C_4$ alkylamino, $C_1$-$C_4$ hydroxyalkyl, $C_1$-$C_4$ cyanoalkyl, $C_3$-$C_6$ cycloalkyl-L-, 3-6 membered heterocyclyl-L-, $C_6$-$C_{10}$ aryl-L- or 5-6 membered heteroaryl-L-; wherein, $R^2$ is unsubstituted or substituted by 1, 2, 3, 4 or 5 $R^w$;

$R^w$ is independently H, D, F, Cl, Br, I, -CN, $-NO_2$, $-NH_2$, -OH, -SH, -COOH, $-C(=O)NH_2$, $-C(=O)NHCH_3$, - $C(=O)N(CH_3)_2$, $-C(=O)-(C_1$-$C_4$ alkyl), $-C(=O)-(C_1$-$C_4$ alkoxy), $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino, $C_1$-$C_4$ hydroxyalkyl, $C_3$-$C_6$ cycloalkyl, 3-6 membered heterocyclyl, $C_6$-$C_{10}$ aryl or 5-6 membered heteroaryl.
wherein L is as defined herein.

In one embodiment, each of $R^{2a}$ and $R^{2b}$ is independently H, D, F, Cl, Br, I, -CN, $-NO_2$, $-NH_2$, -OH, -SH, - COOH, $-C(=O)NH_2$, $-C(=O)NHCH_3$, $-C(=O)N(CH_3)_2$, $-C(=O)-(C_1$-$C_2$ alkyl), $-C(=O)-(C_1$-$C_2$ alkoxy), methyl, ethyl, n-propyl, isopropyl, allyl, propenyl, propargyl, propynyl, $-CHF_2$, $-CF_3$, $-CHFCH_2F$, $-CF_2CHF_2$, $-CH_2CF_3$, - $CH_2CF_2CHF_2$, methoxy, ethoxy, *n*-propyloxy, isopropyloxy, $-OCHF_2$, $-OCF_3$, $-OCHFCH_2F$, $-OCF_2CHF_2$, - $OCH_2CF_3$, $-OCH_2CF_2CHF_2$, methylthio, ethylthio, methylamino, dimethylamino, ethylamino, hydroxymethyl, 2-hydroxyethyl, cyanomethyl, cyclopropyl, cyclobutyl, cyclopentyl, cyclohexyl, aziridine, pyrrolyl, tetrahydrofuranyl, piperidinyl, piperazine, morpholinyl, phenyl, indole,

naphthyl, pyrroleyl, pyrazolyl, imidazolyl, triazolyl, tetrazolyl, furanyl, thiophenyl, thiazolyl, oxazolyl, pyridinyl, pyrimidinyl, pyrazinyl or pyridazinyl.

In one embodiment, each of $R^n$ and $R^{n1}$ is independently H, D, methyl, ethyl, n-propyl, isopropyl, *n*-butyl, *tert*-butyl, $-CHF_2$, $-CF_3$, $-CHFCH_2F$, $-CF_2CHF_2$, $-CH_2CF_3$, $-CH_2CF_2CHF_2$, cyclopropyl, cyclobutyl, cyclopentyl, cyclohexyl, aziridine, pyrrolyl, tetrahydrofuranyl, piperidinyl, piperazine, morpholinyl, phenyl, pyrrolyl, pyrazolyl, imidazolyl, triazolyl, tetrazolyl, furanyl, thienyl, thiazolyl, oxazolyl, pyridinyl, pyrimidinyl, pyrazinyl or pyridazinyl;

each of $R^a$ and $R^b$ is independently H, D, F, Cl, Br, I, -CN, $-NO_2$, $-NH_2$, -OH, -SH, methyl, ethyl, *n*-propyl, isopropyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, *n*-propyloxy, isopropyloxy, $-OCHF_2$, $-OCF_3$, $-OCHFCH_2F$, $-OCF_2CHF_2$, $-OCH_2CF_3$, - $OCH_2CF_2CHF_2$, methylthio, ethylthio, methylamino, dimethylamino, ethylamino, hydroxymethyl or 2-hydroxyethyl.

In one embodiment, $R^2$ is -CN, $-NO_2$, $-NH_2$, -OH, -SH, -COOH, $-C(=O)NH_2$, $-C(=O)NHCH_3$, $-C(=O)N(CH_3)_2$, $-C(=O)-CH_3$, $-C(=O)-OCH_3$, methyl, ethyl, *n*-propyl, isopropyl, *n*-butyl, *tert*-butyl, allyl, propenyl, propargyl, propynyl, methoxy, ethoxy, *n*-propyloxy, isopropyloxy, $-OCHF_2$, $-OCF_3$, $-OCHFCH_2F$, $-OCF_2CHF_2$, $-OCH_2CF_3$, - $OCH_2CF_2CHF_2$, methylthio, ethylthio, methylamino, dimethylamino, ethylamino, hydroxymethyl, 2-hydroxyethyl, cyanomethyl, cyclopropyl-L-, cyclobutyl-L-, cyclopentyl-L-, cyclohexyl-L-, oxacyclopropyl-L-, azacyclopropyl-L-, oxacyclobutyl-L-, azacyclobutyl-L-, tetrahydrofuranyl-L-, pyrrolidinyl-L-, tetrahydropyranyl-L-, piperidinyl-L-, piperazinyl-L-, morpholinyl-L-, phenyl-L-, naphthyl-L-, pyrrolidinyl-L-, furanyl-L-, thiophenyl-L-, pyrazolyl-L-, imidazolyl-L-, thiazolyl-L-, oxazolyl-L-, triazolyl-L-, tetrazolyl-L-, pyridinyl-L-, pyrimidinyl-L-, pyrazinyl-L- or pyridazinyl-L-; wherein $R^2$ is unsubstituted or substituted by 1, 2, 3, 4 or 5 $R^w$;

each $R^w$ is independently H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, $-C(=O)-(C_1-C_3$ alkyl), $-C(=O)-(C_1-C_3$ alkoxy), methyl, ethyl, n-propyl, isopropyl, n-butyl, tert-butyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, n-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, hydroxymethyl, 2-hydroxyethyl, cyclopropyl, cyclobutyl, cyclopentyl, cyclohexyl, aziridine, pyrrolyl, tetrahydrofuranyl, piperidinyl, piperazine, morpholinyl, phenyl, pyrrolyl, pyrazolyl, imidazolyl, triazolyl, tetrazolyl, furanyl, thiophenyl, thiazolyl, oxazolyl, pyridinyl, pyrimidinyl, pyrazinyl or pyridazinyl; each L has the definition as described in this invention.

In some embodiments, the present invention relates to a compound, which is a compound of formula (II) or formula (III), or a stereoisomer, tautomer, N-oxide, hydrate, solvate, metabolite, pharmaceutically acceptable salt or prodrug thereof of a compound of formula (II) or formula (III),

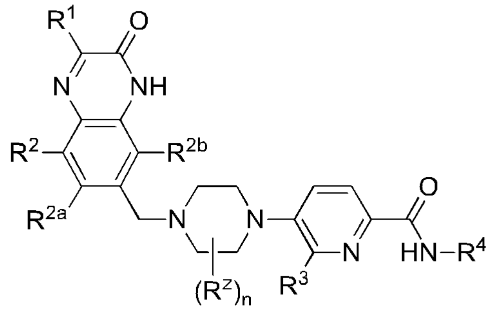

(II)

or

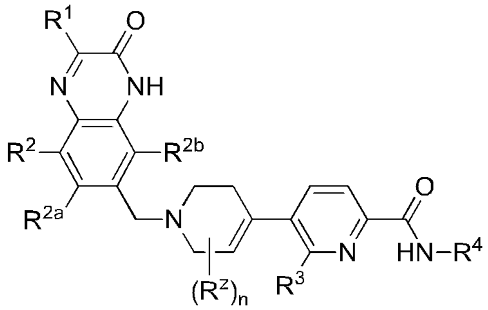

(III),

wherein each of $R^1$, $R^2$, $R^{2a}$, $R^{2b}$, $R^3$, $R^z$, $R^4$ and n are as defined herein.

In the other aspect, the present invention relates to a pharmaceutical composition comprising a compound of

formula (I), (II) or (III) disclosed herein.

In one embodiment, the pharmaceutical composition of the present invention further comprises a pharmaceutically acceptable excipient, carrier, adjuvant or any combination thereof.

In yet another aspect, the present invention relates to the use of a compound of formula (I), (II) or (III) disclosed herein, or a pharmaceutical composition thereof, in the manufacture of a medicament for the prevention, treatment or relief of PARP1 mediated diseases.

In one embodiment, the PARP1 mediated diseases are cancer, neurodegenerative diseases, cardiovascular diseases, ischemic diseases, diabetic neuropathy, reperfusion injury, osteoarthritis, osteoporosis, inflammatory diseases and metabolic diseases.

In yet another embodiment, the cancers are laryngeal cancer, esophageal cancer, gastric cancer, intestinal cancer, liver cancer, kidney cancer, lung cancer, brain cancer, head and neck cancer, squamous cell carcinoma, lymphatic system cancer, thyroid cancer, bladder cancer, ovarian cancer, cervical cancer, prostate cancer, genitourinary tract cancer, breast cancer, hematologic malignancies, small cell lung cancer, lung adenocarcinoma, pancreatic cancer, colon cancer, glioblastoma and/or monocytic leukemia.

In other aspect, this invention relates to the use of compounds of formula (I), (II) or (III) disclosed herein, or pharmaceutical compositions thereof, in the manufacture of a medicament for inhibiting PARP1.

On the other hand, this invention relates to methods for the preparation, isolation, and purification of compounds of formula (I), (II) or (III).

Biological assays have shown that the compounds of this invention can inhibit PARP1, and exhibit significantly higher selectivity for PARP1 than other PARP subtypes (such as PARP2). Therefore, they can serve as highly selective PARP1 inhibitors.

Any embodiment disclosed herein can be combined with other embodiments as long as they are not contradictory to one another, even though the embodiments are described under different aspects of the invention. In addition, any technical feature in one embodiment can be applied to the corresponding technical feature in other embodiments as long as they are not contradictory to one another, even though the embodiments are described under different aspects of the invention.

The foregoing merely summarizes certain aspects disclosed herein and is not intended to be limiting in nature. These aspects and other aspects and embodiments are described more fully below. All references of this specification are incorporated herein by reference in their entirety. In the event of any discrepancy between the information disclosed in this specification and the cited references, the information disclosed in this specification shall prevail.

## DETAILED DESCRIPTION OF THE INVENTION

### DEFINITIONS AND GENERAL TERMINOLOGY

Reference will now be made in detail to certain embodiments of the invention, examples of which are illustrated in the accompanying structures and formulas. The invention is intended to cover all alternatives, modifications, and equivalents which may be included within the scope of the present invention as defined by the claims. One skilled in the art will recognize many methods and materials similar or equivalent to those described herein, which could be used in the practice of the present invention. The present invention is in no way limited to the methods and materials described herein. In the event that one or more of the incorporated literature, patents, and similar materials differs from or contradicts this application (including but not limited to defined terms, term usage, described techniques, or the like) this application controls.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

As used herein, the following definitions shall apply unless otherwise indicated. For purposes of this invention, the chemical elements are identified in accordance with the Periodic Table of the Elements, CAS version, and the Handbook of Chemistry and Physics, 75th Ed. 1994. Additionally, general principles of organic chemistry are described in "Organic Chemistry", Thomas Sorrell, University Science Books, Sausalito: 1999, 1999, and Smith et al., "March's Advanced Organic Chemistry", John Wiley & Sons, New York: 2007 the entire contents of which are hereby incorporated by reference.

The grammatical articles "a", "an" and "the", as used herein, are intended to include "at least one" or "one or more" unless otherwise indicated herein or clearly contradicted by the context. Thus, the articles are used herein to refer to one or more than one (i.e. at least one) of the grammatical objects of the article. By way of example, "a component" means one or more components, and thus, possibly, more than one component is contemplated and may be employed or used in an implementation of the described embodiments.

"Stereoisomers" refers to compounds which have identical chemical constitution, but differ with regard to the arrangement of the atoms or groups in space. Stereoisomers include enantiomer, diastereomers, conformer (rotamer), geometric (cis/trans) isomer, atropisomer, etc.

"Chiral molecule" refers to molecules which have the property of non-superimposability of the mirror image partner, while the term "achiral molecule" refers to molecules which are superimposable on their mirror image partner.

"Enantiomers" refer to two stereoisomers of a compound which are non-superimposable mirror images of one another.

The term "racemate" or "racemic mixture" refers to an equimolar mixture of two enantiomers that lacks optical activity.

"Diastereomer" refers to a stereoisomer with two or more centers of chirality and whose molecules are not mirror images of one another. Diastereomers have different physical properties, e.g. melting points, boling points, spectral properties or biological activities. Mixture of diastereomers may separate under high resolution analytical procedures such as electrophoresis and chromatography such as HPLC.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994, all of which are incorporated herein by reference. Many organic compounds exist in optically active forms, i.e., they have the ability to rotate the plane of plane-polarized light. In describing an optically active compound, the prefixes *D* and *L,* or *R* and *S,* are used to denote the absolute configuration of the molecule about its chiral center(s). The prefixes *d* and *l* or (+) and (-) are employed to designate the sign of rotation of plane-polarized light by the compound, with (-) or *l* meaning that the compound is levorotatory. A compound prefixed with (+) or *d* is dextrorotatory. A specific stereoisomer may be referred to as an enantiomer, and a mixture of such stereoisomers is called an enantiomeric mixture. A 50:50 mixture of enantiomers is referred to as a racemic mixture or a racemate, which may occur where there has been no stereoselection or stereospecificity in a chemical reaction or process.

Any asymmetric atom (e.g., carbon or the like) of the compound(s) disclosed herein can be present in racemic or enantiomerically enriched, for example the (*R*)-, (*S*)- or (*R,S*)- configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (*R*)- or (*S*)- configuration.

Depending on the choice of the starting materials and procedures, the compounds can be present in the form of one of the possible stereoisomers or as mixtures thereof, such as racemates and diastereoisomer mixtures, depending on the number of asymmetric carbon atoms. Optically active (*R*)- or (*S*)- isomers may be prepared using chiral synthons or chiral reagents, or resolved using conventional techniques. If the compound contains a double bond, the substituent may be *E* or *Z* configuration: If the compound contains a disubstituted cycloalkyl, the cycloalkyl substituent may have a *cis*- or trans-configuration.

Any resulting mixtures of stereoisomers can be separated on the basis of the physicochemical differences of the constituents, into the pure or substantially pure geometric isomers, enantiomers, diastereomers, for example, by chromatography and/or fractional crystallization. Cis and trans isomers are diastereomer.

Any resulting racemates of final products or intermediates can be resolved into the optical antipodes by methods known to those skilled in the art, e.g., by separation of the diastereomeric salts thereof. Racemic products can also be resolved by chiral chromatography, e.g., high performance liquid chromatography (HPLC) using a chiral adsorbent. Preferred enantiomers can also be prepared by asymmetric syntheses. See, for example, Jacques, et al., Enantiomers, Racemates and Resolutions (Wiley Interscience, New York, 1981**);** Principles of Asymmetric Synthesis (2nd Ed. Robert E. Gawley, Jeffrey Aube, Elsevier, Oxford, UK, 2012); Eliel, E.L. Stereochemistry of Carbon Compounds (McGraw-Hill, NY, 1962); Wilen, S.H. Tables of Resolving Agents and Optical Resolutions p. 268 (E.L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

The term "tautomer" or "tautomeric form" refers to structural isomers of different energies which are interconvertible via a low energy barrier. Where tautomerization is possible (e.g. in solution), a chemical equilibrium of tautomers can be reached. For example, proton tautomers (also known as prototropic tautomers) include interconversions via migration of a proton, such as keto-enol and imine-enamine isomerizations.

The term "pharmaceutically acceptable," as used herein, refers to those compounds, materials, compositions, and/or dosage forms which are, within the scope of sound medical judgment, suitable for use in contacting with the tissues of patients without excessive toxicity, irritation, allergic response, or other problem or complication commensurate with a reasonable benefit/risk ratio, and are effective for their intended use.

The term "optionally substituted by..." can be used interchangeably with the term "unsubstituted or substituted by...", meaning that the structure is unsubstituted or substituted by one or more substituents as described in this invention, including, but not limited to, D, F, Cl, Br, I, $N_3$, -CN, $-NO_2$, $-NH_2$, -OH, -SH, -COOH, $-CONH_2$, - $C(=O)NHCH_3$, $-C(=O)N(CH_3)_2$, -C(=O)-alkyl, -C(=O)-alkoxy, alkyl, alkenyl, alkynyl, haloalkyl, alkoxy, haloalkoxy, alkylthio, alkylamino, hydroxyalkyl, cyanoalkyl, aminoalkyl, (alkoxy)-alkylene, (alkylamino)-alkylene, (cycloalkyl)-alkylene, (heterocyclyl)-alkylene, (aryl)-alkylene, (heteroaryl)-alkylene, cycloalkyl, heterocyclyl, aryl, heteroaryl, etc.

In general, the term "substituted" refers to the replacement of one or more hydrogen radicals in a given structure or group with the radical of a specified substituent. Unless otherwise indicated, a substituent may be substituted at any of the reasonable positions in the group. When more than one position in the given structural formula can be substituted by one or more specific substituents selected from the group, the substituents may be substituted at the reasonable positions in the structural formula, either in the same or different ways.

Furthermore, what need to be explained is that the phrase "each...is independently" and "each of... and... is independently", unless otherwise stated, should be broadly understood. The specific options expressed by the same symbol are independent of each other in different groups; or the specific options expressed by the same symbol are independent of each other in same groups.

As used herein, the term "subject" refers to an animal. Typically the animal is a mammal. A subject also refers to for example, primates (e.g., humans, male or female), cows, sheep, goats, horses, dogs, cats, rabbits, rats, mice, fish, birds and the like. In certain embodiments, the subject is a primate. In yet other embodiments, the subject is a human.

As used herein, "patient" refers to a human (including adults and children) or other animal. In one embodiment, "patient" refers to a human.

The term "comprise" is an open expression, it means comprising the contents disclosed herein, but don't exclude other contents.

At various places in the present specification, substituents of compounds disclosed herein are disclosed in groups or in ranges. It is specifically intended that the invention include each and every individual subcombination of the members of such groups and ranges. For example, the term "$C_1$-$C_6$ alkyl" is specifically intended to individually disclose methyl, ethyl, $C_3$ alkyl, $C_4$ alkyl, $C_5$ alkyl, and $C_6$ alkyl.

At various places in the present specification, linking substituents are described. Where the structure clearly requires a linking group, the Markush variables listed for that group are understood to be linking groups. For example, if the structure requires a linking group and the Markush group definition for that variable lists "alkyl" or "aryl" then it is understood that the "alkyl" or "aryl" represents a linking alkylene group or arylene group, respectively.

The term "D" refers to a single deuterium atom.

The terms "halogen" and "halogenated" are used interchangeably in this invention and refer to fluorine (F), chlorine (Cl), bromine (Br) or iodine (I).

The term "heteroatom" refers to O, S, N, P, and Si, including any oxidation state of N, S, and P; primary, secondary, tertiary amines, and quaternary ammonium salts; or forms in which the hydrogen atom on the nitrogen atom of the heterocycle is substituted, for example, N (like N in 3,4-dihydro-2H-pyrrole), NH (like NH in pyrrolidinyl) or NR' (like NR' in N-substituted pyrrolidinyl, where R' is a substituent as described in this invention).

The term "alkyl" or "alkyl group" refers to a saturated linear or branched-chain monovalent hydrocarbon group of 1-20 carbon atoms, wherein the alkyl group is optionally substituted with one or more substituents described herein. In one embodiment, the alkyl group contains 1-6 carbon atoms; in another embodiment, the alkyl group contains 1-4 carbon atoms; and in yet another embodiment, the alkyl group contains 1-3 carbon atoms. Examples of alkyl groups include, but are not limited to, methyl (Me, $-CH_3$), ethyl (Et, $-CH_2CH_3$), *n*-propyl (*n*-Pr, $-CH_2CH_2CH_3$), isopropyl (*i*-Pr, $-CH(CH_3)_2$), *n*-butyl (n-Bu, $-CH_2CH_2CH_2CH_3$), isobutyl (*i*-Bu, $-CH_2CH(CH_3)_2$), *sec*-butyl (*s*-Bu, $-CH(CH_3)CH_2CH_3$), *tert*-butyl (*t*-Bu, $-C(CH_3)_3$), and so on.

The term "alkylene" refers to a saturated divalent hydrocarbon group derived from a straight or branched chain saturated hydrocarbon by the removal of two hydrogen atoms. Unless otherwise specified, the alkylene group contains 1-12 carbon atoms. In some embodiments, the alkylene group contains 1-6 carbon atoms; In other embodiments, the alkylene group contains 1-4 carbon atoms; In still other embodiments, the alkylene group contains 1-3 carbon atoms; In yet other embodiments, the alkylene group contains 1-2 carbon atoms. And alkylene group is exemplified by methylene ($-CH_2-$), ethylene ($-CH_2CH_2-$), isopropylene ($-CH(CH_3)CH_2-$), and the like. Wherein the alkylene group is optionally substituted with one or more substitutents described herein.

The term "alkenyl" refers to linear or branched-chain monovalent hydrocarbon radical of 2 to 12 carbon atoms with at least one site of unsaturation, i.e., a carbon-carbon, $sp^2$ double bond, wherein the alkenyl radical may be optionally substituted independently with one or more substituents described herein, and includes radicals having *"cis"* and *"trans"* orientations, or alternatively, "*E*" and "*Z*" orientations. In some embodiments, the alkenyl contains 2 to 8 carbon atoms. In other embodiments, the alkenyl contains 2 to 6 carbon atoms. In still other embodiments, the alkenyl contains 2 to 4 carbon atoms. Some non-limiting examples of the alkenyl group include ethenyl or vinyl ($-CH=CH_2$), allyl ($-CH_2CH=CH_2$), 1-propenyl (i.e., propenyl, $-CH=CH-CH_3$), and the like.

The term "alkynyl" refers to a linear or branched monovalent hydrocarbon radical of 2 to 12 carbon atoms with at least one site of unsaturation, i.e., a carbon-carbon, sp triple bond, wherein the alkynyl radical may be optionally substituted independently with one or more substituents described herein. In some embodiments, the alkynyl contains 2 to 8 carbon atoms. In other embodiments, the alkynyl contains 2 to 6 carbon atoms. In still other embodiments, the alkynyl

contains 2 to 4 carbon atoms. Examples of alkynyl groups include, but are not limited to, ethynyl (-C≡CH), propynyl ($-CH_2C≡CH$), 1-propynyl (i.e., propynyl, $-C≡C-CH_3$), and the like.

The term "alkoxy" refers to an alkyl group, as previously defined, attached to the parent molecular moiety via an oxygen atom. Unless otherwise specified, the alkoxy group contains 1-12 carbon atoms. In one embodiment, the alkoxy group contains 1-6 carbon atoms. In other embodiment, the alkoxy group contains 1-4 carbon atoms. In still other embodiment, the alkoxy group contains 1-3 carbon atoms. The alkoxy group may be optionally substituted with one or more substituents disclosed herein.

Examples of alkoxy groups include, but are not limited to, methoxy (MeO, $-OCH_3$), ethoxy (EtO, $-OCH_2CH_3$), 1-propoxy (*n*-PrO, *n*-propoxy, $-OCH_2CH_2CH_3$), 2-propoxy (*i*-PrO, *i*-propoxy, $-OCH(CH_3)_2$), 1-butoxy (*n*-BuO, *n*-butoxy, $-OCH_2CH_2CH_2CH_3$), 2-methyl-1-propoxy (*i*-BuO, *i*-butoxy, $-OCH_2CH(CH_3)_2$), 2-butoxy (*s*-BuO, *s*-butoxy, $-OCH(CH_3)CH_2CH_3$), 2-methyl-2-propoxy (*t*-BuO, *t*-butoxy, $-OC(CH_3)_3$), and the like.

The term "alkathio" indicates that an alkyl group is attached to the remainder of the molecule via a sulfur atom, wherein the alkyl group has the meaning as described in this invention. Unless otherwise specified, the alkathio group contains 1-12 carbon atoms. In one embodiment, the alkathio group contains 1-6 carbon atoms; in another embodiment, the alkathio group contains 1-4 carbon atoms; and in yet another embodiment, the alkathio group contains 1-3 carbon atoms. The alkathio group may optionally be substituted by one or more substituents described in this invention.

Examples of alkylthio groups include, but are not limited to, methylthio (MeS, $-SCH_3$), ethylthio (EtS, - $SCH_2CH_3$), 1-propanethio (*n*-PrS, *n*-propanethio, $-SCH_2CH_2CH_3$), 2-propanethio (*i*-PrS, i-propanethio, - $SCH(CH_3)_2$), 1-butanethio (*n*-BuS, *n*-butanethio, $-SCH_2CH_2CH_2CH_3$), 2-methyl-1-propanethio (*i*-BuS, *i*-butanethio, $-SCH_2CH(CH_3)_2$), 2-butanethio (*s*-BuS, *s*-butanethio, $-SCH(CH_3)CH_2CH_3$), 2-methyl-2-propanethio (*t*-BuS, *t*-butanethio, $-SC(CH_3)_3$), and the like.

The term "alkamino" or "alkylamino" refers to "*N*-alkylamino" and "*N,N*-dialkylamino", wherein the amino groups are independently substituted with one or two alkyl groups, respectively, and wherein the alkyl group is as defined herein. Suitable alkylamino groups can be monoalkylamino or dialkylamino, examples of which include, but are not limited to, *N*-methylamino (methylamino), *N*-ethylamino (ethylamino), *N,N*-dimethylamino (dimethylamino), *N,N*-diethylamino (diethylamino), and the like. And wherein the alkylamino radical is optionally substituted with one or more substituents described herein.

The term "hydroxyalkyl" means that an alkyl group is substituted by one or more hydroxyl groups, wherein the alkyl group has the meaning as described in this invention; examples of this include, but are not limited to, hydroxymethyl, 2-hydroxyethyl, 2-hydroxy-1-propyl, 3-hydroxy-1-propyl, 2,3-dihydroxypropyl, and the like.

The term "cyanoalkyl" means that an alkyl group is replaced by one or more cyano groups, wherein the alkyl group has the meaning as described in this invention; examples of this include, but are not limited to, cyanomethyl, 2-cyanoethyl, 2-cyano-1-propyl, 3-cyano-1-propyl, 2,3-dicyanopropyl, and the like.

The term "aminoalkyl" means that an alkyl group is substituted with one or more amino groups, wherein the alkyl group has the meaning as described in this invention; examples of this include, but are not limited to, aminomethyl, 2-aminoethyl, 2-amino-1-propyl, 3-amino-1-propyl, 2,3-diaminopropyl, and the like.

The term "haloalkyl" means that an alkyl group is substituted with one or more halogen atoms, wherein the alkyl group has the meaning as described in this invention; examples of this include, but are not limited to, $-CHF_2$, $-CF_3$, $-CHFCH_2F$, $-CF_2CHF_2$, $-CH_2CF_3$, $-CHFCH_3$, $-CH_2CH_2F$, $-CF_2CH_3$, $-CHCF_2CHF_2$, and the like. In one embodiment, the $C_1$-$C_6$ haloalkyl comprises a fluorinated $C_1$-$C_6$ alkyl group; in another embodiment, the $C_1$-$C_4$ haloalkyl comprises a fluorinated $C_1$-$C_4$ alkyl group; in yet another embodiment, the $C_1$-$C_2$ haloalkyl comprises a fluorinated $C_1$-$C_2$ alkyl group.

The term "haloalkoxy" indicates that an alkoxy group is substituted by one or more halogen atoms, wherein the alkoxy group has the meaning as described in this invention. Examples of such alkoxy groups include, but are not limited to, $-OCHF_2$, $-OCF_3$, $-OCHFCH_2F$, $-OCF_2CHF_2$, $-OCH_2CF_3$, $-OCHFCH_3$, $-OCH_2CH_2F$, $-OCF_2CH_3$, - $OCH_2CF_2CHF_2$, and the like. In one embodiment, the $C_1$-$C_6$ haloalkoxy group comprises a fluorinated $C_1$-$C_6$ alkoxy group; in another embodiment, the $C_1$-$C_4$ haloalkoxy group comprises a fluorinated $C_1$-$C_4$ alkoxy group; and in yet another embodiment, the $C_1$-$C_2$ haloalkoxy group comprises a fluorinated $C_1$-$C_2$ alkoxy group.

The terms "composed of j-k atoms" or "j-k element" indicate that the cyclic group is composed of j-k ring atoms, including carbon atoms and/or heteroatoms such as O, N, S, P, etc.; j and k are each independently any non-zero natural number, and k > j; "j-k" includes j, k, and any natural number between them. For example, "composed of 3-8 atoms" or "3-8 membered", "composed of 3-6 atoms" or "3-6 membered", "composed of 5-10 atoms" or "5-10 membered", "composed of 5-6 atoms" or "5-6 membered" indicate that the cyclic group is composed of 3-8 (i.e., 3, 4, 5, 6, 7 or 8), 3-6 (i.e., 3, 4, 5 or 6), 5-10 (i.e., 5, 6, 7, 8, 9 or 10) or 5-6 (i.e., 5 or 6) ring atoms, including carbon atoms and/or heteroatoms such as O, N, S, P, etc. For example, piperidinyl is a heterocyclyl or a 6-membered heterocyclyl composed of 6 atoms, while pyridinyl is a heteroaryl group or a 6-membered heteroaryl group composed of 6 atoms.

The terms "(alkoxy)-alkylene", "(alkamino)-alkylene", "(cycloalkyl)-alkylene", "(heterocyclyl)-alkylene", "(aryl)-alkylene", and "(heteroaryl)-alkylene" indicate that each of the alkoxy, alkamino, cycloalkyl, heterocyclyl, aryl or heteroaryl groups is independently linked to the remainder of the molecule via an alkylene group, wherein the alkoxy, alkamino, cycloalkyl, heterocyclyl, aryl, heteroaryl, and alkylene groups each have the meaning described in this

invention. For example, examples of (cycloalkyl)-alkylene include, but are not limited to, cyclopropylmethylene, cyclobutylmethylene, cyclopentylmethylene and cyclohexylmethylene. As another example, examples of (aryl)-alkylene include, but are not limited to, phenylmethylene, phenylethylene and phenylpropylene. Wherein each of the (alkoxy)-alkylene, (alkylamino)-alkylene, (cycloalkyl)-alkylene, (heterocyclyl)-alkylene, (aryl)-alkylene and (heteroaryl)-alkylene is optionally and independently replaced by one or more substituents described in this invention.

The term "carbocyclyl" or "carbocycle" refers to a monovalent or multivalent, nonaromatic, saturated or partially unsaturated ring having 3 to 12 carbon atoms as a monocyclic, bicyclic or tricyclic ring system. A carbobicyclyl group includes a spiro carbobicyclyl group or a fused carbobicyclyl group. Suitable carbocyclyl groups include, but are not limited to, cycloalkyl, cycloalkenyl and cycloalkynyl. In one embodiment, the carbocyclic group comprises 3-10 carbon atoms, such as a $C_3$-$C_{10}$ carbocyclic group; in another embodiment, the carbocyclic group comprises 3-8 carbon atoms, such as a $C_3$-$C_8$ carbocyclic group; in yet another embodiment, the carbocyclic group comprises 3-6 carbon atoms, such as a $C_3$-$C_6$ carbocyclic group. In yet another embodiment, the monocyclic carbocyclic group comprises 4-8 carbon atoms, such as a $C_4$-$C_8$ monocyclic carbocyclic group; in yet another embodiment, the monocyclic carbocyclic group comprises 4-6 carbon atoms, such as a $C_4$-$C_6$ monocyclic carbocyclic group. Further examples of carbocyclyl groups include cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl, cyclododecyl, and the like, and wherein the carbocyclyl group is optionally substituted with one or more substituents described herein.

The term "cycloalkyl" refers to a monovalent or multivalent saturated ring having 3 to 12 carbon atoms as a monocyclic, bicyclic or tricyclic ring system, and wherein the bicyclic or tricyclic ring system may include fused ring, briged ring and spiro ring. In one embodiment, the cycloalkyl group comprises 3-10 carbon atoms, such as $C_3$-$C_{10}$ cycloalkyl; in another embodiment, the cycloalkyl group comprises 3-8 carbon atoms, such as $C_3$-$C_8$ cycloalkyl; in yet another embodiment, the cycloalkyl group comprises 3-6 carbon atoms, such as $C_3$-$C_6$ cycloalkyl. Examples of cycloalkyl groups include, but are not limited to, cyclopropyl, cyclobutyl, cyclopentyl, cyclohexyl, cycloheptyl, cyclooctyl, and the like. Wherein, as described in this invention, $C_3$-$C_8$ cycloalkyl groups include $C_3$-$C_6$ cycloalkyl groups; wherein $C_3$-$C_6$ cycloalkyl groups include cyclopropyl, cyclobutyl, cyclopentyl and cyclohexyl. The cycloalkyl radical is optionally substituted with one or more substituents described herein.

The terms "heterocyclic group" and "heterocyclyl" are used interchangeably herein, referring to a non-aromatic, saturated or partially unsaturated monocyclic, bicyclic or tricyclic system comprising 3-12 ring atoms, wherein the bicyclic or tricyclic system may include fused rings, bridged rings and spirocyclic rings. One or more atoms on the ring are independently replaced by heteroatoms, which have the meaning as described herein. In one embodiment, the heterocyclic group is a monocyclic heterocyclic group consisting of 3-8 ring atoms (2-6 carbon atoms and 1-3 heteroatoms selected from N, O, P, S, wherein S or P is optionally replaced by one or more oxygen atoms to obtain a group like SO, $SO_2$, PO, $PO_2$); in another embodiment, the heterocyclic group is a monocyclic heterocyclic group consisting of 3-6 ring atoms (2-5 carbon atoms and 1-3 heteroatoms selected from N, O, P, S, wherein S or P is optionally replaced by one or more oxygen atoms to obtain a group like SO, $SO_2$, PO, $PO_2$); in yet another embodiment, the heterocyclic group is a bicyclic heterocyclic group consisting of 7-12 ring atoms (4-9 carbon atoms and 1-3 heteroatoms selected from N, O, P, S, wherein S or P is optionally replaced by one or more oxygen atoms to obtain a group like SO, $SO_2$, PO, $PO_2$). and wherein the carbocyclyl group is optionally substituted with one or more substituents described herein.

The ring atom of the heterocyclyl may be a carbon radical or heteroatom radical. Wherein, the $-CH_2-$ group of the ring is replaced by -C(=O)-, the sulfur atom of the ring is optionally oxidized to S-oxide, and the nitrogen atom of the ring is optionally oxidized to N-oxygen compound. Some non-limiting examples of the heterocyclyl group include oxiranyl, azetidinyl, oxetanyl, thietanyl, pyrrolidinyl, 2-pyrrolinyl, 3-pyrrolinyl, pyrazolinyl, pyrazolidinyl, imidazolinyl, imidazolidinyl, tetrahydrofuranyl, dihydrofuranyl, tetrahydrothienyl, dihydrothienyl, 1,3-dioxolanyl, dithiolanyl, tetrahydropyranyl, dihydropyranyl, 2*H*-pyranyl, 4*H*-pyranyl, tetrahydrothiopyranyl, piperidinyl, morpholinyl, thiomorpholinyl, piperazinyl, dioxanyl, dithianyl, thioxanyl, homopiperazinyl, homopiperidinyl, oxepanyl, thiepanyl, oxazepinyl, diazepinyl, thiazepinyl, 2-oxa-5-azabicyclo[2.2.1]hept-5-yl, and the like. Some non-limiting examples of heterocyclyl wherein $-CH_2-$ group is substituted by -C(=O)- moiety include 2-oxopyrrolidinyl, oxo-1,3-thiazolidinyl, 2-piperidinonyl, 3,5-dioxopiperidinyl, pyrimidinedione-yl, and the like. Some non-limiting examples of the heterocyclyl group of which the ring sulfur atom is oxidized include sulfolanyl, 1,1-dioxo-thiomorpholinyl, and the like. The heterocyclyl group is optionally substituted with one or more substituents described herein.

The term "aryl" refers to monocyclic, bicyclic and tricyclic carbocyclic ring systems having a total of six to fourteen ring members, or six to twelve ring members, or six to ten ring members, wherein at least one ring in the system is aromatic, wherein each ring in the system contains 3 to 7 ring members and that has a single point or multipoint of attachment to the rest of the molecule. The aryl group is generally, but not necessarily bonded to the parent molecule through an aromatic ring of the aryl group. The term "aryl" and "aromatic ring" can be used interchangeably herein. Examples of the aryl group would include phenyl, indenyl, naphthyl and anthracene. The aryl radical is optionally substituted with one or more substituents described herein.

The term "heteroaryl" refers to monocyclic, bicyclic and tricyclic carbocyclic ring systems having a total of five to twelve ring members, or five to ten ring members, or five to six ring members, wherein at least one ring in the system is aromatic, and in which at least one ring member is selected from heteroatom, and wherein each ring in the system contains 5 to 7 ring members and that has a single point or multipoint of attachment to the rest of the molecule. The heteroaryl group is generally, but not necessarily bonded to the parent molecule through an aromatic ring of the heteroaryl group. The term "heteroaryl" may be used interchangeably with the term "heteroaryl ring", "aromatic heterocyclic" or the term "heteroaromatic compound". The heteroaryl group is optionally substituted with one or more substituents disclosed herein. In one embodiment, a 5-10 membered heteroaryl comprises 1, 2, 3 or 4 heteroatoms independently selected from O, S and N.

Examples of heteroaryl groups include, but are not limited to, 2-furanyl, 3-furanyl, *N*-imidazolyl, 2-imidazolyl, 4-imidazolyl, 5-imidazolyl, 3-isoxazolyl, 4-isoxazolyl, 5-isoxazolyl, 2-oxazolyl, 4-oxazolyl, 5-oxazolyl, *N*-pyrrolyl, 2-pyrrolyl, 3-pyrrolyl, 2-pyridyl, 3-pyridyl, 4-pyridyl, 2-pyrimidinyl, 4-pyrimidinyl, 2-pyrimidinyl, 4-pyrimidinyl. 5-pyrimidinyl, pyridazinyl (e.g., 3-pyridazinyl), 2-thiazolyl, 4-thiazolyl, 5-thiazolyl, tetrazolyl (e.g., 5-tetrazolyl), triazolyl (e.g., 2-triazolyl and 5-triazolyl), 2-thienyl, 3-thienyl, pyrazolyl (e.g., 2-pyrazolyl), isothiazolyl, 1,2,3-oxadiazolyl, 1,2,5-oxadiazolyl, 1,2,4-oxadiazolyl, 1,2,3-triazolyl 1,2,3-Thiodiazolyl, 1,3,4-thiodiazolyl, 1,2,5-thiodiazolyl, pyrazinyl, 1,3,5-triazinyl; also including, but not limited to, the following bicyclic compounds: benzimidazolyl, benzofuranyl, benzothiopheneyl, indoleyl (e.g., 2-indoleyl), purineyl, quinolinyl (e.g., 2-quinolinyl, 3-quinolinyl, 4-quinolinyl), isoquinolinyl (e.g., 1-isoquinolinyl) (e.g., 3-isoquinolinyl or 4-isoquinolinyl), imidazo[1,2-*a*]pyridyl, pyrazolo[1,5-*a*]pyridyl, pyrazolo[1,5-*a*]pyrimidinyl, imidazo[1,2-*b*]pyridazinyl, [1,2,4]triazolo[4,3-*b*]pyridazinyl, [1,2,4]triazolo[1,5-*a*]pyrimidinyl, [1,2,4]triazolo[1,5-*a*]pyridyl, and the like.

The term "protecting group" or "PG" refers to a substituent that is commonly employed to block or protect a particular functionality while reacting with other functional groups on the compound. For example, an "amino-protecting group" is a substituent attached to an amino group that blocks or protects the amino functionality in the compound. Suitable amino-protecting groups include acetyl, trifluoroacetyl, *t*-butoxy-carbonyl (BOC, Boc), benzyloxycarbonyl (CBZ, Cbz) and 9- fluorenylmethylenoxy-carbonyl (Fmoc). Similarly, a "hydroxy-protecting group" refers to a substituent of a hydroxy group that blocks or protects the hydroxy functionality. Some non-limiting examples of suitable hydroxy-protecting groups include trialkylsilyl, acetyl, benzoyl and benzyl. A "carboxy-protecting group" refers to a substituent of the carboxy group that blocks or protects the carboxy functionality. Common carboxy-protecting groups include $-CH_2CH_2SO_2Ph$, cyanoethyl, 2-(trimethylsilyl)ethyl, 2-(trimethylsilyl) ethoxy-methyl, 2-(p-toluenesulfonyl) ethyl, 2-(*p*-nitrophenylsulfonyl)-ethyl, 2-(diphenylphosphino)-ethyl, nitroethyl and the like. For a general description of protecting groups and their use, see Greene et al., Protective Groups in Organic Synthesis, John Wiley & Sons, New York, 1991 and Kocienski et al., Protecting Groups, Thieme, Stuttgart, 2005.

The term "prodrug" refers to a compound that is transformed in vivo into a compound of Formula (I), (II) or (III). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic ($C_{1-24}$) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound.

A "metabolite" is a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound. Accordingly, the invention includes metabolites of compounds disclosed herein, including metabolites produced by contacting a compound disclosed herein with a mammal for a sufficient time period.

A "pharmaceutically acceptable salts" refers to organic or inorganic salts of a compound disclosed herein. Pharmaceutically acceptable salts are well known in the art. For example,S. M. Berge et al., describe pharmaceutically acceptable salts in detail in J. Pharmaceutical Sciences, 1977, 66: 1-19, which is incorporated herein by reference. Some non-limiting examples of pharmaceutically acceptable and nontoxic salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid and malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, malonate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate,*p*-toluenesulfonate, undecanoate, valerate, and the like. Salts derived from appropriate bases include alkali metal, alkaline earth metal, ammonium and $N^+(C_{1-4}\ alkyl)_4$ salts. This invention also envisions the quaternization of any basic nitrogen-containing groups of the compounds disclosed herein. Water or oil soluble or dispersable products may be obtained by such

quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, $C_1$-$C_8$ sulfonate or aryl sulfonate. Although other salts can be used, such as in the separation or purification of products, non-toxic, physiologically acceptable salts are preferred.

These salts can be formed by conventional means, such as by reacting the free basic form of the product with an equivalent of one or more suitable acids in a solvent or medium (in which the salt is insoluble) or in a solvent (e.g., a solvent in which water has been removed under vacuum), by freeze-drying, or by exchanging the anion of an existing salt for another anion on a suitable ion exchange resin.

The term "solvate" refers to an association or complex of one or more solvent molecules and a compound disclosed herein. Some non-limiting examples of the solvent that form solvates include water, isopropanol, ethanol, methanol, dimethylsulfoxide, ethyl acetate, acetic acid, ethanolamine or mixtures thereof. The term "hydrate" refers to the complex where the solvent molecule is water.

The term "hydrate" can be used when said solvent is water. In one embodiment, one solvent molecule is associated with one molecule of the compounds disclosed herein, such as a hydrate. In another embodiment, more than one solvent molecule may be associated with one molecule of the compounds disclosed herein, such as a dihydrate. In still another embodiment, less than one solvent molecule may be associated with one molecule of the compounds disclosed herein, such as a hemihydrate. Furthermore, all the solvates of the invention retain the biological effectiveness of the non-hydrate form of the compounds disclosed herein.

As used herein, the term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (e.g., stabilization of a discernible symptom), physiologically, (e.g., stabilization of a physical parameter), or both. In yet another embodiment, "treat", "treating" or "treatment" refers to preventing or delaying the onset or development or progression of the disease or disorder.

The term "preventing" or "prevention" refers to a reduction in risk of acquiring a disease or disorder (i.e., causing at least one of the clinical symptoms of the disease not to develop in a subject that may be exposed to or predisposed to the disease but does not yet experience or display symptoms of the disease).

Unless otherwise stated, all suitable isotopic variations, stereoisomers, tautomers, solvates, metabolites, salts, and pharmaceutically acceptable prodrugs of the compounds of this invention are included within the scope of this invention.

In the structures disclosed herein, when the stereochemistry of any particular chiral atom is not specified, all stereoisomers of that structure are considered within the scope of this invention and are included in this invention as disclosed compounds. When the stereochemistry is indicated by a solid wedge or dashed line representing a particular configuration, the stereoisomers of that structure are thus clearly defined.

The "N-oxide" of the compounds of this invention are also included within the scope of this invention. The N-oxide of the compounds of this invention can be prepared by oxidizing the corresponding nitrogen-containing basic substances in the presence of an acid, such as acetic acid, using a common oxidizing agent (e.g., hydrogen peroxide) at elevated temperature, or by reacting with a peracid in a suitable solvent, such as with peracetic acid in dichloromethane, ethyl acetate, or methyl acetate, or with 3-chloroperoxybenzoic acid in chloroform or dichloromethane.

The compounds shown in formula (I), (II) or (III) may exist in the form of salts. In one embodiment, the salt refers to a pharmaceutically acceptable salt. The term "pharmaceutically acceptable" means that the substance or composition must be chemically and/or toxicologically compatible with other components comprising the formulation and/or the mammals treated therein. In another embodiment, the salt is not necessarily a pharmaceutically acceptable salt and may be an intermediate used for the preparation and/or purification of the compounds shown in formula (I), (II) or (III) and/or for the isolation of enantiomeric compounds shown in formula (I), (II) or (III).

The pharmaceutically acceptable salts of the present invention can be synthesized from a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, e.g., in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

Any formula given herein is also intended to represent isotopically unenriched forms as well as isotopically

enriched forms of the compounds. Any formula given herein is also intended to represent isotopically unenriched forms as well as isotopically enriched forms of the compounds. Exemplary isotopes that may be introduced into the compounds of the present invention include isotopes of hydrogen, carbon, nitrogen, oxygen, phosphorus, sulfur, fluorine, chlorine and iodine, such as $^{2}H$, $^{3}H$, $^{11}C$, $^{13}C$, $^{14}C$, $^{15}N$, $^{17}O$, $^{18}O$, $^{18}F$, $^{31}P$, $^{32}P$, $^{35}S$, $^{36}Cl$ and $^{125}I$.

On the other aspect, the present invention relates to intermediates for preparing compounds of formula (I), (II) or (III).

On the other aspect, the present invention provides a pharmaceutical composition comprising the compounds of the present invention. In one embodiment, the pharmaceutical composition of the present invention further comprises a pharmaceutically acceptable carrier, excipient, adjuvant, solvent or combination thereof. In another embodiment, the pharmaceutical composition may be a liquid, solid, semi-solid, gel or spray formulation.

## DESCRIPTION OF COMPOUNDS OF THE INVENTION

The present invention relates to substituted nitrogen-containing bicyclic compounds, pharmaceutically acceptable salts thereof, pharmaceutical preparations and compositions thereof, which can be used to inhibit PARP1 and have potential use in the treatment of PARP1 mediated diseases, particularly cancer. Furthermore, the substituted nitrogen-containing bicyclic compounds described in this invention exhibit significantly higher selectivity for PARP1 than other PARP subtypes (such as PARP2). The present invention further describes a method for synthesizing the compound. The compounds of this invention exhibit good biological activity.

In one aspect, the present invention relates to a compound, which is a compound of formula (I), or a stereoisomer, tautomer, N-oxide, hydrate, solvate, metabolite, pharmaceutically acceptable salt or prodrug thereof of a compound of formula (I).

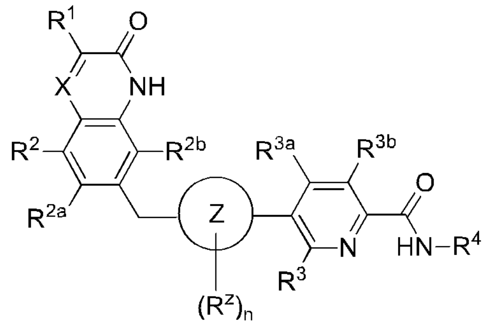

(I),

wherein, each of $R^1$, $R^2$, $R^{2a}$, $R^{2b}$, $R^3$, $R^{3a}$, $R^{3b}$, $R^4$, X,

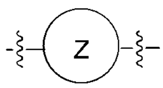

,

$R^z$ and n is as defined herein.

In some embodiments, the compound of formula (I) of the present invention does not include the following compounds:

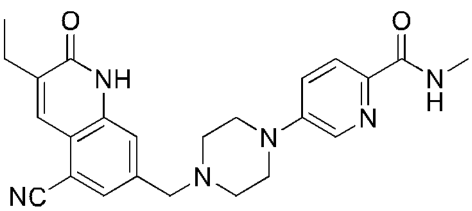

,

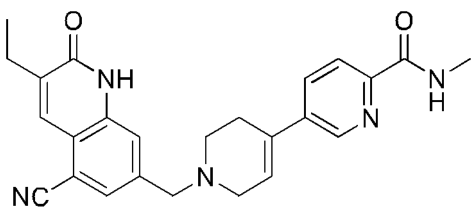

,

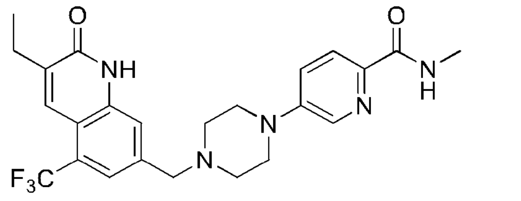
,
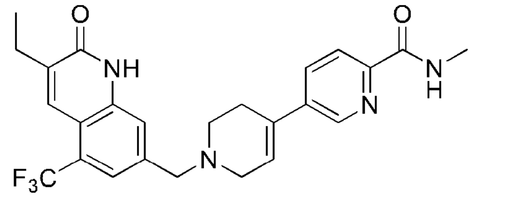
,
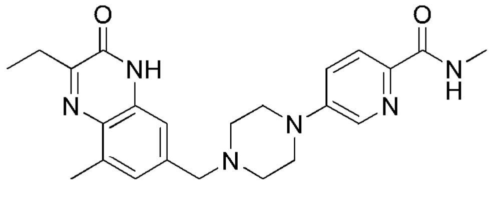
,
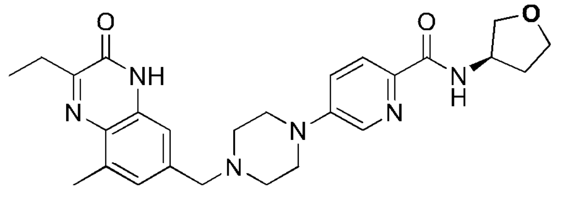
,
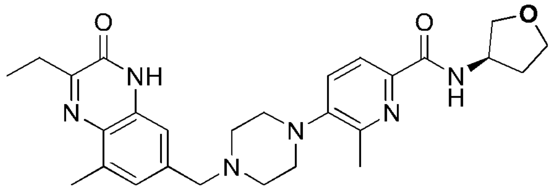
,
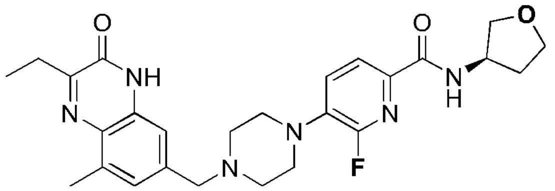
,
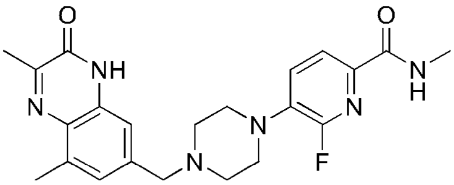
,
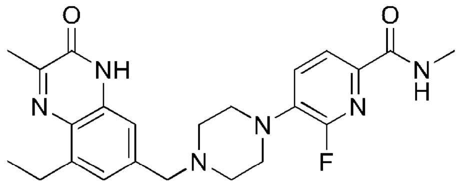
,
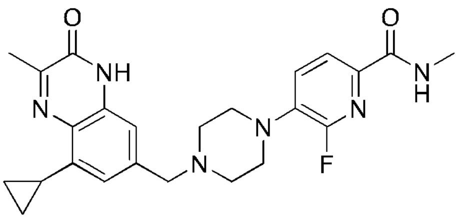
,
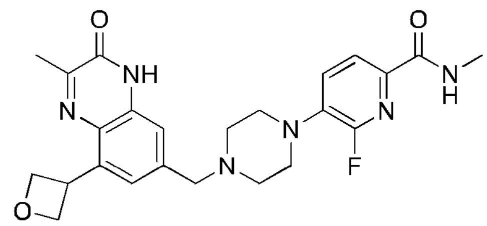
,
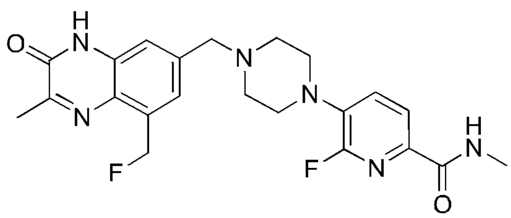
,
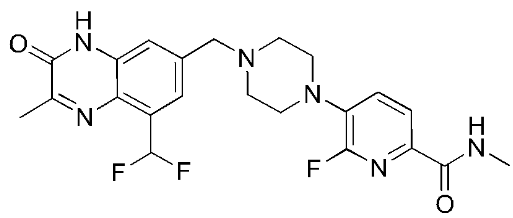
,
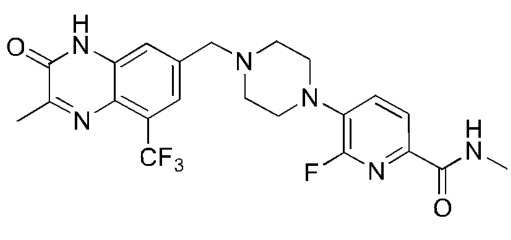
,
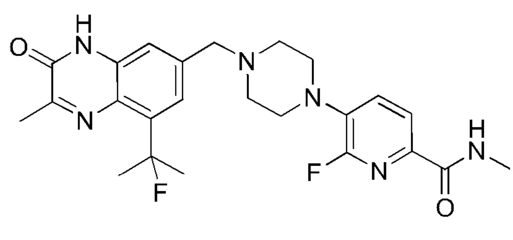
,

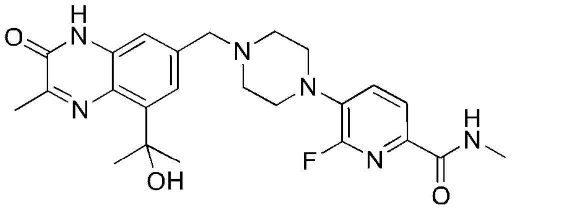
,
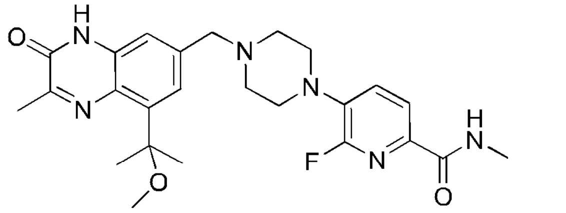
,

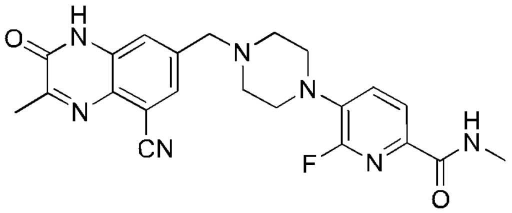
,
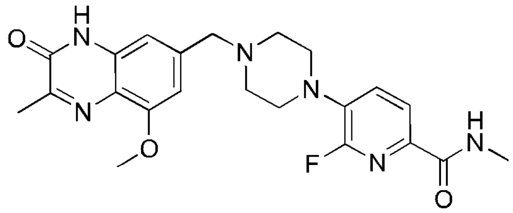
,

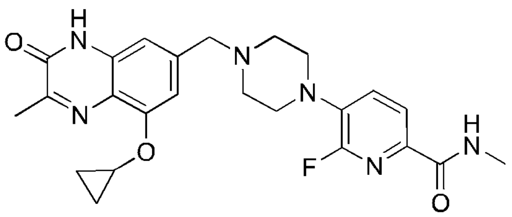
,
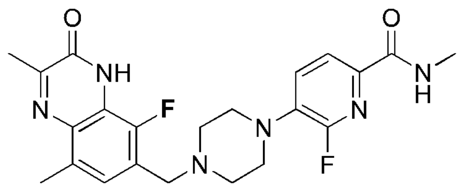
,

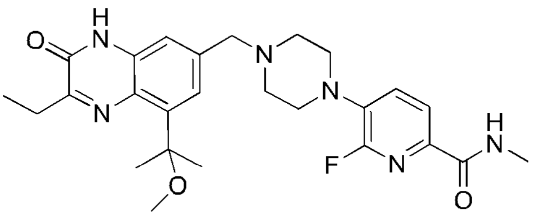
,
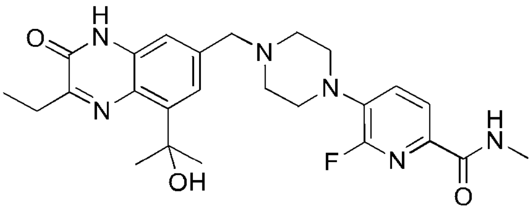
,

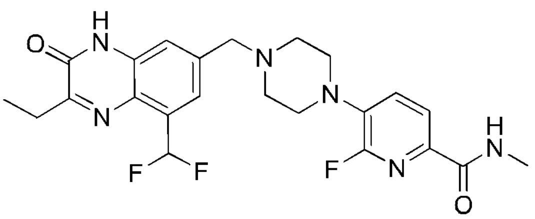
,
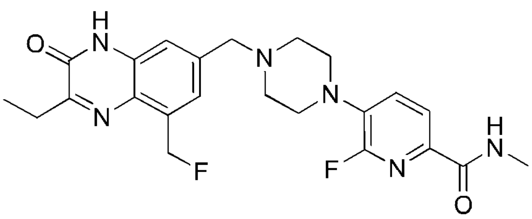
,

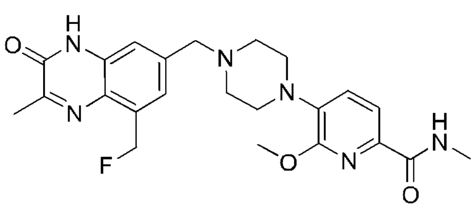
,
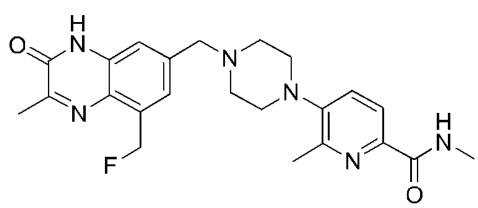
,

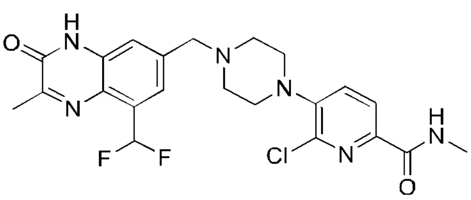
,
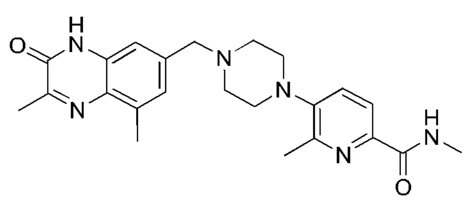

and

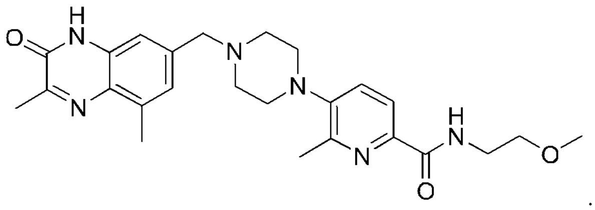

.

In some embodiments, X is $CR^x$ or N;

In some embodiments,

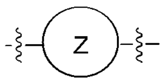

is 3-12 membered heterocyclyl.

In some embodiments,

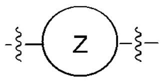

is 3-8 membered heterocyclyl.

In another embodiment,

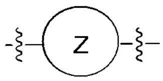

is

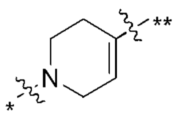 , 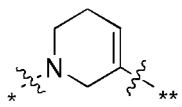 , 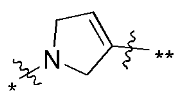 ,

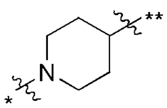 , 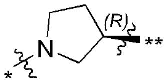 , 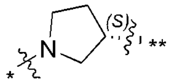 , 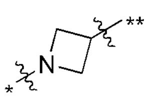 , 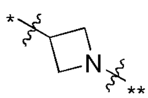 , 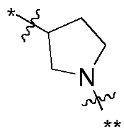 , 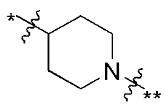 ,

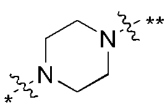 , 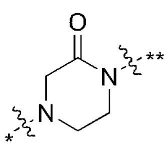 , 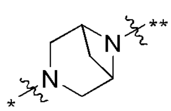 , 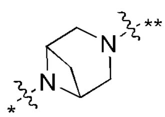 , 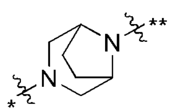 , 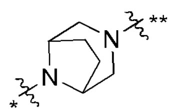 ,

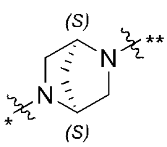 , 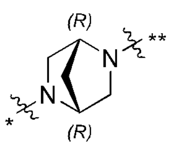 , 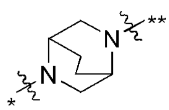 , 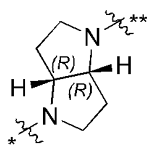 , 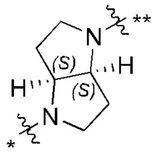 , 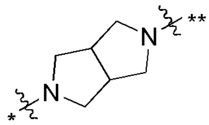 ,

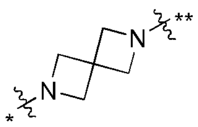 , 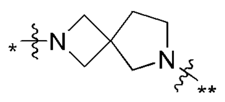 , 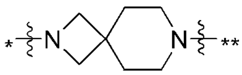 , 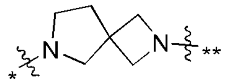 , 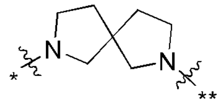 ,

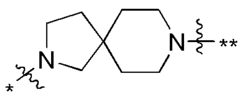 , 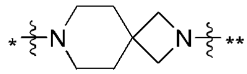 , 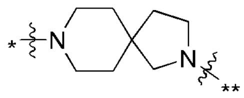 , 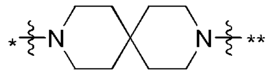 ,

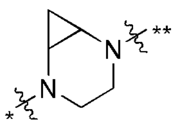 , 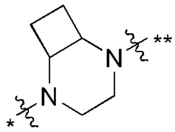 , 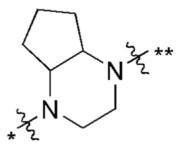 or 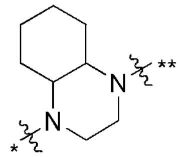 ;

wherein * indicates the connection of - $CH_2$- on the left and ** indicates the connection of pyridinyl group on the right.

In one embodiment, $R^1$ is H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, -C(=O)-($C_1$-$C_6$ alkyl), -C(=O)-($C_1$-$C_6$ alkoxy), $C_1$-$C_6$ alkyl, $C_2$-$C_6$ alkenyl, $C_2$-$C_6$ alkynyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino, $C_1$-$C_6$ hydroxyalkyl, $C_3$-$C_8$ cycloalkyl or 3-8 membered heterocyclyl.

In one embodiment, $R^1$ is H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, -C(=O)-($C_1$-$C_4$ alkyl), -C(=O)-($C_1$-$C_4$ alkoxy), $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino, $C_1$-$C_4$ hydroxyalkyl, $C_3$-$C_6$ cycloalkyl or 3-6 membered heterocyclyl.

In one embodiment, $R^1$ is H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, $-C(=O)-CH_3$, $-C(=O)-OCH_3$, methyl, ethyl, *n*-propyl, isopropyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, *n*-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, hydroxymethyl, 2-hydroxyethyl, cyclopropyl, cyclobutyl, cyclopentyl, cyclohexyl, aziridine, pyrrolidinyl, tetrahydrofuranyl, piperidinyl, piperazinyl or morpholinyl.

In one embodiment, each of $R^{2a}$ and $R^{2b}$ is independently H, D, F, Cl, Br, I, -CN, $-NO_2$, $-NH_2$, -OH, -SH, - COOH, $-C(=O)NH_2$, $-C(=O)NHCH_3$, $-C(=O)N(CH_3)_2$, -C(=O)-($C_1$-$C_4$ alkyl), -C(=O)-($C_1$-$C_6$ alkoxy), $C_1$-$C_6$ alkyl, $C_2$-$C_6$ alkenyl, $C_2$-$C_6$ alkynyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino, $C_1$-$C_6$ hydroxyalkyl, $C_1$-$C_6$ cyanoalkyl, $C_3$-$C_8$ cycloalkyl, 3-8 membered heterocyclyl, $C_6$-$C_{10}$ aryl or 5-10 membered heteroaryl.

In one embodiment, each of $R^{2a}$ and $R^{2b}$ is independently H, D, F, Cl, Br, I, -CN, $-NO_2$, $-NH_2$, -OH, -SH, - COOH, $-C(=O)NH_2$, $-C(=O)NHCH_3$, $-C(=O)N(CH_3)_2$, -C(=O)-($C_1$-$C_4$ alkyl), -C(=O)-($C_1$-$C_4$ alkoxy), $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino, $C_1$-$C_4$ hydroxyalkyl, $C_1$-$C_4$ cyanoalkyl, $C_3$-$C_6$ cycloalkyl, 3-6 membered heterocyclyl, $C_6$-$C_{10}$ aryl or 5-6 membered heteroaryl.

In another embodiment, each of $R^{2a}$ and $R^{2b}$ is independently H, D, F, Cl, Br, I, -CN, $-NO_2$, $-NH_2$, -OH, -SH, - COOH, $-C(=O)NH_2$, $-C(=O)NHCH_3$, $-C(=O)N(CH_3)_2$, -C(=O)-($C_1$-$C_2$ alkyl), -C(=O)-($C_1$-$C_2$ alkoxy), methyl, ethyl, n-propyl, isopropyl, allyl, propenyl, propargyl, propynyl, $-CHF_2$, $-CF_3$, $-CHFCH_2F$, $-CF_2CHF_2$, $-CH_2CF_3$, - $CH_2CF_2CHF_2$, methoxy, ethoxy, *n*-propyloxy, isopropyloxy, $-OCHF_2$, $-OCF_3$, $-OCHFCH_2F$, $-OCF_2CHF_2$, - $OCH_2CF_3$, $-OCH_2CF_2CHF_2$, methylthio, ethylthio, methylamino, dimethylamino, ethylamino, hydroxymethyl, 2-hydroxyethyl, cyanomethyl, cyclopropyl, cyclobutyl, cyclopentyl, cyclohexyl, aziridine, pyrrolyl, tetrahydrofuranyl, piperidinyl, piperazine, morpholinyl, phenyl, indole, naphthyl, pyrroleyl, pyrazolyl, imidazoleyl, triazolyl, tetrazolyl, furanyl, thiophenyl, thiazolyl, oxazolyl, pyridinyl, pyrimidinyl, pyrazinyl or pyridazinyl.

In one embodiment, $R^2$ is -CN, $-NO_2$, $-NH_2$, -OH, -SH, -COOH, $-C(=O)NH_2$, $-C(=O)NHCH_3$, $-C(=O)N(CH_3)_2$, -C(=O)-($C_1$-$C_6$ alkyl), -C(=O)-($C_1$-$C_6$ alkoxy), $C_1$-$C_6$ alkyl, $C_2$-$C_6$ alkenyl, $C_2$-$C_6$ alkynyl, $C_1$-$C_6$ alkoxy, $C_1$-$C_6$ haloalkoxy, $C_1$-$C_6$ alkylthio, $C_1$-$C_6$ alkylamino, $C_1$-$C_6$ hydroxyalkyl, $C_1$-$C_6$ cyanoalkyl, $C_3$-$C_8$ cycloalkyl-L-, 3-8 membered heterocyclyl-L-, $C_6$-$C_{10}$ aryl-L- or 5-10 membered heteroaryl-L-; wherein, $R^2$ is unsubstituted or substituted by 1, 2, 3, 4 or 5 $R^w$; wherein, L and $R^w$ have the meanings as described in this invention.

In one embodiment, $R^2$ is -CN, $-NO_2$, $-NH_2$, -OH, -SH, -COOH, $-C(=O)NH_2$, $-C(=O)NHCH_3$, $-C(=O)N(CH_3)_2$, -C(=O)-($C_1$-$C_4$ alkyl), -C(=O)-($C_1$-$C_4$ alkoxy), $C_1$-$C_4$ alkyl, $C_2$-$C_4$ alkenyl, $C_2$-$C_4$ alkynyl, $C_1$-$C_4$ alkoxy, $C_1$-$C_4$ haloalkoxy, $C_1$-$C_4$ alkylthio, $C_1$-$C_4$ alkylamino, $C_1$-$C_4$ hydroxyalkyl, $C_1$-$C_4$ cyanoalkyl, $C_3$-$C_6$ cycloalkyl-L-, 3-6 membered hetero-

cyclyl-L-, $C_6$-$C_{10}$ aryl-L- or 5-6 membered heteroaryl-L-; wherein, $R^2$ is unsubstituted or substituted by 1, 2, 3, 4 or 5 $R^w$; wherein, L and $R^w$ have the meanings as described in this invention.

In another embodiment, $R^2$ is -CN, $-NO_2$, $-NH_2$, -OH, -SH, -COOH, $-C(=O)NH_2$, $-C(=O)NHCH_3$, - $C(=O)N(CH_3)_2$, $-C(=O)-CH_3$, $-C(=O)-OCH_3$, methyl, ethyl, *n*-propyl, isopropyl, *n*-butyl, *tert*-butyl, allyl, propenyl, propynyl, methoxy, ethoxy, *n*-propyloxy, isopropyloxy, $-OCHF_2$, $-OCF_3$, $-OCHFCH_2F$, $-OCF_2CHF_2$, $-OCH_2CF_3$, - $OCH_2CF_2CHF_2$, methylthio, ethylthio, methylamino, dimethylamino, ethylamino, hydroxymethyl, 2-hydroxyethyl, cyanomethyl, cyclopropyl-L-, cyclobutyl-L-, cyclopentyl-L-, cyclohexyl-L-, oxacyclopropyl-L-, azircyclopropyl-L-, oxacyclobutyl-L-, azircyclobutyl-L-, tetrahydrofuranyl-L-, pyrrolidinyl-L-, tetrahydropyranyl-L-, piperidinyl-L-, piperazinyl-L-, morpholinyl-L-, phenyl-L-, naphthyl-L-, pyrrolidinyl-L-, furanyl-L-, thiophenyl-L-, pyrazolyl-L-, imidazolyl-L-, thiazolyl-L-, oxazolyl-L-, triazolyl-L-, tetrazolyl-L-, pyridinyl-L-, pyrimidinyl-L-, pyrazinyl-L-or pyridazinyl-L-; wherein, $R^2$ is unsubstituted or substituted by 1, 2, 3, 4 or 5 $R^w$; wherein, L and $R^w$ have the meanings as described in this invention.

In one embodiment, each -L- is independently a bond, $-NR^n-$, -O-, -S-, -C(=O)-, -C(=O)O-, $-C(=O)N(R^{n1})-$ or $-(CR^aR^b)_m-$; wherein each $R^n$, $R^{n1}$, $R^a$, $R^b$ and m has the meaning as described in this invention.

In one embodiment, each -L- is independently a bond, -NH-, -O-, -S-, -C(=O)-, -C(=O)O-, -C(=O)NH- or - $(CH_2)_m$-; wherein m has the meaning as described in this invention.

In one embodiment, each $R^n$ and $R^{n1}$ is independently H, D, $C_1$-$C_6$ alkyl, $C_1$-$C_6$ haloalkyl, $C_3$-$C_8$ cycloalkyl, 3-8 membered heterocyclyl, $C_6$-$C_{10}$ aryl or 5-10 membered heteroaryl.

In one embodiment, each of $R^n$ and $R^{n1}$ is independently H, D, $C_1$-$C_4$ alkyl, $C_1$-$C_4$ haloalkyl, $C_3$-$C_6$ cycloalkyl, 3-6 membered heterocyclyl, $C_6$-$C_{10}$ aryl or 5-6 membered heteroaryl.

In another embodiment, each of $R^n$ and $R^{n1}$ is independently H, D, methyl, ethyl, n-propyl, isopropyl, n-butyl, tert-butyl, $-CHF_2$, $-CF_3$, $-CHFCH_2F$, $-CF_2CHF_2$, $-CH_2CF_3$, $-CH_2CF_2CHF_2$, cyclopropyl, cyclobutyl, cyclopentyl, cyclohexyl, aziridine, pyrrolyl, tetrahydrofuranyl, piperidinyl, piperazine, morpholinyl, phenyl, pyrrolyl, pyrazolyl, imidazolyl, triazolyl, tetrazolyl, furanyl, thiophenyl, thiazolyl, oxazolyl, pyridinyl, pyrimidinyl, pyrazinyl or pyridazinyl.

In one embodiment, each of $R^3$, $R^{3a}$ and $R^{3b}$ is independently H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, -C(=O)-($C_1$-$C_6$ alkyl), -C(=O)-($C_1$-$C_6$ alkoxy), $C_1$-$C_6$ alkyl, $C_2$-$C_6$ alkenyl, $C_2$-$C_6$ alkynyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino or $C_1$-$C_6$ hydroxyalkyl.

In one embodiment, each of $R^3$, $R^{3a}$ and $R^{3b}$ is independently H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, -C(=O)-($C_1$-$C_4$ alkyl), -C(=O)-($C_1$-$C_4$ alkoxy), $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino or $C_1$-$C_4$ hydroxyalkyl.

In another embodiment, each of $R^3$, $R^{3a}$ and $R^{3b}$ is independently H, D, F, Cl, Br, I, -CN, $-NO_2$, $-NH_2$, -OH, - SH, -COOH, $-C(=O)NH_2$, $-C(=O)NHCH_3$, $-C(=O)N(CH_3)_2$, $-C(=O)-CH_3$, $-C(=O)-OCH_3$, methyl, ethyl, *n*-propyl, isopropyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, *n*-propyloxy, isopropyloxy, $-OCHF_2$, $-OCF_3$, $-OCHFCH_2F$, $-OCF_2CHF_2$, $-OCH_2CF_3$, - $OCH_2CF_2CHF_2$, methylthio, ethylthio, methylamino, dimethylamino, ethylamino, hydroxymethy or 2-hydroxyethyl.

In one embodiment, each of $R^a$ and $R^b$ is independently H, D, F, Cl, Br, I, -CN, $-NO_2$, $-NH_2$, -OH, -SH, $C_1$-$C_6$ alkyl, $C_2$-$C_6$ alkenyl, $C_2$-$C_6$ alkynyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino or $C_1$-$C_6$ hydroxyalkyl.

In one embodiment, each of $R^a$ and $R^b$ is independently H, D, F, Cl, Br, I, -CN, $-NO_2$, $-NH_2$, -OH, -SH, $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino or $C_1$-$C_4$ hydroxyalkyl.

In another embodiment, each of $R^a$ and $R^b$ is independently H, D, F, Cl, Br, I, -CN, $-NO_2$, $-NH_2$, -OH, -SH, methyl, ethyl, *n*-propyl, isopropyl, allyl, propenyl, propargyl, propynyl, $-CHF_2$, $-CF_3$, $-CHFCH_2F$, $-CF_2CHF_2$, - $CH_2CF_3$, $-CH_2CF_2CHF_2$, methoxy, ethoxy, n-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, hydroxymethy or 2-hydroxyethyl.

In one embodiment, $R^4$ is H, D, $C_1$-$C_6$ alkyl, $C_2$-$C_6$ alkenyl, $C_2$-$C_6$ alkynyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino, $C_3$-$C_8$ cycloalkyl, 3-8 membered heterocyclyl, $C_6$-$C_{10}$ aryl or 5-10 membered heteroaryl, wherein the $C_1$-$C_6$ alkyl, $C_3$-$C_8$ cycloalkyl, 3-8 membered heterocyclyl, $C_6$-$C_{10}$ aryl and 5-10 membered heteroaryl are optionally and independently substituted by 1, 2, 3, 4 or 5 groups selected from D, F, Cl, Br, I, -OH, $-NH_2$, $-NO_2$, -CN, $C_1$-$C_6$ alkyl, $C_1$-$C_6$ haloalkyl, $C_1$-$C_6$ alkoxy and $C_1$-$C_6$ haloalkoxy.

In one embodiment, $R^4$ is H, D, $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino, $C_3$-$C_6$ cycloalkyl, 3-6 membered heterocyclyl, $C_6$-$C_{10}$ aryl, or 5-6 membered heteroaryl, wherein the $C_1$-$C_4$ alkyl, $C_3$-$C_6$ cycloalkyl, 3-6 membered heterocyclyl, $C_6$-$C_{10}$ aryl and 5-6 membered heteroaryl are optionally and independently substituted by 1, 2, 3, 4 or 5 groups selected from D, F, Cl, Br, 1, -OH, $-NH_2$, $-NO_2$, -CN, $C_1$-$C_4$ alkyl, $C_1$-$C_4$ haloalkyl, $C_1$-$C_4$ alkoxy and $C_1$-$C_4$ haloalkoxy.

In another embodiment, $R^4$ is H, D, methyl, ethyl, *n*-propyl, isopropyl, allyl, propenyl, propargyl, propynyl, - $CHF_2$, $-CF_3$, $-CHFCH_2F$, $-CF_2CHF_2$, $-CH_2CF_3$, $-CH_2CF_2CHF_2$, methoxy, ethoxy, n-propyloxy, isopropyloxy, - $OCHF_2$, $-OCF_3$,

$-OCHFCH_2F$, $-OCF_2CHF_2$, $-OCH_2CF_3$, $-OCH_2CF_2CHF_2$, methylthio, ethylthio, methylamino, dimethylamino, ethylamino, cyclopropyl, cyclobutyl, cyclopentyl, cyclohexyl, azacyclobutyl, oxacyclobutyl, pyrrolyl, tetrahydrofuranyl, tetrahydropyranyl, piperidinyl, piperazinyl, morpholinyl, phenyl, indole, naphthyl, pyrroleyl, pyrazolyl, imidazoleyl, triazolyl, tetrazolyl, furanyl, thiophenyl, thiazolyl, oxazolyl, pyridinyl, pyrimidinyl, pyrazinyl or pyridazinyl, wherein, each of the methyl, ethyl, *n*-propyl, isopropyl, cyclopropyl, cyclobutyl, cyclopentyl, cyclohexyl, aziridine, oxaziridine, pyrrolyl, tetrahydrofuranyl, tetrahydropyranyl, piperidinyl, piperazine, morpholinyl, phenyl, indole, naphthyl, pyrrolyl, pyrazolyl, imidazoleyl, triazolyl, tetrazolyl, furanyl, thiophenyl, thiazolyl, oxazolyl, pyridinyl, pyrimidinyl, pyrazinyl and pyridazinyl is independently and optionally substituted by 1, 2, 3, 4 or 5 groups selected from D, F, Cl, Br, I, -OH, $-NH_2$, $-NO_2$, -CN, methyl, ethyl, n-propyl, isopropyl, $-CHF_2$, $-CF_3$, $-CHFCH_2F$, $-CF_2CHF_2$, $-CH_2CF_3$, $-CH_2CF_2CHF_2$, methoxy, ethoxy, n-propyloxy, isopropyloxy, $-OCHF_2$, $-OCF_3$, $-OCHFCH_2F$, $-OCF_2CHF_2$, $-OCH_2CF_3$ and $-OCH_2CF_2CHF_2$.

In one embodiment, each of $R^x$ and $R^z$ is independently H, D, F, Cl, Br, I, -CN, $-NO_2$, $-NH_2$, -OH, -COOH, - C(=O)$NH_2$, $C_1$-$C_6$ alkyl, $C_1$-$C_6$ haloalkyl, $C_1$-$C_6$ alkoxy, $C_1$-$C_6$ haloalkoxy or $C_1$-$C_6$ hydroxyalkyl.

In one embodiment, each of $R^x$ and $R^z$ is independently H, D, F, Cl, Br, I, -CN, $-NO_2$, $-NH_2$, -OH, -COOH, - C(=O)$NH_2$, $C_1$-$C_4$ alkyl, $C_1$-$C_4$ haloalkyl, $C_1$-$C_4$ alkoxy, $C_1$-$C_4$ haloalkoxy or $C_1$-$C_4$ hydroxyalkyl.

In another embodiment, each of $R^x$ and $R^z$ is independently H, D, F, Cl, Br, I, -CN, $-NO_2$, $-NH_2$, -OH, -COOH, -C(=O)$NH_2$, methyl, ethyl, *n*-propyl, isopropyl, $-CHF_2$, $-CF_3$, $-CHFCH_2F$, $-CF_2CHF_2$, $-CH_2CF_3$, $-CH_2CF_2CHF_2$, methoxy, ethoxy, *n*-propyloxy, isopropyloxy, $-OCHF_2$, $-OCF_3$, $-OCHFCH_2F$, $-OCF_2CHF_2$, $-OCH_2CF_3$, - $OCH_2CF_2CHF_2$ hydroxymethy or 2-hydroxyethyl.

In one embodiment, each $R^w$ is independently H, D, F, Cl, Br, I, -CN, $-NO_2$, $-NH_2$, -OH, -SH, -COOH, - C(=O)$NH_2$, -C(=O)$NHCH_3$, -C(=O)N$(CH_3)_2$, -C(=O)-($C_1$-$C_6$ alkyl), -C(=O)-($C_1$-$C_6$ alkoxy), $C_1$-$C_6$ alkyl, $C_2$-$C_6$ alkenyl, $C_2$-$C_6$ alkynyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino or $C_1$-$C_6$ hydroxyalkyl, $C_3$-$C_8$ cycloalkyl, 3-8 membered heterocyclyl, $C_6$-$C_{10}$ aryl or 5-10 membered heteroaryl.

In one embodiment, each $R^w$ is independently H, D, F, Cl, Br, I, -CN, $-NO_2$, $-NH_2$, -OH, -SH, -COOH, - C(=O)$NH_2$, -C(=O)$NHCH_3$, -C(=O)N$(CH_3)_2$, -C(=O)-($C_1$-$C_4$ alkyl), -C(=O)-($C_1$-$C_4$ alkoxy), $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino, $C_1$-$C_4$ hydroxyalkyl, $C_3$-$C_6$ cycloalkyl, 3-6 membered heterocyclyl, $C_6$-$C_{10}$ aryl or 5-6 membered heteroaryl.

In another embodiment, each $R^w$ is independently H, D, F, Cl, Br, I, -CN, $-NO_2$, $-NH_2$, -OH, -SH, -COOH, - C(=O)$NH_2$, -C(=O)$NHCH_3$, -C(=O)N$(CH_3)_2$, -C(=O)-($C_1$-$C_3$ alkyl), -C(=O)-($C_1$-$C_3$ alkoxy), methyl, ethyl, *n*-propyl, isopropyl, *n*-butyl, *tert*-butyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, *n*-propyloxy, isopropyloxy, $-OCHF_2$, $-OCF_3$, $-OCHFCH_2F$, $-OCF_2CHF_2$, - $OCH_2CF_3$, $-OCH_2CF_2CHF_2$, methylthio, ethylthio, methylamino, dimethylamino, ethylamino, hydroxymethyl, 2-hydroxyethyl, cyclopropyl, cyclobutyl, cyclopentyl, cyclohexyl, aziridine, pyrrolyl, tetrahydrofuranyl, piperidinyl, piperazine, morpholinyl, phenyl, pyrrolyl, pyrazolyl, imidazolyl, triazolyl, tetrazolyl, furanyl, thiophenyl, thiazolyl, oxazolyl, pyridinyl, pyrimidinyl, pyrazinyl or pyridazinyl.

In one embodiment, m is 1, 2, 3, 4, 5 or 6.

In one embodiment, n is 1, 2, 3, 4, 5 or 6.

In some embodiments, the present invention relates to a compound of formula (II), or a stereoisomer, tautomer, N-oxide, hydrate, solvate, metabolite, pharmaceutically acceptable salt or prodrug thereof of a compound of formula (II),

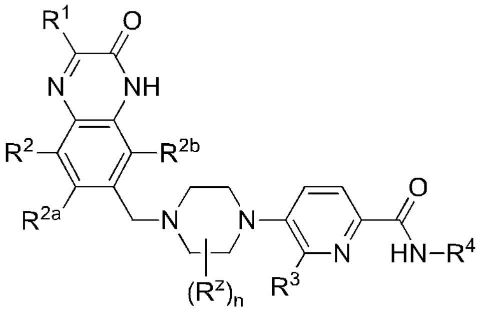

(II),

wherein each of $R^1$, $R^2$, $R^{2a}$, $R^{2b}$, $R^3$, $R^4$, $R^z$ and n is as defined herein.

In other embodiments, the present invention relates to a compound of formula (III), or a stereoisomer, tautomer, N-oxide, hydrate, solvate, metabolite, pharmaceutically acceptable salt, or prodrug thereof of a compound of formula (III),

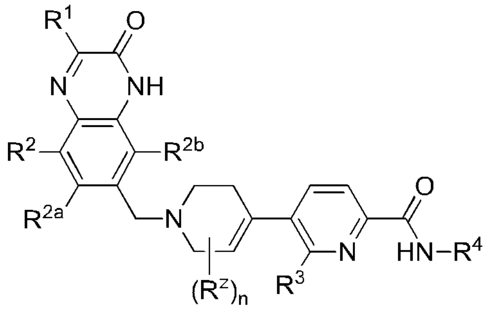
(III),

wherein each of $R^1$, $R^2$, $R^{2a}$, $R^{2b}$, $R^3$, $R^4$, $R^z$ and n is as defined herein.

In one embodiment, the compound of the present invention is a compound having one of the following structures or a stereoisomer, tautomer, N-oxide, hydrate, solvate, metabolite, pharmaceutically acceptable salt, or prodrug thereof, but is by no means limited to:

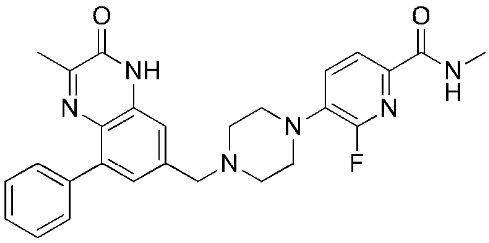
2,

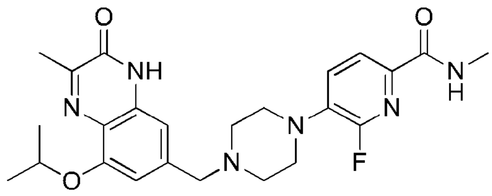
3,

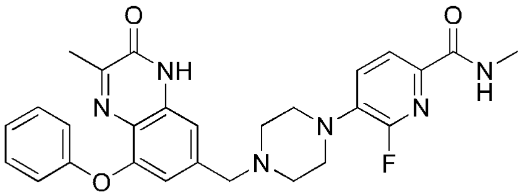
4,

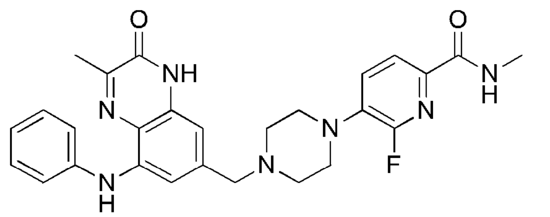
5,

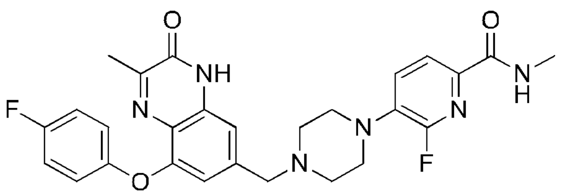
6,

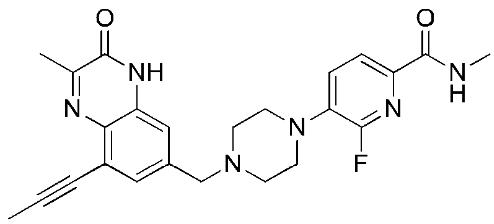
7,

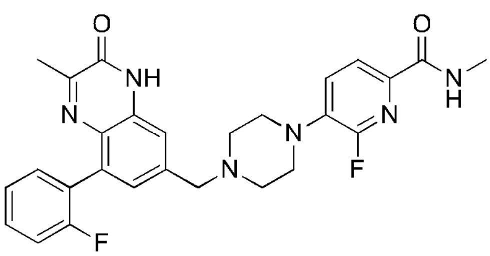
8,

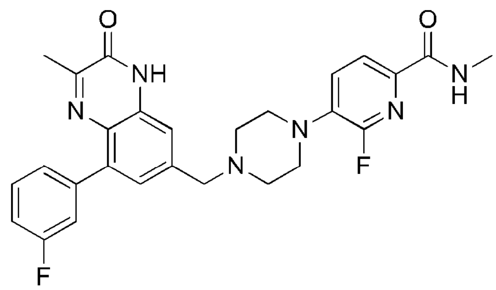
9,

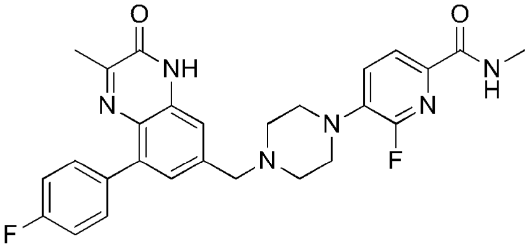

10,

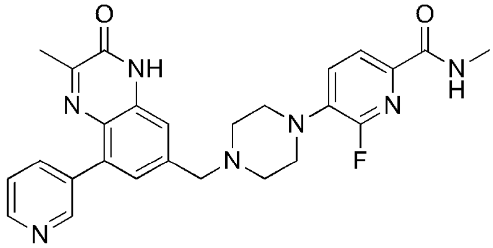

11,

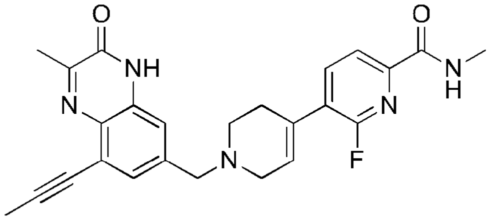

12,

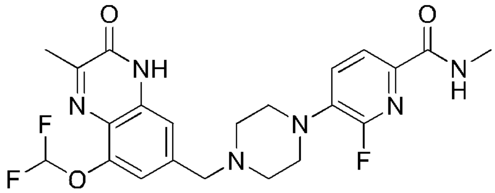

13,

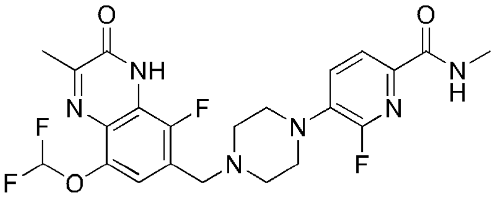

14,

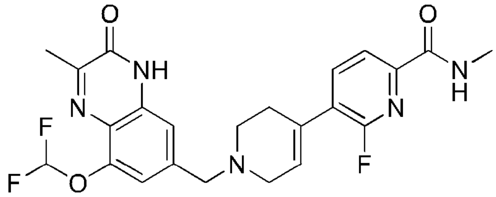

15,

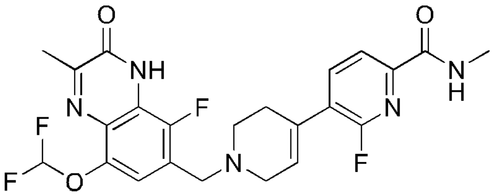

16,

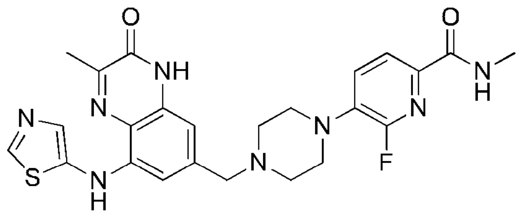

17,

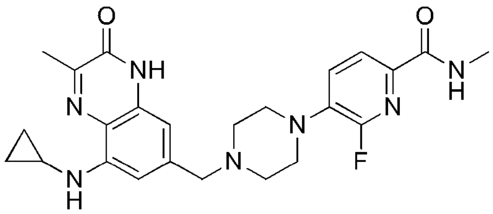

18,

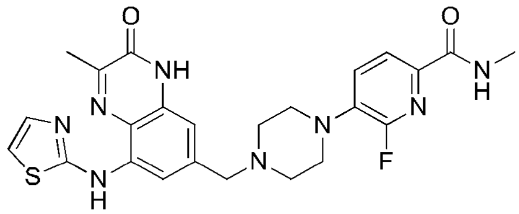

19,

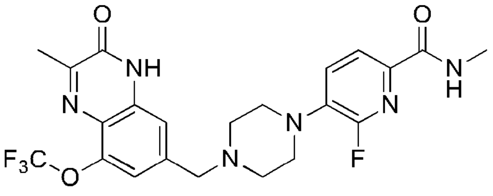

20,

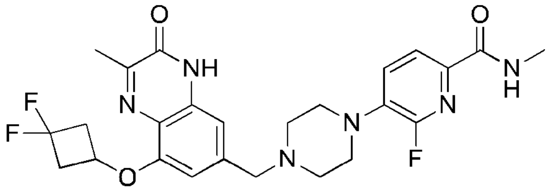

21,

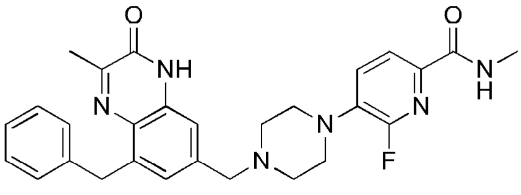

22,

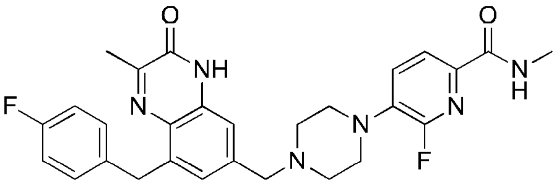

23,

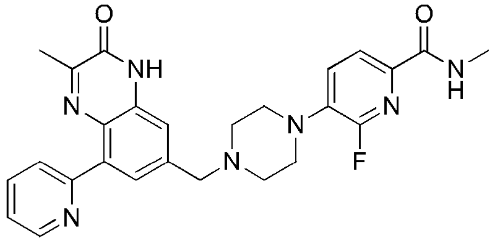
24,

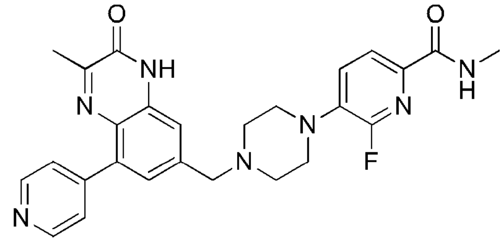
25,

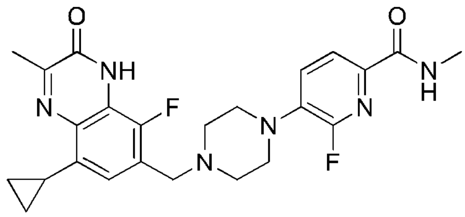
26,

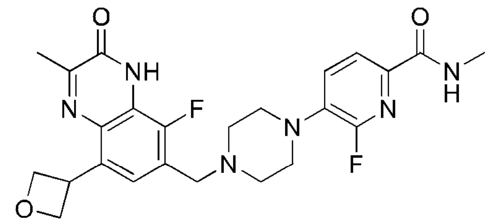
27,

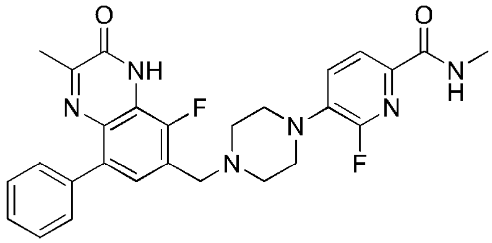
28,

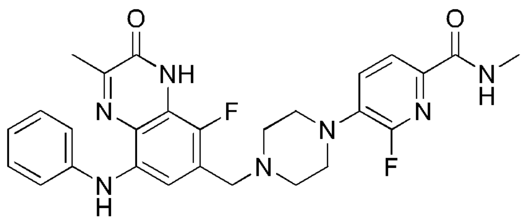
29,

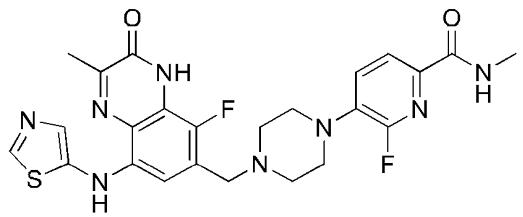
30,

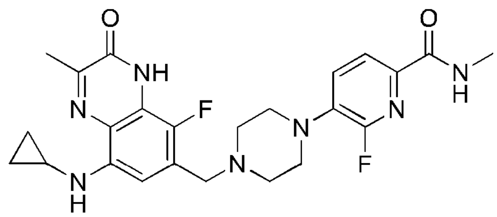
31,

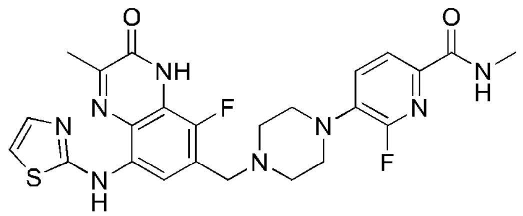
32,

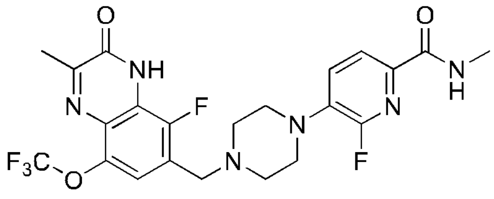
33,

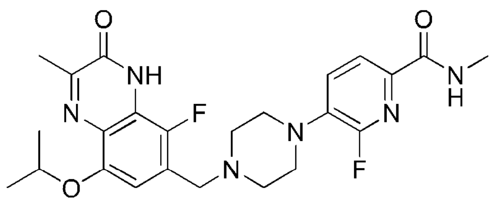
34,

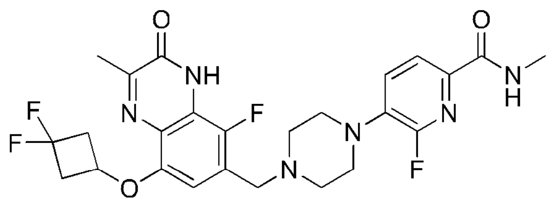
35,

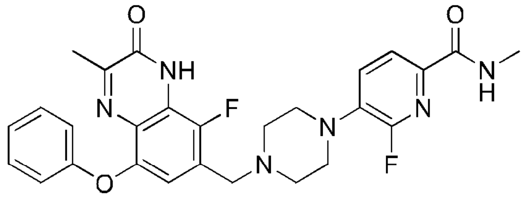

36,

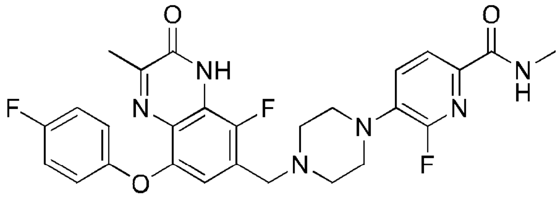

37,

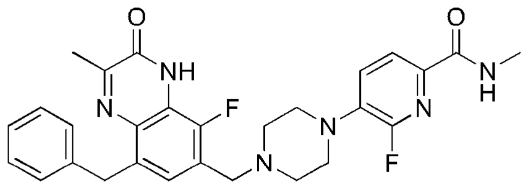

38,

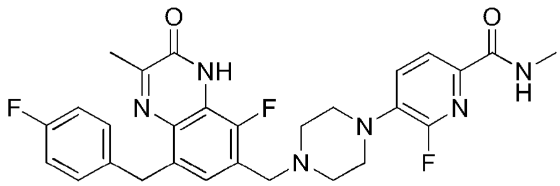

39,

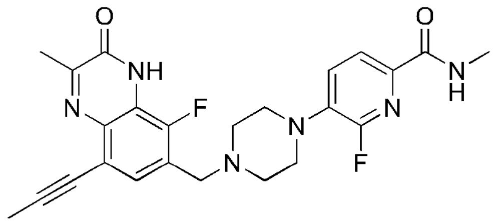

40,

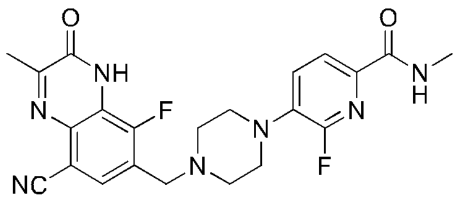

41,

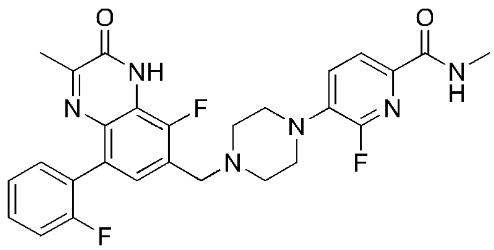

42,

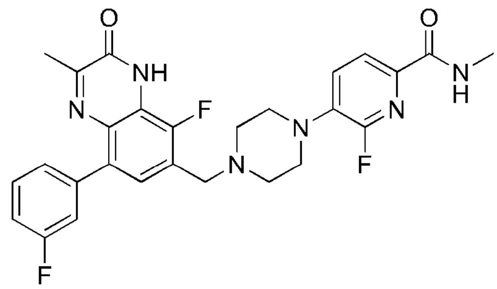

43,

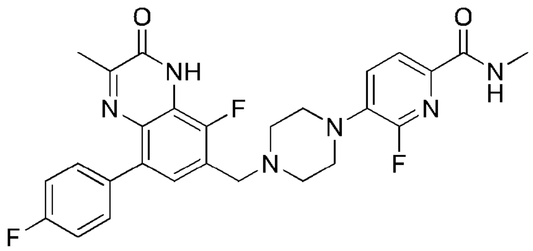

44,

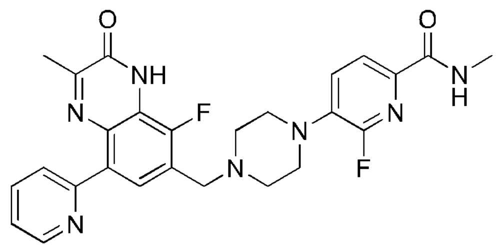

45,

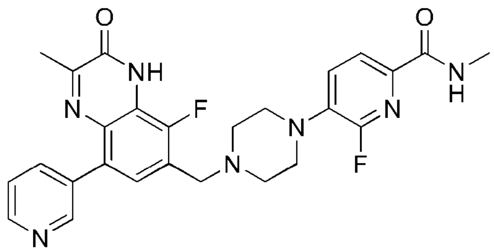

46,

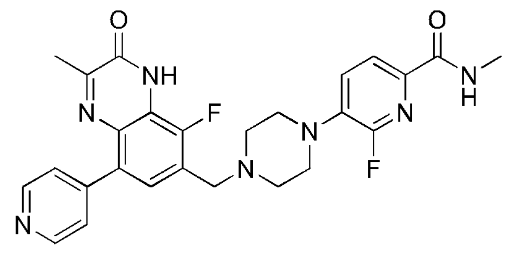

47,

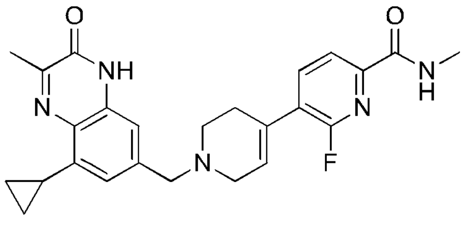
48,

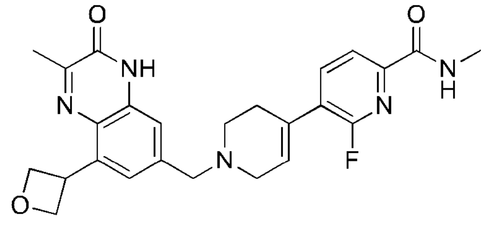
49,

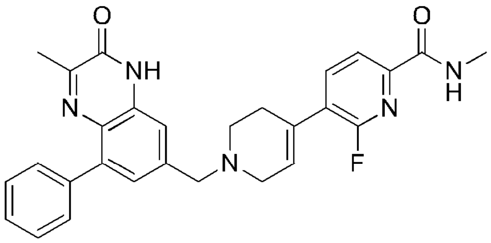
50,

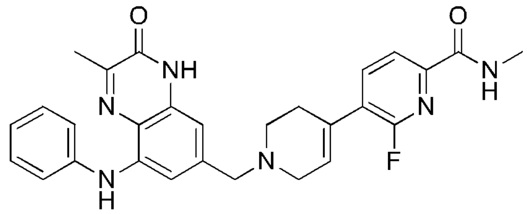
51,

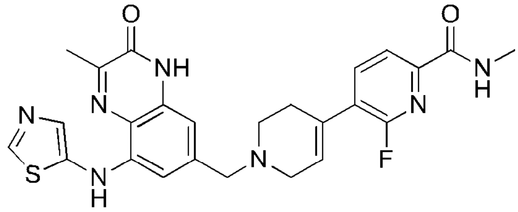
52,

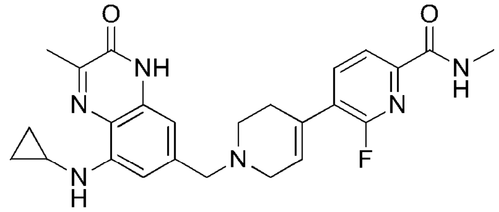
53,

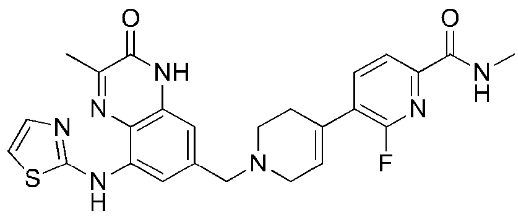
54,

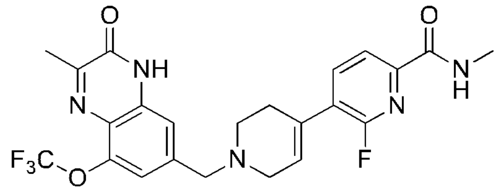
55,

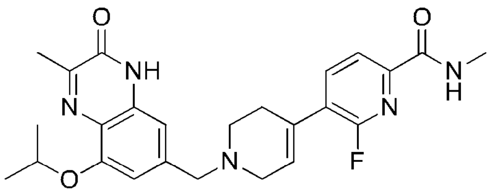
56,

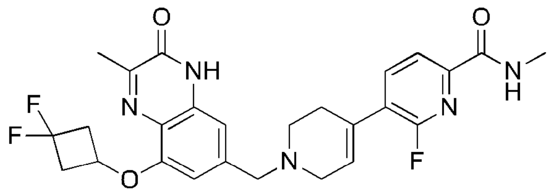
57,

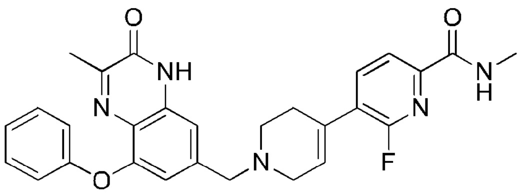
58,

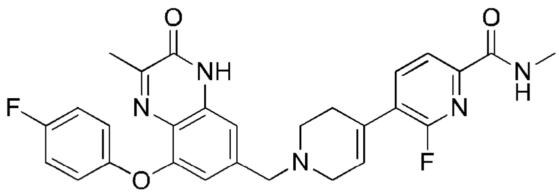
59,

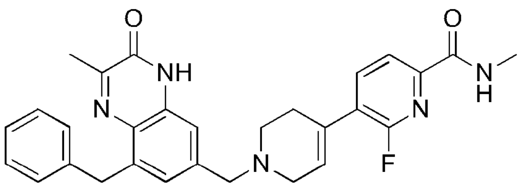
60,

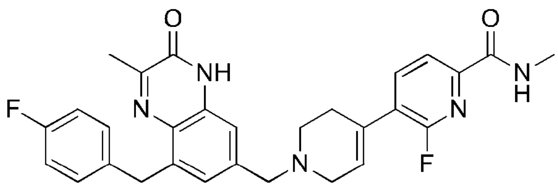
61,

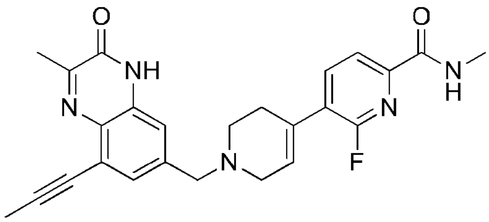 62, 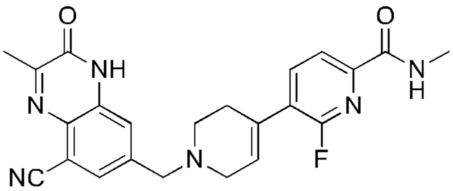 63,

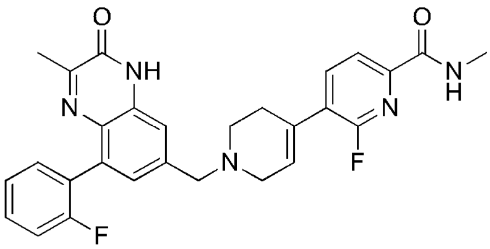 64, 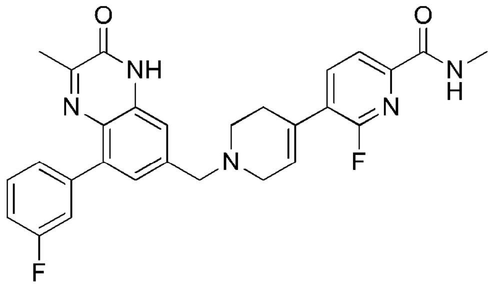 65,

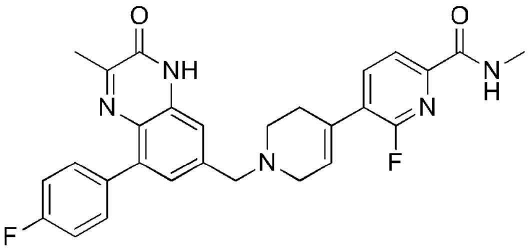 66, 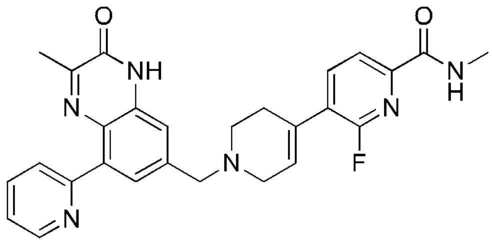 67,

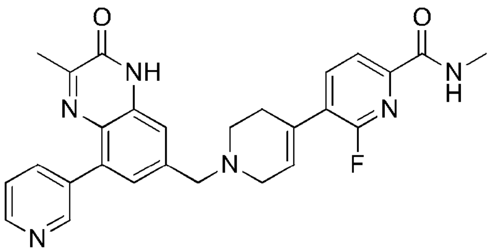 68, 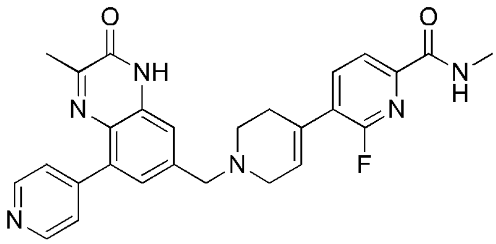 69,

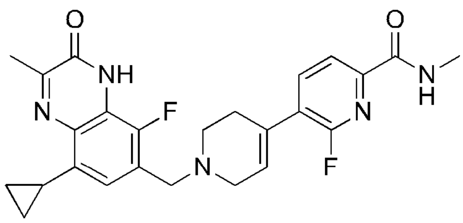 70, 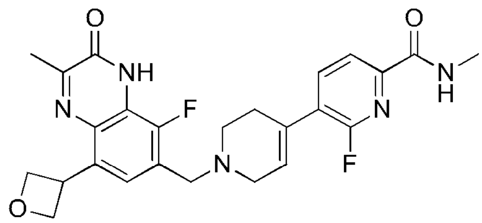 71,

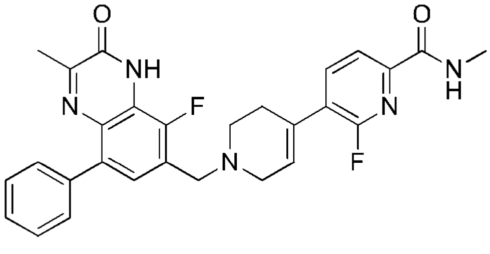 72, 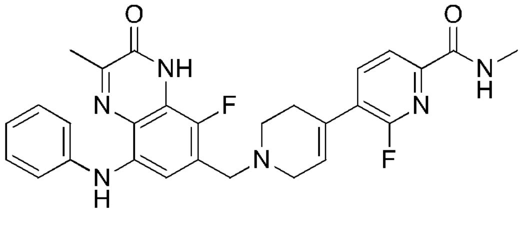 73,

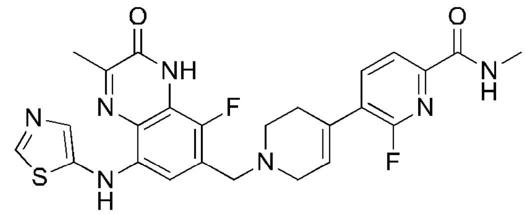
74,

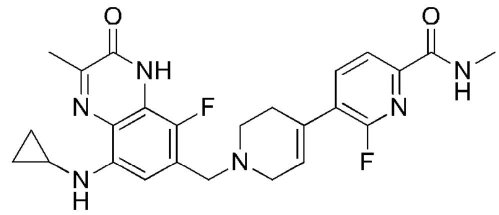
75,

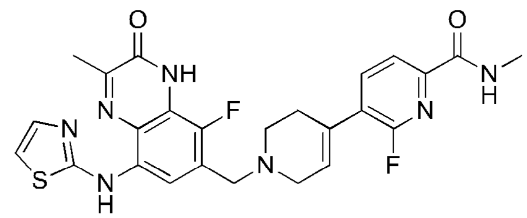
76,

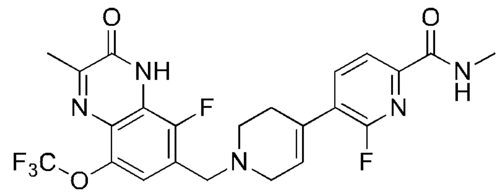
77,

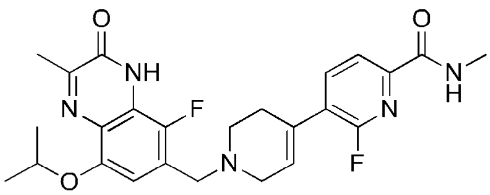
78,

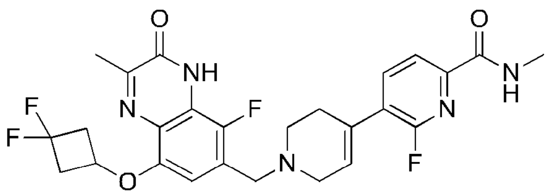
79,

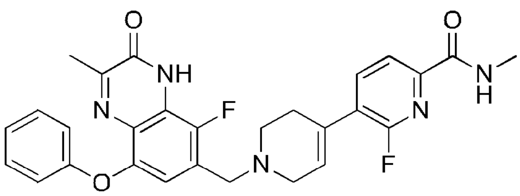
80,

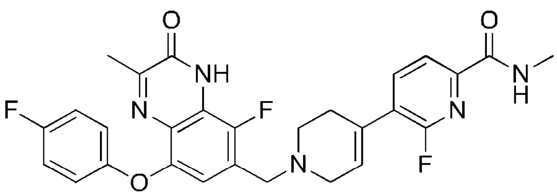
81,

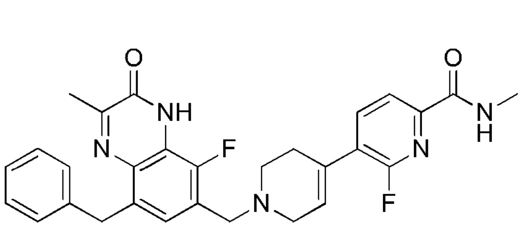
82,

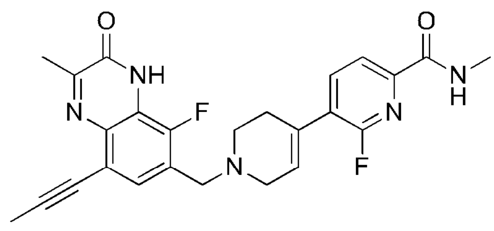
83,

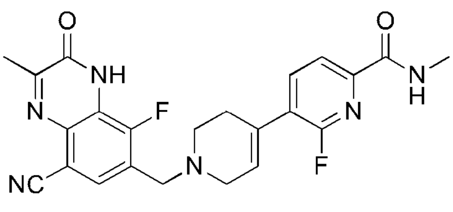
84,

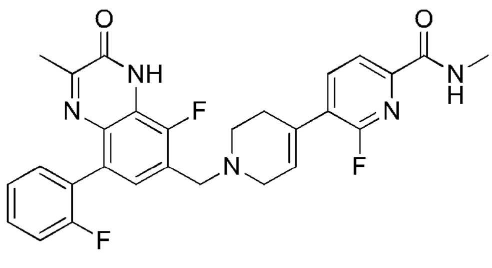
85,

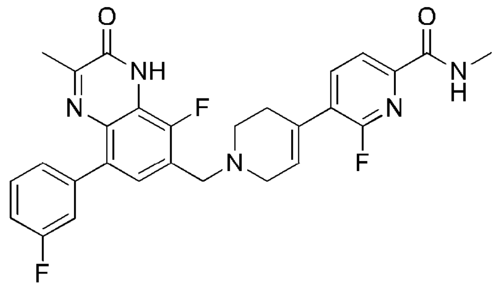
86,

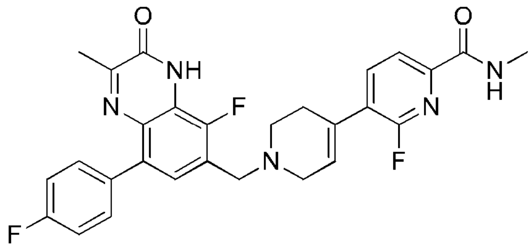
87,

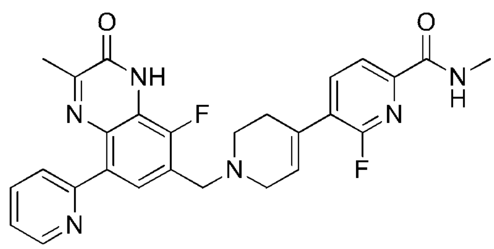
88,

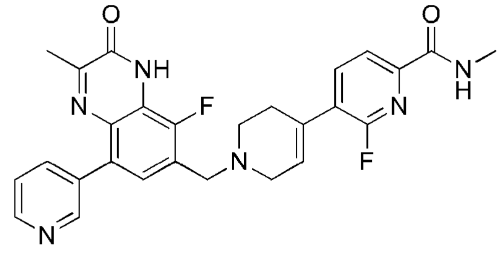
89,

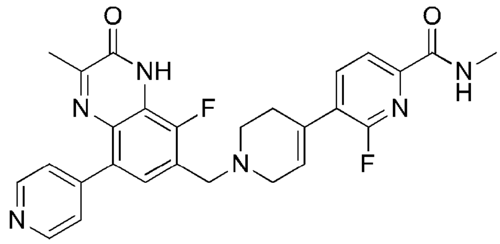
90,

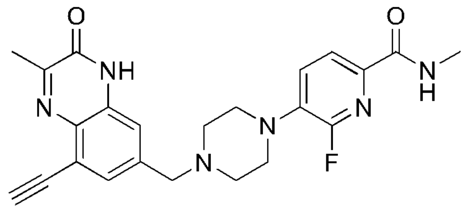
91,

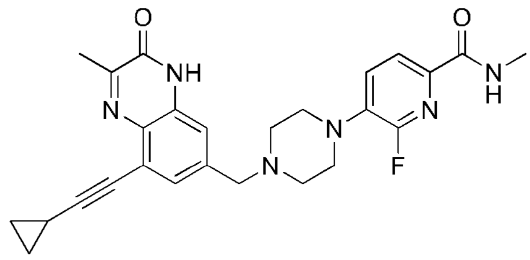
92,

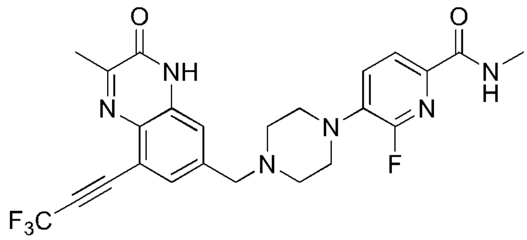
93,

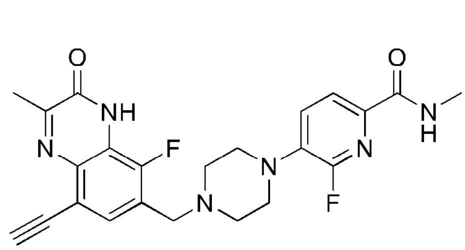
94,

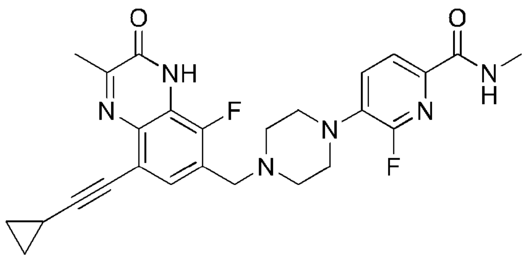
95,

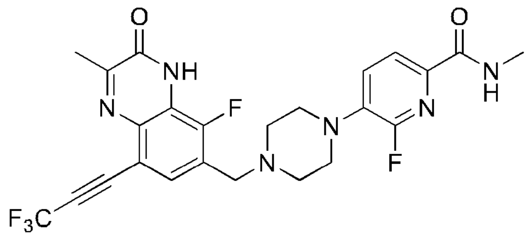
96,

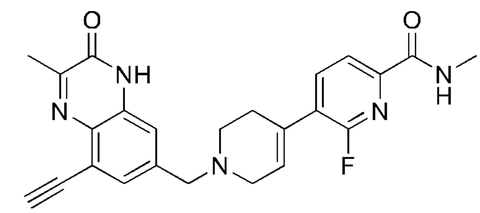
97,

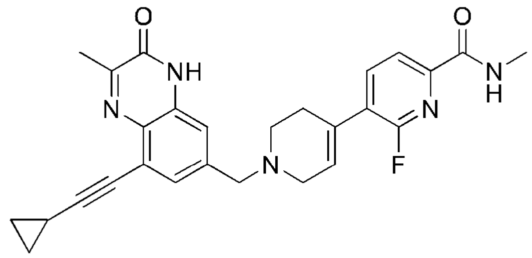 98, 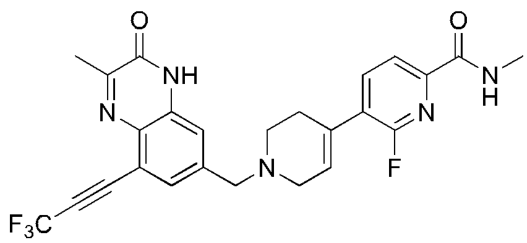 99,

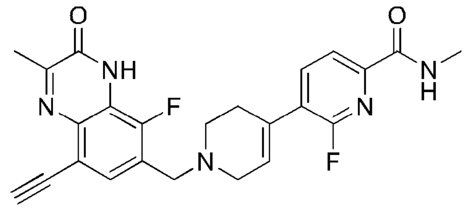 100, 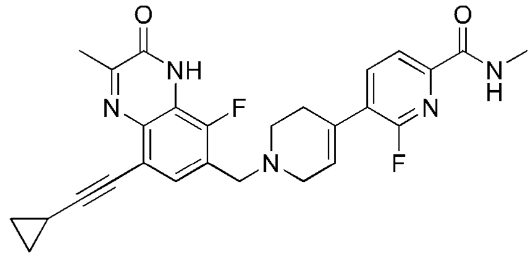 101,

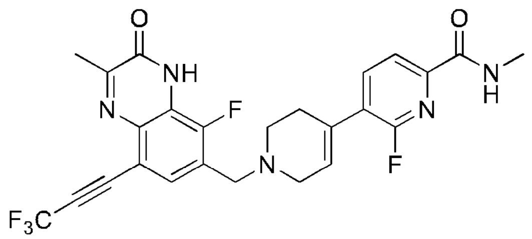 102, 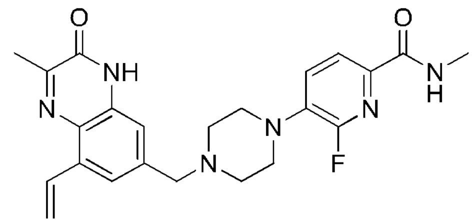 103,

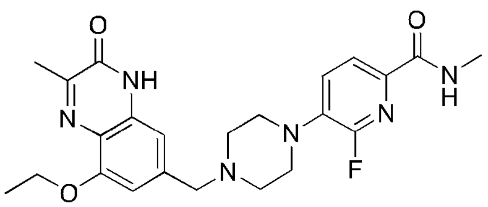 104, 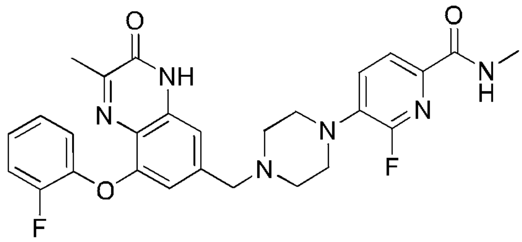 105,

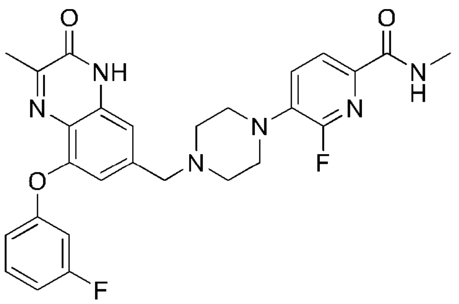 106, 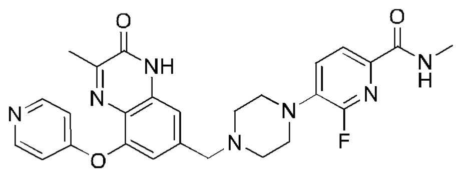 107,

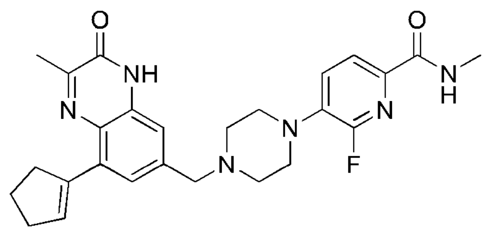 108, 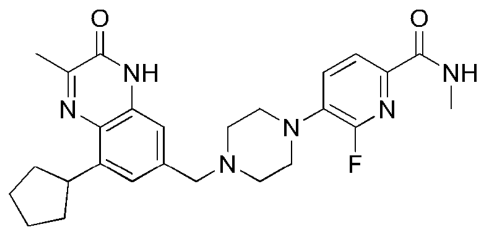 109,

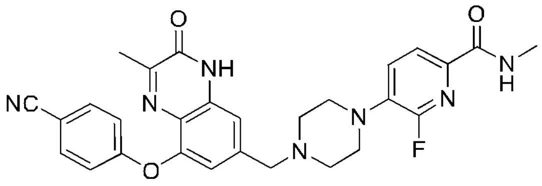 110, 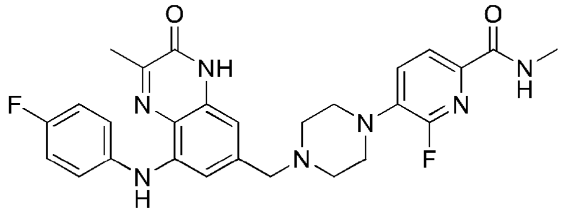 111,

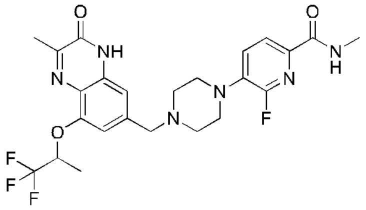 112, 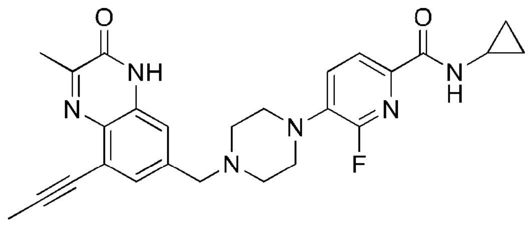 113,

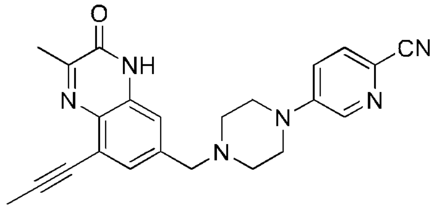 114, 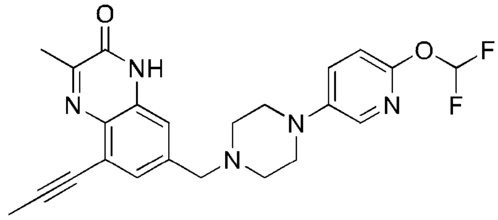 115,

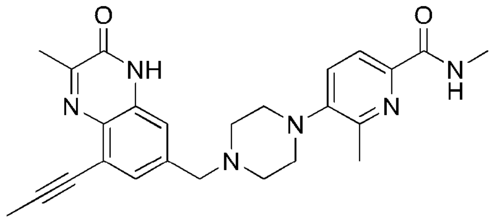 116, 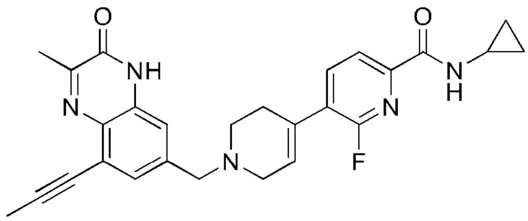 117,

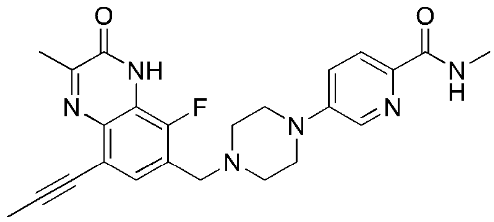 118, 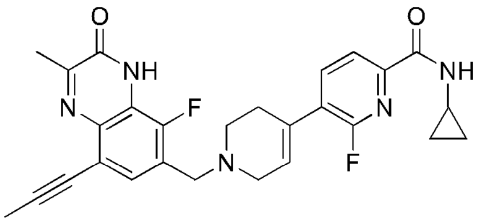 119,

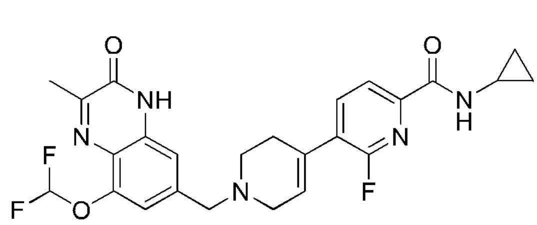 120, 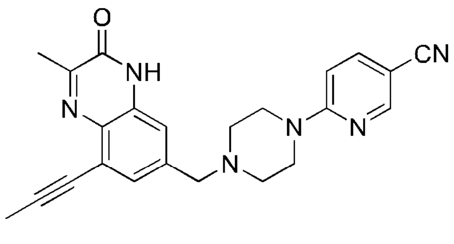 121,

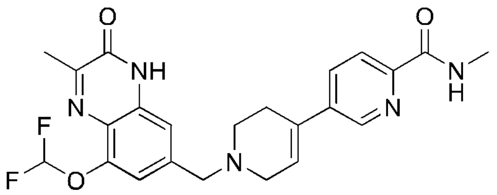

122,

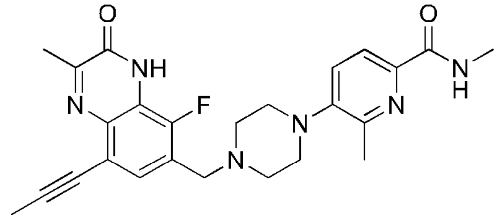

123,

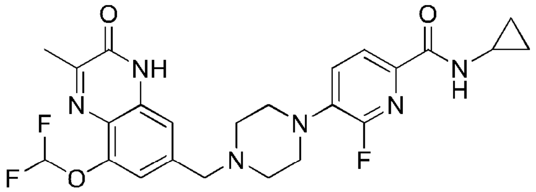

124,

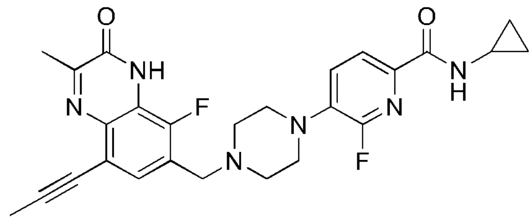

125,

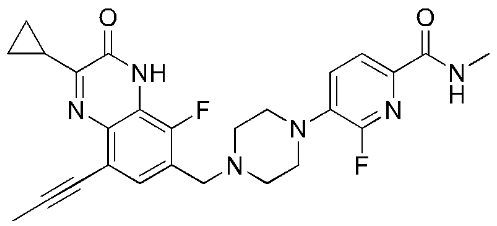

126,

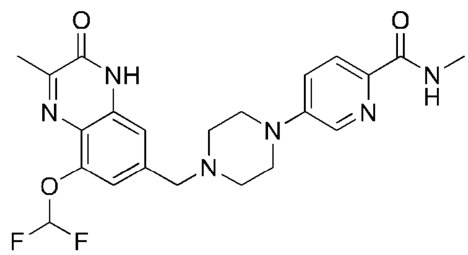

127,

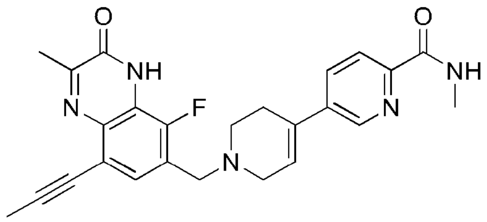

128,

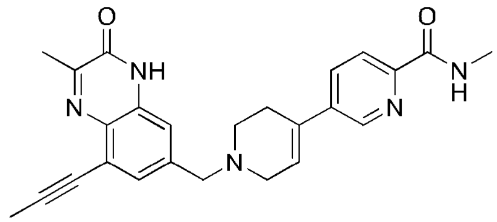

129,

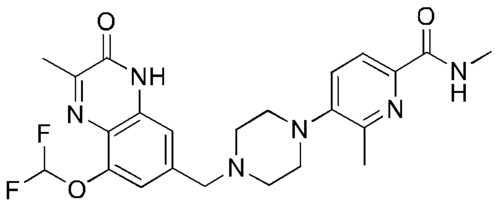

130,

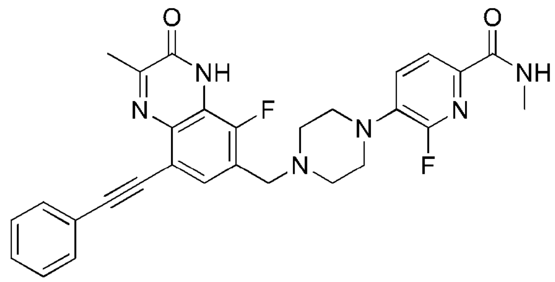

131,

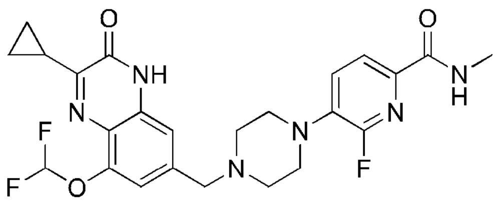

132,

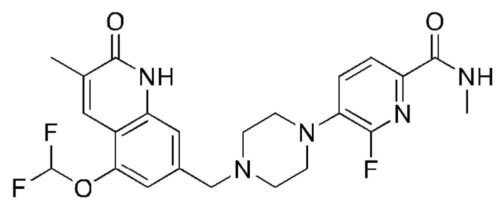

133,

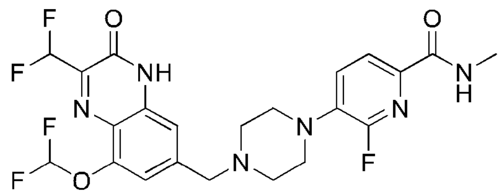 134, 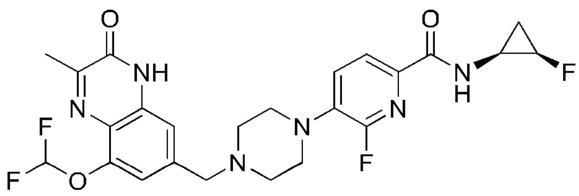 135,

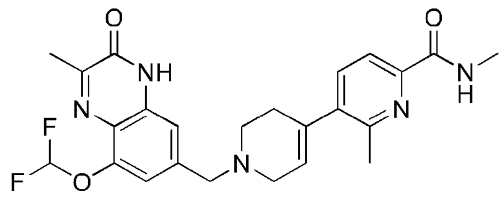 136, 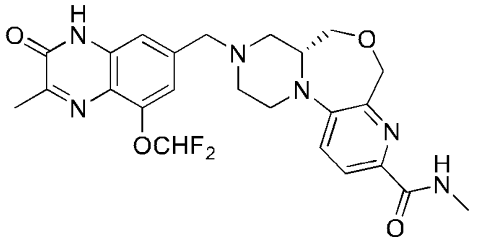 137,

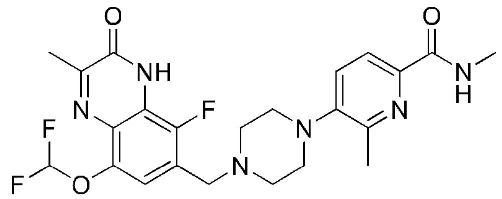 138, 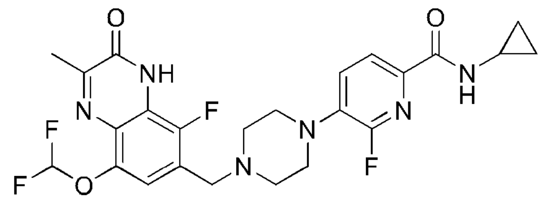 139,

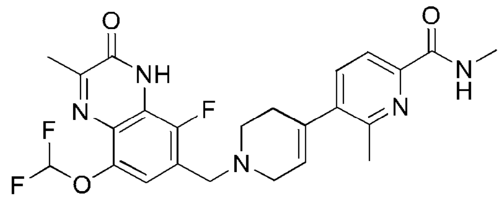 140, 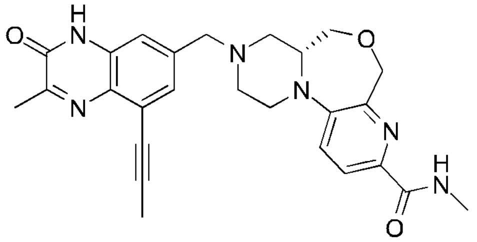 141,

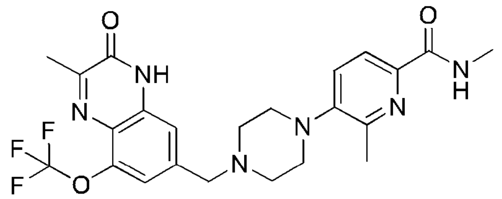 142, 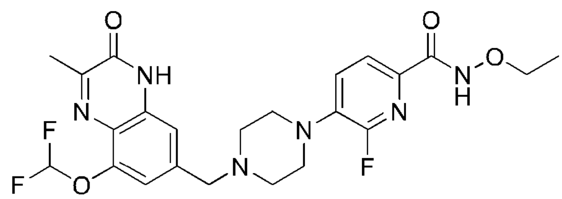 143,

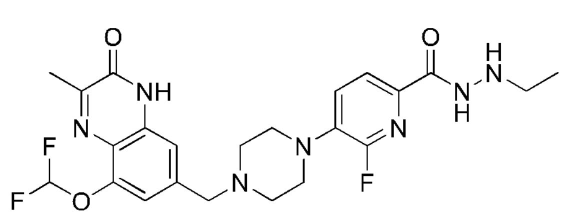 144, 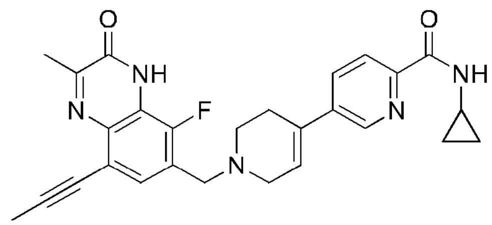 145,

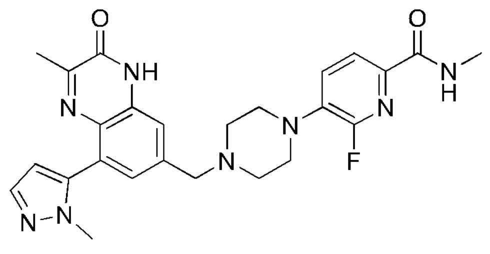
146,

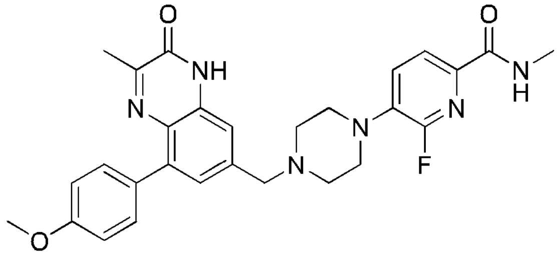
147,

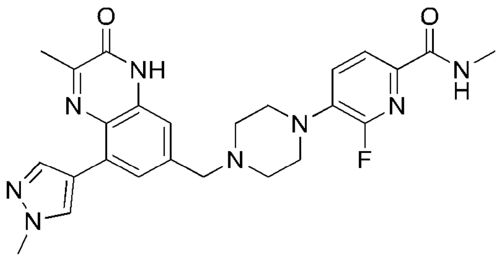
148,

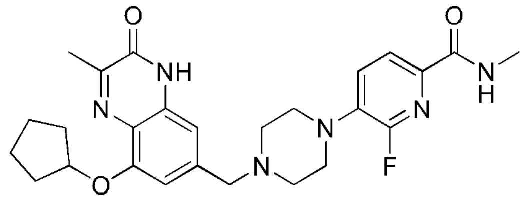
149,

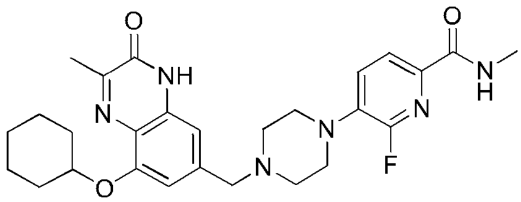
150,

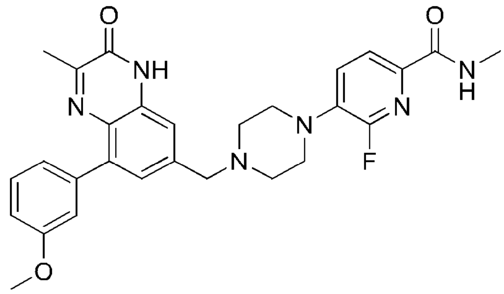
151,

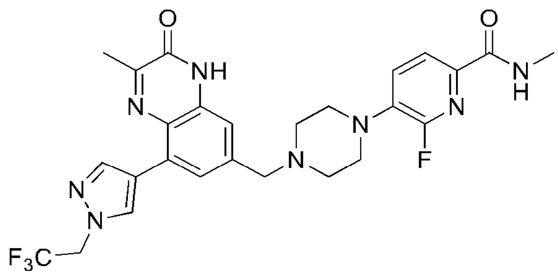
152,

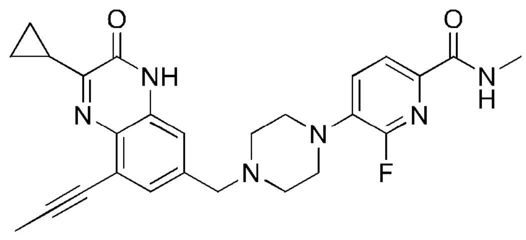
153 or

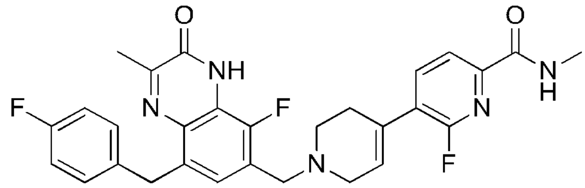
154.

In the other aspect, the present invention relates to a pharmaceutical composition comprising a compound of formula (I), (II) or (III) disclosed herein.

In one embodiment, the pharmaceutical composition of the present invention further comprises a pharmaceutically acceptable excipient, carrier, adjuvant or any combination thereof.

In yet another aspect, the present invention relates to the use of a compound of formula (I), (II), or (III) disclosed herein, or a pharmaceutical composition thereof, in the manufacture of a medicament for the prevention, treatment, or relief of PARP1 mediated diseases.

In one embodiment, the PARP1 mediated diseases are cancer, neurodegenerative diseases, cardiovascular diseases, ischemic diseases, diabetic neuropathy, reperfusion injury, osteoarthritis, osteoporosis, inflammatory diseases and metabolic diseases.

In one embodiment, the cancers mentioned are laryngeal cancer, esophageal cancer, gastric cancer, intestinal cancer, liver cancer, kidney cancer, lung cancer, brain cancer, head and neck cancer, squamous cell carcinoma, lymphatic system cancer, thyroid cancer, bladder cancer, ovarian cancer, cervical cancer, prostate cancer, genitourinary tract cancer, breast cancer, hematological cancer, small cell lung cancer, lung adenocarcinoma, pancreatic cancer, colon cancer, glioblastoma and/or monocytic leukemia.

In another embodiment, the PARP1 mediated disease mentioned is cancer.

In other aspect, this invention relates to the use of compounds of formula (I), (II) or (III) disclosed herein, or pharmaceutical compositions thereof, in the manufacture of a medicament for inhibiting PARP1.

On the other hand, this invention relates to methods for the preparation, isolation, and purification of compounds of formula (I), (II) or (III).

## PHARMACEUTICAL COMPOSITIONS, FORMULATIONS AND ADMINISTRATION OF THE COMPOUNDS OF THE PRESENT INVENTION_

This invention provides a pharmaceutical composition comprising a compound of formula (I), (II) or (III) or a stereoisomer thereof, a racemic or non-racemic mixture of the isomers, or a pharmaceutically acceptable salt or solvate thereof. In one embodiment of the invention, the pharmaceutical composition further comprises at least one pharmaceutically acceptable carrier, excipient, or excipient, and optionally, other therapeutic and/or preventative components.

The dosage form of the compound used in the method of this invention can be determined by the specific compound selected, the pharmacokinetic distribution type required by the route of administration, and the patient's condition.

Preparations suitable for oral, sublingual, intranasal, or injectable administration are prepared according to methods known in the pharmaceutical industry, and said preparations contain at least one active compound. Referring to, for example, REMINGTON'S PHARMACEUTICAL SCIENCES (16th ed. 1980).

Generally, the formulations of the present invention comprise an active ingredient (a compound represented by formula (I), (II) or (III)) and are typically mixed with, diluted by, or encapsulated in a carrier that may be in the form of capsules, pouches, paper or other containers. When the excipient is used as a diluent, it may be a solid, semi-solid, or liquid material, acting as an excipient, carrier, or medium for the active ingredient. Therefore, the formulations may be tablets, pills, powders, lozenges, sachets, capsules, elixirs, suspensions, emulsions, solutions, syrups, aerosols (in solid or liquid media), ointments containing, for example, up to 10% by weight of the active compound, soft and hard capsules, gels, suppositories, sterile injections, and sterile encapsulated powders.

In the preparation of formulations, the active compound may need to be ground to provide a suitable particle size before being mixed with other components. If the active compound is substantially insoluble, it is typically ground to a particle size of less than 200 mesh. If the active compound is substantially water-soluble, its particle size is adjusted by grinding to achieve a uniform particle size distribution in the formulation, for example, about 40 mesh. In one embodiment of the invention, the particle size is about 0.1-100 $\mu$m.

Suitable carriers, adjuvants, and excipients are well known to those skilled in the art and described in detail in, for example, Ansel H. C. et al., Ansel's Pharmaceutical Dosage Forms and Drug Delivery Systems (2004) Lippincott, Williams & Wilkins, Philadelphia; Gennaro A. R. et al., Remington: The Science and Practice of Pharmacy (2000) Lippincott, Williams & Wilkins, Philadelphia; and Rowe R. C., Handbook of Pharmaceutical Excipients (2005) Pharmaceutical Press, Chicago.

"Pharmaceutically acceptable excipient" as used herein means a pharmaceutically acceptable material, composition or vehicle involved in giving form or consistency to the pharmaceutical composition. Each excipient must be compatible with the other ingredients of the pharmaceutical composition when commingled, such that interactions which would substantially reduce the efficacy of the compound of the invention when administered to a patient and/or would result in pharmaceutically unacceptable compositions are avoided. In addition, each excipient must of course be of sufficiently high purity to render it is pharmaceutically acceptable.

Suitable pharmaceutically acceptable excipients will vary depending upon the particular dosage form chosen. In addition, suitable pharmaceutically acceptable excipients may be chosen for a particular function that they may serve in the composition. For example, certain pharmaceutically acceptable excipients may be chosen for their ability to facilitate the production of uniform dosage forms. Certain pharmaceutically acceptable excipients may be chosen for their ability to facilitate the production of stable dosage forms. Certain pharmaceutically acceptable excipients may be chosen for their ability to facilitate the carrying or transporting the compound of the present invention once administered to the patient from one organ, or portion of the body, to another organ, or portion of the body. Certain pharmaceutically acceptable excipients may be chosen for their ability to enhance patient compliance.

Examples of suitable excipients include lactose, glucose, sucrose, sorbitol, mannitol, starch, gum arabic, calcium phosphate, alginate, tragacanth gum, gelatin, calcium silicate, microcrystalline cellulose, polyvinylpyrrolidone, cellulose, water, syrup and methylcellulose. Suitable pharmaceutically acceptable excipients also include the following types:

diluents, fillers, binders, disintegrants, lubricants (such as talc, magnesium stearate, and mineral oil), glidants, granulators, coating agents, wetting agents, solvents, co-solvents, suspending agents, emulsifiers, sweeteners, flavoring agents, masking agents, colorants, anti-caking agents, humectants, chelating agents, plasticizers, thickeners, antioxidants, preservatives (such as methylparaben and propylparaben), stabilizers, surfactants and buffers. The skilled artisan will appreciate that certain pharmaceutically acceptable excipients may serve more than one function and may serve alternative functions depending on how much of the excipient is present in the formulation and what other ingredients are present in the formulation. The compounds of the present invention can be formulated using methods known in the art so as to enable rapid, sustained or delayed release of the active ingredient after administration to a patient.

Skilled artisans possess the knowledge and skill in the art to enable them to select suitable pharmaceutically acceptable excipients in appropriate amounts for use in the invention. In addition, there are a number of resources that are available to the skilled artisan which describe pharmaceutically acceptable excipients and may be useful in selecting suitable pharmaceutically acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

For the preparation of pharmaceutical compositions using the compounds described in this invention, a pharmaceutically acceptable carrier may be a solid or liquid carrier. Solid forms of formulations include powders, tablets, dispersible granules, capsules, pouches, and suppositories. Powders and tablets may contain from about 5% to about 95% of the active ingredient. Suitable solid carriers are known in the art, for example, magnesium carbonate, magnesium stearate, talc, sugar or lactose. Tablets, powders, pouches, and capsules can be used as solid dosage forms suitable for oral administration. Examples of pharmaceutically acceptable carriers and methods for preparing various compositions can be found in: A. Gennaro (ed.), Remington's Pharmaceutical Sciences, 18th ed., 1990, Mack Publishing Company Co., Easton, Pennsylvania.

in Remington: Various carriers for preparing pharmaceutically acceptable compositions and known techniques for their preparation are disclosed The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York, the contents of which are incorporated herein by reference. The use of carriers is within the scope of this invention, except for any commonly used carriers that are incompatible with the compounds of this invention, such as by producing any undesirable biological effects or by interacting in a harmful manner with any other component of a pharmaceutically acceptable composition.

The pharmaceutical compositions of the invention are prepared using techniques and methods known to those skilled in the art. Some of the methods commonly used in the art are described in Remington's Pharmaceutical Sciences (Mack Publishing Company).

Therefore, another aspect of the present invention is related to a method for preparing a pharmaceutical composition, the pharmaceutical composition contains the compound disclosed herein and pharmaceutically acceptable excipient, carrier, adjuvant, vehicle or a combination thereof, the method comprises mixing various ingredients. The pharmaceutical composition containing the compound disclosed herein can be prepared at for example environment temperature and under barometric pressure.

The compound of the invention will typically be formulated into a dosage form adapted for administration to the patient by the desired route of administration. For example, dosage forms include those adapted for (1) oral administration such as tablets, capsules, caplets, pills, troches, powders, syrups, elixers, suspensions, solutions, emulsions, sachets and cachets; (2) parenteral administration such as sterile solutions, suspensions, and powders for reconstitution; (3) transdermal administration such as transdermal patches; (4) rectal administration such as suppositories; (5) inhalation such as aerosols, solutions, and dry powders; and (6) topical administration such as creams, ointments, lotions, solutions, pastes, sprays, foams and gels.

It will also be appreciated that certain of the compounds of present invention can exist in free form for treatment, or where appropriate, as a pharmaceutically acceptable derivative or a prodrug thereof. According to the present invention, a pharmaceutically acceptable derivative or a prodrug includes, but is not limited to, pharmaceutically acceptable salts, esters, salts of such esters, or any other adduct or derivative which upon administration to a patient in need thereof is capable of providing, directly or indirectly, a compound as otherwise described herein, or a metabolite or residue thereof.

In one embodiment, the compounds disclosed herein can be prepared to oral. In the other embodiment, the compounds disclosed herein can be prepared to inhalation. In the still other embodiment, the compounds disclosed herein can be prepared to nasal administration. In the yet other embodiment, the compounds disclosed herein can be prepared to transdermal administration. In the still yet other embodiments, the compounds disclosed herein can be prepared to topical administration.

The pharmaceutical compositions provided herein may be provided as compressed tablets, tablet triturates, chewable lozenges, rapidly dissolving tablets, multiple compressed tablets, or enteric-coating tablets, sugar-coated or film-coated tablets. Enteric-coated tablets are compressed tablets coated with substances that resist the action of stomach acid but dissolve or disintegrate in the intestine, thus protecting the active ingredients from the acidic environment

of the stomach. Enteric-coatings include, but are not limited to, fatty acids, fats, phenylsalicylate, waxes, shellac, ammoniated shellac and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but are not limited to, hydroxyethylcellulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets.

The tablet dosage forms may be prepared from the active ingredient in powdered, crystalline, or granular forms, alone or in combination with one or more carriers or excipients described herein, including binders, disintegrants, controlled-release polymers, lubricants, diluents and/or colorants. Flavoring and sweetening agents are especially useful in the formation of chewable tablets and lozenges.

The pharmaceutical compositions provided herein may be provided as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol or a similar polyol. The soft gelatin shells may contain a preservative to prevent the growth of microorganisms. Suitable preservatives are those as described herein, including methyl-paraben and propyl-paraben and sorbic acid. The liquid, semisolid, and solid dosage forms provided herein may be encapsulated in a capsule. Suitable liquid and semisolid dosage forms include solutions and suspensions in propylene carbonate, vegetable oils or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient.

The pharmaceutical compositions provided herein may be provided in liquid and semisolid dosage forms, including emulsions, solutions, suspensions, elixirs and syrups. An emulsion is a two-phase system, in which one liquid is dispersed in the form of small globules throughout another liquid, which can be oil-in-water or water-in-oil. Emulsions may include a pharmaceutically acceptable non-aqueous liquids or solvent, emulsifying agent and preservative. Suspensions may include a pharmaceutically acceptable suspending agent and preservative. Aqueous alcoholic solutions may include a pharmaceutically acceptable acetal, such as a di(lower alkyl) acetal of a lower alkyl aldehyde, e.g., acetaldehyde diethyl acetal; and a water-miscible solvent having one or more hydroxy groups, such as propylene glycol and ethanol. Elixirs are clear, sweetened hydroalcoholic solutions. Syrups are concentrated aqueous solutions of a sugar, for example, sucrose solution, and may also contain a preservative. For a liquid dosage form, for example, a solution in a polyethylene glycol may be diluted with a sufficient quantity of a pharmaceutically acceptable liquid carrier, e.g., water, to be measured conveniently for administration.

In another aspect, the pharmaceutical composition of the invention is prepared to a dosage form adapted for administration to a patient by inhalation, for example as a dry powder, an aerosol, a suspension or a solution composition. In one embodiment, the invention is directed to a dosage form adapted for administration to a patient by inhalation as a dry powder. In one embodiment, the invention is directed to a dosage form adapted for administration to a patient by inhalation as a dry powder. Dry powder compositions for delivery to the lung by inhalation typically comprise a compound disclosed herein or a pharmaceutically acceptable salt thereof as a finely divided powder together with one or more pharmaceutically-acceptable excipients as finely divided powders. Pharmaceutically-acceptable excipients particularly suited for use in dry powders are known to those skilled in the art and include lactose, starch, mannitol, and mono-, di-, and polysaccharides. The finely divided powder may be prepared by, for example, micronisation and milling. Generally, the size-reduced (eg micronised) compound can be defined by a $D_{50}$ value of about 1 to about 10 microns (for example as measured using laser diffraction).

Pharmaceutical compositions adapted for transdermal administration may be presented as discrete patches intended to remain in intimate contact with the epidermis of the patient for a prolonged period of time. For example, the active ingredient may be delivered from the patch by iontophoresis as generally described in Pharmaceutical Research, 3(6), 318(1986).

Pharmaceutical compositions adapted for topical administration may be formulated as ointments, creams, suspensions, lotions, powders, solutions, pastes, gels, sprays, aerosols or oils. For example, Ointments, creams and gels may be formulated with an aqueous or oily base with the addition of suitable thickening and/or gelling agent and/or solvents. Such bases may thus, for example, include water and/or an oil such as liquid paraffin or a vegetable oil (such as arachis oil or castor oil), or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents.

The compounds disclosed herein can also be coupled to soluble polymers as targeted medicament carriers.

Such polymers may encompass polyvinylpyrrolidone, pyran copolymer, polyhydroxypropylmethacrylamidophenol, polyhydroxyethylaspartamidophenol or polyethylene oxide polylysine substituted by palmitoyl radicals. The compounds may furthermore be coupled to a class of biodegradable polymers which are suitable for achieving controlled release of a medicament, for example polylactic acid, poly-epsilon-caprolactone, polyhydroxybutyric acid, polyorthoesters, polyacetals, polydihydroxypyrans, polycyanoacrylates and crosslinked or amphipathic block copolymers of hydrogels.

The pharmaceutical compositions provided herein may be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial and subcutaneous administration.

The pharmaceutical compositions provided herein may be formulated in any dosage forms that are suitable for parenteral administration, including solutions, suspensions, emulsions, micelles, liposomes, microspheres, nanosystems and solid forms suitable for solutions or suspensions in liquid prior to injection. Such dosage forms can be prepared according to conventional methods known to those skilled in the art of pharmaceutical science (see, Remington: The Science and Practice of Pharmacy, supra).

The pharmaceutical compositions intended for parenteral administration may include one or more pharmaceutically acceptable carriers and excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH adjusting agents and inert gases.

The pharmaceutical composition provided by this invention can be administered via rectal suppositories. The drug is mixed with a suitable, non-irritating excipient (such as cocoa butter or glycerides synthesized from polyethylene glycol), which is solid at room temperature, and then liquefied or dissolved within the rectal lumen to release the drug. Due to individual differences, the severity of symptoms can vary considerably, and each drug has its unique therapeutic properties. Therefore, the precise method of administration, dosage form, and treatment plan for each individual should be determined by a licensed physician.

The pharmaceutical compositions provided herein may be formulated as immediate or modified release dosage forms, including delayed, sustained- pulsed-controlled, targeted and programmed-release forms.

While the compounds of the present invention can be administered directly without any formulation, they are typically taken in the form of pharmaceutical preparations containing pharmaceutically acceptable excipients and at least one active ingredient. These preparations can be administered via various routes, including oral, buccal, rectal, intranasal, transdermal, subcutaneous, intravenous, intramuscular and intranasal administration. Many of the compounds used in the methods of the present invention are effective as injectable and oral compositions.

For transdermal drug delivery, a transdermal delivery device ("patch") is required. Such a transdermal patch can be used to continuously or intermittently infuse a controlled amount of the compound of the present invention. The structure and application of transdermal patches for drug delivery are well known in the art. See, for example, US 5,023,252. Such patches can be formulated to release drugs continuously, pulsatilely, or on demand.

Compounds having formula (I), (II) or (III) or pharmaceutically acceptable salts thereof are typically administered orally in pharmaceutical formulations containing the active ingredient or a pharmaceutically acceptable salt or solvation thereof, or a solvation of a pharmaceutically acceptable salt, in a pharmaceutically acceptable dosage form. The form of administration depends on the disease to be treated and the patient, and the pharmaceutical composition may be administered at different doses.

Pharmaceutical formulations having compounds of formula (I), (II) or (III) described above may be prepared for oral administration, specifically in tablet or capsule form, and particularly relate to techniques aimed at providing drug release to the colon (Patel, M.M. Expert Opin. Drug Deliv. [Expert Opinion on Drug Delivery] 2011, 8(10), 1247-1258).

The pharmaceutical formulations having compounds represented by formulas (I), (II) or (III) described above can be conveniently administered in unit dosage forms and can be prepared by any method well known in the pharmaceutical field, such as that described in Remington's Pharmaceutical Sciences, 17th edition, Mack Publishing Company, Easton, PA. (1985). The term "unit dosage form" refers to a physically separated unit suitable for use as a unit dose for human patients and other mammals, each unit containing a predetermined amount of active ingredient calculated to produce the desired therapeutic effect and suitable pharmaceutically acceptable excipients as described above.

Pharmaceutical formulations suitable for oral administration may contain one or more physiologically compatible carriers and/or excipients and may be in solid or liquid form. Tablets and capsules may be prepared using fillers, binders, lubricants, and/or surfactants (such as sodium lauryl sulfate). Liquid compositions may contain conventional additives such as emulsifiers, suspending agents and/or preservatives. Liquid compositions may be encapsulated in, for example, gelatin to provide a unit dosage form. Solid oral dosage forms include tablets, two-stage hard-shell capsules, and soft elastic gelatin (SEG) capsules. Such two-stage hard-shell capsules may be prepared, for example, by filling a hydroxypropyl methylcellulose (HPMC) or gelatin shell with a compound having formula (I), (II) or (III).

Dry-shell formulations typically contain about 40% to 60% w/w gelatin, about 30% to 40% water, and about 20% to 30% plasticizers (such as glycerin, propylene glycol or sorbitol). Other materials, such as dyes, flavorings, preservatives, and opacifiers may also be present. Liquid-filling materials include solid drugs that have been dissolved, solubilized, or dispersed (using suspending agents such as polyethylene glycol 4000, hydrogenated castor oil, or beeswax) or liquid drugs in a combination of one or more mediators (such as glycols, polyols, vegetable oils, mineral oils, triglycerides, and surfactants).

As used herein, the term "therapeutic effective amount" refers to the total amount of all active components sufficient to produce a beneficial therapeutic effect. For example, it refers to the amount sufficient to treat, cure, or alleviate the symptoms of a disease when administered or brought into equilibrium in the body. The effective amount required for a particular treatment regimen depends on a variety of factors, including the disease being treated, the severity of the disease, the activity of the specific drug used, the route of administration, the clearance rate of the specific drug, the duration of treatment, concomitant medications, age, weight, sex, diet and the patient's overall health. A description of other factors to be considered in this field regarding "therapeutic effective amounts" can be found in Gilman et al., eds., Goodman And Gilman's: The Pharmacological Bases of Therapeutics, 8th ed., Pergamon Press, 1990; Remington's Pharmaceutical Sciences, 17th ed., Mack Publishing Company, Easton, Pa., 1990.

Oral formulations are preferred, particularly tablets or capsules, which can be formulated by methods known to those skilled in the art to provide a dose of the active compound in the range of 0.1 mg to 1000 mg.

In treating humans, a suitable daily dose of a compound having formula (I), (II) or (III) or a pharmaceutically acceptable salt thereof is about 0.0001 to 100 mg/kg body weight. However, it should be understood that the actual amount of compound administered will be determined by the attending physician based on relevant circumstances, including the disease being treated, the route of administration chosen, one or more compounds to be administered, the age, weight, and response of the specific patient, and the severity of the patient's symptoms. Therefore, the above dose range should not limit the scope of the invention in any way. In some cases, dose levels below the lower limit of the above dose range may be more appropriate, while in other cases, higher doses may be used without producing any side effects, provided that such a larger dose is first divided into several smaller doses for administration throughout the day.

The term "drug administration" refers to providing an individual with a therapeutically effective amount of a drug. Routes of administration include oral, sublingual, intravenous, subcutaneous, transdermal, intramuscular, intradermal, intrathecal, epidural, intraocular, intracranial, inhalation, rectal, and vaginal administration. Dosage forms include ointments, lotions, tablets, capsules, pills, powders, granules, suppositories, pills, lozenges, injections, sterile solutions or non-aqueous solutions, suspensions, emulsions, and patches. The active ingredient is compounded with a non-toxic, pharmaceutically acceptable carrier (such as glucose, lactose, gum arabic, gelatin, mannitol, starch paste, magnesium trisilicate, talc, corn starch, keratin, silica gel, potato starch, urea, dextran, etc.).

Preferred routes of administration vary depending on clinical characteristics, and dosage variations must be based on the individual patient's condition. The physician will determine the appropriate dosage on a patient-by-patient basis. The effective therapeutic dose per unit dose depends on body weight, physiological function, and the chosen vaccination regimen. Each unit dose of the compound refers to the weight of the compound at each administration, excluding the weight of the carrier (which is contained within the drug).

The pharmaceutical compositions provided herein may be formulated for single or multiple dosage administration. The single dosage formulations are packaged in an ampoule, a vial or a syringe. The multiple dosage parenteral formulations must contain an antimicrobial agent at bacteriostatic or fungistatic concentrations. All parenteral formulations must be sterile, as known and practiced in the art.

The pharmaceutical compositions provided herein may be co-formulated with other active ingredients which do not impair the desired therapeutic action, or with substances that supplement the desired action.

In one embodiment, the therapeutic methods disclosed herein comprise administrating to a patient in need of the treatment a safe and effective amount of the compound of the invention or the pharmaceutical composition containing the compound of the invention. Various embodiments of the present invention include treating the diseases mentioned in the present invention by administering a safe and effective amount of the compound of the present invention or a pharmaceutical composition comprising the compound of the present invention to a patient in need.

In one embodiment, the compound of the invention or the pharmaceutical composition thereof may be administered by any suitable route of administration, including both systemic administration and topical administration. Systemic administration includes oral administration, parenteral administration, transdermal administration and rectal administration. Parenteral administration refers to routes of administration other than enteral or transdermal, and is typically by injection or infusion. Parenteral administration includes intravenous, intramuscular, and subcutaneous injection or infusion. Topical administration includes application to the skin as well as intraocular, otic, intravaginal, inhaled and intranasal administration. In one embodiment, the compound of the invention or the pharmaceutical composition thereof may be administered orally. In another embodiment, the compound of the invention or the pharmaceutical composition thereof may be administered by inhalation. In another embodiment, the compound of the present invention or a pharmaceutical composition comprising the compound of the present invention may be administered intranasally.

In one embodiment, the compound of the invention or the pharmaceutical composition thereof may be administered once or according to a dosing regimen wherein a number of doses are administered at varying intervals of time for a given period of time. For example, doses may be administered one, two, three or four times per day. In one embodiment, a dose is administered once per day. In a further embodiment, a dose is administered twice per day. Doses may be administered until the desired therapeutic effect is achieved or indefinitely to maintain the desired therapeutic effect. Suitable dosing regimens for the compound of the invention or the pharmaceutical composition thereof depend on the pharmacokinetic properties of that compound, such as absorption, distribution, and half-life, which can be determined by the skilled artisan. In addition, suitable dosing regimens, including the duration such regimens are administered, for the compound of the invention or the pharmaceutical composition thereof depend on the disorder being treated, the severity of the disorder being treated, the age and physical condition of the patient being treated, the medical history of the patient to be treated, the nature of concurrent therapy, the desired therapeutic effect, and like factors within the knowledge and expertise of the skilled artisan. It will be further understood by such skilled artisans that suitable dosing regimens may require adjustment given an individual patient's response to the dosing regimen or over time as individual patient needs change.

The compounds of the present invention may be administered either simultaneously with, or before or after, one or more other therapeutic agents. The compounds of the present invention may be administered separately, by the same or different route of administration, or together in the same pharmaceutical composition as the other agents. This is selected by those skilled in the art based on the patient's health, age, weight, and other physical conditions. If formulated as a fixed dose, such a combination product uses the compounds of the present invention (within the dosage range described herein) and other pharmaceutically active agents (within their dosage range).

Accordingly, in one aspect, the present invention includes a combination of medications comprising a certain amount of at least one compound of the present invention or a pharmaceutically acceptable salt, solvate, ester, or prodrug thereof, and an effective amount of one or more of the aforementioned additional therapeutic agents.

Additionally, the compounds of the invention may be administered as prodrugs. As used herein, a "prodrug" of a compound of the invention is a functional derivative of the compound which, upon administration to a patient, eventually liberates the compound of the invention in vivo. Administration of a compound of the invention as a prodrug may enable the skilled artisan to do one or more of the following: (a) modify the onset of action of the compound in vivo; (b) modify the duration of action of the compound in vivo; (c) modify the transportation or distribution of the compound in vivo; (d) modify the solubility of the compound in vivo; and (e) overcome a side effect or other difficulty encountered with the compound. Typical functional derivatives used to prepare prodrugs include modifications of the compound that are chemically or enzymatically cleaved in vivo. Such modifications, which include the preparation of phosphates, amides, esters, thioesters, carbonates, and carbamates, are well known to those skilled in the art.

## USE OF THE COMPOUNDS AND PHARMACEUTICAL COMPOSITIONS

The compounds and pharmaceutical compositions provided by this invention can be used to prepare medicaments for inhibiting PARP1 and/or PARP2, and also for the prevention, treatment or relief of PARP-mediated diseases, particularly cancer.

Specifically, the amount of the compound or pharmaceutical composition of this invention can effectively and detectably selectively inhibit PARP1 and/or PARP2.

The compounds of this invention can be used, but are not limited to, administering effective amounts of the compounds or pharmaceutical compositions of this invention to patients to prevent, treat, or relieve PARP-mediated diseases. These PARP-mediated diseases further include, but are not limited to, cancer, neurodegenerative diseases, cardiovascular diseases, ischemic diseases, diabetic neuropathy, reperfusion injury, osteoarthritis, osteoporosis, inflammatory diseases and metabolic diseases.

The compounds of the present invention can be used, but are not limited to, administering an effective amount of the compounds or pharmaceutical compositions of the present invention to a patient to prevent, treat or alleviate cancer. The cancers further include, but are not limited to, laryngeal cancer, esophageal cancer, gastric cancer, intestinal cancer, liver cancer, kidney cancer, lung cancer, brain cancer, head and neck cancer, squamous cell carcinoma, lymphatic system cancer, thyroid cancer, bladder cancer, ovarian cancer, cervical cancer, prostate cancer, genitourinary tract cancer, breast cancer, hematologic malignancies, small cell lung cancer, lung adenocarcinoma, pancreatic cancer, colon cancer, glioblastoma and/or monocytic leukemia.

The compounds of the present invention can be used, but are not limited to, administering an effective amount of the compounds or pharmaceutical compositions of the present invention to a patient to prevent, treat or alleviate neurodegenerative diseases. The neurodegenerative diseases further include, but are not limited to, stroke, epilepsy, Parkinson's disease, Huntington's disease, schizophrenia, Alzheimer's disease, amyotrophic lateral sclerosis (ALS), neuropathic pain, chronic or acute pain, ischemic brain injury, neuronal loss following hypoxia, trauma and nerve damage.

The compounds of this invention can be applied, but are not limited to, administering effective amounts of the compounds or pharmaceutical compositions of this invention to patients to prevent, treat or alleviate cardiovascular diseases. These cardiovascular diseases further include, but are not limited to, angina pectoris, myocardial infarction, cardiogenic shock, arteriosclerosis, coronary artery disease, cardiovascular tissue damage and hyperlipidemia.

In addition to their therapeutic benefits in humans, the compounds and pharmaceutical compositions of this invention can also be used in veterinary treatment of mammals, including pets, introduced breeds of animals and farm animals. In other embodiments, the animals disclosed herein include horses, dogs and cats. As used herein, the compounds disclosed herein include the pharmaceutically acceptable derivatives thereof.

## General Synthesis steps:

Examples are listed below to describe the present invention. However, it should be understood that the present invention is not limited to these examples, but merely provides a method for practicing the present invention.

Generally, the compounds disclosed herein may be prepared by methods described herein, wherein the substituents are as defined for Formula (I), (II) or (III) above, except where further noted. The following nonlimiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

In the examples described below, unless otherwise indicated all temperatures are set forth in degrees Celsius. Reagents were purchased from commercial suppliers such as Aldrich Chemical Company, Arco Chemical Company and Alfa Chemical Company, and were used without further purification unless otherwise indicated. Common solvents were purchased from commercial suppliers such as Shantou XiLong Chemical Factory, Guangdong Guanghua Reagent Chemical Factory Co. Ltd., Guangzhou Reagent Chemical Factory, Tianjin YuYu Fine Chemical Ltd., Tianjin Fuchen Chemical Reagent Factory, Wuhan Xinhuayuan Technology Development Co., Ltd., Qingdao Tenglong Reagent Chemical Ltd., and Qingdao Ocean Chemical Factory.

Anhydrous THF, dioxane, toluene, and ether were obtained by refluxing the solvent with sodium. Anhydrous dichloromethane and chloroform were obtained by refluxing the solvent with calcium hydride. Ethyl acetate, petroleum ether, n-hexane, N,N-dimethylacetamide, and N,N-dimethylformamide are used after being dried with anhydrous sodium sulfate.

The reactions set forth below were done generally under a positive pressure of nitrogen or argon or with a drying tube (unless otherwise stated) in anhydrous solvents, and the reaction flasks were typically fitted with rubber septa for the introduction of substrates and reagents via syringe. Glassware was oven dried and/or heat dried. Glassware was oven dried and/or heat dried.

Column chromatography was conducted using a silica gel column. Silica gel (300-400 mesh) was purchased from Qingdao Ocean Chemical Factory.

$^1$H NMR spectra were recorded using a Bruker 400 MHz or 600 MHz NMR spectrometer. $^1$H NMR spectra were obtained using $CDCl_3$, DMSO-$d_6$, $CD_3OD$ or acetone-$d_6$ as solvents (in ppm), with TMS (0 ppm) or chloroform (7.26 ppm) as reference standards. When multiple peaks are observed, the following abbreviations are used: s (single), d (doublet), t (triplet), q (quartet), m (multiplet), br (broadened), brs (broadened singlet), dd (doublet of doublets), ddd (doublet of doublets), dt (doublet of triplets), td (triplet of doublets), tt (triplet of triplets). The coupling constant J is expressed in Hertz (Hz).

The determination conditions for low-resolution mass spectrometry (MS) data were as follows: Agilent 6120 quadrupole HPLC-M (column model: Zorbax SB-C18, 2.1 x 30 mm, 3.5 μm, 6 min, flow rate 0.6 mL/min. mobile phase: 5%-95% ($CH_3CN$ containing 0.1% formic acid) in ($H_2O$ containing 0.1% formic acid), electrospray ionization (ESI) at 210 nm/254 nm, detection by UV.

Pure compounds were detected using UV at 210 nm/254 nm using an Agilent 1260 pre-HPLC or a Calesep pump 250 pre-HPLC (column model: NOVASEP 50/80 mm DAC).

The following abbreviations are used throughout the specification:

| | | | |
|---|---|---|---|
| $CH_2Cl_2$, DCM | DCM | mg | milligram |
| $CDCl_3$ | deuterated chloroform | g | gram |
| DMSO | Dimethyl sulfoxide | mL, ml | milliliter |

(continued)

| | | | |
|---|---|---|---|
| DMSO-$d_6$ | Deuterated dimethyl sulfoxide | μL, μl | microlitre |
| EtOAc, EA | ethyl acetate | nL, nl | nanoliter |
| $CH_3OH$, MeOH | methanol | min | minute, minutes |
| $CD_3OD$ | Deuterated methanol | h | hour, hours |
| nM | nanomole | PE | petroleum ether (60 - 90 °C) |
| μM | Micromole | RT, rt, r.t. | room temperature |
| mmol, mM | millimole | EDTA-$K_2$ | Dipotassium ethylenediaminete-traacetate |
| M | moles per liter | PEG400 | polyethylene glycol 400 |
| ng | Nanogram | MeCN, ACN | Acetonitrile |
| μg | microgram | DMF | *N,N*- dimethylformamide |
| Boc, BOC | *tert*-butoxycarbonyl | THF | tetrahydrofuran |
| MTBE | Methyl *tert*-Butyl ether | DIEA, DIPEA | *N,N*- diisopropylethylamine |
| DDQ | 2,3-Dichloro-5,6-dicyanobenzoquinone | NBS | *N*-Bromosuccinimide |
| LAH, $LiAlH_4$ | Lithium aluminum hydride | $PBr_3$ | Phosphorus tribromide |
| AcOH | Acetic acid | $PPh_3$ | Triphenylphosphine |
| $Pd(PPh_3)_4$ | Tetraphenylphosphine palla-dium | $CBr_4$ | Carbon tetrabromide |
| $K_2CO_3$ | Potassium carbonate | NaCl | Sodium chloride |
| $Na_2SO_4$ | Sodium sulfate | $Fe(NO_3)_3 \cdot 9H_2O$ | Ferric nitrate nonahydrate |
| Fe | Iron powder | $NH_4Cl$ | Ammonium chloride |
| $NaHCO_3$ | Sodium bicarbonate | DEAD | diethyl azodicarboxylate |
| TBAF | Tetrabutylammonium fluoride | $K_3PO_4$ | Potassium phosphate |
| HCl | hydrogen chloride | $H_2O$ | Water |
| $Cs_2CO_3$ | Cesium carbonate | KI | Potassium iodide |
| TLC | Thin-layer chromatography | NaOH | Sodium hydroxide |
| NaH | Sodium hydride | Diox | 1,4-Dioxane |
| Dess-Martin periodinane | (1,1,1-Triacetoxy)-1,1-dihydro-1,2-benziodoxol-3(1*H*)-one | | |
| $Pd(dppf)Cl_2$ | [1,1'-Bis(diphenylphosphino)ferrocene]palladium dichloride | | |
| $Pd(dppf)Cl_2$ | Chloro(2-dicyclohexylphosphino-2',4',6'-triisopropyl-1,1'-biphenyl)[2-(2'-amino-1,1'-biphenyl)] palladium(II) | | |
| Xphos | 2-Dicyclohexylphosphino-2',4',6'-triisopropylbiphenyl | | |
| Ruphos Pd G3 | Methanesulfonic acid (2-dicyclohexylphosphino-2',6'-diisopropoxy-1,1'-biphenyl)(2-amino-1,1'-biphenyl-2-yl)palladium(II) | | |
| HATU | *N,N,N',N'*-Tetramethyl-O-(7-azabenzotriazol-1-yl)hexafluorophosphate | | |

The following synthetic schemes describe the steps for preparing the compounds disclosed in this invention, wherein each of $R^1$, $R^2$, $R^3$ and $R^4$ has the definition described in this invention, unless otherwise stated.

**Synthesis Scheme one**

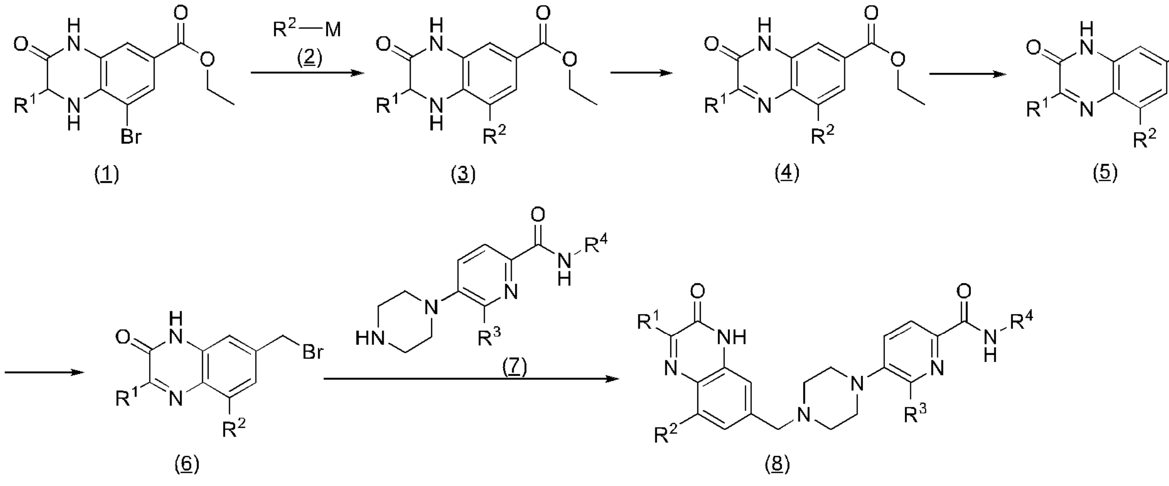


(1) (3) (4) (5) (6) (8)

M represents a leaving group, such as -I, -Br, -Cl, -OH, -OMs, -OTs,

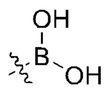 or 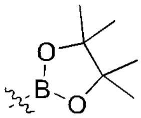

etc.

The compound shown in formula (8) can be prepared by Synthesis Scheme 1: The compound shown in formula (1) and the compound shown in formula (2) undergo a coupling reaction to obtain the compound shown in formula (3); the compound shown in formula (3) undergoes an oxidizing agent to obtain the compound shown in formula (4); the ester group of the compound shown in formula (4) is reduced to obtain the compound shown in formula (5); the hydroxyl group of the compound shown in formula (5) undergoes a bromination reaction to obtain the compound shown in formula (6); the compound shown in formula (6) and the compound shown in formula (7) undergo a substitution reaction to obtain the compound shown in formula (8).

**Synthesis Scheme two**

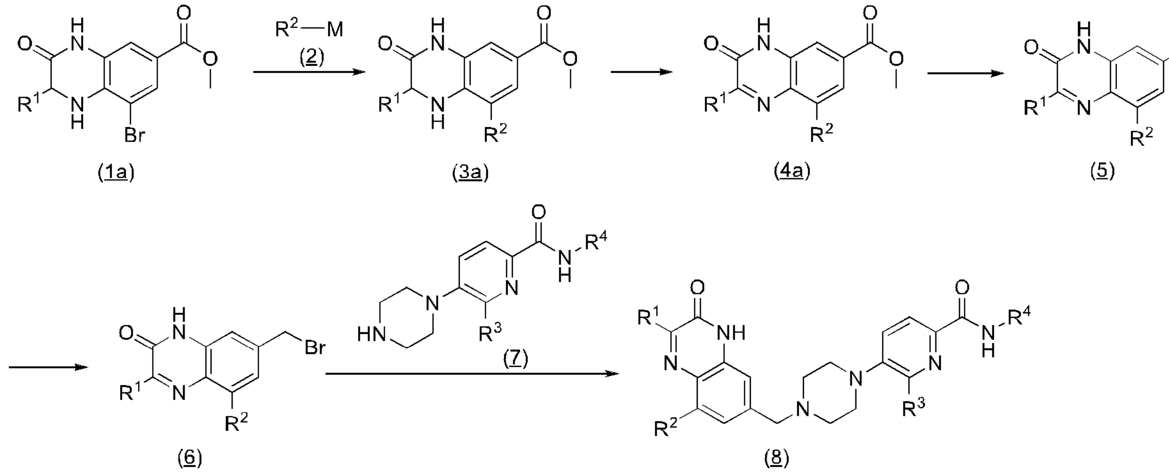


(1a) (3a) (4a) (5) (6) (8)

M represents a leaving group, such as -1, -Br, -Cl, -OH, -OMs, -OTs,

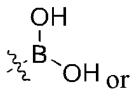
or
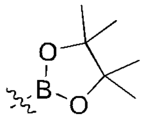

etc.

The compound shown in formula (8) can be prepared by synthetic scheme two: the compound shown in formula (1a) and the compound shown in formula (2) undergo a coupling reaction to obtain the compound shown in formula (3a); the compound shown in formula (3a) is reacted with an oxidizing agent to obtain the compound shown in formula (4a); the ester group of the compound shown in formula (4a) is reduced to obtain the compound shown in formula (5); the hydroxyl group of the compound shown in formula (5) undergoes a bromination reaction to obtain the compound shown in formula (6); the compound shown in formula (6) and the compound shown in formula (7) undergo a substitution reaction to obtain the compound shown in formula (8).

**Synthesis Scheme three**

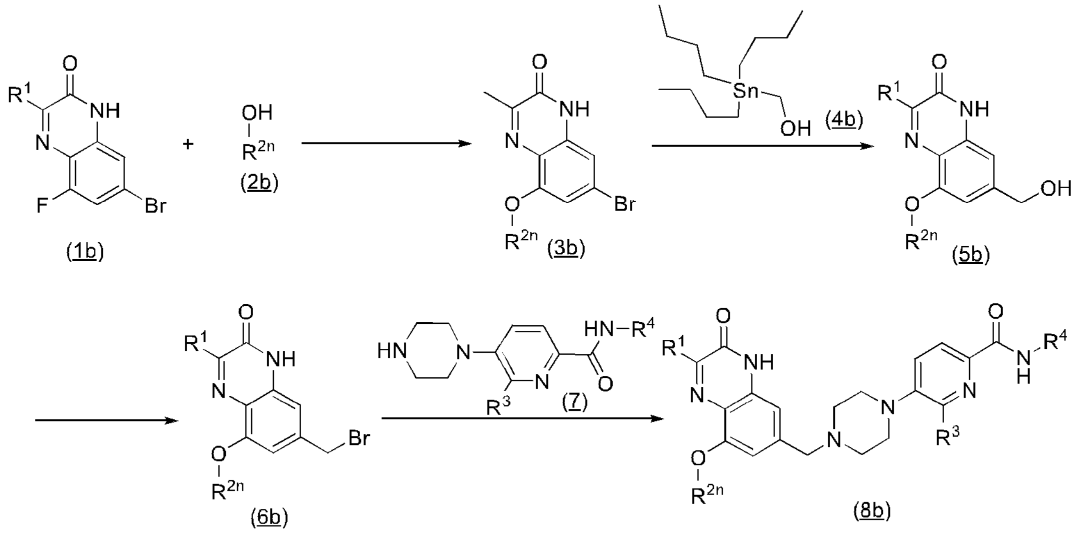


$R^{2n}$ has the definition as described in $R^2$ of this invention, such as $C_1$-$C_6$ alkyl, etc.

The compound shown in formula (8b) can be prepared by synthetic scheme three: the compound shown in formula (1b) and the compound shown in formula (2b) undergo a substitution reaction to obtain the compound shown in formula (3b); the compound shown in formula (3b) and the compound shown in formula (4b) undergo a coupling reaction to obtain the compound shown in formula (5b); the hydroxyl group of the compound shown in formula (5b) undergoes a bromination reaction to obtain the compound shown in formula (6b); the compound shown in formula (6b) and the compound shown in formula (7) undergo a substitution reaction to obtain the compound shown in formula (8b).

**Synthesis Scheme four**

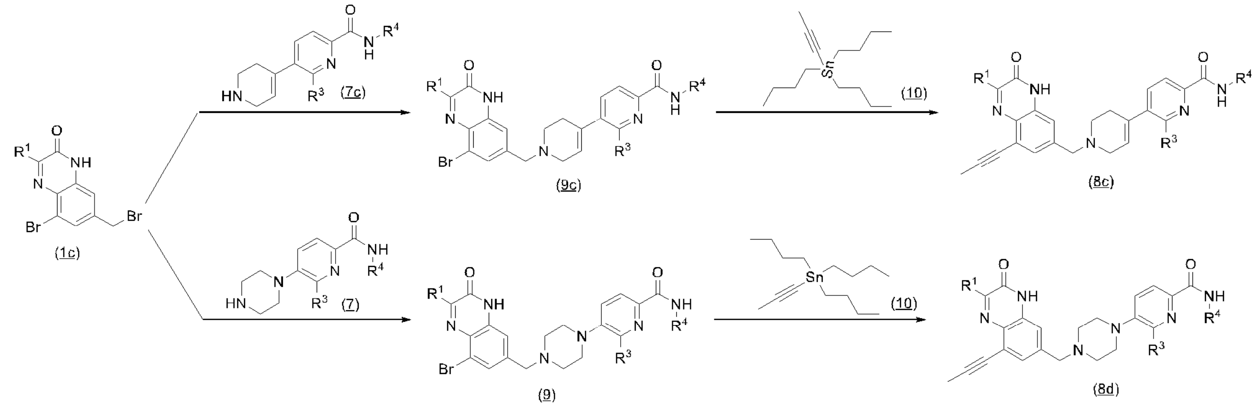


The compound shown in formula (8c) can be prepared by synthetic scheme four: the compound shown in formula (1c) and the compound shown in formula (7c) undergo a substitution reaction to obtain the compound shown in formula (9c); the compound shown in formula (9c) and the compound shown in formula (10) undergo a coupling reaction to obtain the compound shown in formula (8c).

The compound shown in formula (8d) can be prepared by synthetic scheme four: the compound shown in formula (1c) and the compound shown in formula (7) undergo a substitution reaction to obtain the compound shown in formula (9); the compound shown in formula (9) and the compound shown in formula (10) undergo a coupling reaction to obtain the compound shown in formula (8d).

The following examples further illustrate the compounds, pharmaceutical compositions and their applications provided by the present invention.

**Intermediate 1: 6-fluoro-*N*-methyl-5-(piperazin-1-yl)picolinamide hydrochloride**

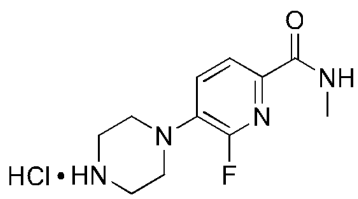

was prepared by the synthesis method of intermediate 23 in Example 7 of patent application WO2021013735.

**Example**

**Example 1: Synthesis of 5-(4-((8-cyclopropyl-2-methyl-3-oxo-3,4-dihydroquinoxalin-6-yl)methyl)piperazin-1-yl)-6-fluoro-*N*-methylpicolinamide**

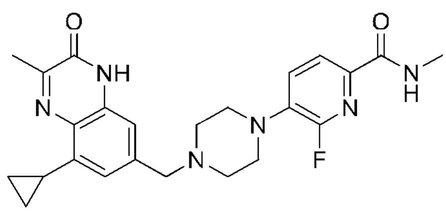

(1)

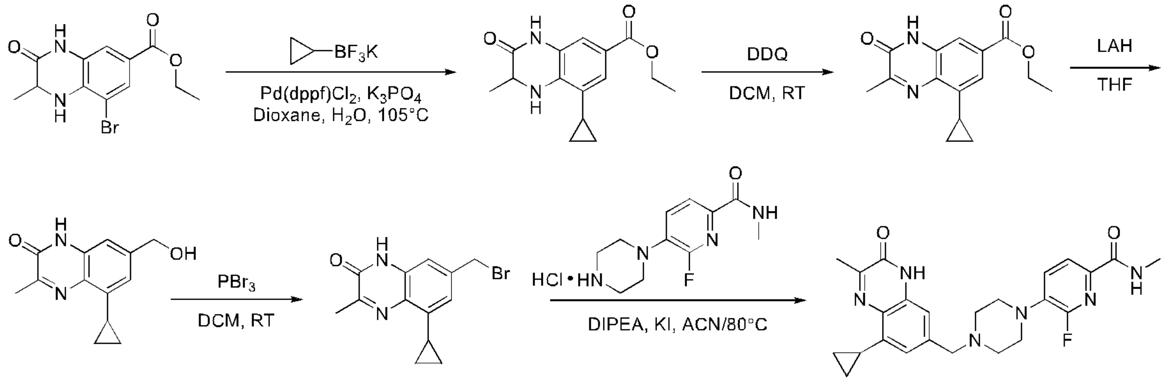


Step 1) Synthesis of ethyl 8-cyclopropyl-2-methyl-3-oxo-1,2,3,4-tetrahydroquinoxalin-6-carboxylate

ethyl 8-bromo-2-methyl-3-oxo-1,2,3,4-tetrahydroquinoxalin-6-carboxylate (0.80 g, 2.55 mmol) and potassium cyclopropyltrifluoroborate (0.75 g, 5.1 mmol) were added sequentially to a flask and stirred to dissolve in 1,4-dioxane (20 mL). Then, an aqueous solution (4 mL) of potassium phosphate (1.62 g, 7.65 mmol) was added and stirred until homogeneous. Finally, Pd(dppf)$Cl_2$ (0.19 g, 0.26 mmol) was added, the mixture was purged with nitrogen, and the reaction was heated to 105 °C for 12 h. Cool to room temperature, quench with water (15 mL), extract with EtOAc (15 mL × 3), combine organic phases, wash with saturated brine (20 mL), dry with anhydrous sodium sulfate, concentrate under reduced pressure, and purify the residue by silica gel column chromatography (petroleum ether/ethyl acetate (V/V) = 3/2) to give a pale yellow oily product (0.655 g, 93.46%).
MS (ESI, pos. ion) *m/z:* 275.20 $[M+H]^+$.

Step 2) Synthesis of ethyl 8-cyclopropyl-2-methyl-3-oxo-3,4-dihydroquinoxalin-6-carboxylate

Ethyl 8-cyclopropyl-2-methyl-3-oxo-1,2,3,4-tetrahydroquinoxalin-6-carboxylate (0.45 g, 1.64 mmol) was added to a flask and stirred to dissolve in DCM (9 mL). After cooling, DDQ (0.41 g, 1.80 mmol) was added under ice bath conditions, and the mixture was allowed to react overnight at room temperature. The solvent was removed by concentration under reduced pressure, and the solvent was quenched by adding saturated sodium bicarbonate aqueous solution (18 mL). A solid precipitated out. The mixture was stirred for 30 min, filtered, and the filter cake was collected. The cake was washed with saturated sodium bicarbonate aqueous solution and dried under vacuum to obtain a grayish-white solid (0.345 g, 77.24%).
MS (ESI, pos. ion) *m/z:* 273.20 $[M+H]^+$.

Step 3) Synthesis of 5-cyclopropyl-7-(hydroxymethyl)-3-methylquinoxalin-2(1*H*)-one

Ethyl 8-cyclopropyl-2-methyl-3-oxo-3,4-dihydroquinoxalin-6-carboxylate (0.341 g, 1.25 mmol) and THF (12.5 mL) were added sequentially to a 100 mL flask. After cooling, LAH (0.098 g, 2.5 mmol) was added in portions under ice bath conditions. The mixture was stirred until no further exothermic reaction occurred, and the reaction was allowed to continue at room temperature for 4 h. Cool, quench with water (2.5 mL) in an ice bath, adjust pH to 3-4 with dilute hydrochloric acid (1 M), extract with THF (10 mL), wash with saturated brine (20 mL), dry with anhydrous sodium sulfate, concentrate under reduced pressure, and purify the residue by silica gel column chromatography (DCM/MeOH (V/V) = 9/1) to give a white solid (0.18 g, 62.42%).
MS (ESI, pos. ion) *m/z:* 231.2 $[M+H]^+$.

Step 4) Synthesis of 7-(bromomethyl)-5-cyclopropyl-3-methylquinoxalin-2(1*H*)-one

5-cyclopropyl-7-(hydroxymethyl)-3-methylquinoxalin-2(1*H*)-one (0.179 g, 0.78 mmol) and DCM (10 mL) were added to a flask. The mixture was cooled, and phosphorus tribromide (0.1 mL, 1.06 mmol) was added dropwise under ice bath conditions. The mixture was stirred until no further exothermic reaction occurred, and the reaction was allowed to continue at room temperature for 4 h. The solvent was removed by concentration under reduced pressure. The residue was washed with MTBE (10 mL × 2) to obtain a yellow solid (0.22 g, 99%), which was directly used for the next reaction.

Step 5) Synthesis of 5-(4-((8-cyclopropyl-2-methyl-3-oxo-3,4-dihydroquinoxalin-6-yl)methyl)piperazin-1-yl)-6-fluoro-*N*-methylpicolinamide

7-(bromomethyl)-5-cyclopropyl-3-methylquinoxalin-2(1*H*)-one (0.22 g, 0.75 mmol), 6-fluoro-*N*-methyl-5-(piperazin-1-yl)picolinamide hydrochloride (0.29 g, 0.83 mmol), and potassium iodide (0.025 g, 0.15 mmol) were added to a flask and stirred until dissolved in ACN (5 mL). The mixture was cooled, and DIPEA (0.91 mL, 5.25 mmol) was slowly added dropwise under ice bath conditions. The mixture was heated to 80 °C and reacted for 2 h. After cooling to room temperature, a solid precipitated out with continued stirring. The filter cake was collected by filtration, washed with MTBE (5 mL × 3), washed with water (5 mL × 3), and dried under vacuum to give a white solid (0.184 g, 54.42%).

MS (ESI, pos. ion) *m*/*z:* 451.3 $[M+H]^+$;
$^1$H NMR (400 MHz, DMSO-$d_6$) δ (ppm) 12.20 (br, 1H), 8.41 (s, 1H), 7.84 (d, *J* = 6.8 Hz, 1H), 7.57 (t, *J* = 9.3 Hz, 1H), 7.05 (s, 1H), 6.66 (s, 1H), 3.54 (s, 2H), 3.20 - 3.12 (m, 4H), 2.95 (br, 1H), 2.77 (d, *J* = 3.1 Hz, 3H), 2.61 - 2.53 (m, 4H), 2.43 (s, 3H), 1.08 - 0.78 (m, 4H).

**Example 2: Synthesis of 6-fluoro-*N*-methyl-5-(4-((2-methyl-3-oxo-8-phenyl-3,4-dihydroquinoxalin-6-yl)methyl) piperazin-1-yl)picolinamide**

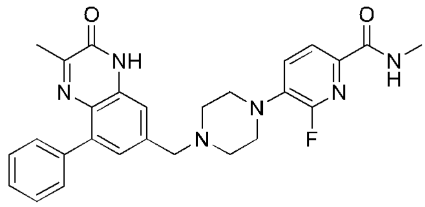

(2)

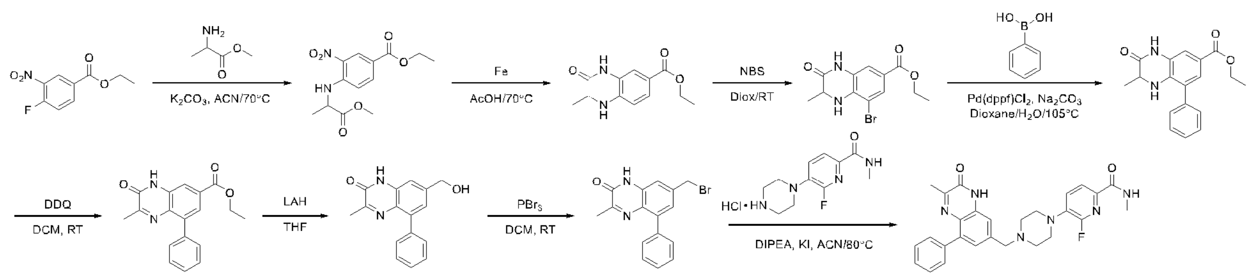


Step 1) Synthesis of ethyl 4-((1-methoxy-1-oxopropane-2-yl)amino)-3-nitrobenzoate

ethyl 4-fluoro-3-nitrobenzoate (3.0 g, 14.07 mmol) and DL-alanine methyl ester hydrochloride (2.36 g, 16.88 mmol) were added to a flask and stirred to dissolve in acetonitrile (30 mL). After cooling, potassium carbonate (4.67 g, 33.77 mmol) was added under ice bath conditions, and the mixture was heated to 70 °C and reacted overnight. After cooling to room temperature, water (60 mL) was added with stirring to quench the reaction. A solid precipitated out; the filter cake was collected and dried under vacuum to give a bright yellow solid (3.86 g, 92.57%).

MS (ESI, pos. ion) *m*/*z:* 297.20 $[M+H]^+$.

Step 2) Synthesis of ethyl 2-methyl-3-oxo-1,2,3,4-tetrahydroquinoxaloline-6-carboxylate

Ethyl 4-((1-methoxy-1-oxopropane-2-yl)amino)-3-nitrobenzoate (2.0 g, 6.75 mmol), AcOH (15 mL), and iron powder (1.51 g, 27 mmol) were added to a flask and heated to 70 °C for 1 h. The mixture was cooled to room temperature, quenched with 15 mL of water, and extracted with EtOAc (20 mL × 2). The organic phases were combined, washed with water (10 mL), washed with saturated brine (10 mL), dried over anhydrous sodium sulfate, and concentrated under reduced pressure to give a yellow solid (1.58 g, 99.92%).

MS (ESI, pos. ion) *m*/*z:* 235.20 $[M+H]^+$.

Step 3) Synthesis of ethyl 8-bromo-2-methyl-3-oxo-1,2,3,4-tetrahydroquinoxaloline-6-carboxylate

ethyl 2-methyl-3-oxo-1,2,3,4-tetrahydroquinoxaloline-6-carboxylate (1.46 g, 6.23 mmol) was added to a flask

and dissolved in 1,4-dioxane (30 mL). The mixture was cooled, and NBS (1.13 g, 6.35 mmol) was added under ice bath conditions. The mixture was gradually brought to room temperature and stirred overnight. The solution was quenched with saturated sodium thiosulfate, extracted with EtOAc (40 mL × 2), and the organic phases were combined. The mixture was washed with saturated brine (30 mL), dried over anhydrous sodium sulfate, concentrated under reduced pressure, and the residue was purified by silica gel column chromatography (PE/EtOAc (V/V) = 2/1) to give a milky white solid (1.56 g, 79.93%).

$^1$H NMR (599 MHz, DMSO-$d_6$) δ (ppm) 10.59 (s, 1H), 7.62 (s, 1H), 7.35 (s, 1H), 6.39 (s, 1H), 4.24 (dd, *J* = 13.7, 6.7 Hz, 2H), 4.06 (dd, J= 12.4, 6.3 Hz, 1H), 1.35 - 1.25 (m, 6H);
MS (ESI, pos. ion) *m*/*z:* 313.20 [M+H]$^+$.

Step 4) Synthesis of ethyl 2-methyl-3-oxo-8-phenyl-1,2,3,4-tetrahydroquinoxaloline-6-carboxylate

Ethyl 8-bromo-2-methyl-3-oxo-1,2,3,4-tetrahydroquinoxaloline-6-carboxylate (0.60 g, 1.92 mmol), phenylboronic acid (0.30 g, 2.50 mmol), Pd(dppf)$Cl_2$ (0.070 g, 0.096 mmol) and sodium carbonate (0.41 g, 3.84 mmol) were added sequentially to a flask. The mixture was stirred and dissolved in 1,4-dioxane (12 mL) and water (3 mL). The mixture was heated to 105 °C and reacted overnight. After cooling to room temperature, water (25 mL) was added to quench the reaction. The mixture was extracted with EtOAc (20 mL × 3). The combined organic phases were washed with saturated brine (20 mL), dried over anhydrous sodium sulfate, and concentrated under reduced pressure. The residue was purified by silica gel column chromatography (PE/EtOAc (V/V) = 3/2) to give a brown solid (0.54 g, 90.81%).

MS (ESI, pos. ion) *m*/*z:* 311.20 [M+H]$^+$.

Step 5) Synthesis of ethyl 2-methyl-3-oxo-8-phenyl-3,4-dihydroquinoxalin-6-carboxylate

ethyl 2-methyl-3-oxo-8-phenyl-1,2,3,4-tetrahydroquinoxalin-6-carboxylate (0.54 g, 1.74 mmol) was added sequentially to a flask and stirred to dissolve in DCM (19 mL). After cooling, DDQ (0.43 g, 1.91 mmol) was added under ice bath conditions, and the reaction was allowed to proceed to room temperature for 8 h. The reaction was quenched with saturated sodium bicarbonate aqueous solution (38 mL), stirred for 2 h, extracted with DCM (25 mL × 3), and the organic phases were combined. The mixture was washed with saturated sodium bicarbonate aqueous solution, dried over anhydrous sodium sulfate, and concentrated under reduced pressure to give an orange-yellow solid (0.517 g, 96.37%).

MS (ESI, pos. ion) *m*/*z:* 309.20 [M+H]$^+$.

Step 6) Synthesis of 7-(hydroxymethyl)-3-methyl-5-phenylquinoxalin-2(1*H*)-one

Ethyl 2-methyl-3-oxo-8-phenyl-3,4-dihydroquinoxalin-6-carboxylate (0.51 g, 1.64 mmol) was added sequentially to a flask and stirred to dissolve in THF (16.4 mL). After cooling, LAH (0.196 g, 5.01 mmol) was added in portions under ice bath conditions. The mixture was stirred until no further exothermic reaction occurred, and the reaction was allowed to continue at room temperature for 4 h. After cooling, water (5 mL) was added under an ice bath to quench the reaction. Dilute hydrochloric acid (1 M) was then added dropwise to adjust the pH to 3-4. The mixture was extracted with THF (25 mL × 2). The combined organic phases were washed with saturated brine (30 mL), dried over anhydrous sodium sulfate, and concentrated under reduced pressure. The residue was purified by silica gel column chromatography (DCM/MeOH (V/V) = 10/1) to give a white solid (0.28 g, 63.57%).

MS (ESI, pos. ion) *m*/*z:* 267.40 [M+H]$^+$.

Step 7) Synthesis of 7-(bromomethyl)-3-methyl-5-phenylquinoxalin-2(1*H*)-one

To the flask were added 7-(hydroxymethyl)-3-methyl-5-phenylquinoxalin-2(1*H*)-one (0.15 g, 0.56 mmol) and DCM (10 mL). After cooling under an ice bath, phosphorus tribromide (0.08 mL, 0.85 mmol) was added dropwise, and the mixture was stirred until no further exothermic reaction was observed. The reaction was then allowed to proceed at room temperature for 4 hours. The solvent was removed by concentration under reduced pressure, and the residue was washed with MTBE (10 mL × 2) to afford 0.185 g of a yellow solid, which was used directly in the next step.

Step 8) Synthesis of 6-fluoro-*N*-methyl-5-(4-((2-methyl-3-oxo-8-phenyl-3,4-dihydroquinoxalin-6-yl)methyl)piperazin-1-yl)picolinamide

To the flask were added 7-(bromomethyl)-3-methyl-5-phenylquinoxalin-2(1*H*)-one (0.185 g, 0.56 mmol), 6-fluoro-*N*-methyl-5-(piperazin-1-yl)picolinamide hydrochloride (0.21 g, 0.62 mmol), and potassium iodide (0.019 g, 0.11 mmol). The mixture was stirred to dissolve in ACN (4 mL), cooled under an ice bath, and DIPEA (0.68 mL, 3.92 mmol) was added dropwise. The reaction mixture was then heated to 80°C and stirred for 2 hours. After cooling to room temperature,

solid precipitated upon continued stirring. The solid was collected by filtration, and the filter cake was washed sequentially with MTBE (4 mL × 3) and water (4 mL × 3), then dried under vacuum to afford a white solid (0.177 g, 64.73%).

MS (ESI, pos. ion) *m/z:* 487.20 $[M+H]^+$;

$^1H$ NMR (400 MHz, DMSO-$d_6$) δ (ppm) 12.32 (br, 1H), 8.38 (d, *J* = 4.2 Hz, 1H), 7.82 (d, *J* = 7.7 Hz, 1H), 7.57 - 7.52 (m, 3H), 7.44 (t, *J* = 7.2 Hz, 2H), 7.41 - 7.35 (m, 1H), 7.25 (d, *J* = 15.5 Hz, 2H), 3.64 (s, 2H), 3.22 - 3.14 (m, 4H), 2.75 (d, *J*= 4.2 Hz, 3H), 2.63 - 2.55 (m, 4H), 2.32 (s, 3H).

**Example 3: Synthesis of 6-fluoro-5-(4-((8-isopropoxy-2-methyl-3-oxo-3,4-dihydroquinoxalin-6-yl)methyl)piperazin-1-yl)-*N*-methylpicolinamide**

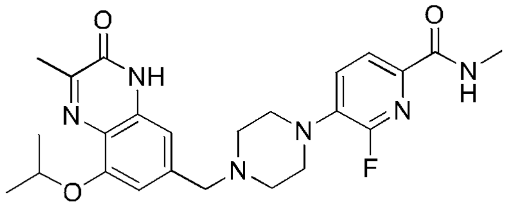

(3)

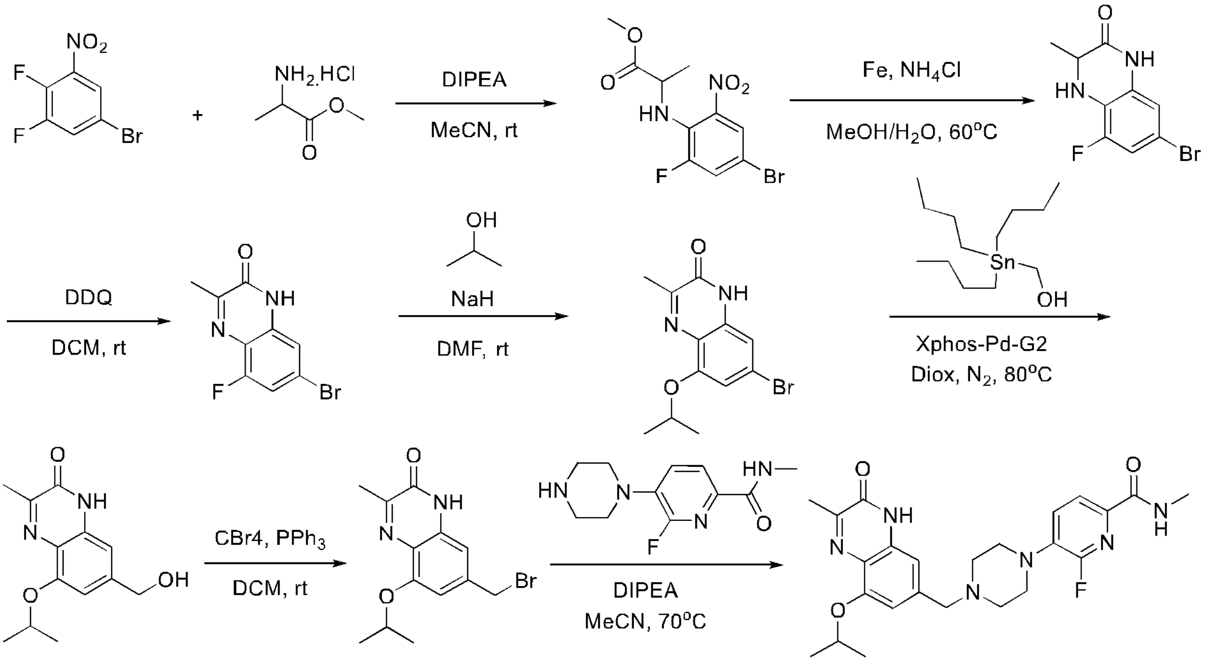


Step 1) Synthesis of methyl (4-bromo-2-fluoro-6-nitrophenyl)aminopropionate

5-bromo-1,2-difluoro-3-nitrobenzene (10 g, 42.02 mmol), DL-alanine methyl ester hydrochloride (6.16 g, 44.12 mmol), and DIPEA (16.29 g, 126.06 mmol) were added to MeCN (120 mL) and reacted at room temperature for 16 h. The reaction solution was concentrated under reduced pressure, diluted with DCM (120 mL), washed with water (80 mL) and saturated NaCl solution (80 mL), dried over anhydrous $Na_2SO_4$, filtered, and concentrated under reduced pressure to give a yellow liquid (13 g, 96%).

MS (ESI, pos. ion) *m/z:* 321.1 $[M+H]^+$.

Step 2) Synthesis of 7-bromo-5-fluoro-3-methyl-3,4-dihydroquinoxalin-2(1*H*)-one

Methyl (4-bromo-2-fluoro-6-nitrophenyl)aminopropionate (13.00 g, 40.49 mmol), iron powder (13.57 g, 242.94 mmol), and ammonium chloride (12.99 g, 242.94 mmol) were added to MeOH (120 mL) and water (40 mL), and reacted at 60 °C for 16 h. The reaction solution was filtered through diatomaceous earth, the filtrate was concentrated under reduced

pressure, water (100 mL) was added, and the mixture was stirred for 1 h. After filtration, the solid was dried at 50 °C for 12 h. to obtain a pale yellow solid (10.49 g, 90%).

MS (ESI, pos. ion) *m/z:* 259.0 $[M+H]^+$.

Step 3) Synthesis of 7-bromo-5-fluoro-3-methylquinoxalin-2(1*H*)-one

DDQ (9.16 g, 40.34 mmol) was added to a DCM (150 mL) solution of 7-bromo-5-fluoro-3-methyl-3,4-dihydroquinoxalin-2(1*H*)-one (9.5 g, 36.67 mmol) and reacted at room temperature for 2 h. The reaction solution was concentrated under reduced pressure, and saturated $NaHCO_3$ solution (400 mL) was added and stirred. The mixture was filtered to obtain a solid, which was dried under vacuum at 50 °C for 12 h to obtain a pale yellow solid (9.0 g, 95%).

MS (ESI, pos. ion) *m/z:* 257.0 $[M+H]^+$.

Step 4) Synthesis of 7-bromo-3-isopropoxy-3-methylquinoxalin-2(1*H*)-one

At 0 °C, NaH (390 mg, 9.75 mmol, 60%) was added to a DMF (10 mL) solution of 7-bromo-5-fluoro-3-methylquinoxalin-2(1*H*)-one (500 mg, 1.95 mmol) and isopropanol (586 mg, 9.75 mmol). After the addition was complete, the reaction was carried out at room temperature for 15 h. The reaction solution was concentrated under reduced pressure, and the residue was purified by silica gel column chromatography (DCM/EtOAc (V/V) = 4/1) to give a pale yellow solid (400 mg, 69%).

MS (ESI, pos. ion) *m/z:* 297.1 $[M+H]^+$.

Step 5) Synthesis of 7-(hydroxymethyl)-5-isopropoxy-3-methylquinoxalin-2(1*H*)-one

7-bromo-5-isopropoxy-3-methylquinoxalin-2(1*H*)-one (1.20 g, 4.04 mmol), (tributyltin)methanol (1.43 mg, 4.44 mmol), and Xphos-Pd-G2 (318 mg, 0.40 mmol) were added to 1,4-dioxane (40 mL). The reaction was carried out under nitrogen protection at 80 °C for 14 h. The reaction solution was concentrated under reduced pressure, and the residue was purified by silica gel column chromatography (DCM/MeOH (V/V) = 20/1) to give a pale yellow solid (910 mg, 90%).

MS (ESI, pos. ion) *m/z:* 249.2 $[M+H]^+$.

Step 6) Synthesis of 7-(bromomethyl)-5-isopropoxy-3-methylquinoxalin-2(1*H*)-one

$CBr_4$ (602 mg, 1.81 mmol) was added to 8 mL of DCM containing 7-(hydroxymethyl)-5-isopropoxy-3-methylquinoxalin-2(1*H*)-one (300 mg, 1.21 mmol) and $PPh_3$ (476 mg, 1.81 mmol), and reacted at room temperature for 18 h. The reaction solution was concentrated under reduced pressure, and the residue was purified by silica gel column chromatography (PE/EtOAc (V/V) = 1/1) to give a yellow solid (300 mg, 80%).

MS (ESI, pos. ion) *m/z:* 311.1 $[M+H]^+$.

Step 7) Synthesis of 6-fluoro-5-(4-((8-isopropoxv-2-methyl-3-oxo-3,4-dihydroquinoxalin-6-yl)methyl)piperazin-1-yl)-*N*-methylpicolinamide

7-(bromomethyl)-5-isopropoxy-3-methylquinoxalin-2(1*H*)-one (300 mg, 0.96 mmol), 6-fluoro-*N*-methyl-5-(piperazin-1-yl)picolinamide (297 mg, 1.25 mmol), and DIPEA (496 mg, 3.84 mmol) were added sequentially to MeCN (8 mL), and the reaction was carried out at 70 °C for 4 h. The reaction solution was concentrated under reduced pressure, diluted with DCM (50 mL), washed with water (30 mL) and saturated NaCl solution (30 mL), dried over anhydrous $Na_2SO_4$, concentrated under reduced pressure, and the residue was purified by silica gel column chromatography (DCM/MeOH (V/V) = 20/1) to give a pale yellow solid (130 mg, 29%).

MS (ESI, pos. ion) *m/z:* 469.3 $[M+H]^+$;
$^1H$ NMR (400 MHz, DMSO-$d_6$) δ (ppm) 12.15 (s, 1H), 8.39 (d, *J* = 4.7 Hz, 1H), 7.84 (d, *J*= 7.9 Hz, 1H), 7.62 - 7.51 (m, 1H), 6.81 (s, 2H), 4.79 - 4.73 (m, 1H), 3.57 (s, 2H), 3.23 - 3.10 (m, 4H), 2.76 (d, *J* = 4.6 Hz, 3H), 2.61 - 2.53 (m, 4H), 2.37 (s, 3H), 1.34 (d, *J* = 6.0 Hz, 6H).

**Example 4: Synthesis of 6-fluoro-*N*-methyl-5-(4-((2-methyl-3-oxo-8-phenoxy-3,4-dihydroquinoxalin-6-yl) methyl)piperazin-1-yl)picolinamide**

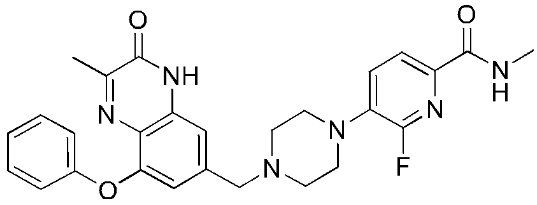

(4)

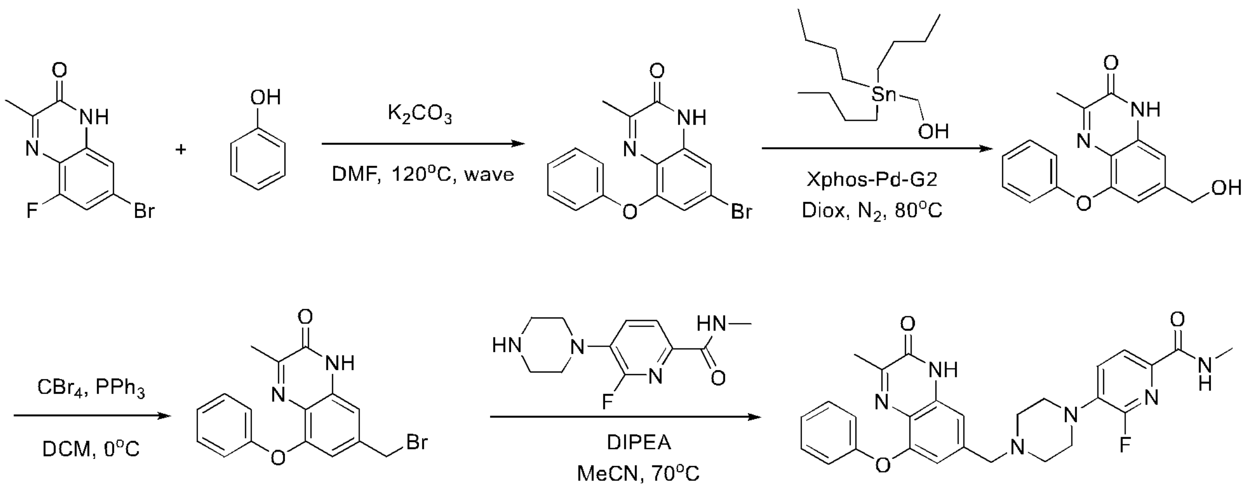


Step 1) Synthesis of 7-bromo-3-methyl-5-phenoxyquinoxalin-2(1*H*)-one

7-bromo-5-fluoro-3-methylquinoxalin-2(1*H*)-one (1.0 g, 3.89 mmol), phenol (730 mg, 7.78 mmol), and $K_2CO_3$ (1.61 g, 11.67 mmol) were added to DMF (12 mL). After addition, the mixture was microwaved at 120 °C for 18 h. The reaction solution was diluted with water (50 mL), and a solid precipitated. The solid was filtered and dissolved in MeOH (5 mL) and DCM (50 mL), dried over anhydrous $Na_2SO_4$, filtered, concentrated under reduced pressure, and the residue was purified by silica gel column chromatography (DCM/EtOAc (V/V) = 4/1) to give a pale yellow solid (300 mg, 23%).

MS (ESI, pos. ion) *m/z:* 331.1 $[M+H]^+$.

Step 2) Synthesis of 7-(hydroxymethyl)-3-methyl-5-phenoxyquinoxalin-2(1*H*)-one

7-bromo-3-methyl-5-phenoxyquinoxalin-2(1*H*)-one (300 g, 0.91 mmol), (tributyltin)methanol (321 mg, 1.00 mmol), and Xphos-Pd-G2 (71 mg, 0.09 mmol) were added to 1,4-dioxane (16 mL) under nitrogen protection and reacted at 80 °C for 16 h. The reaction solution was concentrated under reduced pressure, and the residue was purified by silica gel column chromatography (DCM/MeOH (v/v) = 20/1) to give a pale yellow solid (200 mg, 78%).

MS (ESI, pos. ion) *m/z:* 283.2 $[M+H]^+$.

Step 3) Synthesis of 7-(bromomethyl)-3-methyl-5-phenoxyquinoxalin-2(1*H*)-one

$CBr_4$ (114 mg, 0.35 mmol) was added to 6 mL of DCM containing 7-(hydroxymethyl)-3-methyl-5-phenoxyquinoxalin-2(1*H*)-one (65 mg, 0.23 mmol) and $PPh_3$ (90 mg, 0.35 mmol), and the reaction was carried out at room temperature for 16 h. The reaction solution was concentrated under reduced pressure to give a yellow solid (75 mg, 94%).

MS (ESI, pos. ion) *m/z:* 345.1 $[M+H]^+$.

Step 4) Synthesis of 6-fluoro-*N*-methyl-5-(4-((2-methyl-3-oxo-8-phenoxy-3,4-dihydroquinoxalin-6-yl)methyl)piperazin-1-yl)picolinamide

7-(bromomethyl)-3-methyl-5-phenoxyquinoxalin-2(1*H*)-one (50 mg, 0.14 mmol), 6-fluoro-*N*-methyl-5-(piperazin-1-yl)picolinamide (43 mg, 0.18 mmol), and DIPEA (72 mg, 0.56 mmol) were added sequentially to MeCN (4 mL), and the reaction was carried out at 70 °C for 4 h. The reaction solution was concentrated under reduced pressure, diluted with DCM (50 mL), washed with water (30 mL) and saturated NaCl solution (30 mL), dried over anhydrous $Na_2SO_4$, concentrated under reduced pressure, and the residue was purified by silica gel column chromatography (DCM/MeOH (V/V) = 20/1) to give a pale yellow solid (25 mg, 34%).

MS (ESI, pos. ion) *m/z:* 503.3 $[M+H]^+$.
$^1H$ NMR (400 MHz, DMSO-$d_6$) δ (ppm) 12.37 (s, 1H), 8.40 (d, *J*= 4.7 Hz, 1H), 7.84 (d, *J*= 7.9 Hz, 1H), 7.56 (dd, *J*= 10.5, 8.2 Hz, 1H), 7.40 (t, *J* = 7.9 Hz, 2H), 7.15 (dd, *J* = 15.8, 8.4 Hz, 1H), 7.09 - 7.01 (m, 3H), 6.75 (s, 1H), 3.55 (s, 2H), 3.17 - 3.09 (m, 4H), 2.77 (d, *J* = 4.7 Hz, 3H), 2.59 - 2.53 (m, 4H), 2.36 (s, 3H).

**Example 5: Synthesis of 6-fluoro-*N*-methyl-5-(4-((2-methyl-3-oxo-8-(phenylamino)-3,4-dihydroquinoxalin-6-yl) methyl)piperazin-1-yl)picolinamide**

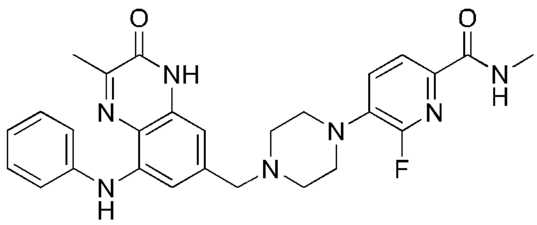

(5)

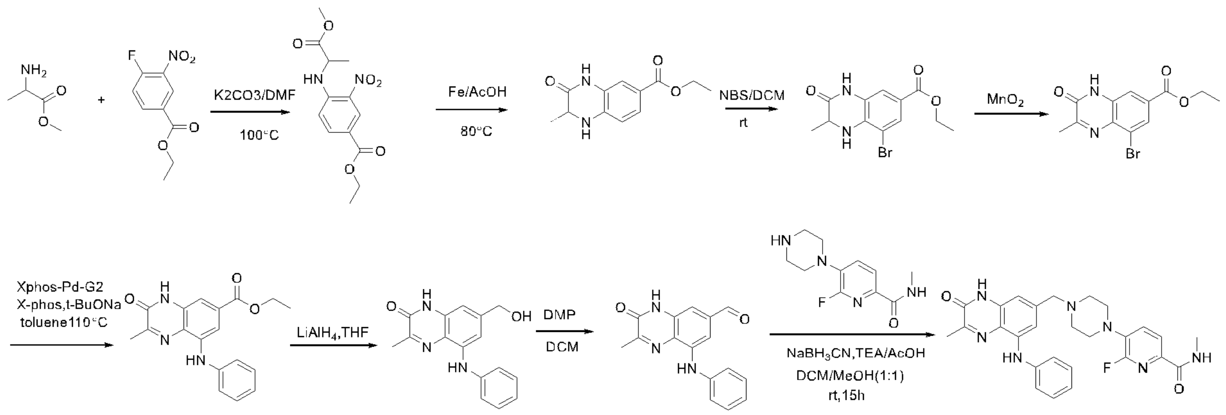


Step 1) Synthesis of ethyl 4-((1-methoxy-1-oxopropyl-2-yl)amino)-3-nitrobenzoate

ethyl 4-fluoro-3-nitrobenzoate (6.42 g, 30.13 mmol) was dissolved in *N*,*N*-dimethylformamide (100 mL), potassium carbonate (12.49 g, 90.36 mmol) and DL-alanine methyl ester hydrochloride (4.63 g, 33.13 mmol) were added. The mixture was heated to 100 °C and reacted for 3 hours. The reaction was then stopped, cooled to room temperature, and water (500 mL) was added. The mixture was extracted with ethyl acetate (400 mL * 3). The organic phase was washed with saturated brine, dried over anhydrous sodium sulfate, concentrated, and the residue was purified by silica gel column chromatography (eluent: petroleum ether/ethyl acetate (V/V) = 4/1) to give a yellow solid (1.6 g, 17.9%).
MS (ESI, pos. ion) *m/z:* 297.4 $[M+H]^+$.

Step 2) Synthesis of ethyl 2-methyl-3-oxo-1,2,3,4-tetrahydroquinoxalin-6-carboxylate

Ethyl 4-((1-methoxy-1-oxopropyl-2-yl)amino)-3-nitrobenzoate (1.5 g, 5.06 mmol), acetic acid (10 mL), and iron powder (1.41 g, 25.30 mmol) were added to a reaction flask, heated to 80 °C, and reacted for 5 hours. The reaction was then stopped, cooled to room temperature, and water (500 mL) was added. The mixture was extracted with dichloromethane (150 mL x 2). The organic phase was washed with saturated brine, dried over anhydrous sodium sulfate, filtered, and the filtrate was concentrated under reduced pressure to give a pale yellow solid (1.10 g, 92.7%).
MS (ESI, pos. ion) *m/z:* 235.2 $[M+H]^+$.

Step 3) Synthesis of ethyl 8-bromo-2-methyl-3-oxo-1,2,3,4-tetrahydroquinoxalin-6-carboxylate

Ethyl 2-methyl-3-oxo-1,2,3,4-tetrahydroquinoxalin-6-carboxylate (0.53 g, 2.27 mmol) was dissolved in 1,4-dioxane (10 mL), and *N*-bromosuccinimide (0.42 g, 2.37 mmol) was added with stirring at room temperature. The reaction was carried out for 16 hours, followed by the addition of water (150 mL). The mixture was extracted with dichloromethane (200 mL), and the organic phase was washed with saturated brine, dried over anhydrous sodium sulfate, filtered, and the

filtrate was concentrated under reduced pressure to give a yellow solid (0.52 g, 73.3%).
MS (ESI, pos. ion) *m/z:* 313.3 $[M+H]^+$.

Step 4) Synthesis of ethyl 8-bromo-2-methyl-3-oxo-3,4-dihydroquinoxalin-6-carboxylate

ethyl 8-bromo-2-methyl-3-oxo-1,2,3,4-tetrahydroquinoxalin-6-carboxylate (0.1 g, 0.32 mmol) was dissolved in dichloromethane (4 mL). Active manganese dioxide (0.2 g, 1.96 mmol, 85%) was added with stirring at room temperature. The mixture was stirred and reacted at room temperature for 2.5 hours. The mixture was concentrated under reduced pressure, and the residue was purified by silica gel column chromatography (eluent: petroleum ether/ethyl acetate (V/V) = 4/1) to give a pale yellow solid (0.078 g, 78.5%).
MS (ESI, pos. ion) *m/z:* 311.2 $[M+H]^+$.

Step 5) Synthesis of Ethyl 2-methyl-3-oxo-8-(phenylamino)-3,4-dihydroquinoxalin-6-carboxylate

Ethyl 8-bromo-2-methyl-3-oxo-3,4-dihydroquinoxalin-6-carboxylate (0.084 g, 0.27 mmol), aniline (0.038 g, 0.41 mmol), chloro(2-dicyclohexylphosphino-2',4',6'-triisopropyl-1,1'-biphenyl)[2-(2'-amino-1,1'-biphenyl)]palladium(II) (0.021 g, 0.027 mmol), 2-dicyclohexylphosphino-2',4',6'-triisopropylbiphenyl (0.026 g, 0.054 mmol), sodium *tert*-butoxide (0.052 g, 0.54 mmol), and toluene (5 mL) were prepared. Add to the reaction flask, replace with nitrogen, heat to 100°C under nitrogen protection, react for 18 hours, turn off the reaction, cool to room temperature, concentrate under reduced pressure, and purify the residue by silica gel column chromatography (eluent: petroleum ether/ethyl acetate (V/V) = 3/1) to give a yellow solid (0.04 g, 45.8%).
MS (ESI, pos. ion) *m/z:* 324.2 $[M+H]^+$.

Step 6) Synthesis of 7-(hydroxymethyl)-3-methyl-5-(phenylamino)quinoxalin-2(1*H*)-one

Ethyl 2-methyl-3-oxo-8-(phenylamino)-3,4-dihydroquinoxalin-6-carboxylate (0.16 g, 0.49 mmol) was dissolved in tetrahydrofuran (5 mL). Lithium aluminum hydride (0.07 g, 1.85 mmol) was added at 0 °C. After the addition was complete, the reaction was maintained at this temperature for 22 h. The temperature was then raised to 65 °C and the reaction was continued for another 4.5 h. The reaction was then stopped, cooled to room temperature, and water (0.5 mL) was added dropwise to quench the reaction. The mixture was concentrated under reduced pressure, and the residue was purified by silica gel column chromatography (eluent: dichloromethane/methanol (V/V) = 20/1) to give a yellow solid (0.05 g, 35.92%).
MS (ESI, pos. ion) *m/z:* 282.2 $[M+H]^+$.

Step 7) Synthesis of 2-methyl-3-oxo-8-(phenylamino)-3,4-dihydroquinoxalin-6-carboxaldehyde

7-(hydroxymethyl)-3-methyl-5-(phenylamino)quinoxalin-2(1*H*)-one (0.04 g, 0.14 mmol) was dissolved in a mixture of tetrahydrofuran (2 mL) and dichloromethane (2 mL). Dess-Martin Periodinane (0.12 g, 0.28 mmol) was added at 0 °C, and the reaction was allowed to proceed to room temperature for 16 hours. The reaction was then stopped, filtered, and the filter cake was washed with dichloromethane (10 mL). The filtrate was concentrated to obtain a yellow oily substance (0.038 g, 95.6%).
MS (ESI, pos. ion) *m/z:* 280.2 $[M+H]^+$.

Step 8) Synthesis of 6-fluoro-*N*-methyl-5-(4-((2-methyl-3-oxo-8-(phenylamino)-3,4-dihydroquinoxalin-6-yl)methyl)piperazin-1-yl)picolinamide

6-Fluoro-*N*-methyl-5-(piperazin-1-yl)pyridine amide (0.05 g, 0.21 mmol) was dissolved in a mixed solution of dichloromethane (2 mL) and methanol (2 mL). Triethylamine (0.028 g, 0.28 mmol), 2-methyl-3-oxo-8-(phenylamino)-3,4-dihydroquinoxalin-6-carboxaldehyde (0.038 g, 0.14 mmol), acetic acid (0.0017 g, 0.028 mmol), and sodium cyanoborohydride (0.026 g, 0.42 mmol) were added. The mixture was reacted at room temperature for 20 hours, concentrated under reduced pressure, and the residue was purified by silica gel column chromatography (eluent: dichloromethane/-methanol (V/V) = 15/1) to give a yellow solid (0.014 g, 20.5%).
MS (ESI, pos. ion) *m/z:* 502.3 $[M+H]^+$;

HRMS: calcd. for $C_{27}H_{28}FN_7O_2$ $[M+H]^+$: 502.2361, found: 502.2368;
$^1H$ NMR (400 MHz, DMSO-$d_6$) δ (ppm) 12.19 (s, 1H), 8.40 (d, *J* = 4.9 Hz, 1H), 8.21 (s, 1H), 7.84 (d, *J* = 7.8 Hz, 1H), 7.68 - 7.51 (m, 1H), 7.34 (d, *J* = 4.2 Hz, 4H), 7.05 (s, 1H), 7.03 - 6.92 (m, 1H), 6.65 (s, 1H), 3.51 (s, 2H), 3.19 - 3.12 (m, 4H), 2.77 (d, *J*= 4.7 Hz, 3H), 2.59 - 2.52 (m, 4H), 2.44 (s, 3H).

**Example 6: Synthesis of 6-fluoro-5-(4-((8-(4-fluorophenoxy)-2-methyl-3-oxo-3,4-dihydroquinoxalin-6-yl) methyl)piperazin-1-yl)-*N*-methylpicolinamide**

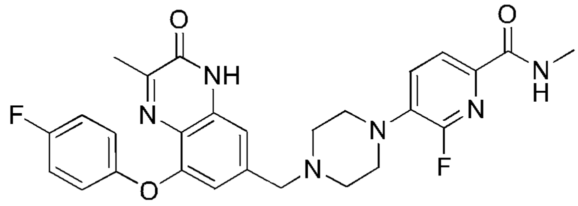

(6)

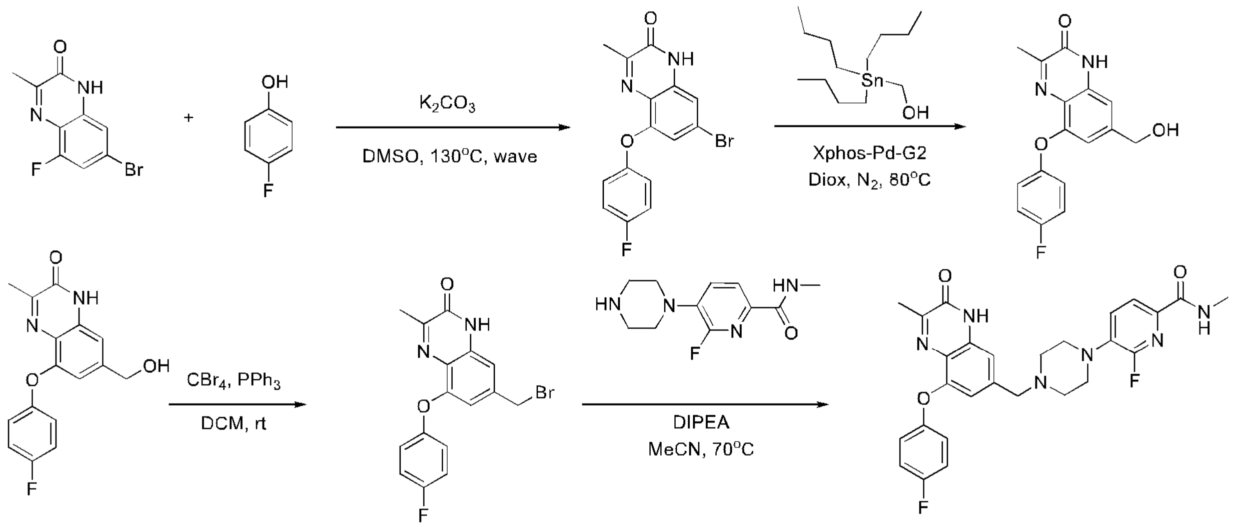


Step 1) Synthesis of 7-bromo-5-(4-fluorophenoxv)-3-methylquinoxalin-2(1*H*)-one

7-bromo-5-fluoro-3-methylquinoxalin-2(1*H*)-one (0.8 g, 3.11 mmol, refer to Step 3 of Example 3), 4-fluorophenol (1.05 g, 9.33 mmol), and $K_2CO_3$ (1.29 g, 9.33 mmol) were added to DMSO (12 mL) solution. After addition, the mixture was microwaved at 130 °C for 16 h. The reaction solution was diluted with water (60 mL), and a solid precipitated. The solid was filtered and dissolved in methanol (5 mL) and dichloromethane (50 mL), dried over anhydrous sodium sulfate, filtered, and the filtrate was concentrated under reduced pressure to obtain a pale yellow solid (0.6 g, 55%).

MS (ESI, pos. ion) *m/z:* 349.2 $[M+H]^+$.

Step 2) Synthesis of 5-(4-fluorophenoxy)-7-(hydroxymethyl)-3-methylquinoxalin-2(1*H*)-one

7-bromo-5-(4-fluorophenoxy)-3-methylquinoxalin-2(1*H*)-one (600 mg, 1.72 mmol), (tributyltin)methanol (607 mg, 1.89 mmol), and Xphos-Pd-G2 (135 mg, 0.17 mmol) were added to 1,4-dioxane (16 mL), and the mixture was reacted under nitrogen protection at 80 °C for 16 h. The reaction solution was concentrated under reduced pressure, and the residue was purified by silica gel column chromatography (dichloromethane/methanol (V/V) = 20/1) to give a pale yellow solid (350 mg, 68%).

MS (ESI, pos. ion) *m/z:* 301.2 $[M+H]^+$.

Step 3) Synthesis of 7-(bromomethyl)-5-(4-fluorophenoxy)-3-methylquinoxalin-2(1*H*)-one

At 0 °C, $CBr_4$ (776 mg, 2.34 mmol) was added to dichloromethane (20 mL) containing 5-(4-fluorophenoxy)-7-(hydroxymethyl)-3-methylquinoxalin-2(1*H*)-one (350 mg, 1.17 mmol) and $PPh_3$ (613 mg, 2.34 mmol). After 1 h, the reaction was carried out at room temperature for 16 h. The reaction solution was concentrated under reduced pressure, and the residue was purified by silica gel column chromatography (eluent: petroleum ether/ethyl acetate (V/V) = 1/1) to give a yellow solid (300 mg, 71%).

MS (ESI, pos. ion) *m/z:* 363.1 $[M+H]^+$.

Step 4) Synthesis of 6-fluoro-5-(4-((8-(4-fluorophenoxy)-2-methyl-3-oxo-3,4-dihydroquinoxalin-6-yl)methyl)piperazin-1-yl)-*N*-methylpicolinamide

7-(bromomethyl)-5-(4-fluorophenoxy)-3-methylquinoxalin-2(1*H*)-one (300 mg, 0.83 mmol), 6-fluoro-N-methyl-5-(piperazin-1-yl)pyridineamide (198 mg, 0.83 mmol), and *N*,*N*-diisopropylethylamine (429 mg, 3.32 mmol) were added sequentially to acetonitrile (8 mL), and the reaction was carried out at 70 °C for 3 h. The reaction solution was concentrated under reduced pressure, diluted with dichloromethane (50 mL), washed with water (30 mL) and saturated NaCl solution (30 mL), dried over anhydrous $Na_2SO_4$, and the filtrate was concentrated under reduced pressure. The residue was purified by silica gel column chromatography (eluent: dichloromethane/methanol (V/V) = 20/1) to give a pale yellow solid (260 mg, 60%).

MS (ESI, pos. ion) *m/z:* 521.3 $[M+H]^+$;

HRMS: calcd. for $C_{27}H_{26}F_2N_6O_3$ $[M+H]^+$: 521.2034, found: 521.2111;

$^1$H NMR (400 MHz, DMSO-$d_6$) δ (ppm) 12.34 (s, 1H), 8.37 (d, *J* = 4.7 Hz, 1H), 7.84 (d, *J*= 7.9 Hz, 1H), 7.58 - 7.51 (m, 1H), 7.22 (t, *J* = 8.7 Hz, 2H), 7.12 - 7.05 (m, 2H), 7.04 (s, 1H), 6.71 (s, 1H), 3.54 (s, 2H), 3.17 - 3.09 (m, 4H), 2.76 (d, *J*= 4.7 Hz, 3H), 2.56 - 2.51 (m, 4H), 2.36 (s, 3H).

**Example 7: Synthesis of 6-fluoro-*N*-methyl-5-(4-((2-methyl-3-oxo-8-(prop-1-yn-1-yl)-3,4-dihydroquinoxalin-6-yl) methyl)piperazin-1-yl)picolinamide**

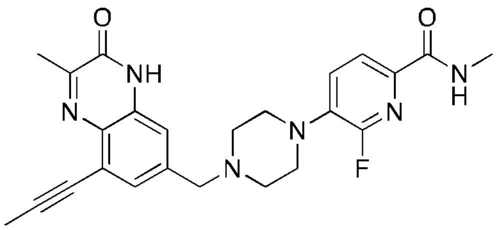

(7)

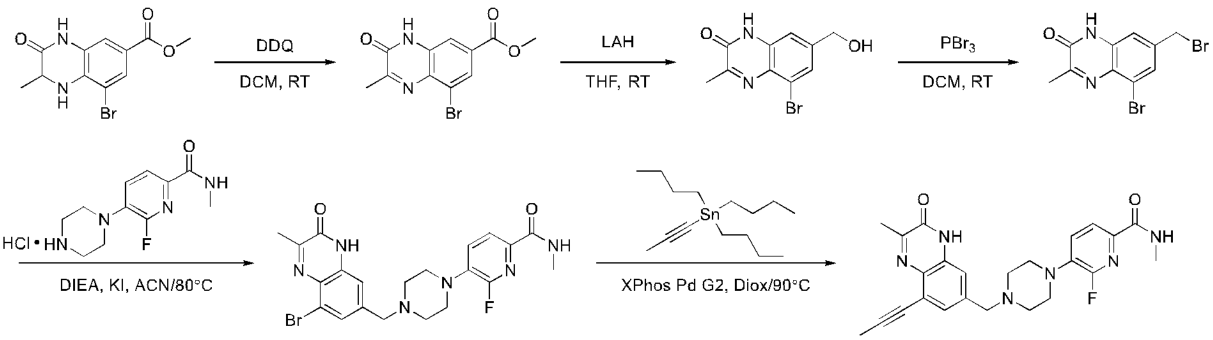


Step 1) Synthesis of methyl 8-bromo-2-methyl-3-oxo-3,4-dihydroquinoxalin-6-carboxylate

Methyl 8-bromo-2-methyl-3-oxo-1,2,3,4-tetrahydroquinoxalin-6-carboxylate (0.31 g, 1.04 mmol, refer to Step 3 of Example 8) was added to a 100 mL reaction flask and stirred to dissolve in dichloromethane (12 mL). After cooling, 2,3-dichloro-5,6-dicyanobenzoquinone (0.26 g, 1.14 mmol) was added in portions under ice bath, stirred until homogeneous, and reacted at room temperature for 4 h. The solvent was removed by concentration under reduced pressure, cooled, and quenched by adding saturated sodium bicarbonate aqueous solution (24 mL) under ice bath. A solid precipitated out. The mixture was stirred for 30 min, filtered, and the filter cake was collected. The filter cake was washed with saturated sodium bicarbonate aqueous solution and dried under vacuum to obtain a brownish-red solid (0.18 g, 58.46%).

MS (ESI, pos. ion) *m/z:* 297.10, 299.05 $[M+H]^+$.

Step 2) Synthesis of 5-bromo-7-(hydroxvmethyl)-3-methylquinoxalin-2(1*H*)-one

Methyl 8-bromo-2-methyl-3-oxo-3,4-dihydroquinoxalin-6-carboxylate (0.18 g, 0.61 mmol) was added to a 50 mL reaction flask and stirred to dissolve in tetrahydrofuran (9 mL). After cooling, lithium aluminum hydride (0.070 g, 1.79 mmol)

was added under ice bath conditions, and the reaction was continued at 0 °C for 2 h. The mixture was quenched with water (1.79 mL) under ice bath conditions. The pH was adjusted to 3-4 by adding 1 M dilute hydrochloric acid. The mixture was extracted with tetrahydrofuran (10 mL * 2). The organic phases were combined, washed with saturated brine, dried over anhydrous sodium sulfate, concentrated under reduced pressure, and the residue was purified by silica gel column chromatography (eluent: dichloromethane/methanol (V/V) = 95/5) to give a brick-red solid (0.112 g, 68.70%).

MS (ESI, pos. ion) *m/z:* 269.20, 271.15 $[M+H]^+$.

Step 3) Synthesis of 5-bromo-7-(bromomethyl)-3-methylquinoxalin-2(1*H*)-one

5-bromo-7-(hydroxymethyl)-3-methylquinoxalin-2(1*H*)-one (0.11 g, 0.41 mmol) and dichloromethane (10 mL) were added to a 100 mL reaction flask. The mixture was cooled, and phosphorus tribromide (0.060 mL, 0.64 mmol) was added dropwise under ice bath conditions. The mixture was stirred until no further exothermic reaction occurred, and then allowed to react overnight at room temperature. The solvent was removed by concentration under reduced pressure to obtain a yellow solid product (0.135 g, 99.5%), which was then directly used for the next reaction.

Step 4) Synthesis of 5-(4-((8-bromo-2-methyl-3-oxo-3,4-dihydroquinoxalin-6-yl)methyl)piperazin-1-yl)-6-fluoro-*N*-methylpyridine amide

In a 100 mL reaction flask, 5-bromo-7-(bromomethyl)-3-methylquinoxalin-2(1*H*)-one (0.135 g, 0.41 mmol) was added, followed by 6-fluoro-*N*-methyl-5-(piperazin-1-yl)picolinamide hydrochloride (0.12 g, 0.45 mmol) and potassium iodide (0.014 g, 0.082 mmol). The mixture was stirred to dissolve in acetonitrile (2.5 mL). After cooling, *N*,*N*-diisopropylethylamine (0.50 mL, 2.87 mmol) was slowly added dropwise under an ice bath. The mixture was stirred uniformly and then heated to 80°C to react for 2 hours. After cooling to room temperature, solids precipitated. The resulting solids were collected by filtration, washed with methyl *tert*-butyl ether (2.5 mL * 2), then washed with water (2.5 mL * 2), and dried under vacuum to afford an off-white solid (145 mg, 72.87%).

MS (ESI, pos. ion) *m/z:* 489.15, 490.10 $[M+H]^+$.

Step 5) Synthesis of 6-fluoro-*N*-methyl-5-(4-((2-methyl-3-oxo-8-(prop-1-yn-1-yl)-3,4-dihvdroquinoxalin-6-yl)methyl)piperazin-1-yl)picolinamide

5-(4-((8-bromo-2-methyl-3-oxo-3,4-dihydroquinoxalin-6-yl)methyl)piperazin-1-yl)-6-fluoro-*N*-methylpyridineamide (0.10 g, 0.20 mmol) and tributyl(prop-1-yn-1-yl)stannane (0.12 g, 0.36 mmol) were added sequentially to a 100 mL reaction flask and stirred to dissolve in 1,4-dioxane (8 mL). Xphos-Pd-G2 (0.024 g, 0.031 mmol) was then added, the mixture was purged with nitrogen, and the reaction was heated to 90 °C overnight. After cooling to room temperature, the solvent was removed by concentration under reduced pressure. The residue was purified by silica gel column chromatography (eluent: dichloromethane/methanol (V/V) = 97/3) to give a white solid (0.030 g, 32.73%).

MS (ESI, pos. ion) *m/z:* 449.40 $[M+H]^+$;

HRMS: calcd. for $C_{24}H_{25}FN_6O_2$ $[M+H]^+$: 449.2096, found: 449.2128;
$^1H$ NMR (400 MHz, DMSO-$d_6$) δ (ppm) 12.32 (s, 1H), 8.37 (s, 1H), 7.85 (s, 1H), 7.59 (s, 1H), 7.31 - 7.22 (m, 2H), 3.59 (s, 2H), 3.21 - 3.13 (m, 4H), 2.77 (s, 3H), 2.59 - 2.53 (m, 4H), 2.43 (s, 3H), 2.14 (s, 3H).

**Example 8: Synthesis of 6-fluoro-5-(4-((8-(2-fluorophenyl)-2-methyl-3-oxo-3,4-dihydroquinoxalin-6-yl)methyl) piperazin-1-yl)-*N*-methylpicolinamide**

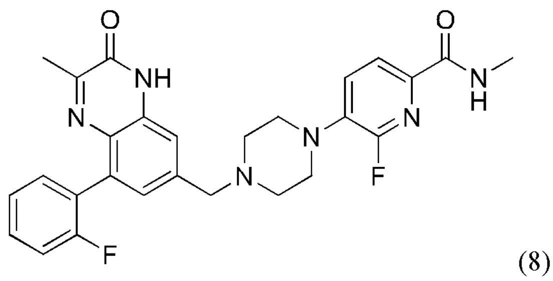

(8)

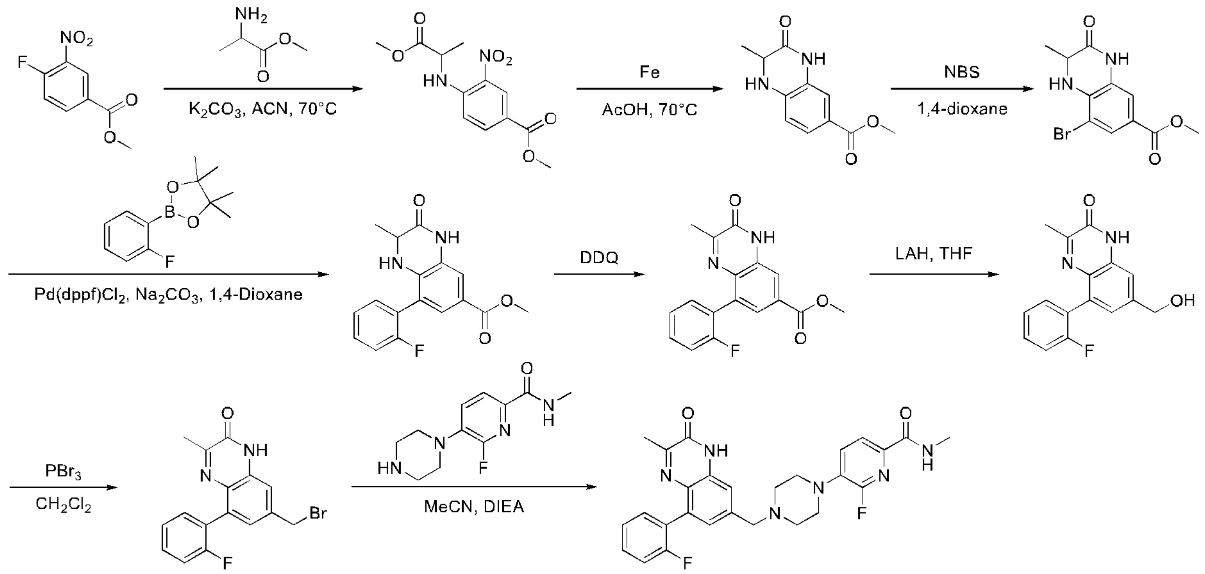


Step 1) Synthesis of methyl 4-((1-methoxy-1-oxopropyl-2-yl)amino)-3-nitrobenzoate

At room temperature, DL-alanine methyl ester hydrochloride (9.81 g, 70.31 mmol) and $K_2CO_3$ (17.35 g, 125.55 mmol) were added to a solution of methyl 4-fluoro-3-nitrobenzoate (10 g, 50.22 mmol) in acetonitrile (100 mL), and the mixture was heated to 70 °C for 12 h. After cooling, the reaction solution was filtered through diatomaceous earth. The filtrate was concentrated under reduced pressure to remove the solvent. The residue was purified by silica gel column chromatography (eluent: petroleum ether/ethyl acetate (V/V) = 3/1) to give a yellow solid product (14 g, 98.78%).
MS (ESI, pos. ion) *m/z:* 283.1 $[M+H]^+$.

Step 2) Synthesis of methyl 2-methyl-3-oxo-1,2,3,4-tetrahydroquinoxalin-6-carboxylate

At room temperature, iron powder (13.85 g, 248 mmol) was added to a solution of methyl 4-((1-methoxy-1-oxopropyl-2-yl)amino)-3-nitrobenzene (14 g, 49.60 mmol) in acetic acid (200 mL), and the mixture was heated to 80 °C and stirred for 5 h. After the reaction solution cooled, water (500 mL) was added, filtered, and the solid was dried to give a yellow solid product (8.5 g, 77.82%).
MS (ESI, pos. ion) *m/z:* 221.3 $[M+H]^+$.

Step 3) Synthesis of methyl 8-bromo-2-methyl-3-oxo-1,2,3,4-tetrahydroquinoxalin-6-carboxylate

At room temperature, NBS (4.00 g, 22.47 mmol) was added to a solution of methyl 2-methyl-3-oxo-1,2,3,4-tetrahydroquinoxalin-6-carboxylate (4.5 g, 20.43 mmol) in 1,4-dioxane (50 mL), and the reaction was stirred for 8 h at room temperature. Water (150 mL) was added to the reaction mixture, and the mixture was extracted with dichloromethane (150 mL). The organic phase was washed with saturated brine, dried over anhydrous sodium sulfate, filtered, and the filtrate was concentrated under reduced pressure to remove the solvent. The residue was purified by silica gel column chromatography (eluent: petroleum ether/ethyl acetate (V/V) = 3/1) to give a yellow solid product (4.5 g, 73.62%).
MS (ESI, pos. ion) *m/z:* 299.1, 301.1 $[M+H]^+$.

Step 4) Synthesis of methyl 8-(2-fluorophenyl)-2-methyl-3-oxo-1,2,3,4-tetrahydroquinoxalin-6-carboxylate

At room temperature, methyl 8-bromo-2-methyl-3-oxo-1,2,3,4-tetrahydroquinoxalin-6-carboxylate (0.6 g, 2.01 mmol) was mixed with 1,4-dioxane (12 mL) and water (3 mL) and then 2-fluorophenylboronic acid pinacol ester (0.37 g, 2.61 mmol), $Pd(dppf)Cl_2$ (0.074 g, 0.10 mmol) and sodium carbonate (0.43 g, 4.02 mmol) were added. After replacing $N_2$, the mixture was heated to 105 °C and reacted for 12 h. The reaction solution was cooled to room temperature, quenched with water (25 mL), extracted with ethyl acetate (50 mL*3), the organic phases were combined, washed with saturated brine, dried over anhydrous sodium sulfate, concentrated under reduced pressure to remove the solvent, and the residue was purified by silica gel column chromatography (eluent: petroleum ether/ethyl acetate (V/V) = 3/1) to give a yellow solid product (0.4 g, 63.44%).
MS (ESI, pos. ion) *m/z:* 315.2 $[M+H]^+$.

Step 5) Synthesis of methyl 8-(2-fluorophenyl)-2-methyl-3-oxo-3,4-dihydroquinoxaline-6-carboxylate

Under ice bath conditions, 2,3-dichloro-5,6-dicyanobenzoquinone (0.32 g, 1.40 mmol) was added to a solution of methyl 8-(2-fluorophenyl)-2-methyl-3-oxo-1,2,3,4-tetrahydroquinoxalin-6-carboxylate (0.40 g, 1.27 mmol) in dichloromethane (12 mL). The mixture was then slowly brought to room temperature and stirred for 3 h. The reaction mixture was quenched with saturated sodium bicarbonate solution (38 mL), stirred for 2 h, extracted with dichloromethane (25 mL*3) , and the organic phases were combined. The mixture was then washed with saturated sodium bicarbonate solution, dried over anhydrous sodium sulfate, and concentrated under reduced pressure to give an orange-yellow solid product (0.21 g, 52.84%).

MS (ESI, pos. ion) *m/z:* 313.1 $[M+H]^+$.

Step 6) Synthesis of 5-(2-fluorophenyl)-7-(hydroxymethyl)-3-methylquinoxalin-2(1*H*)-one

Under ice bath conditions, lithium aluminum hydride (0.080 g, 2.04 mmol) was added in portions to a solution of methyl 8-(2-fluorophenyl)-2-methyl-3-oxo-3,4-dihydroquinoxalin-6-carboxylate (0.21 g, 0.67 mmol) in tetrahydrofuran (16.4 mL). The mixture was stirred until no further exothermic reaction occurred, and then the reaction was allowed to proceed at room temperature for 4 h. The mixture was cooled, and water (5 mL) was added under an ice bath to quench the reaction. Then, 1 M dilute hydrochloric acid was added dropwise to adjust the pH to 3-4. The mixture was extracted with tetrahydrofuran (25 mL * 2), and the organic phases were combined. The combined organic phase was washed with saturated brine and dried over anhydrous sodium sulfate. After filtration, the solvent was removed from the filtrate under reduced pressure. The residue was purified by silica gel column chromatography (eluent: dichloromethane/methanol (V/V) = 20/1) to afford a white solid (0.11 g, 57.54%).

MS (ESI, pos. ion) *m/z:* 285.2 $[M+H]^+$.

Step 7) Synthesis of 7-(bromomethyl)-5-(2-fluorophenyl)-3-methylquinoxalin-2(1*H*)-one

Under ice bath conditions, 5-(2-fluorophenyl)-7-(hydroxymethyl)-3-methylquinoxalin-2(1*H*)-one (0.16 g, 0.56 mmol), dichloromethane (10 mL), and then phosphorus tribromide (0.056 mL, 0.59 mmol) were added dropwise. The mixture was stirred for 5 min, then moved to room temperature and reacted for another 2 h. The reaction solution was concentrated under reduced pressure to remove the solvent. The residue was washed with methyl *tert*-butyl ether (10 mL*2), dried, and yielded a yellow solid crude product (0.13 g, 96.77%), which was directly used for the next reaction.

MS (ESI, pos. ion) *m/z:* 347.1, 349.1 $[M+H]^+$.

Step 7) Synthesis of 6-fluoro-5-(4-((8-(2-fluorophenyl)-2-methyl-3-oxo-3,4-dihydroquinoxalin-6-yl)methyl)piperazin-1-yl)-*N*-methylpicolinamide

At room temperature, 6-fluoro-*N*-methyl-5-(piperazin-1-yl)pyridineamide (0.13 g, 0.55 mmol) and *N,N*-diisopropylethylamine (0.36 g, 2.75 mmol) were added to a solution of 7-(bromomethyl)-5-(2-fluorophenyl)-3-methylquinoxalin-2(1*H*)-one (0.19 g, 0.55 mmol) in acetonitrile (20 mL), and the mixture was heated to 70 °C and stirred for 3 h. The reaction solution was concentrated under reduced pressure to remove the solvent, and the residue was purified by silica gel column chromatography (eluent: dichloromethane/methanol (V/V) = 10/1) to give a yellow solid product (0.029 g, 10.50%).

MS (ESI, pos. ion) *m/z:* 505.4 $[M+H]^+$;

HRMS: calcd. for $C_{27}H_{26}F_2N_6O_2$ $[M+H]^+$: 505.2085, found: 505.2186;
$^1$H NMR (400 MHz, DMSO-$d_6$) δ (ppm) 12.34 (s, 1H), 8.36 (d, *J*= 5.7 Hz, 1H), 7.84 (d, *J* = 8.0 Hz, 1H), 7.56 (t, *J* = 9.3 Hz, 1H), 7.48 - 7.39 (m, 2H), 7.34 (s, 1H), 7.32 - 7.24 (m, 2H), 7.21 (s, 1H), 3.67 (s, 2H), 3.21 - 3.16 (m, 4H), 2.77 (d, *J*= 4.7 Hz, 3H), 2.63 - 2.58 (m, 4H), 2.29 (s, 3H).

**Example 9: Synthesis of 6-fluoro-5-(4-((8-(3-fluorophenyl)-2-methyl-3-oxo-3,4-dihydroquinoxalin-6-yl)methyl) piperazin-1-yl)-*N*-methylpicolinamide**

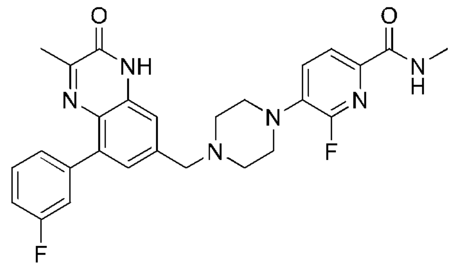

(9)

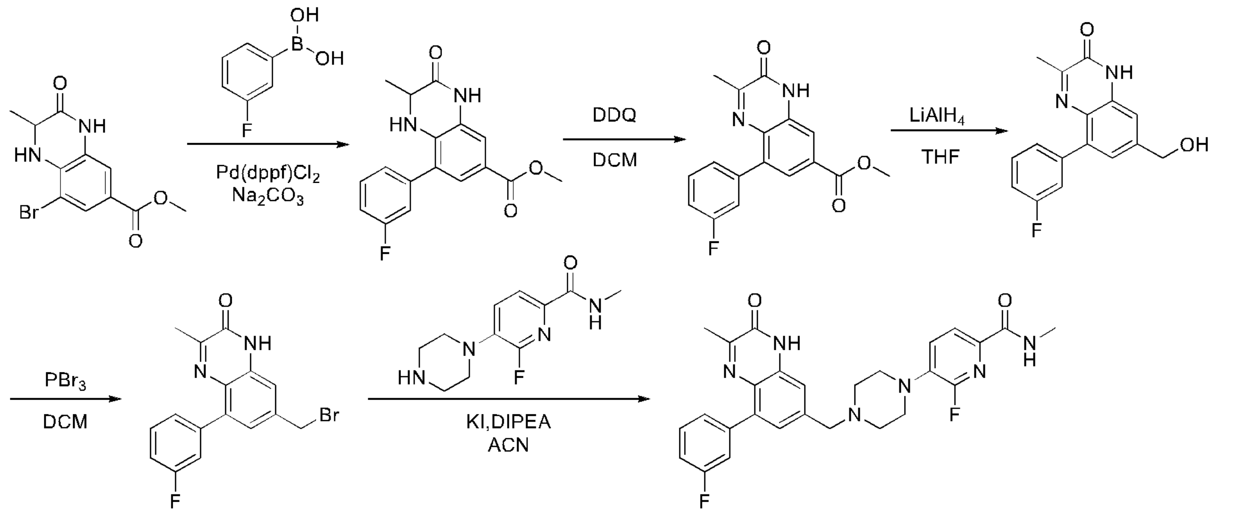


Step 1) Synthesis of methyl 8-(3-fluorophenyl)-2-methyl-3-oxo-1,2,3,4-tetrahydroquinoxalin-6-carboxylate

At room temperature, (3-fluorophenyl)boronic acid (0.45 g, 7.43 mmol), Pd(dppf)Cl$_2$ (0.18 g, 0.25 mmol), and sodium carbonate (1.06 g, 10.02 mmol) were added to a solution of methyl 8-bromo-2-methyl-3-oxo-1,2,3,4-tetrahydroquinoxalin-6-carboxylate (1.5 g, 5.01 mmol) in 1,4-dioxane (20 mL) and water (5 mL). The mixture was then purged with nitrogen and reacted at 105 °C for 10 h. The reaction solution was diluted with dichloromethane, filtered through diatomaceous earth, and the organic phase was concentrated under reduced pressure. The residue was purified by silica gel column chromatography (eluent: dichloromethane/ethyl acetate (V/V) = 90/10) to give a yellow solid product (0.65 g, 41.24%).

MS (ESI, pos. ion) *m/z:* 315.1, 316.2 [M+H]$^+$.

Step 2) Methyl 8-(3-fluorophenyl)-2-methyl-3-oxo-3,4-dihydroquinoxalin-6-carboxylate

At 0°C, 2,3-dichloro-5,6-dicyanobenzoquinone (0.56 g, 2.48 mmol) was added to a solution of methyl 8-(3-fluorophenyl)-2-methyl-3-oxo-1,2,3,4-tetrahydroquinoxalin-6-carboxylate (0.65 g, 2.07 mmol) in dichloromethane (50 mL). The reaction was monitored by TLC at room temperature for 5 h. The reaction was quenched by adding saturated sodium bicarbonate aqueous solution (10 mL), extracted with dichloromethane (50 mL*3), dried over anhydrous sodium sulfate, filtered, concentrated under reduced pressure in the organic phase, and the residue was purified by silica gel column chromatography (eluent: dichloromethane/ethyl acetate (V/V) = 9/1) to give a white solid product (0.55 g, 85.16%).

MS (ESI, pos. ion) *m/z:* 313.15 [M+H]$^+$.

Step 3) Synthesis of 5-(3-fluorophenyl)-7-(hydroxymethyl)-3-methylquinoxalin-2(1*H*)-one

At 0°C and N$_2$, lithium aluminum hydride (0.2 g, 5.28 mmol) was slowly added to a tetrahydrofuran (20 mL) solution of methyl 8-(3-fluorophenyl)-2-methyl-3-oxo-3,4-dihydroquinoxalin-6-carboxylate (0.55 g, 1.76 mmol). The mixture was stirred at 0°C for 5 min, then allowed to rise to room temperature and stirred for 3 h. After the reaction was complete, water (0.2 mL), NaOH aqueous solution (0.2 mL, 15%), and water (0.6 mL) were added sequentially at 0°C, and the mixture was stirred for 10 min. The mixture was filtered through diatomaceous earth, and the filter cake was dissolved in water and dilute hydrochloric acid (14 mL, 1 M) to dissolve the solid. The solution was then extracted with dichloromethane (50 mL * 3). The product was dried over anhydrous sodium sulfate, concentrated under reduced

pressure, and the residue was purified by silica gel column chromatography (eluent: dichloromethane/methanol (V/V) = 97/3) to give a gray solid product (0.3 g, 59.92%).

MS (ESI, pos. ion) *m/z:* 285.2 $[M+H]^+$.

Step 4) Synthesis of 7-(bromomethyl)-5-(3-fluorophenyl)-3-methylquinoxalin-2(1*H*)-one

Under ice bath conditions, phosphorus tribromide (0.15 mL, 1.59 mmol) was added dropwise to a solution of 5-(3-fluorophenyl)-7-(hydroxymethyl)-3-methylquinoxalin-2(1*H*)-one (0.3 g, 1.06 mmol) in dichloromethane (8 mL), and the mixture was stirred for 5-10 min, followed by reaction at room temperature for 3 h. The mixture was concentrated under reduced pressure, and the residue was purified by silica gel column chromatography (eluent: dichloromethane/methanol (V/V) = 98/2) to give a white solid powder (0.27 g, 73.70%).

MS (ESI, pos. ion) *m/z:* 347.15, 349.15 $[M+H]^+$.

Step 5) Synthesis of 6-fluoro-5-(4-((5-(3-fluorophenyl)-2-methyl-3-oxo-3,4-dihydroquinoxalin-6-yl)methyl)piperazin-1-yl)-*N*-methylpicolinamide

At room temperature, potassium iodide (6.5 mg, 0.0039 mmol), 6-fluoro-*N*-methyl-5-(piperazin-1-yl)pyridine-2-carboxamide (0.2 g, 0.86 mmol), and *N,N*-diisopropylethylamine (1.03 mL, 6.24 mmol) were added to a solution of 7-(bromomethyl)-5-(3-fluorophenyl)-3-methylquinoxalin-2(1*H*)-one (0.27 g, 0.78 mmol) in acetonitrile (15 mL). The mixture was stirred for 5-10 min, and then the reaction was brought to room temperature for 5 h. The mixture was concentrated under reduced pressure, and the residue was purified by silica gel column chromatography (eluent: dichloromethane/methanol (V/V) = 97/3) to give a white solid powder (0.15 g, 38.23%).

MS (ESI, pos. ion) *m/z:* 505.25 $[M+H]^+$;

HRMS: calcd. for $C_{27}H_{26}F_2N_6O_2$ $[M+H]^+$: 505.2158, found: 505.2191;

$^1$H NMR (400 MHz, DMSO-$d_6$) δ (ppm) 12.34 (s, 1H), 8.40 - 8.32 (m, 1H), 7.87 - 7.80 (m, 1H), 7.60 - 7.46 (m, 2H), 7.44 - 7.38 (m, 2H), 7.33 - 7.26 (m, 2H), 7.26 - 7.19 (m, 1H), 3.66 (s, 2H), 3.22 - 3.14 (m, 4H), 2.76 (d, *J*= 4.8 Hz, 3H), 2.64 - 2.56 (m, 4H), 2.34 (s, 3H).

**Example 10: Synthesis of 6-fluoro-5-(4-((8-(4-fluorophenyl)-2-methyl-3-oxo-3,4-dihydroquinoxalin-6-yl)methyl) piperazin-1-yl)-*N*-methylpicolinamide**

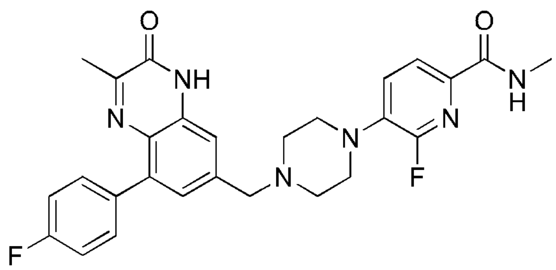

(10)

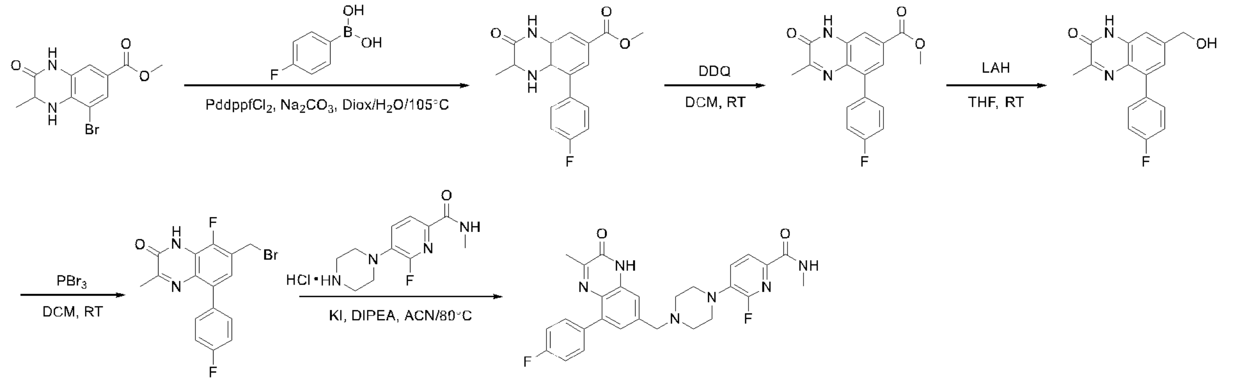

Step 1) Synthesis of methyl 8-(4-fluorophenyl)-2-methyl-3-oxo-1,2,3,4-tetrahydroguinoxalin-6-carboxylate

In a 100 mL reaction flask, methyl 8-bromo-2-methyl-3-oxo-1,2,3,4-tetrahydroquinoxaloline-6-carboxylate (0.60 g, 2.01 mmol), 4-fluorophenylboronic acid (0.37 g, 2.61 mmol), Pd(dppf)$Cl_2$ (0.074 g, 0.10 mmol), and sodium carbonate (0.43 g, 4.02 mmol) were added sequentially. The mixture was stirred and dissolved in 1,4-dioxane (12 mL) and water (3 mL). The mixture was then purged with nitrogen and heated to 105 °C overnight. Cool to room temperature, filter to remove insoluble matter, collect the organic phase, add water to separate the layers, extract with ethyl acetate (25 mL*2), combine the organic phases, wash with saturated brine, dry with anhydrous sodium sulfate, concentrate under reduced pressure, and purify the residue by silica gel column chromatography (eluent: dichloromethane/methanol (V/V) = 97/3) to give a white solid (0.383 g, 60.75%).

MS (ESI, pos. ion) *m/z:* 315.15 $[M+H]^+$.

Step 2) Synthesis of methyl 8-(4-fluorophenyl)-2-methyl-3-oxo-3,4-dihydroquinoxalin-6-carboxylate

Methyl 8-(4-fluorophenyl)-2-methyl-3-oxo-1,2,3,4-tetrahydroquinoxalin-6-carboxylate (0.38 g, 1.22 mmol) was added to a 100 mL reaction flask and stirred until dissolved in 12 mL of dichloromethane. After cooling, 2,3-dichloro-5,6-dicyanobenzoquinone (0.30 g, 1.33 mmol) was added under ice bath conditions, and the reaction was allowed to proceed at room temperature for 6 h. The solvent was removed by concentration under reduced pressure, and the solvent was quenched by adding 24 mL of saturated sodium bicarbonate aqueous solution. The mixture was stirred for 30 min, and a solid precipitated. The precipitate was collected by filtration, washed with saturated sodium bicarbonate aqueous solution, and dried under vacuum to give a brownish-yellow solid (0.30 g, 79.46%).

MS (ESI, pos. ion) *m/z:* 313.20 $[M+H]^+$.

Step 3) Synthesis of 5-(4-fluorophenyl)-7-(hydroxymethyl)-3-methylquinoxalin-2(1*H*)-one

Methyl 8-(4-fluorophenyl)-2-methyl-3-oxo-3,4-dihydroquinoxalin-6-carboxylate (0.30 g, 0.96 mmol) was added to a 100 mL reaction flask and stirred to dissolve in tetrahydrofuran (11 mL). After cooling, lithium aluminum hydride (0.106 g, 2.71 mmol) was added under ice bath conditions, and the mixture was gradually brought to room temperature and reacted overnight. After cooling, water (2.7 mL) was added under ice bath conditions to quench the reaction, and then 1 M dilute hydrochloric acid was added dropwise to adjust the pH to 3-4. The mixture was extracted with tetrahydrofuran (10 mL * 2), and the organic phases were combined, washed with saturated brine, dried over anhydrous sodium sulfate, concentrated under reduced pressure, and the residue was purified by silica gel column chromatography (eluent: dichloromethane/methanol (V/V) = 95/5) to give a white solid (0.186 g, 68.11%).

MS (ESI, pos. ion) *m/z:* 285.20 $[M+H]^+$.

Step 4) Synthesis of 7-(bromomethyl)-8-fluoro-5-(4-fluorophenyl)-3-methylquinoxalin-2(1*H*)-one

8-fluoro-5-(4-fluorophenyl)-7-(hydroxymethyl)-3-methylquinoxalin-2(1*H*)-one (0.186 g, 0.65 mmol) and dichloromethane (8.5 mL) were added to a 100 mL reaction flask. The mixture was cooled, and phosphorus tribromide (0.079 mL, 0.85 mmol) was added dropwise under ice bath conditions. The mixture was stirred until no further exothermic reaction occurred, and the reaction was allowed to proceed to room temperature for 4 h. The solvent was removed by concentration under reduced pressure to obtain a yellow crude solid, which was then directly used for the next reaction.

Step 5) Synthesis of 6-fluoro-5-(4-((8-(4-fluorophenyl)-2-methyl-3-oxo-3,4-dihydroquinoxalin-6-yl)methyl)piperazin-1-yl)-*N*-methylpicolinamide

7-(bromomethyl)-8-fluoro-5-(4-fluorophenyl)-3-methylquinoxalin-2(1*H*)-one (0.22 g, 0.63 mmol), 6-fluoro-*N*-methyl-5-(piperazin-1-yl)pyridineamide hydrochloride (0.19 g, 0.69 mmol), and potassium iodide (0.021 g, 0.13 mmol) were added to a 100 mL reaction flask and stirred to dissolve in acetonitrile (4 mL). After cooling, *N,N*-diisopropylethylamine (0.77 mL, 4.41 mmol) was added dropwise under ice bath conditions. The mixture was heated to 80 °C and reacted for 2 h. After cooling to room temperature, a solid precipitated. The filter cake was collected by filtration, washed with methyl tert-butyl ether (4 mL*3), washed with water (4 mL*3), and dried under vacuum to give a white solid (0.19 g, 59.43%).

MS (ESI, pos. ion) *m/z:* 505.30 $[M+H]^+$;

HRMS: calcd. for $C_{27}H_{26}F_2N_6O_2$ $[M+H]^+$: 505.2158, found: 505.2160;
$^1H$ NMR (400 MHz, DMSO-$d_6$) $\delta$ (ppm) 12.36 (s, 1H), 8.40 (d, *J*= 4.8 Hz, 1H), 7.84 (d, *J*= 7.9 Hz, 1H), 7.66 - 7.53 (m, 3H), 7.31 - 7.25 (m, 4H), 3.66 (s, 2H), 3.22 - 3.13 (m, 4H), 2.77 (d, *J* = 4.6 Hz, 3H), 2.63 - 2.55 (m, 4H), 2.34 (s, 3H).

**Example 11: Synthesis of 6-fluoro-*N*-methyl-5-(4-((2-methyl-3-oxo-8-(pyridin-3-yl)-3,4-dihydroquinoxalin-6-yl) methyl)piperazin-1-yl)picolinamide**

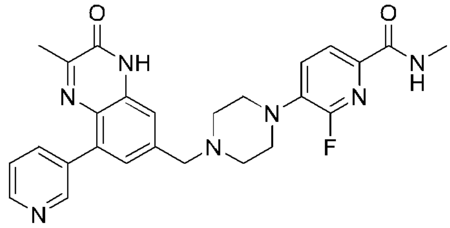

(11)

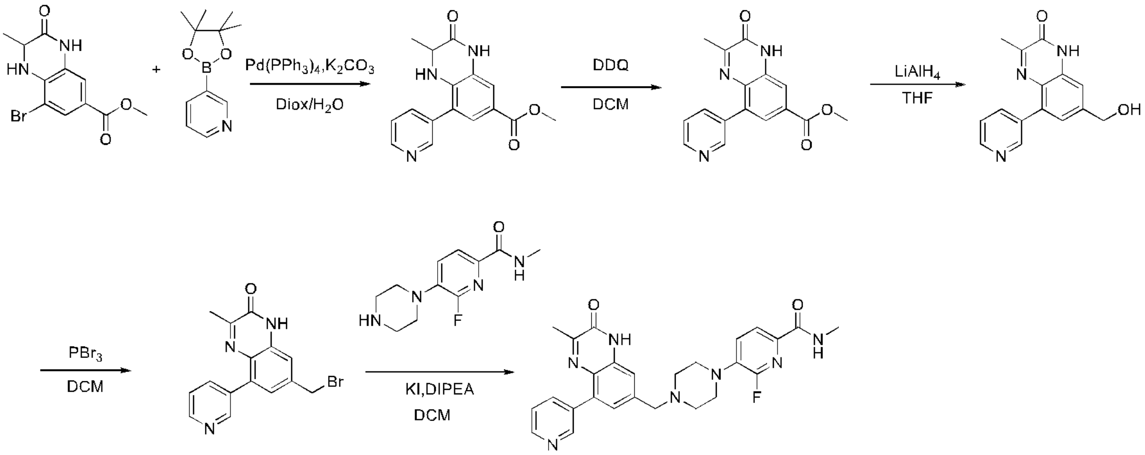


Step 1) Synthesis of methyl 2-methyl-3-oxo-8-(pyridin-3-yl)-1,2,3,4-tetrahydroquinoxalin-6-carboxylate

At room temperature, methyl 8-bromo-2-methyl-3-oxo-1,2,3,4-tetrahydroquinoxalin-6-carboxylate (1 g, 3.34 mmol) was mixed with 1,4-dioxane (20 mL) and water (5 mL) and then 3-pyridineboronic acid pinacol ester (0.89 g, 4.34 mmol), $Pd(PPh_3)_4$ (0.19 g, 0.17 mmol) and $K_2CO_3$ (0.92 g, 6.68 mmol), and the mixture was purged with nitrogen and the reaction was carried out at 110 °C for 8 h. The reaction solution was diluted with dichloromethane, filtered through diatomaceous earth, concentrated under reduced pressure in the organic phase, and the residue was purified by silica gel column chromatography (eluent: dichloromethane/methanol (V/V) = 97/3) to give a light red solid product (0.55 g, 55.33%).

MS (ESI, pos. ion) *m/z:* 298.25 $[M+H]^+$.

Step 2) Synthesis of methyl 2-methyl-3-oxo-8-(pyridin-3-yl)-3,4-dihydroquinoxalin-6-carboxylate

2,3-dichloro-5,6-dicyanobenzoquinone (0.63 g, 2.78 mmol) was added to a solution of methyl 2-methyl-3-oxo-8-(pyridin-3-yl)-1,2,3,4-tetrahydroquinoxalin-6-carboxylate (0.55 g, 1.85 mmol) in dichloromethane (30 mL) at 0 °C. The reaction was monitored by TLC at room temperature for 5 h. The reaction was quenched by adding saturated sodium bicarbonate aqueous solution, extracted with dichloromethane (50 ml*3), dried over anhydrous sodium sulfate, filtered, concentrated under reduced pressure, and the residue was purified by silica gel column chromatography (eluent: dichloromethane/ethyl acetate (V/V) = 9/1) to give a white solid product (0.44 g, 80.55%).

MS (ESI, pos. ion) *m/z:* 296.15 $[M+H]^+$.

Step 3) Synthesis of 7-(hydroxymethyl)-3-methyl-5-(pyridin-3-yl)quinoxalin-2(1*H*)-one

At 0 °C and $N_2$, lithium aluminum hydride (0.17 g, 4.47 mmol) was slowly added to a solution of methyl 2-methyl-3-oxo-8-(pyridin-3-yl)-3,4-dihydroquinoxalin-6-carboxylate (0.44 g, 1.49 mmol) in tetrahydrofuran (20 mL). The mixture was stirred at 0 °C for 5 min, then allowed to rise to room temperature and stirred for 3 h. After the reaction was complete, water (0.2 mL), 15% NaOH aqueous solution (0.2 mL), and water (0.6 mL) were added at 0 °C, and the mixture was stirred for 10 min. The mixture was filtered through diatomaceous earth and washed with dichloromethane (50 mL) and tetrahydrofuran (50 mL). The anhydrous sodium sulfate was dried and then evaporated to dryness. The residue was purified by silica gel

column chromatography (eluent: dichloromethane/methanol (V/V) = 97/3) to give a gray solid product (0.19 g, 47.71%).

MS (ESI, neg. ion) *m/z:* 266.15 [M-H]$^-$.

Step 4) Synthesis of 7-(bromomethyl)-3-methyl-3-(pyridin-3-yl)quinoxalin-2(1*H*)-one

Under ice bath conditions, phosphorus tribromide (0.1 mL, 1.06 mmol) was added dropwise to a solution of 7-(hydroxymethyl)-3-methyl-5-(pyridin-3-yl)quinoxalin-2(1*H*)-one (0.19 g, 0.71 mmol) in dichloromethane (20 mL) and stirred for 5-10 min. The mixture was then allowed to react at room temperature for 3.5 h. The solution was then evaporated under vacuum and directly added to the next reaction step.

Step 4) Synthesis of 6-fluoro-*N*-methyl-5-(4-((2-methyl-3-oxo-8-(pyridin-3-yl)-3,4-dihydroquinoxalin-6-yl)methyl)piperazin-1-yl)picolinamide

At room temperature, potassium iodide (4.8 mg, 0.029 mmol), 6-fluoro-*N*-methyl-5-(piperazin-1-yl)pyridine-2-carboxamide (0.17 g, 0.70 mmol), and *N*,*N*-diisopropylethylamine (0.96 mL, 5.8 mmol) were added to a solution of 7-(bromomethyl)-3-methyl-5-(piperazin-1-yl)pyridine-2-carboxamide (0.17 g, 0.70 mmol) in acetonitrile (10 mL). The mixture was stirred for 5-10 min, and then allowed to react at room temperature for 5 h. The mixture was evaporated to dryness under vacuum, and the residue was purified by silica gel column chromatography (eluent: dichloromethane/-methanol (V/V) = 97/3) to give a white solid powder (0.034 g, 12.12%).

MS (ESI, pos. ion) *m/z:* 488.20 [M+H]$^+$;

HRMS: calcd. for $C_{26}H_{26}FN_7O_2$ [M+H]$^+$: 488.5469, found: 488.2227;

$^1$H NMR (400 MHz, DMSO-$d_6$) δ (ppm) 12.36 (s, 1H), 8.77 (dd, *J* = 2.1, 1.0 Hz, 1H), 8.57 (dd, *J* = 4.8, 1.6 Hz, 1H), 8.38 (q, *J* = 4.9 Hz, 1H), 8.02 - 7.95 (m, 1H), 7.85 - 7.80 (m, 1H), 7.56 (dd, *J* = 10.6, 8.1 Hz, 1H), 7.49 (dd, *J* = 7.9, 4.8 Hz, 1H), 7.36 - 7.28 (m, 2H), 3.67 (s, 2H), 3.21 - 3.15 (m, 4H), 2.75 (d, *J* = 4.8 Hz, 3H), 2.63 - 2.56 (m, 4H), 2.33 (s, 3H).

**Example 12: Synthesis of 2-fluoro-*N*-methyl-1'-((2-methyl-3-oxo-8-(prop-1-yn-1-yl)-3,4-dihydroquinoxalin-6-yl)methyl)-1',2',3',6'-tetrahydro-[3,4'-bipyridine]-6-carboxamide**

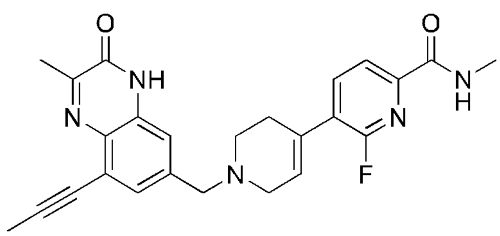

(12)

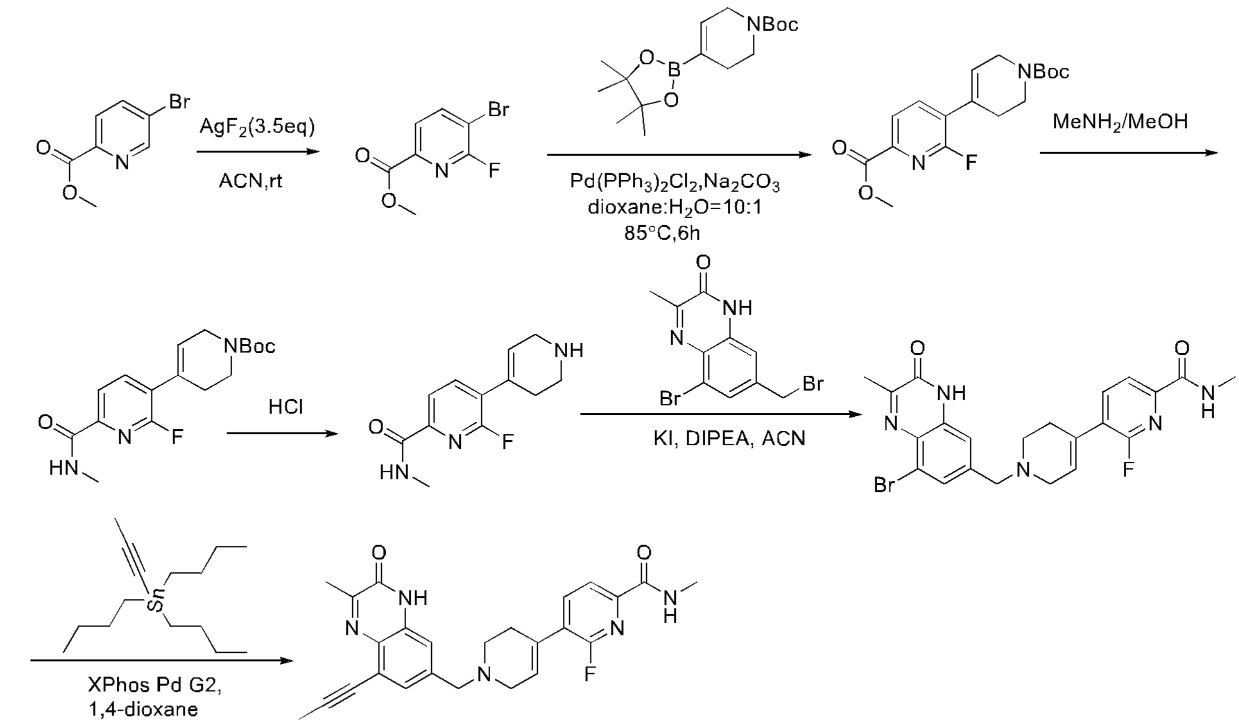


Step 1) Synthesis of methyl 5-bromo-6-fluoropicolinate

Methyl 5-bromopyridine-2-carboxylate (2.00 g, 9.26 mmol), $AgF_2$ (4.73 g, 32.41 mmol) and acetonitrile (30 mL) were added sequentially to a sealed tube and reacted at room temperature for 48 h. The mixture was filtered, and the filter cake was washed with EA (20 mL). The mixture was concentrated under reduced pressure, and the concentrated residue was purified by silica gel column chromatography (eluent: petroleum ether/ethyl acetate (V/V) = 4/1) to give a white solid (0.57 g, 26%).

MS (ESI, pos. ion) *m/z:* 234.0, 236.0 $[M+H]^+$.

Step 2) Synthesis of 1'-*tert*-butyl-6-methyl-2-fluoro-5',6'-dihydro-[3,4'-bipyridine]-1',6(2'*H*)-dicarboxylate

Methyl 5-bromo-6-fluoropyridine-2-carboxylate (0.5 g, 2.14 mmol), *tert*-butyl 4-(4,4,5,5-tetramethyl-1,3,2-dioxoboronyl-2-yl)-3,6-dihydropyridine-1(2*H*)-carboxylate (0.79 g, 2.57 mmol), sodium carbonate (0.91 g, 8.56 mmol), bis(triphenylphosphine)palladium(II) chloride (0.15 g, 0.21 mmol) and $H_2O$ (2 mL) were added sequentially to 1,4-dioxane (20 mL). Under nitrogen protection, the mixture was heated to 85 °C and reacted for 16 h. The reaction solution was concentrated under reduced pressure, and water (50 mL) and EA (80 mL) were added. The mixture was extracted and separated. The organic phase was washed with saturated brine, dried over anhydrous sodium sulfate, filtered, concentrated under reduced pressure, and the residue was purified by silica gel column chromatography (eluent: petroleum ether/ethyl acetate (V/V) = 4/1) to give a pale yellow solid (0.48 g, 66.8%).

MS (ESI, pos. ion) *m/z:* 337.4 $[M+H]^+$.

Step 3) Synthesis of *tert*-butyl-2-fluoro-6-(methylcarbamoyl)-5',6'-dihydro-[3,4'-bipyridine]-1'(2'*H*)-carboxylate

Methyl 5-(1-(*tert*-butoxycarbonyl)-1,2,3,6-tetrahydropyridin-4-yl)-6-fluoropyridine-2-carboxylate (0.54 g, 1.61 mmol) and methanol (5 mL) were added to a methanol solution of methylamine (10 mL, 2 M), and the reaction was carried out at 50 °C for 12 h in a sealed tube. The reaction solution was concentrated under reduced pressure, and the residue was purified by silica gel column chromatography (eluent: petroleum ether/ethyl acetate (V/V) = 4/1) to give a colorless liquid (0.38 g, 70.6%).

MS (ESI, pos. ion) *m/z:* 336.2 $[M+H]^+$.

Step 4) Synthesis of 2-fluoro-*N*-methyl-1',2',3',6'-tetrahydro-[3,4'-bipyridine]-6-carboxamide

*tert*-butyl 4-(2-fluoro-6-(methylcarbamoyl)pyridin-3-yl)-1,2,3,6-tetrahydropyridine-1-carboxylate (0.35 g, 1.04 mmol) was added to EtOAc (10 mL), followed by dropwise addition of (1 mL, 4 M) of ethyl acetate solution of HCl. The

reaction mixture was reacted at room temperature for 5 h. The reaction solution was concentrated under reduced pressure to give a brown oily product (0.21 g, 85.5%).

MS (ESI, pos. ion) *m/z:* 236.3 $[M+H]^+$.

Step 5) Synthesis of 1'-((8-bromo-2-methyl-3-oxo-3,4-dihydroquinoxalin-6-yl)methyl)-2-fluoro-*N*-methyl-1',2',3',6'-tetrahydro-[3,4'-bipyridine]-6-carboxamide

At room temperature, 2-fluoro-*N*-methyl-1',2',3',6'-tetrahydro-[3,4'-bipyridine]-6-carboxamide (0.53 g, 2.22 mmol), *N*,*N*-diisopropylethylamine (3.06 g, 23.68 mmol) and potassium iodide (0.012 g, 0.074 mmol) were added to a solution of 5-bromo-7-(bromomethyl)-3-methylquinoxalin-2(1*H*)-one (0.49 g, 1.48 mmol, refer to Step 3 of Example 7) in acetonitrile (20 mL). The reaction mixture was heated to 80 °C for 3 h. The solvent was removed by concentration under reduced pressure, and the residue was purified by silica gel column chromatography (eluent: dichloromethane/methanol (V/V) = 20/1) to give a yellow solid product (0.32 g, 44.58%).

MS (ESI, pos. ion) *m/z:* 486.30, 488.3 $[M+H]^+$.

Step 6) Synthesis of 2-fluoro-*N*-methyl-1'-((2-methyl-3-oxo-8-(prop-1-yn-1-yl)-3,4-dihydroquinoxalin-6-yl)methyl)-1',2',3',6'-tetrahydro-[3,4'-bipyridine]-6-carboxamide

At room temperature, tributyl(prop-1-yn-1-yl)stannane (0.18 g, 0.56 mmol) and Xphos-Pd-G2 (0.037 g, 0.046 mmol) were added to a solution of 1'-((8-bromo-2-methyl-3-oxo-3,4-dihydroquinoxalin-6-yl)methyl)-2-fluoro-N-methyl-1',2',3',6'-tetrahydro-[3,4'-bipyridine]-6-carboxamide (0.15 g, 0.31 mmol) in 1,4-dioxane (20 mL). After the system was purged with $N_2$, it was heated to 90°C and stirred for 12 hours. Upon completion, the reaction mixture was allowed to cool to room temperature and then filtered through diatomaceous earth. The filtrate was concentrated under reduced pressure to remove the solvent, and the resulting residue was purified by silica gel column chromatography (eluent: dichloromethane/methanol (V/V) = 20/1) to give a yellow solid product (0.075 g, 54.58%).

MS (ESI, pos. ion) *m/z:* 446.5 $[M+H]^+$;

HRMS: calcd. for $C_{25}H_{24}FN_5O_2$ $[M+H]^+$: 446.1914, found: 446.2001;
$^1H$ NMR (400 MHz, DMSO-$d_6$) $\delta$ (ppm) 12.31 (s, 1H), 8.67 - 8.61 (m, 1H), 8.09 (t, *J* = 8.7 Hz, 1H), 7.93 (d, *J* = 7.7 Hz, 1H), 7.31 (s, 1H), 7.24 (s, 1H), 6.26 (s, 1H), 3.64 (s, 2H), 3.31 - 3.25 (m, 2H), 3.16 - 3.12 (m, 2H), 2.80 (d, *J*= 4.8 Hz, 3H), 2.70 - 2.66 (m, 2H), 2.42 (s, 3H), 2.14 (s, 3H).

**Example 13: Synthesis of 5-(4-((8-(difluoromethoxy)-2-methyl-3-oxo-3,4-dihydroquinoxalin-6-yl)methyl)piperazin-1-yl)-6-fluoro-*N*-methylpicolinamide**

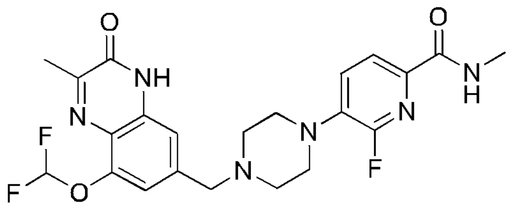

(13)

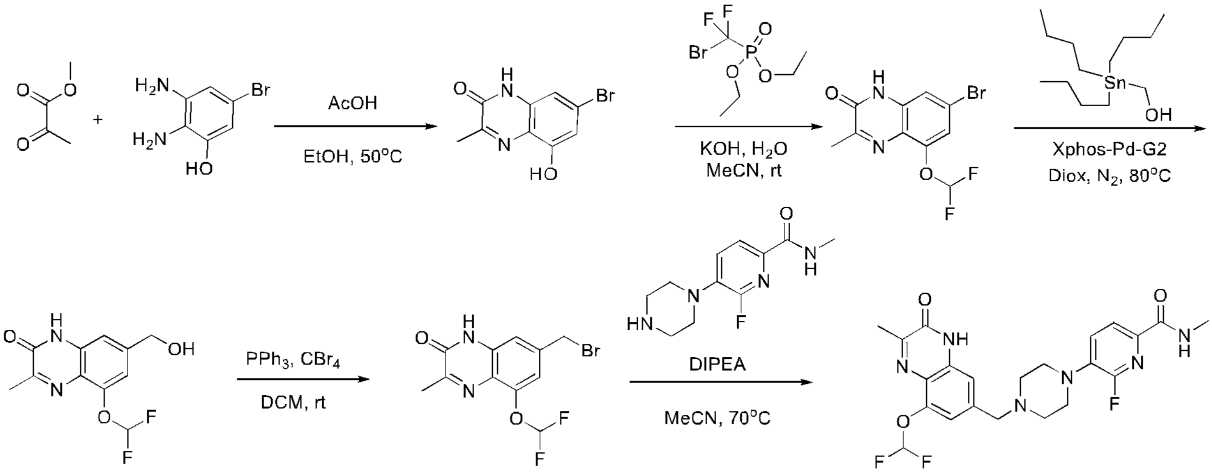


Step 1) Synthesis of 7-bromo-5-hydroxy-3-methylquinoxalin-2(1*H*)-one

2,3-Diamino-5-bromophenol (100 mg, 0.49 mmol), methyl 2-oxopropionate (75 mg, 0.73 mmol), and acetic acid (29 mg, 0.49 mmol) were added to an ethanol solution (6 mL). After addition, the reaction mixture was reacted at 50 °C for 8 h. The reaction solution was concentrated under reduced pressure, and the residue was purified by silica gel column chromatography (eluent: dichloromethane/ethyl acetate (V/V) = 3/1) to give a yellow solid (50 mg, 40%).

MS (ESI, pos. ion) *m/z:* 255.1 $[M+H]^+$.

Step 2) Synthesis of 7-bromo-5-(difluoromethoxy)-3-methylquinoxalin-2(1*H*)-one

At 0°C, a solution of KOH (881 mg, 15.70 mmol) in water (6 mL) was added to a solution of 7-bromo-5-hydroxy-3-methylquinoxalin-2(1*H*)-one (400 mg, 1.57 mmol) in acetonitrile (16 mL), followed by the addition of diethyl (bromodifluoromethyl)phosphonate (2.10 g, 7.85 mmol). The reaction mixture was allowed to react at room temperature for 16 h. The reaction solution was diluted with ethyl acetate (30 mL) and water (20 mL), extracted, and separated. The upper organic phase was dried over anhydrous $Na_2SO_4$, filtered, concentrated under reduced pressure, and the residue was purified by silica gel column chromatography (eluent: petroleum ether/ethyl acetate (V/V) = 2/1) to give a white solid (100 mg, 21%).

MS (ESI, pos. ion) *m/z:* 305.0 $[M+H]^+$.

Step 3) Synthesis of 5-(difluoromethoxy)-7-(hydroxymethyl)-3-methylquinoxalin-2(1*H*)-one

7-bromo-5-(difluoromethoxy)-3-methylquinoxalin-2(1*H*)-one (100 mg, 0.33 mmol), (tributyltin)methanol (127 mg, 0.40 mmol), and Xphos-Pd-G2 (26 mg, 0.03 mmol) were added to 1,4-dioxane (4 mL) under nitrogen protection and reacted at 80 °C for 3 h. The reaction solution was concentrated under reduced pressure, and the residue was purified by silica gel column chromatography (eluent: dichloromethane/methanol (V/V) = 20/1) to give a pale yellow solid (50 mg, 60%).

MS (ESI, pos. ion) *m/z:* 257.1 $[M+H]^+$.

Step 4) Synthesis of 7-(bromomethyl)-5-(difluoromethoxy)-3-methylquinoxalin-2(1*H*)-one

At 0 °C, $CBr_4$ (133 mg, 0.40 mmol) was added to 5-(difluoromethoxy)-7-(hydroxymethyl)-3-methylquinoxalin-2(1*H*)-one (50 mg, 0.20 mmol) and $PPh_3$ (105 mg, 0.40 mmol) in dichloromethane (3 mL). After addition, the reaction mixture was allowed to react at room temperature for 4 h. The reaction solution was concentrated under reduced pressure, and the residue was purified by silica gel column chromatography (eluent: petroleum ether/ethyl acetate (V/V) = 1/1) to give a white solid (40 mg, 64%).

MS (ESI, pos. ion) *m/z:* 319.1 $[M+H]^+$.

Step 5) Synthesis of 5-(4-((8-(difluoromethoxy)-2-methyl-3-oxo-3,4-dihydroquinoxalin-6-yl)methyl)piperazin-1-yl)-6-fluoro-*N*-methylpicolinamide

7-(bromomethyl)-5-(difluoromethoxy)-3-methylquinoxalin-2(1*H*)-one (40 mg, 0.13 mmol), 6-fluoro-*N*-methyl-5-(piperazin-1-yl)pyridineamide (40 mg, 0.17 mmol), and *N,N*-diisopropylethylamine (67 mg, 0.52 mmol) were

added sequentially to acetonitrile (3 mL), and the reaction was carried out at 70 °C for 3 h. The reaction solution was concentrated under reduced pressure, diluted with dichloromethane (50 mL), washed with water (30 mL) and saturated NaCl solution (30 mL), dried over anhydrous $Na_2SO_4$, concentrated under reduced pressure, and the residue was purified by silica gel column chromatography (eluent: dichloromethane/methanol (V/V) = 20/1) to give a pale yellow solid (40 mg, 67%).

MS (ESI, pos. ion) *m/z:* 477.4 $[M+H]^+$;

HRMS: calcd. for $C_{22}H_{23}F_3N_6O_3$ $[M+H]^+$: 477.1784, found: 477.1880;

$^1$H NMR (400 MHz, DMSO-$d_6$) δ (ppm) 10.57 (s, 1H), 8.00 (d, *J* = 8.2 Hz, 1H), 7.50 (d, *J*= 4.4 Hz, 1H), 7.34 - 7.28 (m, 1H), 7.19 (s, 1H), 7.11 (s, 1H), 6.99 (t, *J* = 74.7 Hz, 1H), 3.65 (s, 2H), 3.29 - 3.19 (m, 4H), 3.00 (d, *J*= 4.9 Hz, 3H), 2.72 - 2.64 (m, 4H), 2.63 (s, 3H).

**Example 14: 5-(4-((8-(difluoromethoxy)-5-fluoro-2-methyl-3-oxo-3,4-dihydroquinoxalin-6-yl)methyl)piperazin-1-yl)-6-fluoro-*N*-methylpicolinamide**

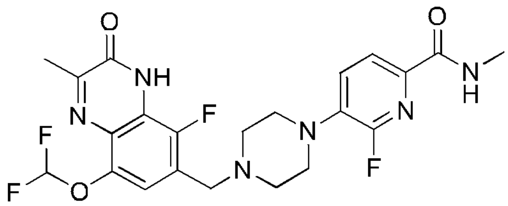

(14)

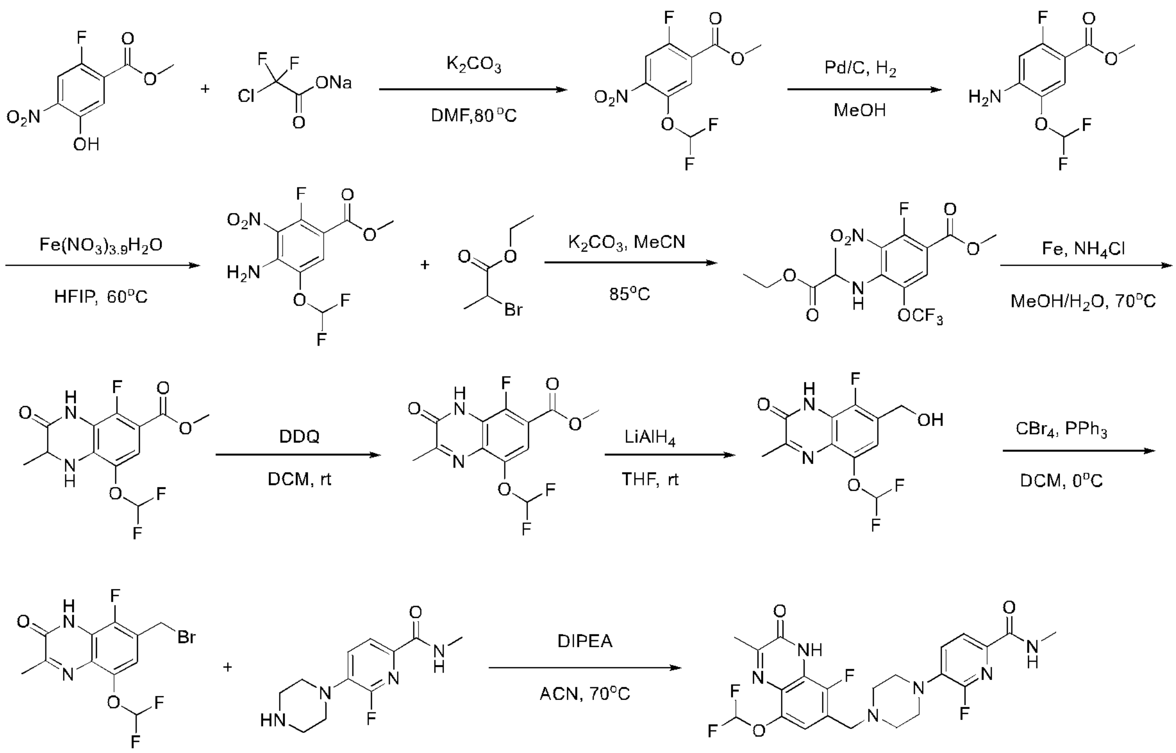


Step 1) Synthesis of methyl 5-(difluoromethoxy)-2-fluoro-4-nitrobenzoate

Methyl 2-fluoro-5-hydroxy-4-nitrobenzoate (10 g, 46.48 mmol), sodium 2-chloro-2,2-difluoroacetate (9.21 g, 60.42 mmol), and $K_2CO_3$ (7.07 mg, 51.13 mmol) were added to DMF (100 mL) solution and reacted at 80 °C for 22 h. The reaction solution was diluted with EtOAc (400 mL), washed with water (200 mL × 3), washed with saturated NaCl solution (200 mL), dried over anhydrous $Na_2SO_4$, filtered, concentrated under reduced pressure, and the residue was purified by silica gel column chromatography (PE/EtOAc (v/v) = 6/1) to give a pale yellow solid (4.0 g, 32%).

MS (ESI, pos. ion) *m/z:* 266.1 $[M+H]^+$.

Step 2) Synthesis of methyl 4-amino-5-(difluoromethoxy)-2-fluorobenzoate

Pd/C (600 mg, wt 10%) was added to a MeOH (40 mL) solution of methyl 5-(difluoromethoxy)-2-fluoro-4-nitrobenzoate (6.0 g, 22.63 mmol) to displace $H_2$, and the reaction was carried out at room temperature for 22 h. The reaction solution was filtered through diatomaceous earth, the filtrate was concentrated under reduced pressure, and the residue was purified by silica gel column chromatography (PE/EtOAc (v/v) = 4/1) to give a pale yellow solid (4.6 g, 86%).
MS (ESI, pos. ion) *m/z:* 236.1 $[M+H]^+$.

Step 3) Synthesis of methyl 4-amino-5-(difluoromethoxy)-2-fluoro-3-nitrobenzene

Methyl 4-amino-5-(difluoromethoxy)-2-fluorobenzoate (2.0 g, 8.50 mmol) and $Fe(NO_3)_3{\bullet}9H_2O$ (3.43 g, 8.50 mmol) were added to hexafluoroisopropanol (17 mL) and reacted at 60 °C for 24 h. The reaction solution was concentrated under reduced pressure, and the residue was purified by silica gel column chromatography (PE/EtOAc (v/v) = 3/1) to give a yellow solid (900 mg, 38%).
MS (ESI, pos. ion) *m/z:* 281.0 $[M+H]^+$.

Step 4) Synthesis of methyl 4-((1-ethoxy-1-oxopropyl-2-yl)amino)-2-fluoro-3-nitro-5-(trifluoromethoxy)benzoate

4-amino-5-(difluoromethoxy)-2-fluoro-3-nitrobenzoic acid (400 mg, 1.43 mmol), ethyl 2-bromopropionate (777 mg, 4.29 mmol), and $K_2CO_3$ (395 mg, 2.86 mmol) were added to ACN (10 mL) and reacted at 85 °C for 16 h. The reaction solution was filtered through diatomaceous earth, the filtrate was concentrated under reduced pressure, and the residue was purified by silica gel column chromatography (PE/EtOAc (v/v) = 4/1) to give a yellow liquid (150 mg, 27%).
MS (ESI, pos. ion) *m/z:* 399.1 $[M+H]^+$;

Step 5) Synthesis of methyl 8-(difluoromethoxy)-5-fluoro-2-methyl-3-oxo-1,2,3,4-tetrahydroquinoxaloline-6-carboxylic acid

4-((1-ethoxy-1-oxopropyl-2-yl)amino)-2-fluoro-3-nitro-3-(trifluoromethoxy)benzoate (150 mg, 0.41 mmol), Fe (137 mg, 2.46 mmol), and $NH_4Cl$ (132 mg, 2.46 mmol) were added to a mixture of MeOH (4 mL) and water (1 mL), and reacted at 70 °C for 4 h. The reaction solution was filtered through diatomaceous earth, the filtrate was concentrated under reduced pressure, and EtOAc (40 mL) and water (20 mL) were added for extraction and separation. The organic phase was washed with saturated NaCl solution (20 mL), dried over anhydrous $Na_2SO_4$, filtered, concentrated under reduced pressure, and the residue was purified by silica gel column chromatography (DCM/EtOAc (v/v) = 4/1) to give a white solid (84 mg, 67%).
MS (ESI, pos. ion) *m/z:* 305.1 $[M+H]^+$;

Step 6) Synthesis of methyl 8-(difluoromethoxy)-5-fluoro-2-methyl-3-oxo-3,4-dihydroquinoxalin-6-carboxylate

DDQ (117 mg, 0.52 mmol) was added to a DCM (6 mL) solution of methyl 8-(difluoromethoxy)-5-fluoro-2-methyl-3-oxo-1,2,3,4-tetrahydroquinoxalin-6-carboxylate (130 mg, 0.43 mmol). After addition, the reaction mixture was allowed to react at room temperature for 14 h. The reaction solution was concentrated under reduced pressure, diluted with water (20 mL), and then saturated $NaHCO_3$ solution (20 mL) was added. The mixture was filtered to obtain a pale yellow solid (100 mg, 77%).
MS (ESI, pos. ion) *m/z:* 303.1 $[M+H]^+$;

Step 7) Synthesis of 5-(difluoromethoxy)-8-fluoro-7-(hydroxymethyl)-3-methylquinoxalin-2(1*H*)-one

At 0°C, $LiAlH_4$ (28 mg, 0.66 mmol) was added to a THF (4 mL) solution of methyl 8-(difluoromethoxy)-5-fluoro-2-methyl-3-oxo-3,4-dihydroquinoxalin-6-carboxylate (100 mg, 0.33 mmol). After addition, the reaction was allowed to proceed at room temperature for 3 h. The reaction was quenched with MeOH (10 mL), diluted with DCM (50 mL), filtered through diatomaceous earth, and the filtrate was concentrated under reduced pressure. The reaction solution was also concentrated under reduced pressure, and the residue was purified by silica gel column chromatography (DCM/MeOH (v/v) = 20/1) to give a white solid (40 mg, 44%).
MS (ESI, pos. ion) *m/z:* 275.1 $[M+H]^+$;

Step 8) Synthesis of 7-(bromomethyl)-5-(difluoromethoxy)-8-fluoro-3-methylquinoxalin-2(1*H*)-one

At 0 °C, $CBr_4$ (99 mg, 0.30 mmol) was added to DCM (4 mL) containing 5-(difluoromethoxy)-8-fluoro-7-(hydroxymethyl)-3-methylquinoxalin-2(1*H*)-one (40 mg, 0.15 mmol) and $PPh_3$ (79 mg, 0.30 mmol), and the reaction was carried out at 0 °C for 6 h. The reaction solution was concentrated under reduced pressure, and the residue was purified by silica gel column chromatography (PE/EtOAc (v/v) = 1/1) to give a white solid (30 mg, 61%).

MS (ESI, pos. ion) *m/z:* 337.0 $[M+H]^+$;

Step 9) Synthesis of 5-(4-((8-(difluoromethoxy)-5-fluoro-2-methyl-3-oxo-3,4-dihydroquinoxalin-6-yl)methyl)piperazin-1-yl)-6-fluoro-*N*-methylpicolinamide

7-(bromomethyl)-5-(difluoromethoxy)-8-fluoro-3-methylquinoxalin-2(1*H*)-one (30 mg, 0.09 mmol), 6-fluoro-*N*-methyl-5-(piperazin-1-yl)picolinamide (32 mg, 0.13 mmol), and DIPEA (35 mg, 0.28 mmol) were added sequentially to ACN (4 mL), and the reaction was carried out at 70 °C for 2 h. The reaction solution was concentrated under reduced pressure, diluted with DCM (50 mL), washed with water (30 mL) and saturated NaCl solution (30 mL), dried over anhydrous $Na_2SO_4$, concentrated under reduced pressure, and the residue was purified by silica gel column chromatography (DCM/MeOH (v/v) = 20/1) to give a pale white solid (35 mg, 80%).

MS (ESI, pos. ion) *m/z:* 495.2 $[M+H]^+$;

$^1$H NMR (400 MHz, DMSO-$d_6$) δ 12.63 (s, 1H), 8.40 (d, *J* = 4.9 Hz, 1H), 7.84 (d, *J* = 8.0 Hz, 1H), 7.56 (dd, *J* = 10.4, 8.3 Hz, 1H), 7.29 (t, *J* = 74.4 Hz, 1H), 7.13 (d, *J* = 5.4 Hz, 1H), 3.70 (s, 2H), 3.17 (s, 4H), 2.76 (d, *J* = 4.7 Hz, 3H), 2.60 (s, 4H), 2.44 (s, 3H).

**Example 15 1'-((8-(difluoromethoxy)-2-methyl-3-oxo-3,4-dihydroquinoxalin-6-yl)methyl)-2-fluoro-*N*-methyl-1',2',3',6'-tetrahydro-[3,4'-bipyridine]-6-carboxamide**

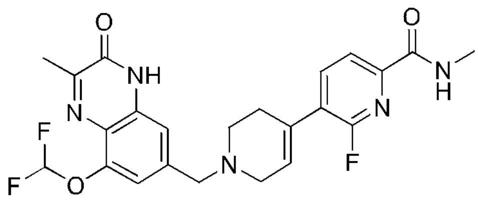

(15)

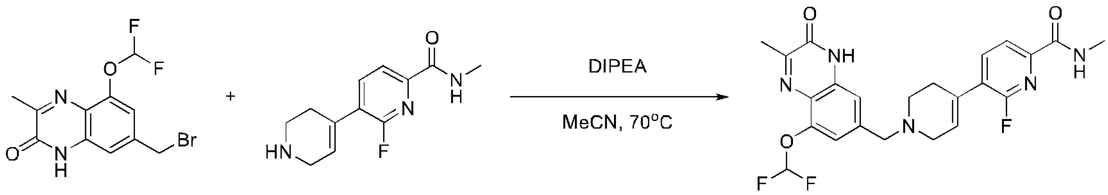


7-(bromomethyl)-5-(difluoromethoxy)-3-methylquinoxalin-2(1*H*)-one (160 mg, 0.50 mmol), 2-fluoro-*N*-methyl-1',2',3',6'-tetrahydro-[3,4'-bipyridine]-6-carboxamide (153 mg, 0.65 mmol), and DIPEA (323 mg, 0.65 mmol) were added sequentially to MeCN (8 mL), and the reaction was carried out at 70 °C for 3 h. The reaction solution was concentrated under reduced pressure, diluted with DCM (60 mL), washed with water (30 mL) and saturated NaCl solution (30 mL), dried over anhydrous $Na_2SO_4$, concentrated under reduced pressure, and the residue was purified by silica gel column chromatography (DCM/MeOH (v/v) = 20/1) to give a pale yellow solid (140 mg, 59%).

MS (ESI, pos. ion) m/z: 474.1 $[M+H]^+$;

1H NMR (400 MHz, DMSO-$d_6$) δ 12.43 (s, 1H), 8.66 (dd, J = 9.3, 4.5 Hz, 1H), 8.09 (dd, J = 9.8, 7.8 Hz, 1H), 7.92 (dd, J = 7.9, 1.5 Hz, 1H), 7.38 (t, J = 74.4 Hz, 1H), 7.17 (s, 1H), 7.06 (s, 1H), 6.27 (s, 1H), 3.68 (s, 2H), 3.16 (s, 2H), 2.79 (d, J = 4.8 Hz, 3H), 2.67 (t, J = 5.1 Hz, 2H), 2.52 (s, 2H), 2.41 (s, 3H).

**Example 16 1'-((8-(difluoromethoxy)-5-fluoro-2-methyl-3-oxo-3,4-dihydroquinoxalin-6-yl)methyl)-2-fluoro-*N*-methyl-1',2',3',6'-tetrahydro-[3,4'-bipyridine]-6-carboxamide**

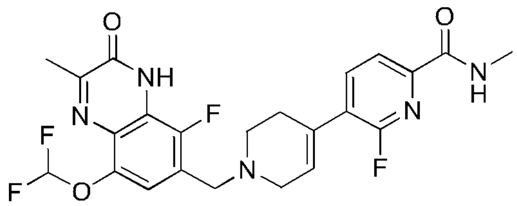

(16)

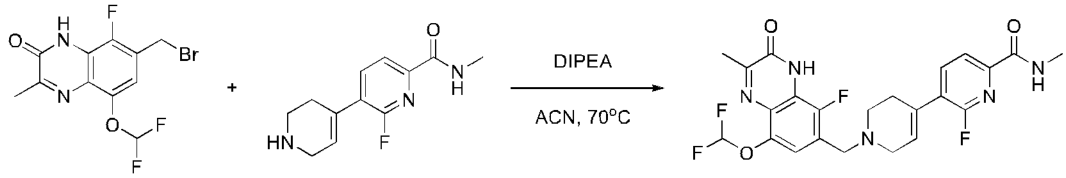


Step 1) Synthesis of 1'-((8-(difluoromethoxy)-5-fluoro-2-methyl-3-oxo-3,4-dihydroquinoxalin-6-yl)methyl)-2-fluoro-N-methyl-1',2',3',6'-tetrahydro-[3,4'-bipyridine]-6-carboxamide

7-(bromomethyl)-5-(difluoromethoxy)-8-fluoro-3-methylquinoxalin-2(1*H*)-one (80 mg, 0.24 mmol), 2-fluoro-*N*-methyl-1',2',3',6'-tetrahydro-[3,4'-bipyridine]-6-carboxamide (85 mg, 0.36 mmol), and DIPEA (124 mg, 0.96 mmol) were added sequentially to ACN (8 mL), and the reaction was carried out at 70 °C for 3 h. The reaction solution was concentrated under reduced pressure, diluted with DCM (50 mL), washed with water (30 mL) and saturated NaCl solution (30 mL), dried over anhydrous $Na_2SO_4$, concentrated under reduced pressure, and the residue was purified by silica gel column chromatography (DCM/MeOH (v/v) = 20/1) to give a pale white solid (95 mg, 81%).

MS (ESI, pos. ion) *m*/*z*: 492.2 $[M+H]^+$;
$^1H$ NMR (400 MHz, DMSO-$d_6$) δ 12.64 (s, 1H), 8.64 (q, *J* = 4.7 Hz, 1H), 8.09 (dd, *J* = 9.8, 7.8 Hz, 1H), 7.92 (dd, *J*= 7.7, 1.3 Hz, 1H), 7.31 (d, *J* = 74.4 Hz, 1H), 7.15 (d, *J* = 5.4 Hz, 1H), 6.26 (s, 1H), 3.76 (s, 2H), 3.20 (d, *J*= 2.7 Hz, 2H), 2.80 (d, *J* = 4.8 Hz, 3H), 2.71 (t, *J* = 5.5 Hz, 2H), 2.53 (s, 2H), 2.45 (s, 3H).

**Example 17 6-fluoro-*N*-methyl-5-(4-((2-methyl-3-oxo-8-(trifluoromethoxy)-3,4-dihydroquinoxalin-6-yl)methyl) piperazin-1-yl)picolinamide**

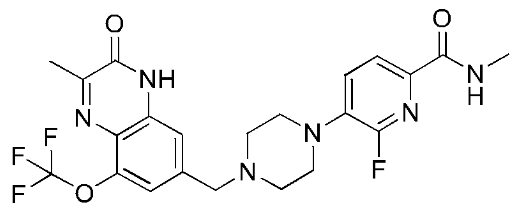

(20)

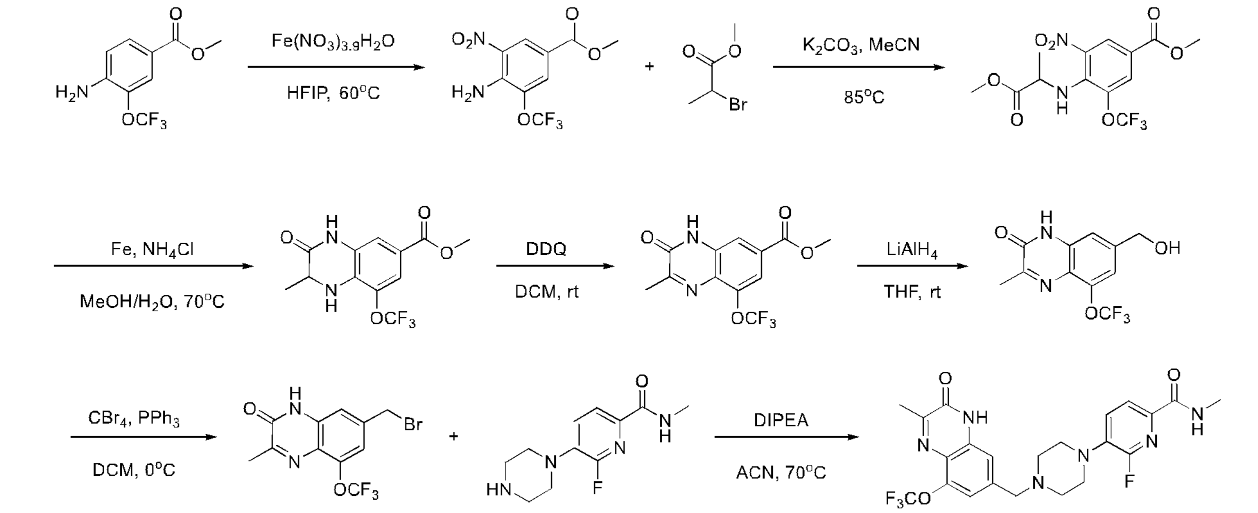


Step 1) Synthesis of methyl 4-amino-3-nitro-5-(trifluoromethoxy)benzoate

Methyl 4-amino-3-(trifluorofluoromethoxy)benzoate (4.0 g, 17.01 mmol) and $Fe(NO_3)_3$•$9H_2O$ (7.56 g, 18.71 mmol) were added to hexafluoroisopropanol (35 mL) and reacted at 60 °C for 18 h. The reaction solution was concentrated under reduced pressure, and the residue was purified by silica gel column chromatography (PE/EtOAc (v/v) = 9/1) to give a yellow solid (3.5 g, 73%).

MS (ESI, pos. ion) *m/z:* 281.0 $[M+H]^+$.

Step 2) Synthesis of methyl 4-((1-methoxy-1-oxopropyl-2-yl)amino)-3-nitro-5-(trifluoromethoxy)benzoate

Methyl 4-amino-3-nitro-5-(trifluoromethoxy)benzoate (3.5 g, 12.49 mmol), methyl 2-bromopropionate (6.26 g, 37.47 mmol), and $K_2CO_3$ (3.45 mg, 24.98 mmol) were added to ACN (40 mL) and reacted at 85 °C for 24 h. The reaction solution was filtered through diatomaceous earth, the filtrate was concentrated under reduced pressure, and the residue was purified by silica gel column chromatography (PE/EtOAc (v/v) = 9/1) to give a yellow liquid (1.5 g, 33%).

MS (ESI, pos. ion) *m/z:* 367.1 $[M+H]^+$;

Step 3) Synthesis of methyl 2-methyl-3-oxo-8-(trifluoromethoxy)-1,2,3,4-tetrahydroquinoxaloline-6-carboxylate

Methyl 4-((1-methoxy-1-oxopropyl-2-yl)amino)-3-nitro-3-(trifluoromethoxy)benzoate (1.0 g, 2.73 mmol), Fe (0.91 g, 16.38 mmol), and $NH_4Cl$ (0.88 g, 16.88 mmol) were added to a mixture of MeOH (20 mL) and water (4 mL), and reacted at 70 °C for 4 h. The mixture was filtered through diatomaceous earth, and the filtrate was concentrated under reduced pressure. Extraction was performed by adding EtOAc (60 mL) and water (30 mL). The organic phase was washed with saturated NaCl solution (30 mL), dried over anhydrous $Na_2SO_4$, filtered, concentrated under reduced pressure, and the residue was purified by silica gel column chromatography (DCM/EtOAc (v/v) = 4/1) to give a white solid (580 mg, 70%).

MS (ESI, pos. ion) *m/z:* 305.1 $[M+H]^+$;

Step 4) Synthesis of methyl 2-methyl-3-oxo-8-(trifluoromethoxy)-3,4-dihydroquinoxaloline-6-carboxylic acid

DDQ (520 mg, 2.29 mmol) was added to a DCM (16 mL) solution of methyl 2-methyl-3-oxo-8-(trifluoromethoxy)-1,2,3,4-tetrahydroquinoxaloline-6-carboxylic acid (580 mg, 1.91 mmol). After addition, the reaction mixture was allowed to react at room temperature for 11 h. The reaction solution was concentrated under reduced pressure, diluted with water (30 mL), and alkalized with saturated $NaHCO_3$ solution (30 mL) while stirring. The mixture was filtered to obtain a pale yellow solid (530 mg, 92%).

MS (ESI, pos. ion) *m/z:* 303.1 $[M+H]^+$;

Step 5) Synthesis of 7-(hydroxymethyl)-3-methyl-3-(trifluoromethoxy)quinoxalin-2(1*H*)-one

At 0°C, $LiAlH_4$ (87 mg, 2.06 mmol) was added to a THF (15 mL) solution of methyl 2-methyl-3-oxo-8-(trifluoromethoxy)-3,4-dihydroquinoxalin-6-carboxylic acid ester (520 mg, 1.72 mmol). After the addition was complete, the reaction was allowed to proceed at room temperature for 3 h. The reaction solution was quenched with MeOH (2 mL),

concentrated under reduced pressure, and the residue was diluted with DCM (100 mL) and MeOH (10 mL). The mixture was filtered through diatomaceous earth, the filtrate was concentrated under reduced pressure, and the residue was purified by silica gel column chromatography (DCM/MeOH (v/v) = 20/1) to give a white solid (260 mg, 55%).

MS (ESI, pos. ion) *m/z:* 275.0 $[M+H]^+$;

Step 6) Synthesis of 7-(bromomethyl)-8-fluoro-3-methyl-5-(trifluoromethoxy)quinoxalin-2(1*H*)-one

At 0 °C, $CBr_4$ (630 mg, 1.90 mmol) was added to 10 mL of DCM containing 7-(hydroxymethyl)-3-methyl-5-(trifluoromethoxy)quinoxalin-2(1*H*)-one (260 mg, 0.95 mmol) and $PPh_3$ (498 mg, 1.90 mmol). The reaction was carried out under nitrogen protection at 0 °C for 15 h. The reaction solution was concentrated under reduced pressure, and the residue was purified by silica gel column chromatography (PE/EtOAc (v/v) = 1/1) to give a white solid (280 mg, 88%).

MS (ESI, pos. ion) *m/z:* 337.0 $[M+H]^+$;

Step 7) Synthesis of 6-fluoro-N-methyl-5-(4-((2-methyl-3-oxo-8-(trifluoromethoxy)-3,4-dihydroquinoxalin-6-yl)methyl)piperazin-1-yl)picolinamide

7-(bromomethyl)-8-fluoro-3-methyl-5-(trifluoromethoxy)quinoxalin-2(1*H*)-one (90 mg, 0.27 mmol), 6-fluoro-*N*-methyl-5-(piperazin-1-yl)picolinamide (97 mg, 0.41 mmol), and DIPEA (105 mg, 0.81 mmol) were added sequentially to ACN (6 mL), and the reaction was carried out at 70 °C for 3 h. The reaction solution was concentrated under reduced pressure, diluted with DCM (50 mL), washed with water (30 mL) and saturated NaCl solution (30 mL), dried over anhydrous $Na_2SO_4$, concentrated under reduced pressure, and the residue was purified by silica gel column chromatography (DCM/MeOH (v/v) = 20/1) to give a pale white solid (100 mg, 76%).

MS (ESI, pos. ion) *m/z:* 495.2 $[M+H]^+$;

$^1$H NMR (400 MHz, DMSO-$d_6$) δ 12.51 (s, 1H), 8.40 (dd, *J* = 9.1, 4.3 Hz, 1H), 7.84 (d, *J* = 8.0 Hz, 1H), 7.57 (dd, *J* = 10.2, 8.5 Hz, 1H), 7.28 (s, 1H), 7.26 (s, 1H), 3.66 (s, 2H), 3.18 (s, 4H), 2.76 (d, *J* = 4.7 Hz, 3H), 2.58 (s, 4H), 2.42 (s, 3H).

**Example 18 5-(4-((8-(3,3-difluorocyclobutoxy)-2-methyl-3-oxo-3,4-dihydroquinoxalin-6-yl)methyl)piperazin-1-yl)-6-fluoro-*N*-methylpicolinamide**

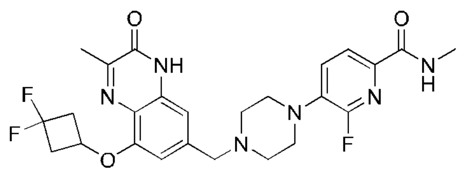

(21)

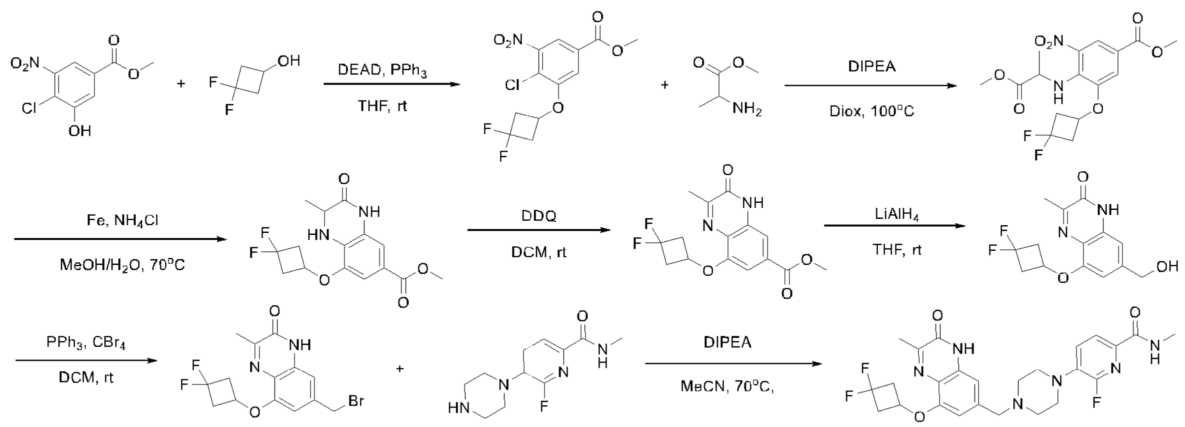


Step 1) Synthesis of methyl 4-chloro-3-(3,3-difluorocyclobutoxy)-5-nitrobenzene

Methyl 4-chloro-3-hydroxy-5-nitrobenzene (2.0 g, 8.64 mmol), 3,3-difluorocyclobutyl-1-ol (1.21 g, 11.23 mmol), and $PPh_3$ (3.40 g, 12.96 mmol) were added to THF (20 mL) under nitrogen protection and at 0 °C. DEAD (2.26 g, 12.96 mmol) was added, and the reaction was allowed to proceed at room temperature for 20 h. The reaction solution was

concentrated under reduced pressure, and the residue was purified by silica gel column chromatography (PE/EtOAc (v/v) = 9/1) to give a white solid (1.8 g, 65%).

MS (ESI, pos. ion) *m/z:* 322.9 $[M+H]^+$.

Step 2) Synthesis of methyl 3-(3,3-difluorocyclobutyloxy)-4-((1-methoxy-1-oxopropyl-2-yl)amino)-5-nitrobenzoate

Methyl 4-chloro-3-(3,3-difluorocyclobutyloxy)-5-nitrobenzoate (600 mg, 1.87 mmol), methyl DL-2-aminopropionate hydrochloride (391 mg, 2.81 mmol), and DIPEA (725 mg, 5.61 mmol) were added to 1,4-dioxane (8 mL), and the reaction was carried out at 100 °C for 16 h. The reaction was concentrated under reduced pressure, and the residue was purified by silica gel column chromatography (PE/EtOAc (v/v) = 3/2) to give a yellow oily liquid (410 mg, 57%).

MS (ESI, pos. ion) *m/z:* 389.2 $[M+H]^+$.

Step 3) Synthesis of methyl 8-(3,3-difluorocyclobutoxy)-2-methyl-3-oxo-1,2,3,4-tetrahydroquinoxaloline-6-carboxylic acid

Iron powder (519 mg, 9.30 mmol), $NH_4Cl$ (497 mg, 9.30 mmol), and methyl 3-(3,3-difluorocyclobutoxy)-4-((1-methoxy-1-oxopropyl-2-yl)amino)-5-nitrobenzene (600 mg, 1.55 mmol) were added to MeOH (8 mL) and water (4 mL), and the reaction was carried out at 70 °C for 8 h. The reaction solution was filtered with diatomaceous earth, and the filtrate was concentrated under reduced pressure to remove the solvent. The residue was added to water (30 mL), and extracted with EtOAc (60 mL × 3). The organic phase was washed with saturated NaCl aqueous solution (30 mL), dried over anhydrous $Na_2SO_4$, filtered, and the filtrate was concentrated under reduced pressure. The residue was purified by silica gel column chromatography (PE/EtOAc (v/v) = 3/2) to give a white solid product (400 mg, 79%).

MS (ESI, pos. ion) *m/z:* 327.0 $[M+H]^+$.

Step 4) Synthesis of methyl 8-(3,3-difluorocyclobutyloxy)-2-methyl-3-oxo-3,4-dihydroquinoxaloline-6-carboxylic acid ester

DDQ (459 mg, 2.02 mmol) was added to a DCM (12 mL) solution of methyl 8-(3,3-difluorocyclobutyloxy)-2-methyl-3-oxo-1,2,3,4-tetrahydroquinoxaloline-6-carboxylic acid ester (600 mg, 1.84 mmol), and the reaction was carried out at room temperature for 16 h. The reaction solution was concentrated under reduced pressure, and the residue was neutralized by stirring in a saturated $NaHCO_3$ solution (40 mL). A solid precipitated, which was filtered to obtain a solid. The solid was dried under vacuum at 50 °C for 12 h to obtain a yellow solid (400 mg, 67%).

MS (ESI, pos. ion) *m/z:* 325.1 $[M+H]^+$.

Step 5) Synthesis of 5-(3,3-difluorocyclobutoxy)-7-(hydroxymethyl)-3-methylquinoxalin-2(1*H*)-one

$LiAlH_4$ (103 mg, 2.46 mmol) was added to a solution of methyl 8-(3,3-difluorocyclobutoxy)-2-methyl-3-oxo-3,4-dihydroquinoxalin-6-carboxylic acid ester (400 mg, 1.23 mmol) in THF (8 mL), and the reaction was carried out at room temperature for 6 h. The reaction was quenched with MeOH (5 mL), diluted with DCM (20 mL), concentrated under reduced pressure, and the residue was purified by silica gel column chromatography (DCM/MeOH (v/v) = 10/1) to give a white solid (110 mg, 30%).

MS (ESI, pos. ion) *m/z:* 297.0 $[M+H]^+$.

Step 6) Synthesis of 7-(bromomethyl)-5-(3,3-difluorocyclobutoxy)-3-methylquinoxalin-2(1*H*)-one

At 0 °C, $CBr_4$ (184 mg, 0.55 mmol) was added to a solution of 5-(3,3-difluorocyclobutoxy)-7-(hydroxymethyl)-3-methylquinoxalin-2(1*H*)-one (110 mg, 0.37 mmol) and $PPh_3$ (146 mg, 0.55 mmol) in DCM (6 mL). After addition, the reaction was carried out at room temperature for 10 h. The reaction solution was concentrated under reduced pressure, and the residue was purified by silica gel column chromatography (PE/EtOAc (v/v) = 1/1) to give a white solid (80 mg, 60%).

MS (ESI, pos. ion) *m/z:* 359.0 $[M+H]^+$.

Step 7) Synthesis of 5-(4-((8-(3,3-difluorocyclobutoxy)-2-methyl-3-oxo-3,4-dihydroquinoxalin-6-yl)methyl)piperazin-1-yl)-6-fluoro-*N*-methylpicolinamide

7-(bromomethyl)-5-(3,3-difluorocyclobutyloxy)-3-methylquinoxalin-2(1*H*)-one (80 mg, 0.22 mmol), 6-fluoro-N-methyl-5-(piperazin-1-yl)picolinamide (68 mg, 0.29 mmol), and DIPEA (85 mg, 0.66 mmol) were added sequentially to MeCN (5 mL), and the reaction was carried out at 70 °C for 3 h. The reaction solution was concentrated under reduced pressure, diluted with DCM (50 mL), washed with water (30 mL) and saturated NaCl solution (30 mL), dried over anhydrous

$Na_2SO_4$, concentrated under reduced pressure, and the residue was purified by silica gel column chromatography (DCM/MeOH (v/v) = 20/1) to give a white solid (60 mg, 52%).

MS (ESI, pos. ion) *m/z:* 517.1 $[M+H]^+$;

$^1H$ NMR (400 MHz, $CDCl_3$) δ (ppm) 10.30 (s, 1H), 8.00 (d, *J* = 7.9 Hz, 1H), 7.50 (d, *J* = 4.6 Hz, 1H), 7.32 (dd, *J* = 9.9, 8.1 Hz, 1H), 6.84 (s, 1H), 6.67 (s, 1H), 4.87 - 4.80 (m, 1H), 3.62 (s, 2H), 3.25 (s, 4H), 3.21 - 3.14 (m, 2H), 3.04 - 2.96 (m, 5H), 2.66 (s, 4H), 2.63 (s, 3H).

**Example 19 5-(4-((8-cyclopropyl-5-fluoro-2-methyl-3-oxo-3,4-dihydroquinoxalin-6-yl)methyl)piperazin-1-yl)-6-fluoro-*N*-methylpicolin amide**

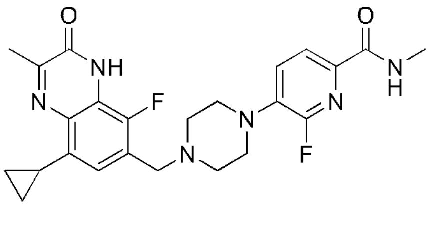

(26)

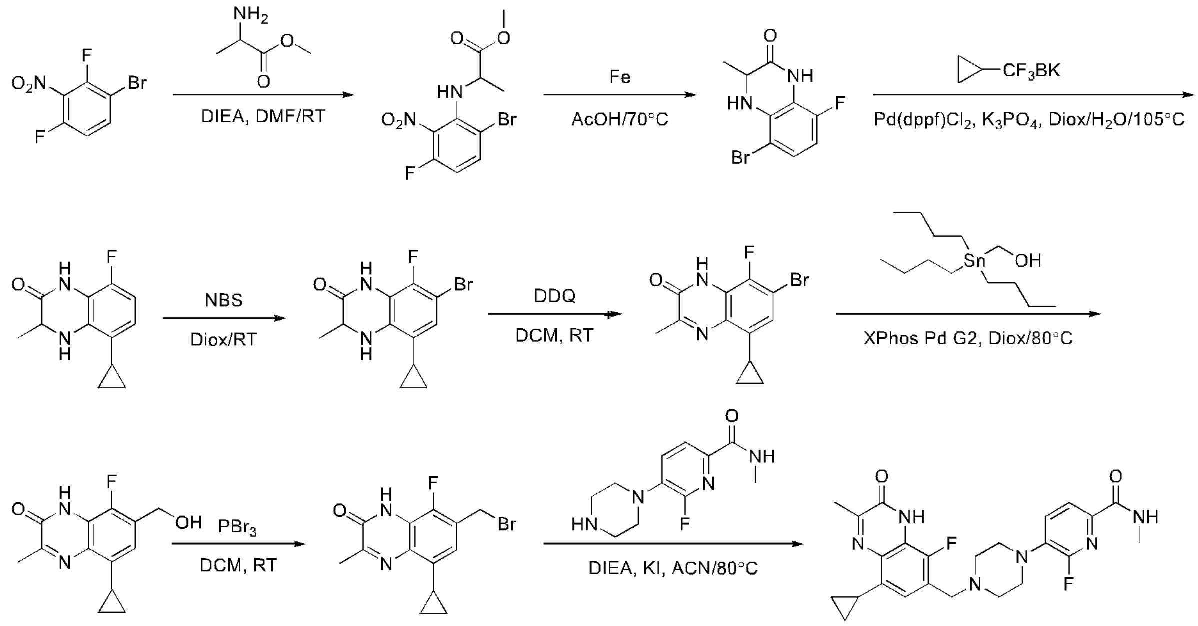


Step 1) Synthesis of methyl (6-bromo-3-fluoro-2-nitrophenyl)aminopropionate

1-Bromo-2,4-difluoro-3-nitrobenzene (6.05 g, 25.42 mmol) and methyl 2-aminopropionate hydrochloride (3.58 g, 25.67 mmol) were added to a 250 mL flask and stirred to dissolve in DMF (30 mL). The mixture was cooled, and DIPEA (13.28 mL, 76.26 mmol) was added dropwise under ice bath conditions, gradually increasing the temperature until the reaction was complete overnight. The reaction was quenched with 30 mL of water, extracted with EtOAc (30 mL × 3), and the organic phases were combined, washed with water and saturated brine, dried over anhydrous sodium sulfate, concentrated under reduced pressure, and the residue was purified by silica gel column chromatography (PE/EtOAc (v/v) = 4/1) to give a yellow solid (2.78 g, 34.06%).

MS (ESI, pos. ion) *m/z:* 321.10 $[M+H]^+$.

Step 2) Synthesis of 5-bromo-8-fluoro-3-methyl-1,2,3,4-tetrahydroquinoxalin-2-one

Methyl (6-bromo-3-fluoro-2-nitrophenyl)aminopropionate (2.78 g, 8.66 mmol) was added to a 100 mL flask and dissolved in AcOH (19 mL). Iron powder (1.93 g, 34.64 mmol) was then added, and the mixture was heated to 70 °C for 2 h. After cooling to room temperature, the mixture was quenched with 38 mL of water, resulting in the precipitation of a solid.

The filter cake was collected by filtration, dried under vacuum, and then purified by silica gel column chromatography (PE/EtOAc (v/v) = 1/1) to obtain a white solid (1.6 g, yield 71.33%).
MS (ESI, pos. ion) *m/z:* 259.10 $[M+H]^+$.

Step 3) Synthesis of 5-cyclopropyl-8-fluoro-3-methyl-1,2,3,4-tetrahydroquinoxalin-2-one

In a 100 mL flask, 5-bromo-8-fluoro-3-methyl-1,2,3,4-tetrahydroquinoxalin-2-one (0.80 g, 3.09 mmol) and potassium cyclopropyltrifluoroborate (0.91 g, 6.18 mmol) were added sequentially, dissolved in 1,4-dioxane (25 mL). Then, an aqueous solution (4 mL) of potassium phosphate (1.97 g, 9.27 mmol) was added, and the mixture was stirred until homogeneous. Finally, $Pd(dppf)Cl_2$ (0.23 g, 0.31 mmol) was added, the mixture was purged with nitrogen, and the mixture was heated to 105 °C and reacted overnight. Cool to room temperature, quench with 20 mL of water, extract with EtOAc (30 mL × 2), combine organic phases, wash with saturated brine, dry with anhydrous sodium sulfate, concentrate under reduced pressure, and purify the residue by silica gel column chromatography (DCM/MeOH (v/v) = 30/1) to give a pale yellow solid (0.31 g, 45.58%).
MS (ESI, pos. ion) *m/z:* 221.20 $[M+H]^+$.

Step 4) Synthesis of 7-bromo-5-cyclopropyl-8-fluoro-3-methyl-1,2,3,4-tetrahydroquinoxalin-2-one

5-cyclopropyl-8-fluoro-3-methyl-1,2,3,4-tetrahydroquinoxalin-2-one (0.304 g, 1.38 mmol) was added to a 100 mL flask and stirred to dissolve in 1,4-dioxane (14 mL). After cooling, NBS (0.279 g, 1.57 mmol) was added under ice bath conditions. The mixture was stirred until homogeneous, then transferred to room temperature and stirred overnight under nitrogen protection. The mixture was quenched with saturated sodium thiosulfate, extracted with EtOAc (15 mL × 2), and the organic phases were combined. The mixture was washed with saturated brine, dried over anhydrous sodium sulfate, concentrated under reduced pressure, and the residue was purified by silica gel column chromatography (DCM/MeOH (v/v) = 30/1) to give (0.20 g, 48.44%).
MS (ESI, pos. ion) *m/z*: 299.20 $[M+H]^+$.

Step 5) Synthesis of 7-bromo-5-cyclopropyl-8-fluoro-3-methyl-1,2-dihydroquinoxalin-2-one

7-bromo-5-cyclopropyl-8-fluoro-3-methyl-1,2,3,4-tetrahydroquinoxalin-2-one (0.197 g, 0.66 mmol) was added to a 100 mL flask and stirred to dissolve in DCM (6 mL). After cooling, DDQ (0.16 g, 0.73 mmol) was added in portions under ice bath conditions, and the mixture was stirred until homogeneous. The mixture was then moved to room temperature and stirred for another 5 h. The mixture was concentrated under reduced pressure, cooled, and quenched with saturated sodium bicarbonate (12 mL) under ice bath conditions. A solid precipitated out. The mixture was stirred for 1 h, filtered to collect the filter cake, washed with saturated sodium bicarbonate, and dried under vacuum to obtain a brownish-pink solid (0.13 g, yield 66.44%).
MS (ESI, pos. ion) *m/z:* 297.10 $[M+H]^+$;

Step 6) Synthesis of 5-cyclopropyl-8-fluoro-7-(hydroxymethyl)-3-methyl-1,2-dihydroquinoxalin-2-one

7-bromo-5-cyclopropyl-8-fluoro-3-methyl-1,2-dihydroquinoxalin-2-one (0.13 g, 0.44 mmol) and (tributyltin) methanol (0.20 g, 0.59 mmol, Purity 95%) were added sequentially to a 50 mL flask and stirred to dissolve in 1,4-dioxane (4 mL). Xphos Pd G2 (0.021 g, 0.027 mmol) was then added, the mixture was purged with nitrogen, and the reaction was heated to 80 °C for 7 h. After cooling to room temperature, the mixture was concentrated under reduced pressure. The residue was purified by silica gel column chromatography (DCM/MeOH (v/v) = 30/1) to give a white solid (0.060 g, yield 55.24%).
MS (ESI, pos. ion) *m/z*: 249.20 $[M+H]^+$;

Step 7) Synthesis of 7-(bromomethyl)-5-cyclopropyl-8-fluoro-3-methyl-1,2-dihydroquinoxalin-2-one

5-cyclopropyl-8-fluoro-7-(hydroxymethyl)-3-methyl-1,2-dihydroquinoxalin-2-one (0.20 g, 0.81 mmol) and DCM (10 mL) were added to a 100 mL flask. The mixture was cooled, and phosphorus tribromide (0.1 mL, 1.06 mmol) was added dropwise under ice bath conditions. The mixture was stirred until no further exothermic reaction occurred, and the reaction was continued at room temperature for 4 h. The mixture was concentrated under reduced pressure, and the residue was washed with MTBE (5 mL × 2) and then purified by silica gel column chromatography (DCM/MeOH (v/v) = 30/1) to give a white solid (0.072 g, 28.72%).

Step 8) Synthesis of 5-(4-((8-cyclopropyl-5-fluoro-2-methyl-3-oxo-3,4-dihydroquinoxalin-6-yl)methyl)piperazin-1-yl)-6-fluoro-N-methylpicolinamide

7-(bromomethyl)-5-cyclopropyl-8-fluoro-3-methyl-1,2-dihydroquinoxalin-2-one (0.072 g, 0.23 mmol), 6-fluoro-N-methyl-5-(piperazin-1-yl)pyridine-2-carboxamide dihydrochloride (0.085 g, 0.27 mmol), and potassium iodide (0.0076 g, 0.046 mmol) were added to a 50 mL flask and stirred until dissolved in ACN (2.5 mL). The mixture was cooled, and DIPEA (0.18 mL, 1.03 mmol) was slowly added dropwise under ice bath conditions. The mixture was stirred until homogeneous and heated to 80 °C for 2 h. After cooling to room temperature, a solid precipitated with stirring. The precipitate was collected by filtration and dried under vacuum to give a white solid (0.058 g, yield 53.50%).

MS (ESI, pos. ion) *m*/*z*: 469.26 $[M+H]^+$;

$^1$H NMR (400 MHz, DMSO-$d_6$) δ 12.26 (s, 1H), 8.39 (dd, *J* = 9.6, 4.9 Hz, 1H), 7.83 (d, *J* = 7.9 Hz, 1H), 7.57 - 7.52 (m, 1H), 6.70 (d, *J* = 6.6 Hz, 1H), 3.64 (s, 2H), 3.15 (br, 4H), 2.90 - 2.82 (m, 1H), 2.76 (d, *J* = 4.5 Hz, 3H), 2.55 (br, 4H), 2.45 (s, 3H), 1.06 (q, *J* = 5.7 Hz, 2H), 0.76 (q, *J* = 6.2 Hz, 2H).

$^{19}$F NMR (376 MHz, DMSO-$d_6$) δ -72.55 (s), -139.71 (s).

**Example 20 5-(4-((8-ethynyl-2-methyl-3-oxo-3,4-dihydroquinoxalin-6-yl)methyl)piperazin-1-yl)-6-fluoro-*N*-methylpicolinamide**

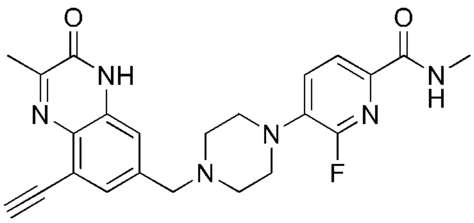

(91)

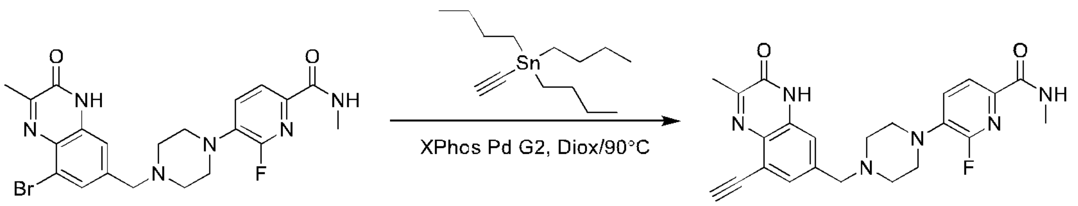


5-(4-((8-bromo-2-methyl-3-oxo-3,4-dihydroquinoxalin-6-yl)methyl)piperazin-1-yl)-6-fluoro-*N*-methylpyridin-2-carboxamide (0.127 g, 0.26 mmol) and (tributyltin)acetylene (0.15 g, 0.47 mmol) were added sequentially to a 100 mL flask, dissolved in 1,4-dioxane (5 mL), followed by the addition of Xphos Pd G2 (0.016 g, 0.021 mmol). The mixture was purged with nitrogen and heated to 90 °C overnight. After cooling to room temperature, the mixture was concentrated under reduced pressure. The residue was purified by silica gel column chromatography (DCM/MeOH (v/v) = 20/1) to give a yellow solid (0.014 g, yield 12.4%).

MS (ESI, pos. ion) m/z: 435.30 $[M+H]^+$;

**Example 21 6-fluoro-5-(4-((5-fluoro-2-methyl-3-oxo-8-(prop-1-yn-1-yl)-3,4-dihydroquinoxalin-6-yl)methyl)piperazin-1-yl)-*N*-methylpicolinamide**

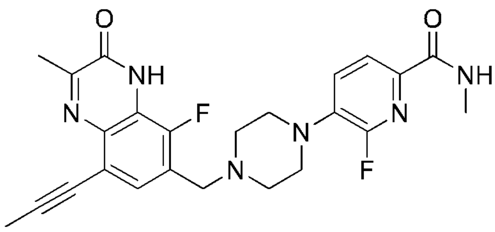

(40)

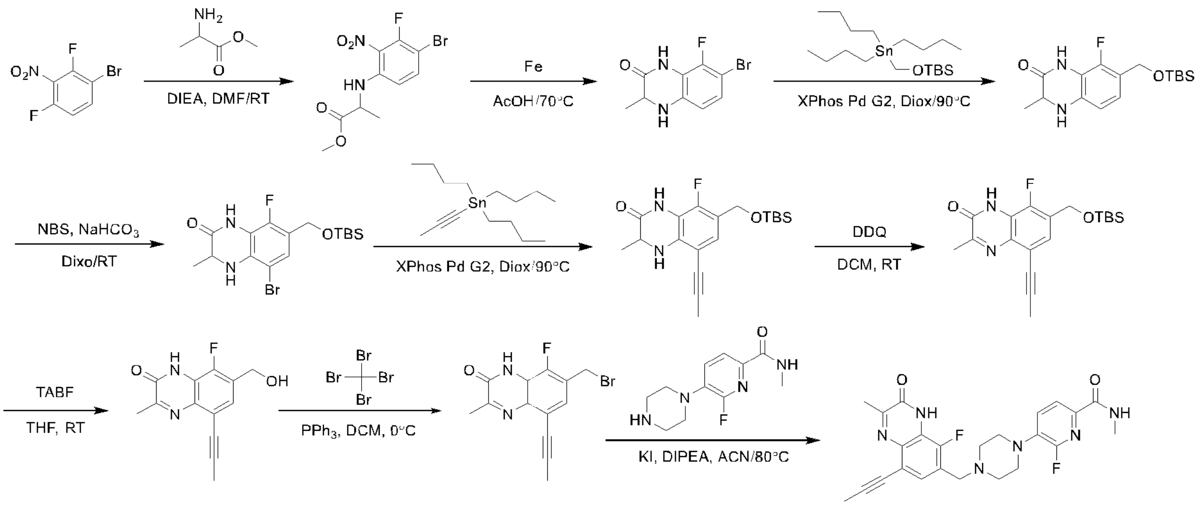


Step 1) Synthesis of Methyl 2-((6-bromo-3-fluoro-2-nitrophenyl)amino)propanoate

Into a 250 mL flask were added 1-bromo-2,4-difluoro-3-nitrobenzene (6.05 g, 25.42 mmol) and methyl 2-aminopropanoate hydrochloride (3.58 g, 25.67 mmol), which were stirred and dissolved in DMF (30 mL). The mixture was cooled, and DIPEA (13.28 mL, 76.26 mmol) was added dropwise under an ice bath. The reaction was then allowed to warm gradually to room temperature and stirred overnight. The reaction was quenched with water (30 mL) and extracted with EtOAc (30 mL × 3). The combined organic phases were washed with water (20 mL) and brine (20 mL), dried over anhydrous sodium sulfate, and concentrated under reduced pressure. The residue was purified by silica gel column chromatography (dichloromethane/ethyl acetate (v/v) = 20/1) to afford a yellow solid (2.78 g, 34.06%).
MS (ESI, pos. ion) *m/z:* 321.10 $[M+H]^+$.

Step 2) Synthesis of 7-bromo-8-fluoro-3-methyl-3,4-dihydroquinoxalin-2(1*H*)-one

Methyl 2-((4-bromo-3-fluoro-2-nitrophenyl)amino)propionate (1.5 g, 4.67 mmol) was added to a 250 mL flask and stirred to dissolve in AcOH (18 mL). Iron powder (1.04 g, 18.68 mmol) was added, and the mixture was heated to 70 °C for 2 h. After cooling to room temperature, EtOAc (40 mL) was added, and the mixture was stirred for 15 min. The mixture was filtered, and the filtrate was collected and washed successively with water, saturated brine, dried over anhydrous sodium sulfate, and concentrated under reduced pressure to obtain 1.25 g of a brownish-red solid, which was then directly proceeded to the next step.
MS (ESI, pos. ion) *m/z:* 259.1 $[M+H]^+$.

Step 3) Synthesis of 7-(((tert-butyldimethylsilyl)oxy)methyl)-8-fluoro-3-methyl-1,2,3,4-tetrahydroquinoxalin-2-one

7-bromo-8-fluoro-3-methyl-1,2,3,4-tetrahydroquinoxalin-2-one (1.25 g, 4.82 mmol) and *tert*-butyldimethyl(tributylmethoxy)silane (3.78 g, 8.68 mmol) were added sequentially to a 250 mL flask and stirred to dissolve in 1,4-dioxane (30 mL). Then, Xphos Pd G2 (0.30 g, 0.39 mmol) was added, the mixture was purged with nitrogen, and the mixture was heated to 90 °C and reacted overnight. The mixture was cooled to room temperature and concentrated under reduced pressure. The residue was then purified by silica gel column chromatography (PE/EtOAc (v/v) = 4/1) to give a pale yellow solid (1.02 g, 65.16%).
MS (ESI, pos. ion) *m/z:* 325.20 $[M+H]^+$.

Step 4) Synthesis of 5-bromo-7-(((*tert*-butyldimethylsilyl)oxy)methyl)-8-fluoro-3-methyl-1,2,3,4-tetrahydroquinoxalin-2-one

7 -(((*tert*-butyldimethylsilyl)oxy)methyl)-8-fluoro-3-methyl-1,2,3,4-tetrahydroquinoxalin-2-one (0.61 g, 1.88 mmol) and sodium bicarbonate (0.24 g, 2.82 mmol) were added to a 250 mL flask and stirred to dissolve in 1,4-dioxane (10 mL). After cooling, NBS (0.35 g, 1.97 mmol) was added under ice bath conditions, and the mixture was purged with nitrogen and stirred at room temperature for 2 h. Quenching was performed with saturated sodium thiosulfate (20 mL), followed by extraction with EtOAc (25 mL × 3). The organic phases were combined, washed with saturated brine, dried over anhydrous sodium sulfate, concentrated under reduced pressure, and the residue was purified by silica gel column chromatography (petroleum ether/ethyl acetate (v/v) = 10/1) to give a white solid (0.66 g, 87.04%).
MS (ESI, pos. ion) *m*/*z:* 403.2 $[M+H]^+$;

Step 5) Synthesis of 7-(((*tert*-butyldimethylsilyl)oxy)methyl)-8-fluoro-3-methyl-5-(prop-1-yn-1-yl)-1,2,3,4-tetrahydroquinoxalin-2-one

In a 100 mL flask, 5-bromo-7-(((*tert*-butyldimethylsilyl)oxy)methyl)-8-fluoro-3-methyl-1,2,3,4-tetrahydroquinoxalin-2-one (0.65 g, 1.61 mmol) and tributyl(prop-1-yn-1-yl)tin (0.79 g, 2.42 mmol) were added sequentially and stirred to dissolve in 1,4-dioxane (13 mL). Then, Xphos Pd G2 (0.10 g, 0.13 mmol) was added, the mixture was purged with nitrogen, and the mixture was heated to 90 °C and reacted overnight. The mixture was cooled to room temperature and concentrated under reduced pressure. The residue was purified by silica gel column chromatography (petroleum ether/ethyl acetate (v/v) = 5/1) to give a pale yellow solid (0.57 g, 97.04%).
MS (ESI, pos. ion) *m*/*z:* 363.35 $[M+H]^+$;

Step 6) Synthesis of 7-(((*tert*-butyldimethylsilyl)oxy)methyl)-8-fluoro-3-methyl-5-(prop-1-yn-1-yl)-1,2-dihydroquinoxalin-2-one

To a 100 mL flask was added 7-(((*tert*-butyldimethylsilyl)oxy)methyl)-8-fluoro-3-methyl-5-(prop-1-yn-1-yl)-1,2,3,4-tetrahydroquinoxalin-2-one (0.56 g, 1.54 mmol), which was stirred and dissolved in DCM (16 mL). The mixture was cooled, and DDQ (0.38 g, 1.69 mmol) was added under an ice bath. The reaction was then allowed to warm to room temperature and stirred for 4 hours. The mixture was concentrated under reduced pressure, cooled, and quenched by the addition of saturated sodium bicarbonate (32 mL) under an ice bath. After stirring for 30 minutes, a solid precipitated. The solid was collected by filtration, washed with saturated sodium bicarbonate, and dried under vacuum to afford a brownish-yellow solid (0.77 g), which was used directly in the next step.
MS (ESI, pos. ion) *m*/*z:* 361.05 $[M+H]^+$;

Step 7) Synthesis of 8-fluoro-7-(hydroxymethyl)-3-methyl-5-(prop-1-yn-1-yl)-1,2-dihydroquinoxalin-2-one

7-(((*tert*-butyldimethylsilyl)oxy)methyl)-8-fluoro-3-methyl-5-(prop-1-yn-1-yl)-1,2-dihydroquinoxalin-2-one (0.56 g, 1.55 mmol) was added to a 100 mL flask, and stirred to dissolve in THF (12 mL), then TBAF tetrahydrofuran solution was added dropwise, and stirred at room temperature for 6 h. Quenching was performed with saturated brine (25 mL), followed by extraction with EtOAc (25 mL × 2). The organic phases were combined, washed with water, dried over anhydrous sodium sulfate, concentrated under reduced pressure, and the residue was purified by silica gel column chromatography (dichloromethane/methanol (v/v) = 20/1) to give a brown solid (0.204 g, 53.33%).
MS (ESI, pos. ion) *m*/*z:* 247.00 $[M+H]^+$;

Step 8) Synthesis of 7-(bromomethyl)-8-fluoro-3-methyl-5-(prop-1-yn-1-yl)-1,2-dihydroquinoxalin-2-one

In a 100 mL flask, 8-fluoro-7-(hydroxymethy-1)-3-methyl-5-(prop-1-yn-1-yl)-1,2-dihydroquinoxalin-2-one (0.12 g, 0.49 mmol) and triphenylphosphine (0.19 g, 0.73 mmol) were added and stirred to dissolve in DCM (5 mL). The mixture was cooled, and carbon tetrabromide (0.24 g, 0.73 mmol) was added under ice bath conditions. The mixture was stirred at 0 °C for 1 h. The mixture was concentrated under reduced pressure, and the residue was purified by silica gel column chromatography (petroleum ether/ethyl acetate (v/v) = 3/1) to give a white solid (0.052 g, 34.52%).
MS (ESI, pos. ion) *m*/*z:* 309.1 $[M+H]^+$;

Step 9) Synthesis of 6-fluoro-5-(4-((5-fluoro-2-methyl-3-oxo-8-(prop-1-yn-1-yl)-3,4-dihydroquinoxalin-6-yl)methyl)piperazin-1-yl)-*N*-methylpicolinamide

7-(bromomethyl)-8-fluoro-3-methyl-5-(prop-1-yn-1-yl)-1,2-dihydroquinoxalin-2-one (0.051 g, 0.16 mmol), 6-fluoro-N-methyl-5-(piperazin-1-yl)pyridine-2-carboxamide dihydrochloride (0.055 g, 0.18 mmol) and potassium iodide (0.0053 g, 0.032 mmol) were added to a 50 mL flask, stirred to dissolve in ACN (1.5 mL), The mixture was cooled, and DIPEA (0.11 mL, 0.64 mmol) was added dropwise slowly under ice bath, stirred until homogeneous, and heated to 80 °C for 2 h. Cooled to room temperature, solids precipitated under stirring. The filter cake was collected by filtration, washed with MTBE (1.5 mL × 3), followed by water (1.5 mL × 3), and dried under vacuum to afford a white solid (0.031 g, 40.28%).

MS (ESI, pos. ion) *m/z:* 467.05 $[M+H]^+$;

$^1H$ NMR (599 MHz, DMSO-$d_6$) δ 12.51 (s, 1H), 8.42 - 8.37 (m, 1H), 7.83 (d, *J* = 7.8 Hz, 1H), 7.55 (dd, *J* = 10.6, 8.0 Hz, 1H), 7.33 (d, *J* = 6.7 Hz, 1H), 3.66 (s, 2H), 3.18 - 3.14 (m, 4H), 2.76 (d, *J* = 4.8 Hz, 3H), 2.60 - 2.55 (m, 4H), 2.43 (s, 3H), 2.11 (s, 3H).

**Example 22 5-(4-((8-(cyclopropylethynyl)-5-fluoro-2-methyl-3-oxo-3,4-dihydroquinoxalin-6-yl)methyl)piperazin-1-yl)-6-fluoro-*N*-methylpicolinamide**

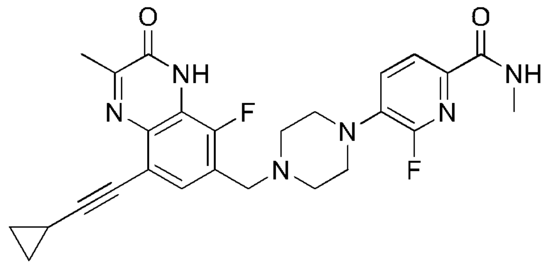

(95)

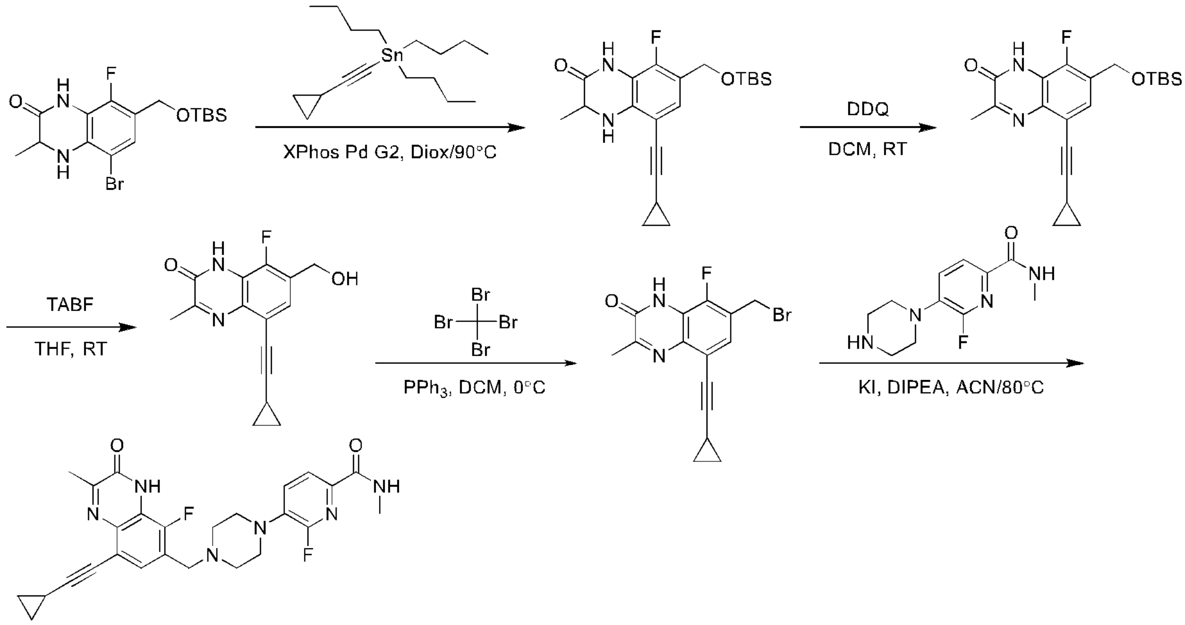


Step 1) Synthesis of 7-(((*tert*-butyldimethylsilyl)oxy)methyl)-5-(2-cyclopropylethynyl)-8-fluoro-3-methyl-1,2,3,4-tetrahydroquinoxalin-2-one

5-bromo-7-(((*tert*-butyldimethylsilyl)oxy)methyl)-8-fluoro-3-methyl-1,2,3,4-tetrahydroquinoxalin-2-one (1.0 g, 2.48 mmol) and tributyl(2-cyclopropylethynyl)tin (1.15 g, 3.22 mmol) were added sequentially to a 100 mL flask, dissolved in 1,4-dioxane (15 mL), and then Xphos Pd G2 (0.16 g, 0.20 mmol) was added. The mixture was purged with nitrogen and heated to 90 °C for 6 h. The mixture was cooled to room temperature and concentrated under reduced pressure. The residue was purified by silica gel column chromatography (petroleum ether/ethyl acetate (v/v) = 5/1) to give a yellow solid (0.895 g, 92.91%).

MS (ESI, pos. ion) *m/z:* 389.10 $[M+H]^+$.

Step 2) Synthesis of 7-(((*tert*-butyldimethylsilyl)oxy)methyl)-5-(2-cyclopropylethynyl)-8-fluoro-3-methyl-1,2-dihydroquinoxalin-2-one

To a 250 mL flask, 7-(((*tert*-butyldimethylsilyl)oxy)methyl)-5-(2-cyclopropylethynyl)-8-fluoro-3-methyl-1,2,3,4-tetrahydroquinoxalin-2-one (0.89 g, 2.29 mmol) was added and dissolved in DCM (16 mL). After cooling in an ice bath, DDQ (0.57 g, 2.52 mmol) was added. The mixture was allowed to warm to room temperature and stirred overnight. The mixture was then concentrated under reduced pressure, cooled again, and quenched by adding saturated sodium bicarbonate solution (32 mL) under ice bath conditions, followed by stirring for 30 minutes. Solids precipitated out, and the mixture was filtered. The filter cake was collected, washed with saturated sodium bicarbonate, and dried under vacuum to yield a brown solid (0.81 g, 91.48%).
MS (ESI, pos. ion) *m*/*z:* 387.10 $[M+H]^+$.

Step 3) Synthesis of 5-(2-cyclopropylethynyl)-8-fluoro-7-(hydroxymethyl)-3-methyl-1,2-dihydroquinoxalin-2-one

To a 100 mL flask, 7-(((*tert*-butyldimethylsilyl)oxy)methyl)-5-(2-cyclopropylethynyl)-8-fluoro-3-methyl-1,2-dihydroquinoxalin-2-one (0.80 g, 2.07 mmol) was added and dissolved in THF (16 mL) with stirring. Then, a TBAF solution in THF (4.14 mL, 4.14 mmol) was added dropwise, and the mixture was stirred at room temperature overnight. The reaction was quenched by adding saturated brine (30 mL) and extracted with EtOAc (25 mL × 2). The organic phases were combined, washed with water, dried over anhydrous sodium sulfate, and concentrated under reduced pressure. The residue was purified by silica gel column chromatography (dichloromethane/methanol (v/v) = 20/1) to afford a brownish-yellow solid (0.32 g, 56.79 %).
MS (ESI, pos. ion) *m*/*z:* 273.15 $[M+H]^+$.

Step 4) Synthesis of 7-(bromomethyl)-5-(2-cyclopropylethynyl)-8-fluoro-3-methyl-1,2-dihydroquinoxalin-2-one

5-(2-cyclopropylethynyl)-8-fluoro-7-(hydroxymethyl)-3-methyl-1,2-dihydroquinoxalin-2-one (0.318 g, 1.17 mmol) and triphenylphosphine (0.46 g, 1.75 mmol) were added to a 250 mL flask and stirred to dissolve in DCM (9 mL). After cooling, carbon tetrabromide (0.58 g, 1.75 mmol) was added in portions under ice bath conditions, and stirring was continued at 0 °C for 1 h. The mixture was concentrated under reduced pressure, and the residue was purified by silica gel column chromatography (petroleum ether/ethyl acetate (v/v) = 1/1) to give a yellow solid (0.396 g, 101.16 %).
MS (ESI, pos. ion) *m*/*z:* 335.05 $[M+H]^+$;

Step 5) Synthesis of 5-(4-((8-(2-cyclopropylethynyl)-5-fluoro-2-methyl-3-oxo-3,4-dihydroquinoxalin-6-yl)methyl)piperazin-1-yl)-6-fluoro-*N*-methylpyridin-2-carboxamide

7-(bromomethyl)-5-(2-cyclopropylethynyl)-8-fluoro-3-methyl-1,2-dihydroquinoxalin-2-one (0.14 g, 0.42 mmol), 6-fluoro-N-methyl-5-(piperazin-1-yl)pyridin-2-carboxamide dihydrochloride (0.144 g, 0.46 mmol), and potassium iodide (0.014 g, 0.084 mmol) were added to a 50 mL flask and stirred to dissolve in ACN (3 mL), after cooling, DIPEA (0.29 mL, 1.68 mmol) was added slowly dropwise under ice bath. The mixture was stirred thoroughly and heated to 80°C for 2 hours. After cooling to room temperature, a solid precipitated out with stirring. The filter cake was collected by filtration, washed with MTBE (3 mL × 3) and water (3 mL × 3), dried under vacuum, and then purified by slurrying with methanol (1.5 mL) to obtain a white solid (0.030 g, 14.58%).
MS (ESI, pos. ion) *m*/*z:* 493.15 $[M+H]^+$;
$^1$H NMR (400 MHz, DMSO-$d_6$) δ 8.40 (q, *J* = 4.8 Hz, 1H), 7.84 (d, *J* = 7.9 Hz, 1H), 7.56 (dd, *J* = 10.5, 8.2 Hz, 1H), 7.31 *(d, J* = 6.9 Hz, 1H), 3.65 (s, 2H), 3.19 - 3.13 (m, 4H), 2.76 (d, *J* = 4.8 Hz, 3H), 2.61 - 2.54 (m, 4H), 2.44 (s, 3H), 0.97 - 0.90 (m, 2H), 0.85 (t, *J* = 6.7 Hz, 1H), 0.81 - 0.75 (m, 2H).

**Example 23 2-fluoro-1'-((5-fluoro-2-methyl-3-oxo-8-(prop-1-yn-1-yl)-3,4-dihydroquinoxalin-6-yl)methyl)-*N*-methyl-1',2',3',6'-tetrahydro-[3,4'-bipyridine]-6-carboxamide**

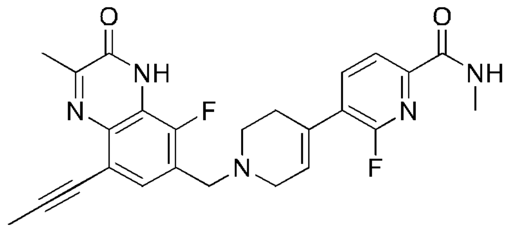

(83)

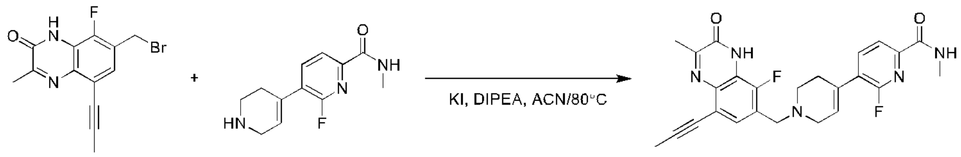


To a 50 mL flask, 7-(bromomethyl)-8-fluoro-3-methyl-5-(prop-1-yn-1-yl)-1,2-dihydroquinoxalin-2-one (0.21 g, 0.68 mmol), 6-fluoro-*N*-methyl-5-(1,2,3,6-tetrahydropyridin-4-yl)pyridine-2-carboxamide hydrochloride (0.268 g, 0.99 mmol), and potassium iodide (0.023 g, 0.14 mmol) were added and dissolved in ACN (3 mL) with stirring. After cooling in an ice bath, DIPEA (0.36 mL, 2.04 mmol) was slowly added dropwise, and the mixture was stirred well. It was then heated to 80°C and reacted for 2 hours. After cooling to room temperature, solids precipitated under stirring, and the mixture was filtered. The filter cake was collected, washed with MTBE (1.5 mL × 3) and water (1.5 mL × 3), and dried under vacuum to yield a yellow solid (0.025 g, 7.94 %).

MS (ESI, pos. ion) *m/z:* 464.15 $[M+H]^+$.

**Example 24 1'-((8-(cyclopropylethynyl)-5-fluoro-2-methyl-3-oxo-3,4-dihydroquinoxalin-6-yl)methyl)-2-fluoro-*N*-methyl-1',2',3',6'-tetrahydro-[3,4'-bipyridine]-6-carboxamide**

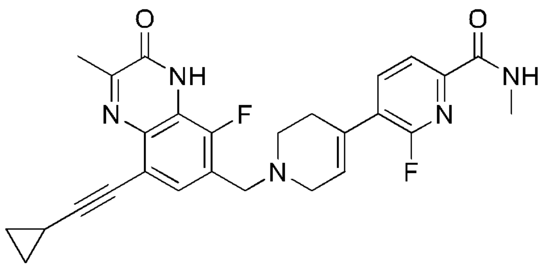

(101)

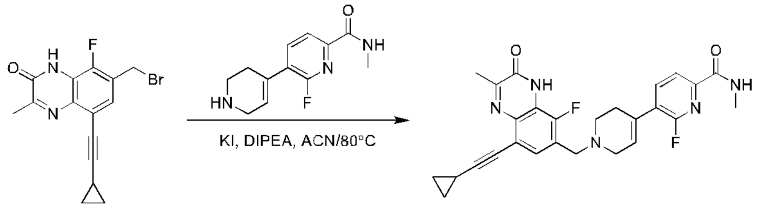


To a 50 mL flask, 7-(bromomethyl)-5-(2-cyclopropylethynyl)-8-fluoro-3-methyl-1,2-dihydroquinoxalin-2-one (0.15 g, 0.45 mmol), 6-fluoro-*N*-methyl-5-(1,2,3,6-tetrahydropyridin-4-yl)pyridine-2-carboxamide hydrochloride (0.12 g, 0.45 mmol), and potassium iodide (0.015 g, 0.090 mmol) were added and dissolved in ACN (4 mL) with stirring. After cooling in an ice bath, DIPEA (0.24 mL, 1.35 mmol) was slowly added dropwise, and the mixture was stirred well. It was then heated to 80°C and reacted for 2 hours. After cooling to room temperature, the mixture was diluted with DCM (20 mL), washed with water (15 mL × 2), followed by saturated brine. The organic phase was dried over anhydrous sodium sulfate and concentrated under reduced pressure. The residue was purified by preparative thin-layer silica gel chromatography to afford a white solid (0.018 g, 8.22 %).

MS (ESI, pos. ion) *m/z:* 489.80 $[M+H]^+$.

**Example 25 Synthesis of 6-fluoro-*N*-methyl-5-(4-((2-methyl-3-oxo-8-vinyl-3,4-dihydroquinoxalin-6-yl)methyl)piperazin-1-yl)picolinamide**

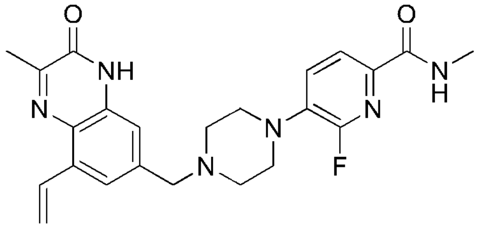

(103)

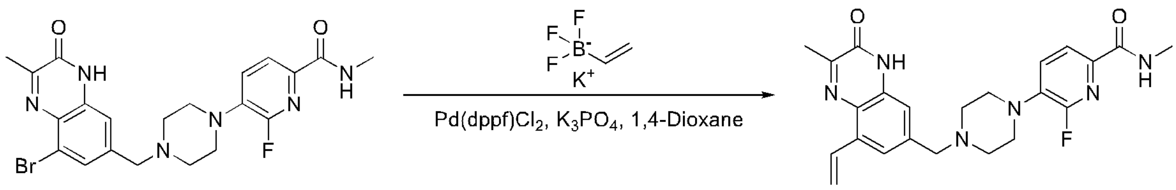


At room temperature, potassium ethylene trifluoroborate (0.11 g, 0.82 mmol), Pd(dppf)$Cl_2$ (0.030 g, 0.041 mmol), and $K_3PO_4$ (0.17 g, 0.82 mmol) were added to a solution of 5-(4-((8-bromo-2-methyl-3-oxo-3,4-dihydroquinoxalin-6-yl) methyl)piperazin-1-yl)-6-fluoro-N-methylpicolinamide (0.2 g, 0.41 mmol) in 1,4-dioxane (8 mL) and water (2 mL). After purging with nitrogen, the mixture was heated to 110 °C and stirred for 12 h. The reaction solution was cooled to room temperature, filtered with diatomaceous earth, and the filtrate was concentrated under reduced pressure to remove the solvent. The residue was purified by silica gel column chromatography (eluting agent: dichloromethane/methanol (v/v) = 20/1) to give a yellow solid product (0.1 g, 56.06%).

MS (ESI, pos. ion) *m/z:* 437.2 $[M+H]^+$;
$^1$H NMR (400 MHz, DMSO-$d_6$) δ (ppm) 12.26 (s, 1H), 8.44 - 8.35 (m, 1H), 7.84 (d, *J* = 8.0 Hz, 1H), 7.73 - 7.61 (m, 1H), 7.61 - 7.53 (m, 1H), 7.53 - 7.48 (m, 1H), 7.23 - 7.19 (m, 1H), 6.00 (d, *J* = 17.9 Hz, 1H), 5.46 (d, *J* = 11.3 Hz, 1H), 3.62 (s, 2H), 3.22 - 3.15 (m, 4H), 2.76 (d, *J* = 4.8 Hz, 3H), 2.61 - 2.54 (m, 4H), 2.42 (s, 3H).

**Example 26 5-(4-((8-ethoxy-2-methyl-3-oxo-3,4-dihydroquinoxalin-6-yl)methyl)piperazin-1-yl)-6-fluoro-*N*-methylpicolinamide**

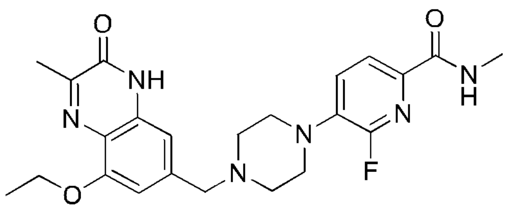

(104)

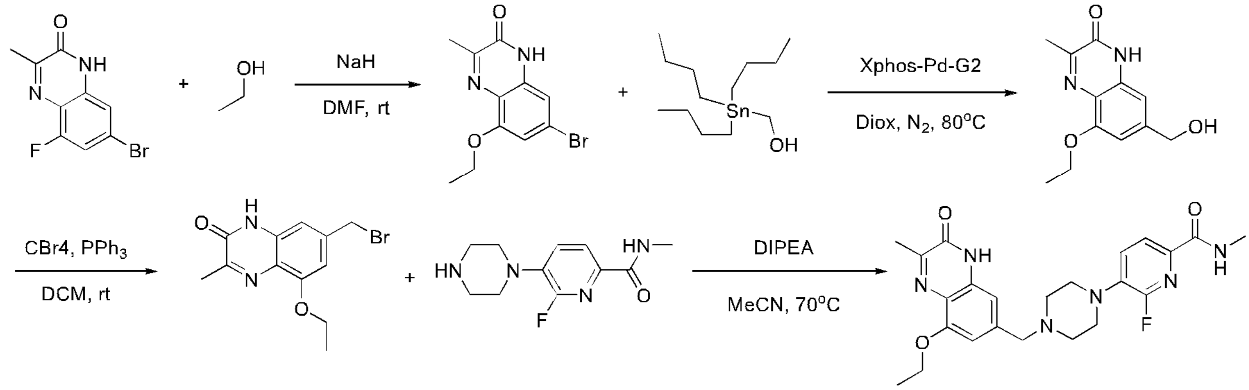


Step 1) Synthesis of 7-bromo-5-ethoxy-3-methylquinoxalin-2(1H)-one

Under nitrogen protection at 0°C, NaH (466 mg, 11.65 mmol, wt 60%) was added to a DMF (10 mL) solution of 7-bromo-5-fluoro-3-methylquinoxalin-2(1H)-one (600 mg, 2.33 mmol) and ethanol (537 mg, 11.65 mmol). After addition, the reaction was carried out at room temperature for 10 h. The reaction solution was concentrated under reduced pressure, and saturated NaCl solution (50 mL) and DCM (100 mL) were added. The mixture was extracted and separated. The organic phase was dried over anhydrous $Na_2SO_4$, filtered, and concentrated under reduced pressure to give a pale yellow solid (550 mg, 83%).
MS (ESI, pos. ion) *m/z:* 283.0 $[M+H]^+$.

Step 2) Synthesis of 5-ethoxy-7-(hydroxymethyl)-3-methylquinoxalin-2(1*H*)-one

7-bromo-5-ethoxy-3-methylquinoxalin-2(1*H*)-one (550 mg, 1.94 mmol), (tributyltin)methanol (685 mg, 2.13 mmol), and Xphos-Pd-G2 (153 mg, 0.19 mmol) were added to 1,4-dioxane (12 mL) under nitrogen protection and reacted at 80 °C for 6 h. The reaction solution was concentrated under reduced pressure, and the residue was purified by silica gel column chromatography (DCM/MeOH (v/v) = 20/1) to give a pale yellow solid (231 mg, 51%).
MS (ESI, pos. ion) *m/z:* 235.3 $[M+H]^+$.

Step 3) Synthesis of 7-(bromomethyl)-5-ethoxy-3-methylquinoxalin-2(1*H*)-one

$CBr_4$ (478 mg, 1.44 mmol) was added to DCM (8 mL) containing 5-ethoxy-7-(hydroxymethyl)-3-methylquinoxalin-2(1*H*)-one (210 mg, 0.90 mmol) and $PPh_3$ (378 mg, 1.44 mmol), and the reaction was carried out at room temperature for 20 h. The reaction solution was concentrated under reduced pressure, and the residue was purified by silica gel column chromatography (PE/EtOAc (v/v) = 1/1) to give a white solid (130 mg, 49%).
MS (ESI, pos. ion) *m/z:* 297.1 $[M+H]^+$;

Step 4) Synthesis of 5-(4-((8-ethoxy-2-methyl-3-oxo-3,4-dihydroquinoxalin-6-yl)methyl)piperazin-1-yl)-6-fluoro-N-methylpicolinamide

7-(bromomethyl)-5-ethoxy-3-methylquinoxalin-2(1*H*)-one (107 mg, 0.36 mmol), 6-fluoro-*N*-methyl-5-(piperazin-1-yl)picolinamide (94 mg, 0.40 mmol), and DIPEA (186 mg, 1.44 mmol) were added sequentially to MeCN (8 mL), and the reaction was carried out at 70 °C for 2 h. The reaction solution was concentrated under reduced pressure, diluted with DCM (50 mL), washed with water (30 mL) and saturated NaCl solution (30 mL), dried over anhydrous $Na_2SO_4$, concentrated under reduced pressure, and the residue was purified by silica gel column chromatography (DCM/MeOH (v/v) = 20/1) to give a pale yellow solid (100 mg, 61%).
MS (ESI, pos. ion) *m/z:* 455.4 $[M+H]^+$;
$^1H$ NMR (400 MHz, DMSO-$d_6$) $\delta$ (ppm) 10.62 (s, 1H), 7.99 (d, *J* = 7.9 Hz, 1H), 7.50 (d, *J* = 4.6 Hz, 1H), 7.33 - 7.28 (m, 1H), 6.82 (d, *J* = 13.1 Hz, 2H), 4.30 (q, *J* = 6.9 Hz, 2H), 3.62 (s, 2H), 3.24 (s, 4H), 2.99 (d, *J* = 5.0 Hz, 3H), 2.66 (s, 4H), 2.63 (s, 3H), 1.57 (t, *J* = 7.0 Hz, 3H).

**Example 27 6-fluoro-5-(4-((8-(2-fluorophenoxy)-2-methyl-3-oxo-3,4-dihydroquinoxalin-6-yl)methyl)piperazin-1-yl)-*N*-methylpicolinamide**

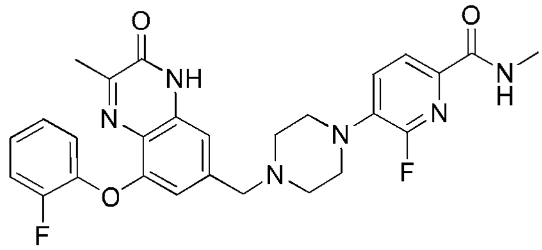

(105)

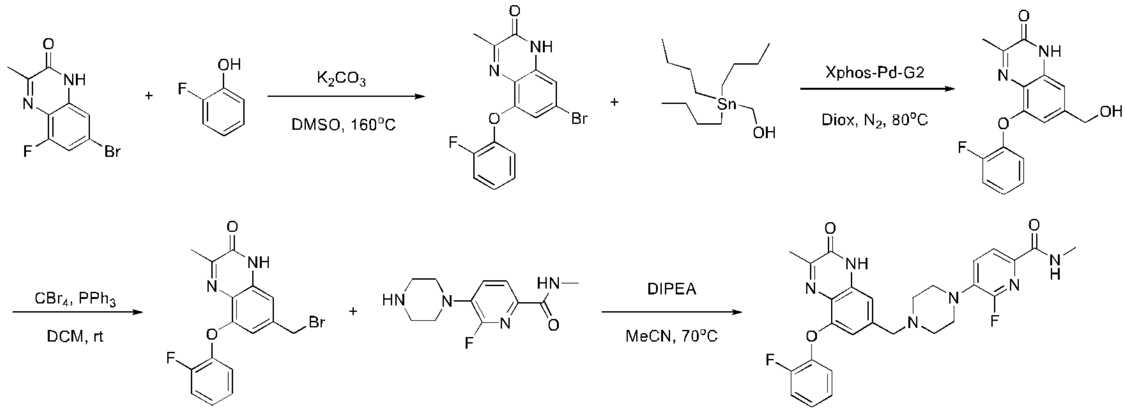


Step 1) Synthesis of 7-bromo-5-(2-fluorophenoxy)-3-methylquinoxalin-2(1*H*)-one

7-bromo-5-fluoro-3-methylquinoxalin-2(1*H*)-one (600 mg, 2.33 mmol), 2-fluorophenol (780 mg, 6.99 mmol), and $K_2CO_3$ (970 mg, 6.99 mmol) were added to DMSO (12 mL) solution. After addition, the reaction mixture was reacted at 160 °C for 24 h. The reaction solution was diluted with water (60 mL), and a solid precipitated. The solid was filtered to obtain a solid, dissolved in MeOH (5 mL) and DCM (50 mL), dried over anhydrous $Na_2SO_4$, filtered, and concentrated under reduced pressure to obtain a yellow solid (500 mg, 61%).

MS (ESI, pos. ion) *m/z:* 349.1 $[M+H]^+$.

Step 2) Synthesis of 5-(2-fluorophenoxy)-7-(hydroxymethyl)-3-methylquinoxalin-2(1*H*)-one

7-bromo-5-(2-fluorophenoxy)-3-methylquinoxalin-2(1*H*)-one (500 mg, 1.43 mmol), (tributyltin)methanol (505 mg, 1.57 mmol), and Xphos-Pd-G2 (113 mg, 0.14 mmol) were added to 1,4-dioxane (10 mL) under nitrogen protection and reacted at 80 °C for 16 h. The reaction solution was concentrated under reduced pressure, and the residue was purified by silica gel column chromatography (DCM/MeOH (v/v) = 20/1) to give a pale yellow solid (250 mg, 58%).

MS (ESI, pos. ion) *m/z:* 301.1 $[M+H]^+$.

Step 3) Synthesis of 7-(bromomethyl)-5-(2-fluorophenoxy)-3-methylquinoxalin-2(1H)-one

At 0 °C, $CBr_4$ (444 mg, 1.34 mmol) was added to 15 mL of DCM containing 5-(2-fluorophenoxy)-7-(hydroxymethyl)-3-methylquinoxalin-2(1*H*)-one (200 mg, 0.67 mmol) and PPh3 (351 mg, 1.34 mmol). After 1 h, the reaction was carried out at room temperature for 16 h. The reaction solution was concentrated under reduced pressure, and the residue was purified by silica gel column chromatography (PE/EtOAc (v/v) = 1/1) to give a pale yellow solid (150 mg, 62%).

MS (ESI, pos. ion) *m/z:* 363.1 $[M+H]^+$;

Step 4) Synthesis of 6-fluoro-5-(4-((8-(2-fluorophenoxy)-2-methyl-3-oxo-3,4-dihydroquinoxalin-6-yl)methyl)piperazin-1-yl)-*N*-methylpicolinamide

7-(bromomethyl)-5-(2-fluorophenoxy)-3-methylquinoxalin-2(1H)-one (150 mg, 0.41 mmol), 6-fluoro-N-methyl-5-(piperazin-1-yl)picolinamide (107 mg, 0.45 mmol), and DIPEA (212 mg, 1.64 mmol) were added sequentially to MeCN (8 mL), and reacted at 70 °C for 3 h. The reaction solution was concentrated under reduced pressure, diluted with DCM (60 mL), washed with water (30 mL) and saturated NaCl solution (30 mL), dried over anhydrous $Na_2SO_4$, concentrated under reduced pressure, and the residue was purified by silica gel column chromatography (DCM/MeOH (v/v) = 20/1) to give a pale yellow solid (120 mg, 56%).

MS (ESI, pos. ion) *m/z:* 521.3 $[M+H]^+$;
$^1H$ NMR (400 MHz, DMSO-$d_6$) $\delta$ (ppm) 10.93 (s, 1H), 7.99 (d, *J* = 7.9 Hz, 1H), 7.50 (dd, *J* = 10.0, 5.1 Hz, 1H), 7.32 - 7.27 (m, 1H), 7.25 - 7.13 (m, 4H), 6.98 (s, 1H), 6.63 (s, 1H), 3.55 (s, 2H), 3.17 (s, 4H), 2.99 (d, *J* = 5.0 Hz, 3H), 2.66 (s, 3H), 2.59 (s, 4H).

**Example 28 6-fluoro-5-(4-((8-(3-fluorophenoxy)-2-methyl-3-oxo-3,4-dihydroquinoxalin-6-yl)methyl)piperazin-1-yl)-*N*-methylpicolinamide**

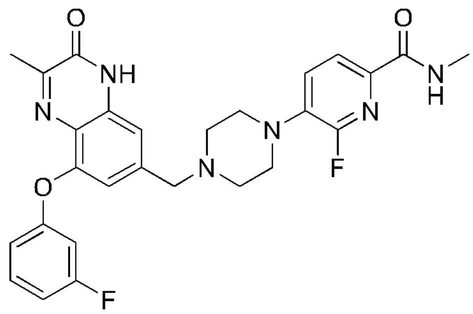

(106)

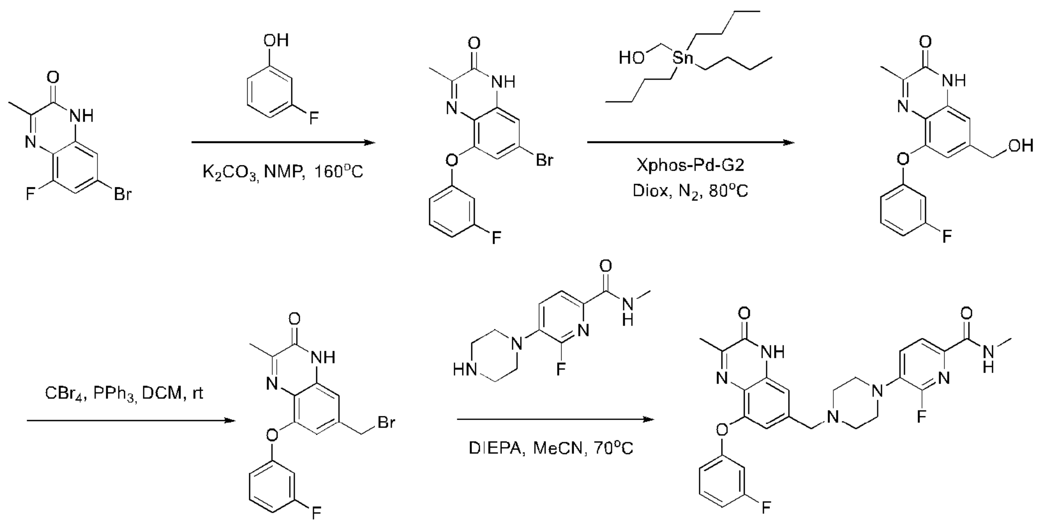


Step 1) Synthesis of 7-bromo-5-(3-fluorophenoxy)-3-methylquinoxalin-2(1H)one

At room temperature, 7-bromo-5-fluoro-3-methyl-1,2-dihydroquinoxalin-2-one (600 mg, 2.33 mmol), potassium carbonate (970 mg, 7.02 mmol) and 3-fluorophenol (780 mg, 6.96 mmol) were added to a solution of *N*-methylpyrrolidone (20 mL), and the mixture was heated to 160 °C and stirred for 12 h. The reaction mixture was quenched with water (20 mL), and saturated NaCl solution (10 mL) was added. The mixture was stirred for 1 h, filtered, and the filter cake was dissolved in a mixed solvent (dichloromethane/methanol (v/v) = 10/1, 20 mL). The solution was concentrated under reduced pressure to give a black solid (620 mg, 76%).
MS (ESI, pos. ion) *m/z:* 349.1 $[M+H]^+$.

Step 2) Synthesis of 5-(3-fluorophenoxy)-7-hydroxymethyl-3-methylquinoxalin-2(1*H*)one

At room temperature, 7-bromo-5-(3-fluorophenoxy)-3-methyl-1,2-dihydroquinoxalin-2-one (500 mg, 1.43 mmol), (tributyltin)methanol (505.07 mg, 1.57 mmol), and chloro(2-dicyclohexylphosphino-2',4',6'-triisopropyl-1,1'-biphenyl)[2-(2'-amino-1,1'-biphenyl)]palladium(II) (112.51 mg, 0.14 mmol) catalyst were added to 1,4-dioxane (15 mL), and the reaction was carried out under nitrogen protection at 80 °C for 12 h. The reaction solution was concentrated under reduced pressure to remove the solvent, and the residue was purified by column chromatography (dichloromethane/-methanol (V/V) = 20/1) to give a yellow solid product (220 mg, 51.16%).

MS (ESI, pos. ion) *m/z:* 301.2 [M+H]+.

Step 3) Synthesis of 7-(bromomethyl)-5-(3-fluorophenoxy)-3-methylquinoxalin-2(1*H*)-one

At 0 °C, tetrabromomethane (444.38 mg, 1.34 mmol) was added to a solution of 5-(3-fluorophenoxy)-7-hydroxymethyl-3-methylquinoxalin-2(1*H*)one (200 mg, 0.67 mmol) and triphenylphosphine (351.47 mg, 1.34 mmol) in dichloromethane (12 ml). After the reaction solution became clear, the mixture was transferred to room temperature and reacted for 22 h. The reaction solution was concentrated under reduced pressure to remove the solvent. The residue was purified by column chromatography (petroleum ether/ethyl acetate (V/V) = 1/1) to give a gray solid product (120 mg, 49.61%).

MS (ESI, pos. ion) *m/z:* 363.1 [M+H]+.

Step 4) Synthesis of 6-fluoro-5-(4-((8-(3-fluorophenoxy)-2-methyl-3-oxo-3,4-dihydroquinoxalin-6-yl)methyl)piperazin-1-yl)-*N*-methylpicolinamide

At room temperature, ethyl diisopropylamine was added to a solution of 7-(bromomethyl)-5-(3-fluorophenoxy)-3-methylquinoxalin-2(1*H*)-one (213.25 mg, 1.65 mmol) and 6-fluoro-*N*-methyl-5-(piperazin-1-yl)pyridine-2-carboxamide (120 mg, 0.33 mmol) in acetonitrile (10 ml). The temperature was then raised to 70 °C, and the reaction was stirred for 2 h. After cooling, the reaction solution was concentrated under reduced pressure to remove the solvent. The residue was purified by silica gel column chromatography (dichloromethane/methanol (v/v) = 20/1) to give a white solid product (134 mg, 77.91%).

MS (ESI, pos. ion) *m/z:* 521.3 [M+H]+;
$^1$H NMR (400 MHz, DMSO-$d_6$) δ 8.39 (d, *J* = 4.8 Hz, 1H), 7.83 (d, *J* = 7.9 Hz, 1H), 7.59 - 7.50 (m, 1H), 7.38 (q, *J* = 8.0 Hz, 1H), 7.10 (s, 1H), 6.93 (t, *J* = 8.5 Hz, 1H), 6.85 (d, *J* = 10.1 Hz, 2H), 6.81 (d, *J* = 8.3 Hz, 1H), 3.14 (s, 4H), 2.76 (d, *J* = 4.7 Hz, 3H), 2.54 (s, 4H), 2.32 (s, 3H).

$^{13}$C NMR (151 MHz, DMSO-$d_6$) δ 163.82, 163.56, 162.20, 159.14, 159.06, 158.49, 155.22, 153.68, 152.08, 150.99, 141.12, 138.58, 138.51, 137.38, 137.23, 133.85, 131.40, 131.33, 128.43, 128.40, 123.57, 121.31, 113.95, 113.86, 113.85, 111.25, 110.02, 109.88, 105.56, 105.39, 61.32, 52.31, 49.36, 49.33, 26.18, 20.83.

**Example 29 6-fluoro-*N*-methyl-5-(4-((2-methyl-3-oxo-8-(pyridin-4-yloxy)-3,4-dihydroquinoxalin-6-yl)methyl)piperazin-1-yl)picolinamide**

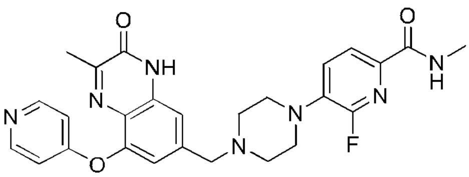

(107)

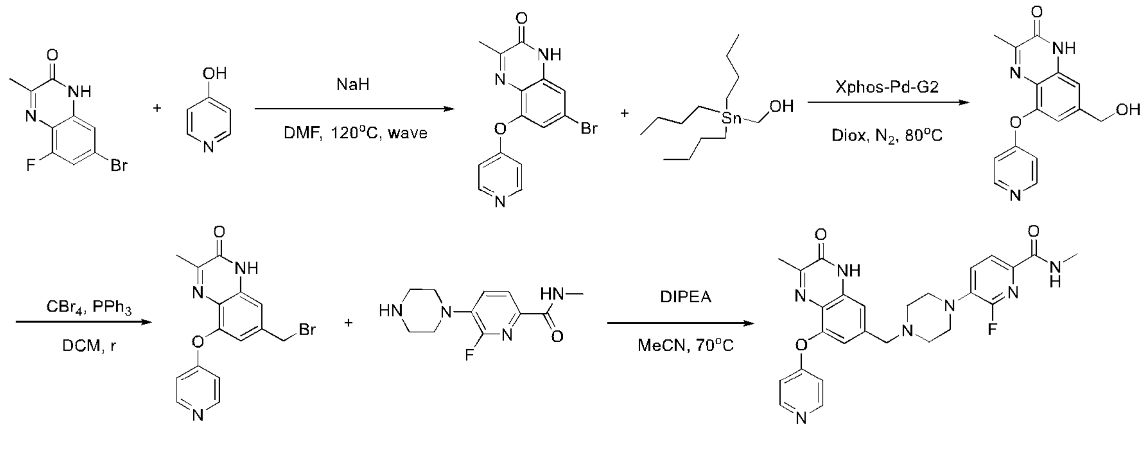

Step 1) Synthesis of 7-bromo-3-methyl-5-(pyridin-4-yloxy)quinoxalin-2(1*H*)-one

7-bromo-5-fluoro-3-methylquinoxalin-2(1*H*)-one (1.00 g, 3.89 mmol), pyridin-4-ol (1.11 g, 11.67 mmol), and $K_2CO_3$ (1.61 g, 11.67 mmol) were added to DMF (16 mL). After addition, the mixture was microwaved at 120 °C for 14 h. The reaction solution was diluted with water (60 mL), and the pH was adjusted to 5 with HCl solution (4 M). A solid was precipitated, and filtered to obtain the solid. The solid was dried under vacuum at 50 °C for 8 h to obtain a pale yellow solid (900 mg, 70%).
MS (ESI, pos. ion) *m/z:* 332.0 $[M+H]^+$.

Step 2) Synthesis of 7-(hydroxymethyl)-3-methyl-5-(pyridin-4-yloxy)quinoxalin-2(1*H*)-one

7-bromo-3-methyl-5-(pyridin-4-yloxy)quinoxalin-2(1*H*)-one (500 mg, 1.51 mmol), (tributyltin)methanol (970 mg, 3.02 mmol), and Xphos-Pd-G2 (475 mg, 0.60 mmol) were added to 1,4-dioxane (12 mL) under nitrogen protection and reacted at 80 °C for 24 h. The reaction solution was concentrated under reduced pressure, and the residue was purified by silica gel column chromatography (DCM/MeOH (v/v) = 4/1) to give a pale yellow solid (140 mg, 33%).
MS (ESI, pos. ion) *m/z:* 284.2 $[M+H]^+$.

Step 3) Synthesis of 7-(bromomethyl)-3-methyl-5-(pyridin-4-yloxy)quinoxalin-2(1H)-one

At 0 °C, $CBr_4$ (325 mg, 0.98 mmol) was added to DCM (10 mL) containing 7-(hydroxymethyl)-3-methyl-5-(pyridin-4-yloxy)quinoxalin-2(1*H*)-one (138 mg, 0.49 mmol) and $PPh_3$ (257 mg, 0.98 mmol). After 1 h, the reaction was carried out at room temperature for 14 h. The reaction solution was concentrated under reduced pressure, and the residue was purified by silica gel column chromatography (DCM/MeOH (v/v) = 10/1) to give a yellow solid (100 mg, 59%).
MS (ESI, pos. ion) *m/z:* 346.1 $[M+H]^+$;

Step 4) Synthesis of 6-fluoro-*N*-methyl-5-(4-((2-methyl-3-oxo-8-(pyridin-4-yloxy)-3,4-dihydroquinoxalin-6-yl)methyl)piperazin-1-yl)picolinamide

7-(bromomethyl)-3-methyl-5-(pyridin-4-yloxy)quinoxalin-2(1*H*)-one (100 mg, 0.29 mmol), 6-fluoro-*N*-methyl-5-(piperazin-1-yl)picolinamide (76 mg, 0.32 mmol), and DIPEA (150 mg, 1.16 mmol) were added sequentially to MeCN (8 mL), and the reaction was carried out at 70 °C for 3 h. The reaction solution was concentrated under reduced pressure, and the residue was purified by silica gel column chromatography (DCM/MeOH (v/v) = 10/1) to give a pale yellow solid (60 mg, 41%).
MS (ESI, pos. ion) *m/z:* 504.3 $[M+H]^+$;
$^1H$ NMR (400 MHz, DMSO-$d_6$) δ (ppm) 12.62 (s, 1H), 9.72 (s, 1H), 8.40 (d, *J* = 4.7 Hz, 1H), 7.85 (t, *J* = 8.2 Hz, 3H), 7.64 - 7.53 (m, 1H), 7.41 (s, 1H), 6.25 (d, *J* = 7.5 Hz, 2H), 3.36 (s, 4H), 3.22 (s, 2H), 3.06 (d, *J* = 6.7 Hz, 4H), 2.76 (d, *J* = 4.6 Hz, 3H), 2.37 (s, 3H).

**Example 30 5-(4-((8-(cyclopent-1-en-1-yl)-2-methyl-3-oxo-3,4-dihydroquinoxalin-6-yl)methyl)piperazin-1-yl)-6-fluoro-*N*-methylpicolinamide**

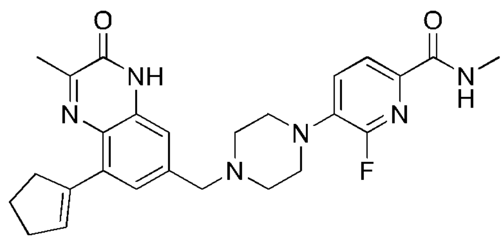

(108)

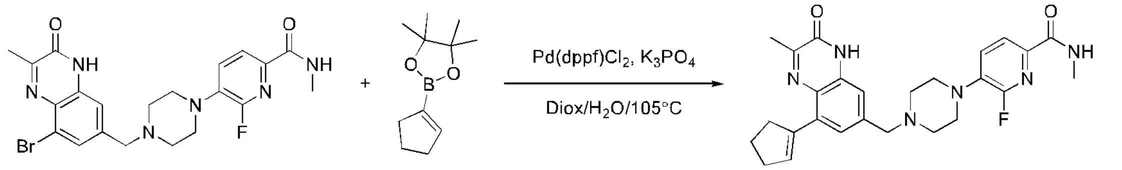

To a 100 mL flask were sequentially added 5-(4-((8-bromo-2-methyl-3-oxo-3,4-dihydroquinoxalin-6-yl)methyl)piperazin-1-yl)-6-fluoro-*N*-methylpicolinamide (0.060 g, 0.12 mmol) and 1-cyclopenteneboronic acid pinacol ester (0.054 g, 0.28 mmol). The mixture was dissolved in 1,4-dioxane (2.5 mL) under stirring. Subsequently, an aqueous solution (0.5 mL) of potassium phosphate (0.054 g, 0.25 mmol) was added, and the mixture was stirred until homogeneous. Finally, Pd(dppf)$Cl_2$ (0.0092 g, 0.013 mmol) was added. The system was subjected to nitrogen displacement and then heated to 105°C for reaction. After cooling to room temperature, the mixture was diluted with EtOAc (15 mL), washed with saturated brine (10 mL × 2), and dried over anhydrous sodium sulfate. The solvent was removed under reduced pressure, and the residue was purified by silica gel column chromatography (DCM/MeOH (v/v) = 20/1) to afford a white solid (0.032 g, yield 54.76 %).

MS (ESI, pos. ion) *m/z*: 477.40 $[M+H]^+$;
$^1H$ NMR (400 MHz, DMSO-$d_6$) δ 12.26 (s, 1H), 8.39 (s, 1H), 7.83 (s, 1H), 7.56 (s, 1H), 7.16 (s, 2H), 6.62 (s, 1H), 3.59 (s, 2H), 3.18 (br, 4H), 2.86 (s, 3H), 2.76 (br, 4H), 2.40 (s, 3H), 1.95 (br, 4H), 1.33 (br, 2H).

**Example 31 5-(4-((8-cyclopentyl-2-methyl-3-oxo-3,4-dihydroquinoxalin-6-yl)methyl)piperazin-1-yl)-6-fluoro-*N*-methylpicolinamide**

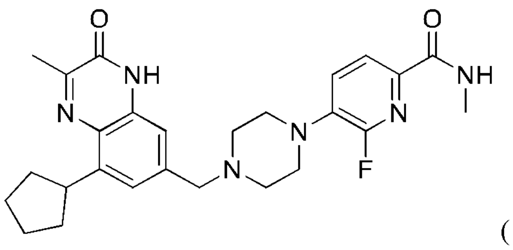

(109)

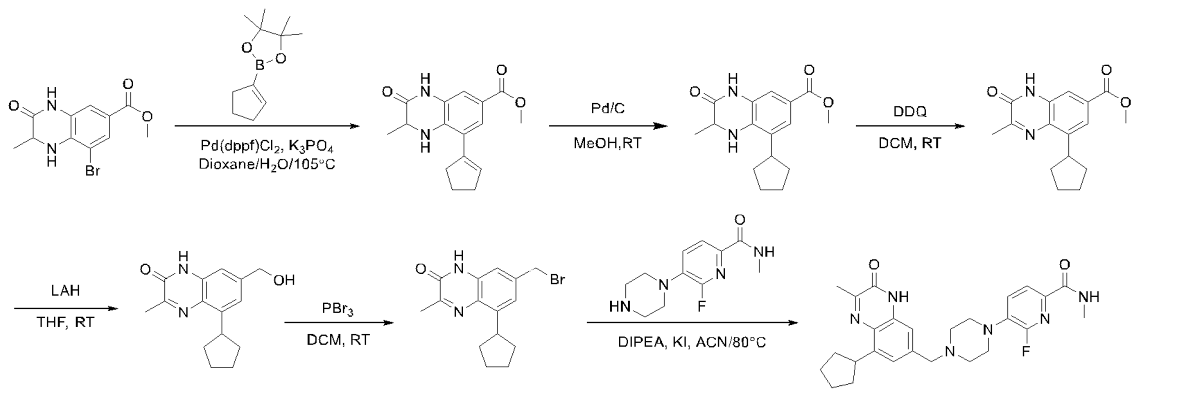


Step 1) Synthesis of methyl 8-(cyclopent-1-en-1-yl)-2-methyl-3-oxo-1,2,3,4-tetrahydroquinoxalin-6-carboxylate

Methyl 8-bromo-2-methyl-3-oxo-1,2,3,4-tetrahydroquinoxalin-6-carboxylate (0.60 g, 2.01 mmol) and 2-(cyclopent-1-en-1-yl)-4,4,5,5-tetramethyl-1,3,2-dioxaborhexacyclopentane (0.70 g, 3.62 mmol) were added sequentially to a 100 mL flask and stirred until dissolved in 1,4-dioxane (12 mL). Then, an aqueous solution of potassium phosphate (0.85 g, 4.02 mmol) was added and stirred until homogeneous. Finally, Pd(dppf)$Cl_2$ (0.15 g, 0.20 mmol) was added. The mixture was heated to 105°C overnight with nitrogen purging. After cooling to room temperature, 15 mL of water was added to quench the reaction. The mixture was extracted with EtOAc (15 mL × 3), and the organic phases were combined, washed with saturated brine, dried over anhydrous sodium sulfate, concentrated under reduced pressure, and the residue was purified by silica gel column chromatography (PE/EtOAc (v/v) = 3/1) to give a pale yellow oil (0.253 g, 44.05%).

MS (ESI, pos. ion) m/z: 287.30 $[M+H]^+$.

Step 2) Synthesis of methyl 8-cyclopentyl-2-methyl-3-oxo-1,2,3,4-tetrahydroquinoxalin-6-carboxylate

Methyl 8-(cyclopent-1-en-1-yl)-2-methyl-3-oxo-1,2,3,4-tetrahydroquinoxalin-6-carboxylate (0.253 g, 0.88 mmol) was added to a 100 mL flask, dissolved in methanol (4 mL) and tetrahydrofuran (4 mL). Then, 10% palladium on carbon (0.051 g, 0.048 mmol) was added, and the mixture was stirred overnight at room temperature after purging with

hydrogen. The reaction solution was filtered, and the filtrate was concentrated under reduced pressure to obtain 0.26 g of a colorless oil, which was then directly used for the next step.

MS (ESI, pos. ion) m/z: 289.30 $[M+H]^+$.

Step 3) Synthesis of methyl 8-cyclopentyl-2-methyl-3-oxo-3,4-dihydroquinoxalin-6-carboxylate

Methyl 8-cyclopentyl-2-methyl-3-oxo-1,2,3,4-tetrahydroquinoxalin-6-carboxylate (0.255 g, 0.88 mmol) was added to a 100 mL flask and dissolved in DCM (10 mL). After cooling, DDQ (0.22 g, 0.97 mmol) was added under ice bath, and the mixture was stirred overnight at room temperature. The solution was concentrated under reduced pressure, cooled, and quenched with saturated sodium bicarbonate (20 mL) under ice bath. After stirring for 30 min, a solid precipitated. The precipitate was collected by filtration, washed with saturated sodium bicarbonate (30 mL), and dried under vacuum to obtain a brownish-yellow solid (0.212 g, 83.72%).

MS (ESI, pos. ion) m/z: 287.30 $[M+H]^+$;

Step 4) Synthesis of 5-cyclopentyl-7-(hydroxymethyl)-3-methyl-1,2-dihydroquinoxalin-2-one

Methyl 8-cyclopentyl-2-methyl-3-oxo-3,4-dihydroquinoxalin-6-carboxylate (0.212 g, 0.74 mmol) was added to a 100 mL flask and stirred to dissolve in THF (8 mL). After cooling, LAH (0.077 g, 1.97 mmol, Purity 97%) was added under ice bath conditions, and the reaction was continued at room temperature for 4 h. The mixture was cooled, and water (2 mL) was added under an ice bath to quench the reaction. Then, 1M dilute hydrochloric acid was added dropwise to adjust the pH to 3-4. The mixture was extracted with THF (8 mL $\times$ 2). The organic phases were combined, washed with saturated brine, and dried over anhydrous sodium sulfate. The solvent was removed under reduced pressure, and the residue was purified by silica gel column chromatography (DCM/MeOH (v/v) = 20/1) to afford a white solid (0.090 g, yield 47.06 %).

MS (ESI, pos. ion) m/z: 259.20 $[M+H]^+$;

Step 5) Synthesis of 7-(bromomethyl)-5-cyclopentyl-3-methylquinoxalin-2(1*H*)-one

To a 100 mL flask was added 5-cyclopentyl-7-(hydroxymethyl)-3-methyl-1,2-dihydroquinoxalin-2-one (0.090 g, 0.35 mmol) and DCM (4.5 mL). The mixture was cooled, and phosphorus tribromide (0.043 mL, 0.45 mmol) was added dropwise under an ice bath. Stirring was continued until no further exothermic reaction was observed, and the mixture was then allowed to warm to room temperature and stirred for an additional 4 hours. The mixture was concentrated under reduced pressure, and the residue was washed with MTBE (4.5 mL $\times$ 2). The resulting crude product was directly used in the next step.

Step 6) Synthesis of 5-(4-((8-cyclopentyl-2-methyl-3-oxo-3,4-dihydroquinoxalin-6-yl)methyl)piperazin-1-yl)-6-fluoro-*N*-methylpicolinamide

To a 100 mL flask were added 7-(bromomethyl)-5-cyclopentyl-3-methylquinoxalin-2(1*H*)-one (0.11 g, 0.34 mmol), 6-fluoro-*N*-methyl-5-(piperazin-1-yl)picolinamide dihydrochloride (0.12 g, 0.37 mmol), and potassium iodide (0.011 g, 0.068 mmol). The mixture was dissolved in acetonitrile (3.5 mL), cooled, and DIPEA (0.41 mL, 2.38 mmol) was slowly added dropwise under an ice bath with stirring to ensure uniformity. The mixture was heated to 80°C and reacted for 2 hours. After cooling to room temperature, EtOAc (15 mL) was added for dilution, followed by washing with water (10 mL) and saturated brine. The organic layer was dried over anhydrous sodium sulfate, and the solvent was removed under reduced pressure. The residue was purified by silica gel column chromatography (DCM/MeOH (v/v) = 20/1) to afford a white solid (0.075 g, yield 45.76 %).

MS (ESI, pos. ion) m/z: 479.30 $[M+H]^+$;

1H NMR (599 MHz, DMSO-$d_6$) $\delta$ 12.20 (s, 1H), 8.40 (d, *J* = 4.0 Hz, 1H), 7.84 (d, *J* = 7.8 Hz, 1H), 7.61 - 7.54 (m, 1H), 7.17 (s, 1H), 7.10 (s, 1H), 4.02 - 3.96 (m, 1H), 3.59 (s, 2H), 3.18 (br, 4H), 2.77 (d, *J* = 4.2 Hz, 3H), 2.56 (br, 4H), 2.41 (br, 3H), 2.03 (br, 2H), 1.83 (br, 2H), 1.70 (br, 2H), 1.61 (br, 2H).

**Example 32 5-(4-((8-(4-cyanophenoxy)-2-methyl-3-oxo-3,4-dihydroquinoxalin-6-yl)methyl)piperazin-1-yl)-6-fluoro-*N*-methylpicolinamide**

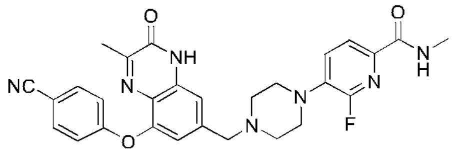

(110)

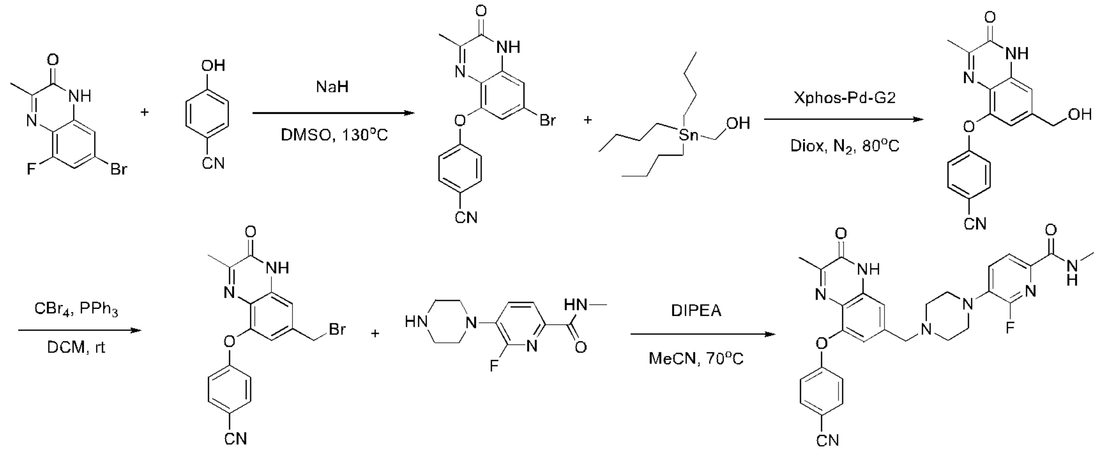


Step 1) Synthesis of 4-((7-bromo-3-methyl-2-oxo-1,2-dihydroquinoxalin-5-yl)oxy)benzonitrile

7-bromo-5-fluoro-3-methylquinoxalin-2(1*H*)-one (800 mg, 3.11 mmol), 4-hydroxybenzonitrile (1.11 g, 9.33 mmol), and $K_2CO_3$ (1.29 g, 9.33 mmol) were added to DMSO (16 mL). After addition, the mixture was microwaved at 130 °C for 15 h. The reaction solution was diluted with water (60 mL), and HCl solution (4 M) was added to adjust the pH to 5. A solid precipitated, which was filtered to obtain the solid. The solid was dried under vacuum at 50 °C for 8 h to obtain a pale yellow solid (600 mg, 54%).

MS (ESI, pos. ion) *m/z:* 356.2 $[M+H]^+$.

Step 2) Synthesis of 4-((7-(hydroxymethyl)-3-methyl-2-oxo-1,2-dihydroquinoxalin-5-yl)oxy)benzonitrile

4-((7-bromo-3-methyl-2-oxo-1,2-dihydroquinoxalin-5-yl)oxy)benzonitrile (300 mg, 0.84 mmol), (tributyltin) methanol (297 mg, 0.92 mmol), and Xphos-Pd-G2 (66 mg, 0.08 mmol) were added to 1,4-dioxane (8 mL). The reaction was carried out under nitrogen protection at 80 °C for 10 h. The reaction solution was concentrated under reduced pressure, and the residue was purified by silica gel column chromatography (DCM/MeOH (v/v) = 20/1) to give a pale yellow solid (150 mg, 58%).

MS (ESI, pos. ion) *m/z:* 308.2 $[M+H]^+$.

Step 3) Synthesis of 4-((7-(bromomethyl)-3-methyl-2-oxo-1,2-dihydroquinoxalin-5-yl)oxy)benzonitrile

At 0 °C, $CBr_4$ (259 mg, 0.78 mmol) was added to DCM (8 mL) containing 4-((7-(hydroxymethyl)-3-methyl-2-oxo-1,2-dihydroquinoxalin-5-yl)oxy)benzonitrile (120 mg, 0.39 mmol) and $PPh_3$ (205 mg, 0.78 mmol). After 1 h, the reaction was carried out at room temperature for 16 h. The reaction solution was concentrated under reduced pressure, and the residue was purified by silica gel column chromatography (PE/EtOAc (v/v) = 1/1) to give a yellow solid (50 mg, 35%).

MS (ESI, pos. ion) *m/z:* 370.1 $[M+H]^+$;

Step 4) Synthesis of 5-(4-((8-(4-cyanophenoxy)-2-methyl-3-oxo-3,4-dihydroquinoxalin-6-yl)methyl)piperazin-1-yl)-6-fluoro-*N*-methylpicolinamide

4-((7-(bromomethyl)-3-methyl-2-oxo-1,2-dihydroquinoxalin-5-yl)oxy)benzonitrile (50 mg, 0.14 mmol), 6-fluoro-N-methyl-5-(piperazin-1-yl)picolinamide (40 mg, 0.17 mmol), and DIPEA (72 mg, 0.56 mmol) were added sequentially to MeCN (4 mL), and the reaction was carried out at 70 °C for 3 h. The reaction solution was concentrated under reduced pressure, diluted with DCM (60 mL), washed successively with water (30 mL) and saturated NaCl solution (30 mL), dried over anhydrous $Na_2SO_4$, filtered, concentrated under reduced pressure, and the residue was purified by silica gel column chromatography (DCM/MeOH (v/v) = 20/1) to give a pale yellow solid (40 mg, 56%).

MS (ESI, pos. ion) *m/z:* 528.2 [M+H]$^+$;
$^1$H NMR (400 MHz, DMSO-$d_6$) δ (ppm) 10.58 (s, 1H), 8.00 (d, *J* = 7.9 Hz, 1H), 7.63 (d, *J*= 8.8 Hz, 2H), 7.50 (dd, *J* = 10.1, 4.9 Hz, 1H), 7.28 (dd, *J* = 9.2, 1.8 Hz, 1H), 7.11 (s, 1H), 7.06 (d, *J* = 8.8 Hz, 2H), 6.99 (s, 1H), 3.63 (s, 2H), 3.22 (s, 4H), 3.00 (d, *J* = 5.0 Hz, 3H), 2.65 (s, 4H), 2.53 (s, 3H).

**Example 33 6-fluoro-5-(4-((8-((4-fluorophenyl)amino)-2-methyl-3-oxo-3,4-dihydroquinoxalin-6-yl)methyl)piperazin-1-yl)-*N*-methylpicolinamide**

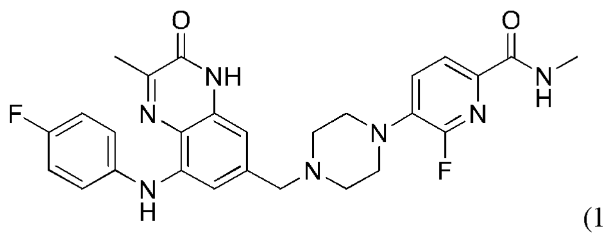

(111)

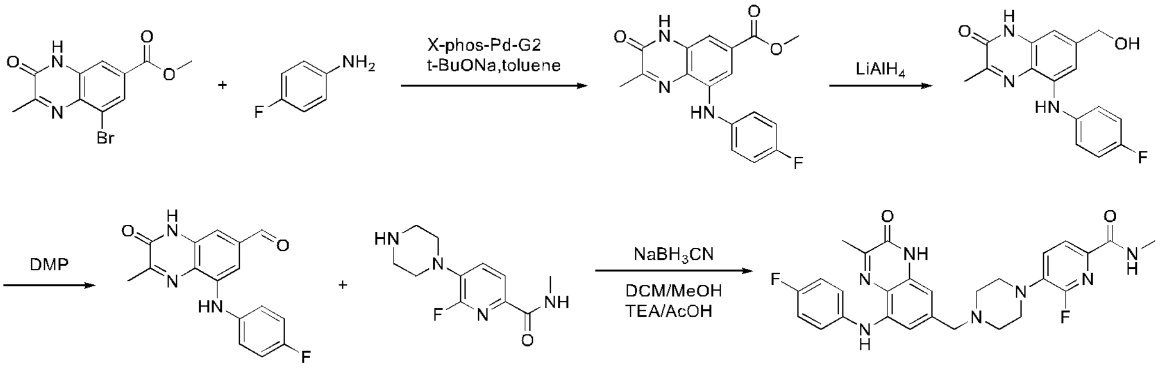


Step 1) Synthesis of methyl 8-((4-fluorophenyl)amino)-2-methyl-3-oxo-3,4-dihydroquinoxalin-6-carboxylate

Methyl 8-bromo-2-methyl-3-oxo-3,4-dihydroquinoxalin-6-carboxylate (1.4 g, 4.71 mmol), 4-fluoroaniline (0.68 g, 6.12 mmol), chloro(2-dicyclohexylphosphino-2',4',6'-triisopropyl-1,1'-biphenyl)(2-(2'-amino-1,1'-biphenyl))palladium(II) (0.26 g, 0.33 mmol), 2-di-*tert*-butylphosphino-2',4',6'-triisopropylbiphenyl (0.28 g, 0.66 mmol), sodium tert-butoxide (0.63 g, 6.59 mmol) and toluene (10 mL) were added to a reaction flask. The mixture was protected under nitrogen, heated to 110°C, and reacted for 8 hours. The reaction was then quenched, and water (100 mL) was added. The mixture was extracted with EtOAc (300 mL). The organic phase was washed sequentially with water (100 mL) and saturated brine, dried over anhydrous sodium sulfate, filtered, and concentrated under reduced pressure. The residue was purified by column chromatography (DCM/EtOAc (v/v) = 2/1) to yield the product as a yellow solid (0.58 g, 36%).
MS (ESI, pos. ion) m/z: 328.3 [M+H]$^+$.

Step 2) Synthesis of 5-((4-fluorophenyl)amino)-7-(hydroxymethyl)-3-methylquinoxalin-2(1*H*)-one

Methyl 8-((4-fluorophenyl)amino)-2-methyl-3-oxo-3,4-dihydroquinoxalin-6-carboxylate (0.57 g, 1.74 mmol) and tetrahydrofuran (20 mL) were added to a reaction flask. Lithium aluminum hydride (0.17 g, 4.52 mmol) was added under stirring at 0°C. After the addition was complete, the reaction was allowed to proceed at room temperature for 4.5 hours. The reaction was quenched by adding water (0.2 mL), followed by adding 15% sodium hydroxide solution (0.2 mL). The mixture was concentrated under reduced pressure. The residue was purified by column chromatography (DCM/MeOH(v/v) = 20/1) to give a yellow solid (85 mg, 16%).
MS (ESI, pos. ion) m/z: 300.2 [M+H]$^+$.

Step 3) Synthesis of 8-((4-fluorophenyl)amino)-2-methyl-3-oxo-3,4-dihydroquinoxalin-6-carboxaldehyde

5-((4-fluorophenyl)amino)-7-(hydroxymethyl)-3-methylquinoxalin-2(1*H*)-one (0.12 g, 0.40 mmol) was dissolved in a mixture of tetrahydrofuran (2 mL) and dichloromethane (2 mL). Dess-Martin periodinane (0.34 g, 0.80 mmol) was

added at room temperature. After the addition was complete, the reaction was continued at room temperature for 4 h. The reaction was then stopped, water (140 mL) was added, and the mixture was extracted with DCM (40 mL). The organic phase was washed successively with saturated sodium bicarbonate (60 mL) and brine, dried over anhydrous sodium sulfate, filtered, and concentrated under reduced pressure to obtain a yellow solid (0.09 g, 75%).

MS (ESI, pos. ion) m/z: 298.3 $[M+H]^+$.

Step 4) Synthesis of 6-fluoro-5-(4-((8-((4-fluorophenyl)amino)-2-methyl-3-oxo-3,4-dihydroquinoxalin-6-yl)methyl)piperazin-1-yl)-*N*-methylpicolinamide

6-Fluoro-*N*-methyl-5-(piperazin-1-yl)picolinamide (0.10 g, 0.42 mmol) was dissolved in dichloromethane (2 mL) and methanol (2 mL). Triethylamine (0.097 g, 0.96 mmol), 8-((4-fluorophenyl)amino)-2-methyl-3-oxo-3,4-dihydroquinoxalolin-6-carboxaldehyde (0.095 g, 0.32 mmol), acetic acid (0.0038 g, 0.064 mmol) and sodium cyanoborohydride (0.060 g, 0.96 mmol) were added, and the mixture was reacted at room temperature for 5 h. The solution was concentrated under reduced pressure and separated by thin-layer silica gel chromatography (DCM/MeOH(v/v) = 20/1) to give a yellow solid (0.02 g, 12%).

MS (ESI, pos. ion) *m/z:* 520.2 $[M+H]^+$;

$^1$H NMR (400 MHz, DMSO-$d_6$) δ 12.06 (s, 1H), 8.40 (d, *J* = 4.7 Hz, 1H), 8.19 (s, 1H), 7.84 (d, *J* = 7.8 Hz, 1H), 7.60 - 7.52 (m, 1H), 7.34 (dd, *J* = 8.8, 4.9 Hz, 2H), 7.17 (t, *J* = 8.8 Hz, 2H), 6.90 (s, 1H), 6.63 (s, 1H), 3.60 (s, 2H), 3.15 (s, 4H), 2.76 (d, *J* = 4.7 Hz, 3H), 2.52 (s, 4H), 2.43 (s, 3H).

**Example 34 6-fluoro-*N*-methyl-5-(4-((2-methyl-3-oxo-8-((1,1,1-trifluoropropan-2-yl)oxy)-3,4-dihydroquinoxalin-6-yl)methyl)piperazin-1-yl)picolinamide**

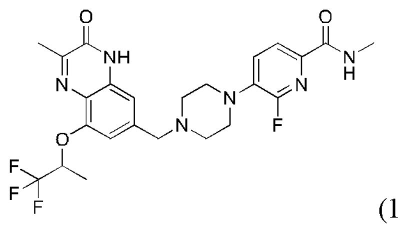

(112)

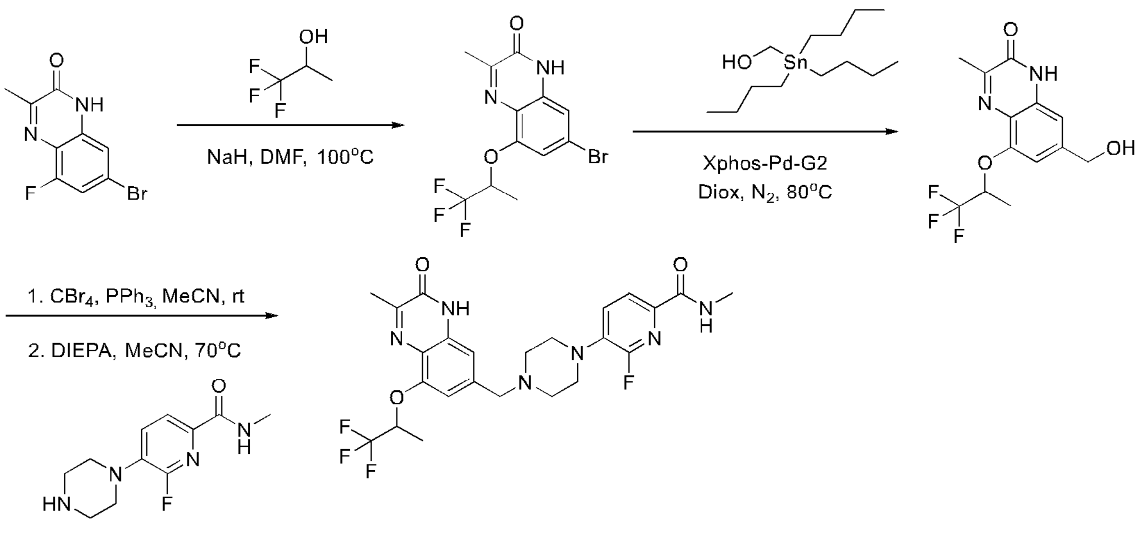


Step 1) Synthesis of 7-bromo-3-methyl-5-((1,1,1-trifluoroprop-2-yl)oxy)quinoxalin-2(1*H*)-one

Under a nitrogen atmosphere at 0 °C, 1,1,1-trifluoroprop-2-ol (2.66 g, 23.3 mmol) was added to *N,N*-dimethylformamide solution (10 ml). Sodium hydride (0.93 g, 23.3 mmol) was added in portions, and the mixture was stirred for 20 min. The mixture was then transferred to room temperature and reacted for another 30 min. Finally, 7-bromo-5-fluoro-3-methyl-1,2-dihydroquinoxalin-2-one (600 mg, 2.33 mmol) was added, and the temperature was raised to 100 °C for 6 h. The reaction solution was quenched with $H_2O$ (50 mL), and HCl (1 mol/L) aqueous solution was added dropwise until

neutral. The mixture was stirred for 1 h, filtered, and the filter cake was dried under vacuum at 50 °C to obtain a gray solid product (800 mg, 97.62%).

MS (ESI, pos. ion) *m/z:* 351.0 [M+H]+.

Step 2) Synthesis of 7-(hydroxymethyl)-3-methyl-5-((1,1,1-trifluoroprop-2-yl)oxy)quinoxalin-2(1H)-one

At room temperature, 7-bromo-3-methyl-5-((1,1,1-trifluoroprop-2-yl)oxy)quinoxalin-2(1*H*)-one (800 mg, 2.28 mmol), (tributyltin)methanol (1127.41 mg, 3.51 mmol) and chloro(2-dicyclohexylphosphino-2',4',6'-triisopropyl-1,1'-biphenyl)[2-(2'-amino-1,1'-biphenyl)]palladium(II) (269.09 mg, 0.34 mmol) catalyst were added to 1,4-dioxane (15 ml), and the reaction was carried out at 80 °C for 3 h under nitrogen protection. The reaction solution was concentrated under reduced pressure to remove the solvent, and the residue was purified by column chromatography (dichloromethane/ethyl acetate (V/V) = 1/1) to give a white solid product (485 mg, 70.43%).

MS (ESI, pos. ion) *m/z:* 303.2 [M+H]+.

Step 3) Synthesis of 6-fluoro-N-methyl-5-(4-((2-methyl-3-oxo-8-((1,1,1-trifluoropropan-2-yl)oxy)-3,4-dihydroquinoxalin-6-yl)methyl)piperazin-1-yl)picolinamide

At 0 °C, tetrabromomethane (617.64 mg, 2.35 mmol) was added to a solution of 7-(hydroxymethyl)-3-methyl-5-((1,1,1-trifluoroprop-2-yl)oxy)quinoxalin-2(1*H*)-one (350 mg, 1.16 mmol) in acetonitrile (10 mL), stirred for 20 min, and then transferred to room temperature for 1 h. Then, ethyl diisopropylamine (1357.02 mg, 10.5 mmol) and 6-fluoro-*N*-methyl-5-(piperazin-1-yl)pyridine-2-carboxamide (380 mg, 1.58 mmol) were added to the reaction solution, and the temperature was raised to 70 °C, and the reaction was stirred for 2 h. After cooling, the reaction solution was concentrated under reduced pressure to remove the solvent. The residue was purified by silica gel column chromatography (dichloromethane/methanol (v/v) = 20/1) to give a white solid product (510 mg, 92.52%).

MS (ESI, pos. ion) *m/z:* 523.4 [M+H]+;
$^1$H NMR (400 MHz, DMSO-$d_6$) δ 12.29 (s, 1H), 8.39 (d, *J* = 4.4 Hz, 1H), 7.84 (d, *J* = 7.8 Hz, 1H), 7.57 (t, *J* = 9.2 Hz, 1H), 6.98 (s, 2H), 5.32 (dt, *J* = 12.7, 6.3 Hz, 1H), 3.58 (s, 2H), 3.18 (s, 4H), 2.76 (d, *J* = 4.6 Hz, 3H), 2.56 (s, 4H), 2.40 (s, 3H), 1.52 (d, *J* = 6.1 Hz, 3H).

**Example 35 *N*-cyclopropyl-6-fluoro-5-(4-((2-methyl-3-oxo-8-(prop-1-yn-1-yl)-3,4-dihydroquinoxalin-6-yl)methyl) piperazin-1-yl)picolinamide**

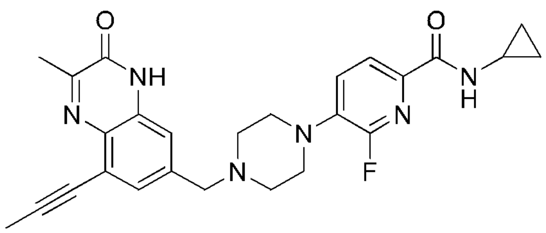

(113)

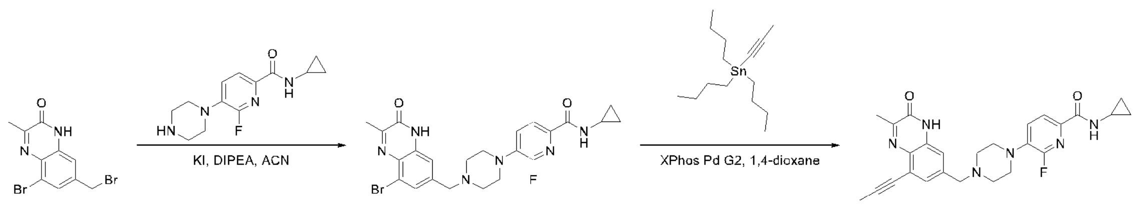


Step 1) Synthesis of 5-(4-((8-bromo-2-methyl-3-oxo-3,4-dihydroquinoxalin-6-yl)methyl)piperazin-1-yl)-N-cyclopropyl-6-fluoropicolinamide

At room temperature, *N*-cyclopropyl-6-fluoro-5-(piperazin-1-yl)picolinamide (0.48 g, 1.83 mmol), DIPEA (1.07 g, 8.30 mmol), and potassium iodide (0.014 g, 0.083 mmol) were added to a solution of 5-bromo-7-(bromomethyl)-3-methylquinoxalin-2(1*H*)-one (0.55 g, 1.66 mmol) in ACN (20 mL), and then the reaction was heated to 80 °C for 3 h. The reaction solution was concentrated under reduced pressure, and the residue was purified by silica gel column chromatography

graphy (eluting agent: $CH_2Cl_2$/MeOH (v/v) = 20/1) to give a yellow solid product (0.45 g, 52.7%).

MS (ESI, pos. ion) *m/z:* 515.0 $[M+H]^+$;

Step 2) Synthesis of N-cyclopropyl-6-fluoro-5-(4-((2-methyl-3-oxo-8-(prop-1-yn-1-yl)-3,4-dihydroquinoxalin-6-yl) methyl)piperazin-1-yl)picolinamide

At room temperature, tributyl(prop-1-yn-1-yl)stanane (0.52 g, 1.57 mmol) and Xphos Pd G2 (0.10 g, 0.13 mmol) were added to 1,4-dioxane (20 mL) of 5-(4-((8-bromo-2-methyl-3-oxo-3,4-dihydroquinoxolin-6-yl)methyl)piperazin-1-yl)-N-cyclopropyl-6-fluoropicolinamide (0.45 g, 0.87 mmol) and $N_2$ were replaced. The mixture was then heated to 90 °C and stirred for 12 h. After the reaction solution cooled to room temperature, it was filtered through diatomaceous earth. The filtrate was concentrated under reduced pressure, and the residue was purified by silica gel column chromatography (eluent: DCM/MeOH (v/v) = 20/1) to give a yellow solid product (0.061 g, 14.72%).

MS (ESI, pos. ion) *m/z:* 475.2 $[M+H]^+$;

$^1$H NMR (400 MHz, DMSO-$d_6$) δ (ppm) 12.32 (s, 1H), 8.37 - 8.32 (m, 1H), 7.84 (d, *J* = 8.0 Hz, 1H), 7.56 (t, *J* = 9.3 Hz, 1H), 7.29 (s, 1H), 7.20 (s, 1H), 3.57 (s, 2H), 3.19 - 3.12 (m, 4H), 2.87 - 2.82 (m, 1H), 2.62 - 2.52 (m, 7H), 2.41 (s, 3H), 2.13 (s, 3H), 1.40 - 1.30 (m, 4H), 0.68 - 0.61 (m, 4H).

**Example 36 5-(4-((2-methyl-3-oxo-8-(prop-1-yn-1-yl)-3,4-dihydroquinoxalin-6-yl)methyl)piperazin-1-yl)picolinonitrile**

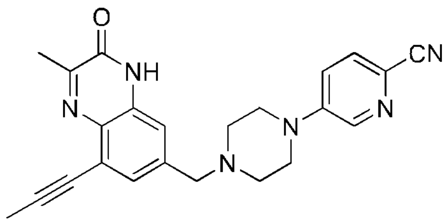

(114)

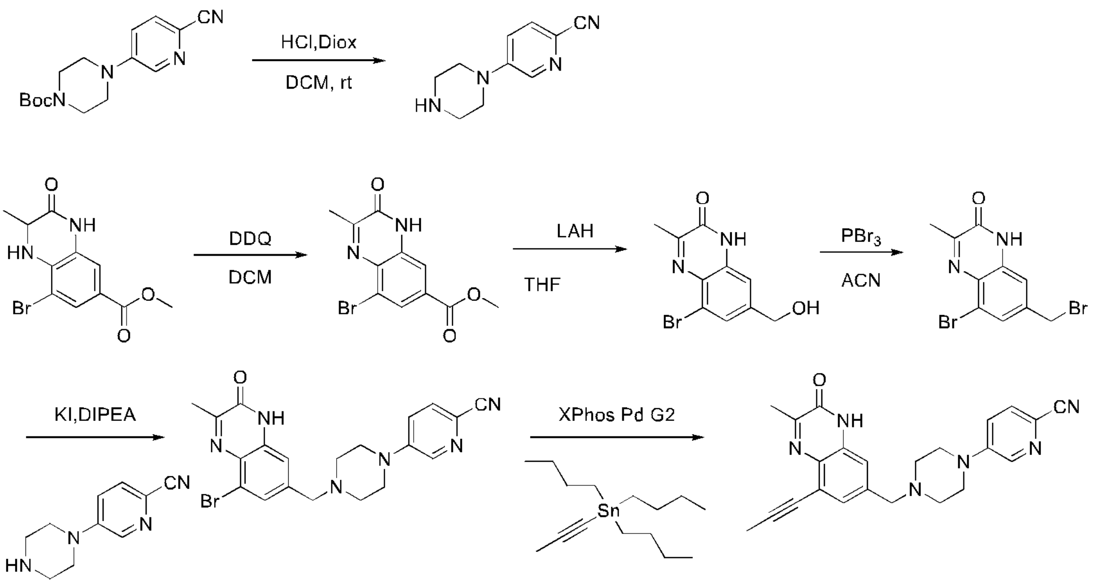


Step 1) Synthesis of methyl 8-bromo-2-methyl-3-oxo-3,4-dihydroquinoxalin-6-carboxylate

Under ice bath conditions, 2,3-dichloro-5,6-dicyano-1,4-benzoquinone (3.98 g, 17.55 mmol) was added in portions to a solution of methyl 8-bromo-2-methyl-3-oxo-1,2,3,4-tetrahydroquinoxalin-6-carboxylate (3.5 g, 11.70 mmol) in dichloromethane (100 mL). The reaction mixture was then brought to room temperature and reacted for 10 h. The reaction solution was concentrated under reduced pressure, and saturated sodium bicarbonate (100 mL) was added at 0 °C. The mixture was then brought to room temperature and stirred for 1 h. After filtration, the filter cake was washed with

water (50 mL) to give an orange-yellow solid product (3.23 g, 92.91%).
MS (ESI, pos. ion) *m/z:* 297.05 $[M+H]^+$.

Step 2) Synthesis of 5-bromo-7-(hydroxymethyl)-3-methylquinoxalin-2(1H)-one

At 0 °C and $N_2$, lithium aluminum hydride (0.83 g, 21.74 mmol) was added in portions to a tetrahydrofuran (100 mL) solution of methyl 8-bromo-2-methyl-3-oxo-3,4-dihydroquinoxalin-6-carboxylate (3.23 g, 10.87 mmol), and the mixture was then stirred at room temperature for 5 h. The sample was quenched with water at 0°C, and then 1M hydrochloric acid was added dropwise to pH 3-4. Saturated brine (30 mL) was added, followed by extraction with tetrahydrofuran (50 mL × 3). The sample was dried over anhydrous sodium sulfate, filtered, concentrated under reduced pressure, and the residue was purified by silica gel column chromatography (eluent: dichloromethane/methanol (v/v) = 97/5) to give a brown solid product (1.77 g, 60.05%).
MS (ESI, neg. ion) *m/z:* 267.0 $[M-H]^-$.

Step 3) Synthesis of 5-(piperazin-1-yl)pyridinium nitrile hydrochloride

At room temperature, 4M dioxane hydrochloride solution (5 mL) was added to ethyl acetate (20 mL) of 4-(6-cyanopyridin-3-yl)piperazine-1-carboxylic acid *tert*-butyl ester (0.95 g, 3.29 mmol) and reacted for 2 h. After the reaction was complete, the reaction solution was directly filtered to obtain a solid product, which was dried at room temperature to give a pale yellow solid (0.71 g, 96.75%).
MS (ESI, pos. ion) *m/z:* 189.25 $[M+H]^+$.

Step 4) Synthesis of 5-bromo-7-(bromomethyl)-3-methylquinoxalin-2(1*H*)-one

At 0 °C and $N_2$, phosphorus tribromide (0.24 mL, 2.60 mmol) was added dropwise to a solution of 5-bromo-7-(hydroxymethyl)-3-methylquinoxalin-2(1*H*)-one (0.5 g, 1.86 mmol) in acetonitrile (20 mL), and the mixture was stirred for 5-10 min. The temperature was then increased to 35 °C for 4 h. TLC results showed complete reaction, and the reaction was directly proceeded to the next step without further processing.

Step 5) Synthesis of 5-(4-((8-bromo-2-methyl-3-oxo-3,4-dihydroquinoxalin-6-yl)methyl)piperazin-1-yl)pyridine nitrile

In an ice bath, potassium iodide (15 mg, 0.091 mmol), 5-(piperazin-1-yl)pyridine nitrile (0.51 g, 1.71 mmol), and *N,N*-diisopropylethylamine (4.79 mL, 28.96 mmol) were added to a solution of 5-bromo-7-(bromomethyl)-3-methylquinoxalin-2(1*H*)-one (0.6 g, 1.81 mmol) in acetonitrile (20 mL), and the mixture was heated to room temperature for 2 h. After the reaction was complete, the reaction solution was diluted with dichloromethane (30 mL), washed with water (20 mL × 2), concentrated under reduced pressure, and the residue was purified by silica gel column chromatography (eluent: dichloromethane/methanol (v/v) = 97/4) to obtain an orange-yellow solid powder (0.35 g, 44.71%).
MS (ESI, pos. ion) *m/z:* 439.10 $[M+H]^+$;

Step 6) Synthesis of 5-(4-((2-methyl-3-oxo-8-(prop-1-yn-1-yl)-3,4-dihydroquinoxalin-6-yl)methyl)piperazin-1-yl)picolinonitrile

At room temperature, tributyl(prop-1-yn-1-yl)tin (0.19 mL, 0.61 mmol) was added to a solution of 5-(4-((8-bromo-2-methyl-3-oxo-3,4-dihydroquinoxolin-6-yl)methyl)piperazin-1-yl)picolinonitrile (0.15 g, 0.31 mmol) in 1,4-dioxane (15 mL), followed by the addition of XPhos Pd G2 (0.040 g, 0.051 mmol). The reaction mixture was then replaced with $N_2$ and reacted overnight at 90 °C. The reaction solution was filtered through diatomaceous earth, the organic phase was concentrated under reduced pressure, and the residue was purified by silica gel column chromatography (eluent: dichloromethane/methanol (v/v) = 96/4) to give a white solid powder (51 mg, 37.49%).
MS (ESI, pos. ion) *m/z:* 399.18 $[M+H]^+$
$^1H$ NMR (400 MHz, DMSO-$d_6$) δ 12.34 (s, 1H), 8.44-8.37 (m, 1H), 7.77-7.70 (m, 1H), 7.35 (dd, *J* = 8.9, 3.0 Hz, 1H), 7.30-7.19 (m, 2H), 3.56 (s, 2H), 3.43-3.39 (m, 4H), 2.55-2.49 (m, 10H), 2.41 (s, 3H), 2.13 (s, 3H).

**Example 37 7-((4-(6-(difluoromethoxy)pyridin-3-yl)piperazin-1-yl)methyl)-3-methyl-5-(prop-1-yn-1-yl)quinoxalin-2(1*H*)-one**

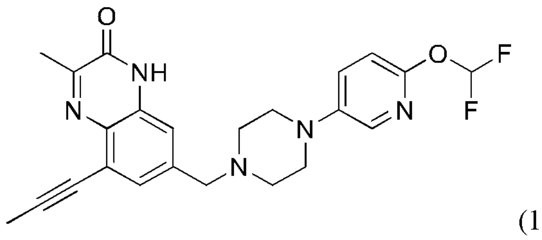

(115)

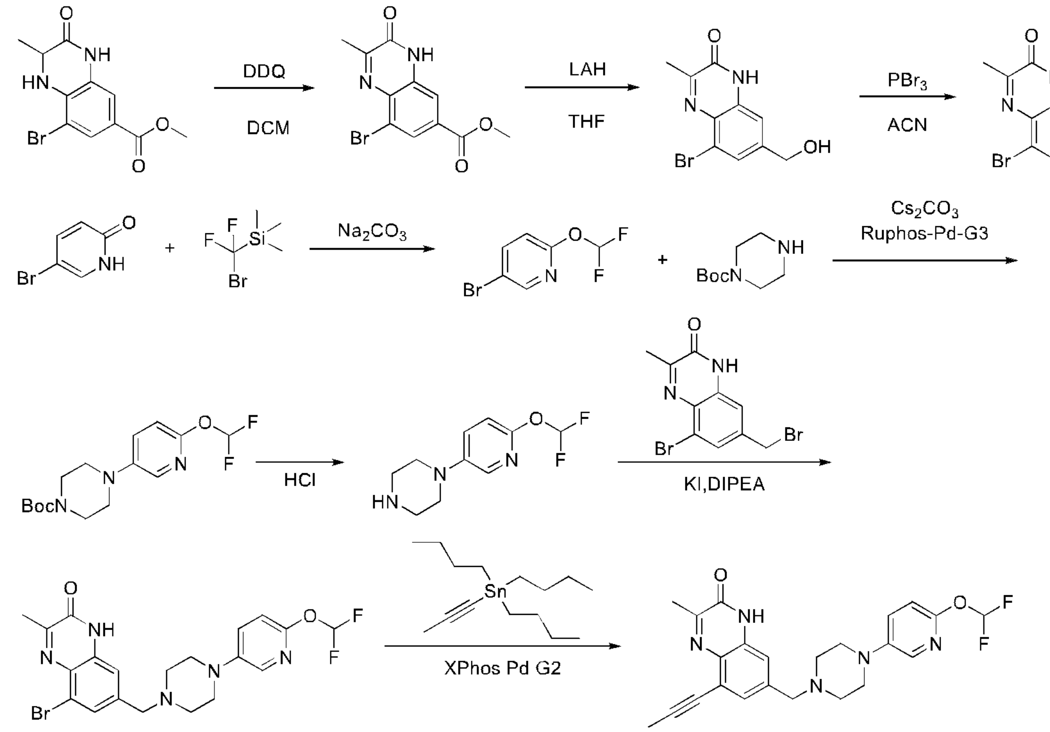


Step 1) Synthesis of methyl 8-bromo-2-methyl-3-oxo-3,4-dihydroquinoxalin-6-carboxylate

Under ice bath conditions, 2,3-dichloro-5,6-dicyano-1,4-benzoquinone (5.69 g, 25.08 mmol) was added in portions to a solution of methyl 8-bromo-2-methyl-3-oxo-1,2,3,4-tetrahydroquinoxalin-6-carboxylate (5 g, 16.72 mmol) in 150 mL of dichloromethane. The reaction mixture was then brought to room temperature and reacted for 6 h. The reaction solution was concentrated under reduced pressure, and saturated sodium bicarbonate aqueous solution (100 mL) was added at 0 °C. The mixture was then heated to room temperature and stirred for 3 h. After filtration, the filter cake was washed with water (50 mL × 3) to obtain an orange-yellow solid product (4.7 g, 94.64%).
MS (ESI, pos. ion) *m/z:* 297.15 $[M+H]^+$.

Step 2) Synthesis of 5-bromo-7-(hydroxymethyl)-3-methylquinoxalin-2(1*H*)-one

At 0 °C and $N_2$, lithium aluminum hydride (1.12 g, 29.62 mmol) was added in portions to a tetrahydrofuran (100 mL) solution of methyl 8-bromo-2-methyl-3-oxo-3,4-dihydroquinoxalin-6-carboxylate (4.4 g, 14.81 mmol), and the reaction was stirred at room temperature for 8 h. The reaction was quenched with water at 0 °C, and then 1 M hydrochloric acid was added dropwise to pH 3-4. Saturated brine was then added, and the mixture was extracted with tetrahydrofuran (100 mL × 3). The extract was dried over anhydrous sodium sulfate, filtered, concentrated under reduced pressure, and the residue was purified by silica gel column chromatography (eluent: dichloromethane/methanol (v/v) = 97/5) to give a brown solid product (3.36 g, 84.31%).
MS (ESI, neg. ion) *m/z:* 267.00 $[M-H]^-$.

Step 3) Synthesis of 5-bromo-7-(bromomethyl)-3-methylquinoxalin-2(1*H*)-one

Phosphorus tribromide (0.24 mL, 2.60 mmol) was added dropwise to a 25 mL solution of 5-bromo-7-(hydroxymethyl)-3-methylquinoxalin-2(1*H*)-one (0.5 g, 1.86 mmol) in acetonitrile and stirred for 5-10 min, then the temperature was increased to 35 °C and the reaction was carried out for 4 h. TLC results showed complete reaction, and the reaction was directly proceeded to the next step without further processing.

Step 4) Synthesis of 5-bromo-2-(difluoromethoxy)pyridine

At room temperature, sodium carbonate (6.70 g, 63.22 mmol) was added to a solution of 5-bromo-1,2-dihydropyridin-2-one (10 g, 57.47 mmol) in acetonitrile (120 mL). The mixture was heated to 60 °C and stirred for 1 h. Then, (bromodifluoromethyl)trimethylsilane (14.01 g, 68.96 mmol) was slowly added, and the reaction was continued at 60 °C with stirring. After the reaction was complete, water (100 mL) was added to the reaction solution, and the mixture was extracted with DCM (150 mL × 3). The organic phases were combined, washed with saturated brine, dried over anhydrous sodium sulfate, filtered, and the filtrate was concentrated under reduced pressure and dried to give a yellow solid product (11.5 g, 89.33%).

Step 5) Synthesis of 4-(6-(difluoromethoxy)pyridin-3-yl)piperazine-1-carboxylic acid *tert*-butyl ester

At room temperature, Ruphos-Pd-G3 (0.52 g, 0.63 mmol) was added to a solution of 5-bromo-2-(difluoromethoxy)pyridine (2 g, 8.93 mmol), piperazine-1-carboxylic acid *tert*-butyl ester (1.75 g, 9.38 mmol), and cesium carbonate (5.82 g, 17.86 mmol) in 1,4-dioxane (60 mL). The solution was then replaced with $N_2$, and the reaction was carried out overnight at 110 °C. The mixture was filtered through diatomaceous earth, concentrated under reduced pressure, and purified by silica gel column chromatography (eluent: PE:EtOAc (v/v) = 6/1) to give a yellow solid product (2.6 g, 88.42%).

MS (ESI, pos. ion) *m/z:* 330.20 $[M+H]^+$.

Step 6) Synthesis of 1-(6-(difluoromethoxy)pyridin-3-yl)piperazine

At room temperature, 4M dioxane hydrochloride solution (30.4 mmol) was added to ethyl acetate (20 mL) containing of 4-(6-(difluoromethoxy)pyridin-3-yl)piperazine-1-carboxylic acid *tert*-butyl ester (1 g, 3.04 mmol), and the reaction was allowed to proceed overnight. After the reaction was complete, the reaction solution was directly filtered to obtain a solid product, which was dried at room temperature to give a pale yellow solid (0.62 g, 89.08%).

MS (ESI, pos. ion) *m/z:* 230.10 $[M+H]^+$.

Step 7) Synthesis of 5-bromo-7-((4-(6-(difluoromethoxy)pyridin-3-yl)piperazin-1-yl)methyl)-3-methylquinoxalin-2(1H)-one

At room temperature, potassium iodide (15 mg, 0.091 mmol), 1-(6-(difluoromethoxy)pyridin-3-yl)piperazine (0.62 g, 2.71 mmol), and *N,N*-diisopropylethylamine (4.79 mL, 28.96 mmol) were added to a solution of 5-bromo-7-(bromomethyl)-3-methylquinoxalin-2(1*H*)-one (0.6 g, 1.81 mmol) in acetonitrile (25 mL), and the mixture was heated to room temperature and reacted for 2 h. After the reaction was completed, the reaction solution was diluted with dichloromethane, washed with water (20 mL × 3) three times, concentrated under reduced pressure, and the residue was purified by silica gel column chromatography (eluent: dichloromethane/methanol (v/v) = 97/4) to obtain an orange-yellow solid powder (0.42 g, 48.38%).

MS (ESI, pos. ion) *m/z*: 479.95 $[M+H]^+$;

Step 8) Synthesis of 7-((4-(6-(difluoromethoxy)pyridin-3-yl)piperazin-1-yl)methyl)-3-methyl-5-(prop-1-yn-1-yl)quinoxalin-2(1*H*)-one

At room temperature, tributyl(prop-1-yn-1-yl)tin (0.23 mL, 0.76 mmol) was added to a solution of 5-bromo-7-((4-(6-(difluoromethoxy)pyridin-3-yl)piperazin-1-yl)methyl)-3-methylquinoxalin-2(1*H*)-one (0.2 g, 0.42 mmol) in 1,4-dioxane (15 mL), followed by the addition of XPhos Pd G2 (0.050 g, 0.063 mmol). The mixture was then replaced with $N_2$, and the temperature was raised to 90 °C and reacted overnight. The reaction solution was filtered through diatomaceous earth, the organic phase was concentrated under reduced pressure, and the residue was purified by silica gel column chromatography (eluent: dichloromethane/methanol (v/v) = 96/4) to give a white solid powder (22 mg, 12.02%).

MS (ESI, pos. ion) *m/z:* 440.19 $[M+H]^+$.

$^1H$ NMR (400 MHz, DMSO-$d_6$) δ 7.89 - 7.84 (m, 1H), 7.54(t, *J* = 73.6, 1H), 7.56 - 7.51 (m, 1H), 7.31 - 7.18 (m, 2H), 6.98 - 6.93 (m, 1H), 3.16 - 3.11 (m, 4H), 2.57 - 2.51 (m, 4H), 2.40 (s, 3H), 2.13 (s, 3H).

**Example 38 *N*,6-dimethyl-5-(4-((2-methyl-3-oxo-8-(prop-1-yn-1-yl)-3,4-dihydroquinoxalin-6-yl)methyl)piperazin-1-yl)picolinamide**

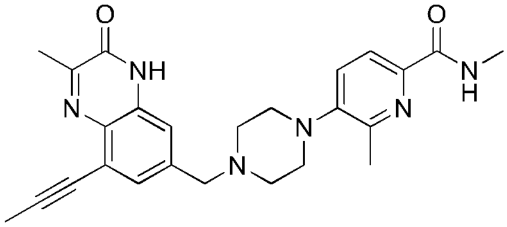

(116)

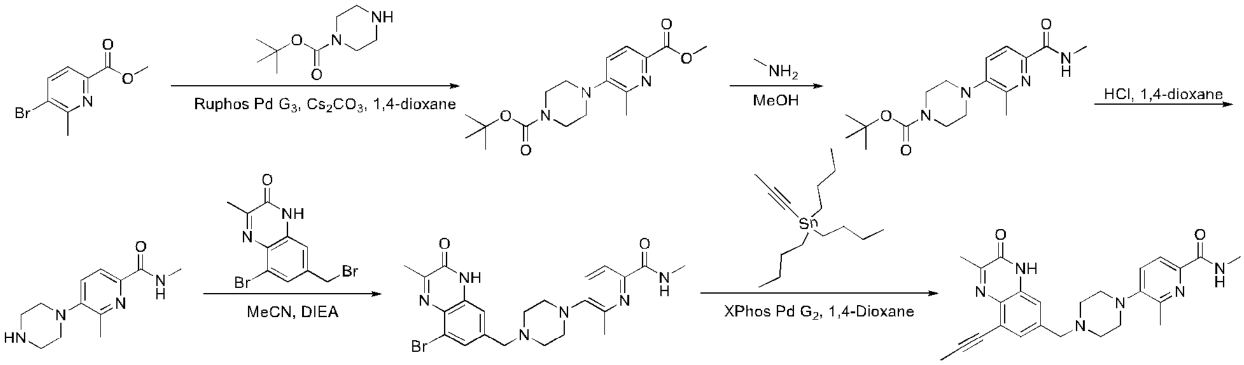


Step 1) Synthesis of *tert*-butyl 4-(6-(methoxycarbonyl)-2-methylpyridin-3-yl)piperazine-1-carboxylate

At room temperature, Ruphos Pd G3 (0.64 g, 0.76 mmol) was added to a solution of methyl 5-bromo-6-methylpyridin-2-carboxylic acid (5 g, 21.73 mmol), piperazine-1-carboxylic acid *tert*-butyl ester (4.25 g, 22.82 mmol), and $Cs_2CO_3$ (14.16 g, 43.46 mmol) in 1,4-dioxane (250 mL), replacing $N_2$, and then heated to 110 °C with stirring for 12 h. After cooling, the reaction solution was filtered with diatomaceous earth. The filtrate was concentrated under reduced pressure, and the residue was purified by silica gel column chromatography (eluting agent: PE/EtOAc (v/v) = 1/1) to obtain a yellow solid product (5.7 g, 78.20%).

MS (ESI, pos. ion) *m/z:* 336.2 $[M+H]^+$;

Step 2) Synthesis of *tert*-butyl 4-(2-methyl-6-(methylcarbamoyl)pyridin-3-yl)piperazine-1-carboxylate

Methylamine (15.83 g, 509.70 mmol) was added to a MeOH (100 mL) solution of *tert*-butyl 4-(6-methoxycarbonyl)-2-methylpyridin-3-yl)piperazine-1-carboxylate (5.7 g, 16.99 mmol) at room temperature, and the mixture was stirred for 8 h at room temperature. The reaction solution was concentrated under reduced pressure, and the residue was purified by silica gel column chromatography (eluting solvent: PE/EtOAc (v/v) = 1/1) to give a yellow solid product (5.6 g, 98.54%).

MS (ESI, pos. ion) *m/z:* 335.2 $[M+H]^+$;

Step 3) Synthesis of *N*,6-dimethyl-5-(piperazin-1-yl)picolinamide

At room temperature, HCl-1,4-dioxane (3.05 g, 83.75 mmol, 4 M) was added to a solution of *tert*-butyl 4-(2-methyl-6-(methoxycarbonyl)pyridin-3-yl)piperazin-1-carboxylate (5.6 g, 16.75 mmol) in 1,4-dioxane (20 mL), and the mixture was stirred at room temperature for 3 h. The reaction solution was filtered, and the resulting solid was added to DCM (50 mL). The pH was adjusted to 9-10 with triethylamine, and the mixture was concentrated under reduced pressure. The residue was purified by silica gel column chromatography (eluent: $CH_2Cl_2$/MeOH (v/v) = 10/1) to give a yellow solid product (3.63 g, yield 92.52%).

MS (ESI, pos. ion) *m/z:* 235.2 $[M+H]^+$;

Step 4) Synthesis of 5-(4-((8-bromo-2-methyl-3-oxo-3,4-dihydroquinoxalin-6-yl)methyl)piperazin-1-yl)-N,6-dimethylpicolinamide

At room temperature, *N*,6-dimethyl-5-(piperazin-1-yl)picolinamide (0.51 g, 2.17 mmol), DIPEA (1.17 g, 9.05 mmol), and KI (0.015 g, 0.091 mmol) were added to a solution of 5-bromo-7-(bromomethyl)-3-methylquinoxalin-2(1H)-one (0.6 g, 1.81 mmol) in ACN (20 mL), followed by reaction at 80 °C for 3 h. The reaction solution was concentrated under reduced pressure, and the residue was purified by silica gel column chromatography (eluent: $CH_2Cl_2$/MeOH (v/v) = 20/1) to give a yellow solid product (0.21 g, 23.94%).
MS (ESI, pos. ion) *m/z:* 485.2 $[M+H]^+$;

Step 5) Synthesis of *N*,6-dimethyl-5-(4-((2-methyl-3-oxo-8-(prop-1-yn-1-yl)-3,4-dihydroquinoxalin-6-yl)methyl)piperazin-1-yl)picolinamide

At room temperature, tributyl(prop-1-yn-1-yl)stannane (0.24 g, 0.74 mmol) and Xphos Pd G2 (0.048 g, 0.061 mmol) were added to a solution of 5-(4-((8-bromo-2-methyl-3-oxo-3,4-dihydroquinoxolin-6-yl)methyl)piperazin-1-yl)-N,6-dimethylpicolinamide (0.2 g, 0.41 mmol) in 1,4-dioxane (20 mL). After replacing $N_2$, the mixture was heated to 90 °C and stirred for 12 h. After the reaction solution cooled to room temperature, it was filtered through diatomaceous earth. The filtrate was concentrated under reduced pressure, and the residue was purified by silica gel column chromatography (eluent: DCM/MeOH (v/v) = 20/1) to give a yellow solid product (0.02 g, 10.92%).
MS (ESI, pos. ion) *m/z:* 445.3 $[M+H]^+$;
$^1$H NMR (400 MHz, DMSO-$d_6$) δ (ppm) 12.35 (s, 1H), 8.42 (s, 1H), 7.79 (d, *J* = 7.9 Hz, 1H), 7.47 (d, *J* = 8.3 Hz, 1H), 7.30 (s, 1H), 7.21 (s, 1H), 3.58 (s, 2H), 3.31 - 3.20 (m, 4H), 2.95 (s, 3H), 2.82 - 2.77 (m, 3H), 2.61 - 2.55 (m, 4H), 2.41 (s, 3H), 2.13 (s, 3H).

**Example 39 *N*-cyclopropyl-2-fluoro-1'-((2-methyl-3-oxo-8-(prop-1-yn-1-yl)-3,4-dihydroquinoxalin-6-yl) methyl)-1',2',3',6'-tetrahydro-[3,4'-bipyridine]-6-carboxamide**

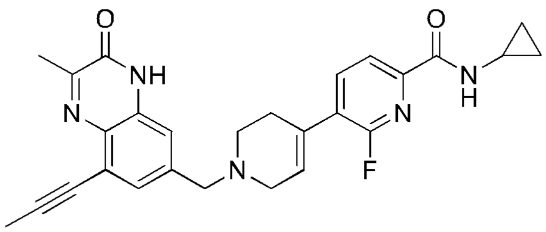

(117)

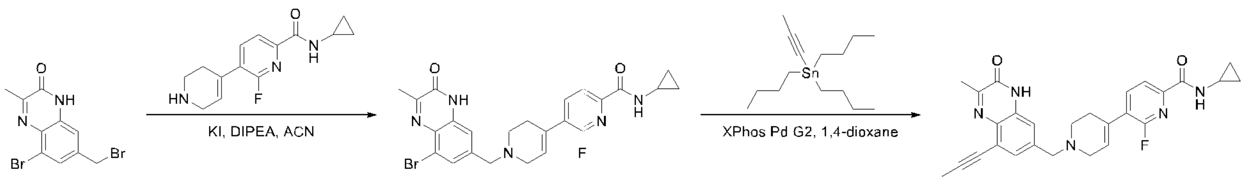


Step 1) Synthesis of 1'-((8-bromo-2-methyl-3-oxo-3,4-dihydroquinoxalin-6-yl)methyl)-*N*-cyclopropyl-2-fluoro-1',2',3',6'-tetrahydro-[3,4'-bipyridine]-6-carboxamide

At room temperature, *N*-cyclopropyl-2-fluoro-1',2',3',6'-tetrahydro-[3,4'-bipyridine]-6-carboxamide (0.71 g, 2.71 mmol), DIPEA (1.17 g, 9.05 mmol) and KI (0.015 g, 0.091 mmol) were added to a solution of 5-bromo-7-(bromomethyl)-3-methylquinoxalin-2(1*H*)-one (0.6 g, 1.81 mmol) in ACN (20 mL), followed by reaction at 80 °C for 3 h. The reaction solution was concentrated under reduced pressure, and the residue was purified by silica gel column chromatography (eluent: $CH_2Cl_2$/MeOH (v/v) = 20/1) to give a yellow solid product (0.9 g, 97.19%).
MS (ESI, pos. ion) *m/z:* 512.9 $[M+H]^+$;

Step 2) Synthesis of *N*-cyclopropyl-2-fluoro-1'-((2-methyl-3-oxo-8-(prop-1-yn-1-yl)-3,4-dihydroquinoxalin-6-yl) methyl)-1,',2',3 ',6'-tetrahydro-[3,4'-bipyridine]-6-carboxamide

At room temperature, tributyl(prop-1-yn-1-yl)tin (0.20 g, 0.59 mmol) and Xphos Pd G2 (0.039 g, 0.050 mmol) were added to a solution of 1'-((8-bromo-2-methyl-3-oxo-3,4-dihydroquinoxalin-6-yl)methyl)-*N*-cyclopropyl-2-fluoro-1',2',3',6'-tetrahydro-[3,4'-bipyridine]-6-carboxamide (0.17 g, 0.33 mmol) in 1,4-dioxane (20 mL), $N_2$ was replaced, and the mixture was heated to 90 °C and stirred for 12 h. After the reaction solution cooled to room temperature, it was filtered through diatomaceous earth. The filtrate was concentrated under reduced pressure, and the residue was purified by silica gel column chromatography (eluent: DCM/MeOH (v/v) = 20/1) to give a yellow solid product (0.08 g, 51.13%).

MS (ESI, pos. ion) *m/z:* 472.0 $[M+H]^+$;

$^1$H NMR (400 MHz, DMSO-$d_6$) δ (ppm) 12.32 (s, 1H), 8.61 (s, 1H), 8.10 - 8.06 (m, 1H), 7.95 - 7.88 (m, 1H), 7.30 (s, 1H), 7.23 (s, 1H), 6.27 - 6.22 (m, 1H), 3.63 (s, 2H), 3.15 - 3.11 (m, 2H), 2.90 - 2.87 (m, 1H), 2.72 - 2.63 (m, 4H), 2.41 (s, 3H), 2.13 (s, 3H), 0.70 - 0.65 (m, 4H).

**Example 40 5-(4-((5-fluoro-2-methyl-3-oxo-8-(prop-1-yn-1-yl)-3,4-dihydroquinoxalin-6-yl)methyl)piperazin-1-yl)-*N*-methylpicolinamide**

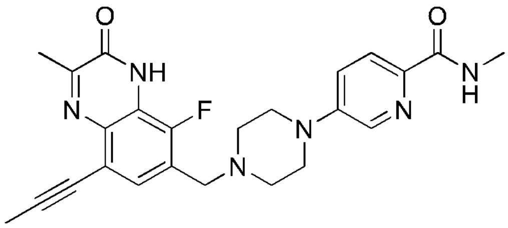

(118)

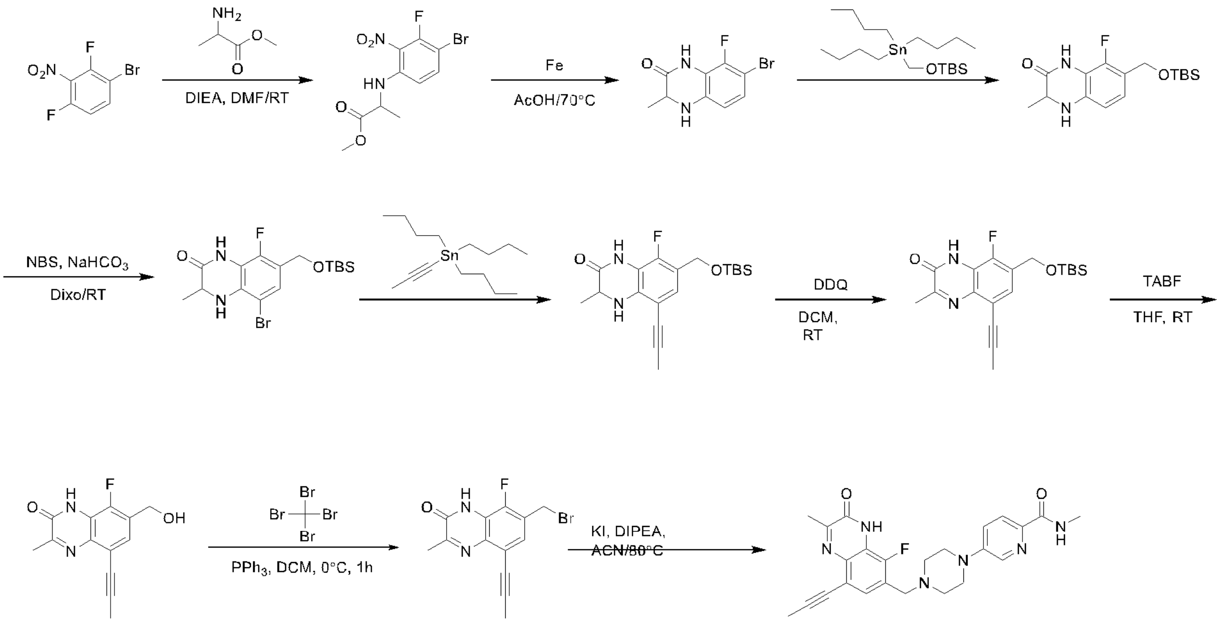


Step 1) Synthesis of Methyl (4-bromo-3-fluoro-2-nitrophenyl)malonate

Ethyl diisopropylamine (16.29 g, 126.06 mmol) was added dropwise to a solution of 1-bromo-2,4-difluoro-3-nitrobenzene (10 g, 42.02 mmol) and methyl 2-aminopropionate (4.42 g, 42.86 mmol), followed heating to room temperature overnight. The reaction was stopped, the reaction solution was diluted with DCM, washed three times with water (50 mL), concentrated under reduced pressure, and the residue was purified by silica gel column chromatography (PE/EtOAc(v/v) = 6/1) to give a yellow solid product (6 g, 44.47%).

MS (ESI, pos. ion) m/z: 321.00 $[M+H]^+$.

Step 2) Synthesis of 7-bromo-8-fluoro-3-methyl-3,4-dihydroquinoxalin-2(1*H*)-one

At room temperature, iron powder (8.35 g, 149.48 mmol) was added to a solution of methyl (4-bromo-3-fluoro-2-nitrophenyl)malonate (12 g, 37.37 mmol) in acetic acid (100 mL) with stirring. The reaction was carried out at 70 °C and monitored by TLC. After cooling to room temperature, the reaction solution was diluted with ethyl acetate (300 mL), and the insoluble matter was filtered off. The organic phase was washed with water (100 mL × 1) and saturated brine (100 mL × 3) to give a yellow solid product (9.2 g, 95.02%).
MS (ESI, pos. ion) m/z: 259.00 $[M+H]^+$.

Step 3) Synthesis of 7-(((*tert*-butyldimethylsilyl)oxy)methyl)-8-fluoro-3-methyl-3,4-dihydroquinoxalin-2(1H)-one

At room temperature, chloro(2-dicyclohexylphosphine-2',4',6'-triisopropyl-1,1'-biphenyl)[2-(2'-amino-1,1'-biphenyl))palladium(II) (1.40 g, 1.78 mmol) was added to a solution of 7-bromo-8-fluoro-3-methyl-3,4-dihydroquinoxalin-2(1*H*)-one (9.2 g, 35.51 mmol) and *tert*-butyldimethyl((tributyltin)methoxy)silane (20.10 g, 46.16 mmol), followed by replacement with $N_2$ and heating to 90 °C overnight. The reaction was stopped, the reaction solution was cooled to room temperature, filtered through diatomaceous earth, concentrated under reduced pressure, and the residue was purified by silica gel column chromatography (PE/EtOAc (v/v) = 4/1) to give a pale yellow solid product (8.1 g, 70.30%).
MS (ESI, pos. ion) m/z: 325.20 $[M+H]^+$.

Step 4) Synthesis of 5-bromo-7-(((*tert*-butyldimethylsilyl)oxy)methyl)-8-fluoro-3-methyl-3,4-dihydroquinoxalin-2(1*H*)-one

Under $N_2$ and 0 °C, *N*-bromosuccinimide (4.66 g, 26.21 mmol) was added to a solution of 7-(((*tert*butyldimethylsilyl)oxy)methyl)-8-fluoro-3-methyl-3,4-dihydroquinoxalin-2(1*H*)-one (8.1 g, 24.96 mmol) and sodium bicarbonate (3.15 g, 37.44 mmol) in 1,4-dioxane (100 mL). The mixture was then brought to room temperature. TLC results showed the reaction was complete. The reaction was stopped, and 100 mL of saturated sodium thiosulfate aqueous solution was added to quench the reaction. EtOAc was extracted (100 mL × 2). The organic phase was dried over anhydrous sodium sulfate, concentrated under reduced pressure, and the residue was purified by silica gel column chromatography (PE/EtOAc(v/v) = 4/1) to give a yellow solid product (9.8 g, yield 97.32%).
MS (ESI, pos. ion) m/z: 402.90 $[M+H]^+$.

Step 5) Synthesis of 7-(((*tert*-butyldimethylsilyl)oxy)methyl)-8-fluoro-3-methyl-5-(prop-1-yn-1-yl)-3,4-dihydroquinoxalin-2(1*H*)-one

At room temperature, XPhos-G2-Pd (0.20 g, 0.25 mmol) was added to a solution of 5-bromo-7-(((*tert*butyldimethylsilyl)oxy)methyl)-8-fluoro-3-methyl-3,4-dihydroquinoxalin-2(1*H*)-one (2 g, 4.96 mmol) in tributyl(prop-1-yn-yl)stanane (2.12 g, 6.45 mmol), and then the reaction was carried out at 90 °C for 12 h. The reaction was stopped, the reaction solution was diluted with DCM (100 mL), filtered with diatomaceous earth, the organic phase was concentrated under reduced pressure, and the residue was purified by silica gel column chromatography (PE/EtOAc(v/v) = 9/1) to give a pale yellow solid product (1.6 g, 89.01%).
MS (ESI, pos. ion) m/z: 363.20 $[M+H]^+$.

Step 6) Synthesis of 7-(((*tert*-butyldimethylsilyl)oxy)methyl)-8-fluoro-3-methyl-5-(prop-1-yn-1-yl)quinoxalin-2(1*H*)-one

A solution of 7-(((*tert*-butyldimethylsilyl)oxy)methyl)-8-fluoro-3-methyl-5-(prop-1-yn-1-yl)-3,4-dihydroquinoxalin-2(1*H*)-one (4 g, 11.03 mmol) in dichloromethane (120 mL) was cooled to 0 °C. 2,3-dichloro-5,6-dicyano-1,4-benzoquinone (2.75 g, 12.13 mmol) was added, and the reaction was allowed to proceed overnight at room temperature, and then the reaction was stopped. The reaction solution was then concentrated under reduced pressure, and saturated $NaHCO_3$ was slowly added at room temperature until no more bubbles were produced. The mixture was stirred at room temperature for 6 h, filtered, and the filter cake was washed with water until the filtrate was colorless. The obtained filter cake was dissolved in methanol (50 mL), the product was concentrated under reduced pressure to give a light yellow solid product (3.8 g, 95.53%).
MS (ESI, pos. ion) m/z: 361.20 $[M+H]^+$.

Step 7) Synthesis of 8-fluoro-7-(hydroxymethyl)-3-methyl-5-(prop-1-yn-1-yl)quinoxalin-2(1*H*)-one

At room temperature, tetrabutylammonium fluoride (5.51 g, 21.08 mmol) was added dropwise to a tetrahydrofuran (20 mL) solution of 7-(((*tert*-butyldimethylsilyl)oxy)methyl)-8-fluoro-3-methyl-5-(prop-1-yn-1-yl)quinoxalin-2(1*H*)-one (3.8 g, 10.54 mmol), and the reaction was allowed to proceed for 7 h at room temperature. The reaction was quenched with saturated brine, followed by the addition of ethyl acetate (200 mL), washing with water (30 mL × 3), and the organic phase was collected and dried over anhydrous sodium sulfate. Concentrate under reduced pressure, and purify the residue by silica gel column chromatography (DCM/MeOH (v/v) = 20/1) to give a pale yellow solid product (1 g, 38.53%).

MS (ESI, neg. ion) m/z: 245.10 $[M-H]^-$.

Step 8) Synthesis of 7-(bromomethyl)-8-fluoro-3-methyl-5-(prop-1-yn-1-yl)quinoxalin-2(1*H*)-one

In an ice bath, triphenylphosphine (0.80 g, 3.04 mmol) was added to a solution of 8-fluoro-7-(hydroxymethyl)-3-methyl-5-(prop-1-yn-1-yl)quinoxalin-2(1*H*)-one (0.5 g, 2.03 mmol) in dichloromethane (40 mL), followed by fractional addition of carbon tetrabromide (1.01 g, 3.04 mmol). The reaction was continued in an ice bath for 1 h. The reaction was stopped, the reaction solution was concentrated under reduced pressure, and the residue was purified by silica gel column chromatography (PE/EtOAc (v/v) = 3/2) to give a white solid product (0.4 g, 63.72%).

Step 8) Synthesis of 5-(4-((5-fluoro-2-methyl-3-oxo-8-(prop-1-yn-1-yl)-3,4-dihydroquinoxalin-6-yl)methyl)piperazin-1-yl)-*N*-methylpicolinamide

At room temperature, potassium iodide (0.0022 g, 0.013 mmol), ethyl diisopropylamine (0.067 g, 0.52 mmol), and N-methyl-5-(piperazin-1-yl)pyridine-2-carboxamide (0.086 g, 0.39 mmol) were added to a solution of 7-(bromomethyl)-8-fluoro-3-methyl-5-(prop-1-yn-1-yl)-1,2-dihydroquinoxalin-2-one (0.08 g, 0.26 mmol) in acetonitrile (6 mL). The reaction was then heated to 80 °C and monitored by TLC. The reaction was stopped, cooled to room temperature, filtered, and the resulting solid was washed with acetonitrile (10 mL). The filter cake was purified by silica gel column chromatography (DCM/MeOH (v/v) = 20/1) to give an off-white solid product (20 mg, 17.23%).

MS (ESI, pos. ion) m/z: 449.21 $[M+H]^+$.

$^1H$ NMR (400 MHz, DMSO-$d_6$) δ 12.51 (s, 1H), 8.43-8.37 (m, 1H), 8.25 (d, *J* = 2.8 Hz, 1H), 7.81 (d, *J* = 8.8 Hz, 1H), 7.40-7.32 (m, 2H), 3.66 (s, 2H), 3.31-3.24 (m, 4H), 2.77 (d, *J* = 4.8 Hz, 3H), 2.59-2.53 (m, 4H), 2.43 (s, 3H), 2.12 (s, 3H).

**Example 41 *N*-cyclopropyl-2-fluoro-1'-((5-fluoro-2-methyl-3-oxo-8-(prop-1-yn-1-yl)-3,4-dihydroquinoxalin-6-yl) methyl)-1',2',3',6'-tetrahydro-[3,4'-bipyridine]-6-carboxamide**

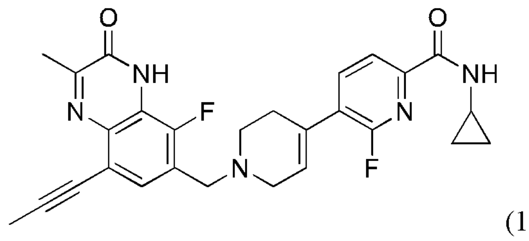

(119)

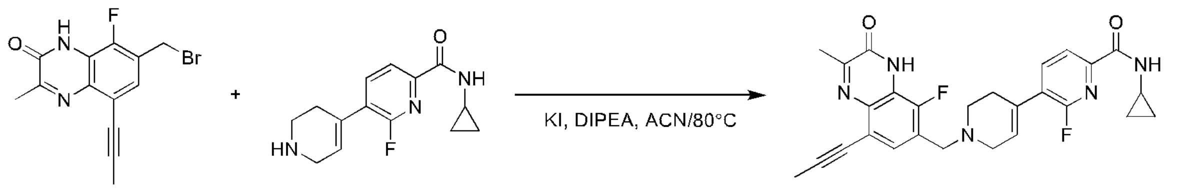


7-(bromomethyl)-8-fluoro-3-methyl-5-(prop-1-yn-1-yl)-1,2-dihydroquinoxalin-2-one (0.095 g, 0.31 mmol), *N*-cyclopropyl-6-fluoro-5-(1,2,3,6-tetrahydropyridin-4-yl)pyridine-2-carboxamide hydrochloride (0.13 g, 0.43 mmol), and potassium iodide (0.010 g, 0.062 mmol) were added to a 25 mL flask, and stirrde to dissolve in ACN (4 mL), after cooling, DIPEA (0.16 mL, 0.93 mmol) was added slowly dropwise under ice bath, stirred until homogeneous, and the mixture was heated to 80 °C for 2 h. Cooled to room temperature, and under stirring, a solid precipitated out. The filter cake was collected by filtration, washed with acetonitrile (1 mL × 3), MTBE (4 mL × 3), and water (4 mL × 3), then dried under vacuum to give a white solid (0.117 g, yield 77.77 %).

MS (ESI, pos. ion) *m/z:* 490.00 $[M+H]^+$;

$^1H$ NMR (400 MHz, DMSO-$d_6$) δ 12.52 (s, 1H), 8.62 (s, 1H), 7.98 (s, s, 2H), 7.36 (s, 1H), 6.23 (s, 1H), 3.71 (s, 2H), 3.16 (s,

2H), 2.87 (s, 2H), 2.68 (s, 3H), 2.44 - 2.36 (m, 3H), 2.11 (s, 3H), 0.66 (s, 4H).

**Example 42 *N*-cyclopropyl-1'-((8-(difluoromethoxy)-2-methyl-3-oxo-3,4-dihydroquinoxalin-6-yl)methyl)-2-fluoro-1',2',3',6'-tetrahydro-[3,4'-bipyridine]-6-carboxamide**

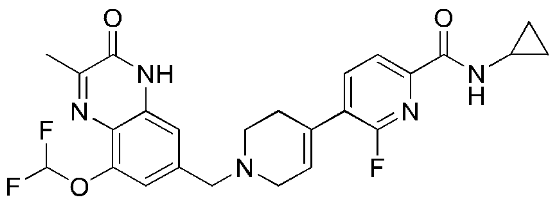

(120)

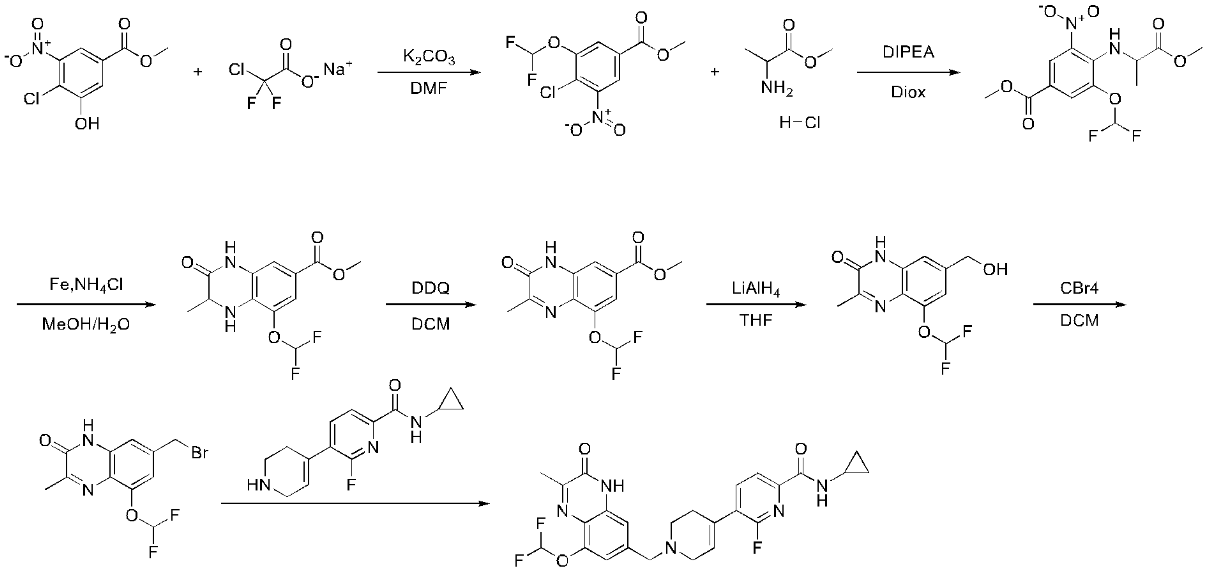


Step 1) Synthesis of methyl 4-chloro-3-(difluoromethoxy)-5-nitrobenzene

At room temperature, methyl 4-chloro-3-hydroxy-5-nitrobenzene (1 g, 4.32 mmol), sodium 2-chloro-2,2-difluoroacetate (0.86 g, 5.62 mmol), and potassium carbonate (0.66 g, 4.75 mmol) were added to a 50 mL flask, followed by the addition of dimethylformamide (15 mL). The mixture was then heated to 80 °C and reacted for 2 h. The reaction was monitored by TLC until complete. EtOAc was added to dilute the reaction mixture, followed by the addition of water (80 mL). The mixture was extracted, and the organic phase was washed with saturated sodium chloride solution. The solution was concentrated under reduced pressure, and the residue was purified by silica gel column chromatography (eluent: PE/EtOAc (v/v) = 1/10) to obtain light green crystals (0.48 g, 39.48%).
MS (ESI, pos. ion) *m/z:* 282.00 $[M+H]^+$.

Step 2) Synthesis of methyl 3-(difluoromethoxy)-4-((1-methoxy-1-oxopropyl-2-yl)amino)-5-nitrobenzoate

At room temperature, DL-alanine methyl ester hydrochloride (3.30 g, 23.66 mmol) and ethyl diisopropylamine (6.12 g, 47.32 mmol) were added to a solution of methyl 4-chloro-3-(difluoromethoxy)-5-nitrobenzoate (3.33 g, 11.83 mmol) in 1,4-dioxane (60 mL), followed by heating to 100 °C and reacting overnight. The reaction was stopped, cooled to room temperature, and the reaction mixture was evaporated to dryness. The residue was subjected to silica gel column chromatography (eluent: PE/EtOAc (v/v) = 10/1) to give a yellow viscous liquid (2.7 g, 65.56%).
MS (ESI, pos. ion) *m/z:* 349.00 $[M+H]^+$.

Step 3) Synthesis of methyl 8-(difluoromethoxy)-2-methyl-3-oxo-1,2,3,4-tetrahydroquinoxalin-6-carboxylate

At room temperature, iron powder (2.67 g, 47.88 mmol) and ammonium chloride (2.56 g, 47.88 mmol) were

added to a solution of methyl 3-(difluoromethoxy)-4-((1-methoxy-1-oxopropyl-2-yl)amino)-5-nitrobenzoate (2.78 g, 7.98 mmol) in water (15 mL) and methanol (30 mL), followed by heating to 70 °C and reacting for 4.5 h. The reaction was stopped, and the reaction solution was allowed to cool to room temperature. The reaction solution was diluted with dichloromethane/methanol (v/v) = 10/1, 100 mL. The mixture was filtered through diatomaceous earth, and extracted with saturated brine and dichloromethane/methanol (v/v) = 10/1, 100 mL × 3. The residue was purified by silica gel column chromatography (eluent: dichloromethane/ethyl acetate (v/v) = 4/1) to give a white solid (1.78 g, 77.90%).

MS (ESI, pos. ion) *m/z:* 287.10 $[M+H]^+$.

Step 4) Synthesis of methyl 8-(difluoromethoxy)-2-methyl-3-oxo-3,4-dihydroquinoxalin-6-carboxylate

At 0 °C, 2,3-dichloro-5,6-dicyano-1,4-benzoquinone (1.55 g, 6.84 mmol) was added to a solution of methyl 8-(difluoromethoxy)-2-methyl-3-oxo-1,2,3,4-tetrahydroquinoxalin-6-carboxylate (1.78 g, 6.22 mmol) in dichloromethane (50 mL). The mixture was then brought to room temperature and reacted for 7 h. The reaction solution was concentrated under reduced pressure, cooled to 0 °C, and saturated sodium bicarbonate solution was added until no bubbles were produced. The mixture was then stirred overnight at room temperature. The mixture was filtered, washed with water, and the filter cake was dissolved in methanol (50 mL). The solution was evaporated to dryness to give a white solid product (1.6 g, 90.52%).

MS (ESI, pos. ion) *m/z:* 285.10 $[M+H]^+$.

Step 5) Synthesis of 5-(difluoromethoxy)-7-(hydroxymethyl)-3-methylquinoxalin-2(1*H*)-one

At 0 °C, lithium aluminum hydride (0.34 g, 9.01 mmol) was added in portions to a tetrahydrofuran (25 mL) solution of methyl 8-(difluoromethoxy)-2-methyl-3-oxo-3,4-dihydroquinoxalin-6-carboxylate (1.6 g, 5.63 mmol), and then the reaction was brought to room temperature and carried out for 6 h. The reaction was stopped, the reaction solution was cooled to 0°C, and methanol (50 mL) was slowly added dropwise to quench the reaction. The reaction solution was diluted with dichloromethane/methanol (v/v) = 10/1, 300 mL. The solution was filtered through diatomaceous earth, the organic phase was concentrated under reduced pressure, and the residue was purified by silica gel column chromatography (eluent: dichloromethane/methanol (v/v) = 20/1) to give a white solid product (0.94 g, 65.17%).

MS (ESI, pos. ion) *m/z:* 257.10 $[M+H]^+$.

Step 6) Synthesis of 7-(bromomethyl)-5-(difluoromethoxy)-3-methylquinoxalin-2(1*H*)-one

At 0 °C, carbon tetrabromide (0.52 g, 1.56 mmol) and triphenylphosphine (0.41 g, 1.56 mmol) were added to a solution of 5-(difluoromethoxy)-7-(hydroxymethyl)-3-methylquinoxalin-2(1*H*)-one (0.2 g, 0.78 mmol) in dichloromethane (10 mL). The reaction was then brought to room temperature and allowed to proceed for 5.5 h. The reaction was stopped, and the reaction mixture was concentrated under reduced pressure. The residue was purified by silica gel column chromatography (petroleum ether/ethyl acetate (v/v) = 10/1) to give a white solid product (0.05 g, 20.07%).

Step 7) Synthesis of *N*-cyclopropyl-1'-((8-(difluoromethoxy)-2-methyl-3-oxo-3,4-dihydroquinoxalin-6-yl)methyl)-2-fluoro-1',2',3',6'-tetrahydro-[3,4'-bipyridine]-6-carboxamide

At room temperature, *N*-cyclopropyl-6-fluoro-5-(1,2,3,6-tetrahydropyridin-4-yl)pyridine-2-carboxamide (0.063 g, 0.24 mmol) and ethyl diisopropylamine (0.31 g, 2.4 mmol) were added to a solution of 7-(bromomethyl)-5-(difluoromethoxy)-3-methylquinoxalin-2(1*H*)-one (0.05 g, 0.16 mmol) in acetonitrile (5 mL), followed by reaction at 80 °C for 2 h. The reaction was stopped, cooled to room temperature, and the reaction mixture was evaporated to dryness. The residue was dissolved in dichloromethane, washed with water, dried over anhydrous sodium sulfate, the residue was evaporated to dryness, and purified by silica gel column chromatography (dichloromethane/methanol (v/v) = 20/1). The obtained solid was further purified by plate chromatography to give a white solid product (0.04 g, yield 51.11%).

MS (ESI, pos. ion) *m/z:* 500.19 $[M+H]^+$.

$^1$H NMR (400 MHz, DMSO-$d_6$) δ 12.41 (s, 1H), 8.65-8.57 (m, 1H), 8.14-8.04 (m, 1H), 7.96-7.88 (m, 1H), 7.37 (t, *J* = 74.4 Hz, 1H), 7.10 - 7.01 (m, 1H), 6.29-6.23 (m, 1H), 3.67 (s, 2H), 3.20 - 3.12 (m, 2H), 2.88 (h, *J* = 5.5 Hz, 1H), 2.71-2.63 (m, 2H), 2.54-2.51 (m, 2H), 2.41 (s, 3H), 0.71- 0.63 (m, 4H).

**Example 43 6-(4-((2-methyl-3-oxo-8-(prop-1-yn-1-yl)-3,4-dihydroquinoxalin-6-yl)methyl)piperazin-1-yl)nicotinonitrile**

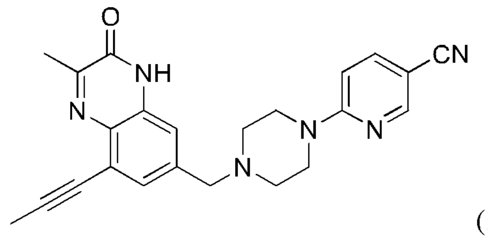

(121)

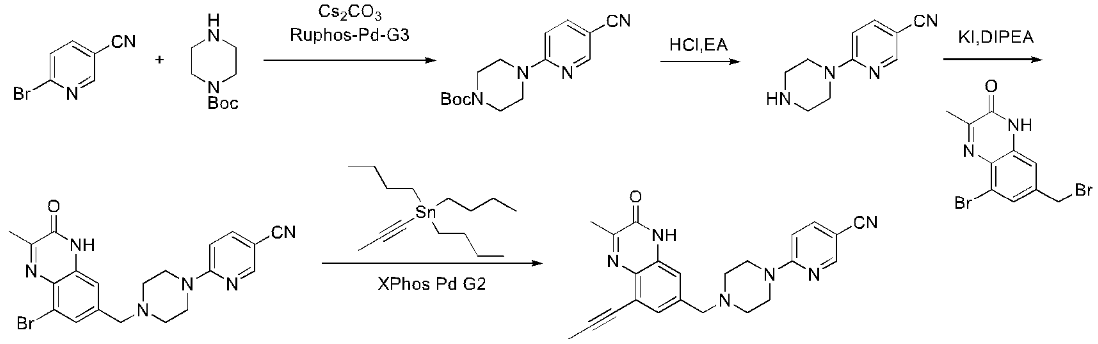


Step 1) Synthesis of 4-(5-cyanopyridin-2-yl)piperazine-1-carboxylic acid *tert*-butyl ester

At room temperature, Ruphos-Pd-G3 (0.48 g, 0.57 mmol) was added to a solution of 6-bromopyridin-3-onitrile (1.5 g, 8.20 mmol), piperazine-1-carboxylic acid *tert*-butyl ester (1.53 g, 8.20 mmol), and cesium carbonate (5.34 g, 16.4 mmol) in 1,4-dioxane (30 mL). The solution was then replaced with $N_2$, and the reaction was carried out overnight at 110 °C. The mixture was filtered through diatomaceous earth, concentrated under reduced pressure, and the residue was purified by silica gel column chromatography (eluent: PE:EtOAc (v/v) = 6/1) to give a white solid product (2.36 g, 71.93%).

MS (ESI, pos. ion) *m*/*z:* 289.10 $[M+H]^+$.

Step 2) Synthesis of 6-(piperazin-1-yl)nicotinonitrile

At room temperature, dioxane hydrochloride solution (59.0 mmol, 4 M) was added to a solution of 4-(5-cyanopyridin-2-yl)piperazin-1-carboxylic acid *tert*-butyl ester (1.7 g, 5.90 mmol) in ethyl acetate (25 mL), and the reaction was allowed to proceed overnight. After the reaction was complete, the reaction solution was directly filtered to obtain a solid product, which was dried at room temperature to give a white solid (0.65 g, 58.57%).

MS (ESI, pos. ion) *m*/*z:* 189.20 $[M+H]^+$

Step 3) Synthesis of 6-(4-((8-bromo-2-methyl-3-oxo-3,4-dihydroquinoxalin-6-yl)methyl)piperazin-1-yl)nicotinonitrile

At room temperature, potassium iodide (14 mg, 0.083 mmol), 6-(piperazin-1-yl)nicotinonitrile (0.62 g, 3.32 mmol), and *N,N*-diisopropylethylamine (4.39 mL, 26.56 mmol) were added to a solution of 5-bromo-7-(bromomethyl)-3-methylquinoxalin-2(1*H*)-one (0.55 g, 1.66 mmol) in acetonitrile (40 mL), and the reaction was brought to room temperature for 4 h. After the reaction was completed, the reaction solution was diluted with dichloromethane (50 mL), washed with water (30 mL × 3), concentrated under reduced pressure, and the residue was purified by silica gel column chromatography (eluent: dichloromethane/methanol (v/v) = 97/4) to obtain an orange-yellow solid powder (0.36 g, 49.46%).

MS (ESI, pos. ion) *m*/*z:* 438.90 $[M+H]^+$;

Step 4) Synthesis of 6-(4-((2-methyl-3-oxo-8-(prop-1-yn-1-yl)-3,4-dihydroquinoxalin-6-yl)methyl)piperazin-1-yl)nicotinonitrile

At room temperature, tributyl(prop-1-yn-1-yl)tin (0.45 mL, 1.48 mmol) was added to a solution of 6-(4-((8-bromo-2-methyl-3-oxo-3,4-dihydroquinoxalin-6-yl)methyl)piperazin-1-yl)nicotinonitrile (0.36 g, 0.82 mmol) in 1,4-dioxane (25 mL), followed by the addition of XPhos Pd G2 (0.097 g, 0.12 mmol). The reaction mixture was then replaced with $N_2$ and reacted overnight at 90 °C. The reaction solution was filtered through diatomaceous earth, the organic phase was concentrated under reduced pressure, and the residue was purified by silica gel column chromatography (eluent: dichloromethane/methanol (v/v) = 96/4) to give a white solid powder (85 mg, 26.03%).

MS (ESI, pos. ion) *m*/ *z*: 399.19 $[M+H]^+$;

$^1H$ NMR (400 MHz, DMSO-$d_6$) δ 12.32 (s, 1H), 8.52-8.44 (m, 1H), 7.89-7.79 (m, 1H), 7.31-7.18 (m, 2H), 6.97-6.87 (m, 1H), 3.70-3.62 (m, 4H), 3.54 (s, 2H), 2.48-2.43 (m, 4H), 2.41 (s, 3H), 2.13 (s, 3H).

**Example 44 1'-((8-(difluoromethoxy)-2-methyl-3-oxo-3,4-dihydroquinoxalin-6-yl)methyl)-*N*-methyl-1',2',3',6'-tetrahydro-[3,4'-bipyridine]-6-carboxamide**

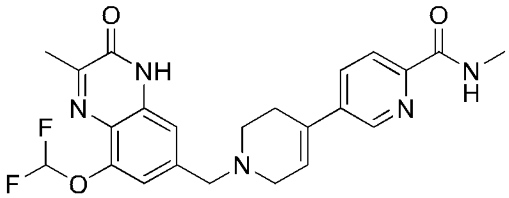

(122)

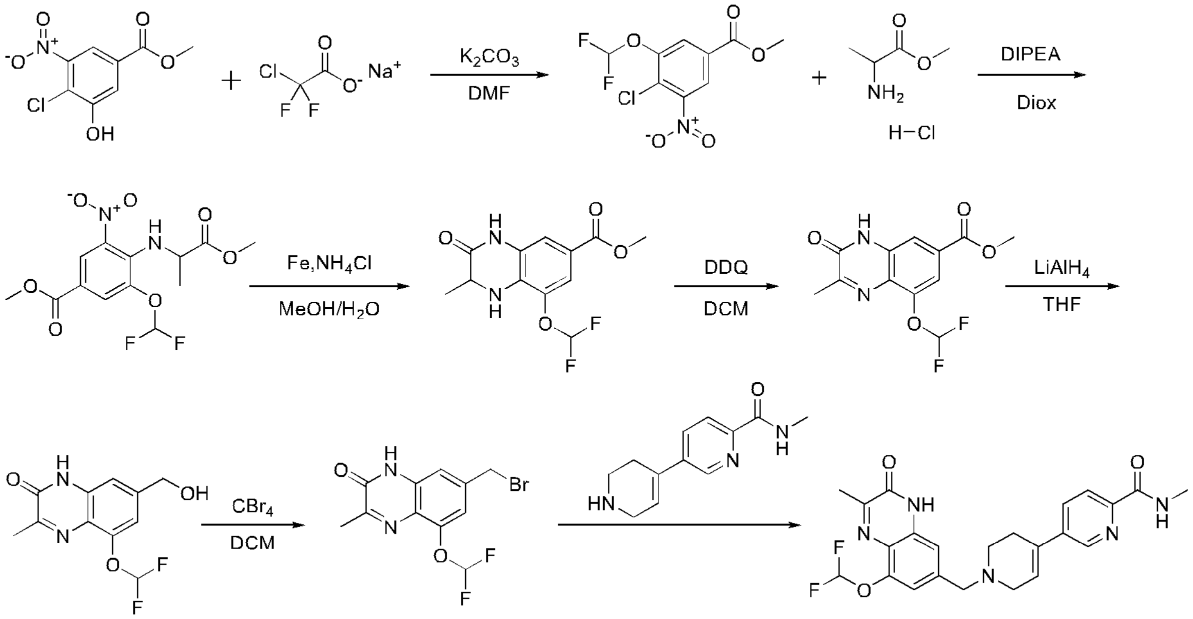


Step 1) Synthesis of methyl 4-chloro-3-(difluoromethoxy)-5-nitrobenzene

At room temperature, methyl 4-chloro-3-hydroxy-5-nitrobenzoate (1 g, 4.32 mmol), sodium 2-chloro-2,2-difluoroacetate (0.86 g, 5.62 mmol), and potassium carbonate (0.66 g, 4.75 mmol) were added to a 50 mL flask, followed by the addition of dimethylformamide (15 mL). The temperature was raised to 80 °C, and the reaction was monitored by TLC until complete. The reaction mixture was diluted by adding EtOAc (20 mL). Extraction was performed with water (80 mL) and DCM (50 mL × 3). The organic phase was washed with saturated sodium chloride solution (50 mL × 3) and then concentrated under reduced pressure. The residue was purified by silica gel column chromatography (eluent: PE/EtOAc (v/v) = 1/10) to obtain a light green product (0.48 g, 39.48%).
MS (ESI, pos. ion) *m/z:* 282.00 $[M+H]^+$.

Step 2) Synthesis of methyl 3-(difluoromethoxy)-4-((1-methoxy-1-oxopropyl-2-yl)amino)-5-nitrobenzoate

At room temperature, DL-alanine methyl ester hydrochloride (3.30 g, 23.66 mmol) and ethyl diisopropylamine (6.12 g, 47.32 mmol) were added to a solution of methyl 4-chloro-3-(difluoromethoxy)-5-nitrobenzoate (3.33 g, 11.83 mmol) in 1,4-dioxane (60 mL), followed by heating to 100 °C and reacting overnight. The reaction was stopped, cooled to room temperature, and the reaction mixture was concentrated under reduced pressure. The residue was subjected to silica gel column chromatography (eluent: PE/EtOAc (v/v) = 10/1) to give a yellow viscous liquid (2.7 g, 65.56%).
MS (ESI, pos. ion) *m/z:* 349.00 $[M+H]^+$.

Step 3) Synthesis of methyl 8-(difluoromethoxy)-2-methyl-3-oxo-1,2,3,4-tetrahydroquinoxalin-6-carboxylate

At room temperature, iron powder (2.67 g, 47.88 mmol) and ammonium chloride (2.56 g, 47.88 mmol) were added to a solution of methyl 3-(difluoromethoxy)-4-((1-methoxy-1-oxopropyl-2-yl)amino)-5-nitrobenzoate (2.78 g, 7.98 mmol) in water (15 mL) and methanol (30 mL), followed by heating to 70 °C and reacting for 4.5 h. The reaction was stopped, and the reaction solution was allowed to cool to room temperature. The reaction solution was diluted with a mixed solution (dichloromethane/methanol (v/v) = 10/1, 50 mL), filtered through diatomaceous earth, and extracted with saturated saline (50 mL) and a mixed solution (dichloromethane/methanol (v/v) = 10/1, 60 mL × 3). The residue was purified by silica gel column chromatography (eluent: dichloromethane/ethyl acetate (v/v) = 4/1) to give a yellow viscous product (1.78 g, 77.90%).
MS (ESI, pos. ion) *m*/*z:* 287.10 $[M+H]^+$.

Step 4) Synthesis of methyl 8-(difluoromethoxy)-2-methyl-3-oxo-3,4-dihydroquinoxalin-6-carboxylate

At 0 °C, 2,3-dichloro-5,6-dicyano-1,4-benzoquinone (1.55 g, 6.84 mmol) was added to a solution of methyl 8-(difluoromethoxy)-2-methyl-3-oxo-1,2,3,4-tetrahydroquinoxalin-6-carboxylate (1.78 g, 6.22 mmol) in dichloromethane (50 mL). The mixture was then brought to room temperature and reacted for 7 h. The reaction solution was concentrated under reduced pressure, cooled to 0°C, and saturated sodium bicarbonate aqueous solution was added until no bubbles were generated. The mixture was then heated to room temperature and stirred overnight. The mixture was filtered, washed with water until the filtrate was colorless, and the filter cake was dissolved in methanol (50 mL). The solution was concentrated under reduced pressure to give a white solid product (1.6 g, 90.52%).
MS (ESI, pos. ion) *m*/*z:* 285.10 $[M+H]^+$.

Step 5) Synthesis of 5-(difluoromethoxy)-7-(hydroxymethyl)-3-methylquinoxalin-2(1*H*)-one

At 0 °C, lithium aluminum hydride (0.34 g, 9.01 mmol) was added in portions to a tetrahydrofuran (25 mL) solution of methyl 8-(difluoromethoxy)-2-methyl-3-oxo-3,4-dihydroquinoxalin-6-carboxylate (1.6 g, 5.63 mmol), and then the reaction was brought to room temperature and carried out for 6 h. The reaction was stopped, the reaction solution was cooled to 0°C, and methanol (20 mL) was slowly added dropwise to quench the reaction. The reaction solution was diluted with dichloromethane/methanol (v/v) = 10/1, 200 mL. The solution was filtered through diatomaceous earth, the organic phase was concentrated under reduced pressure, and the residue was purified by silica gel column chromatography (eluent: dichloromethane/methanol (v/v) = 20/1) to give a white solid product (0.94 g, 65.17%).
MS (ESI, pos. ion) *m*/*z:* 257.10 $[M+H]^+$.

Step 6) Synthesis of 7-(bromomethyl)-5-(difluoromethoxy)-3-methylquinoxalin-2(1*H*)-one

At 0 °C, carbon tetrabromide (1.97 g, 5.95 mmol) and triphenylphosphine (1.56 g, 5.95 mmol) were added to a solution of 5-(difluoromethoxy)-7-(hydroxymethyl)-3-methylquinoxalin-2(1*H*)-one (0.61 g, 2.38 mmol) in dichloromethane (40 mL). The reaction was then brought to room temperature and allowed to proceed for 5.5 h. The reaction was stopped, and the reaction mixture was concentrated under reduced pressure. The residue was purified by silica gel column chromatography (petroleum ether/ethyl acetate (v/v) = 10/1) to give a white solid product (0.3 g, 39.49%).

Step 7) Synthesis of 1'-((8-(difluoromethoxy)-2-methyl-3-oxo-3,4-dihydroquinoxalin-6-yl)methyl)-*N*-methyl-1',2',3',6'-tetrahydro-[3,4'-bipyridine]-6-carboxamide

At room temperature, *N*-methyl-1',2',3',6'-tetrahydro-[3,4'-bipyridine]-6-carboxamide (0.052 g, 0.24 mmol) and ethyl diisopropylamine (0.31 g, 2.4 mmol) were added to a solution of 7-(bromomethyl)-5-(difluoromethoxy)-3-methylquinoxalin-2(1*H*)-one (0.05 g, 0.16 mmol) in acetonitrile (5 mL), and then the mixture was heated to 80 °C and reacted for 2 h. The reaction was stopped, cooled to room temperature, and the reaction mixture was evaporated to dryness. Dichloromethane (30 mL) was added, and the mixture was washed with water (15 mL × 3). The solution was dried over anhydrous sodium sulfate, concentrated under reduced pressure, and the residue was purified by silica gel column chromatography (dichloromethane/methanol (v/v) = 20/1). The obtained solid was further purified by plate chromatography to give a white solid product (0.01 g, yield 14.01%).
MS (ESI, pos. ion) *m*/*z:* 456.18 $[M+H]^+$.
$^1H$ NMR (400 MHz, DMSO-$d_6$) δ 12.42 (s, 1H), 8.75 - 8.66 (m, 2H), 8.03 - 7.94 (m, 2H), 7.37 (t, *J* = 74.4 Hz, 1H), 7.18 - 7.04 (m, 2H), 6.47 - 6.42 (m, 1H), 3.68 (s, 2H), 3.20 - 3.13 (m, 2H), 2.81 (d, *J* = 4.8 Hz, 3H), 2.72 - 2.67 (m, 2H), 2.59 - 2.54 (m, 2H), 2.41 (s, 3H).

**Example 45 5-(4-((5-fluoro-2-methyl-3-oxo-8-(prop-1-yn-1-yl)-3,4-dihydroquinoxalin-6-yl)methyl)piperazin-1-yl)-*N*,6-dimethylpicolinamide**

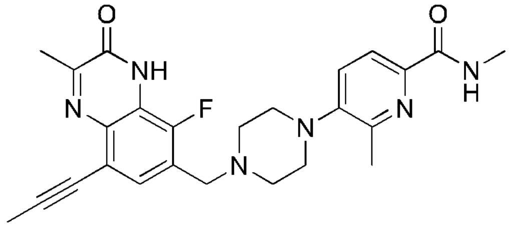

(123)

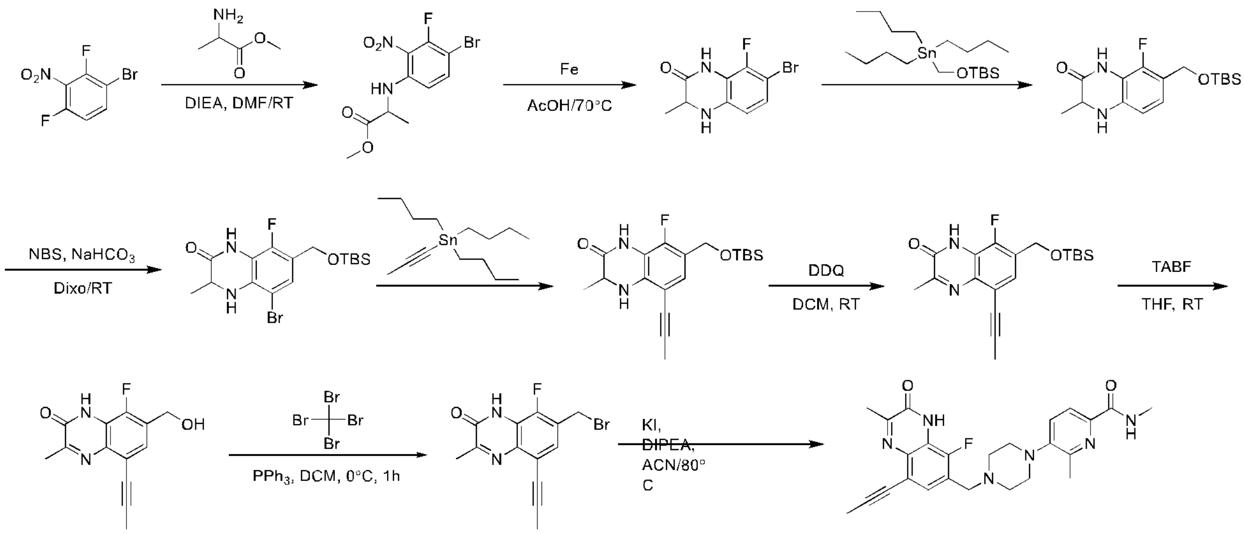


Step 1) Synthesis of Methyl (4-bromo-3-fluoro-2-nitrophenyl)malonate

At 0 °C, ethyl diisopropylamine (16.29 g, 126.06 mmol) was added dropwise to a DMF (40 mL) solution of 1-bromo-2,4-difluoro-3-nitrobenzene (10 g, 42.02 mmol) and methyl 2-aminopropionate (4.42 g, 42.86 mmol), followed by overnight incubation at room temperature. The reaction was stopped, the reaction solution was diluted with DCM (100 mL), washed three times with water (50 mL), concentrated under reduced pressure, and the residue was purified by silica gel column chromatography (PE/EtOAc (v/v) = 4/1) to give a yellow solid product (6 g, 44.47%).

MS (ESI, pos. ion) *m/z:* 321.00 $[M+H]^+$.

Step 2) Synthesis of 7-bromo-8-fluoro-3-methyl-3,4-dihydroquinoxalin-2(1*H*)-one

Iron powder (8.35 g, 149.48 mmol) was added to a solution of methyl (4-bromo-3-fluoro-2-nitrophenyl)malonate (12 g, 37.37 mmol) in acetic acid (100 mL) with stirring at room temperature. The reaction was carried out at 70 °C for 4 h, and the reaction was monitored by TLC. After cooling to room temperature, the reaction solution was diluted with ethyl acetate (300 mL), the insoluble matter was filtered off, and the organic phase was washed with water (60 mL × 2) and saturated brine (80 mL × 3) to give a brown solid product (9.2 g, 95.02%).

MS (ESI, pos. ion) *m/z:* 259.00 $[M+H]^+$.

Step 3) Synthesis of 7-(((*tert*-butyldimethylsilyl)oxy)methyl)-8-fluoro-3-methyl-3,4-dihydroquinoxalin-2(1*H*)-one

At room temperature, chloro(2-dicyclohexylphosphine-2',4',6'-triisopropyl-1,1'-biphenyl)[2-(2'-amino-1,1'-biphenyl))palladium(II) (1.40 g, 1.78 mmol) was added to a solution of 7-bromo-8-fluoro-3-methyl-3,4-dihydroquinoxalin-2(1*H*)-one (9.2 g, 35.51 mmol) and *tert*-butyldimethyl((tributyltin)methoxy)silane (20.10 g, 46.16 mmol), followed by replacement with $N_2$ and heating to 90 °C overnight. The reaction was stopped, the reaction solution was cooled to room temperature, filtered through diatomaceous earth, concentrated under reduced pressure, and the residue was purified by silica gel column chromatography (PE/EtOAc(v/v) = 4/1) to give a yellow solid product (8.1 g, 70.30%).

MS (ESI, pos. ion) *m/z:* 325.20 [M+H]$^+$.

Step 4) Synthesis of 5-bromo-7-(((*tert*-butyldimethylsilyl)oxy)methyl)-8-fluoro-3-methyl-3,4-dihydroquinoxalin-2(1*H*)-one

Under $N_2$ and 0 °C, N-bromosuccinimide (4.66 g, 26.21 mmol) was added to a solution of 7-(((*tert*-butyldimethylsilyl)oxy)methyl)-8-fluoro-3-methyl-3,4-dihydroquinoxalin-2(1*H*)-one (8.1 g, 24.96 mmol), sodium bicarbonate (3.15 g, 37.44 mmol), and then the mixture was brought to room temperature. TLC showed the reaction was complete. The reaction was stopped, quenched with saturated sodium thiosulfate aqueous solution (100 mL), extracted with EtOAc (100 mL × 2), dried over anhydrous sodium sulfate, concentrated under reduced pressure, and purified by silica gel column chromatography (PE/EtOAc (v/v) = 5/1) to give a yellow solid product (9.8 g, yield 97.32%).
MS (ESI, pos. ion) *m/z:* 402.90 [M+H]$^+$.

Step 5) Synthesis of 7-(((*tert*-butyldimethylsilyl)oxy)methyl)-8-fluoro-3-methyl-5-(prop-1-yn-1-yl)-3,4-dihydroquinoxalin-2(1*H*)-one

At room temperature, XPhos-G2-Pd (0.20 g, 0.25 mmol) was added to a solution of 5-bromo-7-(((*tert*-butyldimethylsilyl)oxy)methyl)-8-fluoro-3-methyl-3,4-dihydroquinoxalin-2(1*H*)-one (2 g, 4.96 mmol) in tributyl(prop-1-yn-yl)stanane (2.12 g, 6.45 mmol), and then the reaction was carried out at 90 °C for 12 h. The reaction was stopped, the reaction solution was diluted with DCM (100 mL), filtered with diatomaceous earth, the organic phase was concentrated under reduced pressure, and the residue was purified by silica gel column chromatography (PE/EtOAc (v/v) = 10/1) to give a white solid product (1.6 g, 89.01%).
MS (ESI, pos. ion) *m/z:* 363.20 [M+H]$^+$.

Step 6) Synthesis of 7-(((*tert*-butyldimethylsilyl)oxy)methyl)-8-fluoro-3-methyl-5-(prop-1-yn-1-yl)quinoxalin-2(1*H*)-one

A solution of 7-(((*tert*-butyldimethylsilyl)oxy)methyl)-8-fluoro-3-methyl-5-(prop-1-yn-1-yl)-3,4-dihydroquinoxalin-2(1*H*)-one (4 g, 11.03 mmol) in dichloromethane (120 mL) was cooled to 0 °C, and 2,3-dichloro-5,6-dicyano-1,4-benzoquinone (2.75 g, 12.13 mmol) was added. The reaction was then allowed to proceed overnight at room temperature. The reaction solution was concentrated under reduced pressure, and saturated $NaHCO_3$ was slowly added at room temperature until no bubbles were generated. The mixture was stirred at room temperature for 6 h, filtered, and the filter cake was washed with water until the filtrate was colorless. The obtained filter cake was dissolved in methanol (60 mL) and evaporated to dryness to give a brownish-red solid product (3.8 g, 95.53%).
MS (ESI, pos. ion) *m/z:* 361.20 [M+H]$^+$.

Step 7) Synthesis of 8-fluoro-7-(hydroxymethyl)-3-methyl-5-(prop-1-yn-1-yl)quinoxalin-2(1*H*)-one

At room temperature, tetrabutylammonium fluoride (5.51 g, 21.08 mmol) was added dropwise to a tetrahydrofuran (20 mL) solution of 7-(((*tert*-butyldimethylsilyl)oxy)methyl)-8-fluoro-3-methyl-5-(prop-1-yn-1-yl)quinoxalin-2(1*H*)-one (3.8 g, 10.54 mmol), and the reaction was allowed to proceed for 7 h at room temperature. The reaction was quenched by adding saturated brine (20 mL), followed by the addition of ethyl acetate (200 mL), washing with water (50 mL × 3), and collecting the organic phase. The solution was dried over anhydrous sodium sulfate. The solution was concentrated under reduced pressure, and the residue was purified by silica gel column chromatography (DCM/MeOH (v/v) = 20/1) to give a yellow solid product (1 g, 38.53%).
MS (ESI, neg. ion) m/z: 245.10 [M-H]$^-$.

Step 8) Synthesis of 7-(bromomethyl)-8-fluoro-3-methyl-5-(prop-1-yn-1-yl)quinoxalin-2(1*H*)-one

In an ice bath, triphenylphosphine (0.80 g, 3.04 mmol) was added to a solution of 8-fluoro-7-(hydroxymethyl)-3-methyl-5-(prop-1-yn-1-yl)quinoxalin-2(1*H*)-one (0.5 g, 2.03 mmol) in dichloromethane (40 mL), followed by fractional addition of carbon tetrabromide (1.01 g, 3.04 mmol). The reaction was continued in an ice bath for 1 h. The reaction was stopped, the reaction solution was concentrated under reduced pressure, and the residue was purified by silica gel column chromatography (PE/EtOAc (v/v) = 40%) to give an orange-yellow solid product (0.4 g, 63.72%).

**Step 9) Synthesis of 5-(4-((5-fluoro-2-methyl-3-oxo-8-(prop-1-yn-1-yl)-3,4-dihydroquinoxalin-6-yl)methyl)piperazin-1-yl)-*N*,6-dimethylpicolinamide**

At room temperature, potassium iodide (0.0046 g, 0.028 mmol), ethyl diisopropylamine (0.21 g, 1.65 mmol), and

*N*,6-dimethyl-5-(piperazin-1-yl)pyridine-2-carboxamide (0.19 g, 0.83 mmol) were added to a solution of 7-(bromomethyl)-8-fluoro-3-methyl-5-(prop-1-yn-1-yl)-1,2-dihydroquinoxalin-2-one (0.170 g, 0.55 mmol) in acetonitrile (5 mL), followed by heating to 80 °C. The reaction was stopped, the reaction solution was concentrated under reduced pressure, the residue was dissolved in DCM, washed with water, the organic phase was dried, concentrated under reduced pressure, and the residue was purified by silica gel column chromatography (DCM/MeOH (v/v) = 20/1). The product was purified by plate chromatography to give a white solid product (0.052 g, 20.44%).

MS (ESI, pos. ion) m/z: 463.23 $[M+H]^+$.

$^1H$ NMR (400 MHz, DMSO-$d_6$) δ 12.42 (s, 1H), 8.46 - 8.39 (m, 1H), 7.78 (d, *J* = 8.3 Hz, 1H), 7.47 (d, *J* = 8.3 Hz, 1H), 7.33 (d, *J* = 6.8 Hz, 1H), 3.67 (s, 2H), 2.96 - 2.91 (m, 4H), 2.79 (d, *J* = 4.9 Hz, 3H), 2.64 - 2.56 (m, 4H), 2.47 (s, 3H), 2.43 (s, 3H), 2.12 (s, 3H).

**Example 46 *N*-cyclopropyl-5-(4-((8-(ditluoromethoxy)-2-methyl-3-oxo-3,4-dihydroquinoxalin-6-yl)methyl)piperazin-1-yl)-6-fluoropicolinamide**

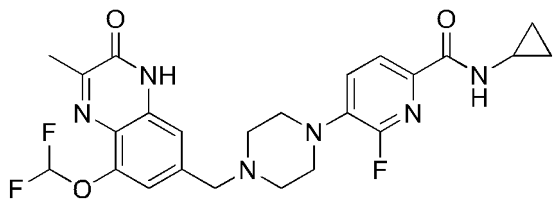

(124)

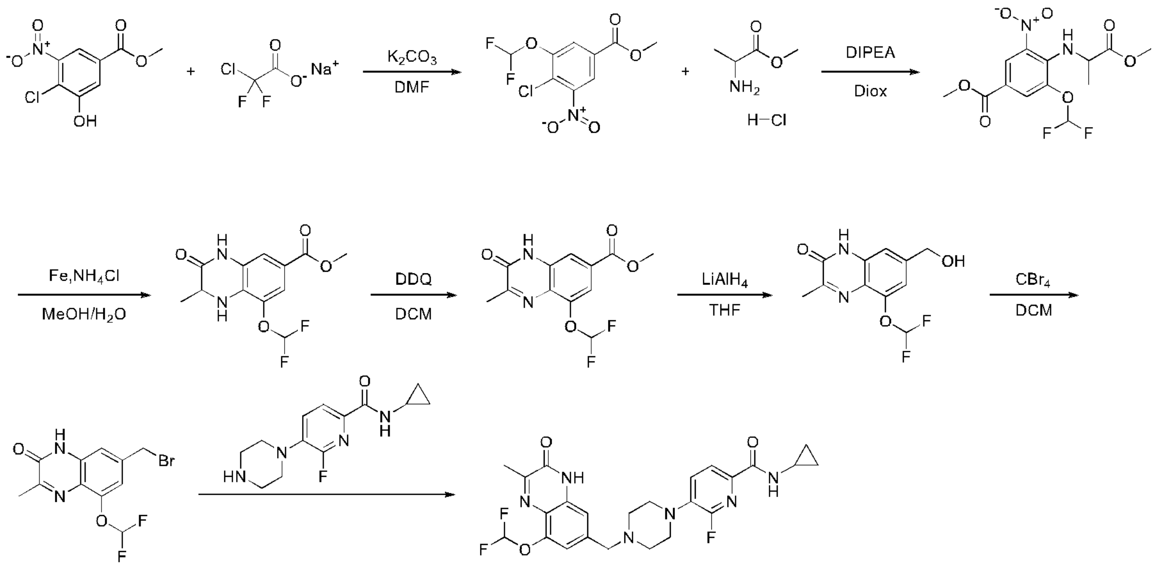


Step 1) Synthesis of methyl 4-chloro-3-(difluoromethoxy)-5-nitrobenzene

At room temperature, methyl 4-chloro-3-hydroxy-5-nitrobenzoate (1 g, 4.32 mmol), sodium 2-chloro-2,2-difluoroacetate (0.86 g, 5.62 mmol), and potassium carbonate (0.66 g, 4.75 mmol) were added to a 50 mL flask, followed by the addition of dimethylformamide (15 mL). The temperature was raised to 80 °C, and the reaction was monitored by TLC until complete. The reaction mixture was diluted with EtOAc, extracted with water (80 mL), and the organic phase was washed with saturated sodium chloride solution (50 mL × 3). The solution was then concentrated under reduced pressure, and the residue was purified by silica gel column chromatography (eluent: PE/EtOAc (v/v) = 1/10) to give a light green crystalline product (0.48 g, 39.48%).

MS (ESI, pos. ion) *m/z:* 282.00 $[M+H]^+$.

Step 2) Synthesis of methyl 3-(difluoromethoxy)-4-((1-methoxy-1-oxopropyl-2-yl)amino)-5-nitrobenzoate

At room temperature, DL-alanine methyl ester hydrochloride (3.30 g, 23.66 mmol) and ethyl diisopropylamine (6.12 g, 47.32 mmol) were added to a solution of methyl 4-chloro-3-(difluoromethoxy)-5-nitrobenzoate (3.33 g, 11.83 mmol) in 1,4-dioxane (60 mL), followed by heating to 100 °C and reacting overnight. The reaction was stopped, cooled to room temperature, and the reaction mixture was evaporated to dryness. The residue was purified by silica gel column chromatography (eluent: PE/EtOAc (v/v) = 10/1) to give a yellow viscous liquid (2.7 g, 65.56%).
MS (ESI, pos. ion) *m/z:* 349.00 $[M+H]^+$.

Step 3) Synthesis of methyl 8-(difluoromethoxy)-2-methyl-3-oxo-1,2,3,4-tetrahydroquinoxalin-6-carboxylate

At room temperature, iron powder (2.67 g, 47.88 mmol) and ammonium chloride (2.56 g, 47.88 mmol) were added to a solution of methyl 3-(difluoromethoxy)-4-((1-methoxy-1-oxopropyl-2-yl)amino)-5-nitrobenzoate (2.78 g, 7.98 mmol) in water (15 mL) and methanol (30 mL), followed by heating to 70 °C and reacting for 4.5 h. The reaction was stopped, and the reaction solution was allowed to cool to room temperature. The reaction solution was diluted with a mixed solution (dichloromethane/methanol (v/v) = 10/1), filtered through diatomaceous earth, and extracted with saturated saline (50 mL) and a mixed solution (dichloromethane/methanol (v/v) = 10/1, 60 mL × 3). The residue was purified by silica gel column chromatography (eluent: dichloromethane/ethyl acetate (v/v) = 4/1) to give a white solid (1.78 g, 77.90%).
MS (ESI, pos. ion) *m/z:* 287.10 $[M+H]^+$.

Step 4) Synthesis of methyl 8-(difluoromethoxy)-2-methyl-3-oxo-3,4-dihydroquinoxalin-6-carboxylate

At 0 °C, 2,3-dichloro-5,6-dicyano-1,4-benzoquinone (1.55 g, 6.84 mmol) was added to a solution of methyl 8-(difluoromethoxy)-2-methyl-3-oxo-1,2,3,4-tetrahydroquinoxalin-6-carboxylate (1.78 g, 6.22 mmol) in dichloromethane (50 mL). The mixture was then brought to room temperature and reacted for 7 h. The reaction solution was concentrated under reduced pressure, cooled to 0°C, and saturated sodium bicarbonate aqueous solution was added until no bubbles were generated. The mixture was then heated to room temperature and stirred overnight. The mixture was filtered, washed with water until the filtrate was colorless, and the filter cake was dissolved in methanol (50 mL). The solution was then evaporated to dryness to give a white solid product (1.6 g, 90.52%).
MS (ESI, pos. ion) *m/z:* 285.10 $[M+H]^+$.

Step 5) Synthesis of 5-(difluoromethoxy)-7-(hydroxymethyl)-3-methylquinoxalin-2(1*H*)-one

At 0 °C, lithium aluminum hydride (0.34 g, 9.01 mmol) was added in portions to a tetrahydrofuran (25 mL) solution of methyl 8-(difluoromethoxy)-2-methyl-3-oxo-3,4-dihydroquinoxalin-6-carboxylate (1.6 g, 5.63 mmol), and then the reaction was brought to room temperature and carried out for 6 h. The reaction was stopped, the reaction solution was cooled to 0°C, and methanol (20 mL) was slowly added dropwise to quench the reaction. The reaction solution was diluted with dichloromethane/methanol (v/v) = 10/1, 200 mL. The solution was filtered through diatomaceous earth, the organic phase was concentrated under reduced pressure, and the residue was purified by silica gel column chromatography (eluent: dichloromethane/methanol (v/v) = 20/1) to give a white solid product (0.94 g, 65.17%).
MS (ESI, pos. ion) *m/z:* 257.10 $[M+H]^+$.

Step 6) Synthesis of 7-(bromomethyl)-5-(difluoromethoxy)-3-methylquinoxalin-2(1*H*)-one

At 0 °C, carbon tetrabromide (1.97 g, 5.95 mmol) and triphenylphosphine (1.56 g, 5.95 mmol) were added to a solution of 5-(difluoromethoxy)-7-(hydroxymethyl)-3-methylquinoxalin-2(1*H*)-one (0.61 g, 2.38 mmol) in dichloromethane (40 mL). The reaction was then brought to room temperature and allowed to proceed for 5.5 h. The reaction was stopped, the reaction solution was concentrated under reduced pressure, and the residue was purified by silica gel column chromatography (petroleum ether/ethyl acetate (v/v) = 10/1) to give a white solid product (0.3 g, 39.49%).

Step 7) Synthesis of 1'-((8-(difluoromethoxy)-2-methyl-3-oxo-3,4-dihydroquinoxalin-6-yl)methyl)-*N*-methyl-1',2',3',6'-tetrahydro-[3,4'-bipyridine]-6-carboxamide

At room temperature, ethyl diisopropylamine (0.093 g, 0.72 mmol) and *N*-cyclopropyl-6-fluoro-5-(piperazin-1-yl) pyridine-2-carboxamide (0.095 g, 0.36 mmol) were added to a solution of 7-(bromomethyl)-5-(difluoromethoxy)-3-methyl-1,2-dihydroquinoxalin-2-one (0.075 g, 0.24 mmol) in acetonitrile (5 mL). The reaction was then stopped at 80 °C. The reaction solution was concentrated under reduced pressure, and dichloromethane (30 mL) was added. The solution was washed with water (15 mL × 3), the organic phase was dried, and concentrated under reduced pressure. The

residue was purified by silica gel column chromatography (DCM/MeOH (v/v) = 20/1). The product was purified by plate chromatography to give a white solid product (0.02 g, 16.93%).

MS (ESI, pos. ion) *m/z:* 503.20 [M+H]$^+$.

H NMR (400 MHz, DMSO-$d_6$) δ 8.40-8.33 (m, 1H), 7.87-7.81 (m, 1H), 7.61-7.55 (m, 1H), 7.27 (t, *J* = 74.4 Hz, 1H), 7.15-7.02 (m, 2H), 3.61 (s, 2H), 3.21-3.14 (m, 4H), 2.88 - 2.80 (m, 1H), 2.62 - 2.53 (m, 4H), 2.41 (s, 3H), 0.71 - 0.58 (m, 4H).

**Example 47 *N*-cyclopropyl-6-fluoro-5-(4-((5-fluoro-2-methyl-3-oxo-8-(prop-1-yn-1-yl)-3,4-dihydroquinoxalin-6-yl)methyl)piperazin-1-yl)picolinamide**

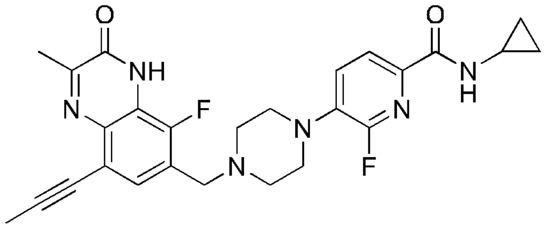

(125)

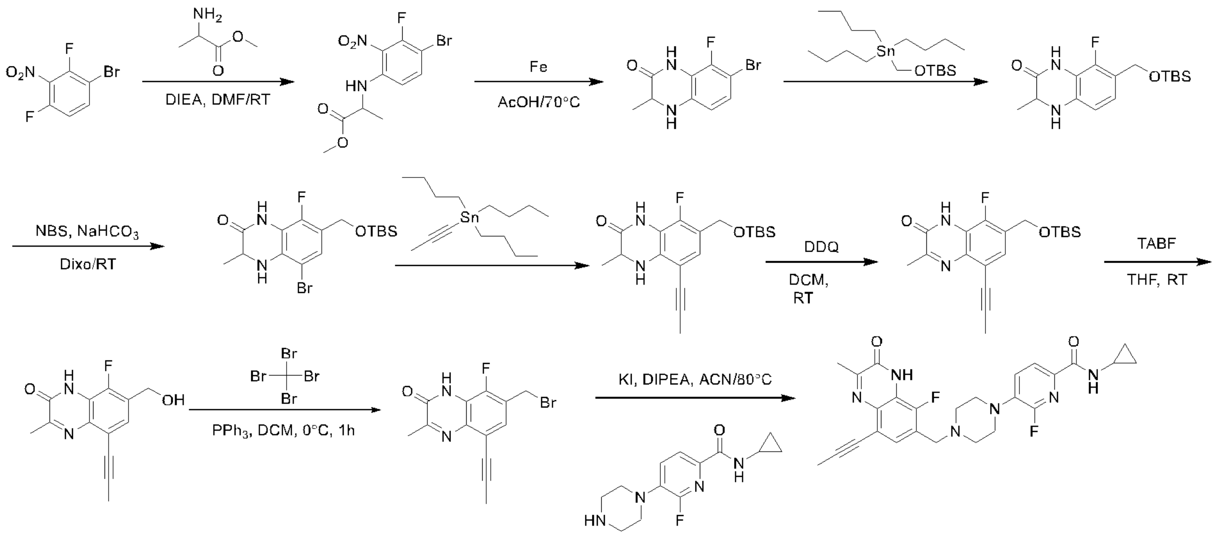


Step 1) Synthesis of Methyl (4-bromo-3-fluoro-2-nitrophenyl)malonate

Ethyl diisopropylamine (16.29 g, 126.06 mmol) was added dropwise to a solution of 1-bromo-2,4-difluoro-3-nitrobenzene (10 g, 42.02 mmol) and methyl 2-aminopropionate (4.42 g, 42.86 mmol), followed by overnight incubation at room temperature. The reaction was stopped, the reaction solution was diluted with DCM (100 mL), washed three times with water (50 mL), concentrated under reduced pressure, and the residue was purified by silica gel column chromatography (DCM/MeOH (v/v) = 20/1) to give a yellow solid product (6 g, 44.47%).

MS (ESI, pos. ion) *m/z:* 321.00 [M+H]$^+$.

Step 2) Synthesis of 7-bromo-8-fluoro-3-methyl-3,4-dihydroquinoxalin-2(1H)-one

Iron powder (8.35 g, 149.48 mmol) was added to a solution of methyl (4-bromo-3-fluoro-2-nitrophenyl)malonate (12 g, 37.37 mmol) in acetic acid (100 mL) with stirring at room temperature. The mixture was then heated to 70 °C and stirred for 4 h. After the reaction solution cooled to room temperature, it was diluted with ethyl acetate (300 mL), the insoluble matter was filtered off, the organic phase was washed with water (60 mL), and then washed with saturated brine (80 mL × 2) to give a brown solid product (9.2 g, 95.02%).

MS (ESI, pos. ion) *m/z:* 259.00 [M+H]$^+$.

Step 3) Synthesis of 7-(((*tert*-butyldimethylsilyl)oxy)methyl)-8-fluoro-3-methyl-3,4-dihydroquinoxalin-2(1H)-one

At room temperature, chloro(2-dicyclohexylphosphine-2',4',6'-triisopropyl-1,1'-biphenyl)(2-(2'-amino-1,1'-biphenyl))palladium(II) (1.40 g, 1.78 mmol) was added to a solution of 7-bromo-8-fluoro-3-methyl-3,4-dihydroquinoxalin-2(1*H*)-one (9.2 g, 35.51 mmol) and tert-butyldimethyl((tributyltin)methoxy)silane (20.10 g, 46.16 mmol), followed by $N_2$ displacement and heating to 90 °C overnight. The reaction was stopped, the reaction solution was cooled to room temperature, filtered through diatomaceous earth, concentrated under reduced pressure, and the residue was purified by silica gel column chromatography (PE/EtOAc(v/v) = 4/1) to give a yellow solid product (8.1 g, 70.30%).

MS (ESI, pos. ion) *m/z:* 325.20 $[M+H]^+$.

Step 4) Synthesis of 5-bromo-7-(((*tert*-butyldimethylsilyl)oxy)methyl)-8-fluoro-3-methyl-3,4-dihydroquinoxalin-2(1*H*)-one

Under $N_2$ protection at 0 °C, N-bromosuccinimide (4.66 g, 26.21 mmol) was added to a solution of 1,4-dioxane (100 mL) containing 7-(((*tert*-butyldimethylsilyl)oxy)methyl)-8-fluoro-3-methyl-3,4-dihydroquinoxalin-2(1*H*)-one (8.1 g, 24.96 mmol) and sodium bicarbonate (3.15 g, 37.44 mmol), and then the mixture was brought to room temperature. TLC results showed the reaction was complete. The reaction was stopped, quenched with saturated sodium thiosulfate aqueous solution (100 mL), extracted with EtOAc (100 mL × 2), dried over anhydrous sodium sulfate, concentrated under reduced pressure, and purified by silica gel column chromatography (PE/EtOAc(v/v) = 5/1) to give a yellow solid product (9.8 g, yield 97.32%).

MS (ESI, pos. ion) *m/z:* 402.90 $[M+H]^+$.

Step 5) Synthesis of 7-(((*tert*-butyldimethylsilyl)oxy)methyl)-8-fluoro-3-methyl-5-(prop-1-yn-1-yl)-3,4-dihydroquinoxalin-2(1H)-one

At room temperature, XPhos-G2-Pd (0.20 g, 0.25 mmol) was added to a solution of 5-bromo-7-(((*tert*-butyldimethylsilyl)oxy)methyl)-8-fluoro-3-methyl-3,4-dihydroquinoxalin-2(1*H*)-one (2 g, 4.96 mmol) in tributyl(prop-1-yn-yl)stannane (2.12 g, 6.45 mmol), and then the reaction was carried out at 90 °C for 12 h. The reaction was stopped, the reaction solution was diluted with DCM (100 mL), filtered with diatomaceous earth, the organic phase was concentrated under reduced pressure, and the residue was purified by silica gel column chromatography (PE/EtOAc (v/v) = 10/1) to give a white solid product (1.6 g, 89.01%).

MS (ESI, pos. ion) *m/z:* 363.20 $[M+H]^+$.

Step 6) Synthesis of 7-(((*tert*-butyldimethylsilyl)oxy)methyl)-8-fluoro-3-methyl-5-(prop-1-yn-1-yl)quinoxalin-2(1H)-one

A solution of 7-(((*tert*-butyldimethylsilyl)oxy)methyl)-8-fluoro-3-methyl-5-(prop-1-yn-1-yl)-3,4-dihydroquinoxalin-2(1*H*)-one (4 g, 11.03 mmol) in dichloromethane (120 mL) was cooled to 0 °C, and 2,3-dichloro-5,6-dicyano-1,4-benzoquinone (2.75 g, 12.13 mmol) was added. The reaction was then allowed to proceed overnight at room temperature. The reaction solution was concentrated under reduced pressure, and saturated $NaHCO_3$ was slowly added at room temperature. The mixture was stirred at room temperature for 6 h, filtered, and the filter cake was washed with water until the filtrate was colorless. The obtained filter cake was dissolved in methanol (60 mL) and evaporated to dryness to give a brownish-red solid product (3.8 g, 95.53%).

MS (ESI, pos. ion) *m/z:* 361.20 $[M+H]^+$.

Step 7) Synthesis of 8-fluoro-7-(hydroxymethyl)-3-methyl-5-(prop-1-yn-1-yl)quinoxalin-2(1*H*)-one

At room temperature, tetrabutylammonium fluoride (5.51 g, 21.08 mmol) was added dropwise to a tetrahydrofuran (20 mL) solution of 7-(((*tert*-butyldimethylsilyl)oxy)methyl)-8-fluoro-3-methyl-5-(prop-1-yn-1-yl)quinoxalin-2(1*H*)-one (3.8 g, 10.54 mmol), and the reaction was allowed to proceed for 7 h at room temperature. The reaction was quenched by adding saturated brine (20 mL), followed by the addition of ethyl acetate (200 mL), washing with water (50 mL × 3), and collecting the organic phase. The solution was dried over anhydrous sodium sulfate. The solution was concentrated under reduced pressure, and the residue was purified by silica gel column chromatography (DCM/MeOH (v/v) = 20/1) to give a yellow solid product (1 g, 38.53%).

MS (ESI, neg. ion) m/z: 245.10 $[M-H]^-$.

Step 8) Synthesis of *N*-cyclopropyl-6-fluoro-5-(4-((5-fluoro-2-methyl-3-oxo-8-(prop-1-yn-1-yl)-3,4-dihydroquinoxalin-6-yl)methyl)piperazin-1-yl)picolinamide

In an ice bath, triphenylphosphine (0.80 g, 3.04 mmol) was added to a solution of 8-fluoro-7-(hydroxymethyl)-3-methyl-5-(prop-1-yn-1-yl)quinoxalin-2(1*H*)-one (0.5 g, 2.03 mmol) in dichloromethane (40 mL), followed by fractional addition of carbon tetrabromide (1.01 g, 3.04 mmol). The reaction was continued in an ice bath for 1 h. The reaction was stopped, the reaction solution was concentrated under reduced pressure, and the residue was purified by silica gel column chromatography (PE/EtOAc(v/v) = 3/2) to give a white solid product (0.3 g, 47.79%).

Step 9) Synthesis of 5-(4-((5-fluoro-2-methyl-3-oxo-8-(prop-1-yn-1-yl)-3,4-dihydroquinoxalin-6-yl)methyl)piperazin-1-yl)-*N*,6-dimethylpicolinamide

At room temperature, potassium iodide (0.0041 g, 0.025 mmol), ethyl diisopropylamine (0.19 g, 1.47 mmol) and N-cyclopropyl-6-fluoro-3-(piperazin-1-yl)pyridine-2-carboxamide (0.19 g, 0.73 mmol) were added to a solution of 7-(bromomethyl)-8-fluoro-3-methyl-5-(prop-1-yn-1-yl)-1,2-dihydroquinoxalin-2-one (0.150 g, 0.49 mmol) in acetonitrile (5 mL), and then the temperature was raised to 80 °C. The reaction was stopped, the reaction solution was concentrated under reduced pressure, dichloromethane was added until the residue dissolved, the mixture was washed with water (20 mL × 2), the organic phase was dried, concentrated under reduced pressure, and the residue was purified by silica gel column chromatography (DCM/MeOH(v/v) = 20/1) to give a white solid product (0.035 g, yield 14.65%).

MS (ESI, pos. ion) *m*/*z*: 493.22 $[M+H]^+$.

$^1H$ NMR (400 MHz, DMSO-$d_6$) δ 12.53 (s, 1H), 8.36 (d, *J* = 4.9 Hz, 1H), 7.87 - 7.81 (m, 1H), 7.60 - 7.52 (m, 1H), 7.35 (d, *J* = 6.7 Hz, 1H), 3.67 (s, 2H), 3.22 - 3.12 (m, 4H), 2.88 - 2.81 (m, 1H), 2.63 - 2.55 (m, 4H), 2.44 (s, 3H), 2.13 (s, 3H), 0.68 - 0.62 (m, 4H).

**Example 48 5-(4-((2-cyclopropyl-5-fluoro-3-oxo-8-(prop-1-yn-1-yl)-3,4-dihydroquinoxalin-6-yl)methyl)piperazin-1-yl)-6-fluoro-*N*-methylpicolinamide**

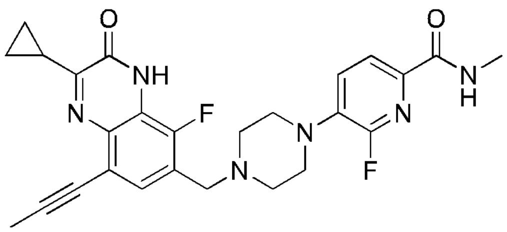

(126)

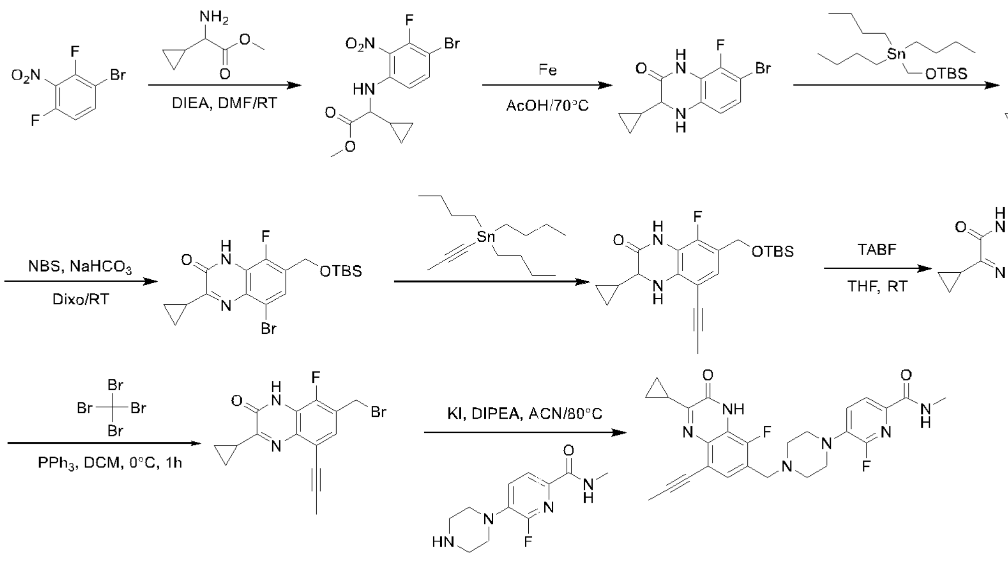

Step 1) Synthesis of methyl 2-((4-bromo-3-fluoro-2-nitrophenyl)amino)-2-cyclopropylacetate

Ethyl diisopropylamine (16.29 g, 126.06 mmol) was added dropwise to a DMF (40 mL) solution of 1-bromo-2,4-difluoro-3-nitrobenzene (10 g, 42.02 mmol) and methyl 2-amino-2-cyclopropylacetate (5.54 g, 42.86 mmol), and the reaction was allowed to proceed to room temperature for 24 h. The reaction was then stopped, and the reaction solution was diluted with dichloromethane (100 mL), washed with water (40 mL × 3), concentrated under reduced pressure, and purified by silica gel column chromatography (petroleum ether/ethyl acetate (v/v) = 10/1) to give a yellow solid product (6 g, yield 41.13%).
MS (ESI, pos. ion) *m/z:* 347.00 $[M+H]^+$.

Step 2) Synthesis of 7-bromo-3-cyclopropyl-8-fluoro-3,4-dihydroquinoxalin-2(1*H*)-one

At room temperature, iron powder (3.86 g, 69.12 mmol) was added to a solution of methyl 2-((4-bromo-3-fluoro-2-nitrophenyl)amino)-2-cyclopropylacetate (6 g, 17.28 mmol) in acetic acid (80 mL) with stirring. The reaction was carried out at 70 °C for 4 h. The reaction was stopped, and the reaction solution was cooled to room temperature. The reaction solution was poured into water (1200 mL), filtered, and the resulting filter cake was washed with water (100 mL × 2). The filter cake was dissolved in (dichloromethane/methanol (v/v) = 10/1, 1 L), filtered through diatomaceous earth, and the resulting organic phase was concentrated under reduced pressure to give a brown solid product (4 g, yield 81.17%).
MS (ESI, pos. ion) *m/z:* 285.00 $[M+H]^+$.

Step 3) Synthesis of 7-(((*tert*-butyldimethylsilyl)oxy)methyl)-3-cyclopropyl-8-fluoro-3,4-dihydroquinoxalin-2(1H)-one

At room temperature, chloro(2-dicyclohexylphosphine-2',4',6'-triisopropyl-1,1'-biphenyl)[2-(2'-amino-1,1'-biphenyl))palladium(II) (0.55 g, 0.70 mmol) was added to a solution of 7-bromo-3-cyclopropyl-8-fluoro-3,4-dihydroquinoxalin-2(1*H*)-one (4 g, 14.03 mmol) and tert-butyldimethyl((tributyltin)methoxy)silane (7.94 g, 18.24 mmol), followed by purging with nitrogen and heating to 90°C overnight. The reaction was stopped, the reaction solution was cooled to room temperature, filtered through diatomaceous earth, concentrated under reduced pressure, and the residue was purified by silica gel column chromatography (PE/EtOAc(v/v) = 4/1) to give a yellow solid product (4.6 g, yield 93.55%).
MS (ESI, pos. ion) *m/z:* 351.20 $[M+H]^+$.

Step 4) Synthesis of 5-bromo-7-(((*tert*-butyldimethylsilyl)oxy)methyl)-3-cyclopropyl-8-fluoroquinoxalin-2(1*H*)-one

Under nitrogen protection at 0°C, *N*-bromosuccinimide (3.50 g, 19.68 mmol) was added to a solution of 7-(((*tert*-butyldimethylsilyl)oxy)methyl)-3-cyclopropyl-8-fluoro-3,4-dihydroquinoxalin-2(1*H*)-one (4.6 g, 13.12 mmol), sodium bicarbonate (3.31 g, 39.36 mmol) in 1,4-dioxane (100 mL). The mixture was then brought to room temperature and reacted for 5 h. The reaction was stopped, and 100 mL of saturated sodium thiosulfate aqueous solution was added to quench the reaction. The product was extracted with ethyl acetate (100 mL × 2), the organic phase was dried over anhydrous sodium sulfate, concentrated under reduced pressure, and the residue was purified by silica gel column chromatography (PE/EtOAc (v/v) = 5/1) to give a yellow solid product (2.6 g, yield 46.35%).
MS (ESI, pos. ion) *m/z:* 427.10 $[M+H]^+$.

Step 5) Synthesis of 7-((*tert*-butyldimethylsilyl)oxy)methyl)-3-cyclopropyl-8-fluoro-5-(prop-1-yn-1-yl)-3,4-dihydroquinoxalin-2(1*H*)-one

At room temperature, tributyl(prop-1-yn-1-yl)tin (2.59 g, 7.88 mmol) was added to a 1,4-dioxane (30 mL) solution of 5-bromo-7-(((*tert*-butyldimethylsilyl)oxy)methyl)-3-cyclopropyl-8-fluoroquinoxalin-2(1*H*)-one (2.59 g, 6.05 mmol), then Xphos Pd G2 (0.24 g, 0.30 mmol) was added. The nitrogen was purged, and the mixture was heated to 90 °C and reacted overnight. TLC results showed that the starting material had not reacted completely. Xphos-G2-Pd (0.48 g, 0.6 mol) and tributyl(prop-1-yn-1-yl)tin (3 g, 9.05 mmol) were added, and the mixture was heated to 105 °C and reacted for another 8 h. The reaction was then stopped, cooled to room temperature, filtered through diatomaceous earth, concentrated under reduced pressure, and the residue was purified by silica gel column chromatography (PE/DCM (v/v) = 1/1) to give a yellow solid product (1.2 g, yield 51.23%).
MS (ESI, pos. ion) *m/z:* 389.25 $[M+H]^+$.

Step 6) Synthesis of 3-cyclopropyl-8-fluoro-7-(hydroxymethyl)-5-(prop-1-yn-1-yl)quinoxalin-2(1*H*)-one

At room temperature, tetrabutylammonium fluoride (1.62 g, 6.2 mmol, 1 M in THF) was added dropwise to 7-((*tert*-butyldimethylsilyl)oxy)methyl)-3-cyclopropyl-8-fluoro-5-(prop-1-yn-1-yl)-3,4-dihydroquinoxalin-2(1*H*)-one (1.2 g,

3.10 mmol), and the reaction was allowed to proceed at room temperature for 2.5 h. TLC results showed that the reaction was complete. The reaction was quenched with saturated brine (30 mL), washed with ethyl acetate (200 mL), and the organic phase was collected and dried over anhydrous sodium sulfate. The residue was purified by silica gel column chromatography (DCM/MeOH(v/v) = 20/1) to give a yellow solid product (0.75 g, yield 88.73%).

MS (ESI, pos. ion) *m/z:* 273.20 $[M+H]^+$.

Step 7) Synthesis of 7-(bromomethyl)-3-cyclopropyl-8-fluoro-5-(prop-1-yn-1-yl)quinoxalin-2(1H)-one

In an ice bath, triphenylphosphine (0.38 g, 1.46 mmol) was added to a solution of 3-cyclopropyl-8-fluoro-7-(hydroxymethyl)-5-(prop-1-yn-1-yl)quinoxalin-2(1H)-one (0.2 g, 0.73 mmol) in dichloromethane (10 mL), followed by fractional addition of carbon tetrabromide (0.48 g, 1.46 mmol). The reaction was continued in an ice bath for 2 h. The reaction was stopped, the reaction solution was concentrated under reduced pressure, and the residue was purified by silica gel column chromatography (PE/EtOAc (v/v) = 3/2) to give a pale yellow solid product (0.13 g, yield 52.80%).

Step 8) Synthesis of 5-(4-((2-cyclopropyl-5-fluoro-3-oxo-8-(prop-1-yn-1-yl)-3,4-dihydroquinoxalin-6-yl)methyl)piperazin-1-yl)-6-fluoro-N-methylpicolinamide

At room temperature, potassium iodide (0.0032 g, 0.020 mmol), N,N-diisopropylethylamine (0.15 g, 1.17 mmol), and 6-fluoro-*N*-methyl-5-(piperazin-1-yl)pyridine-2-carboxamide (0.14 g, 0.58 mmol) were added to a solution of 7-(bromomethyl)-3-cyclopropyl-8-fluoro-5-(prop-1-yn-1-yl)quinoxalin-2(1*H*)-one (0.13 g, 0.39 mmol) in acetonitrile (10 mL), and then the temperature was raised to 80 °C. The reaction was stopped, the reaction solution was concentrated under reduced pressure, DCM was added until the residue dissolved, the mixture was washed with water (20 mL × 3), the organic phase was dried, concentrated under reduced pressure, and the residue was purified by column chromatography (DCM/MeOH (v/v) = 20/1). The product was purified by plate chromatography and the resulting white solid product (0.035 g, yield 18.32%) was obtained.

MS (ESI, pos. ion) *m/z:* 493.25 $[M+H]^+$.

$^1$H NMR (400 MHz, DMSO-$d_6$) δ 12.50 (s, 1H), 8.44 - 8.35 (m, 1H), 7.83 (dd, *J* = 8.1, 1.4 Hz, 1H), 7.55 (dd, *J* = 10.6, 8.1 Hz, 1H), 7.29 (d, *J* = 6.8 Hz, 1H), 3.65 (s, 2H), 3.20 - 3.11 (m, 4H), 2.76 (d, *J* = 4.7 Hz, 3H), 2.73 - 2.64 (m, 1H), 2.61 - 2.53 (m, 4H), 2.11 (s, 3H), 1.16 - 1.04 (m, 4H).

**Example 49 5-(4-((8-(difluoromethoxy)-2-methyl-3-oxo-3,4-dihydroquinoxalin-6-yl)methyl)piperazin-1-yl)-*N*-methylpicolinamide**

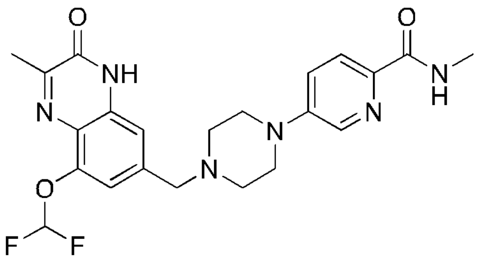

(127)

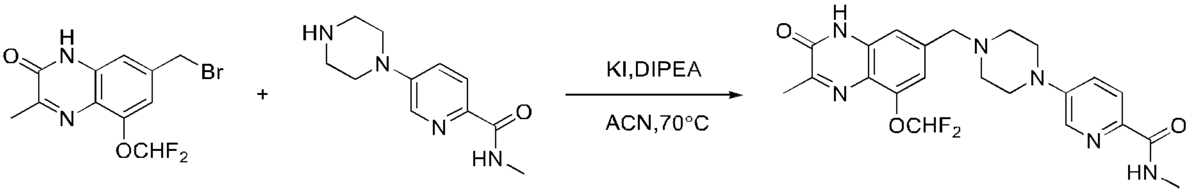


7-(bromomethyl)-5-(difluoromethoxy)-3-methyl-1,2-dihydroquinoxalin-2-one (0.1 g, 0.31 mmol), *N*-methyl-5-(piperazin-1-yl)picolinamide (0.070 g, 0.32 mmol), potassium iodide (0.051 g, 0.31 mmol), acetonitrile (10 mL) and *N,N*-diisopropylethylamine (0.080 g, 0.62 mmol) were added sequentially to a reaction flask. The mixture was heated to 70 °C and reacted for 2 hours. The reaction was then stopped and cooled to room temperature. The mixture was concentrated under reduced pressure, and 30 mL of water was added. The mixture was extracted with a mixed solution (DCM/MeOH (v/v) = 10/1) (40 mL × 2). The organic phase was washed with saturated brine, dried over anhydrous sodium

sulfate, filtered, concentrated under reduced pressure, and the residue was purified by column chromatography (DCM/MeOH (v/v) = 20/1) yielded a white solid (0.059 g, 41%).

MS (ESI, pos. ion) *m/z:* 459.2 $[M+H]^+$;

$^1H$ NMR (400 MHz, DMSO-$d_6$) δ 12.43 (s, 1H), 8.44 - 8.38 (m, 1H), 8.29 - 8.24 (m, 1H), 7.85 - 7.80 (m, 1H), 7.42 - 7.37 (m, 1H), 7.36 (t, J =74.4 Hz ,1H), 7.17 - 7.04 (m, 2H), 3.61 (s, 2H), 3.35 - 3.31 (m, 4H), 2.77 (d, J = 4.8 Hz, 3H), 2.59 - 2.51 (m, 4H), 2.41 (s, 3H).

**Example 50 1'-((5-fluoro-2-methyl-3-oxo-8-(prop-1-yn-1-yl)-3,4-dihydroquinoxalin-6-yl)methyl)-*N*-methyl-1',2',3',6'-tetrahydro-[3,4'-bipyridine]-6-carboxamide**

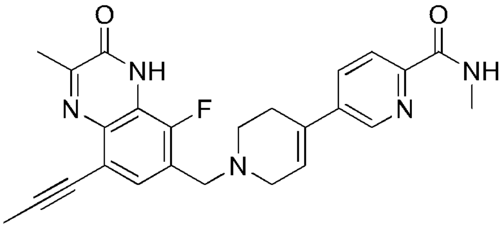

(128)

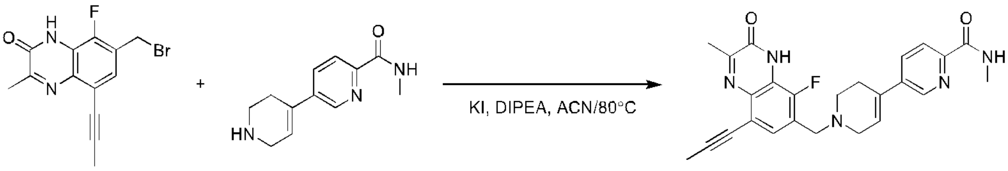


7-(bromomethyl)-8-fluoro-3-methyl-5-(prop-1-yn-1-yl)-1,2-dihydroquinoxalin-2-one (0.10 g, 0.32 mmol), *N*-cyclopropyl-5-(1,2,3,6-tetrahydropyridin-4-yl)pyridine-2-carboxamide hydrochloride (0.12 g, 0.42 mmol) and potassium iodide (0.011 g, 0.064 mmol) were added to a 100 mL flask, stirred to dissolve in ACN (3.5 mL), after cooling, DIPEA (0.17 mL, 0.96 mmol) was added slowly dropwise under ice bath, stirred until homogeneous, and heated to 80 °C for 2 h. After cooling to room temperature, a solid precipitated out with stirring. The filter cake was collected by filtration, washed with MTBE (3.5 mL × 3) and water (3.5 mL × 3), and dried under vacuum to obtain a yellow crude solid. The crude solid was then purified by silica gel column chromatography (DCM/MeOH(v/v) = 20/1) to obtain a white solid (0.070 g, yield 45.89%).

MS (ESI, pos. ion) *m/z:* 446.20 $[M+H]^+$;

$^1H$ NMR (400 MHz, DMSO-$d_6$) δ 12.53 (s, 1H), 8.90 - 8.65 (m, 2H), 7.97 (s, 2H), 7.36 (d, *J* = 6.3 Hz, 1H), 6.39 (s, 1H), 3.72 (s, 2H), 3.15 (br, 2H), 2.89 (br, 2H), 2.71 (br, 3H), 2.44 (s, 3H), 2.11 (s, 3H), 0.66 (s, 4H).

**Example 51 *N*-methyl-1'-((2-methyl-3-oxo-8-(prop-1-yn-1-yl)-3,4-dihydroquinoxalin-6-yl)methyl)-1',2',3',6'-tetrahydro-[3,4'-bipyridine]-6-carboxamide**

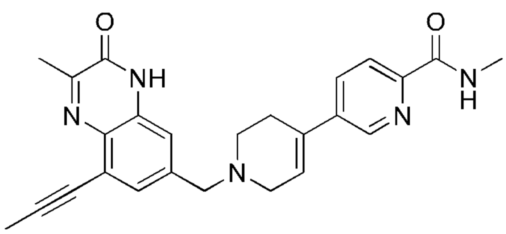

(129)

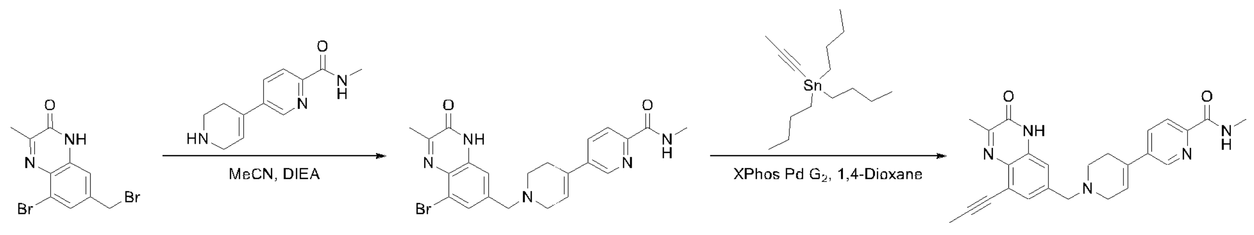


Step 1) Synthesis of 1'-((8-bromo-2-methyl-3-oxo-3,4-dihydroquinoxalin-6-yl)methyl)-*N*-methyl-1',2',3',6'-tetrahydro-[3,4'-bipyridine]-6-carboxamide

At room temperature, *N*-methyl-1',2',3',6'-tetrahydro-[3,4'-bipyridine]-6-carboxamide (0.43 g, 1.99 mmol), DIPEA (1.17 g, 9.05 mmol), and KI (0.015 g, 0.091 mmol) were added to a solution of 5-bromo-7-(bromomethyl)-3-methylquinoxalin-2(1*H*)-one (0.6 g, 1.81 mmol) in ACN (20 mL), followed by reaction at 80 °C for 3 h. The reaction solution was concentrated under reduced pressure, and the residue was purified by silica gel column chromatography (eluent: $CH_2Cl_2$/MeOH (v/v) = 20/1) to give a yellow solid product (0.11 g, 13%).
MS (ESI, pos. ion) *m/z:* 428.3 $[M+H]^+$;

Step 2) Synthesis of *N*-methyl-1'-((2-methyl-3-oxo-8-(prop-1-yn-1-yl)-3,4-dihydroquinoxalin-6-yl)methyl)-1',2',3',6'-tetrahydro-[3,4'-bipyridine]-6-carboxamide

At room temperature, tributyl(propynyl-1-acetylenol-1-yl)tin (0.12 g, 0.38 mmol) and Xphos Pd G2 (0.025 g, 0.032 mmol) were added to a solution of 1'-((8-bromo-2-methyl-3-oxo-3,4-dihydroquinoxalin-6-yl)methyl)-*N*-methyl-1',2',3',6'-tetrahydro-[3,4'-bipyridine]-6-carboxamide (0.1 g, 0.21 mmol) in 1,4-dioxane (20 mL), $N_2$ was replaced, and the mixture was heated to 90 °C and stirred for 12 h. After the reaction solution cooled to room temperature, it was filtered through diatomaceous earth. The filtrate was concentrated under reduced pressure, and the residue was purified by silica gel column chromatography (eluent: DCM/MeOH (v/v) = 20/1) to give a yellow solid product (0.01 g, 10.96%).
MS (ESI, pos. ion) *m/z:* 428.2 $[M+H]^+$;
$^1$H NMR (400 MHz, DMSO-$d_6$) δ (ppm) 12.32 (s, 1H), 8.76 - 8.67 (m, 2H), 8.03 - 7.93 (m, 2H), 7.31 (s, 1H), 7.23 (s, 1H), 6.42 (s, 1H), 3.64 (s, 2H), 3.30 - 3.25 (m, 9H), 3.14 - 3.11 (m, 2H), 2.81 (d, *J* = 4.8 Hz, 3H), 2.72 - 2.65 (m, 3H), 2.41 (s, 3H), 2.13 (s, 3H).

**Example 52 5-(4-((8-(difluoromethoxy)-2-methyl-3-oxo-3,4-dihydroquinoxalin-6-yl)methyl)piperazin-1-yl)-*N*,6-dimethylpicolinamide**

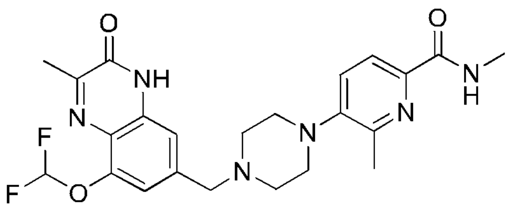

(130)

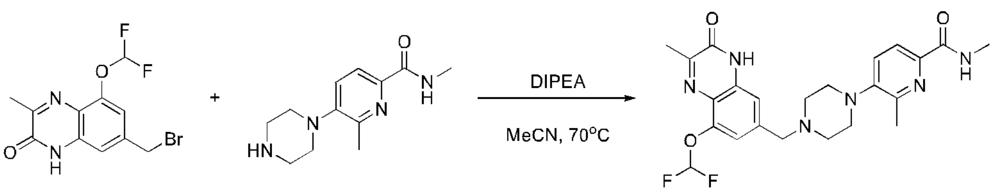


7-(bromomethyl)-5-(difluoromethoxy)-3-methylquinoxalin-2(1*H*)-one (100 mg, 0.31 mmol), *N*,6-dimethyl-5-(piperazin-1-yl)picolinamide (109 mg, 0.46 mmol), and DIPEA (120 mg, 0.93 mmol) were added sequentially to MeCN (8 mL), and the reaction was carried out at 70 °C for 2 h. The reaction solution was concentrated under reduced pressure, diluted with DCM (60 mL), washed with water (30 mL) and saturated NaCl solution (30 mL), dried over anhydrous $Na_2SO_4$, concentrated under reduced pressure, and the residue was purified by silica gel column chromatography (DCM/MeOH

(v/v) = 20/1) to give a pale yellow solid (100 mg, 68%).

MS (ESI, pos. ion) *m/z:* 473.3 $[M+H]^+$;

$^1$H NMR (400 MHz, DMSO-$d_6$) δ 12.44 (s, 1H), 8.43 (q, *J* = 4.9 Hz, 1H), 7.79 (d, *J* = 8.2 Hz, 1H), 7.55 (s, 1H), 7.49 (d, *J* = 8.3 Hz, 1H), 7.27 (d, *J* = 74.4 Hz, 1H), 7.15 (s, 1H), 7.07 (s, 1H), 3.63 (s, 2H), 2.96 (s, 4H), 2.79 (d, *J* = 4.8 Hz, 3H), 2.59 (s, 4H), 2.41 (s, 3H).

**Example 53 6-fluoro-5-(4-((5-fluoro-2-methyl-3-oxo-8-(phenylethynyl)-3,4-dihydroquinoxalin-6-yl)methyl)piperazin-1-yl)-*N*-methylpicolinamide**

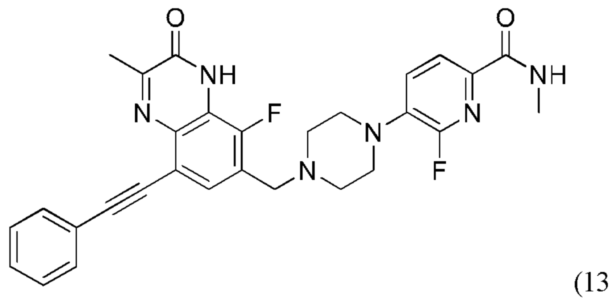

(131)

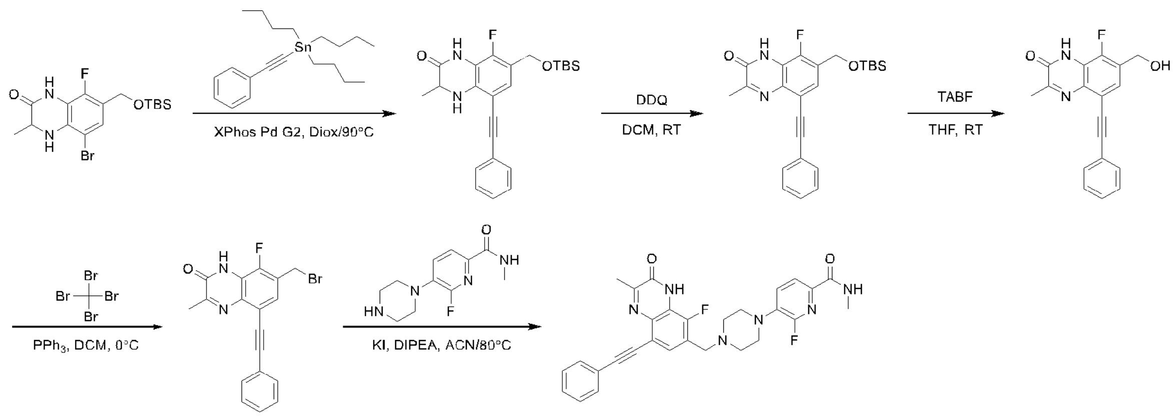


Step 1) Synthesis of 7-(((tert-butyldimethylsilyl)oxy)methyl)-8-fluoro-3-methyl-5-(2-phenylethynyl)-1,2,3,4-tetrahydroquinoxalin-2-one

In a 250 mL flask, 5-bromo-7-(((tert-butyldimethylsilyl)oxy)methyl)-8-fluoro-3-methyl-1,2,3,4-tetrahydroquinoxalin-2-one (1.0 g, 2.48 mmol) and tributyl(2-phenylethynyl)tin (1.16 g, 2.98 mmol) were added sequentially and stirred to dissolve in 1,4-dioxane (10 mL). Then, XPhos Pd G2 (0.098 g, 0.12 mmol) was added, the mixture was purged with nitrogen, and the mixture was heated to 90 °C and reacted overnight. The reaction solution was cooled to room temperature, filtered, concentrated under reduced pressure, and the residue was purified by silica gel column chromatography (dichloromethane/ethyl acetate (v/v) = 10/1) to give a yellow solid (1.05 g, 99.75%).

MS (ESI, pos. ion) *m/z:* 425.20 $[M+H]^+$.

Step 2) Synthesis of 7-(((*tert*-butyldimethylsilyl)oxy)methyl)-8-fluoro-3-methyl-5-(2-phenylethynyl)-1,2-dihydroquinoxalin-2-one

7-(((*tert*-butyldimethylsilyl)oxy)methyl)-8-fluoro-3-methyl-5-(2-phenylethynyl)-1,2,3,4-tetrahydroquinoxalin-2-one (1.05 g, 2.47 mmol) was added to a 250 mL flask and stirred to dissolve in DCM (22 mL). After cooling, DDQ (0.62 g, 2.72 mmol) was added under ice bath conditions, and the mixture was stirred overnight at room temperature. The solution was concentrated under reduced pressure, quenched with saturated sodium bicarbonate (40 mL) at room temperature, and stirred for 1 h. A solid precipitated out, the solid was filtered, the filter cake was washed with sodium bicarbonate solution, and dried under vacuum to obtain 1.0 g of a brownishyellow solid, which was then directly proceeded to the next step.

MS (ESI, pos. ion) *m/z:* 423.30 $[M+H]^+$.

Step 3) Synthesis of 8-fluoro-7-(hydroxymethyl)-3-methyl-5-(2-phenylethynyl)-1,2-dihydroquinoxalin-2-one

Into a 250 mL flask, 7-(((*tert*-butyldimethylsilyl)oxy)methyl)-8-fluoro-3-methyl-5-(2-phenylethynyl)-1,2-dihydroquinoxalin-2-one (0.99 g, 2.34 mmol) was added and dissolved in THF (18 mL) with stirring. Then, a TBAF tetrahydrofuran solution (5.85 mL, 5.85 mmol) was added dropwise, and the mixture was stirred at room temperature for 6 hours. The reaction was quenched by adding saturated brine (30 mL), followed by extraction with EtOAc (20 mL × 2). The organic phases were combined, dried over anhydrous sodium sulfate, concentrated under reduced pressure, and the residue was purified by silica gel column chromatography (DCM/MeOH (v/v) = 20/1) to give a brown solid (0.965 g, 134 %).

MS (ESI, pos. ion) *m/z:* 309.20 $[M+H]^+$.

Step 4) Synthesis of 7-(bromomethyl)-8-fluoro-3-methyl-5-(2-phenylethynyl)-1,2-dihydroquinoxalin-2-one

8-fluoro-7-(hydroxymethyl)-3-methyl-5-(2-phenylethynyl)-1,2-dihydroquinoxalin-2-one (0.72 g, 2.34 mmol) and triphenylphosphine (0.92 g, 3.51 mmol) were added to a 100 mL flask and stirred to dissolve in DCM (15 mL). The mixture was cooled, and carbon tetrabromide (1.16 g, 3.51 mmol) was added under ice bath conditions. The mixture was stirred at 0 °C for 1 h. The mixture was concentrated under reduced pressure, and the residue was purified by silica gel column chromatography (petroleum ether/ethyl acetate (v/v) = 3/1) to give a white solid (0.13 g, 15%).

Step 5) Synthesis of 6-fluoro-5-(4-((5-fluoro-2-methyl-3-oxo-8-(phenylethynyl)-3,4-dihydroquinoxalin-6-yl)methyl)piperazin-1-yl)-*N*-methylpyridine-2-carboxamide

7-(bromomethyl)-8-fluoro-3-methyl-5-(2-phenylethynyl)-1,2-dihydroquinoxalin-2-one (0.12 g, 0.32 mmol), 6-fluoro-*N*-methyl-5-(piperazin-1-yl)pyridine-2-carboxamide (0.090 g, 0.38 mmol) and potassium iodide (0.011 g, 0.064 mmol) were added to a 100 mL flask and stirred to dissolve in ACN (3 mL), the mixture was cooled, and DIPEA (0.11 mL, 0.64 mmol) was added slowly dropwise under ice bath. The mixture was stirred thoroughly and heated to 80°C for 2 hours. After cooling to room temperature, a solid precipitated out with stirring. The filter cake was collected by filtration, washed with MTBE (3 mL × 3) and water (3 mL × 3), dried under vacuum, and then purified by silica gel column chromatography (dichloromethane/methanol (v/v) = 10/1) to obtain a white solid (0.074 g, 43.3%).

MS (ESI, pos. ion) *m/z:* 529.30 $[M+H]^+$;
$^1H$ NMR (400 MHz, DMSO-$d_6$) δ 12.61 (s, 1H), 8.41 (dd, *J* = 10.0, 5.5 Hz, 1H), 7.84 (d, *J* = 8.0 Hz, 1H), 7.62 - 7.58 (m, 2H), 7.57 - 7.50 (m, 2H), 7.48 - 7.45 (m, 3H), 3.71 (s, 2H), 3.18 (s, 4H), 2.76 (d, *J* = 4.4 Hz, 3H), 2.62 (s, 4H), 2.48 (s, 3H).

**Example 54 5-(4-((2-cyclopropyl-8-(difluoromethoxy)-3-oxo-3,4-dihydroquinoxalin-6-yl)methyl)piperazin-1-yl)-6-fluoro-*N*-methylpicolinamide**

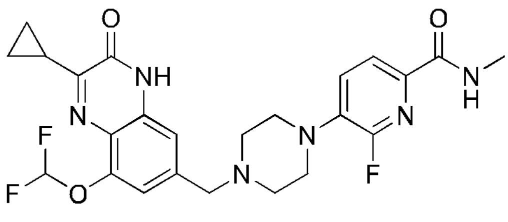

(132)

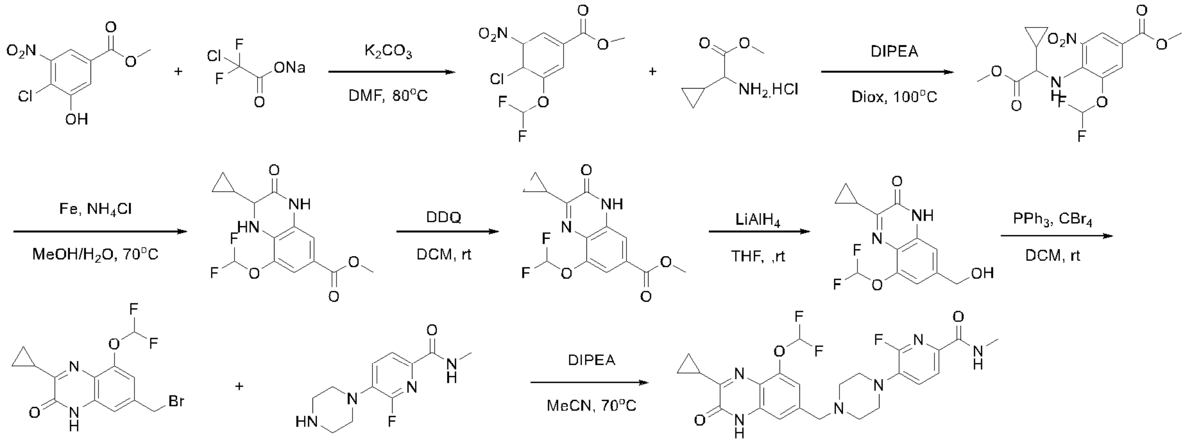


Step 1) Synthesis of methyl 4-chloro-3-(difluoromethoxy)-5-nitrobenzoate

Methyl 4-chloro-3-hydroxy-5-nitrobenzoate (3.0 g, 12.95 mmol), sodium 2-chloro-2,2-difluoroacetate (5.92 g, 38.85 mmol), and $K_2CO_3$ (2.15 g, 15.54 mmol) were added to DMF (40 mL) and reacted at 80 °C for 16 h. The reaction solution was diluted with EtOAc (100 mL), washed with water (80 mL × 3), then washed with saturated NaCl solution (50 mL), dried over anhydrous $Na_2SO_4$, filtered, concentrated under reduced pressure, and the residue was purified by silica gel column chromatography (PE/EtOAc (v/v) = 5/1) to give a pale yellow solid (1.58 g, 43%).

MS (ESI, pos. ion) *m/z:* 282.1 $[M+H]^+$.

Step 2) Synthesis of methyl 4-((1-cyclopropyl-2-methoxy-2-oxoethyl)amino)-3-(difluoromethoxy)-5-nitrobenzoate

Methyl 4-chloro-3-(difluoromethoxy)-5-nitrobenzoate (1.50 g, 5.33 mmol), methyl 2-amino-2-cyclopropylacetate hydrochloride (1.77 g, 10.66 mmol), and DIPEA (2.76 g, 21.32 mmol) were added to 1,4-dioxane (20 mL), and the reaction was carried out at 100 °C for 17 h. The reaction mixture was concentrated under reduced pressure, and the residue was purified by silica gel column chromatography (PE/EtOAc (v/v) = 4/1) to give a yellow solid (1.56 g, 78%).

MS (ESI, pos. ion) *m/z:* 375.1 $[M+H]^+$.

Step 3) Synthesis of methyl 2-cyclopropyl-8-(difluoromethoxy)-3-oxo-1,2,3,4-tetrahydroquinoxaloline-6-carboxylic acid

Iron powder (1.40 g, 25.02 mmol), $NH_4Cl$ (1.34 g, 25.02 mmol) and methyl 4-((1-cyclopropyl-2-methoxy-2-oxoethyl)amino)-3-(difluoromethoxy)-5-nitrobenzoate (1.56 g, 4.17 mmol) were added to a solution of MeOH (20 mL) and water (5 mL), and reacted at 70 °C for 3 h. The reaction solution was filtered through diatomaceous earth, the filtrate was concentrated under reduced pressure, diluted with EtAOc (100 mL) and water (50 mL), extracted and separated, the organic phase was washed with saturated NaCl solution (30 mL), dried over anhydrous $Na_2SO_4$, filtered, concentrated under reduced pressure, and the residue was purified by silica gel column chromatography (PE/EtOAc (v/v) = 3/2) to give a white solid (440 mg, 34%).

MS (ESI, pos. ion) *m/z:* 313.1 $[M+H]^+$.

Step 4) Synthesis of methyl 2-cyclopropyl-8-(difluoromethoxy)-3-oxo-3,4-dihydroquinoxaloline-6-carboxylic acid

DDQ (384 mg, 1.69 mmol) was added to a solution of methyl 2-cyclopropyl-8-(difluoromethoxy)-3-oxo-1,2,3,4-tetrahydroquinoxalolin-6-carboxylic acid (440 mg, 1.41 mmol) in DCM (8 mL), and the reaction was carried out at room temperature for 4 h. The reaction solution was concentrated under reduced pressure, and the residue was neutralized by stirring in a saturated $NaHCO_3$ solution (60 mL). A solid precipitated, which was filtered to obtain a solid. The solid was dried under vacuum at 50 °C for 12 h to obtain a pale yellow solid (400 mg, 91%).

MS (ESI, pos. ion) *m/z:* 311.1 $[M+H]^+$.

Step 5) Synthesis of 3-cyclopropyl-5-(difluoromethoxy)-7-(hydroxymethyl)quinoxalin-2(1*H*)-one

$LiAlH_4$ (108 mg, 2.58 mmol) was added to a THF (10 mL) solution of methyl 2-cyclopropyl-8-(difluoromethoxy)-3-oxo-3,4-dihydroquinoxalin-6-carboxylic acid (400 mg, 1.29 mmol) and reacted at room temperature for 5 h. The reaction was quenched with MeOH (10 mL), diluted with DCM (50 mL), concentrated under reduced pressure, and the residue was

purified by silica gel column chromatography (DCM/MeOH (v/v) = 20/1) to give a white solid (300 mg, 82%).

MS (ESI, pos. ion) *m/z:* 283.1 $[M+H]^+$.

Step 6) Synthesis of 7-(bromomethyl)-3-cyclopropyl-5-(difluoromethoxy)quinoxalin-2(1*H*)-one

At 0 °C, $CBr_4$ (723 mg, 2.18 mmol) was added to a DCM (16 mL) solution of 3-cyclopropyl-5-(difluoromethoxy)-7-(hydroxymethyl)quinoxalin-2(1*H*)-one (300 mg, 1.06 mmol) and $PPh_3$ (572 mg, 2.18 mmol), and the reaction was carried out at 0 °C for 4 h. The reaction solution was concentrated under reduced pressure, and the residue was purified by silica gel column chromatography (PE/EtOAc (v/v) = 1/1) to give a white solid (300 mg, 80%).

MS (ESI, pos. ion) *m/z:* 345.1 $[M+H]^+$;

Step 7) Synthesis of 5-(4-((2-cyclopropyl-8-(difluoromethoxy)-3-oxo-3,4-dihydroquinoxalin-6-yl)methyl)piperazin-1-yl)-6-fluoro-*N*-methylpicolinamide

7-(bromomethyl)-3-cyclopropyl-5-(difluoromethoxy)quinoxalin-2(1*H*)-one (150 mg, 0.43 mmol), 6-fluoro-N-methyl-5-(piperazin-1-yl)picolinamide (133 mg, 0.56 mmol) and DIPEA (167 mg, 1.29 mmol) were added sequentially to MeCN (8 mL), and the reaction was carried out at 70 °C for 2 h. The reaction solution was concentrated under reduced pressure, diluted with DCM (60 mL), washed with water (30 mL) and saturated NaCl solution (30 mL), dried over anhydrous $Na_2SO_4$, concentrated under reduced pressure, and the residue was purified by silica gel column chromatography (DCM/MeOH (v/v) = 20/1) to give a white solid (120 mg, 55%).

MS (ESI, pos. ion) *m/z:* 503.2 $[M+H]^+$;

$^1H$ NMR (400 MHz, DMSO-$d_6$) δ (ppm) 12.46 (s, 1H), 8.41 (q, *J* = 4.3 Hz, 1H), 7.85 (d, *J* = 7.3 Hz, 1H), 7.58 (dd, *J* = 10.6, 8.2 Hz, 1H), 7.15 (t, *J* = 74.2 Hz, 1H), 7.15 (s, 1H), 7.05 (s, 1H), 3.60 (s, 2H), 3.18 (s, 4H), 2.77 (d, *J* = 4.8 Hz, 3H), 2.70 - 2.65 (m, 1H), 2.57 (s, 4H), 1.10 (s, 2H), 1.08 (s, 2H).

**Example 55 5-(4-((5-(difluoromethoxy)-3-methyl-2-oxo-1,2-dihydroquinolin-7-yl)methyl)piperazin-1-yl)-6-fluoro-*N*-methylpicolinamide**

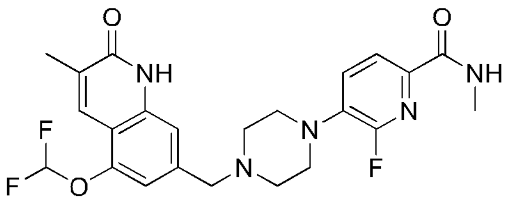

(133)

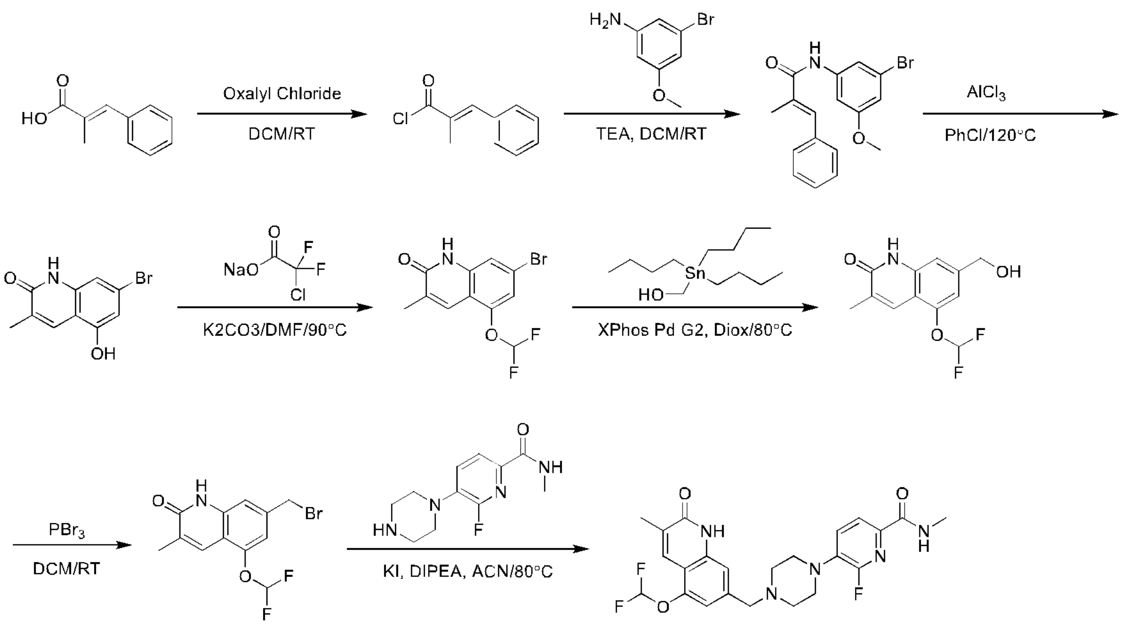

Step 1) Synthesis of 2-methyl-3-phenylacryloyl chloride

A-methylcinnamic acid (3.0 g, 18.50 mmol) was added to a 250 mL flask and stirred to dissolve in DCM (30 mL). 2-3 drops of DMF were added, and the mixture was cooled. Oxaloyl chloride (2.5 mL, 29.54 mmol) was slowly added dropwise under ice bath conditions. After stirring for 5 min, the mixture was moved to room temperature and the reaction continued for 8 h. Stirring was stopped, and the mixture was concentrated under reduced pressure (< 40 °C) to give a yellow solid product (3.3 g, 98.8%), which was then directly used for the next reaction.

Step 2) Synthesis of *N*-(3-bromo-5-methoxyphenyl)-2-methyl-3-phenylacrylamide

2-methyl-3-phenylacryloyl chloride (3.31 g, 18.34 mmol) was added to a 250 mL flask, dissolved in DCM (40 mL) and cooled. Under an ice bath, 3-bromo-5-methoxyaniline (2.85 g, 14.11 mmol) was added, followed by slow dropwise addition of triethylamine (3.92 mL, 28.22 mmol). After completion of the addition, the mixture was moved to room temperature and stirred overnight. The reaction was quenched with saturated brine (50 mL) and extracted with DCM (35 mL × 2). The combined organic phases were washed with saturated brine, dried over anhydrous sodium sulfate, and concentrated under reduced pressure. The residue was purified by silica gel column chromatography (PE/EtOAc (v/v) = 5/1) to afford a brown-yellow solid (3.73 g, 76.38%).
MS (ESI, pos. ion) *m/z:* 346.15 $[M+H]^+$.

Step 3) Synthesis of 7-bromo-5-hydroxy-3-methylquinolin-2(1*H*)-one

*N*-(3-bromo-5-methoxyphenyl)-2-methyl-3-phenylacrylamide (3.7 g, 10.69 mmol) was added to a 250 mL two-necked flask and dissolved in chlorobenzene (70 mL). The mixture was purged with nitrogen, cooled, and anhydrous aluminum trichloride (4.28 g, 32.07 mmol) was added in portions under ice bath conditions. The mixture was stirred thoroughly and refluxed at 120 °C for 3 h. After cooling to room temperature, the reaction solution was quenched in a large amount of ice water, resulting in the precipitation of a solid. The mixture was stirred for 15 min, filtered to collect the filter cake, washed with water until neutral, dried under vacuum, and then purified by silica gel column chromatography (DCM/MeOH (v/v) = 9/1) to obtain a brown solid (0.56 g, 20.62%).
MS (ESI, neg. ion) *m/z:* 252.05 $[M-H]^-$.

Step 4) Synthesis of 7-bromo-5-(difluoromethoxy)-3-methylquinoline-2(1*H*)-one

7-bromo-5-hydroxy-3-methylquinoline-2(1*H*)-one (0.45 g, 1.77 mmol) and sodium difluorochloroacetate (0.56 g, 3.54 mmol) were added to a 100 mL flask and stirred to dissolve in DMF (10 mL). Potassium carbonate (0.37 g, 2.66 mmol) was then added, and the reaction was heated to 90 °C for 2 h. The mixture was cooled in an ice bath and quenched in water (50 mL). A solid precipitated out. The mixture was stirred for 30 min, filtered, and the filter cake was collected. The cake was washed with water until neutral and dried under vacuum to obtain a brown solid (0.51 g, 94.69%).
MS (ESI, pos. ion) *m/z:* 303.95 $[M+H]^+$;

Step 5) Synthesis of 5-(difluoromethoxy)-7-(hydroxymethyl)-3-methylquinolin-2(1*H*)-one

7-bromo-5-(difluoromethoxy)-3-methylquinolin-2(1*H*)-one (0.31 g, 1.02 mmol) and (tributyltin)methanol (0.45 g, 1.33 mmol) were added sequentially to a 100 mL flask and stirred to dissolve in 1,4-dioxane (9 mL). XPhos Pd G2 (0.048 g, 0.061 mmol) was then added, the mixture was purged with nitrogen, and the mixture was heated to 80 °C overnight. After cooling to room temperature, the mixture was filtered, concentrated under reduced pressure, and the residue was separated by silica gel column chromatography (DCM/MeOH (v/v) = 9/1) to give a milky white solid (0.060 g, 23.06%).
MS (ESI, pos. ion) *m/z:* 256.10 $[M+H]^+$;

Step 6) Synthesis of 7-(Bromomethyl)-5-(difluoromethoxy)-3-methylquinolin-2(1*H*)-one

5-(difluoromethoxy)-7-(hydroxymethyl)-3-methylquinolin-2(1*H*)-one (0.050 g, 0.20 mmol) was added to a 100 mL flask, which was dissolved in DCM (5 mL) under stirring and cooled. Under an ice bath, phosphorus tribromide (0.04 mL, 0.43 mmol) was added, and the mixture was allowed to warm gradually to room temperature and stirred for 4 hours. The mixture was concentrated under reduced pressure, and the residue was washed with MTBE (5 mL × 2). The resulting crude product was directly used in the next step.

Step 7) Synthesis of 5-(4-((5-(difluoromethoxy)-3-methyl-2-oxo-1,2-dihydroquinolin-7-yl)methyl)piperazin-1-yl)-6-fluoro-*N*-methylpicolinamide

7-(bromomethyl)-5-(difluoromethoxy)-3-methylquinolin-2(1*H*)-one (0.060 g, 0.19 mmol), 6-fluoro-*N*-methyl-5-(piperazin-1-yl)pyridine-2-carboxamide (0.077 g, 0.25 mmol), and potassium iodide (0.0063 g, 0.038 mmol) were added sequentially to a 100 mL flask and stirred until dissolved in ACN (2 mL). The mixture was cooled, and DIPEA (0.17 mL, 0.95 mmol) was added dropwise under ice bath conditions. The mixture was stirred until homogeneous and then heated to 80 °C for 2 h. Dilute with DCM (40 mL), wash twice with saturated saline (30 mL), dry with anhydrous sodium sulfate, concentrate under reduced pressure, and purify the residue by silica gel column chromatography (DCM/MeOH (v/v) = 20/1) to give a pale yellow solid (0.077 g, 85.86%).
MS (ESI, pos. ion) *m/z:* 476.20 $[M+H]^+$;

**Example 56 5-(4-((8-(difluoromethoxy)-2-(difluoromethyl)-3-oxo-3,4-dihydroquinoxalin-6-yl)methyl)piperazin-1-yl)-6-fluoro-*N*-methylpicolinamide**

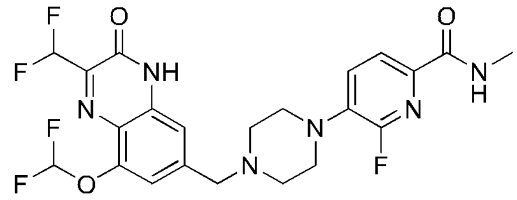

(134)

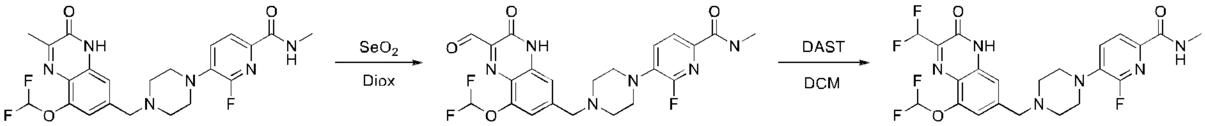


Step 1) Synthesis of 5-(4-((8-(difluoromethoxy)-2-methyl-3-oxo-3,4-dihydroquinoxalin-6-yl)methyl)piperazin-1-yl)-6-fluoro-*N*-methylpicolinamide

At room temperature, selenium dioxide (0.033 g, 0.30 mmol) was added to 1,4-dioxane solution (5 mL) of 5-(4-((8-(difluoromethoxy)-2-methyl-3-oxo-3,4-dihydroquinoxalin-6-yl)methyl)piperazin-1-yl)-6-fluoro-*N*-methylpyridine-2-carboxamide (94 mg, 0.20 mmol), and then the reaction was heated to 80 °C for 2 h. TLC results showed that the starting material was remaining, so selenium dioxide (0.011 mg, 0.10 mmol) was added, and the reaction was heated to 100 °C for 6 h. The reaction was stopped, the reaction solution was concentrated under reduced pressure, and the residue was purified by silica gel column chromatography (eluent: DCM/MeOH (v/v) = 20/1) to give a yellow solid product (0.06 g, 62.01%).
MS (ESI, pos. ion) *m/z:* 490.16 $[M+H]^+$.

Step 2) Synthesis of 5-(4-((8-(difluoromethoxy)-2-(difluoromethyl)-3-oxo-3,4-dihydroquinoxalin-6-yl)methyl)piperazin-1-yl)-6-fluoro-*N*-methylpicolinamide

At 0 °C, diethylaminosulfonate trifluoride (0.058 g, 0.36 mmol) was added dropwise to a solution of 5-(4-((8-(difluoromethoxy)-2-formyl-3-oxo-3,4-dihydroquinoxalin-6-yl)methyl)piperazin-1-yl)-6-fluoro-*N*-methylpyridine-2-carboxamide (60 mg, 0.12 mmol) in dichloromethane (5 mL). The reaction was then brought to room temperature and allowed to proceed for 8 h. The reaction was stopped, and the reaction mixture was quenched with water and saturated sodium bicarbonate solution until no more bubbles were generated. DCM was then added for extraction (15 mL $\times$ 3). The organic phase was dried over anhydrous sodium sulfate, filtered, concentrated under reduced pressure, and the residue was purified by silica gel column chromatography (DCM/MeOH (v/v) = 20/1) to give a pale yellow solid (9 mg, 14.36%).
MS (ESI, pos. ion) *m/z:* 513.16 $[M+H]^+$.
$^1H$ NMR (400 MHz, DMSO-$d_6$) $\delta$ 12.96 (s, 1H), 8.46 - 8.36 (m, 1H), 7.90 - 7.81 (m, 1H), 7.64 - 7.56 (m, 1H), 7.42 (t, *J* = 74 Hz, 1H), 7.25 - 7.15 (m, 3H), 7.04 (t, *J* = 53.2 Hz, 1H), 3.67 (s, 2H), 3.24-3.17 (m, 4H), 2.77 (d, *J* = 4.8 Hz, 3H), 2.63 - 2.57 (m, 4H).

**Example 57 5-(4-((8-(difluoromethoxy)-2-methyl-3-oxo-3,4-dihydroquinoxalin-6-yl)methyl)piperazin-1-yl)-6-fluoro-*N*-((1*S*,2*R*)-2-fluorocyclopropyl)picolinamide**

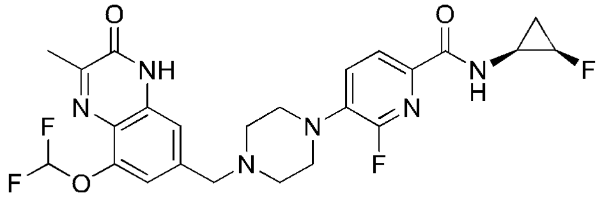

(135)

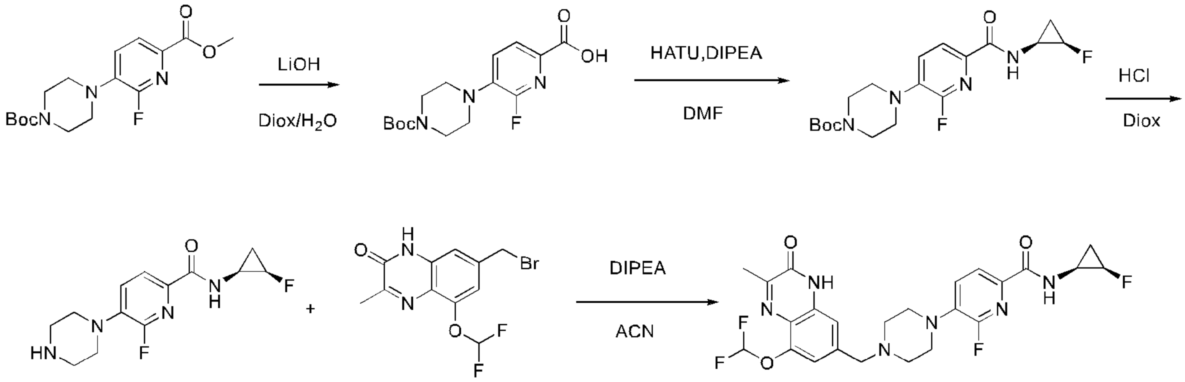


Step 1) Synthesis of 5-(4-(*tert*-butyloxycarbonyl)piperazin-1-yl)-6-fluoropyridinecarboxylic acid

At room temperature, lithium hydroxide monohydrate (1.5 g, 35.75 mmol) was added to a solution of 4-(2-fluoro-6-(methoxycarbonyl)pyridin-3-yl)piperazin-1-carboxylic acid *tert*-butyl ester (4.5 g, 13.26 mmol) in 1,4-dioxane (50 mL) and water (25 mL). The reaction was then heated to 50 °C and carried out overnight. The reaction was stopped, and the pH of the reaction solution was adjusted to 5-6 with dilute hydrochloric acid. The reaction solution was diluted with DCM (15 mL), washed with water (15 mL × 3), dried over a vacuum, concentrated, and the residue was purified by silica gel column chromatography (DCM/MeOH (v/v) = 20/1) to give a pale yellow solid product (4 g, yield 92.72%).
MS (ESI, pos. ion) *m*/*z:* 326.15 $[M+H]^+$.

Step 2) Synthesis of *tert*-butyl 4-(2-fluoro-6-(((*1S*,*2R*)-2-fluorocyclopropyl)carbamoyl)pyridin-3-yl)piperazine-1-carboxylic acid

At room temperature, (*1R*,*2S*)-2-fluorocyclopropane-1-amine (1.62 g, 21.52 mmol), 2-(7-aza-1*H*-benzotriazol-1-yl)-1,1,3,3-tetramethylbromohexafluorophosphate (6.14 g, 16.14 mmol), and ethyl diisopropylamine (4.17 g, 32.28 mmol) was added to a solution of 5-(4-(*tert*-butoxycarbonyl)piperazine-1-yl)-6-fluoropyridinecarboxylic acid (3.5 g, 10.76 mmol) in *N,N*-dimethylformamide (70 mL) and reacted overnight at room temperature. The reaction was stopped, and water (60 mL) was added to the reaction solution. The mixture was extracted with EtOAc (60 mL × 3), washed with saturated brine (60 mL × 3), concentrated under reduced pressure, and the residue was purified by silica gel column chromatography (PE/EtOAc(v/v) = 1/1) to give a light yellow solid product (4 g, yield 97.23%).
MS (ESI, pos. ion) *m*/*z:* 383.20 $[M+H]^+$.

Step 3) Synthesis of 6-fluoro-*N*-((*1S*,*2R*)-2-fluorocyclopropyl)-5-(piperazin-1-yl)picolinamide

At room temperature, a solution of hydrochloric acid in dioxane (4.39 g, 120.3 mmol) was added to a solution of 4-(2-fluoro-6-(((*1S*, *2R*)-2-fluorocyclopropyl)carbamoyl)pyridin-3-yl)piperazin-1-carboxylic acid *tert*-butyl ester (4.6 g, 12.03 mmol) in ethyl acetate (30 mL). The reaction was allowed to proceed overnight at room temperature. The reaction solution was concentrated under reduced pressure and dried to give a pale yellow solid product (3 g, 88.35 %).
MS (ESI, pos. ion) *m*/*z:* 283.10 $[M+H]^+$.

Step 4) Synthesis of 5-(4-((8-(difluoromethoxy)-2-methyl-3-oxo-3,4-dihydroquinoxalin-6-yl)methyl)piperazin-1-yl)-6-fluoro-*N*-((*1S, 2R*)-2-fluorocyclopropyl)picolinamide

At room temperature, ethyl diisopropylamine (0.30 g, 2.34 mmol) and 6-fluoro-*N*-((*1S,2R*)-2-fluorocyclopropyl)-5-(piperazin-1-yl)picolinamide (0.33 g, 1.17 mmol) were added to a solution of 7-(bromomethyl)-5-(difluoromethoxy)-3-methyl-1,2-dihydroquinoxalin-2-one (0.25 g, 0.78 mmol) in acetonitrile (20 mL), and the mixture was then heated to 80 °C and reacted for 40 min. The reaction was stopped, the reaction solution was concentrated under reduced pressure, and the residue was dissolved in dichloromethane (30 mL). The solution was washed with water (50 mL × 3), the organic phase was dried over anhydrous sodium sulfate, the organic phase was concentrated under reduced pressure, and the residue was purified by silica gel column chromatography (DCM/MeOH (v/v) = 25/1) to give a pale yellow solid product (0.259 g, yield 63.52%).

MS (ESI, pos. ion) *m/z:* 521.20 $[M+H]^+$.

$^1H$ NMR (400 MHz, DMSO-$d_6$) δ 12.42 (s, 1H), 8.36 (d, *J* = 4.4 Hz, 1H), 7.87 (dd, *J* = 8.0, 1.4 Hz, 1H), 7.59 (dd, *J*= 10.6, 8.2 Hz, 1H),7.36 (t, *J* = 74.4 Hz, 1H), 7.16 - 7.04 (m, 2H), 4.87 - 4.63 (m, 1H), 3.61 (s, 2H), 3.24 - 3.14 (m, 4H), 2.88 - 2.77 (m, 1H), 2.63 - 2.54 (m, 4H), 2.41 (s, 3H), 1.39 - 1.24 (m, 1H), 1.15 - 1.01 (m, 1H).

**Example 58 1'-((8-(difluoromethoxy)-2-methyl-3-oxo-3,4-dihydroquinoxalin-6-yl)methyl)-*N*,2-dimethyl-1',2',3',6'-tetrahydro-[3,4'-bipyridine]-6-carboxamide**

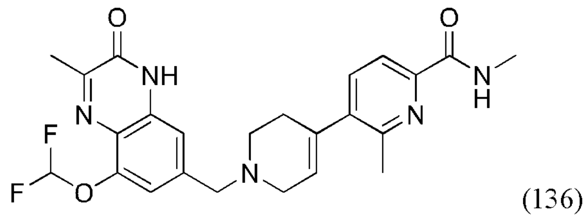

(136)

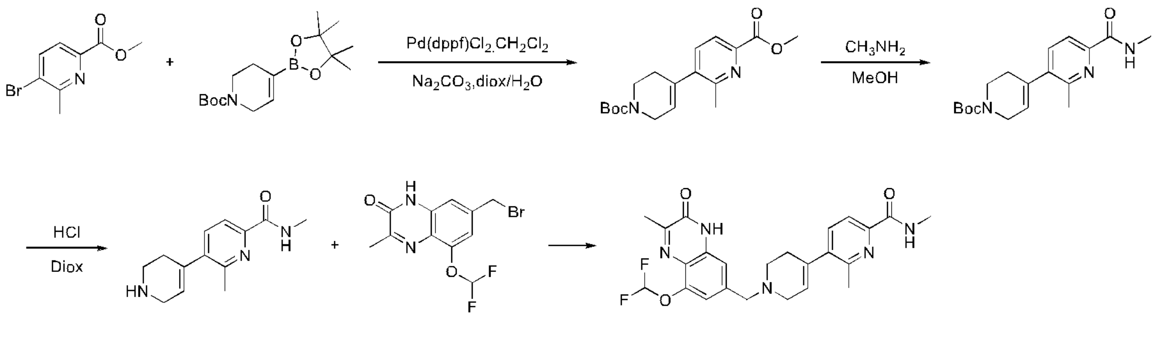


Step 1) Synthesis of 1'-(*tert*-butyl)-6-methyl-2-methyl-3',6'-dihydro-[3,4'-bipyridine]-1',6(2'*H*)-dicarboxylate

At room temperature, sodium carbonate (6.91 g, 65.19 mmol) and a complex of [1,1'-bis(diphenylphosphino) ferrocene]dichloropalladium(II) with dichloromethane (1.24 g, 1.52 mmol) were added to a solution of methyl 5-bromo-6-methylpyridinecarboxylate (5 g, 21.73 mmol), 4-(4,4,5,5-tetramethyl-1,3,2-dioxaborhexacyclopentan-2-yl)-3,6-dihydropyridine-1(2*H*)-carboxylate (7.39 g, 23.90 mmol) in 1,4-dioxane (100 mL), and water (10 mL). The atmosphere was replaced with nitrogen and the temperature was raised to 90 °C. The reaction was allowed to proceed for 10 h, then cooled to room temperature. The mixture was filtered through diatomaceous earth, the organic phase was concentrated under reduced pressure, and the residue was purified by silica gel column chromatography (PE/EtOAc(v/v) = 6/1) to give a yellow oil (5.5 g, yield 76.14%).

MS (ESI, pos. ion) *m/z:* 333.20 $[M+H]^+$.

Step 2) Synthesis of *tert*-butyl 2-methyl-6-(methylcarbamoyl)-3',6'-dihydro-[3,4'-bipyridine]-1'(2'*H*)-carboxylic acid

At room temperature, a methanol solution of methylamine (5.14 g, 165.5 mmol) was added to 1'-(*tert*-butyl)-6-methyl-2-methyl-3',6'-dihydro-[3,4'-bipyridine]-1',6(2'*H*)-dicarboxylic acid salt (5.5 g, 16.55 mmol), and the reaction was then heated to 50 °C for 2.5 h. TLC showed complete reaction of the starting material. The reaction was stopped, the

reaction solution was concentrated under reduced pressure, and the residue was purified by silica gel column chromatography (PE/EtOAc (v/v) = 20/1) to give a pale yellow solid (4.5 g, yield 82.06%).

MS (ESI, pos. ion) *m/z:* 332.20 $[M+H]^+$.

Step 3) Synthesis of *N*,2-dimethyl-1',2',3',6'-tetrahydro-[3,4'-bipyridine]-6-carboxamide

At room temperature, a solution of hydrochloric acid in dioxane (4.95 g, 135.8 mmol) was added to a solution of *tert*-butyl 2-methyl-6-(methylcarbamoyl)-3',6'-dihydro-[3,4'-bipyridine]-1'(2*H*)-carboxylic acid (4.5 g, 13.58 mmol)) in ethyl acetate (20 mL), and the reaction was allowed to proceed overnight at room temperature. After the reaction was complete, the solution was concentrated under reduced pressure, filtered, and dried to give a pale yellow solid (2.6 g, yield 82.79%).

MS (ESI, pos. ion) *m/z:* 232.20 $[M+H]^+$.

Step 4) Synthesis of 1'-((8-(difluoromethoxy)-2-methyl-3-oxo-3,4-dihydroquinoxalin-6-yl)methyl)-*N*,2-dimethyl-1',2',3',6'-tetrahydro-[3,4'-bipyridine]-6-carboxamide

At room temperature, *N,N*-diisopropylethylamine (0.24 g, 1.89 mmol) and *N*,2-dimethyl-1',2',3',6'-tetrahydro-[3,4'-bipyridine]-6-carboxamide (0.22 g, 0.95 mmol) were added to a solution of 7-(bromomethyl)-5-(difluoromethoxy)-3-methylquinoxalin-2(1*H*)-one (0.2 g, 0.63 mmol) in acetonitrile (10 mL), and then the temperature was raised to 80 °C and the reaction was carried out for 2 h. After the reaction was completed, the reaction solution was cooled to room temperature, concentrated under reduced pressure, and the residue was dissolved in dichloromethane (30 mL). The solution was washed with water (15 mL × 3), dried over anhydrous sodium sulfate, concentrated under reduced pressure, and purified by silica gel column chromatography (DCM/MeOH (v/v) = 24/1) to give a white solid (0.13 g, yield 44.18%).

MS (ESI, pos. ion) *m/z:* 470.20 $[M+H]^+$.

$^1$H NMR (400 MHz, DMSO-$d_6$) δ 12.42 (s, 1H), 8.60 - 8.53 (m, 1H), 7.80 (d, *J*= 7.8 Hz, 1H), 7.66 (d, *J* = 7.8 Hz, 1H), 7.34(t, *J* = 74.4 Hz, 1H), 7.19 - 7.06 (m, 2H), 3.69 (s, 2H), 3.30 - 3.29 (m, 1H), 3.15 - 3.10 (m, 2H), 2.82 (d, *J*= 4.9 Hz, 3H), 2.71 - 2.66 (m, 2H), 2.53 (s, 3H), 2.41 (s, 3H), 2.39-2.33 (m, 2H).

**Example 59 (*R*)-3-((8-(difluoromethoxy)-2-methyl-3-oxo-3,4-dihydroquinoxalin-6-yl)methyl)-*N*-methyl-2,3,4,4a,5,7-hexahydro-1*H*-pyrazino[2,1-c]pyrido[3,2-*e*][1,4]oxazepine-9-carboxamide**

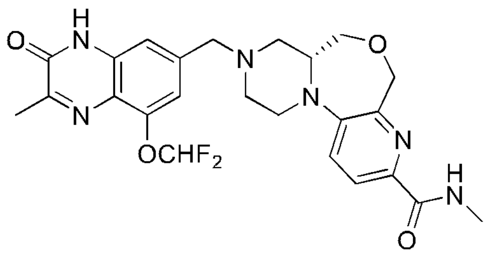

(137)

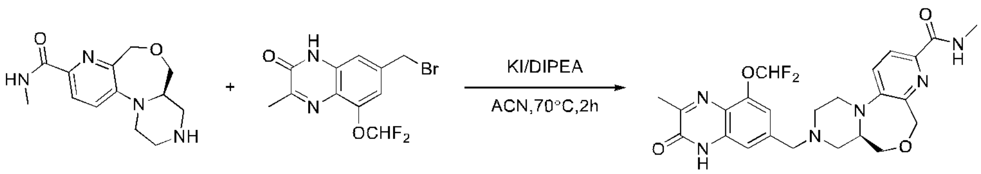


7-(bromomethyl)-5-(difluoromethoxy)-3-methylquinoxalin-2(1*H*)-one (0.15 g, 0.48 mmol), (*R*)-*N*-methyl-2,3,4,4a,5,7-hexahydro-1*H*-pyrazino[2,1-*c*]pyridino[3,2-*e*][1,4]oxazazepone-9-carboxamide (0.13 g, 0.48 mmol), potassium iodide (0.078 g, 0.47 mmol), acetonitrile (10 mL), and *N,N*-diisopropylethylamine (0.30 g, 2.35 mmol) were added to a reaction flask, heated to 70 °C, and reacted for 1.5 hours. The reaction was then stopped, concentrated under reduced pressure, and thin-layer silica gel plates were prepared (DCM:MeOH (v/v) = 20/1) to obtain a pink solid (23 mg, 10%).

MS (ESI, pos. ion) m/z: 501.2 $[M+H]^+$;

1H NMR (400 MHz, DMSO-$d_6$) δ 12.44 (s, 1H), 8.40 (q, J = 4.2 Hz, 1H), 7.81 (d, J = 8.4 Hz, 1H), 7.51 (d, J = 8.5 Hz, 1H), 7.36 (t, J = 14.1 Hz, 1H), 7.10 (d, J = 41.4 Hz, 2H), 4.96 (d, J = 14.1 Hz, 1H), 4.73 (d, J = 14.1 Hz, 1H), 4.09 (dd, J = 12.2, 7.9 Hz, 1H), 3.79 (dd, J = 12.4, 3.8 Hz, 1H), 3.66 - 3.52 (m, 4H), 2.78 (d, J = 4.8 Hz, 3H), 2.68 - 2.52 (m, 3H), 2.44 - 2.36 (m, 4H).

**Example 60 5-(4-((8-(difluoromethoxy)-5-fluoro-2-methyl-3-oxo-3,4-dihydroquinoxalin-6-yl)methyl)piperazin-1-yl)-*N*,6-dimethylpicolinamide**

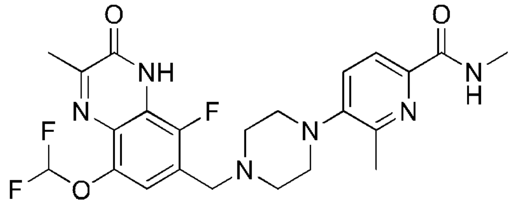

(138)

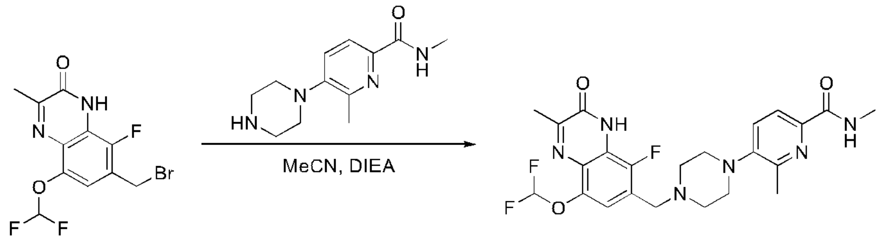


At room temperature, *N*,6-dimethyl-5-(piperazin-1-yl)picolinamide (0.088 g, 0.38 mmol), DIPEA (0.097 g, 0.75 mmol) and KI (0.0021 g, 0.013 mmol) were added to a solution of 7-(bromomethyl)-5-(difluoromethoxy)-8-fluoro-3-methylquinoxalin-2(1*H*)-one (0.084 g, 0.25 mmol) in ACN (20 mL), followed by reaction at 80 °C for 3 h. The reaction solution was concentrated under reduced pressure, and the residue was purified by silica gel column chromatography (eluent: $CH_2Cl_2$/MeOH (v/v) = 20/1) to give a yellow solid product (0.12 g, 98.18%).

MS (ESI, pos. ion) *m*/*z:* 491.2 $[M+H]^+$;

$^1$H NMR (400 MHz, DMSO-$d_6$) δ (ppm) 12.61 (s, 1H), 8.44 - 8.38 (m, 1H), 7.78 (d, *J*= 8.2 Hz, 1H), 7.47 (d, *J*= 8.4 Hz, 1H), 7.29 (t, *J* = 74.4 Hz, 1H), 7.14 (d, *J* = 5.4 Hz, 1H), 3.71 (s, 2H), 2.98 - 2.92 (m, 4H), 2.79 (d, *J* = 4.9 Hz, 3H), 2.62 (s, 3H), 2.49 - 2.46 (m, 4H), 2.43 (s, 3H).

**Example 61 *N*-cyclopropyl-5-(4-((8-(difluoromethoxy)-5-fluoro-2-methyl-3-oxo-3,4-dihydroquinoxalin-6-yl) methyl)piperazin-1-yl)-6-fluoropicolinamide**

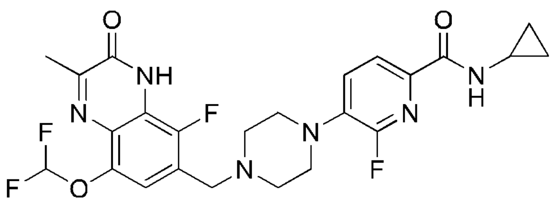

(139)

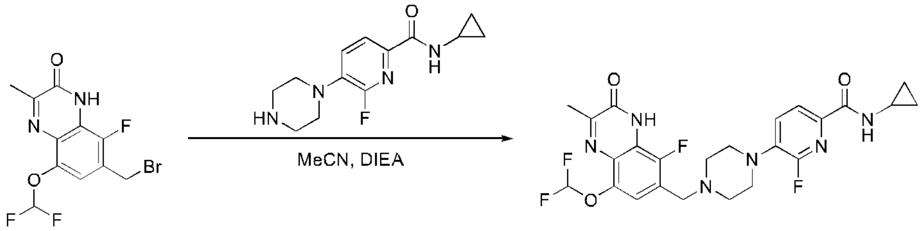


At room temperature, *N*-cyclopropyl-6-fluoro-5-(piperazin-1-yl)picolinamide (0.099 g, 0.38 mmol), DIPEA (0.097 g, 0.75 mmol) and KI (0.0021 g, 0.013 mmol) were added to a solution of 7-(bromomethyl)-5-(difluoromethoxy)-8-fluoro-3-methylquinoxalin-2(1*H*)-one (0.085 g, 0.25 mmol) in ACN (20 mL), followed by reaction at 80 °C for 3 h. The reaction solution was concentrated under reduced pressure, and the residue was purified by silica gel column chromatography

(eluent: $CH_2Cl_2$/MeOH (v/v) = 20/1) to give a yellow solid product (0.11 g, 83.81%).

MS (ESI, pos. ion) *m/z:* 521.2 [M+H]$^+$;

$^1$H NMR (400 MHz, DMSO-$d_6$) δ (ppm) 12.62 (s, 1H), 8.34 (d, *J* = 5.0 Hz, 1H), 7.83 (d, *J*= 8.1 Hz, 1H), 7.55 (t, *J* = 9.3 Hz, 1H), 7.28 (t, *J* = 74.5 Hz, 1H), 7.14 - 7.08 (m, 1H), 3.69 (s, 2H), 3.22 - 3.09 (m, 4H), 2.89 - 2.79 (m, 1H), 2.66 - 2.55 (m, 4H), 2.43 (s, 3H), 0.71 - 0.56 (m, 4H).

**Example 62 1'-((8-(difluoromethoxy)-5-fluoro-2-methyl-3-oxo-3,4-dihydroquinoxalin-6-yl)methyl)-*N*,2-dimethyl-1',2',3',6'-tetrahydro-[3,4'-bipyridine]-6-carboxamide**

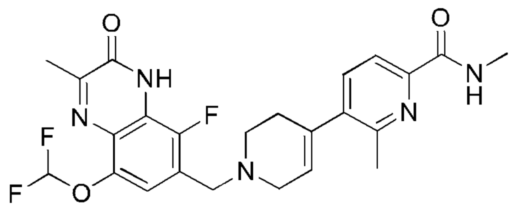

(140)

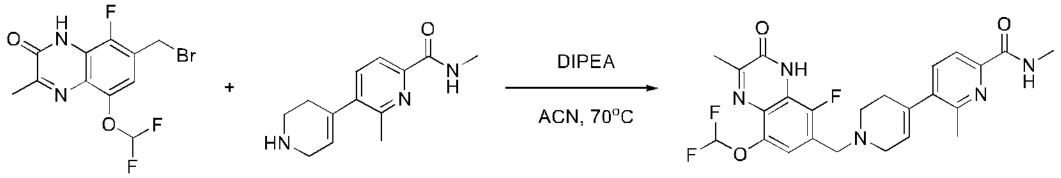


7-(bromomethyl)-5-(difluoromethoxy)-8-fluoro-3-methylquinoxalin-2(1H)-one (80 mg, 0.24 mmol), N,2-dimethyl-1',2',3',6'-tetrahydro-[3,4'-bipyridine]-6-carboxamide (83 mg, 0.36 mmol) and DIPEA (124 mg, 0.96 mmol) were added sequentially to ACN (8 mL), and the reaction was carried out at 70 °C for 3 h. The reaction solution was concentrated under reduced pressure, diluted with DCM (50 mL), washed with water (30 mL) and saturated NaCl solution (30 mL), dried over anhydrous $Na_2SO_4$, concentrated under reduced pressure, and the residue was purified by silica gel column chromatography (DCM/MeOH (v/v) = 20/1) to give a pale white solid (95 mg, 82%).

MS (ESI, pos. ion) *m/z:* 488.2 [M+H]$^+$;

$^1$H NMR (400 MHz, DMSO-$d_6$) δ 12.63 (s, 1H), 8.57 (q, *J* = 4.7 Hz, 1H), 7.80 (d, *J* = 7.8 Hz, 1H), 7.66 (d, *J* = 7.9 Hz, 1H), 7.31 (t, *J* = 74.4 Hz, 1H), 7.17 (d, *J* = 5.5 Hz, 1H), 5.71 (s, 1H), 3.78 (s, 2H), 3.16 (d, *J* = 2.6 Hz, 2H), 2.82 (d, *J*= 4.9 Hz, 3H), 2.72 (t, *J*= 5.5 Hz, 2H), 2.52 (s, 3H), 2.45 (s, 3H), 2.36 (s, 2H).

**Example 63 (*R*)-*N*-methyl-3-((2-methyl-3-oxo-8-(prop-1-yn-1-yl)-3,4-dihydroquinoxalin-6-yl)methyl)-2,3,4,4a,5,7-hexahydro-1H-pyrazino[2,1-*c*]pyrido[3,2-*e*][1,4]oxazepine-9-carboxamide**

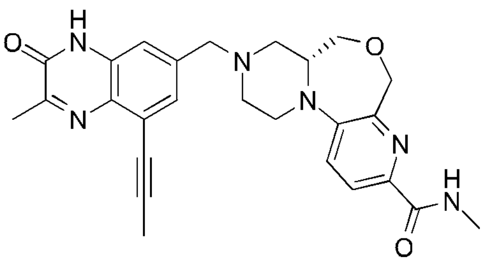

(141)

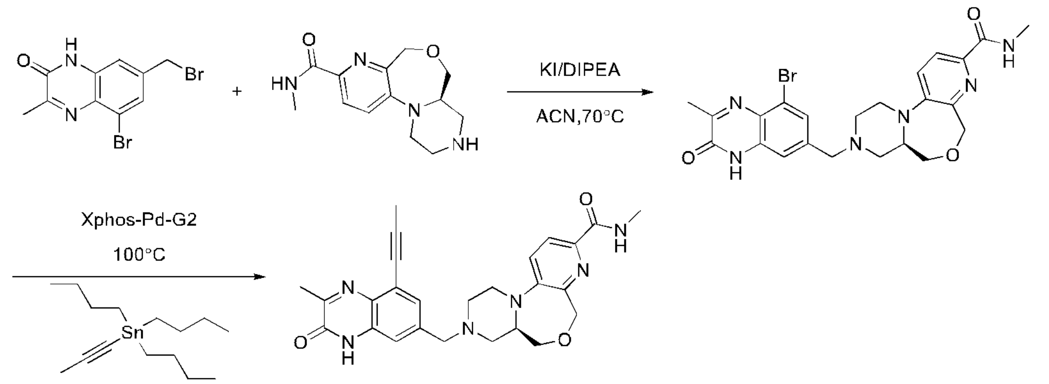


Step 1) Synthesis of (*R*)-3-((8-bromo-2-methyl-3-oxo-3,4-dihydroquinoxalin-6-yl)methyl)-*N*-methyl-2,3,4,4a,5,7-hexahydro-1*H*-pyrazino[2,1-*c*]pyridino[3,2-*e*][1,4]oxazapine-9-carboxamide

5-bromo-7-(bromomethyl)-3-methylquinoxalin-2(1*H*)-one (0.08 g, 0.24 mmol), (*R*)-*N*-methyl-2,3,4,4a,5,7-hexahydro-1H-pyrazino[2,1-*c*]pyridino[3,2-*e*][1,4]oxazapine-9-carboxamide (0.063 g, 0.24 mmol), potassium iodide (0.060 g, 0.36 mmol) 1 mmol), *N,N*-diisopropylethylamine (0.16 g, 1.2 mmol), and acetonitrile (5 mL) were added sequentially to the reaction flask, heated to 70 °C, reacted for 3 h, concentrated under reduced pressure, and the residue was purified by silica gel column chromatography (DCM/MeOH(v/v)=20/1) to give a yellow solid (72 mg, 58%).
MS (ESI, pos. ion) m/z: 513.2 $[M+H]^+$.

Step 2) Synthesis of (*R*)-*N*-methyl-3-((2-methyl-3-oxo-8-(prop-1-yn-1-yl)-3,4-dihydroquinoxalin-6-yl)methyl)-2,3,4,4a,5,7-hexahydro-1*H*-pyrazino[2,1-*c*]pyrido[3,2-*e*][1,4]oxazepine-9-carboxamide

(*R*)-3-((8-bromo-2-methyl-3-oxo-3,4-dihydroquinoxalin-6-yl)methyl)-*N*-methyl-2,3,4,4a,5,7-hexahydro-1*H*-pyrazino[2,1-*c*]pyridino[3,2-*e*][1,4]oxazazepone-9-carboxamide (0.07 g, 0.14 mmol) was dissolved in 1,4-dioxane (5 mL), and tributyl(prop-1-yn-1-yl)stannane (0.14 g, 0.42 mmol) and chloro(2-dicyclohexylphosphino-2',4',6'-triisopropyl-1,1'-biphenyl)[2-(2'-amino-1,1'-biphenyl)]palladium(II) (0.022 g, 0.028 mmol) were added. The mixture was heated to 100 °C under nitrogen protection and reacted for 14 hours. The mixture was concentrated under reduced pressure and the residue was purified by thin-layer silica gel slurry (DCM/MeOH (v/v) = 20/1) to give a yellow solid (14 mg, 21.7%).
MS (ESI, pos. ion) m/z: 473.2 $[M+H]^+$;
$^1H$ NMR (599 MHz, DMSO-$d_6$) δ 12.33 (s, 1H), 8.40 (q, J = 4.8 Hz, 1H), 7.81 (d, J = 8.5 Hz, 1H), 7.50 (d, J = 8.5 Hz, 1H), 7.25 (dd, J = 35.4, 1.7 Hz, 2H), 4.94 (d, J = 14.0 Hz, 1H), 4.73 (d, J = 14.0 Hz, 1H), 4.08 - 3.95 (m, 2H), 3.82 - 3.76 (m, 1H), 3.61 - 3.47 (m, 4H), 2.78 (d, J = 4.8 Hz, 3H), 2.64 - 2.54 (m, 3H), 2.41 (s, 3H), 2.40 - 2.36 (m, 1H), 2.13 (s, 3H).

**Example 64 *N*,6-dimethyl-5-(4-((2-methyl-3-oxo-8-(trifluoromethoxy)-3,4-dihydroquinoxalin-6-yl)methyl)piperazin-1-yl)picolinamide**

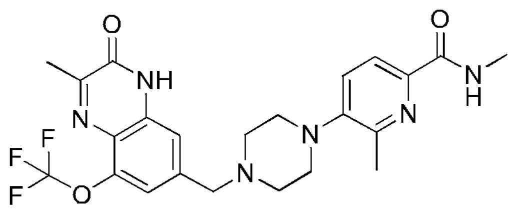

(142)

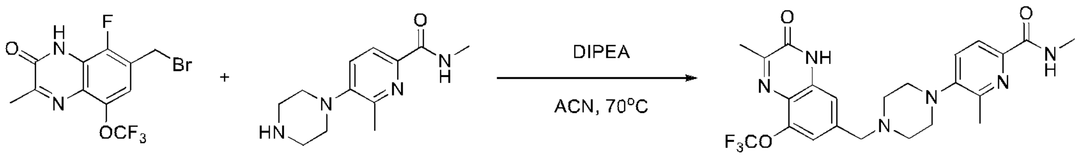


7-(bromomethyl)-8-fluoro-3-methyl-5-(trifluoromethoxy)quinoxalin-2(1H)-one (90 mg, 0.27 mmol), N,6-dimethyl-5-(piperazin-1-yl)picolinamide (95 mg, 0.41 mmol), and DIPEA (105 mg, 0.81 mmol) were added sequentially to ACN (6 mL), and the reaction was carried out at 70 °C for 3 h. The reaction solution was concentrated under reduced pressure, diluted with DCM (50 mL), washed with water (30 mL) and saturated NaCl solution (30 mL), dried over anhydrous $Na_2SO_4$, concentrated under reduced pressure, and the residue was purified by silica gel column chromatography (DCM/MeOH (v/v) = 20/1) to give a pale white solid (105 mg, 80%).

MS (ESI, pos. ion) *m/z:* 491.2 $[M+H]^+$;

$^1H$ NMR (600 MHz, DMSO-$d_6$) $\delta$ 12.51 (s, 1H), 8.42 (q, *J* = 4.6 Hz, 1H), 7.79 (d, *J*= 8.2 Hz, 1H), 7.49 (d, *J* = 8.3 Hz, 1H), 7.28 (s, 1H), 7.26 (s, 1H), 3.67 (s, 2H), 2.96 (s, 4H), 2.80 (d, *J* = 4.9 Hz, 3H), 2.59 (s, 4H), 2.48 (s, 3H), 2.42 (s, 3H).

**Example 65 5-(4-((8-(difluoromethoxy)-2-methyl-3-oxo-3,4-dihydroquinoxalin-6-yl)methyl)piperazin-1-yl)-*N*-ethoxy-6-fluoropicolinamide**

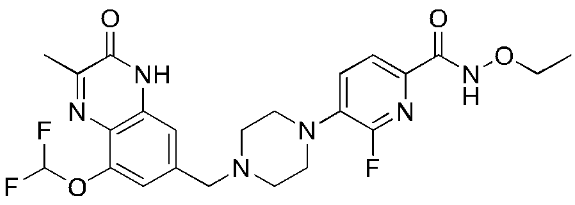

(143)

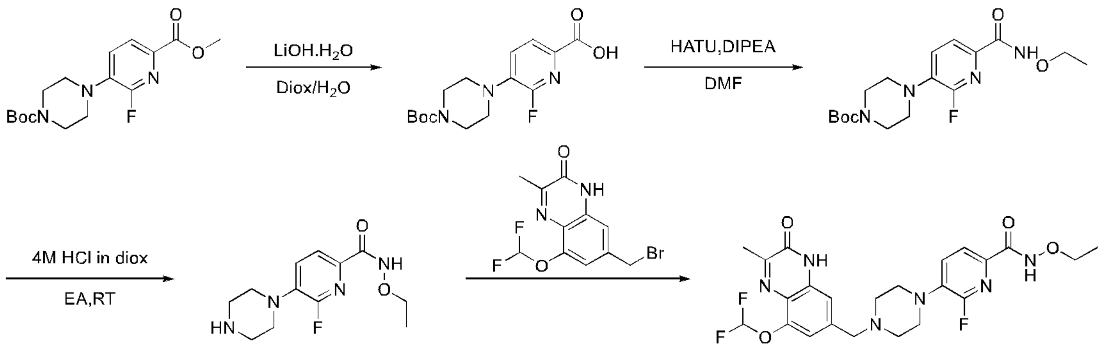


Step 1) Synthesis of 5-(4-(*tert*-butyloxycarbonyl)piperazin-1-yl)-6-fluoropyridinecarboxylic acid

At room temperature, lithium hydroxide monohydrate (1.5 g, 35.75 mmol) was added to a solution of 4-(2-fluoro-6-(methoxycarbonyl)pyridin-3-yl)piperazine-1-carboxylic acid *tert*-butyl ester (4.5 g, 13.26 mmol) in 1,4-dioxane (50 mL) and water (25 mL), followed by reaction at 50 °C overnight. The reaction was stopped, the pH of the reaction solution was adjusted to 5-6 with dilute hydrochloric acid, and the solution was diluted with dichloromethane (80 mL). The solution was washed with water (40 mL × 3), the organic phase was dried, concentrated under reduced pressure, and the residue was purified by silica gel column chromatography (DCM/MeOH (v/v) = 20/1) to give a pale yellow solid product (4.2 g, yield 97.36%).

MS (ESI, pos. ion) *m/z:* 326.15 $[M+H]^+$.

Step 2) Synthesis of 4-(6-(ethoxycarbamoyl)-2-fluoropyridin-3-yl)piperazine-1-carboxylic acid *tert*-buty*l* ester

At room temperature, HATU (2.10 g, 5.54 mmol), ethyl diisopropylamine (1.43 g, 11.07 mmol), and O-ethylhydroxylamine (0.45 g, 7.38 mmol) were added to a solution of 5-(4-(*tert*-butyloxycarbonyl)piperazine-1-yl)-6-fluoropyridinic acid (1.2 g, 3.69 mmol) in *N,N*-dimethylformamide (15 mL), and the reaction was carried out overnight at room temperature. The reaction mixture was cooled to room temperature, and water (60 mL) was added to the reaction solution. The mixture was extracted with ethyl acetate (60 mL × 3), the organic phase was washed with brine (50 mL × 3), dried over anhydrous sodium sulfate, concentrated under reduced pressure, and purified by silica gel column chromatography (petroleum ether/ethyl acetate (v/v) = 10/3) to give a pale yellow solid product (1.3 g, yield 95.67%).

MS (ESI, pos. ion) *m/z:* 369.20 $[M+H]^+$.

Step 3) Synthesis of *N*-ethoxy-6-fluoro-5-(piperazin-1-yl)picolinamide

At room temperature, hydrochloric acid (1.48 g, 40.7 mmol) was added to a solution of 4-(6-(ethoxycarbamoyl)-2-fluoropyridin-3-yl)piperazin-1-carboxylic acid *tert*-butyl ester (1.5 g, 4.07 mmol) in ethyl acetate (10 mL), and the reaction was carried out at room temperature for 8 h. The reaction solution was filtered, washed with ethyl acetate (50 mL), and the resulting filter cake was dried under vacuum to give a pale yellow product (1 g, yield 91.54%).
MS (ESI, pos. ion) m/z: 269.15 $[M+H]^+$.

Step 4) Synthesis of 5-(4-((8-(difluoromethoxy)-2-methyl-3-oxo-3,4-dihydroquinoxalin-6-yl)methyl)piperazin-1-yl)-*N*-ethoxy-6-fluoropicolinamide

At room temperature, ethyl diisopropylamine (0.36 g, 2.82 mmol) and *N*-ethoxy-6-fluoro-5-(piperazin-1-yl) picolinamide (0.38 g, 1.41 mmol) were added to a solution of 7-(bromomethyl)-5-(difluoromethoxy)-3-methylquinoxalin-2(1*H*)-one (0.3 g, 0.94 mmol) in acetonitrile (10 mL), and the mixture was then heated to 80 °C for 2 h. The reaction was stopped, the reaction solution was cooled to room temperature, concentrated under reduced pressure, and the residue was dissolved in DCM (50 mL). The solution was washed with water (15 mL × 2), the organic phase was dried over anhydrous sodium sulfate, concentrated under reduced pressure, and the residue was purified by silica gel column chromatography (DCM/MeOH (v/v) = 25/1) to give a white solid product (0.2 g, yield 42.00%).
MS (ESI, pos. ion) *m/z:* 507.20 $[M+H]^+$.
$^1H$ NMR (599 MHz, DMSO-$d_6$) δ 12.43 (s, 1H), 11.63 (s, 1H), 7.82 (dd, *J* = 8.0, 1.3 Hz, 1H), 7.57 (dd, *J* = 10.6, 8.2 Hz, 1H), 7.36 (t, *J* = 74.4 Hz, 1H), 7.10 (dd, *J* = 48.3, 1.6 Hz, 2H), 3.89 (q, *J* = 7.0 Hz, 2H), 3.61 (s, 2H), 3.22 - 3.15 (m, 4H), 2.60 - 2.54 (m, 4H), 2.41 (s, 3H), 1.17 (t, *J* = 7.1 Hz, 3H).

**Example 66 5-(4-((8-(difluoromethoxy)-2-methyl-3-oxo-3,4-dihydroquinoxalin-6-yl)methyl)piperazin-1-yl)-*N'*-ethyl-6-fluoropicolinohydrazide**

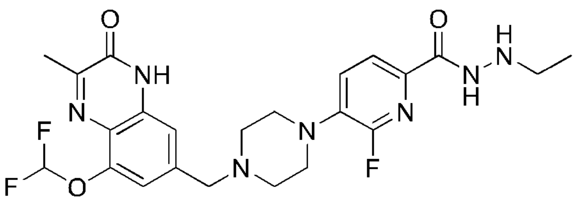

(144)

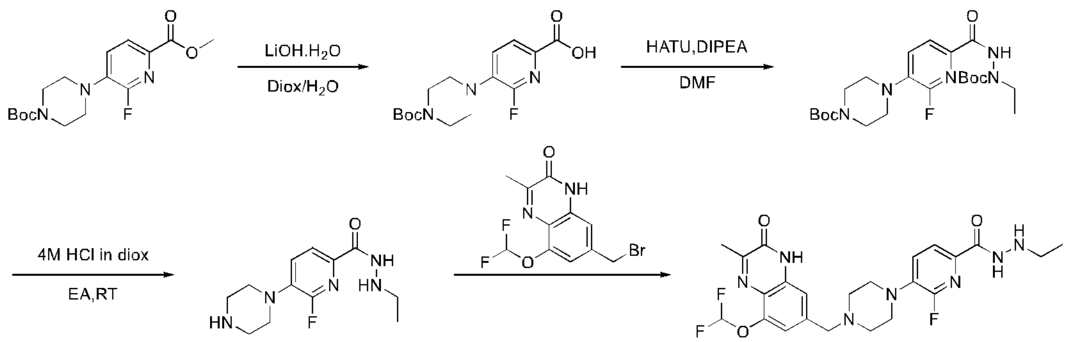


Step 1) Synthesis of 5-(4-(*tert*-butyloxycarbonyl)piperazin-1-yl)-6-fluoropyridinecarboxylic acid

At room temperature, lithium hydroxide monohydrate (1.5 g, 35.75 mmol) was added to a solution of 4-(2-fluoro-6-(methoxycarbonyl)pyridin-3-yl)piperazine-1-carboxylic acid *tert*-butyl ester (4.5 g, 13.26 mmol) in 1,4-dioxane (50 mL) and water (25 mL), followed by reaction at 50 °C overnight. The reaction was stopped, the pH of the reaction solution was adjusted to 5-6 with dilute hydrochloric acid, dilute the reaction solution with dichloromethane (100 mL), washed with water (50 mL × 3), the organic phase was dried, concentrated under reduced pressure, and the residue was purified by silica gel column chromatography (DCM/MeOH(v/v) = 20/1) to give a light yellow solid product (4.2 g, yield 97.36%).
MS (ESI, pos. ion) m/z: 326.15 $[M+H]^+$.

Step 2) Synthesis of *tert*-butyl 4-(6-(2-(*tert*-butoxycarbonyl)-2-ethylhydrazine-1-carbonyl)-2-fluoropyridin-3-yl)piperazine-1-carboxylic acid

At room temperature, HATU (2.10 g, 5.54 mmol), ethyl diisopropylamine (1.43 g, 11.07 mmol) and *N*-ethyl(tert-butoxy)carbazine (1.18 g, 7.38 mmol) were added to *N,N*-dimethylformamide (5 mL) containing 5-(4-(tert-butoxycarbonyl) piperazine-1-yl)-6-fluoropyridinic acid (1.2 g, 3.69 mmol) and reacted overnight at room temperature. The reaction was cooled to room temperature, and water (60 mL) was added to the reaction solution. EtOAc was extracted (20 mL × 3), and the organic phase was washed with brine (20 mL × 3). The organic phase was dried over anhydrous sodium sulfate, concentrated under reduced pressure, and the residue was purified by silica gel column chromatography (PE/EtOAc(v/v) = 10/3) to give a pale yellow solid product (1.5 g, yield 86.98%).
MS (ESI, pos. ion) *m/z:* 468.20 $[M+H]^+$.

Step 3) Synthesis of *N'*-ethyl-6-fluoro-5-(piperazin-1-yl)pyridinylhydrazide

At room temperature, hydrochloric acid (1.33 g, 36.4 mmol) was added to a solution of 4-(6-(2-(*tert*-butoxycarbonyl)-2-ethylhydrazine-1-carbonyl)-2-fluoropyridin-3-yl)piperazin-1-carboxylic acid *tert*-butyl ester (1.7 g, 3.64 mmol) in ethyl acetate (10 mL), and the reaction was carried out at room temperature for 9 h. The reaction solution was filtered, washed with ethyl acetate (50 mL), and the resulting filter cake was dried under vacuum to give a pale yellow solid product (0.9 g, yield 92.60%).
MS (ESI, pos. ion) *m/z:* 268.20 $[M+H]^+$.

Step 4) Synthesis of 5-(4-((8-(difluoromethoxy)-2-methyl-3-oxo-3,4-dihydroquinoxalin-6-yl)methyl)piperazin-1-yl)-*N*'-ethyl-6-fluoropicolinohydrazide

At room temperature, ethyl diisopropylamine (0.36 g, 2.82 mmol) and *N*'-ethyl-6-fluoro-5-(piperazin-1-yl)pyridinyl hydrazide (0.38 g, 1.41 mmol) were added to a solution of 7-(bromomethyl)-5-(difluoromethoxy)-3-methylquinoxalin-2(1*H*)-one (0.3 g, 0.94 mmol) in acetonitrile (10 mL), and the mixture was then heated to 80 °C for 2 h. The reaction was stopped, the reaction solution was cooled to room temperature, concentrated under reduced pressure, and the residue was dissolved in DCM (50 mL). The mixture was washed with water (15 mL × 3), the organic phase was dried over anhydrous sodium sulfate, concentrated under reduced pressure, and the residue was purified by silica gel column chromatography (DCM/MeOH (v/v) = 25/1) to give a yellow solid product (175 mg, yield 36.82%).
MS (ESI, pos. ion) *m/z:* 506.20 $[M+H]^+$.
$^1$H NMR (599 MHz, DMSO-$d_6$) δ 12.43 (s, 1H), 9.84 (s, 1H), 7.82 (dd, *J* = 8.0, 1.3 Hz, 1H), 7.57 (dd, *J* = 10.6, 8.1 Hz, 1H), 7.36 (t, *J* = 74.4 Hz, 1H), 7.10 (dd, *J* = 48.5, 1.6 Hz, 2H), 4.97 (s, 1H), 3.61 (s, 2H), 3.21 - 3.16 (m, 4H), 2.79 (q, *J* = 7.1 Hz, 2H), 2.60 - 2.55 (m, 4H), 2.41 (s, 3H), 1.00 (t, *J* = 7.1 Hz, 3H).

**Example 67 *N*-cyclopropyl-1'-((5-fluoro-2-methyl-3-oxo-8-(prop-1-yn-1-yl)-3,4-dihydroquinoxalin-6-yl) methyl)-1',2',3',6'-tetrahydro-[3,4'-bipyridine]-6-carboxamide**

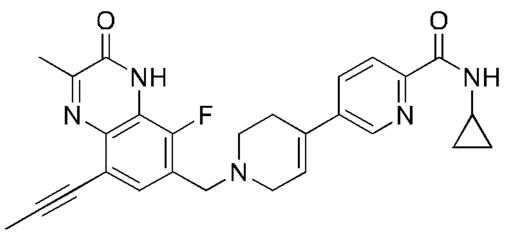

(145)

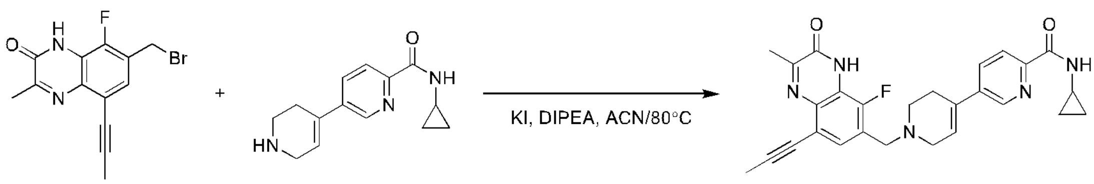


7-(bromomethyl)-8-fluoro-3-methyl-5-(prop-1-yn-1-yl)-1,2-dihydroquinoxalin-2-one (0.10 g, 0.32 mmol), *N*-cyclopropyl-5-(1,2,3,6-tetrahydropyridin-4-yl)pyridine-2-carboxamide hydrochloride (0.12 g, 0.42 mmol) and potassium iodide (0.011 g, 0.064 mmol) were added to a 100 mL flask, stirred to dissolve in ACN (3.5 mL), after cooling, DIPEA (0.17 mL, 0.96 mmol) was added slowly dropwise under ice bath, stirred until homogeneous, and heated to 80 °C for 2 h. After cooling to room temperature, a solid precipitated out with stirring. The filter cake was collected by filtration, washed with MTBE (3.5 mL × 3) and water (3.5 mL × 3), and dried under vacuum to obtain a yellow crude solid. The crude solid was then purified by silica gel column chromatography (DCM/MeOH(v/v) = 20/1) to obtain a white solid (0.070 g, yield 45.89%).

MS (ESI, pos. ion) *m/z:* 472.20 $[M+H]^+$;

$^1$H NMR (400 MHz, DMSO-$d_6$) δ 12.53 (s, 1H), 8.90 - 8.65 (m, 2H), 7.97 (s, 2H), 7.36 (d, *J* = 6.3 Hz, 1H), 6.39 (s, 1H), 3.72 (s, 2H), 3.15 (br, 2H), 2.89 (br, 2H), 2.71 (br, 3H), 2.44 (s, 3H), 2.11 (s, 3H), 0.66 (s, 4H).

**Example 68 Synthesis of 6-fluoro-*N*-methyl-5-(4-((2-methyl-8-(1-methyl-1*H*-pyrazol-5-yl)-3-oxo-3,4-dihydroquinoxalin-6-yl)methyl)piperazin-1-yl)picolinamide**

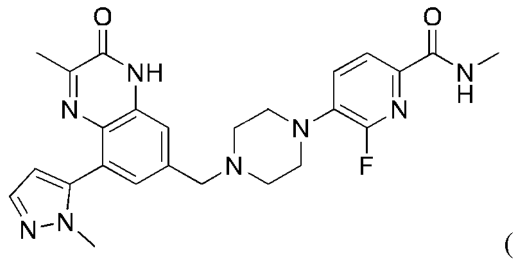

(146)

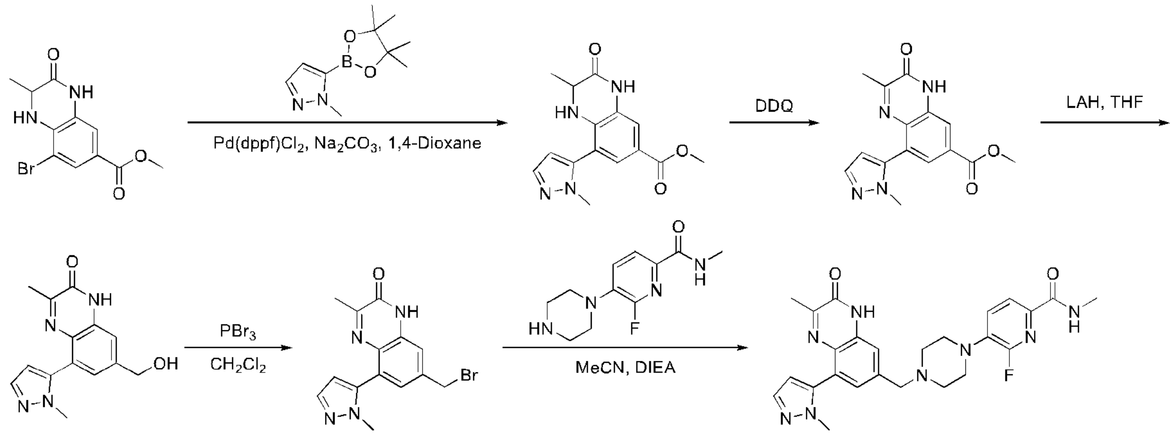


Step 1) Synthesis of methyl 2-methyl-8-(1-methyl-1*H*-pyrazol-5-yl)-3-oxo-1,2,3,4-tetrahydroquinoxalin-6-carboxylate

At room temperature, 1-methyl-5-(4,4,5,5-tetramethyl-1,3,2-dioxaborhexacyclopentan-2-yl)-1*H*-pyrazol (1.36 g, 6.51 mmol), Pd(dppf)$Cl_2$ (0.18 g, 0.25 mmol) and sodium carbonate (1.06 g, 10.02 mmol) were added to a solution of methyl 8-bromo-2-methyl-3-oxo-1,2,3,4-tetrahydroquinoxalin-6-carboxylate (1.5 g, 5.01 mmol) in 1,4-dioxane (24 mL) and water (6 mL). The solution was heated to 105 °C for 12 h after replacing $N_2$. The reaction solution was cooled to room temperature, filtered with diatomaceous earth, and the filtrate was concentrated under reduced pressure to remove the solvent. The residue was purified by silica gel column chromatography (eluting agent: dichloromethane/EA (v/v) = 9/1) to give a yellow solid product (0.08 g, 53.12%).

MS (ESI, pos. ion) *m/z:* 301.2 $[M+H]^+$;

Step 2) Synthesis of methyl 2-methyl-8-(1-methyl-1*H*-pyrazol-5-yl)-3-oxo-3,4-dihydroquinoxalin-6-carboxylate

Under ice bath conditions, 2,3-dichloro-5,6-dicyanobenzoquinone (1.27 g, 5.59 mmol) was added to a solution of methyl 2-methyl-8-(1-methyl-1*H*-pyrazol-5-yl)-3-oxo-1,2,3,4-tetrahydroquinoxalin-6-carboxylate (1.4 g, 4.66 mmol) in dichloromethane (30 mL), and then the mixture was slowly brought to room temperature and stirred for 3 h. The reaction solution was quenched with saturated sodium bicarbonate (40 mL), stirred for 2 h, extracted with dichloromethane (25 mL × 3), the organic phases were combined, washed with saturated sodium bicarbonate, dried over anhydrous sodium

sulfate, and concentrated under reduced pressure to give an orange-yellow solid product (0.75 g, 53.93%).

MS (ESI, pos. ion) *m/z:* 299.2 $[M+H]^+$;

Step 3) Synthesis of 7-(hydroxymethyl)-3-methyl-5-(1-methyl-1*H*-pyrazol-3-yl)quinoxalin-2(1*H*)-one

Under ice bath conditions, lithium aluminum hydride (0.35 g, 8.89 mmol) was added in portions to an anhydrous tetrahydrofuran (20 mL) solution of methyl 2-methyl-8-(1-methyl-1*H*-pyrazol-5-yl)-3-oxo-3,4-dihydroquinoxalin-6-carboxylate (0.75 g, 2.51 mmol). After stirring for 5 min, the mixture was slowly brought to room temperature and the reaction was continued with stirring for 4 h. After cooling, water (0.35 mL), 15% NaOH aqueous solution (0.35 mL) and water (1.05 mL) were added sequentially under ice bath conditions to quench the reaction solution. The mixture was then filtered through diatomaceous earth. The filtrate was concentrated under reduced pressure to remove the solvent. The residue was purified by silica gel column chromatography (eluting agent: DCM/MeOH (v/v) = 20/1) to obtain a yellow solid product (0.26 g, 38.26%).

MS (ESI, pos. ion) *m/z:* 271.2 $[M+H]^+$;

Step 4) Synthesis of 7-(bromomethyl)-3-methyl-3-(1-methyl-1*H*-pyrazol-5-yl)quinoxalin-2(1*H*)-one

Under ice bath conditions, 7-(hydroxymethyl)-3-methyl-5-(1-methyl-1*H*-pyrazol-5-yl)quinoxalin-2(1*H*)-one (0.16 g, 0.59 mmol) was added to dichloromethane (10 mL), followed by the dropwise addition of phosphorus tribromide (0.24 g, 0.90 mmol). The mixture was stirred for 5 min and then allowed to react at room temperature for 2 h. The reaction solution was concentrated under reduced pressure to remove the solvent. The residue was washed with methyl *tert*-butyl ether (10 mL x 2) and dried to obtain a yellow solid crude product (0.19 g, 96.34%), which was directly used for the next reaction.

MS (ESI, pos. ion) *m/z:* 333.1 $[M+H]^+$;

Step 5) Synthesis of 6-fluoro-*N*-methyl-5-(4-((2-methyl-8-(1-methyl-1*H*-pyrazol-5-yl)-3-oxo-3,4-dihydroquinoxalin-6-yl) methyl)piperazin-1-yl)picolinamide

At room temperature, 6-fluoro-*N*-methyl-5-(piperazin-1-yl)pyridine-2-carboxamide (0.15 g, 0.63 mmol) and *N,N*-diisopropylethylamine (0.37 g, 2.85 mmol) were added to a solution of 7-(bromomethyl)-3-methyl-5-(1-methyl-1*H*-pyrazol-5-yl)quinoxalin-2(1*H*)-one (0.19 g, 0.57 mmol) in acetonitrile (20 mL), and then the mixture was heated to 70 °C and stirred for 3 h. The reaction mixture was concentrated under reduced pressure to remove the solvent. The residue was purified by silica gel column chromatography (eluent: dichloromethane/methanol (v/v) = 10/1) to give a yellow solid product (0.06 g, 21.45%).

MS (ESI, pos. ion) *m/z:* 491.2 $[M+H]^+$;

$^1H$ NMR (400 MHz, DMSO-$d_6$) δ (ppm) 12.60 (s, 1H), 8.47 - 8.40 (m, 1H), 8.38 - 8.29 (m, 1H), 7.93 - 7.80 (m, 1H), 7.69 - 7.58 (m, 1H), 7.53 - 7.45 (m, 1H), 7.38 (s, 1H), 6.41 (s, 1H), 4.05 (s, 3H), 3.85 (s, 2H), 3.17 - 3.10 (m, 4H), 2.83 - 2.69 (m, 3H), 2.59 - 2.51 (m, 4H), 2.37 (s, 3H).

**Example 69 Synthesis of 6-fluoro-5-(4-((8-(4-methoxyphenyl)-2-methyl-3-oxo-3,4-dihydroquinoxalin-6-yl) methyl)piperazin-1-yl)-*N*-methylpyridine amide**

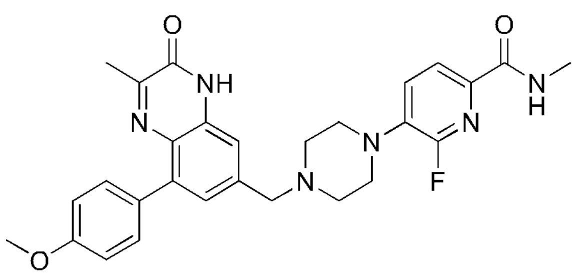

(147)

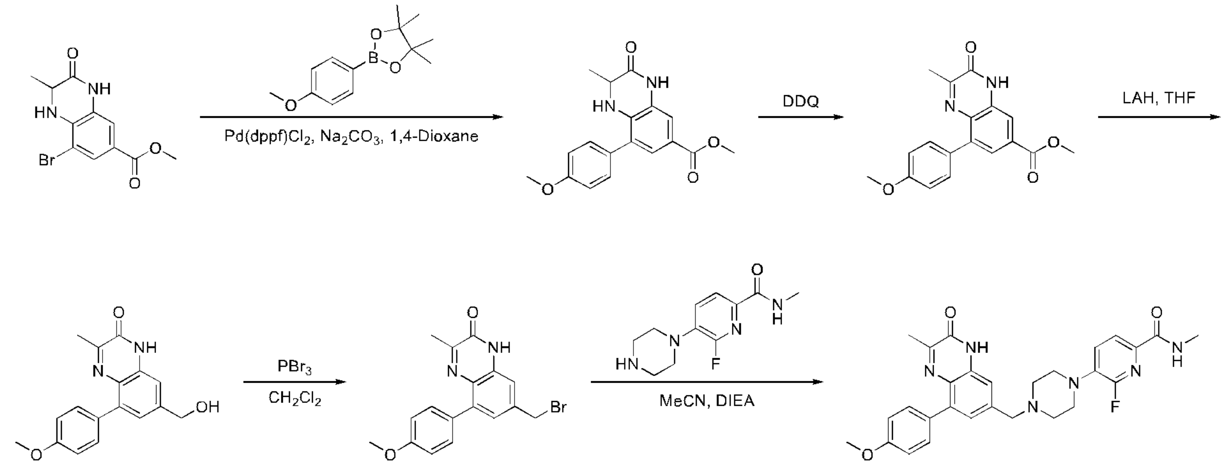


Step 1) Synthesis of methyl 8-(4-methoxyphenyl)-2-methyl-3-oxo-1,2,3,4-tetrahydroquinoxalin-6-carboxylate

At room temperature, 4-methoxyphenylboronic acid pinacol ester (1.56 g, 6.68 mmol), $Pd(dppf)Cl_2$ (0.024 g, 0.34 mmol) and sodium carbonate (1.42 g, 13.36 mmol) were added to a solution of methyl 8-bromo-2-methyl-3-oxo-1,2,3,4-tetrahydroquinoxalin-6-carboxylate (2 g, 6.68 mmol) in 1,4-dioxane (24 mL) and water (6 mL). After replacing $N_2$, the mixture was heated to 105 °C and reacted for 12 h. The reaction solution was cooled to room temperature and filtered with diatomaceous earth. The filtrate was concentrated under reduced pressure to remove the solvent. The residue was purified by silica gel column chromatography (eluting agent: dichloromethane/ethyl acetate (v/v) = 9/1) to give a yellow solid product (1.8 g, 82.49%).

MS (ESI, pos. ion) *m/z:* 327.2 $[M+H]^+$;

Step 2) Synthesis of methyl 8-(4-methoxyphenyl)-2-methyl-3-oxo-3,4-dihydroquinoxalin-6-carboxylate

Under ice bath conditions, 2,3-dichloro-5,6-dicyanobenzoquinone (1.00 g, 4.42 mmol) was added to a solution of methyl 8-(4-methoxyphenyl)-2-methyl-3-oxo-1,2,3,4-tetrahydroquinoxalin-6-carboxylate (1.2 g, 3.68 mmol) in dichloromethane (30 mL). The mixture was then slowly brought to room temperature and stirred for 3 h. The reaction mixture was quenched with saturated sodium bicarbonate (38 mL), stirred for 2 h, extracted with dichloromethane (25 mL $\times$ 3), and the organic phases were combined. The mixture was washed with saturated sodium bicarbonate, dried over anhydrous sodium sulfate, and concentrated under reduced pressure to give an orange-yellow solid product (0.3 g, 25.16%).

MS (ESI, pos. ion) *m/z:* 325.2 $[M+H]^+$;

Step 3) Synthesis of 7-(hydroxymethyl)-5-(4-methoxyphenyl)-3-methylquinoxalin-2(1*H*)-one

Under ice bath conditions, lithium aluminum hydride (0.11 g, 2.81 mmol) was added in portions to a solution of methyl 8-(4-methoxyphenyl)-2-methyl-3-oxo-3,4-dihydroquinoxalin-6-carboxylate (0.3 g, 0.92 mmol) in tetrahydrofuran (20 mL). The mixture was stirred until no further exothermic reaction occurred, and the reaction was allowed to proceed at room temperature for 4 h. The mixture was cooled, the reaction was quenched by adding water (5 mL) under ice bath, followed by dropwise addition of 1M dilute hydrochloric acid to adjust the pH to 3-4. Extraction was performed with tetrahydrofuran (25 mL $\times$ 2), and the organic phases were combined. The combined organic phase was washed with saturated brine, dried over anhydrous sodium sulfate, and filtered. The solvent was removed by concentration under reduced pressure. The residue was purified by silica gel column chromatography (eluent: dichloromethane/methanol (v/v) = 20/1) to afford a white solid (0.2 g, 72.97 %).

MS (ESI, pos. ion) *m/z:* 297.1 $[M+H]^+$;

Step 4) Synthesis of 7-(bromomethyl)-5-(4-methoxyphenyl)-3-methylquinoxalin-2(1*H*)-one

Under ice bath conditions, 7-(hydroxymethyl)-5-(4-methoxyphenyl)-3-methylquinoxalin-2(1*H*)-one (0.2 g, 0.67 mmol) was added to dichloromethane (10 mL), followed by the dropwise addition of phosphorus tribromide (0.28 g, 1.02 mmol). The mixture was stirred for 5 min and then allowed to react at room temperature for 2 h. The reaction solution was concentrated under reduced pressure to remove the solvent. The residue was washed with methyl tert-butyl ether (10 mL $\times$ 2) and dried to obtain a yellow solid crude product (0.12 g, 49.49%), which was directly used for the next reaction.

MS (ESI, pos. ion) *m/z:* 359.1, 361.1 $[M+H]^+$;

Step 5) Synthesis of 6-fluoro-5-(4-((8-(4-methoxyphenyl)-2-methyl-3-oxo-3,4-dihydroquinoxalin-6-yl)methyl)piperazin-1-yl)-*N*-methylpyridine amide

At room temperature, 6-fluoro-*N*-methyl-5-(piperazin-1-yl)pyridine-2-carboxamide (0.086 g, 0.36 mmol) and *N,N*-diisopropylethylamine (0.21 g, 1.65 mmol) were added to a solution of 7-(bromomethyl)-5-(4-methoxyphenyl)-3-methylquinoxalin-2(1*H*)-one (0.12 g, 0.33 mmol) in acetonitrile (20 mL), and then the mixture was heated to 70 °C and stirred for 3 h. The reaction mixture was concentrated under reduced pressure to remove the solvent. The residue was purified by silica gel column chromatography (eluent: dichloromethane/methanol (v/v) = 10/1) to give a yellow solid product (0.1 g, 57.95%).

MS (ESI, pos. ion) *m/z:* 517.4 $[M+H]^+$;

$^1H$ NMR (400 MHz, DMSO-$d_6$) δ (ppm) 12.27 (s, 1H), 8.35 (s, 1H), 7.86 - 7.80 (m, 1H), 7.63 - 7.49 (m, 4H), 7.26 - 7.20 (m, 2H), 7.04 - 6.98 (m, 2H), 3.80 (s, 3H), 3.63 (s, 2H), 3.19 - 3.13 (m, 4H), 2.76 (s, 3H), 2.63 - 2.57 (m, 4H), 2.33 (s, 3H).

**Example 70 Synthesis of 6-fluoro-*N*-methyl-5-(4-((2-methyl-8-(1-methyl-1*H*-pyrazol-4-yl)-3-oxo-3,4-dihydroquinoxalin-6-yl)methyl)piperazin-1-yl)picolinamide**

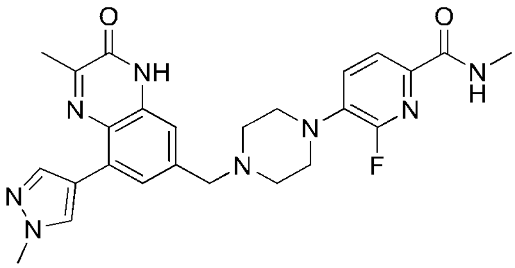

(148)

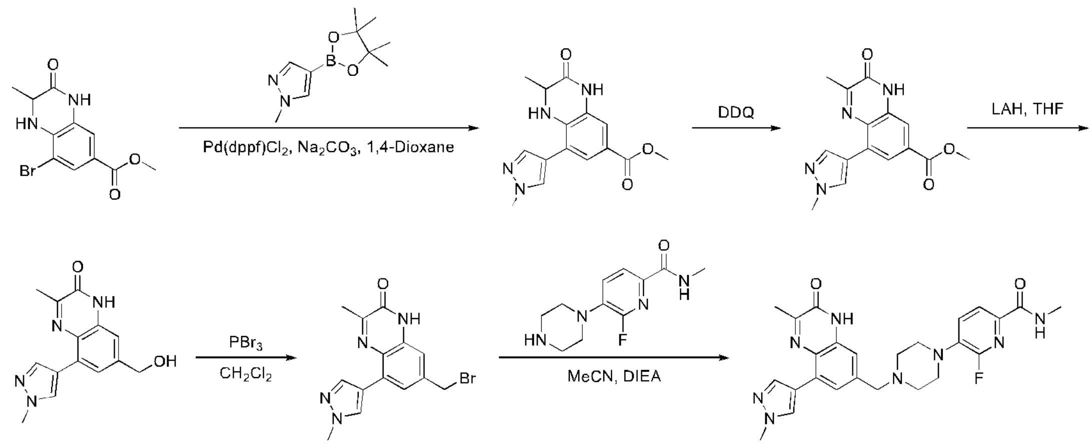


Step 1) Synthesis of methyl 2-methyl-8-(1-methyl-1*H*-pyrazol-4-yl)-3-oxo-1,2,3,4-tetrahydroquinoxalin-6-carboxylate

At room temperature, 1-methyl-4-(tetramethyl-1,3,2-dioxaborhexacyclopentan-2-yl)-1H-pyrazol (1.56 g, 7.51 mmol), Pd(dppf)$Cl_2$ (0.18 g, 0.25 mmol), and sodium carbonate (1.06 g, 10.02 mmol) were added to a solution of methyl 8-bromo-2-methyl-3-oxo-1,2,3,4-tetrahydroquinoxalin-6-carboxylate (1.5 g, 5.01 mmol) in 1,4-dioxane (28 mL) and water (7 mL). After replacing $N_2$, the mixture was heated to 105 °C and reacted for 12 h. The reaction solution was cooled to room temperature and filtered with diatomaceous earth. The filtrate was concentrated under reduced pressure to remove the solvent. The residue was purified by silica gel column chromatography (eluting agent: DCM/EtOAc (v/v) = 9/1) to give a yellow solid product (0.9 g, 59.76%).

MS (ESI, pos. ion) *m/z:* 301.2 $[M+H]^+$;

Step 2) Synthesis of methyl 2-methyl-8-(1-methyl-1*H*-pyrazol-4-yl)-3-oxo-3,4-dihydroquinoxalin-6-carboxylate

Under ice bath conditions, 2,3-dichloro-5,6-dicyanobenzoquinone (0.91 g, 4.00 mmol) was added to a solution of

methyl 2-methyl-8-(1-methyl-1*H*-pyrazol-4-yl)-3-oxo-1,2,3,4-tetrahydroquinoxalin-6-carboxylate (1 g, 3.33 mmol) in dichloromethane (30 mL). The mixture was then slowly brought to room temperature and stirred for 3 h. The reaction mixture was quenched with saturated sodium bicarbonate (40 mL) and stirred for 2 h. The resulting solid was dried to give a yellow solid product (0.9 g, 90.61%).

MS (ESI, pos. ion) *m/z:* 299.2 $[M+H]^+$;

Step 3) Synthesis of 7-(hydroxymethyl)-3-methyl-5-(1-methyl-1*H*-pyrazol-4-yl)quinoxalin-2(1*H*)-one

Under ice bath conditions, lithium aluminum hydride (0.36 g, 9.21 mmol) was added in portions to an anhydrous tetrahydrofuran (30 mL) solution of methyl 2-methyl-8-(1-methyl-1*H*-pyrazol-4-yl)-3-oxo-3,4-dihydroquinoxalin-6-carboxylate (0.9 g, 3.02 mmol). After stirring for 5 min, the mixture was slowly brought to room temperature and the reaction was continued with stirring for 4 h. The solution was cooled and quenched in an ice bath by adding water (0.36 mL), 15% NaOH aqueous solution (0.36 mL) and water (1.08 mL) sequentially. The solution was then filtered through diatomaceous earth, and the filtrate was concentrated under reduced pressure to remove the solvent. The residue was purified by silica gel column chromatography (eluent: DCM/MeOH (v/v) = 20/1) to give a yellow solid product (0.27 g, 33.11%).

MS (ESI, pos. ion) *m/z:* 271.2 $[M+H]^+$;

Step 4) Synthesis of 7-(bromomethyl)-3-methyl-3-(1-methyl-1*H*-pyrazol-4-yl)quinoxalin-2(1*H*)-one

Under ice bath conditions, 7-(hydroxymethyl)-3-methyl-5-(1-methyl-1*H*-pyrazol-4-yl)quinoxalin-2(1*H*)-one (0.27 g, 1.0 mmol) was added to dichloromethane (10 mL), followed by the dropwise addition of phosphorus tribromide (0.41 g, 1.52 mmol). The mixture was stirred for 5 min and then allowed to react at room temperature for 2 h. The reaction solution was concentrated under reduced pressure to remove the solvent. The residue was washed with methyl tert-butyl ether (10 mL × 2) and dried to obtain a yellow solid crude product (0.32 g, 96.15%), which was directly used for the next reaction.

MS (ESI, pos. ion) *m/z:* 333.2, 335.2 $[M+H]^+$;

Step 5) Synthesis of 6-fluoro-*N*-methyl-5-(4-((2-methyl-8-(1-methyl-1*H*-pyrazol-4-yl)-3-oxo-3,4-dihydroquinoxalin-6-yl) methyl)piperazin-1-yl)picolinamide

At room temperature, 6-fluoro-*N*-methyl-5-(piperazin-1-yl)pyridineamide (0.27 g, 1.15 mmol) and *N,N*-diisopropylethylamine (0.62 g, 4.8 mmol) were added to a solution of 7-(bromomethyl)-3-methyl-5-(1-methyl-1*H*-pyrazol-4-yl) quinoxalin-2(1*H*)-one (0.32 g, 0.96 mmol) in acetonitrile (20 mL), and the mixture was heated to 70 °C and stirred for 3 h. The reaction solution was concentrated under reduced pressure to remove the solvent, and the residue was purified by silica gel column chromatography (eluent: dichloromethane/methanol (v/v) = 10/1) to give a yellow solid product (0.45 g, 95.52%).

MS (ESI, pos. ion) *m/z:* 491.4 $[M+H]^+$;

$^1H$ NMR (400 MHz, DMSO-$d_6$) δ (ppm) 12.35 (s, 1H), 9.08 (s, 1H), 8.46 (s, 1H), 8.43 - 8.36 (m, 1H), 8.11 (s, 1H), 7.83 (d, *J* = 8.0 Hz, 1H), 7.65 - 7.61 (m, 1H), 7.61 - 7.53 (m, 1H), 7.18 (s, 1H), 3.90 (s, 3H), 3.84 (s, 2H), 3.49 - 3.37 (m, 4H), 3.27 (s, 3H), 2.77 - 2.72 (m, 4H), 2.46 (s, 3H).

**Example 71 Synthesis of 5-(4-((8-(cyclopentoxy)-2-methyl-3-oxo-3,4-dihydroquinoxalin-6-yl)methyl)piperazin-1-yl)-6-fluoro-*N*-methylpyridine amide**

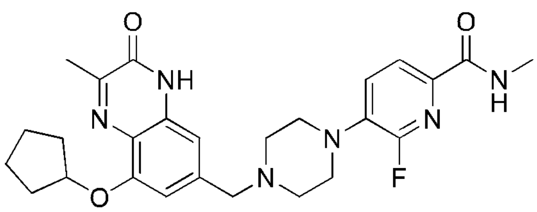

(149)

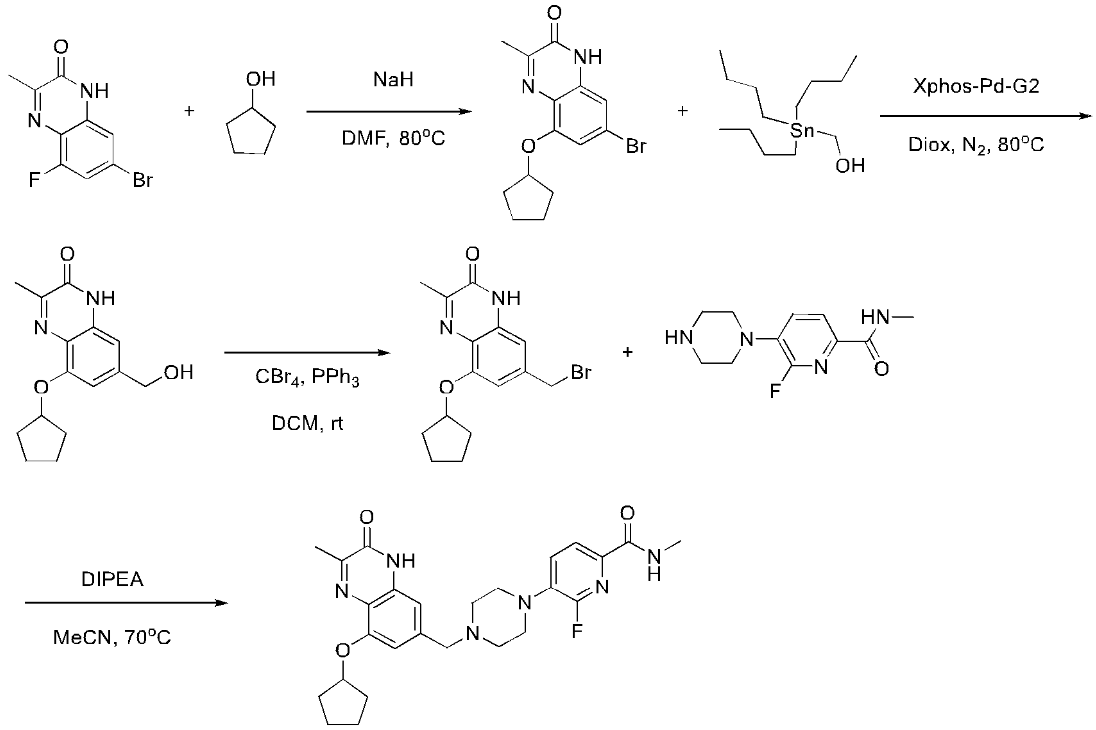


Step 1) Synthesis of 7-bromo-5-(cyclopentoxy)-3-methylquinoxalin-2(1*H*)-one

At 0°C, NaH (390 mg, 9.75 mmol, 60%) was added to a solution of 7-bromo-5-fluoro-3-methylquinoxalin-2(1*H*)-one (500 mg, 1.95 mmol) and cyclopentanol (840 mg, 9.75 mmol) in *N,N*-dimethylformamide (10 mL). After the addition was complete, the reaction was carried out at 80°C for 14 h. The reaction solution was concentrated under reduced pressure, and water (50 mL) was added. The mixture was stirred vigorously for 2 h, filtered to obtain a solid, and dried under vacuum at 50°C for 8 h to obtain a pale yellow solid (550 mg, 87%).

MS (ESI, pos. ion) *m/z:* 323.2 $[M+H]^+$.

Step 2) Synthesis of 5-(cyclopentoxy)-7-(hydroxymethyl)-3-methylquinoxalin-2(1*H*)-one

7-bromo-5-(cyclopentoxy)-3-methylquinoxalin-2(1*H*)-one (550 mg, 1.70 mmol), (tributyltin)methanol (600 mg, 1.87 mmol), and Xphos-Pd-G2 (134 mg, 0.17 mmol) were added to 1,4-dioxane (15 mL) under nitrogen protection and reacted at 80 °C for 15 h. The reaction solution was concentrated under reduced pressure, and the residue was purified by silica gel column chromatography (dichloromethane/methanol (v/v) = 20/1) to give a yellow solid (300 mg, 64%).

MS (ESI, pos. ion) *m/z:* 275.3 $[M+H]^+$.

Step 3) Synthesis of 7-(bromomethyl)-5-(cyclopentoxy)-3-methylquinoxalin-2(1*H*)-one

$CBr_4$ (484 mg, 1.46 mmol) was added to 5-(cyclopentoxy)-7-(hydroxymethyl)-3-methylquinoxalin-2(1*H*)-one (200 mg, 0.73 mmol) and $PPh_3$ (383 mg, 1.46 mmol) in dichloromethane (16 mL), and the reaction was carried out at room temperature for 16 h. The reaction solution was concentrated under reduced pressure, and the residue was purified by silica gel column chromatography (PE/EtOAc (v/v) = 1/1) to give a white solid (100 mg, 41%).

MS (ESI, pos. ion) *m/z:* 337.1 $[M+H]^+$;

Step 4) Synthesis of 5-(4-((8-(cyclopentoxy)-2-methyl-3-oxo-3,4-dihydroquinoxalin-6-yl)methyl)piperazin-1-yl)-6-fluoro-*N*-methylpyridine amide

7-(bromomethyl)-5-(cyclopentoxy)-3-methylquinoxalin-2(14)-one (100 mg, 0.30 mmol), 6-fluoro-*N*-methyl-5-(piperazin-1-yl)pyridineamide (79 mg, 0.33 mmol), and *N,N*-diisopropylethylamine (155 mg, 1.20 mmol) were added sequentially to MeCN (8 mL), and the reaction was carried out at 70 °C for 3 h. The reaction solution was concentrated under reduced pressure, diluted with dichloromethane (60 mL), washed with water (30 mL) and saturated NaCl solution (30 mL), dried over anhydrous $Na_2SO_4$, concentrated under reduced pressure, and the residue was purified

by silica gel column chromatography (dichloromethane/methanol (v/v) = 20/1) to give a pale yellow solid (110 mg, 75%).

MS (ESI, pos. ion) *m/z:* 495.3 $[M+H]^+$;

$^1H$ NMR (400 MHz, DMSO-$d_6$) δ (ppm) 12.15 (s, 1H), 8.37 (d, *J* = 4.7 Hz, 1H), 7.84 (d, *J* = 7.6Hz, 1H), 7.57 (dd, *J* = 10.4, 8.3 Hz, 1H), 6.80 (s, 2H), 4.95 (s, 1H), 3.57 (s, 2H), 3.19 (s, 4H), 2.77 (d, *J* = 4.7 Hz, 3H), 2.57 (s, 4H), 2.37 (s, 3H), 2.02 - 1.94 (m, 2H), 1.85 - 1.72 (m, 4H), 1.66 - 1.55 (m, 2H).

**Example 72 Synthesis of 5-(4-((8-(cyclohexyloxy)-2-methyl-3-oxo-3,4-dihydroquinoxalin-6-yl)methyl)piperazin-1-yl)-6-fluoro-*N*-methylpyridine amide**

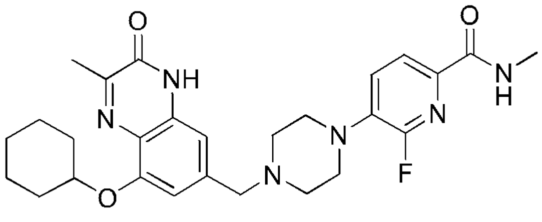

(150)

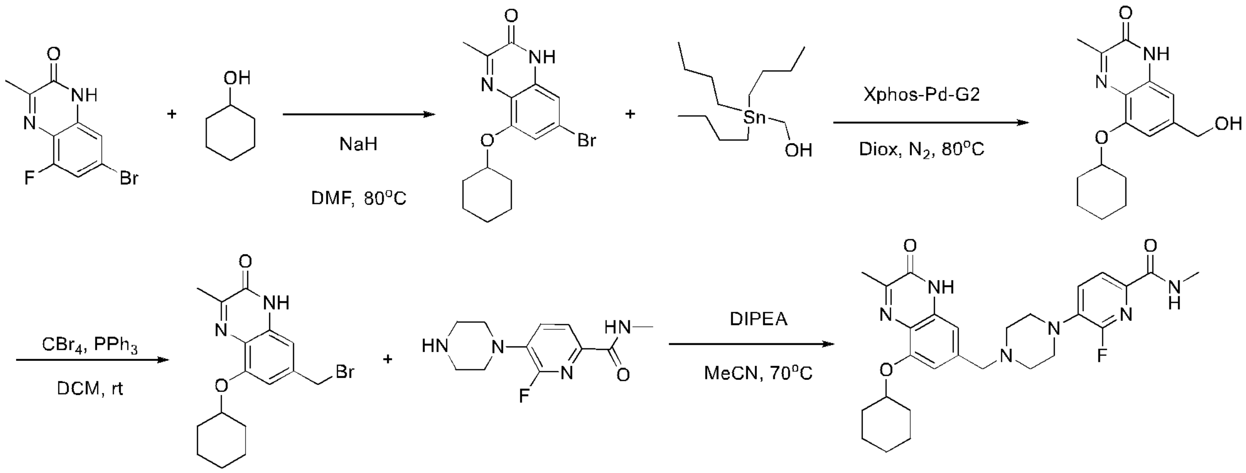


Step 1) Synthesis of 7-bromo-5-(cyclohexyloxy)-3-methylquinoxalin-2(1*H*)-one

At 0°C, NaH (390 mg, 9.75 mmol, 60%) was added to a solution of 7-bromo-5-fluoro-3-methylquinoxalin-2(1*H*)-one (500 mg, 1.95 mmol) and cyclohexanol (976 mg, 9.75 mmol) in *N,N*-dimethylformamide (10 mL). After the addition was complete, the reaction was carried out at 80°C for 14 h. The reaction solution was concentrated under reduced pressure, and water (50 mL) was added. The mixture was stirred vigorously for 2 h, filtered to obtain a solid, and dried under vacuum at 50°C for 8 h to obtain a pale yellow solid (500 mg, 76%).

MS (ESI, pos. ion) *m/z:* 337.1 $[M+H]^+$.

Step 2) Synthesis of 5-(cyclohexyloxy)-7-(hydroxymethyl)-3-methylquinoxalin-2(1*H*)-one

7-bromo-5-(cyclohexyloxy)-3-methylquinoxalin-2(1*H*)-one (400 mg, 1.19 mmol), (tributyltin)methanol (420 mg, 1.31 mmol), and Xphos-Pd-G2 (94 mg, 0.12 mmol) were added to 1,4-dioxane (12 mL) under nitrogen protection and reacted at 80 °C for 16 h. The reaction solution was concentrated under reduced pressure, and the residue was purified by silica gel column chromatography (dichloromethane/methanol (v/v) = 20/1) to give a pale yellow solid (184 mg, 54%).

MS (ESI, pos. ion) *m/z:* 289.3 $[M+H]^+$.

Step 3) Synthesis of 7-(bromomethyl)-5-(cyclohexyloxy)-3-methylquinoxalin-2(1*H*)-one

$CBr_4$ (411 mg, 1.24 mmol) was added to dichloromethane (16 mL) containing 5-(cyclohexyloxy)-7-(hydroxymethyl)-3-methylquinoxalin-2(1*H*)-one (180 mg, 0.62 mmol) and $PPh_3$ (325 mg, 1.24 mmol), and the reaction was carried out at room temperature for 15 h. The reaction solution was concentrated under reduced pressure, and the residue was

purified by silica gel column chromatography (PE/EtOAc (v/v) = 1/1) to give a white solid (144 mg, 66%).
MS (ESI, pos. ion) *m/z:* 351.1 [M+H]+;

Step 4) Synthesis of 5-(4-((8-(cyclohexyloxy)-2-methyl-3-oxo-3,4-dihydroquinoxalin-6-yl)methyl)piperazin-1-yl)-6-fluoro-*N*-methylpyridine amide

7-(bromomethyl)-5-(cyclohexyloxy)-3-methylquinoxalin-2(1*H*)-one (140 mg, 0.40 mmol), 6-fluoro-*N*-methyl-5-(piperazin-1-yl)pyridineamide (104 mg, 0.44 mmol), and *N,N*-diisopropylethylamine (206 mg, 1.60 mmol) were added sequentially to MeCN (8 mL), and the reaction was carried out at 70 °C for 3 h. The reaction solution was concentrated under reduced pressure, diluted with dichloromethane (60 mL), washed with water (30 mL) and saturated NaCl solution (30 mL), dried over anhydrous $Na_2SO_4$, concentrated under reduced pressure, and the residue was purified by silica gel column chromatography (dichloromethane/methanol (v/v) = 20/1) to give a pale yellow solid (170 mg, 84%).
MS (ESI, pos. ion) *m/z:* 509.3 [M+H]+;
$^1H$ NMR (400 MHz, DMSO-$d_6$) δ (ppm) 12.14 (s, 1H), 8.37 (d, *J* = 4.8 Hz, 1H), 7.84 (d, *J* = 7.9Hz, 1H), 7.60 - 7.52 (m, 1H), 6.83 (d, *J*= 2.9 Hz, 2H), 4.52 - 4.46 (m, 1H), 3.57 (s, 2H), 3.18 (s, 4H), 2.77 (d, *J* = 4.7 Hz, 3H), 2.57 (s, 4H), 2.37 (s, 3H), 2.04 - 1.93 (m, 2H), 1.82 - 1.72 (m, 2H), 1.55 - 1.48 (m, 2H), 1.44 - 1.26 (m, 4H).

**Example 73 Synthesis of 6-fluoro-5-(4-(((8-(3-methoxyphenyl)-2-methyl-3-oxo-3,4-dihydroquinoxalin-6-yl) methyl)piperazin-1-yl)-*N*-methylpyridine amide**

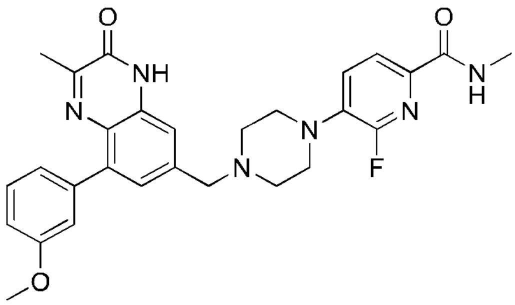

(151)

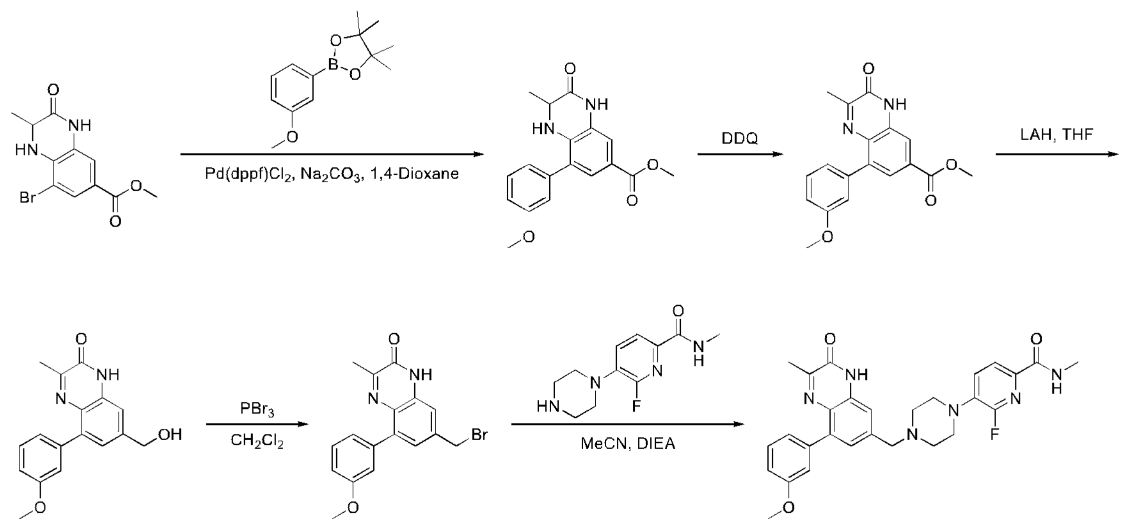


Step 1) Synthesis of methyl 8-(3-methoxyphenyl)-2-methyl-3-oxo-1,2,3,4-tetrahydroquinoxalin-6-carboxylate

At room temperature, 3-methoxyphenylboronic acid pinacol ester (1.17 g, 5.01 mmol), Pd(dppf)$Cl_2$ (0.12 g, 0.17 mmol), and sodium carbonate (0.71 g, 6.68 mmol) were added to a solution of methyl 8-bromo-2-methyl-3-oxo-1,2,3,4-tetrahydroquinoxaloline-6-carboxylate (1 g, 3.34 mmol) in 1,4-dioxane (24 mL) and water (6 mL). After replacing $N_2$, the mixture was heated to 105 °C and reacted for 12 h. The reaction solution was cooled to room temperature and filtered with diatomaceous earth. The filtrate was concentrated under reduced pressure to remove the solvent. The residue was

purified by silica gel column chromatography (eluting agent: dichloromethane/ethyl acetate (v/v) = 9/1) to give a yellow solid product (0.9 g, 82.49%).
MS (ESI, pos. ion) *m/z:* 327.3 $[M+H]^+$;

Step 2) Synthesis of methyl 8-(3-methoxyphenyl)-2-methyl-3-oxo-3,4-dihydroquinoxalin-6-carboxylate

Under ice bath conditions, 2,3-dichloro-5,6-dicyanobenzoquinone (0.47 g, 2.06 mmol) was added to a solution of methyl 8-(3-methoxyphenyl)-2-methyl-3-oxo-1,2,3,4-tetrahydroquinoxalin-6-carboxylate (0.56 g, 1.72 mmol) in dichloromethane (30 mL), and the mixture was slowly brought to room temperature and stirred for 3 h. The reaction mixture was quenched with saturated sodium bicarbonate (38 mL), stirred for 2 h, extracted with dichloromethane (25 mL × 3), and the organic phases were combined. The mixture was washed with saturated sodium bicarbonate, dried over anhydrous sodium sulfate, and concentrated under reduced pressure to give an orange-yellow solid product (0.5 g, 89.84%).
MS (ESI, pos. ion) *m/z:* 325.3 $[M+H]^+$;

Step 3) Synthesis of 7-(hydroxymethyl)-5-(3-methoxyphenyl)-3-methylquinoxalin-2(1*H*)-one

Under ice bath conditions, lithium aluminum hydride (0.18 g, 4.70 mmol) was added in portions to a solution of methyl 8-(3-methoxyphenyl)-2-methyl-3-oxo-3,4-dihydroquinoxalin-6-carboxylate (0.50 g, 1.54 mmol) in tetrahydrofuran (20 mL). The mixture was stirred until no further exothermic reaction occurred, and the reaction was allowed to proceed to room temperature for 4 h. After cooling, the mixture was quenched with water (5 mL) under ice bath conditions, and the pH was adjusted to 3-4 by adding 1M dilute hydrochloric acid. The mixture was extracted with tetrahydrofuran (25 mL × 2), and the organic phases were combined, washed with saturated brine, dried over anhydrous sodium sulfate, and the filtrate was concentrated under reduced pressure to remove the solvent. The residue was purified by silica gel column chromatography (eluent: DCM/MeOH (v/v) = 20/1) to give a white solid (0.2 g, 43.78%).
MS (ESI, pos. ion) *m/z:* 297.2 $[M+H]^+$;

Step 4) Synthesis of 7-(bromomethyl)-5-(3-methoxyphenyl)-3-methylquinoxalin-2(1*H*)-one

Under ice bath conditions, 7-(hydroxymethyl)-5-(3-methoxyphenyl)-3-methylquinoxalin-2(1*H*)-one (0.20 g, 0.67 mmol) was added to dichloromethane (10 mL), followed by the dropwise addition of phosphorus tribromide (0.28 g, 1.02 mmol). After stirring for 5 min, the reaction was allowed to proceed to room temperature for 2 h. The reaction solution was concentrated under reduced pressure to remove the solvent. The residue was washed with methyl *tert*-butyl ether (10 mL × 2) and dried to obtain a yellow solid crude product (0.15 g, 61.87%), which was directly used for the next reaction.
MS (ESI, pos. ion) *m/z:* 359.3 $[M+H]^+$;

Step 5) Synthesis of 6-fluoro-5-(4-(((8-(3-methoxyphenyl)-2-methyl-3-oxo-3,4-dihydroquinoxalin-6-yl)methyl)piperazin-1-yl)-*N*-methylpyridine amide

At room temperature, 6-fluoro-*N*-methyl-5-(piperazin-1-yl)pyridineamide (0.11 g, 0.46 mmol) and *N,N*-diisopropylethylamine (0.27 g, 2.1 mmol) were added to a solution of 7-(bromomethyl)-5-(3-methoxyphenyl)-3-methylquinoxalin-2(1*H*)-one (0.15 g, 0.42 mmol) in acetonitrile (20 mL), and then the mixture was heated to 70 °C and stirred for 3 h. The reaction mixture was concentrated under reduced pressure to remove the solvent. The residue was purified by silica gel column chromatography (eluent: dichloromethane/methanol (v/v) = 10/1) to give a yellow solid product (0.08 g, 37.09%).
MS (ESI, pos. ion) *m/z:* 517.3 $[M+H]^+$;
$^1$H NMR (400 MHz, DMSO-$d_6$) δ (ppm) 8.39 (s, 1H), 7.83 (d, *J* = 7.9 Hz, 1H), 7.55 (t, *J* = 9.3 Hz, 1H), 7.36 (t, *J* = 8.0 Hz, 1H), 7.27 (d, *J* = 10.6 Hz, 2H), 7.12 (d, *J* = 8.3 Hz, 2H), 6.96 (d, *J* = 8.4 Hz, 1H), 3.78 (s, 2H), 3.70 - 3.68 (m, 3H), 3.19 - 3.15 (m, 4H), 2.75 (d, *J* = 4.7 Hz, 3H), 2.64 - 2.57 (m, 4H), 2.33 (s, 3H).

**Example 74 Synthesis of 6-fluoro-*N*-methyl-5-(4-((2-methyl-8-(1-methyl-1*H*-pyrazol-5-yl)-3-oxo-3,4-dihydroquinoxalin-6-yl)methyl)piperazin-1-yl)picolinamide**

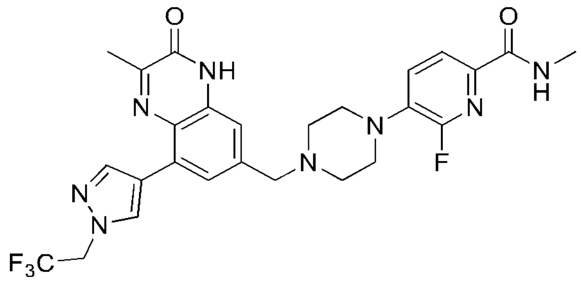

(152)

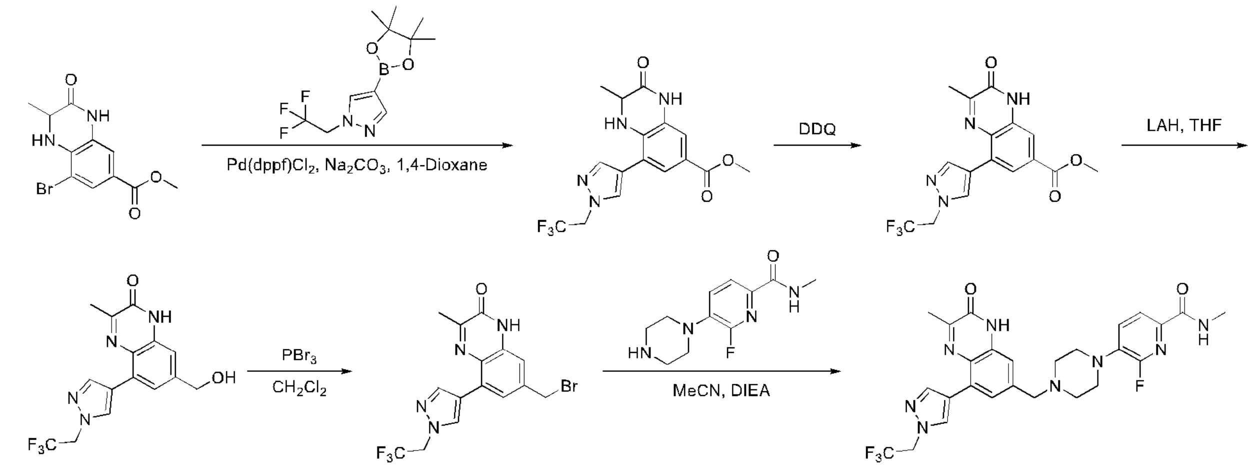


Step 1) Synthesis of methyl 2-methyl-3-oxo-8-(1-(2,2,2-trifluoroethyl)-1*H*-pyrazol-4-yl)-1,2,3,4-tetrahydroquinoxalin-6-carboxylate

At room temperature, 4-(4,4,5,5-tetramethyl-1,3,2-dioxaborhexacyclopentan-2-yl)-1-(2,2,2-trifluoroethyl)-1*H*-pyrazol (2.07 g, 7.51 mmol), Pd(dppf)$Cl_2$ (0.18 g, 0.25 mmol) and sodium carbonate (1.06 g, 10.02 mmol) were added to a solution of methyl 8-bromo-2-methyl-3-oxo-1,2,3,4-tetrahydroquinoxalin-6-carboxylate (1.5 g, 5.01 mmol) in 1,4-dioxane (28 mL) and water (7 mL), replaced with $N_2$, and heated to 105 °C for 12 h. The reaction solution was cooled to room temperature and filtered with diatomaceous earth. The filtrate was concentrated under reduced pressure to remove the solvent. The residue was purified by silica gel column chromatography (eluting agent: dichloromethane/ethyl acetate (v/v) = 9/1) to give a yellow solid product (1.8 g, 97.46%).

MS (ESI, pos. ion) *m/z:* 359.3 $[M+H]^+$;

Step 2) Synthesis of methyl 2-methyl-3-oxo-8-(1-(2,2,2-trifluoroethyl)-1*H*-pyrazol-4-yl)-3,4-dihydroquinoxalin-6-carboxylate

Under ice bath conditions, 2,3-dichloro-5,6-dicyanobenzoquinone (1.33 g, 5.87 mmol) was added to a solution of methyl 2-methyl-3-oxo-8-(1-(2,2,2-trifluoroethyl)-1*H*-pyrazol-4-yl)-1,2,3,4-tetrahydroquinoxalin-6-carboxylate (1.8 g, 4.89 mmol) in dichloromethane (30 mL). The mixture was then slowly brought to room temperature and the reaction was stirred for 3 h. The reaction mixture was quenched with saturated sodium bicarbonate (40 mL), stirred for 2 h, extracted with dichloromethane (25 mL × 3), and the organic phases were combined. The mixture was washed with saturated sodium bicarbonate, dried over anhydrous sodium sulfate, and concentrated under reduced pressure to give an orange-yellow solid product (1.75 g, 97.76%).

MS (ESI, pos. ion) *m/z:* 367.1 $[M+H]^+$;

Step 3) Synthesis of 7-(hydroxymethyl)-3-methyl-5-(1-(2,2,2-trifluoroethyl)-1*H*-pyrazol-4-yl)quinoxalin-2(1*H*)-one

Under ice bath conditions, lithium aluminum hydride (0.35 g, 8.89 mmol) was added in portions to an anhydrous tetrahydrofuran (20 mL) solution of methyl 2-methyl-3-oxo-8-(1-(2,2,2-trifluoroethyl)-1*H*-pyrazol-4-yl)-3,4-dihydroquinoxalin-6-carboxylate (0.75 g, 2.51 mmol). After stirring for 5 min, the mixture was slowly brought to room temperature and the reaction was continued with stirring for 4 h. After cooling, water (0.35 mL), 15% NaOH aqueous solution (0.35 mL) and water (1.05 mL) were added sequentially under ice bath conditions to quench the solvent. The mixture was then filtered

through diatomaceous earth. The filtrate was concentrated under reduced pressure to remove the solvent. The residue was purified by silica gel column chromatography (eluent: dichloromethane/methanol (v/v) = 20/1) to give a yellow solid product (0.26 g, 38.26%).

MS (ESI, neg. ion) *m/z:* 337.3 $[M-H]^-$;

Step 4) Synthesis of 7-(bromomethyl)-3-methyl-5-(1-(2,2,2-trifluoroethyl)-1*H*-pyrazol-4-yl)quinoxalin-2(1*H*)-one

Under ice bath conditions, 7-(hydroxymethyl)-3-methyl-5-(1-(2,2,2-trifluoroethyl)-1*H*-pyrazol-4-yl)quinoxalin-2(1*H*)-one (1.45 g, 4.29 mmol) was added to dichloromethane (10 mL), followed by the dropwise addition of phosphorus tribromide (1.77 g, 6.52 mmol). After stirring for 5 min, the reaction was allowed to proceed to room temperature for 2 h. The reaction solution was concentrated under reduced pressure to remove the solvent. The residue was washed with methyl *tert*-butyl ether (10 mL × 2) and dried to obtain a yellow solid crude product (1.7 g, 98.86%), which was directly used for the next reaction.

Step 5) Synthesis of 6-fluoro-*N*-methyl-5-(4-((2-methyl-3-oxo-8-(1-(2,2,2-trifluoroethyl)-1*H*-pyrazol-4-yl)-3,4-dihydroquinoxalin-6-yl)methyl)piperazin-1-yl)pyridine amide

At room temperature, 6-fluoro-*N*-methyl-5-(piperazin-1-yl)pyridine amide (0.36 g, 1.5 mmol) and *N,N*-diisopropylethylamine (2.58 g, 20 mmol) were added to a solution of 7-(bromomethyl)-3-methyl-5-(1-(2,2,2-trifluoroethyl)-1*H*-pyrazol-4-yl)quinoxalin-2(14)-one in acetonitrile (20 mL), and then the mixture was heated to 70 °C and stirred for 3 h. The reaction mixture was concentrated under reduced pressure to remove the solvent. The residue was purified by silica gel column chromatography (eluent: dichloromethane/methanol (v/v) = 20/1) to give a yellow solid product (0.2 g, 28.73%).

MS (ESI, pos. ion) *m/z:* 559.2 $[M+H]^+$;
$^1H$ NMR (400 MHz, DMSO-$d_6$) δ (ppm) 12.29 (s, 1H), 8.67 (s, 1H), 8.43 - 8.35 (m, 1H), 8.26 (s, 1H), 7.84 (d, *J*= 8.0 Hz, 1H), 7.62 - 7.55 (m, 1H), 7.54 - 7.51 (m, 1H), 7.19 - 7.14 (m, 1H), 5.29 - 5.18 (m, 2H), 3.63 (s, 2H), 3.23 - 3.16 (m, 4H), 2.76 (d, *J*= 4.7 Hz, 3H), 2.63 - 2.56 (m, 4H), 2.48 (s, 3H).

**Biological Assay**

**Example A: Evaluation of the Inhibitory Effect of the Compounds of the Present Invention on the Enzyme Activities of PARP1 and PARP2**

Experimental Objective: To evaluate the inhibitory effect of the compounds of the present invention on the enzyme activities of PARP1 and PARP2 using ELISA.

Brief Experimental Procedure:

1. histone-coated 384-well plates was prepared: 25 μL of histone solution was added to each well and incubated overnight at 4 °C.
2. PBST buffer, blocking buffer (PBS buffer with 5% BSA and 0.05% Tween-20 added) and detection buffer (20 mM HEPES pH 7.5 + 100 mM NaCl + 2 mM DTT + 0.002% Tween-20 + 0.1% BSA) were prepared.
3. The histone-coated 3 84-well plate was washed three times with PBST buffer, blocked at room temperature with 50 μL blocking buffer for 1 hour, and then washed three times with PBST buffer.
4. Test compounds were prepared as 2000-fold stock solutions in DMSO. 50 nL of the stock solution was mixed with 19.95 μL detection buffer, and 5 μL of the gradient-diluted test compound was transferred to each well.
5. Enzymatic reaction (PARP1): PARP1 & DNA mix was pre-incubated at 25 °C for 10 minutes. 10 μL of the PARP1 & DNA mix was added to each well and incubated with the test compound at room temperature for 10 minutes. Then, 10 μL of 2.5× $NAD^+$ was added to each well, followed by incubation at 25 °C for 60 minutes. Finally, the 384-well plate was washed three times with PBST buffer.
Enzymatic reaction (PARP2): PARP2 was pre-incubated at 25 °C for 10 minutes. 10 μL of PARP2 was added to each well and incubated with the test compound at room temperature for 10 minutes. Then, 10 μL of 2.5× Biotin-$NAD^+$ was added to each well, followed by incubation at 25 °C for 90 minutes. Finally, the 384-well plate was washed three times with PBST buffer.
6. Detection (PARP1): 20 μL of anti-Poly/Mono-ADP Ribose Rabbit mAb was added and incubated at room temperature for 1.5 hours, followed by three washes of the 384-well plate with PBST buffer. Then, 20 μL of diluted anti-rabbit IgG, HRP-linked Antibody (diluted 1:2000 in blocking buffer) was added and incubated at room temperature for 1 hour. After washing the plate three times with PBST buffer, 25 μL of a 1:1 mixture of Femto-ECL Substrate A and Femto-ECL Substrate B was added.
Detection (PARP2): 25 μL of Strep-HRP was added and incubated at room temperature for 1 hour, followed by three washes of the 384-well plate with PBS buffer. Then, 25 μL of a 1:1 mixture of Femto-ECL Substrate A and Femto-ECL Substrate B was added.
7. Chemiluminescence intensity was measured using an Envision multimode microplate reader.
8. Data Processing

The inhibition rate was calculated in Excel using formula (1).

$$\text{Formula (1): inh \%} = (\text{Max - Signal}) / (\text{Max - Min}) * 100$$

The $IC_{50}$ value was obtained by fitting the data in XL-Fit using formula (2).

$$\text{Formula (2): } Y = \text{bottom} + (\text{top - bottom}) / (1 + (IC_{50} / X) * \text{HillSlope});$$

wherein Y is the inhibition percentage and X is the compound concentration.
Olaparib and AZD5305 were used as positive controls in this experiment to ensure the experimental system was functioning correctly. Experimental results show that the $IC_{50}$ of most compounds of the present invention for PARP1 is <10 nM, preferably <5 nM, and more preferably <1 nM; the selectivity of most compounds of the present invention for PARP2/PARP1 is >100, preferably >500, more preferably >1000, and particularly preferably >2000. The experimental results are shown in Table A. The experimental results indicate that the compounds of the present invention have strong inhibitory effects on PARP1 enzyme activity, and most compounds of the present invention have significantly higher selectivity for PARP1 than for PARP2.

Table A: Results of Inhibitory Effects of Compounds of the Present Invention on PARP1 and PARP2 Enzyme Activity

| **Example No.** | **$IC_{50}$ (nM)** | | **Selectivity** |
|---|---|---|---|
| | PARP1 | PARP2 | PARP2/PARP1 |
| Example 2 | 1.5 | 3127 | 2084.7 |
| Example 3 | 2.67 | 142 | 53.2 |
| Example 4 | 1.84 | 1782.1 | 968.5 |
| Example 5 | 0.8043 | 370.8 | 461.0 |
| Example 6 | 1.199 | 839.4 | 700.1 |
| Example 7 | 1.303 | 471.8 | 362.1 |
| Example 8 | 4.1 | 2694.7 | 657.2 |
| Example 9 | 1.612 | 1137 | 705.3 |
| Example 10 | 3.61 | 687.5 | 190.4 |
| Example 11 | 3.011 | 676.2 | 224.6 |
| Example 13 | 0.7129 | 182.9 | 256.6 |
| Example 14 | 27.7 | 9656.8 | 348.3 |
| Example 15 | 0.6 | 137.0 | 248.4 |
| Example 16 | 2.8 | >10000 | >3625 |
| Example 17 | 3.6 | 169 | 46.9 |
| Example 18 | 1.7 | 93.3 | 54.7 |
| Example 19 | 0.7 | 70.0 | 107.3 |
| Example 20 | 0.6 | 68.9 | 124.8 |
| Example 21 | 6.7 | >10000 | >1480 |
| Example 22 | 5.5 | >10000 | 1813.7 |

(continued)

| Example No. | IC$_{50}$ (nM) | | Selectivity |
|---|---|---|---|
| | PARP1 | PARP2 | PARP2/PARP1 |
| Example 23 | 2.4 | >10000 | 4113.1 |
| Example 24 | 10.0 | >10000 | 1004.6 |
| Example 25 | 0.7 | 182.9 | 256.6 |
| Example 26 | 2.1 | 192.0 | 91.5 |
| Example 27 | 2.2 | 626.3 | 278.9 |
| Example 28 | 1.8 | 653.9 | 356.3 |
| Example 29 | 11.5 | 2499.0 | 217.3 |
| Example 31 | 2.5 | 45.4 | 18.4 |
| Example 32 | 2.8 | >10000 | >833 |
| Example 33 | 1.2 | 234.1 | 188.8 |
| Example 34 | 2.8 | 1022.0 | 359.1 |
| Example 35 | 2.0 | 90.2 | 44.3 |
| Example 36 | 3.0 | 412.8 | 139.8 |
| Example 37 | 12.9 | 1905.8 | 148.2 |
| Example 38 | 4.4 | 499.0 | 112.0 |
| Example 39 | 2.9 | 599.0 | 206.8 |
| Example 40 | 5.7 | >10000 | >1756.9 |
| Example 41 | 3.1 | >10000 | 3254.4 |
| Example 42 | 0.9 | 221.0 | 239.7 |
| Example 43 | 1.5 | 583.1 | 398.1 |
| Example 44 | 0.8 | 148.0 | 184.1 |
| Example 45 | 7.7 | >10000 | >1290.7 |
| Example 46 | 2.5 | 64.0 | 25.0 |
| Example 47 | 6.9 | >10000 | >1456.3 |
| Example 48 | 9.8 | >10000 | >1020 |
| Example 49 | 3.2 | 54.0 | 17.0 |
| Example 50 | 3.3 | >10000 | >3042 |
| Example 51 | 7.2 | 1425.0 | 198.0 |
| Example 52 | 2.7 | 235.0 | 87.0 |
| Example 54 | 7.6 | 203.0 | 26.7 |
| Example 55 | 1.9 | 14.3 | 7.6 |
| Example 56 | 4.2 | 38.3 | 9.1 |
| Example 57 | 1.7 | 79.5 | 47.3 |
| Example 58 | 1.1 | 2477.0 | 2192.4 |
| Example 59 | 1.9 | 1221.6 | 650.4 |
| Example 60 | 2.9 | 5823.0 | 2030.7 |
| Example 61 | 1.8 | 619.0 | 345.0 |
| Example 62 | 6.4 | >10000 | >1554 |

(continued)

| Example No. | $IC_{50}$ (nM) | | Selectivity |
|---|---|---|---|
| | PARP1 | PARP2 | PARP2/PARP1 |
| Example 63 | 3.2 | 1279.0 | 398.8 |
| Example 64 | 3.6 | 169 | 46.9 |
| Example 65 | 1.9 | 80 | 42.1 |
| Example 66 | 2.4 | 143 | 59.6 |
| Example 67 | 6.9 | >10000 | >1444 |
| Example 68 | 6.2 | 6492.0 | 1045.1 |
| Example 69 | 3.2 | 8822.0 | 2751.7 |
| Example 70 | 3.6 | 254.5 | 70.9 |
| Example 71 | 1.5 | 1287.0 | 873.7 |
| Example 72 | 1.3 | 992.3 | 756.9 |
| Example 73 | 5.7 | 2682.0 | 472.0 |

**Example B: Evaluation of the Inhibitory Effect of the Compounds of the Present Invention on the Proliferation of DLD-1 BRCA2(-/-) Cells**

Experimental Objective: To evaluate the inhibitory effect of the compounds of the present invention on the proliferation of DLD-1 BRCA2(-/-) cells using the CTG method.

Brief Experimental Procedure:

1. Cell Pretreatment: DLD-1 BRCA2(-/-) cells were cultured in growth medium 1640 + 10% FBS (fetal bovine serum) + 1% Penicillin-Streptomycin. Cells in the logarithmic growth phase were digested with trypsin-EDTA, centrifuged, and resuspended. DLD-1 BRCA2(-/-) cells were seeded at 1,000 cells/well in sterile 96-well opaque plates, 75 μl per well. After seeding, the cell culture plates were incubated overnight at 37 °C with 5% $CO_2$ before adhesion, followed by treatment with the test compounds.
2. DLD-1 BRCA2(-/-) Cell Drug Treatment: The test compound was prepared as a 10 mM stock solution using DMSO. The test compound was then diluted to a 4× working solution using growth medium (starting at 40 μM, with 9 4-fold dilutions). 25 μl of 4× working solution was added to each well to achieve final concentrations of 10000, 2500, 625, 156, 39, 9.7, 2.4, 0.61, and 0.152 nM, respectively. Blank control wells containing the same DMSO concentration were also included, with three replicates for each concentration. After adding the test compound, the cells were incubated at 37 °C with 5% $CO_2$ for 7 days.
3. After incubation, 100 μl of CellTiter-Glo® Reagent was added to each well, and fluorescence intensity was measured. A curve was fitted using GraphPad software, and the $IC_{50}$ value for cell proliferation inhibition was calculated. The experimental results are shown in Table B.

Table B: Results of the assay of the inhibitory effect of the compounds of the present invention on the proliferation of DLD-1 BRCA2(-/-) cells.

| Example No. | DLD-1BRCA2(-/-) $IC_{50}$ (nM) | Example No. | DLD-1BRCA2(-/-) $IC_{50}$ (nM) |
|---|---|---|---|
| Example 2 | 22.9 | Example 43 | 27.0 |
| Example 3 | 4.6 | Example 44 | 9.3 |
| Example 4 | 4.7 | Example 45 | 12 |
| Example 5 | 6.1 | Example 46 | 3.2 |
| Example 6 | 14.5 | Example 47 | 18.6 |

(continued)

| Example No. | DLD-1BRCA2(-/-) $IC_{50}$ (nM) | Example No. | DLD-1BRCA2(-/-) $IC_{50}$ (nM) |
|---|---|---|---|
| Example 7 | 2.5 | Example 48 | 23.6 |
| Example 8 | 32.2 | Example 49 | 12.9 |
| Example 9 | 11.1 | Example 52 | 2.2 |
| Example 10 | 7.5 | Example 53 | 963 |
| Example 11 | 12.9 | Example 54 | 18.4 |
| Example 12 | 1.9 | Example 55 | 3.9 |
| Example 13 | 1.3 | Example 56 | 8.3 |
| Example 14 | 9.3 | Example 57 | 2.9 |
| Example 15 | 4.1 | Example 58 | 13.6 |
| Example 18 | 7.9 | Example 59 | 8.0 |
| Example 19 | 1.5 | Example 60 | 8.58 |
| Example 20 | 2.4 | Example 61 | 8.59 |
| Example 21 | 22.2 | Example 63 | 16.1 |
| Example 22 | 31.8 | Example 64 | 9.8 |
| Example 25 | 1.3 | Example 65 | 4.27 |
| Example 32 | 68.8 | Example 66 | 7.02 |
| Example 33 | 9.3 | Example 68 | 33.3 |
| Example 35 | 13.0 | Example 69 | 32.6 |
| Example 38 | 15.1 | Example 70 | 14.2 |
| Example 39 | 7.7 | Example 71 | 12.3 |
| Example 41 | 37.4 | Example 72 | 9.1 |
| Example 42 | 7.0 | | |

Experimental results showed that the compound of the present invention exhibited strong inhibitory activity against the proliferation of DLD-1 BRCA2(-/-) cells.

**Example C: Evaluation of the stability of the compound of the present invention in human and rat liver microsomes**

Experimental objective: To evaluate the stability of the compound of the present invention in human and rat liver microsomes using LC-MS/MS.

Brief experimental procedure: The test compound and human or rat liver microsomes were co-incubated in 0.1 M potassium phosphate buffer (pH = 7.4 ± 0.1) at 37 °C. The concentration of the test compound at different incubation times was measured. The half-life of the compound was calculated by plotting "relative concentration of compound" against "incubation time" in GraphPad Prism 5.01, and the intrinsic clearance rate was calculated. See Table 1 for the experimental system:

Table 1: Experimental System

| Test Substances | The compound of this invention (dissolved in DMSO and diluted with acetonitrile) |
|---|---|
| Rat liver microsomes (purchased from BD Biosciences) | Mixed individuals, final test concentration: 0.5 mg/mL |
| Human liver microsomes (purchased from BD Biosciences) | Mixed individuals, final test concentration: 0.5 mg/mL |

(continued)

| | |
|---|---|
| Buffer Solution | 0.1 M potassium phosphate buffer (pH = 7.4 ± 0.1) |
| Final Test Concentration of Test Substance | 1 μM |
| Final Organic Solvent Content | 0.1% |
| Final Reaction System | 30 μL buffer solution containing the compound of this invention and human or rat liver microsomes;<br>15 μL NADPH buffer solution (6 mM concentration). |
| Test Conditions | Time points: 0 min, 15 min, 30 min, 60 min<br>Temperature: 37 °C<br>pH: 7.4 ± 0.1 |
| Number of Duplicates | 2 |
| Analytical Method | LC/MS/MS, internal standard: propranolol |
| LC/MS/MS analysis was used to determine the ratio of sample peak area to internal standard peak area. The compound content at 0 min was considered 100%, and the relative content of the compound at each time point was calculated. The half-life of the compound was calculated by plotting the relative content against the incubation time, and the intrinsic clearance rate was also calculated. | |

Experimental results show that the compound of this invention has a longer half-life and a lower clearance rate. Therefore, the compound of this invention exhibits higher stability in human liver microsomes.

**Example D: Pharmacokinetic Evaluation of the Compounds of the Invention by Intravenous Injection or Gavage in Rats, Mice, Dogs, and Monkeys**

The inventors conducted pharmacokinetic evaluations of the compounds of the present invention in rats, mice, dogs, and monkeys. Animal information is detailed in Table 2.

Table 2: Information on Test Animals of the Invention

| Strain | Grade | Sex | Weight | Age | Source |
|---|---|---|---|---|---|
| SD Rats | SPF | Male | 180-350 g | 6-11 weeks | Hunan SJA Laboratory Animal Co., Ltd |
| BALB/c Mice | SPF | Male | 18-30 g | 6-8 weeks | Hunan SJA Laboratory Animal Co., Ltd. |
| Beagle Dogs | Standard Grade | Male | 7~ 12 kg | 6-12 months | Beijing Marshall Biotechnology Co., Ltd. |
| Cynomolgus Monkey | Standard Grade | Male | 2.5~ 6.0 kg | 3-6 years | Guangzhou Huazhen Biotechnology Co., Ltd. |

Test method

The compounds of the present invention were administered to test animals in the form of 5% DMSO + 30% PEG400 + 65% physiological saline, 10% DMSO + 10% Kolliphor HS15 + 80% Saline, 10% DMSO + 89% (25% SBE-B-CD) + (2% HCl), 20% PEG400 + 80% sterile water for injection, or 10% DMA+ 10% HS15 + 30% PEG400 + 50% sterile aqueous solution for injection. Animals were fasted for 12 hours before administration but had free access to water. For the intravenous administration group, the dose was 1 mg/kg. Blood samples (approximately 0.15 mL) were collected intravenously at the following time points after administration: 0.083, 0.25, 0.5, 1.0, 2.0, 4.0, 6.0, 8.0 and 24 h (dogs) or 0.083, 0.25, 0.5, 1.0, 2.0, 5.0, 7.0 and 24 h (mice and rats) or 0.083, 0.25, 0.5, 1.0, 2.0, 6.0, 8.0 and 24 h (monkeys). EDTA-K2 was pre-added to the blood collection tube as an anticoagulant. The blood samples were centrifuged at 12,000 rpm for 2 minutes, and plasma was collected and stored at -20 °C or - 70 °C. For the gavage administration group, the dosage was 1 mg/kg (mice, dogs, and monkeys) or 5 mg/kg (rats). After administration, venous blood samples (approximately 0.15 mL) were collected at the following time points: 0.25, 0.5, 1.0, 2.0, 4.0, 6.0, 8.0, and 24 h (dogs) or 0.25, 0.5, 1.0, 2.0, 5.0, 7.0, and 24 h (mice and rats) or 0.25, 0.5, 1.0, 2.0, 6.0, 8.0, and 24 h (monkeys). EDTA-K2 was

pre-added to the blood collection tube as an anticoagulant. The blood samples were centrifuged at 12,000 rpm for 2 minutes, and plasma was collected and stored at -20 °C or -70 °C.

The collected plasma samples were processed (frozen plasma was thawed at room temperature, vortexed for 15 s to mix, 10-20 μL of plasma was taken, 120-150 μL of acetonitrile solution containing internal standard was added, vortexed for 5 min to mix, centrifuged at 4,000 rpm for 5 min, 100 μL of supernatant was taken, and 120-150 Ml/water (v/v = 1/1) was added and mixed). The concentration of compounds in the plasma was then analyzed by LC-MS/MS. The results showed that the compounds of this invention exhibited good pharmacokinetic properties in rats, mice, dogs, and monkeys. This indicates that the compounds of this invention have better drug-like properties and better clinical application prospects. In some embodiments, the pharmacokinetic data of some compounds of this invention in mice are shown in Table C below.

Table C: Pharmacokinetic Parameters of Compounds of this Invention in Mice

| Example No. | Administration Route | Dosage (mg/kg) | $T_{1/2}$ (h) | $C_{max}$ (ng/mL) | $AUC_{last}$ (h*ng/mL) |
|---|---|---|---|---|---|
| Example 2 | ig | 5 | 3.9 | 1450 | 8560 |
| | iv | 2 | 3.1 | 942 | 2870 |
| Example 3 | ig | 5 | 2.4 | 1780 | 4890 |
| | iv | 2 | 1.9 | 1220 | 2140 |
| Example 7 | ig | 5 | 1.8 | 3230 | 6370 |
| | iv | 2 | 1.6 | 1300 | 2140 |
| Example 12 | ig | 5 | 1.9 | 2110 | 4200 |
| | iv | 2 | 1.4 | 1630 | 1430 |
| Example 13 | ig | 5 | 2.9 | 11500 | 33900 |
| | iv | 2 | 1.9 | 6560 | 17000 |
| Example 14 | ig | 5 | 3.3 | 3830 | 16200 |
| | iv | 2 | 3.3 | 2080 | 4680 |
| Example 15 | ig | 5 | 4.2 | 19000 | 189000 |
| | iv | 2 | 3.8 | 13600 | 75300 |
| Example 35 | ig | 5 | 1.5 | 2760 | 4860 |
| | iv | 2 | 1.2 | 3750 | 2750 |
| Example 41 | ig | 5 | 1.9 | 2720 | 4630 |
| | iv | 2 | 1.3 | 3150 | 2230 |
| Example 42 | ig | 5 | 2.5 | 15100 | 61500 |
| | iv | 2 | 2.5 | 8180 | 18100 |
| Example 46 | ig | 5 | 2 | 8750 | 25300 |
| | iv | 2 | 1.6 | 6460 | 11300 |
| Example 52 | ig | 5 | 2.8 | 10700 | 67100 |
| | iv | 2 | 2.5 | 7220 | 17800 |

In some embodiments, the pharmacokinetic data of certain compounds of the present invention in SD rats are shown in Table D below.

Table D Pharmacokinetic parameters of compounds of the present invention in SD rats

| Example No. | Administration Route | Dosage (mg/kg) | $T_{1/2}$ (h) | $C_{max}$ (ng/mL) | $AUC_{last}$ (h*ng/mL) |
|---|---|---|---|---|---|
| Example 2 | ig | 5 | 3.4 | 719 | 5610 |
| | iv | 1 | 2.1 | 534 | 1090 |

(continued)

| Example No. | Administration Route | Dosage (mg/kg) | $T_{1/2}$ (h) | $C_{max}$ (ng/mL) | $AUC_{last}$ (h*ng/mL) |
|---|---|---|---|---|---|
| Example 3 | ig | 5 | 3.7 | 1400 | 13100 |
| | iv | 1 | 3.4 | 653 | 1990 |
| Example 7 | ig | 5 | 3.3 | 2600 | 22400 |
| | iv | 1 | 3.4 | 1170 | 3370 |
| Example 12 | ig | 5 | 2.4 | 922 | 3770 |
| | iv | 1 | 2 | 897 | 1260 |
| Example 13 | ig | 5 | 4.4 | 5810 | 57900 |
| | iv | 1 | 3.2 | 2730 | 7720 |
| Example 15 | ig | 5 | 4.5 | 3630 | 31600 |
| | iv | 1 | 4.1 | 1560 | 6060 |
| Example 35 | ig | 5 | 2.8 | 4970 | 48300 |
| | iv | 1 | 2.4 | 2230 | 5680 |
| Example 41 | ig | 5 | 2.8 | 3130 | 7870 |
| | iv | 1 | 2.1 | 1720 | 1670 |
| Example 46 | ig | 5 | 3.4 | 2150 | 21200 |
| | iv | 1 | 3.2 | 1410 | 4500 |
| Example 52 | ig | 5 | 3.8 | 2580 | 31100 |
| | iv | 1 | 3.5 | 1190 | 3520 |

**Example E: Evaluation of the Inhibitory Effect of the Compounds of the Present Invention on the Proliferation of MDA-MB-436 Cells**

Experimental Objective: To evaluate the inhibitory effect of the compounds of the present invention on the proliferation of MDA-MB-436 cells.

Brief Experimental Procedure:

1. Cell Pretreatment: MDA-MB-436 cells were cultured in DMEM + 10% FBS + 1% penicillin-dip antibiotics + 1% ITS + 16 μg/ml glutathione. Cells in the logarithmic growth phase were digested with trypsin-EDTA, centrifuged and resuspended, and then seeded at a rate of 500-1000 cells/well in sterile 96-well opaque plates, 90 μl per well. After seeding, the cell culture plates were incubated overnight at 37°C with 5% $CO_2$ before drug treatment.
2. Drug treatment of MDA-MB-436 cells: The test compound was prepared as a 10 mM stock solution using DMSO. The drug was then diluted to a 10× working solution using growth medium (the compound was started at 100 μM and diluted 4-fold to achieve 9 concentrations). 10 μl of the 10× working solution was added to each well to achieve final drug concentrations of 10000, 2500, 625, 156, 39, 9.7, 2.4, 0.61, and 0.152 nM, respectively. Blank control wells containing the same DMSO concentration were also included, with three replicates for each concentration. After drug addition, the cells were incubated at 37°C with 5% $CO_2$ for 7 days.
3. After incubation, 100 μl of CellTiter-Glo® Reagent was added to each well, and luminescence was measured. The $IC_{50}$ value of cell proliferation inhibition was calculated using GraphPad software to fit the curve.

Table E: Results of the inhibitory effect of the compound of this invention on the proliferation of MDA-MB-436 cells.

| Example No. | MDA-MB-436 $IC_{50}$ (nM) | Example No. | MDA-MB-436 $IC_{50}$ (nM) |
|---|---|---|---|
| Example 2 | 20.2 | Example 39 | <0.038 |

(continued)

| Example No. | MDA-MB-436 $IC_{50}$ (nM) | Example No. | MDA-MB-436 $IC_{50}$ (nM) |
|---|---|---|---|
| Example 3 | 7.5 | Example 41 | 11.8 |
| Example 4 | 5.3 | Example 42 | 0.3 |
| Example 5 | <0.038 | Example 43 | 8.0 |
| Example 6 | 6.8 | Example 44 | 1.3 |
| Example 7 | 10.7 | Example 45 | 11.1 |
| Example 8 | 23.6 | Example 46 | 0.2 |
| Example 9 | 8.1 | Example 47 | 0.6 |
| Example 10 | 1.7 | Example 49 | 9.1 |
| Example 11 | 10.7 | Example 52 | 1.4 |
| Example 12 | 0.6 | Example 53 | 3935 |
| Example 13 | 3.5 | Example 54 | 28.7 |
| Example 15 | 1.3 | Example 55 | <0.038 |
| Example 16 | 6.2 | Example 56 | 14.7 |
| Example 18 | 0.9 | Example 57 | 1.7 |
| Example 19 | 4.2 | Example 59 | 26.3 |
| Example 20 | 3.3 | Example 60 | 31.2 |
| Example 21 | 22.9 | Example 61 | 20.3 |
| Example 25 | 3.5 | Example 63 | 6.2 |
| Example 32 | 40.1 | Example 68 | 35.6 |
| Example 33 | 4.1 | Example 70 | 13.9 |
| Example 35 | 0.2 | Example 71 | 9.3 |
| Example 38 | 5.0 | Example 72 | 12 |

Experimental results show that the compound of this invention has a strong inhibitory effect on the proliferation of MDA-MB-436 cells.

In the description of this specification, the references to terms such as "an embodiment," "an implementation," "some embodiments," "example," "specific example," or "some examples," etc., indicate that a specific feature, structure, material, or characteristic described in connection with that embodiment, implementation, or example is included in at least one embodiment, implementation, or example of the present invention. In this specification, the illustrative expressions of the above terms do not necessarily refer to the same embodiment, implementation, or example. Furthermore, the specific features, structures, materials, or characteristics described may be combined in any suitable manner in one or more embodiments, implementations, or examples. Moreover, without contradiction, those skilled in the art can combine and integrate different embodiments, implementations, or examples described in this specification, as well as features of different embodiments, implementations, or examples.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A compound, which is a compound of formula (I), or a stereoisomer, tautomer, N-oxide, hydrate, solvate, metabolite, pharmaceutically acceptable salt or prodrug thereof,

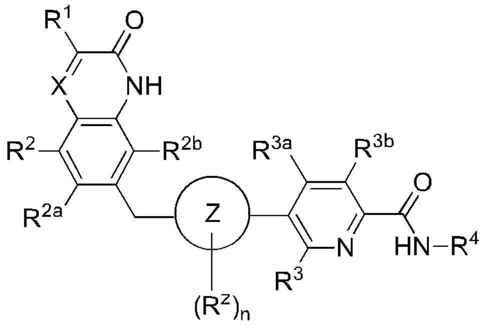

(I),

wherein

X is $CR^x$ or N;

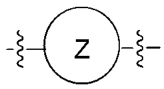

is 3-12 membered heterocyclyl;

$R^1$ is H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, $-C(=O)-(C_1-C_6$ alkyl), $-C(=O)-(C_1-C_6$ alkoxy), $C_1-C_6$ alkyl, $C_2-C_6$ alkenyl, $C_2-C_6$ alkynyl, $C_1-C_6$ haloalkyl, $C_1-C_6$ alkoxy, $C_1-C_6$ haloalkoxy, $C_1-C_6$ alkylthio, $C_1-C_6$ alkylamino, $C_1-C_6$ hydroxyalkyl, $C_3-C_8$ cycloalkyl or 3-8 membered heterocyclyl;

each of $R^{2a}$ and $R^{2b}$ is independently H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, $-C(=O)-(C_1-C_6$ alkyl), $-C(=O)-(C_1-C_6$ alkoxy), $C_1-C_6$ alkyl, $C_2-C_6$ alkenyl, $C_2-C_6$ alkynyl, $C_1-C_6$ haloalkyl, $C_1-C_6$ alkoxy, $C_1-C_6$ haloalkoxy, $C_1-C_6$ alkylthio, $C_1-C_6$ alkylamino, $C_1-C_6$ hydroxyalkyl, $C_1-C_6$ cyanoalkyl, $C_3-C_8$ cycloalkyl, 3-8 membered heterocyclyl, $C_6-C_{10}$ aryl or 5-10 membered heteroaryl;

$R^2$ is -CN, $-NO_2$, $-NH_2$, -OH, -SH, -COOH, $-C(=O)NH_2$, $-C(=O)NHCH_3$, $-C(=O)N(CH_3)_2$, $-C(=O)-(C_1-C_6$ alkyl), $-C(=O)-(C_1-C_6$ alkoxy), $C_1-C_6$ alkyl, $C_2-C_6$ alkenyl, $C_2-C_6$ alkynyl, $C_1-C_6$ alkoxy, $C_1-C_6$ haloalkoxy, $C_1-C_6$ alkylthio, $C_1-C_6$ alkylamino, $C_1-C_6$ hydroxyalkyl, $C_1-C_6$ cyanoalkyl, $C_3-C_8$ cycloalkyl-L-, 3-8 membered heterocyclyl-L-, $C_6-C_{10}$ aryl-L- or 5-10 membered heteroaryl-L-; wherein, $R^2$ is unsubstituted or substituted by 1, 2, 3, 4 or 5 $R^w$;

each -L- is independently a bond, $-NR^n-$, -O-, -S-, -C(=O)-, -C(=O)O-, $-C(=O)N(R^{n1})-$ or $-(CR^aR^b)_m-$;

m is 1, 2, 3, 4, 5 or 6;

each $R^n$ and $R^{n1}$ is independently H, D, $C_1-C_6$ alkyl, $C_1-C_6$ haloalkyl, $C_3-C_8$ cycloalkyl, 3-8 membered heterocyclyl, $C_6-C_{10}$ aryl or 5-10 membered heteroaryl;

each of $R^a$ and $R^b$ is independently H, D, F, Cl, Br, I, -CN, $-NO_2$, $-NH_2$, -OH, -SH, $C_1-C_6$ alkyl, $C_2-C_6$ alkenyl, $C_2-C_6$ alkynyl, $C_1-C_6$ haloalkyl, $C_1-C_6$ alkoxy, $C_1-C_6$ haloalkoxy, $C_1-C_6$ alkylthio, $C_1-C_6$ alkylamino or $C_1-C_6$ hydroxyalkyl;

each of $R^3$, $R^{3a}$ and $R^{3b}$ is independently H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, $-C(=O)-(C_1-C_6$ alkyl), $-C(=O)-(C_1-C_6$ alkoxy), $C_1-C_6$ alkyl, $C_2-C_6$ alkenyl, $C_2-C_6$ alkynyl, $C_1-C_6$ haloalkyl, $C_1-C_6$ alkoxy, $C_1-C_6$ haloalkoxy, $C_1-C_6$ alkylthio, $C_1-C_6$ alkylamino or $C_1-C_6$ hydroxyalkyl;

$R^4$ is H, D, $C_1-C_6$ alkyl, $C_2-C_6$ alkenyl, $C_2-C_6$ alkynyl, $C_1-C_6$ haloalkyl, $C_1-C_6$ alkoxy, $C_1-C_6$ haloalkoxy, $C_1-C_6$ alkylthio, $C_1-C_6$ alkylamino, $C_3-C_8$ cycloalkyl, 3-8 membered heterocyclyl, $C_6-C_{10}$ aryl or 5-10 membered heteroaryl, wherein each of the $C_1-C_6$ alkyl, $C_3-C_8$ cycloalkyl, 3-8 membered heterocyclyl, $C_6-C_{10}$ aryl and 5-10 membered heteroaryl is independently and optionally substituted by 1, 2, 3, 4 or 5 groups selected from D, F, Cl, Br, I, -OH, $-NH_2$, $-NO_2$, -CN, $C_1-C_6$ alkyl, $C_1-C_6$ haloalkyl, $C_1-C_6$ alkoxy and $C_1-C_6$ haloalkoxy;

each of $R^x$ and $R^z$ is independently H, D, F, Cl, Br, I, -CN, $-NO_2$, $-NH_2$, -OH, -COOH, $-C(=O)NH_2$, $C_1-C_6$ alkyl, $C_1-C_6$ haloalkyl, $C_1-C_6$ alkoxy, $C_1-C_6$ haloalkoxy or $C_1-C_6$ hydroxyalkyl;

each $R^w$ is independently H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, $-C(=O)-(C_1-C_6$ alkyl), $-C(=O)-(C_1-C_6$ alkoxy), $C_1-C_6$ alkyl, $C_2-C_6$ alkenyl, $C_2-C_6$ alkynyl, $C_1-C_6$ haloalkyl, $C_1-C_6$ alkoxy, $C_1-C_6$ haloalkoxy, $C_1-C_6$ alkylthio, $C_1-C_6$ alkylamino, $C_1-C_6$ hydroxyalkyl, $C_3-C_8$ cycloalkyl, 3-8 membered heterocyclyl, $C_6-C_{10}$ aryl or 5-10 membered heteroaryl;

n is 1, 2, 3, 4, 5 or 6;

wherein, the compounds represented by formula (I) do not include the following compounds:

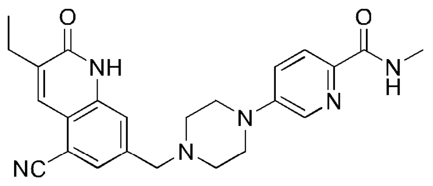
,
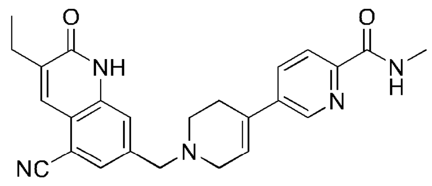
,
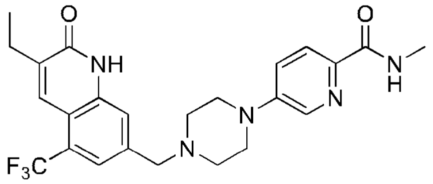
,
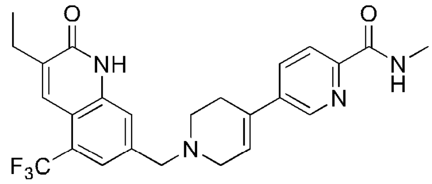
,
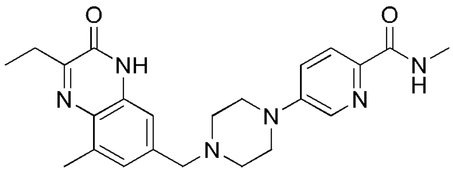
,
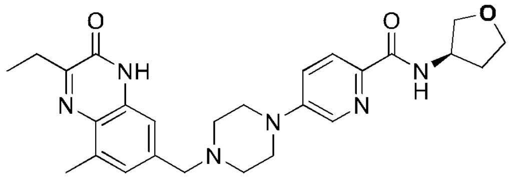
,
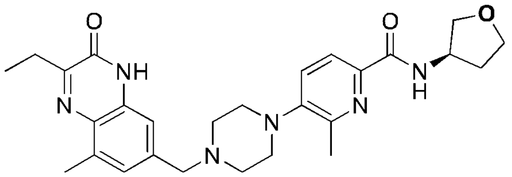
,
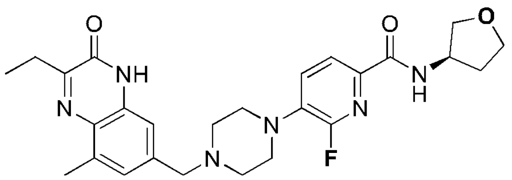
,
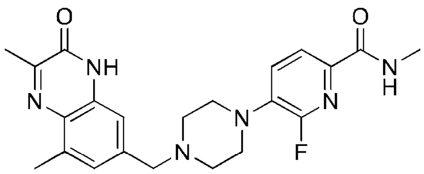
,
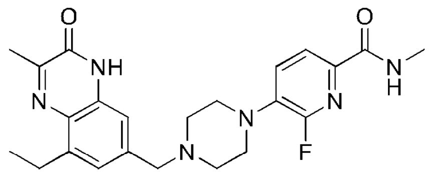
,
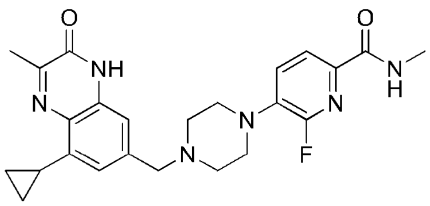
,
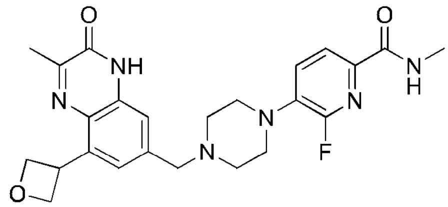
,
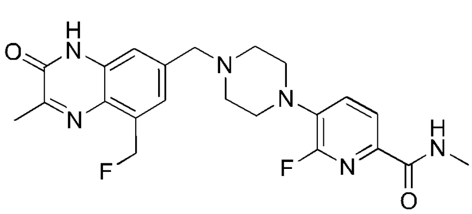
,
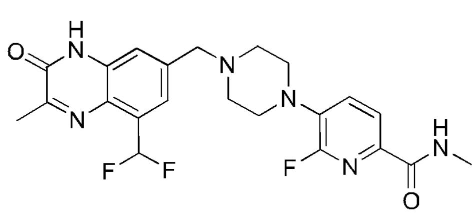
,

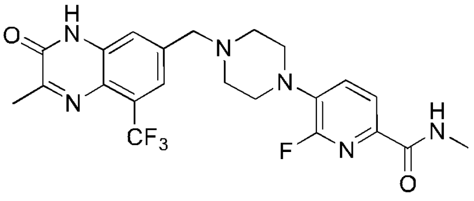

,

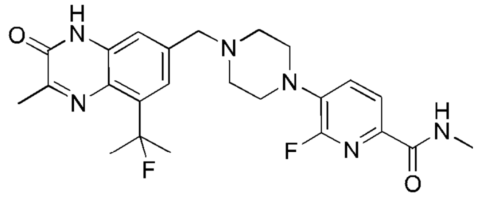

,

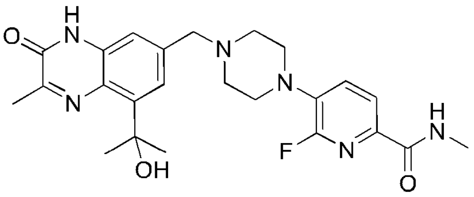

,

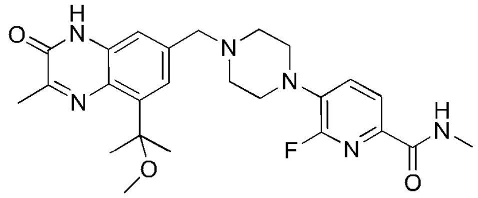

,

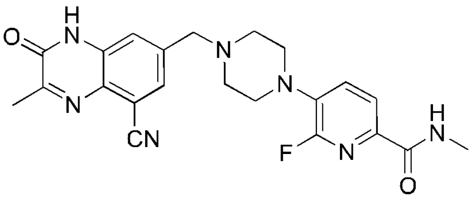

,

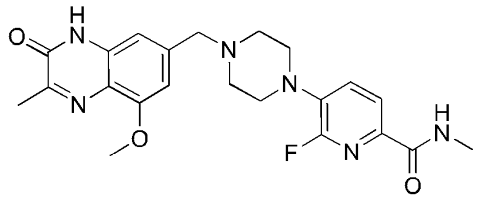

,

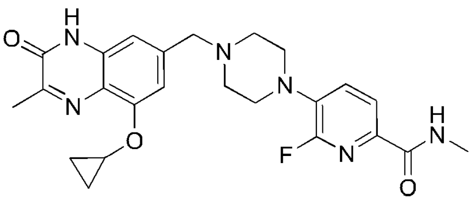

,

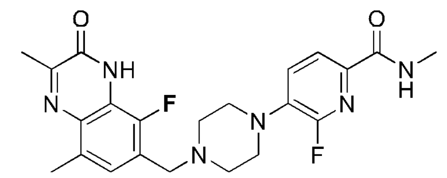

,

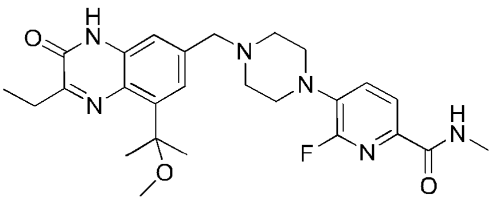

,

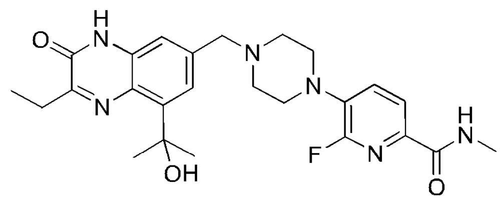

,

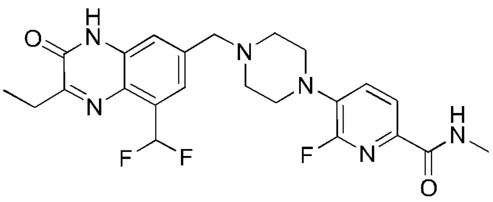

,

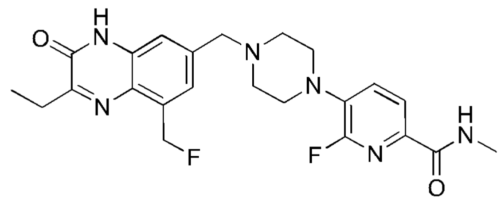

,

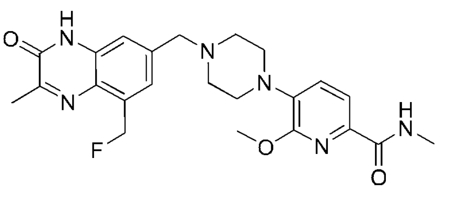

,

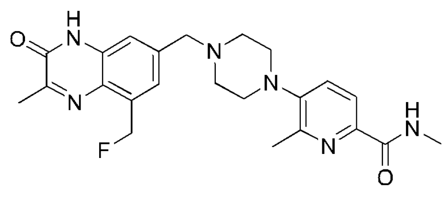

,

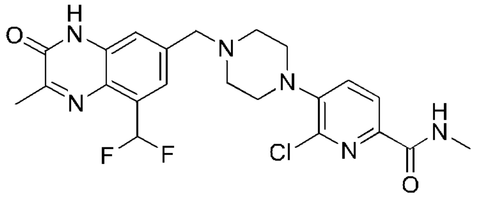

,

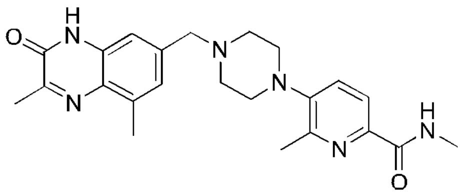

and

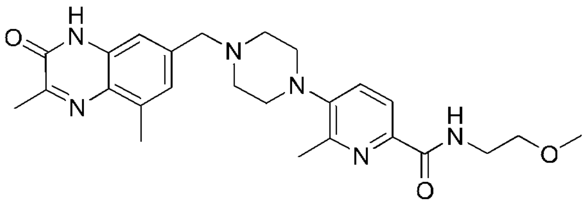

.

**2.** The compound according to claim 1, wherein

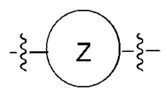

is

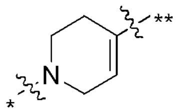

,

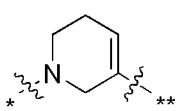

,

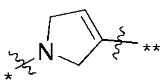

,

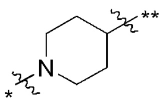

,

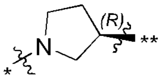

,

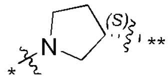

,

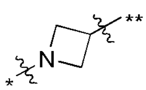

,

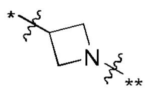

,

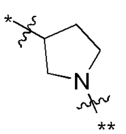

,

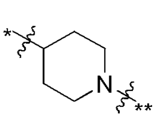

,

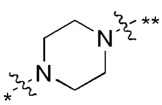

,

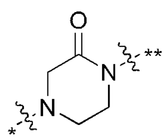

,

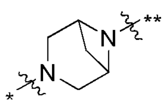

,

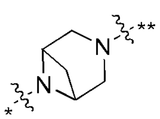

,

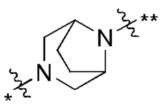

,

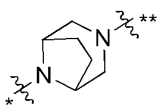

,

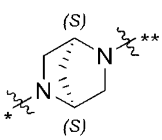

,

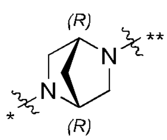

,

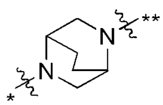

,

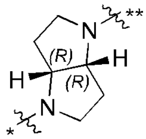

,

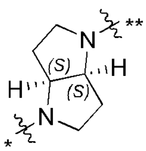

,

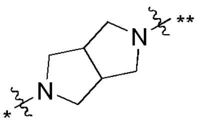

,

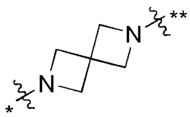

,

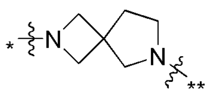

,

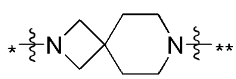

,

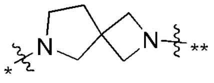 , 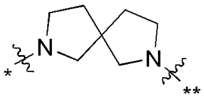 , 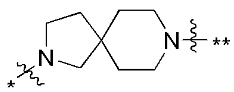 , 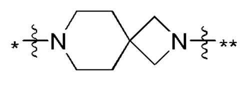 ,

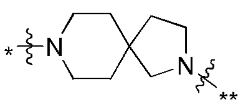 , 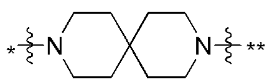 , 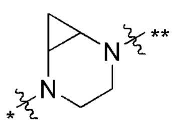 , 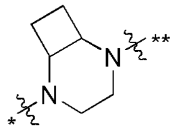 , 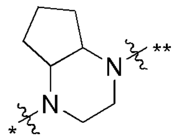 ,

or

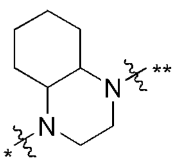 ;

wherein * indicates the connection of the $-CH_2-$ on the left and ** indicates the connection of the pyridinyl on the right.

**3.** The compound according to claim 1 or 2, wherein $R^1$ is H, D, F, Cl, Br, I, -CN, $-NO_2$, $-NH_2$, -OH, -SH, -COOH, -C(=O)$NH_2$, -C(=O)$NHCH_3$, -C(=O)$N(CH_3)_2$, -C(=O)-($C_1$-$C_4$ alkyl), -C(=O)-($C_1$-$C_4$ alkoxy), $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino, $C_1$-$C_4$ hydroxyalkyl, $C_3$-$C_6$ cycloalkyl or 3-6 membered heterocyclyl;

each of $R^3$, $R^{3a}$ and $R^{3b}$ is independently H, D, F, Cl, Br, I, -CN, $-NO_2$, $-NH_2$, -OH, -SH, -COOH, -C(=O)$NH_2$, -C(=O)$NHCH_3$, -C(=O)$N(CH_3)_2$, -C(=O)-($C_1$-$C_4$ alkyl), -C(=O)-($C_1$-$C_4$ alkoxy), $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino or $C_1$-$C_4$ hydroxyalkyl;
each of $R^x$ and $R^z$ is independently H, D, F, Cl, Br, I, -CN, $-NO_2$, $-NH_2$, -OH, -COOH, -C(=O)$NH_2$, $C_1$-$C_4$ alkyl, $C_1$-$C_4$ haloalkyl, $C_1$-$C_4$ alkoxy, $C_1$-$C_4$ haloalkoxy or $C_1$-$C_4$ hydroxyalkyl.

**4.** The compound according to any one of claims 1-3, wherein $R^1$ is H, D, F, Cl, Br, I, -CN, $-NO_2$, $-NH_2$, -OH, -SH, -COOH, -C(=O)$NH_2$, -C(=O)$NHCH_3$, -C(=O)$N(CH_3)_2$, -C(=O)-$CH_3$, -C(=O)-$OCH_3$, methyl, ethyl, n-propyl, isopropyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, n-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, hydroxymethyl, 2-hydroxyethyl, cyclopropyl, cyclobutyl, cyclopentyl, cyclohexyl, aziridine, pyrrolidinyl, tetrahydrofuranyl, piperidinyl, piperazinyl or morpholinyl;

each of $R^3$, $R^{3a}$ and $R^{3b}$ is independently H, D, F, Cl, Br, I, -CN, $-NO_2$, $-NH_2$, -OH, -SH, -COOH, -C(=O)$NH_2$, -C(=O)$NHCH_3$, -C(=O)$N(CH_3)_2$, -C(=O)-$CH_3$, -C(=O)-$OCH_3$, methyl, ethyl, n-propyl, isopropyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, n-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, hydroxymethyl or 2-hydroxyethyl;
each of $R^x$ and $R^z$ is independently H, D, F, Cl, Br, I, -CN, $-NO_2$, $-NH_2$, -OH, -COOH, -C(=O)$NH_2$, methyl, ethyl, n-propyl, isopropyl, $-CHF_2$, $-CF_3$, $-CHFCH_2F$, $-CF_2CHF_2$, $-CH_2CF_3$, $-CH_2CF_2CHF_2$, methoxy, ethoxy, n-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, hydroxymethyl or 2-hydroxyethyl;

**5.** The compound according to any one of claims 1-4, wherein $R^4$ is H, D, $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino, $C_3$-$C_6$ cycloalkyl, 3-6 membered heterocyclyl, $C_6$-$C_{10}$ aryl or 5-6 membered heteroaryl, wherein each of the $C_1$-$C_4$ alkyl, $C_3$-$C_6$ cycloalkyl, 3-6 membered heterocyclyl, $C_6$-$C_{10}$ aryl and 5-6 membered heteroaryl is independently and optionally substituted by 1, 2, 3, 4 or 5 groups selected from D, F, Cl, Br, I, -OH, $-NH_2$, $-NO_2$, -CN, $C_1$-$C_4$ alkyl, $C_1$-$C_4$ haloalkyl, $C_1$-$C_4$ alkoxy and $C_1$-$C_4$ haloalkoxy.

**6.** The compound according to any one of claims 1-5, wherein $R^4$ is H, D, methyl, ethyl, n-propyl, isopropyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, n-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, cyclopropyl, cyclobutyl, cyclopentyl, cyclohexyl, aziridine, oxacyclobutyl, pyrrolyl, tetrahydrofuranyl, tetrahydropyranyl, piperidinyl, piperazine, morpholinyl, phenyl, indanyl, naphthyl, pyrroleyl, pyrazolyl, imidazolyl, triazolyl, tetrazolyl, furanyl, thienyl, thiazolyl, oxazolyl, pyridinyl, pyrimidinyl, pyrazinyl or pyridazinyl, wherein, each of the methyl, ethyl, n-propyl, isopropyl, cyclopropyl, cyclobutyl, cyclopentyl, cyclohexyl, azacyclobutyl, oxacyclobutyl, pyrrolyl, tetrahydrofuranyl, tetrahydropyranyl, piperidinyl, piperazine, morpholinyl, phenyl, indole, naphthyl, pyrrolyl, pyrazolyl, imidazoleyl, triazolyl, tetrazolyl, furanyl, thiophenyl, thiazolyl, oxazolyl, pyridinyl, pyrimidinyl, pyrazinyl and pyridazinyl is independently and optionally substituted by 1, 2, 3, 4 or 5 groups selected from D, F, Cl, Br, I, -OH, $-NH_2$, $-NO_2$, -CN, methyl, ethyl, n-propyl, isopropyl, $-CHF_2$, $-CF_3$, $-CHFCH_2F$, $-CF_2CHF_2$, $-CH_2CF_3$, $-CH_2CF_2CHF_2$, methoxy, ethoxy, n-propyloxy, isopropyloxy, $-OCHF_2$, $-OCF_3$, $-OCHFCH_2F$, $-OCF_2CHF_2$, $-OCH_2CF_3$ and $-OCH_2CF_2CHF_2$.

**7.** The compound according to any one of claims 1-6, wherein each of $R^{2a}$ and $R^{2b}$ is independently H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, -C(=O)-($C_1$-$C_4$ alkyl), -C(=O)-($C_1$-$C_4$ alkoxy), $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino, $C_1$-$C_4$ hydroxyalkyl, $C_1$-$C_4$ cyanoalkyl, $C_3$-$C_6$ cycloalkyl, 3-6 membered heterocyclyl, $C_6$-$C_{10}$ aryl or 5-6 membered heteroaryl;

each of $R^n$ and $R^{n1}$ is independently H, D, $C_1$-$C_4$ alkyl, $C_1$-$C_4$ haloalkyl, $C_3$-$C_6$ cycloalkyl, 3-6 membered heterocyclyl, $C_6$-$C_{10}$ aryl or 5-6 membered heteroaryl;
each of $R^a$ and $R^b$ is independently H, D, F, Cl, Br, I, -CN, $-NO_2$, $-NH_2$, -OH, -SH, $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino or $C_1$-$C_4$ hydroxyalkyl.

**8.** The compound according to any one of claims 1-7, wherein $R^2$ is -CN, $-NO_2$, $-NH_2$, -OH, -SH, -COOH, $-C(=O)NH_2$, $-C(=O)NHCH_3$, $-C(=O)N(CH_3)_2$, -C(=O)-($C_1$-$C_4$ alkyl), -C(=O)-($C_1$-$C_4$ alkoxy), $C_1$-$C_4$ alkyl, $C_2$-$C_4$ alkenyl, $C_2$-$C_4$ alkynyl, $C_1$-$C_4$ alkoxy, $C_1$-$C_4$ haloalkoxy, $C_1$-$C_4$ alkylthio, $C_1$-$C_4$ alkylamino, $C_1$-$C_4$ hydroxyalkyl, $C_1$-$C_4$ cyanoalkyl, $C_3$-$C_6$ cycloalkyl-L-, 3-6 membered heterocyclyl-L-, $C_6$-$C_{10}$ aryl-L- or 5-6 membered heteroaryl-L-; wherein, $R^2$ is unsubstituted or substituted by 1, 2, 3, 4 or 5 $R^w$;
each $R^w$ is independently H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, -C(=O)-($C_1$-$C_4$ alkyl), -C(=O)-($C_1$-$C_4$ alkoxy), $C_1$-$C_4$ alkyl, $C_2$-$C_4$ alkenyl, $C_2$-$C_4$ alkynyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylamino, $C_1$-$C_4$ hydroxyalkyl, $C_3$-$C_6$ cycloalkyl, 3-6 membered heterocyclyl, $C_6$-$C_{10}$ aryl or 5-6 membered heteroaryl.

**9.** The compound according to any one of claims 1-8, wherein each of $R^{2a}$ and $R^{2b}$ is independently H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, -C(=O)-($C_1$-$C_2$ alkyl), -C(=O)-($C_1$-$C_2$ alkoxy), methyl, ethyl, *n*-propyl, isopropyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, *n*-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, hydroxymethyl, 2-hydroxyethyl, cyanomethyl, cyclopropyl, cyclobutyl, cyclopentyl, cyclohexyl, aziridine, pyrrolyl, tetrahydrofuranyl, piperidinyl, piperazine, morpholinyl, phenyl, indole, naphthyl, pyrrolyl, pyrazolyl, imidazolyl, triazolyl, tetrazolyl, furanyl, thiophenyl, thiazolyl, oxazolyl, pyridinyl, pyrimidinyl, pyrazinyl or pyridazinyl;

each of $R^n$ and $R^{n1}$ is independently H, D, methyl, ethyl, n-propyl, isopropyl, n-butyl, tert-butyl, $-CHF_2$, $-CF_3$, $-CHFCH_2F$, $-CF_2CHF_2$, $-CH_2CF_3$, $-CH_2CF_2CHF_2$, cyclopropyl, cyclobutyl, cyclopentyl, cyclohexyl, aziridine, pyrrolyl, tetrahydrofuranyl, piperidinyl, piperazine, morpholinyl, phenyl, pyrrolyl, pyrazolyl, imidazolyl, triazolyl, tetrazolyl, furanyl, thienyl, thiazolyl, oxazolyl, pyridinyl, pyrimidinyl, pyrazinyl or pyridazinyl;
each of $R^a$ and $R^b$ is independently H, D, F, Cl, Br, I, -CN, $-NO_2$, $-NH_2$, -OH, -SH, methyl, ethyl, *n*-propyl, isopropyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, *n*-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, hydroxymethyl or 2-hydroxyethyl.

**10.** The compound according to any one of claims 1-9, wherein

$R^2$ is -CN, $-NO_2$, $-NH_2$, -OH, -SH, -COOH, $-C(=O)NH_2$, $-C(=O)NHCH_3$, $-C(=O)N(CH_3)_2$, $-C(=O)-CH_3$, $-C(=O)-OCH_3$, methyl, ethyl, *n*-propyl, isopropyl, *n*-butyl, *tert*-butyl, allyl, propenyl, propargyl, propynyl, methoxy,

ethoxy, *n*-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, hydroxymethyl, 2-hydroxyethyl, cyanomethyl, cyclopropyl-L-, cyclobutyl-L-, cyclopentyl-L-, cyclohexyl-L-, oxetylpropyl-L-, aziridinepropyl-L-, oxetylbutyl-L-, aziridinebutyl-L-, tetrahydrofuranyl-L-, pyrrolidinyl-L-, tetrahydropyranyl-L-, piperidinyl-L-, piperazinyl-L-, morpholinyl-L-, phenyl-L-, naphthyl-L-, pyrrolidinyl-L-, furanyl-L-, thiophenyl-L-, pyrazolyl-L-, imidazolyl-L-, thiazolyl-L-, oxazolyl-L-, triazolyl-L-, tetrazolyl-L-, pyridinyl-L-, pyrimidinyl-L-, pyrazinyl-L- or pyridazinyl-L-; wherein, $R^2$ is unsubstituted or substituted by 1, 2, 3, 4 or 5 $R^w$;

each $R^w$ is independently H, D, F, Cl, Br, I, -CN, $-NO_2$, $-NH_2$, -OH, -SH, -COOH, $-C(=O)NH_2$, $-C(=O)NHCH_3$, $-C(=O)N(CH_3)_2$, $-C(=O)-(C_1-C_3$ alkyl), $-C(=O)-(C_1-C_3$ alkoxy), methyl, ethyl, *n*-propyl, isopropyl, *n*-butyl, *tert*-butyl, allyl, propenyl, propargyl, propynyl, $-CHF_2$, $-CF_3$, $-CHFCH_2F$, $-CF_2CHF_2$, $-CH_2CF_3$, $-CH_2CF_2CHF_2$, methoxy, ethoxy, *n*-propyloxy, isopropyloxy, $-OCHF_2$, $-OCF_3$, $-OCHFCH_2F$, $-OCF_2CHF_2$, $-OCH_2CF_3$, $-OCH_2CF_2CHF_2$, methylthio, ethylthio, methylamino, dimethylamino, ethylamino, hydroxymethyl, 2-hydroxyethyl, cyclopropyl, cyclobutyl, cyclopentyl, cyclohexyl, aziridine, pyrrolyl, tetrahydrofuranyl, piperidinyl, piperazine, morpholinyl, phenyl, pyrrolyl, pyrazolyl, imidazolyl, triazolyl, tetrazolyl, furanyl, thiophenyl, thiazolyl, oxazolyl, pyridinyl, pyrimidinyl, pyrazinyl or pyridazinyl.

**11.** The compound according to any one of claims 1-10, wherein it is a compound represented by formula (II) or formula (III), or a stereoisomer, tautomer, N-oxide, hydrate, solvate, metabolite, pharmaceutically acceptable salt or prodrug thereof,

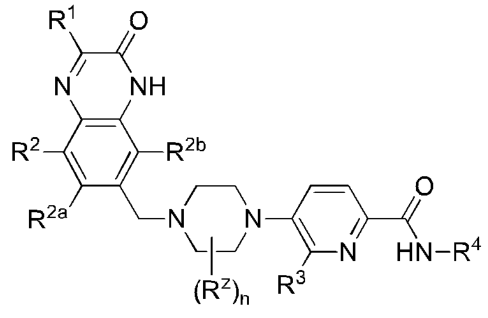

(II)

or

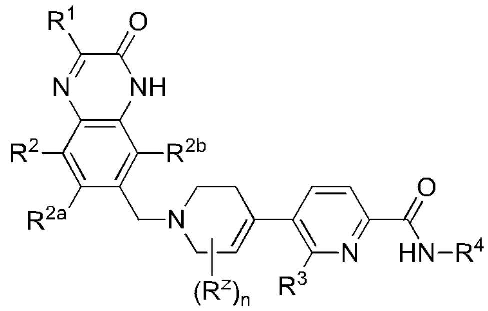

(III),

wherein, each of $R^1$, $R^2$, $R^{2a}$, $R^{2b}$, $R^3$, $R^z$, $R^4$ and n has the meaning as described in any one of claims 1-10.

**12.** A compound having one of the following structures, or a stereoisomer, tautomer, N-oxide, hydrate, solvate, metabolite, pharmaceutically acceptable salt, or prodrug thereof:

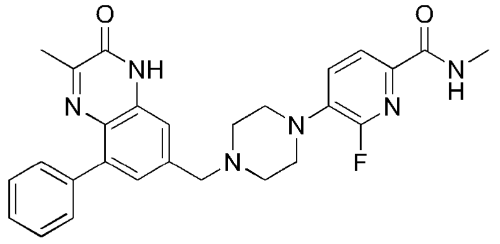 2,

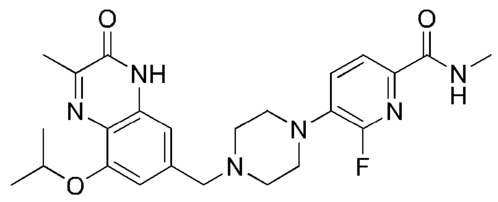 3,

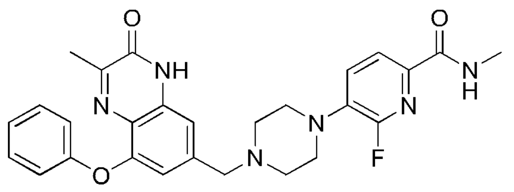 4,

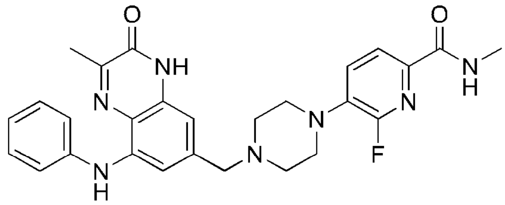 5,

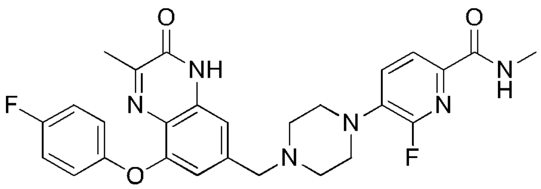 6,

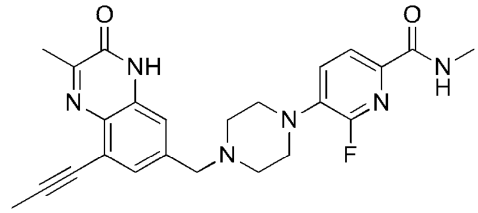 7,

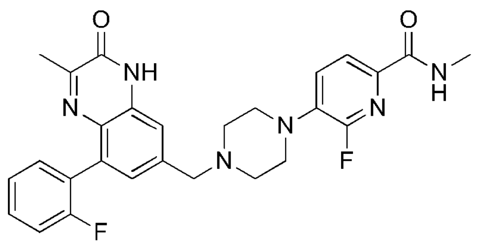 8,

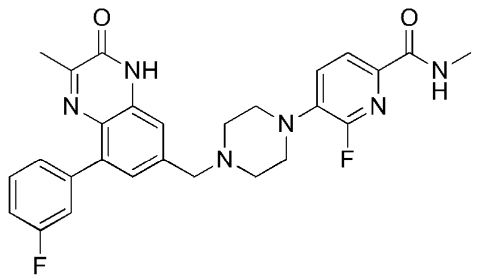 9,

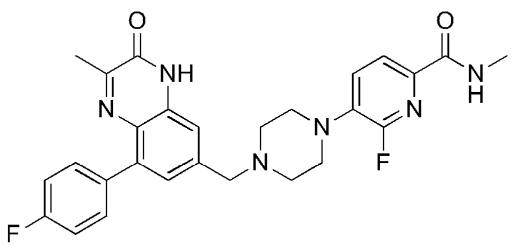 10,

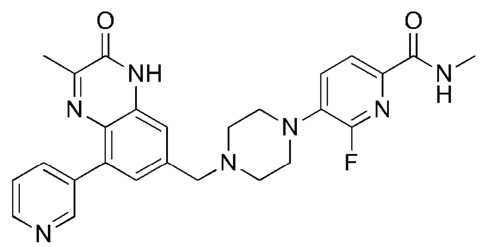 11,

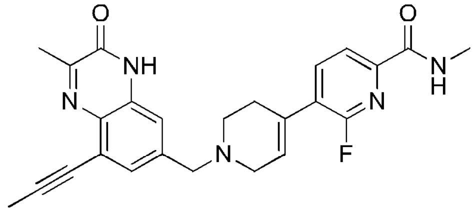 12,

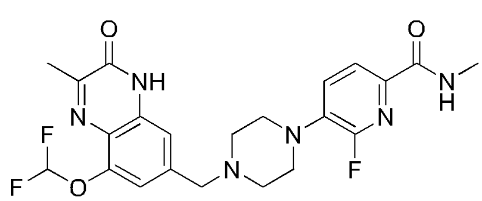 13,

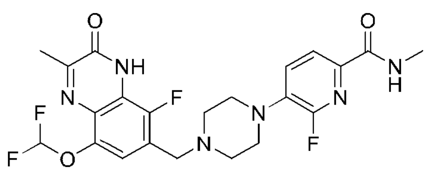
14,

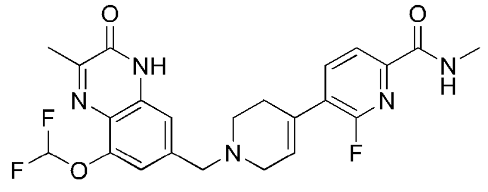
15,

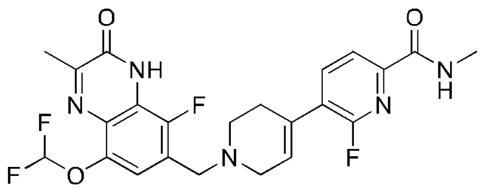
16,

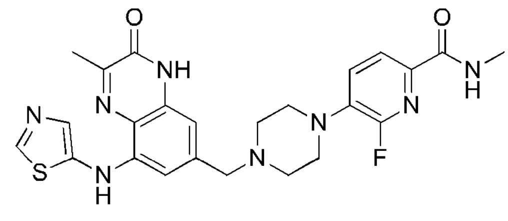
17,

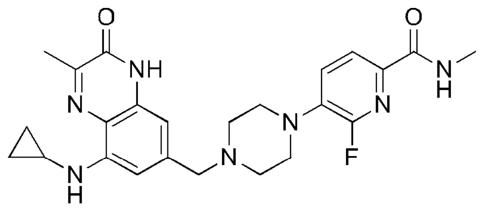
18,

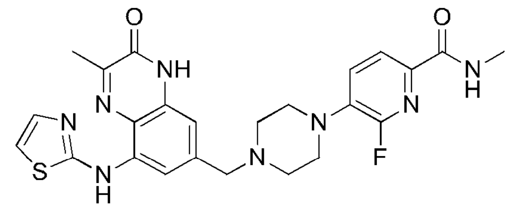
19,

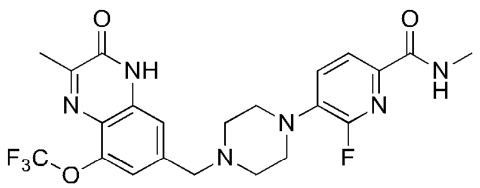
20,

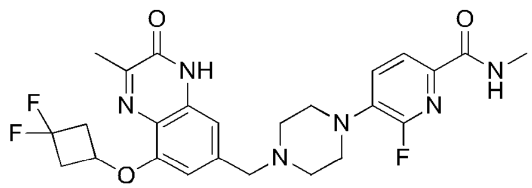
21,

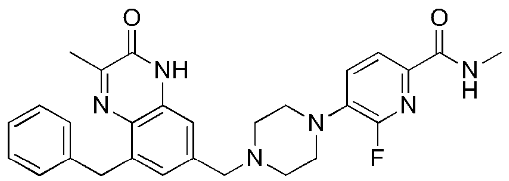
22,

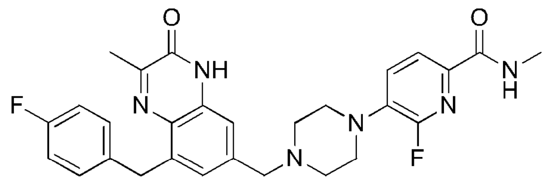
23,

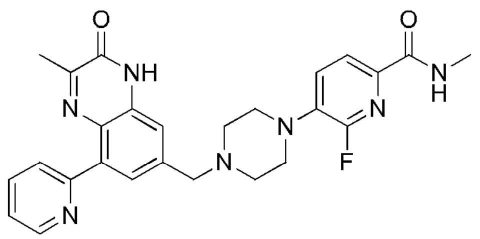
24,

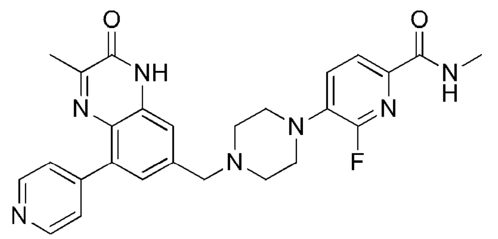
25,

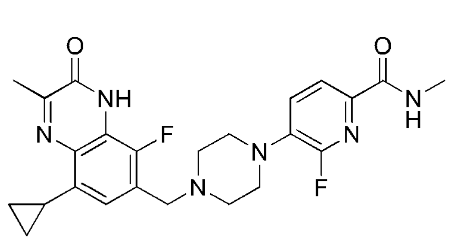
26,

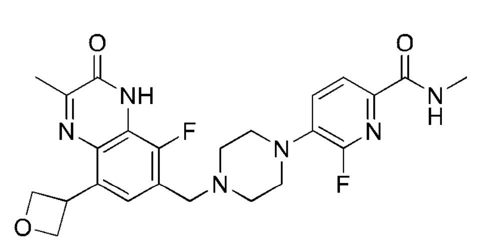
27,

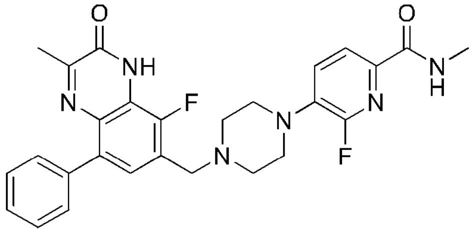
28,

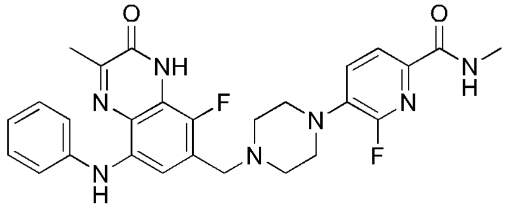
29,

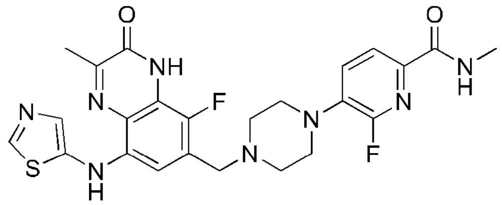
30,

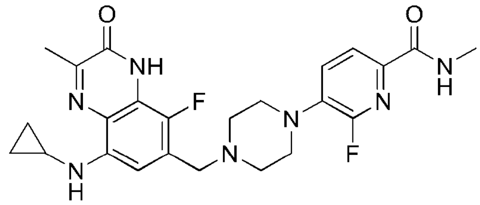
31,

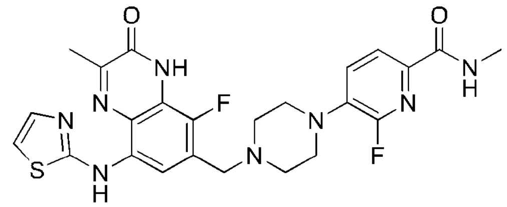
32,

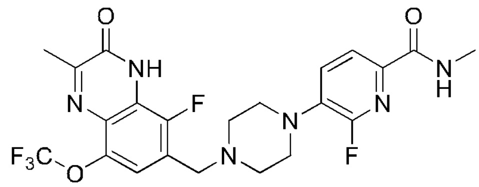
33,

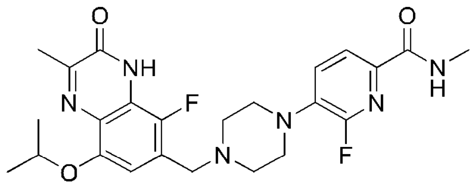
34,

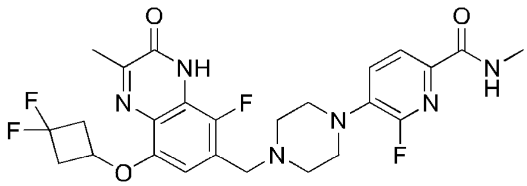
35,

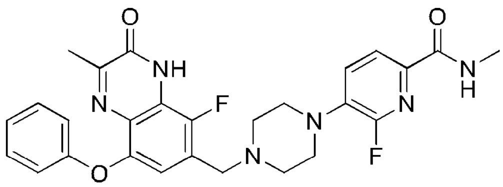
36,

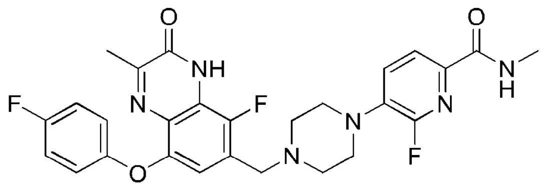
37,

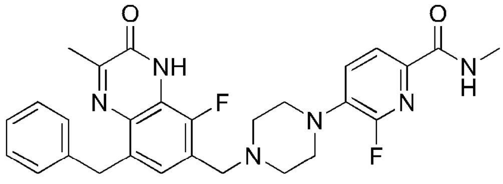
38,

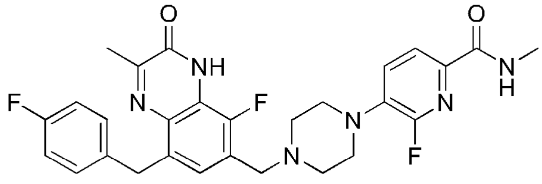
39,

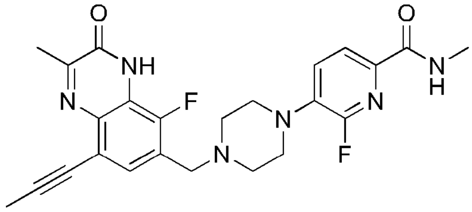
40,

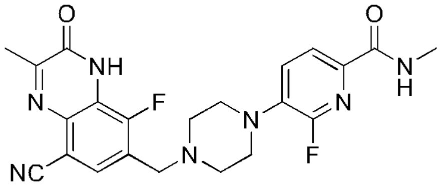
41,

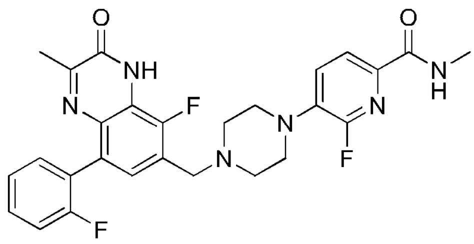 42, 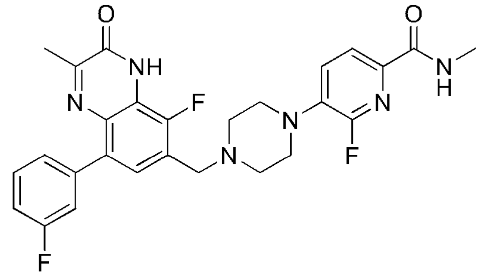 43,

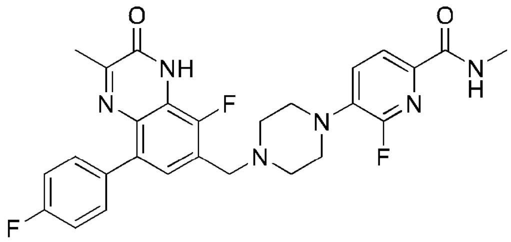 44, 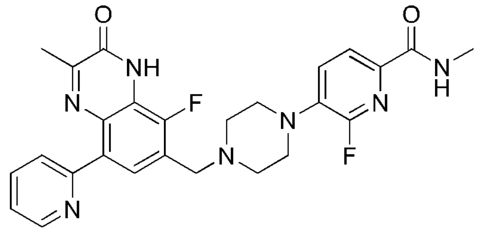 45,

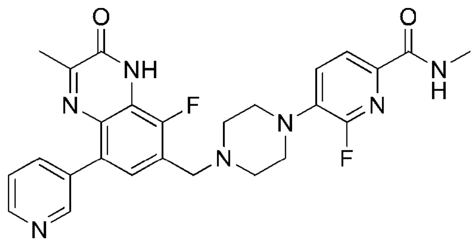 46, 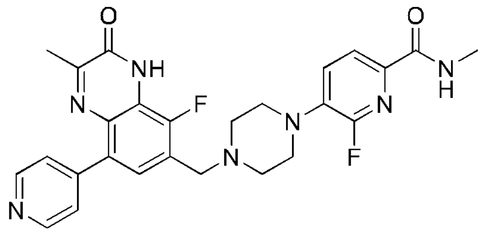 47,

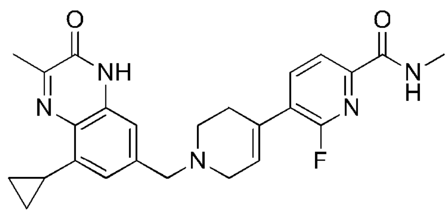 48, 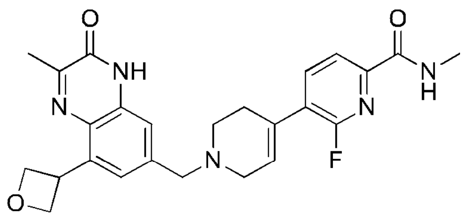 49,

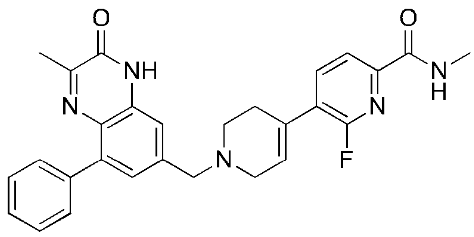 50, 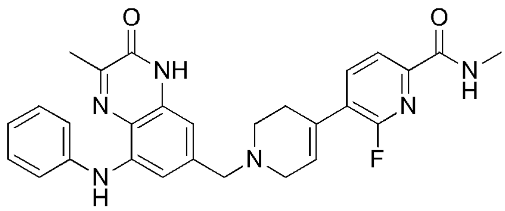 51,

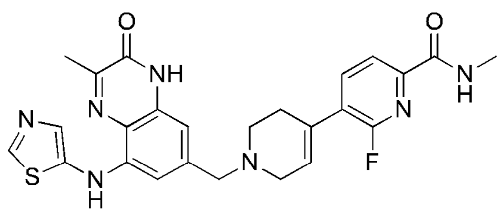 52, 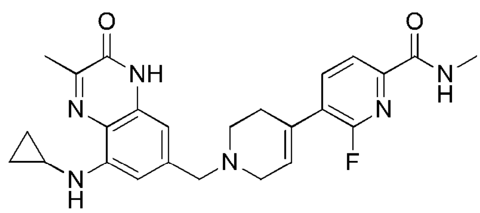 53,

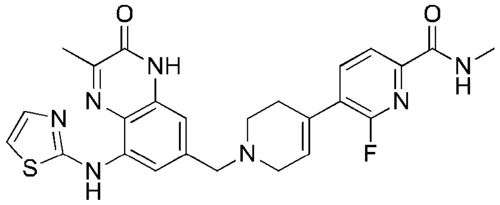 54,

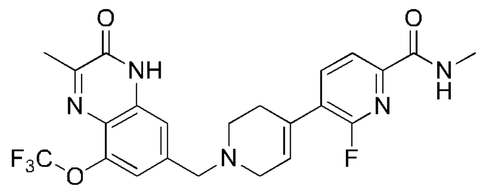 55,

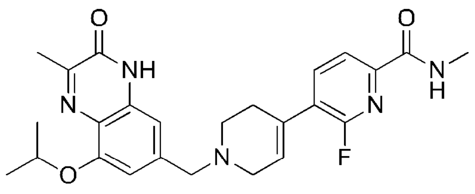 56.

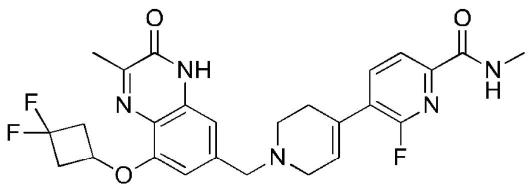 57.

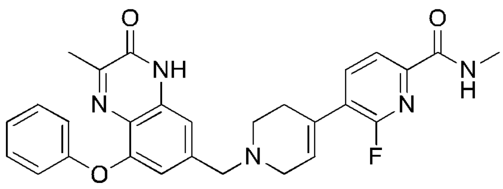 58,

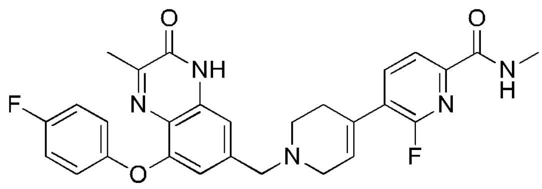 59,

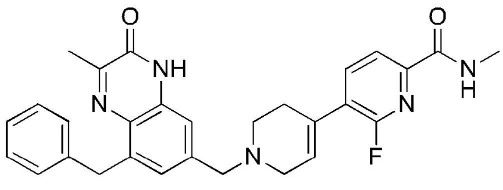 60,

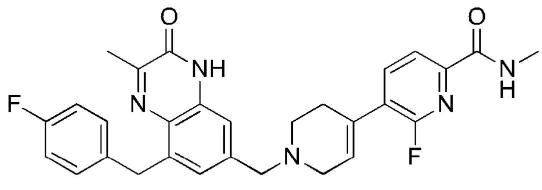 61,

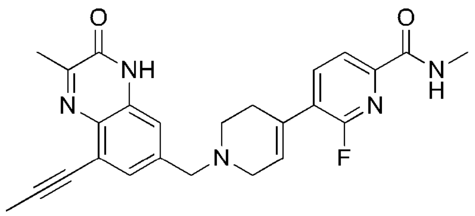 62,

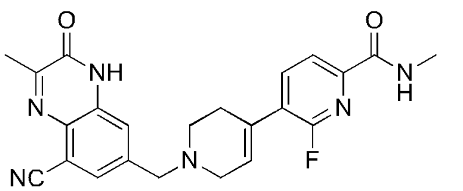 63,

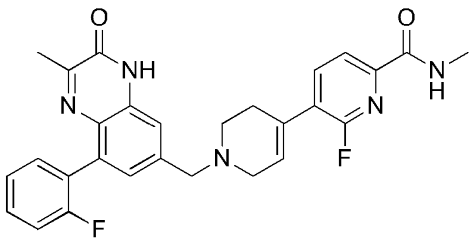 64,

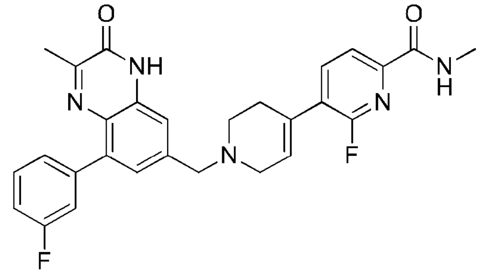 65,

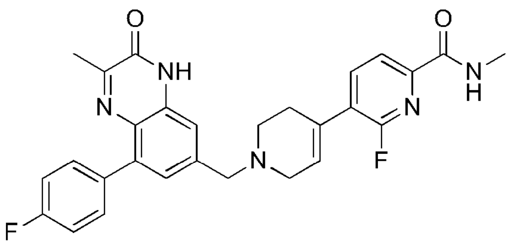 66,

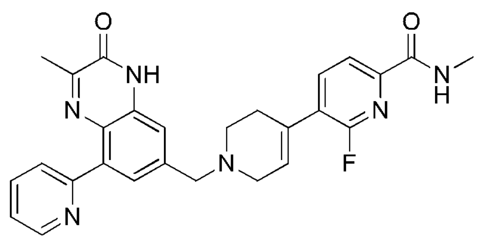 67,

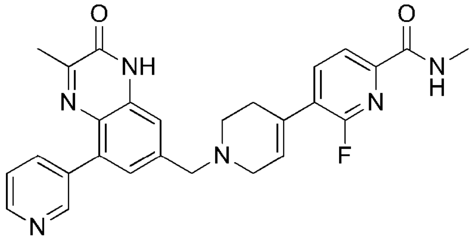
68,

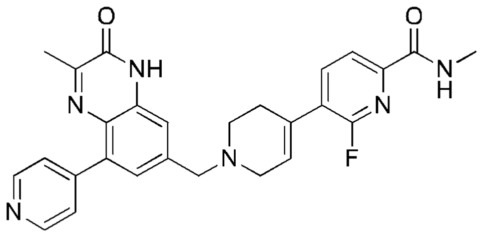
69,

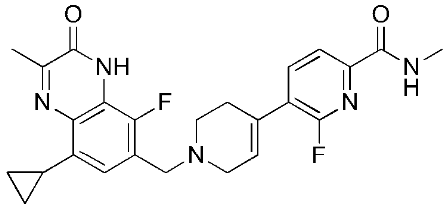
70,

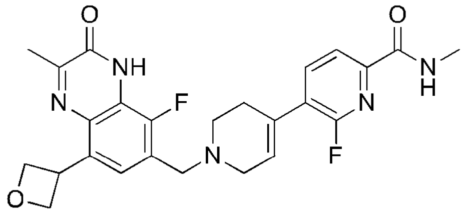
71,

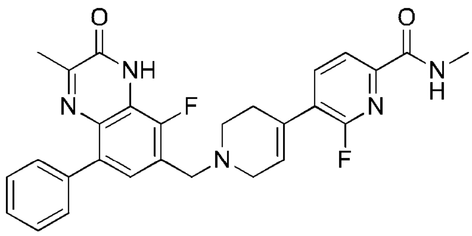
72,

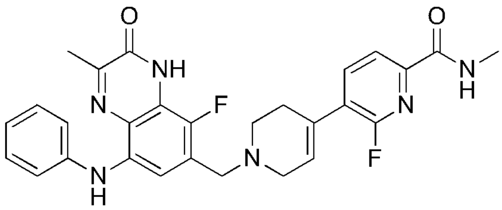
73,

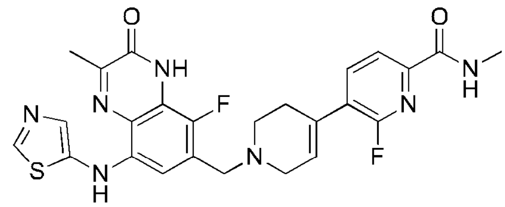
74,

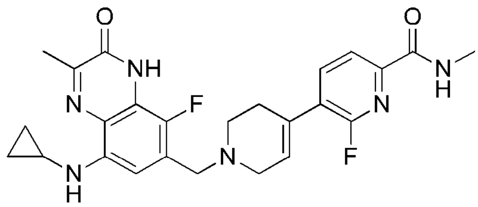
75,

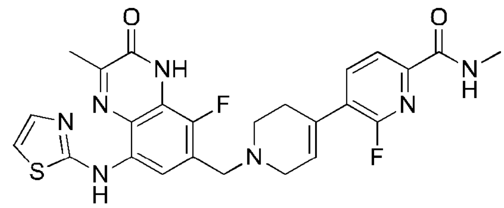
76,

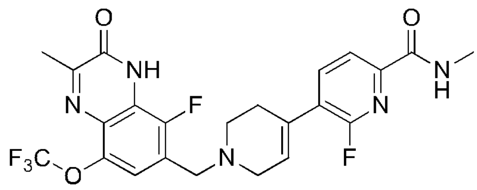
77,

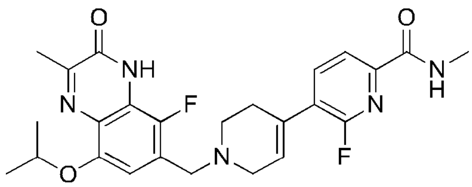
78,

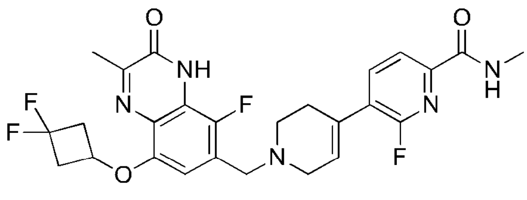
79,

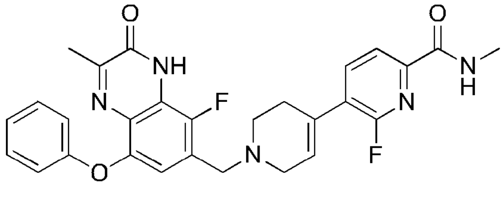
80,

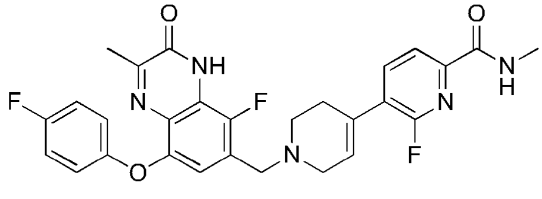
81,

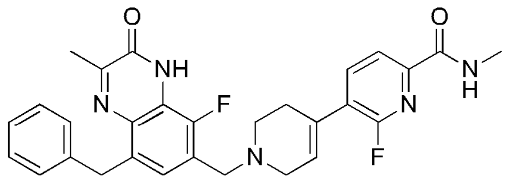 82,

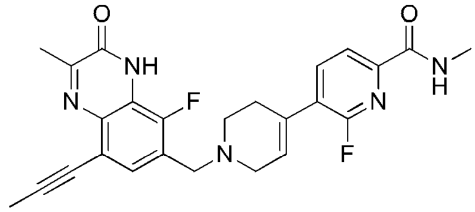 83,

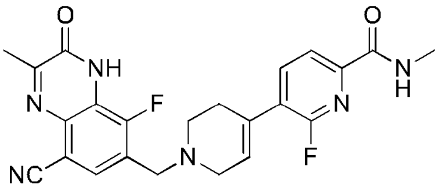 84,

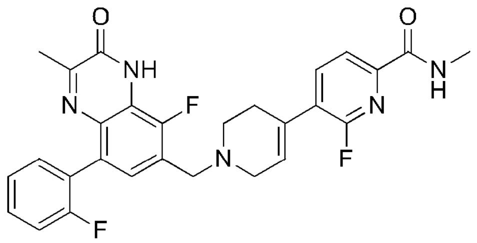 85,

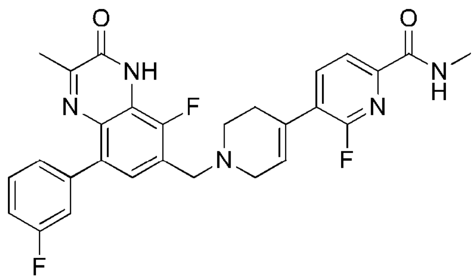 86,

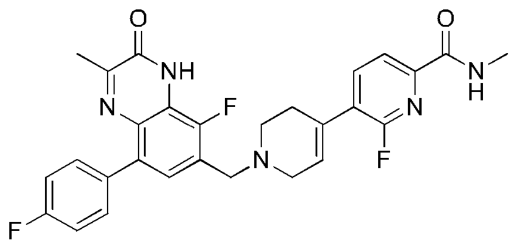 87,

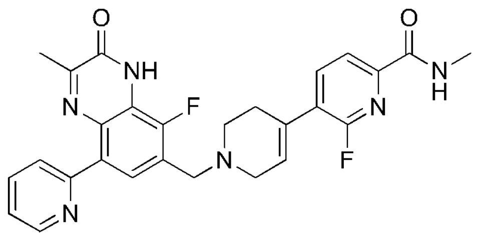 88,

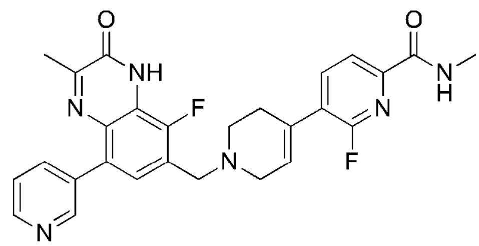 89,

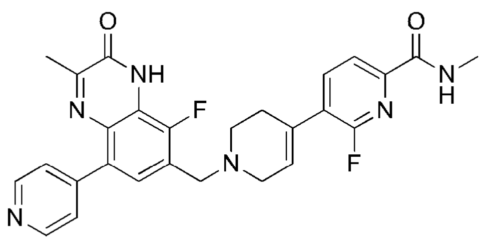 90,

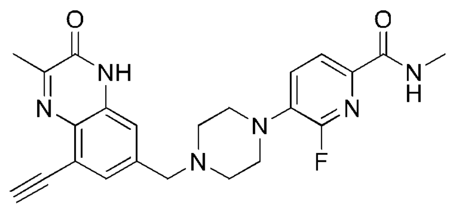 91,

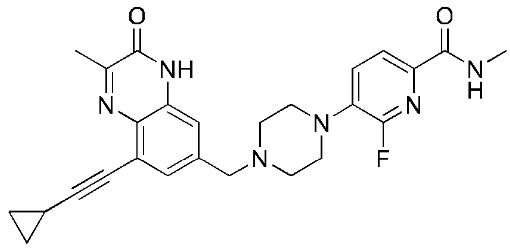 92,

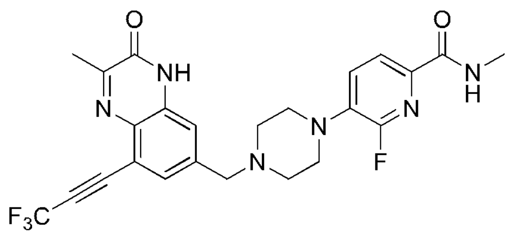 93,

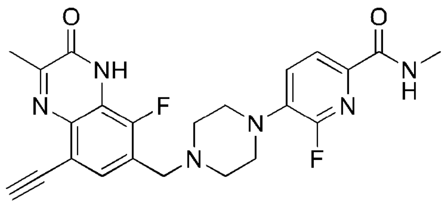 94, 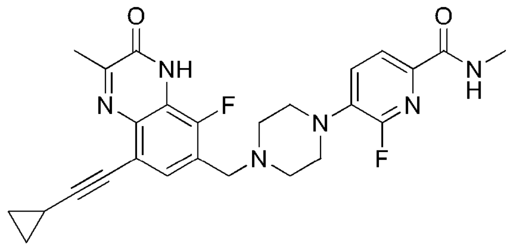 95,

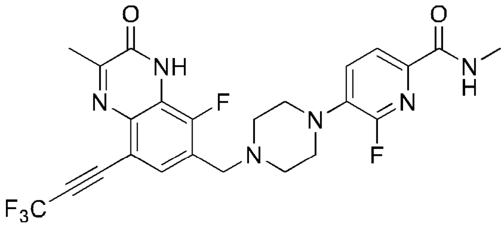 96, 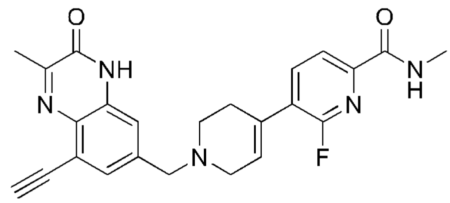 97,

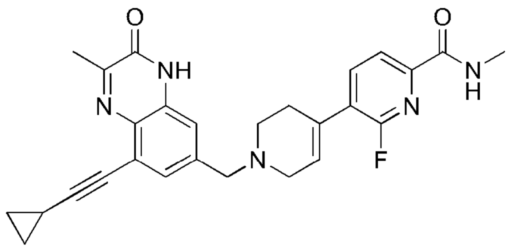 98, 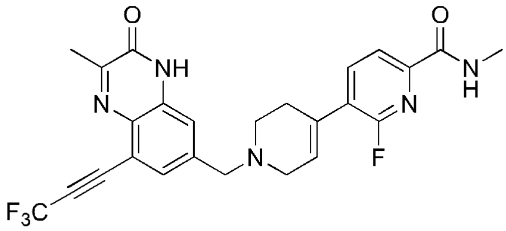 99,

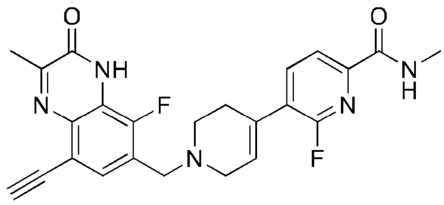 100, 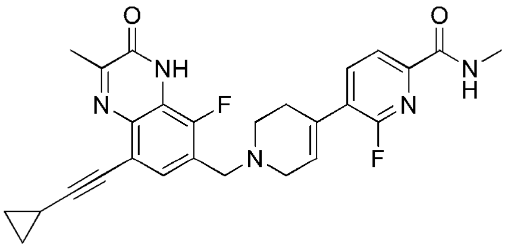 101,

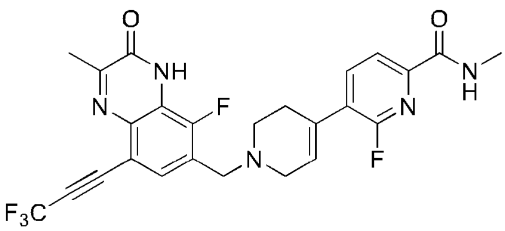 102, 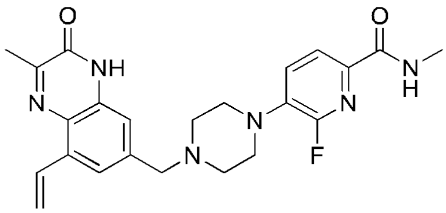 103,

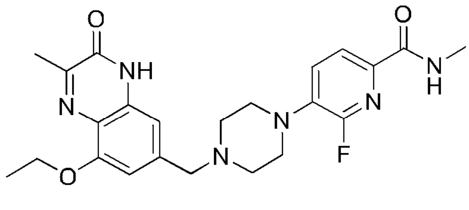 104, 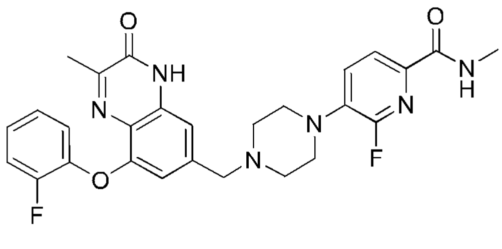 105,

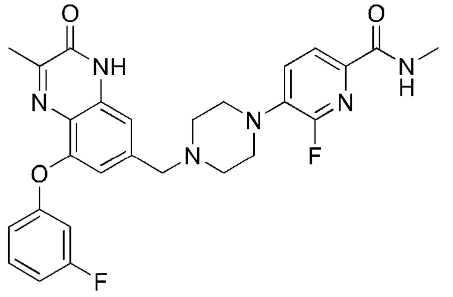

106,

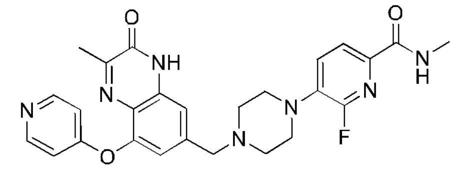

107,

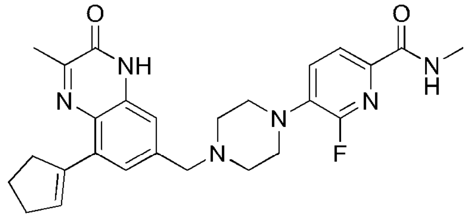

108,

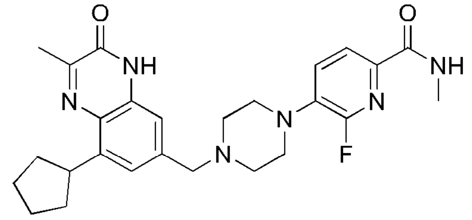

109,

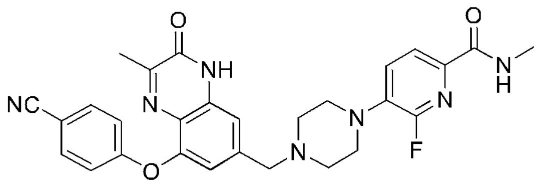

110,

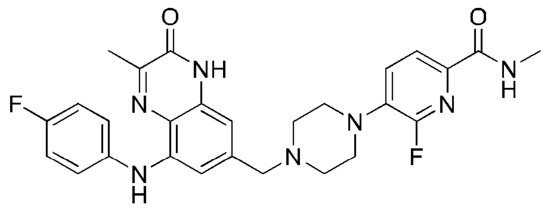

111,

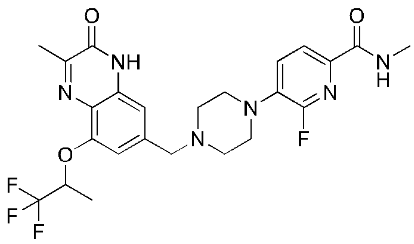

112,

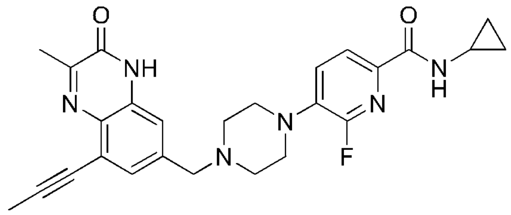

113,

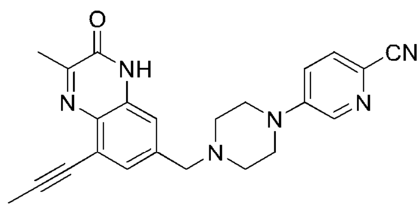

114,

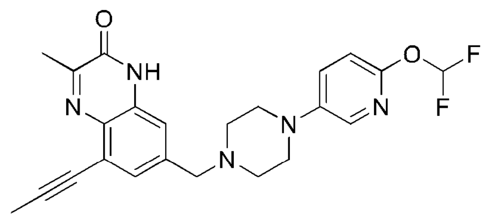

115,

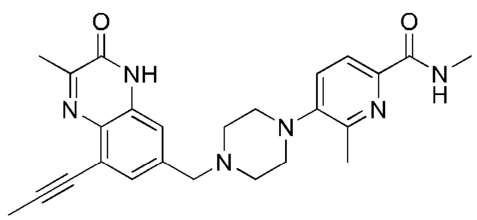

116,

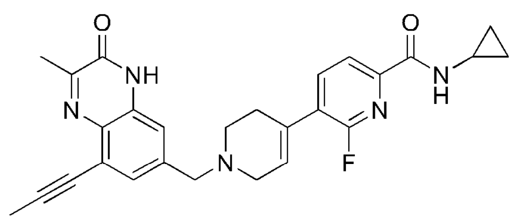

117,

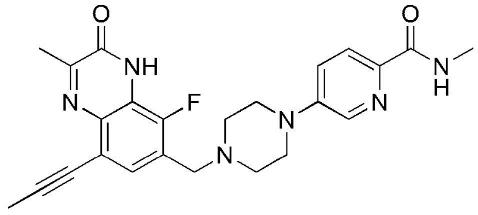
118,

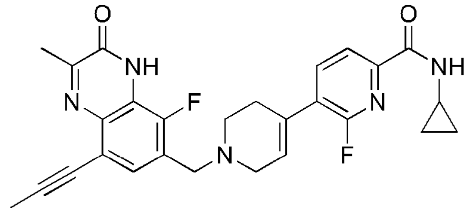
119,

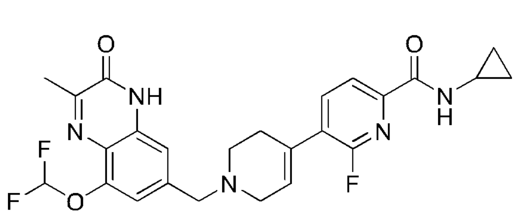
120,

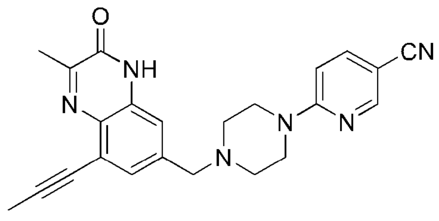
121,

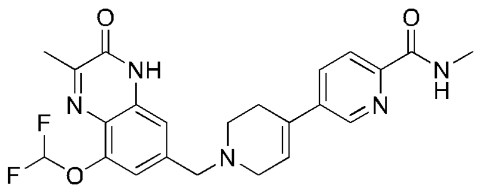
122,

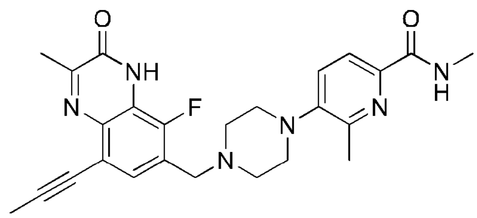
123,

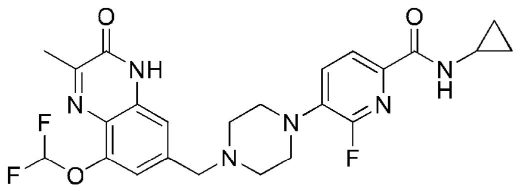
124,

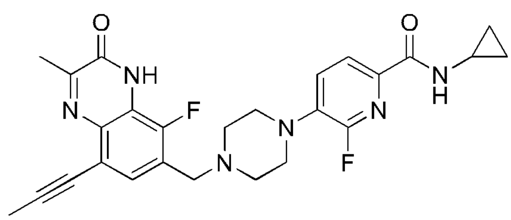
125,

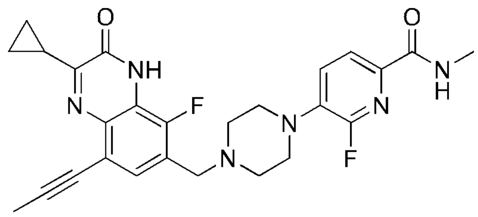
126,

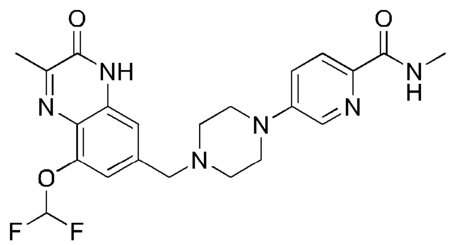
127,

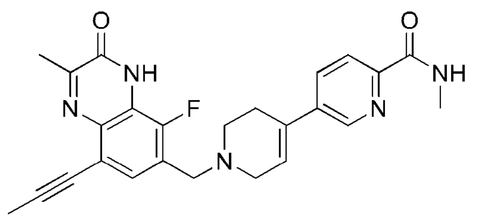
128,

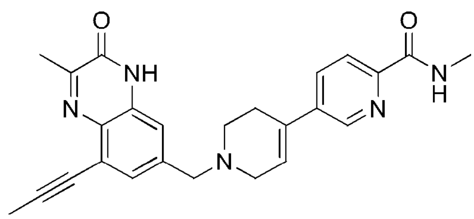
129,

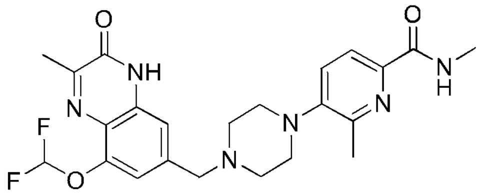 130, 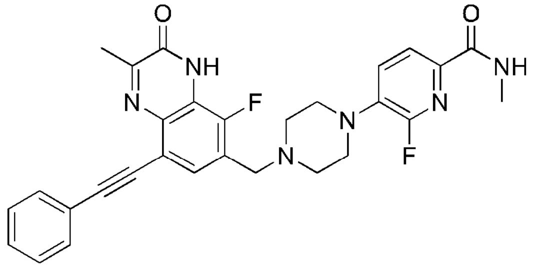 131,

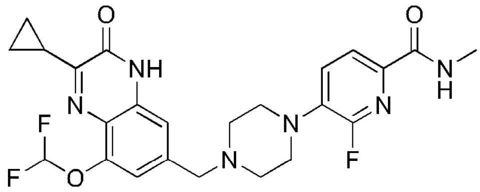 132, 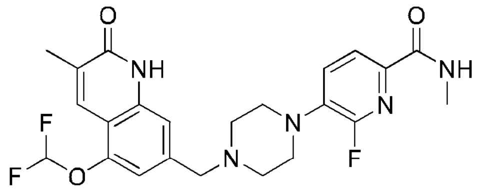 133,

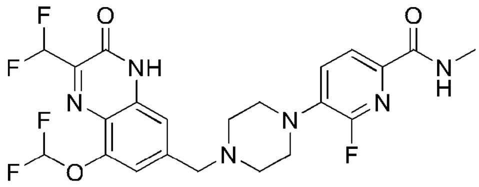 134, 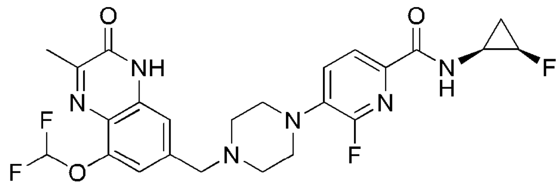 135,

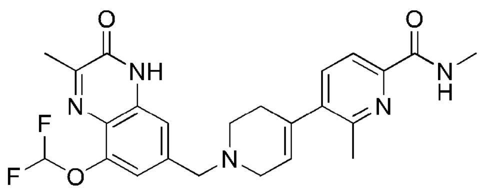 136, 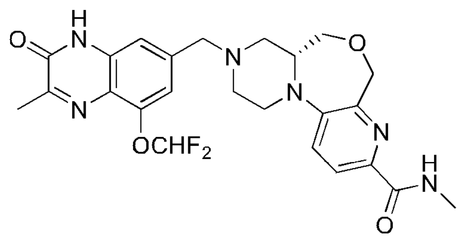 137,

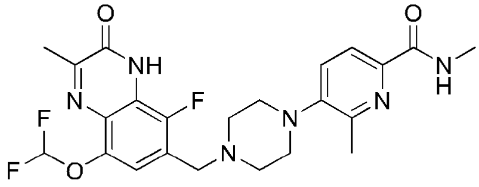 138, 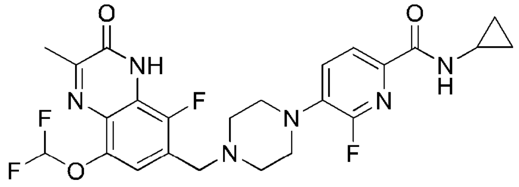 139,

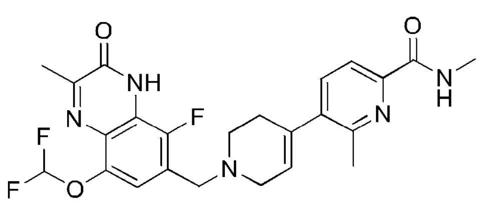 140, 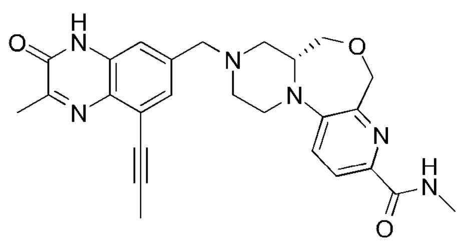 141,

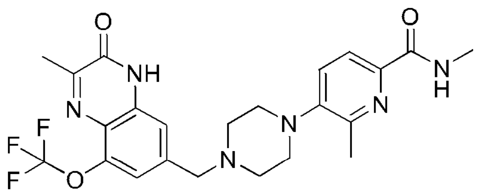 142, 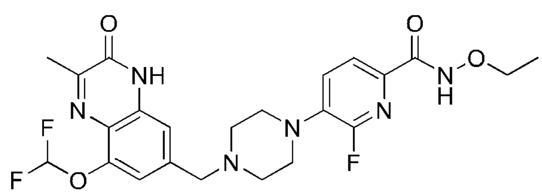 143,

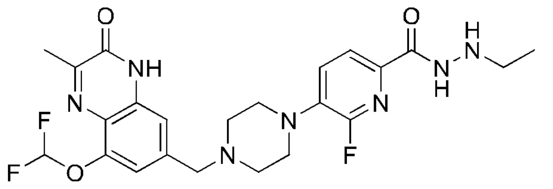 144,

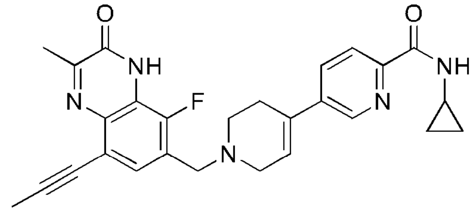 145,

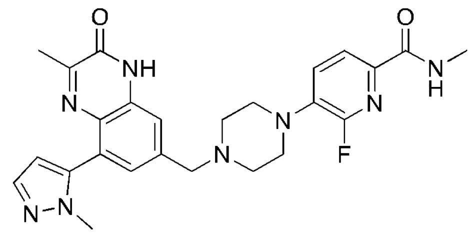 146,

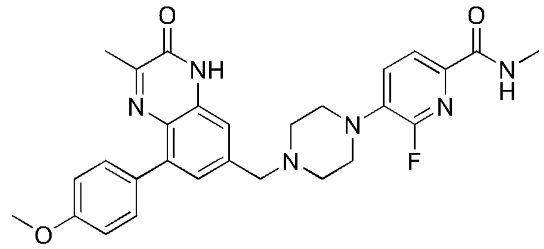 147,

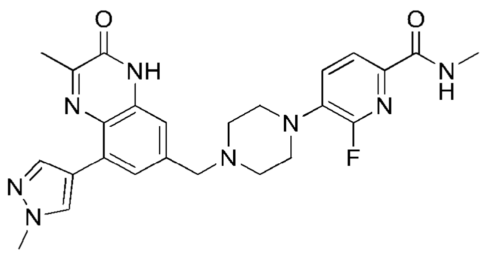 148,

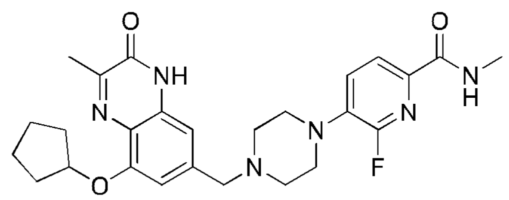 149,

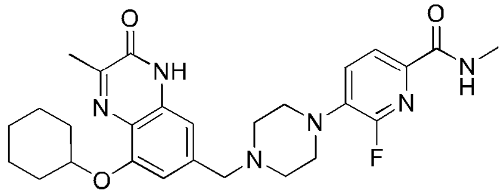 150,

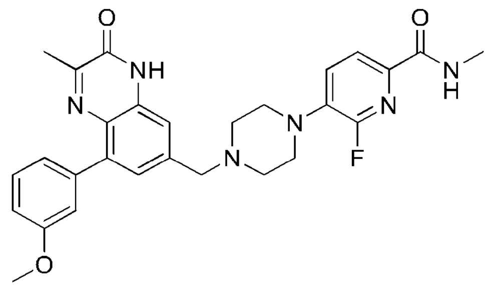 151,

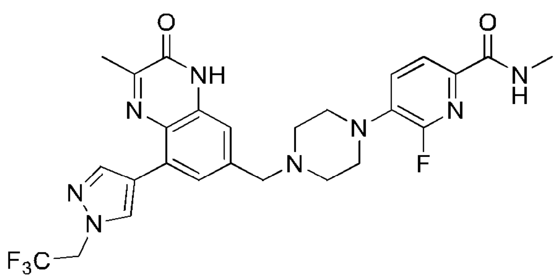 152,

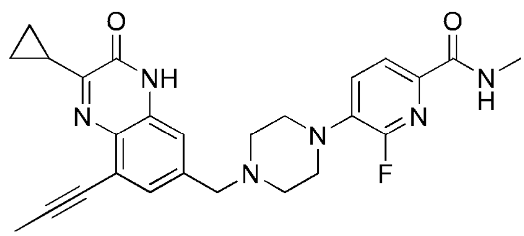 153

or

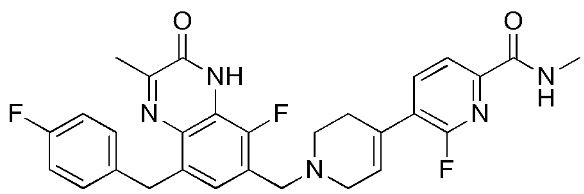 154.

**13.** A pharmaceutical composition comprising the compound of any one of claims 1 to 12; and
the pharmaceutical composition optionally further comprising a pharmaceutically acceptable excipient, carrier, adjuvant or any combination thereof.

**14.** Use of the compound of any one of claims 1-12 or the pharmaceutical composition of claim 13 in the manufacture of a medicament for preventing, treating or relieving of PARP1 mediated diseases;
wherein the PARP1 mediated diseases are cancer, neurodegenerative diseases, cardiovascular diseases, ischemic diseases, diabetic neuropathy, reperfusion injury, osteoarthritis, osteoporosis, inflammatory diseases or metabolic diseases.

**15.** The use according to claim 14, wherein the cancer is laryngeal cancer, esophageal cancer, gastric cancer, intestinal cancer, liver cancer, kidney cancer, lung cancer, brain cancer, head and neck cancer, squamous cell carcinoma, lymphatic system cancer, thyroid cancer, bladder cancer, ovarian cancer, cervical cancer, prostate cancer, urogenital tract cancer, breast cancer, hematologic malignancies, small cell lung cancer, lung adenocarcinoma, pancreatic cancer, colon cancer, glioblastoma and/or monocytic leukemia.

**INTERNATIONAL SEARCH REPORT**

International application No.
**PCT/CN2024/107683**

**A. CLASSIFICATION OF SUBJECT MATTER**

C07D401/12(2006.01)i; C07D401/14(2006.01)i; A61K31/498(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, ENTXT, VEN, CNKI, 百度, BAIDU, 谷歌学术, GOOGLE SCHOLAR, STN (REGISTRY, CAPLUS, MARPAT): 广东东阳光药业, 刘兵, 柏舜, 王峰, 潘嘉伟, 唐剑耀, 林梦灵, 饶志鹏, 张英俊, 喹喔啉, 酮, 氧代, 羰基, 哌嗪, 吡啶, 酰胺, 癌, PARP1, quinoxalin?, oxo?, piperazi?, pyridin?, carboxamid?, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2023232069 A1 (CHENGDU EASTON BIOPHARMACEUTICALS CO., LTD.) 07 December 2023 (2023-12-07)<br>see claims 1-13, and description, embodiments 10 and 22 | 1-15 |
| PX | CN 116693501 A (CHENGDU BAIYU PHARMACEUTICAL CO., LTD.) 05 September 2023 (2023-09-05)<br>see claims 1-9, and description, embodiments 1 and 6, and pages 1 and 10 | 1-15 |
| PX | WO 2023146957 A1 (XINTHERA INC.) 03 August 2023 (2023-08-03)<br>see abstract, claim 1, and table 1 | 1-15 |
| PX | WO 2023217045 A1 (MEDSHINE DISCOVERY INC.) 16 November 2023 (2023-11-16)<br>see abstract, and claims 1 and 24 | 1-15 |
| X | WO 2022223025 A1 (SHANGHAI HANSOH BIOMEDICAL CO., LTD. et al.) 27 October 2022 (2022-10-27)<br>see claims 1, 12, and 20-22, and description, pages 56-57 | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 September 2024** | **09 October 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/107683**

**C. DOCUMENTS CONSIDERED TO BE RELEVANT**

| Category* | Citation of document, with indication, where appropriate, of the relevant passages | Relevant to claim No. |
|---|---|---|
| A | CN 115232154 A (SHANGHAI HANSOH BIOMEDICAL CO., LTD.) 25 October 2022 (2022-10-25)<br>see claims 1-13, and description, page 34 | 1-15 |
| A | CN 116425744 A (YOULING MEDICINE TECHNOLOGY (HONGKONG) CO., LTD.) 14 July 2023 (2023-07-14)<br>see claims 1-19 | 1-15 |

Form PCT/ISA/210 (second sheet) (July 2022)

**INTERNATIONAL SEARCH REPORT**
**Information on patent family members**

International application No.
**PCT/CN2024/107683**

| Patent document cited in search report | Publication date (day/month/year) | Patent family member(s) | Publication date (day/month/year) |
|---|---|---|---|
| WO 2023232069 A1 | 07 December 2023 | CN 118076597 A | 24 May 2024 |
| CN 116693501 A | 05 September 2023 | None | |
| WO 2023146957 A1 | 03 August 2023 | CN 118510767 A | 16 August 2024 |
| WO 2023217045 A1 | 16 November 2023 | None | |
| WO 2022223025 A1 | 27 October 2022 | EP 4328225 A1 | 28 February 2024 |
| | | JP 2024514338 A | 01 April 2024 |
| | | TW 202309026 A | 01 March 2023 |
| | | CA 3215823 A1 | 27 October 2022 |
| | | BR 112023019797 A2 | 07 November 2023 |
| | | MX 2023012054 A | 23 October 2023 |
| | | AU 2022261029 A1 | 19 October 2023 |
| | | US 2024228490 A1 | 11 July 2024 |
| | | IL 307824 A | 01 December 2023 |
| | | KR 20240005756 A | 12 January 2024 |
| CN 115232154 A | 25 October 2022 | EP 4328225 A1 | 28 February 2024 |
| | | JP 2024514338 A | 01 April 2024 |
| | | TW 202309026 A | 01 March 2023 |
| | | CA 3215823 A1 | 27 October 2022 |
| | | BR 112023019797 A2 | 07 November 2023 |
| | | WO 2022223025 A1 | 27 October 2022 |
| | | MX 2023012054 A | 23 October 2023 |
| | | AU 2022261029 A1 | 19 October 2023 |
| | | US 2024228490 A1 | 11 July 2024 |
| | | IL 307824 A | 01 December 2023 |
| | | KR 20240005756 A | 12 January 2024 |
| CN 116425744 A | 14 July 2023 | TW 202327605 A | 16 July 2023 |
| | | WO 2023134647 A1 | 20 July 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4328245 A
- US 4409239 A
- US 4410545 A
- US 5023252 A
- WO 2021013735 A

### Non-patent literature cited in the description


- Additionally, general principles of organic chemistry are described in "Organic Chemistry. Handbook of Chemistry and Physics. University Science Books, 1994
- **SMITH et al.** March's Advanced Organic Chemistry. John Wiley & Sons, 2007
- **S. P. PARKER, ED**. McGraw-Hill Dictionary of Chemical Terms. McGraw-Hill Book Company, 1984
- **ELIEL, E** ; **WILEN, S**. Stereochemistry of Organic Compounds. John Wiley & Sons, Inc, 1994
- **JACQUES et al.** Enantiomers, Racemates and Resolutions. Wiley Interscience, 1981
- Principles of Asymmetric Synthesis. Elsevier, 2012
- **ELIEL, E.L**. Stereochemistry of Carbon Compounds. McGraw-Hill, 1962
- **WILEN, S.H**. Tables of Resolving Agents and Optical Resolutions. E.L. Eliel, Ed., Univ. of Notre Dame Press, Notre Dame, 1972, 268
- Chiral Separation Techniques: A Practical Approach. Wiley-VCH Verlag GmbH & Co. KGaA, 2007
- **GREENE et al.** Protective Groups in Organic Synthesis. John Wiley & Sons, 1991
- **KOCIENSKI et al.** Protecting Groups. *Thieme*, 2005
- **S. M. BERGE et al.** *J. Pharmaceutical Sciences*, 1977, vol. 66, 1-19
- Remington's Pharmaceutical Sciences''. Mack Publishing Company, 1985
- Handbook of Pharmaceutical Salts: Properties, Selection, and Use. Wiley-VCH, 2002
- **ANSEL H. C et al.** Ansel's Pharmaceutical Dosage Forms and Drug Delivery Systems. Lippincott, Williams & Wilkins, 2004
- **GENNARO A. R. et al.** Remington: The Science and Practice of Pharmacy. Lippincott, Williams & Wilkins, 2000
- **ROWE R. C.** Handbook of Pharmaceutical Excipients. Pharmaceutical Press, 2005
- Remington's Pharmaceutical Sciences. Mack Publishing Company
- The Handbook of Pharmaceutical Additives (Gower Publishing Limited), and The Handbook of Pharmaceutical Excipients. American Pharmaceutical Association and the Pharmaceutical Press
- Remington's Pharmaceutical Sciences. Mack Publishing Company Co., 1990
- Various carriers for preparing pharmaceutically acceptable compositions and known techniques for their preparation are disclosed. **REMINGTON**. The Science and Practice of Pharmacy. Lippincott Williams & Wilkins, 2005
- Encyclopedia of Pharmaceutical Technology. Marcel Dekker, 1988
- *Pharmaceutical Research*, 1986, vol. 3 (6), 318
- **PATEL, M.M**. *Expert Opin. Drug Deliv. [Expert Opinion on Drug Delivery*, 2011, vol. 8 (10), 1247-1258
- Remington's Pharmaceutical Sciences. Mack Publishing Company, 1985
- **GILMAN et al.** The Pharmacological Bases of Therapeutics. Pergamon Press, 1990
- Remington's Pharmaceutical Sciences. Mack Publishing Company, 1990